# EUROPEAN PATENT SPECIFICATION

(11) **EP 4 294 800 B1**
(45) Date of publication and mention of the grant of the patent: **06.05.2026**
(21) Application number: 22757039.7
(22) Date of filing: 18.02.2022
(51) Int. Cl.: C07D 403/12, C07D 405/14, A61P 13/12, A61K 31/4025

(54) **APOL1 INHIBITORS AND METHODS OF USE**
APOL1-INHIBITOREN UND VERFAHREN ZUR VERWENDUNG
INHIBITEURS D'APOL1 ET MÉTHODES D'UTILISATION

(30) Priority: 19.02.2021 US 202163151605 P
(43) Date of publication of application: 27.12.2023
(73) Proprietor: Maze Therapeutics, Inc., South San Francisco, CA 94080 (US)
(72) Inventor: LEE, Patrick Sang Tae, South San Francisco, California 94080 (US); EWING, Todd Jonathan August, South San Francisco, California 94080 (US); REID, Adam Neil, South San Francisco, California 94080 (US); SINZ, Christopher Joseph, South San Francisco, California 94080 (US); ZHANG, Birong, South San Francisco, California 94080 (US); BRONNER, Sarah M., South San Francisco, California 94080 (US); MORGANS JR., David John, South San Francisco, California 94080 (US); HOEK, Maarten, South San Francisco, California 94080 (US); ASSIMON, Victoria Anne, South San Francisco, California 94080 (US)
(74) Representative: Mewburn Ellis LLP
(86) International application number: PCT/US2022/017086
(87) International publication number: WO 2022/178315

(56) References cited:
- WO-A1-02/12184
- WO-A1-91/13865
- US-A1- 2004 024 024
- US-A1- 2020 377 479
- DATABASE Pubchem compound ANONYMOUS : "N-[4-[2-Hydroxy-3-(2-phenylethylamino)propyloxy]phenyl]-N-methylmethanesulfonamide", XP055965189, retrieved from NCBI Database accession no. 44287171
- CONNORS ET AL.: "The Synthesis and Potassium Channel Blocking Activity of Some (4- Methanesulfonamidophenoxy)propanolamines as Potential Class III Antiarrhythmic Agents", J. MED. CHEM., vol. 34, 1991, pages 1570 - 1577, XP001093613, DOI: 10.1021/jm00109a007
- PARK ET AL.: "METABOLISM OF FLUORINE-CONTAINING DRUGS", ANNU. REV. PHARMACOL. TOXICOL., vol. 41, 2001, pages 443 - 70, XP009114978, DOI: 10.1146/annurev.pharmtox.41.1.443

## Description

### CROSS REFERENCE TO RELATED APPLICATIONS

This application claims priority to U.S. Provisional Application No. 63/151,605 filed on February 19, 2021.

### BACKGROUND OF THE INVENTION

Apolipoprotein L1 (APOL1) is a pore forming innate immunity factor, protecting individuals from trypanosome parasites (Vanhamme, L. et al. Nature (2003) 422, 83-87). The secreted form of APOL1 circulates in blood as part of distinct high-density lipoprotein (HDL) complexes, known as trypanosome lytic factors (TLFs) (Rifkin, M. R. Proc. Natl. Acad. Sci. USA. (1978) 75, 3450-3454; Raper, J. et al. Infect. Immun. (1999) 67, 1910-1916). TLFs are internalized by the parasites through endocytosis (Hager, K. M. et al. J. Cell Biol. (1994) 126, 155-167). Within trypanosomes, APOL1 forms cation pores, causing ion flux, swelling, and eventual lysis (Rifkin, M. R. Exp. Parasitol. (1984) 58, 81-93; Molina-Portela, M. P. et al. Mol. Biochem. Parasitol. (2005) 144, 218-226; Pérez-Morga, D. et al. Science. (2005) 309, 469-472; Thomson, R. & Finkelstein, A. Proc. Natl. Acad. Sci. USA. (2015) 112, 2894-2899).

Several *Trypanosoma brucei* subspecies (*T b. rhodesiense* and *T. b. gambiense*) developed resistance mechanisms to APOL1-dependent killing (Pays, E. et al. Nat. Rev. Microbiol. (2014) 12, 575-584). Positive selection resulted in APOL1 variants, G1 (S342G, I384M) and G2 (N388Δ, Y389Δ), capable of interfering with these resistance mechanisms (Genovese, G. et al. Science. (2010) 329, 841-845). However, individuals with any binary combination of these variants (G1/G1, G2/G2, or G1/G2), have a greater risk of developing a variety of chronic kidney diseases, including focal segmental glomerulosclerosis (FSGS), hypertension-attributed kidney disease, human immunodeficiency virus-associated nephropathy (HIVAN) (Genovese, G. et al. Science. (2010) 329, 841-845; Tzur, S. et al. Hum. Genet. (2010) 128, 345-350; Kopp, J. B. et al. J. Am. Soc. Nephrol. (2011) 22, 2129-2137), sickle cell nephropathy (Ashley-Koch, A. E. et al. Br. J. Haematol. (2011) 155, 386-394), lupus nephritis (Freedman, B. I. et al. Arthritis Rheumatol. (2014) 66, 390-396), and an increased rate of Glomerular Filtration Rate (GFR) decline in diabetic kidney disease (Parsa, A. et al. N. Engl. J. Med. (2013) 369, 2183-2196). The APOL1 high-risk genotype has also been associated with COVID-19 associated nephropathy and other viral nephropathies (Shetty, A. et al. J. Am. Soc. Nephrol. (2021) 32, 33-40; Chang, J. H. et al. Am. J. Kidney Dis. (2019) 73, 134-139). Moreover, decreased renal allograft survival has been observed after deceased-donor kidney transplantations from APOL1 high-risk genotype donors (Freedman, B. I. et al. Transplantation. (2016) 100, 194-202). In addition, having two APOL1 risk alleles increases risk for preeclampsia (Reidy, K. J. et al. Am. J. Hum. Genet. (2018) 103, 367-376) and sepsis (Chaudhary, N. S. et al. Clin. J. Am. Soc. Nephrol. (2019) 14, 1733-1740). There are no approved therapies for APOL1-associated nephropathy, and patients are treated based on the standard of care for their underlying form of chronic kidney disease. This presents a clear unmet need for therapies targeted to people with the APOL1 high-risk genotype.

Numerous studies have shown that APOL1 risk variants are toxic when overexpressed in human cells (Wan, G. et al. J. Biol. Chem. (2008) 283, 21540-21549; Lan, X. et al. Am. J. Physiol. Renal Physiol. (2014) 307, F326-F336; Olabisi, O. A. et al. Proc. Natl. Acad. Sci. USA. (2016) 113, 830-837; Ma, L. et al. J. Am. Soc. Nephrol. (2017) 28, 1093-1105; Lannon, H. et al. Kidney Int. (2019) 96, 1303-1307). Recent findings suggest that this toxicity is associated with APOL1 pore function (Giovinazzo, J. A. et al. eLife. (2020) 9, e51185). Thus, there is a need to develop compounds suitable for inhibiting APOL1 activity and methods for inhibiting the activity of APOL1 using such compounds.
US 2020/0377479 describes compounds comprising two cyclic units joined by a linking unit which act as APOL1 inhibitors, and their use in treating focal segmental glomerulosclerosis (FSGS) and/or non-diabetic kidney disease. US 2004/024024 describes compounds having a similar structure which act as MMP (matric metalloprotease) inhibitors. WO 91/13865 describes compounds also having this structure which act as antiarrhythmic agents.

### BRIEF SUMMARY OF THE INVENTION

This disclosure describes compounds and compositions that may be useful for the treatment of APOL1-mediated diseases, including a variety of chronic kidney diseases such as FSGS, hypertension-attributed kidney disease, HIVAN, sickle cell nephropathy, lupus nephritis, diabetic kidney disease, viral nephropathy, COVID-19 associated nephropathy, and APOL1-associated nephropathy. The compounds and compositions may also find use in treating other APOL1-mediated disorders such as preeclampsia and sepsis. Additionally, for individuals with the APOL1 high-risk genotype, the disclosed compounds and compositions could have utility in preventing the onset of non-diabetic renal disease and/or delaying the progression of any form of chronic kidney disease. The disclosed chemical matter could also have utility in preventing and/or delaying progressive renal allograft loss in patients who have received a kidney transplant from a high-risk APOL1 genotype donor. Any references to methods of treatment in the subsequent paragraphs of this description are to be interpreted as references to the compounds, pharmaceutical compositions and medicaments of the present invention for use in a method for treatment of the human (or animal) body by therapy (or for diagnosis).

In one aspect, provided herein is a compound of formula (A'): 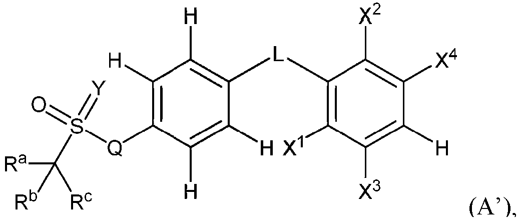 or a stereoisomer or tautomer thereof, or a pharmaceutically acceptable salt of any of the foregoing, wherein:
Q is absent or is -N-(C₁₋₆alkyl);
Y is O or -N-(C₁₋₆alkyl),
   provided that, when Q is -N(C₁₋₆alkyl), then Y is O;
R^{a}, R^{b}, and R^{c} are each independently H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl,
or any two of R^{a}, R^{b}, and R^{c} are taken, together with the atoms to which they are attached, to form a C₃₋₆cycloalkyl or a 3-6 membered heterocyclyl, and the other of R^{a}, R^{b}, and R^{c} is H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl;
L is selected from the group consisting of:
   (i) 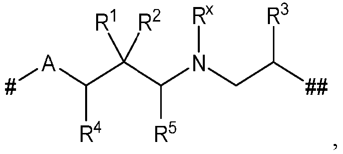 wherein
      A is O, NH, N(C₁₋₆alkyl), CH₂, or CH(C₁₋₆alkyl);
      R^{x} is H,
      or R^{x} is taken together with one of R¹ and R², and the atoms to which they are attached, to form a 3-8 membered heterocyclyl, wherein the 3-8 membered heterocyclyl is substituted with *n* independently selected R^{g} substituents, wherein *n* is an integer from 0-6, and R^{g} is -OH, halo, C₁₋₆alkyl, or C₁₋₆alkoxy;
      R¹ and R² are independently H, halo, or -OH,
      or one of R¹ and R² is taken together with R^{x}, and the atoms to which they are attached, to form a 3-8 membered heterocyclyl, wherein the 3-8 membered heterocyclyl is substituted with *n* independently selected R^{g} substituents, wherein *n* is an integer from 0-6, and R^{g} is -OH, halo, C₁₋₆alkyl, or C₁₋₆alkoxy, and the other of R¹ and R² is H, halo, or - OH;
      R³ is H, -OH, halo, or C₁₋₆alkoxy; and
      R⁴ and R⁵ are independently H,
      or R⁴ and R⁵ are taken, together with the atoms to which they are attached, to form a C₃₋₈cycloalkyl,
      provided that either:
         (1) R^{x} is taken together with one of R¹ and R², and the atoms to which they are attached, to form a 3-8 membered heterocyclyl, wherein the 3-8 membered heterocyclyl is optionally substituted with one or more -OH, C₁₋₆alkyl, or C₁₋₆alkoxy, or
         (2) R⁴ and R⁵ are taken, together with the atoms to which they are attached, to form a C₃₋₈cycloalkyl,
   (ii) 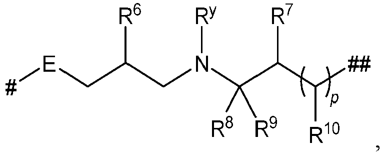 wherein
      E is O, NH, N(C₁₋₆alkyl), CH₂, or CH(C₁₋₆alkyl);
      *p* is 0 or 1,
         provided that, when *p* is 1, then E is O;
      R⁶ is H or -OH;
      R^{y} is H,
      or R^{y} is taken together with R⁷, and the atoms to which they are attached, to form a 3-8 membered heterocyclyl,
      or R^{y} is taken together with one of R⁸ and R⁹, and the atoms to which they are attached, to form a 3-8 membered heterocyclyl;
      R⁷ is H,
      or R⁷ is taken together with R^{y}, and the atoms to which they are attached, to form a 3-8 membered heterocyclyl;
      R⁸ and R⁹ are independently H or C₁₋₆alkyl,
      or one of R⁸ and R⁹ is taken together with R^{y}, and the atoms to which they are attached, to form a 3-8 membered heterocyclyl, and the other of R⁸ and R⁹ is H or C₁₋₆alkyl,
      or one of R⁸ and R⁹ is taken together with R¹⁰, and the atoms to which they are attached, to form a C₃₋₈cycloalkyl, and the other of R⁸ and R⁹ is H or C₁₋₆alkyl; and
      R¹⁰ is H,
      or R¹⁰ is taken together with one of R⁸ and R⁹, and the atoms to which they are attached, to form a C₃₋₈cycloalkyl,
      provided that:
         (1) R^{y} is taken together with R⁷, and the atoms to which they are attached, to form a 3-8 membered heterocyclyl, or
         (2) R^{y} is taken together with one of R⁸ and R⁹, and the atoms to which they are attached, to form a 3-8 membered heterocyclyl, or
         (3) one of R⁸ and R⁹ is taken together with R¹⁰ and the atoms to which they are attached, to form a C₃₋₈cycloalkyl, and
   (iii) 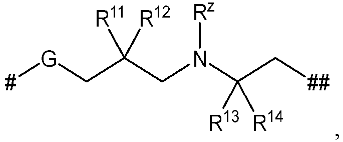 wherein
      G is O, NH, N(C₁₋₆alkyl), CH₂, or CH(C₁₋₆alkyl);
      R^{z} is H or C₁₋₆alkyl, wherein the C₁₋₆alkyl is optionally substituted with one or more C₃₋₈cycloalkyl;
      R¹¹ and R¹² are independently H, -OH, halo, or C₁₋₆alkyl; and
      R¹³ and R¹⁴ are independently H, C₁₋₆alkyl, or C₃₋₈cycloalkyl,
      or R¹³ and R¹⁴ are taken, together with the atoms to which they are attached, to form a 3-8 membered heterocyclyl,
wherein, for each of (i)-(iii), # denotes the point of attachment to the phenyl ring bearing moiety Q, and ## denotes the point of attachment to the phenyl ring bearing moieties X¹-X⁴; and
X¹, X², X³, and X⁴ are, independently of each other, H, halo, -CN, C₁₋₆alkyl, C₁₋₆alkoxy, or SF₅ wherein the C₁₋₆alkyl or C₁₋₆alkoxy is optionally substituted with one or more halo,
   provided that at least one of X¹, X², X³, and X⁴ is halo, -CN, C₁₋₆alkyl, C₁₋₆alkoxy, or SF₅, wherein the C₁₋₆alkyl or C₁₋₆alkoxy is optionally substituted with one or more halo.

In one aspect, provided herein is a compound of formula (A): 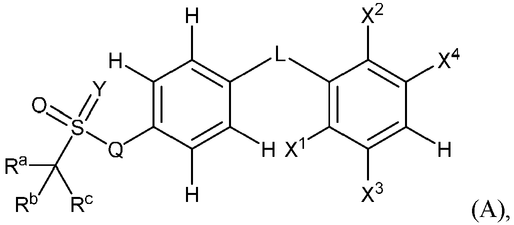 or a stereoisomer or tautomer thereof, or a pharmaceutically acceptable salt of any of the foregoing, wherein:
Q is absent or is -N-(C₁₋₆alkyl);
Y is O or -N-(C₁₋₆alkyl),
   provided that, when Q is -N(C₁₋₆alkyl), then Y is O;
R^{a}, R^{b}, and R^{c} are each independently H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl,
or any two of R^{a}, R^{b}, and R^{c} are taken, together with the atoms to which they are attached, to form a C₃₋₆cycloalkyl or a 3-6 membered heterocyclyl, and the other of R^{a}, R^{b}, and R^{c} is H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl;
L is selected from the group consisting of:
   (i) 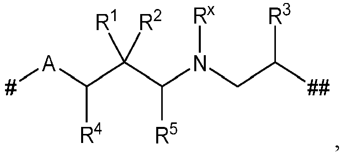 wherein
      A is O, NH, N(C₁₋₆alkyl), CH₂, or CH(C₁₋₆alkyl);
      R^{x} is H,
      or R^{x} is taken together with one of R¹ and R², and the atoms to which they are attached, to form a 3-8 membered heterocyclyl, wherein the 3-8 membered heterocyclyl is substituted with *n* independently selected R^{g} substituents, wherein *n* is an integer from 0-6, and R^{g} is -OH, halo, C₁₋₆alkyl, or C₁₋₆alkoxy;
      R¹ and R² are independently H, halo, or -OH,
      or one of R¹ and R² is taken together with R^{x}, and the atoms to which they are attached, to form a 3-8 membered heterocyclyl, wherein the 3-8 membered heterocyclyl is substituted with *n* independently selected R^{g} substituents, wherein *n* is an integer from 0-6, and R^{g} is -OH, halo, C₁₋₆alkyl, or C₁₋₆alkoxy, and the other of R¹ and R² is H, halo, or - OH;
      R³ is H, -OH, halo, or C₁₋₆alkoxy; and
      R⁴ and R⁵ are independently H,
      or R⁴ and R⁵ are taken, together with the atoms to which they are attached, to form a C₃₋₈cycloalkyl,
      provided that either:
         (1) R^{x} is taken together with one of R¹ and R², and the atoms to which they are attached, to form a 3-8 membered heterocyclyl, wherein the 3-8 membered heterocyclyl is optionally substituted with one or more -OH, C₁₋₆alkyl, or C₁₋₆alkoxy, or
         (2) R⁴ and R⁵ are taken, together with the atoms to which they are attached, to form a C₃₋₈cycloalkyl,
   (ii) 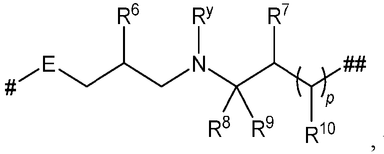 wherein
      E is O, NH, N(C₁₋₆alkyl), CH₂, or CH(C₁₋₆alkyl);
      *p* is 0 or 1,
         provided that, when *p* is 1, then E is O;
      R⁶ is H or -OH;
      R^{y} is H,
      or R^{y} is taken together with R⁷, and the atoms to which they are attached, to form a 3-8 membered heterocyclyl,
      or R^{y} is taken together with one of R⁸ and R⁹, and the atoms to which they are attached, to form a 3-8 membered heterocyclyl;
      R⁷ is H,
      or R⁷ is taken together with R^{y}, and the atoms to which they are attached, to form a 3-8 membered heterocyclyl;
      R⁸ and R⁹ are independently H or C₁₋₆alkyl,
      or one of R⁸ and R⁹ is taken together with R^{y}, and the atoms to which they are attached, to form a 3-8 membered heterocyclyl, and the other of R⁸ and R⁹ is H or C₁₋₆alkyl,
      or one of R⁸ and R⁹ is taken together with R¹⁰, and the atoms to which they are attached, to form a C₃₋₈cycloalkyl, and the other of R⁸ and R⁹ is H or C₁₋₆alkyl; and
      R¹⁰ is H,
      or R¹⁰ is taken together with one of R⁸ and R⁹, and the atoms to which they are attached, to form a C₃₋₈cycloalkyl,
      provided that:
         (1) R^{y} is taken together with R⁷, and the atoms to which they are attached, to form a 3-8 membered heterocyclyl, or
         (2) R^{y} is taken together with one of R⁸ and R⁹, and the atoms to which they are attached, to form a 3-8 membered heterocyclyl, or
         (3) one of R⁸ and R⁹ is taken together with R¹⁰ and the atoms to which they are attached, to form a C₃₋₈cycloalkyl, and
   (iii) 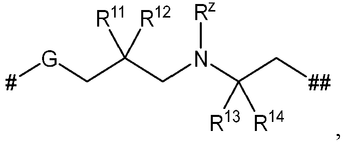 wherein
      G is O, NH, N(C₁₋₆alkyl), CH₂, or CH(C₁₋₆alkyl);
      R^{z} is H or C₁₋₆alkyl, wherein the C₁₋₆alkyl is optionally substituted with one or more C₃₋₈cycloalkyl;
      R¹¹ and R¹² are independently H, -OH, halo, or C₁₋₆alkyl; and
      R¹³ and R¹⁴ are independently H, C₁₋₆alkyl, or C₃₋₈cycloalkyl,
      or R¹³ and R¹⁴ are taken, together with the atoms to which they are attached, to form a 3-8 membered heterocyclyl,
wherein, for each of (i)-(iii), # denotes the point of attachment to the phenyl ring bearing moiety Q, and ## denotes the point of attachment to the phenyl ring bearing moieties X¹-X⁴; and
X¹, X², X³, and X⁴ are, independently of each other, H, halo, -CN, C₁₋₆alkyl, or C₁₋₆alkoxy, wherein the C₁₋₆alkyl or C₁₋₆alkoxy is optionally substituted with one or more halo,
   provided that at least one of X¹, X², X³, and X⁴ is halo, -CN, C₁₋₆alkyl, or C₁₋₆alkoxy, wherein the C₁₋₆alkyl or C₁₋₆alkoxy is optionally substituted with one or more halo.

Any embodiments provided herein of a compound of formula (A), or a stereoisomer or tautomer thereof, or a pharmaceutically acceptable salt of any of the foregoing, or any variation or embodiment thereof, are also embodiments of a compound of formula (A'), or a stereoisomer or tautomer thereof, or a pharmaceutically acceptable salt of any of the foregoing, or any variation or embodiment thereof.

In one aspect, provided herein is a compound of formula (I): 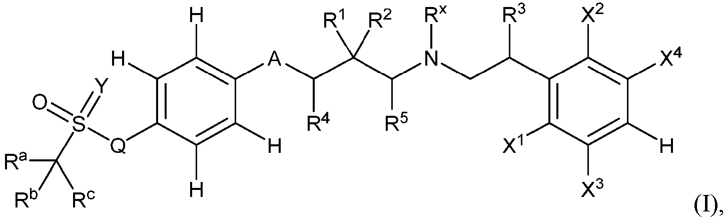 or a stereoisomer or tautomer thereof, or a pharmaceutically acceptable salt of any of the foregoing, wherein X¹, X², X³, X⁴, R¹, R², R³, R⁴, R⁵, R^{a}, R^{b}, R^{c}, R^{x}, A, Q, and Y are as defined for a compound of formula (A), or a stereoisomer or tautomer thereof, or a pharmaceutically acceptable salt of any of the foregoing, or any variation or embodiment thereof. In another variation, X¹, X², X³, X⁴, R¹, R², R³, R⁴, R⁵, R^{a}, R^{b}, R^{c}, R^{x}, A, Q, and Y of formula (I) are as defined for a compound of formula (A'), or a stereoisomer or tautomer thereof, or a pharmaceutically acceptable salt of any of the foregoing, or any variation or embodiment thereof.

In one aspect, provided herein is a compound of formula (II):

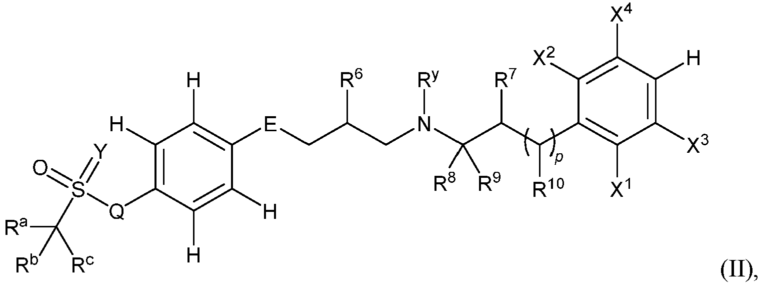

or a stereoisomer or tautomer thereof, or a pharmaceutically acceptable salt of any of the foregoing, wherein X¹, X², X³, X⁴, R⁶, R⁷, R⁸, R⁹, R¹⁰, R^{a}, R^{b}, R^{c}, R^{y}, *p,* E, Q, and Y are as defined for a compound of formula (A), or a stereoisomer or tautomer thereof, or a pharmaceutically acceptable salt of any of the foregoing, or any variation or embodiment thereof. In another variation, X¹, X², X³, X⁴, R⁶, R⁷, R⁸, R⁹, R¹⁰, R^{a}, R^{b}, R^{c}, R^{y}, *p,* E, Q, and Y of formula (II) are as defined for a compound of formula (A'), or a stereoisomer or tautomer thereof, or a pharmaceutically acceptable salt of any of the foregoing, or any variation or embodiment thereof.In one aspect, provided herein is a compound of formula (III): 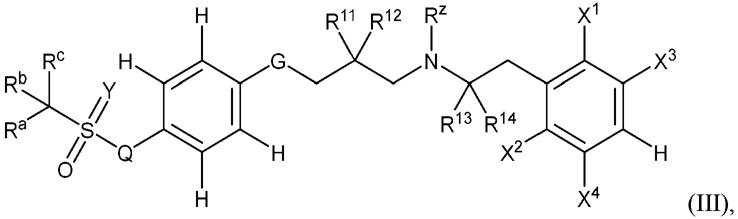 or a stereoisomer or tautomer thereof, or a pharmaceutically acceptable salt of any of the foregoing, wherein X¹, X², X³, X⁴, R¹¹, R¹², R¹³, R¹⁴, R^{a}, R^{b}, R^{c}, R^{z}, G, Q, and Y are as defined for a compound of formula (A), or a stereoisomer or tautomer thereof, or a pharmaceutically acceptable salt of any of the foregoing, or any variation or embodiment thereof. In another variation, X¹, X², X³, X⁴, R¹¹, R¹², R¹³, R¹⁴, R^{a}, R^{b}, R^{c}, R^{z}, G, Q, and Y of formula (III) are as defined for a compound of formula (A'), or a stereoisomer or tautomer thereof, or a pharmaceutically acceptable salt of any of the foregoing, or any variation or embodiment thereof.

In one aspect, provided herein is a compound of formula (B-2): 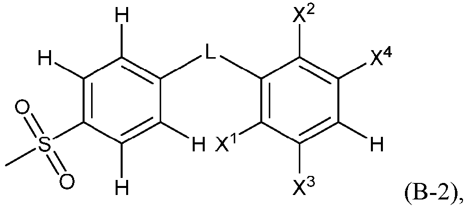 or a stereoisomer or tautomer thereof, or a pharmaceutically acceptable salt of any of the foregoing, wherein X¹, X², X³, X⁴, and L are as defined for a compound of formula (A), or a stereoisomer or tautomer thereof, or a pharmaceutically acceptable salt of any of the foregoing, or any variation or embodiment thereof. In another variation, X¹, X², X³, X⁴, and L of formula (B-2) are as defined for a compound of formula (A'), or a stereoisomer or tautomer thereof, or a pharmaceutically acceptable salt of any of the foregoing, or any variation or embodiment thereof.

In one aspect, provided herein is a compound of formula (B-5): 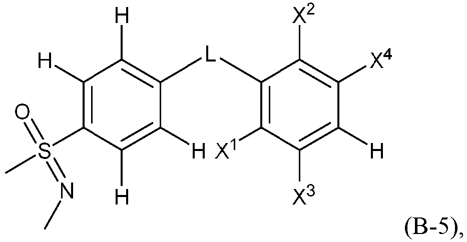 or a stereoisomer or tautomer thereof, or a pharmaceutically acceptable salt of any of the foregoing, wherein X¹, X², X³, X⁴, and L are as defined for a compound of formula (A), or a stereoisomer or tautomer thereof, or a pharmaceutically acceptable salt of any of the foregoing, or any variation or embodiment thereof. In another variation, X¹, X², X³, X⁴, and L of formula (B-5) are as defined for a compound of formula (A'), or a stereoisomer or tautomer thereof, or a pharmaceutically acceptable salt of any of the foregoing, or any variation or embodiment thereof.

In one aspect, provided herein is a compound of formula (C-1): 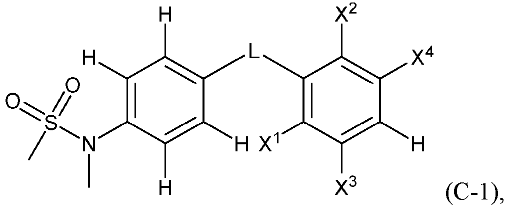 or a stereoisomer or tautomer thereof, or a pharmaceutically acceptable salt of any of the foregoing, wherein X¹, X², X³, X⁴, and L are as defined for a compound of formula (A), or a stereoisomer or tautomer thereof, or a pharmaceutically acceptable salt of any of the foregoing, or any variation or embodiment thereof. In another variation, X¹, X², X³, X⁴, and L of formula (C-1) are as defined for a compound of formula (A'), or a stereoisomer or tautomer thereof, or a pharmaceutically acceptable salt of any of the foregoing, or any variation or embodiment thereof.

In one aspect, provided herein is a pharmaceutical composition, comprising (i) a compound of formula (A), or any embodiment or variation thereof, such as a compound of formula (I), (II), (III), (B-2), (B-5), or (C-1), or a stereoisomer or tautomer thereof, or a pharmaceutically acceptable salt of any of the foregoing, and (ii) one or more pharmaceutically acceptable excipients. In another variation, provided herein is a pharmaceutical composition, comprising (i) a compound of formula (A'), or any embodiment or variation thereof, such as a compound of formula (I), (II), (III), (B-2), (B-5), or (C-1), or a stereoisomer or tautomer thereof, or a pharmaceutically acceptable salt of any of the foregoing, and (ii) one or more pharmaceutically acceptable excipients.

In one aspect, provided herein is a compound of formula (A') or (A), or any embodiment or variation thereof, such as a compound of formula (I), (II), (III), (B-2), (B-5), or (C-1), or a stereoisomer or tautomer thereof, or a pharmaceutically acceptable salt of any of the foregoing, or a pharmaceutical composition comprising (i) a compound of formula (A) or (A') or any embodiment or variation thereof, such as a compound of formula (I), (II), (III), (B-2), (B-5) or (C-1), or a stereoisomer or tautomer thereof, or a pharmaceutically acceptable salt of any of the foregoing, and (ii) one or more pharmaceutically acceptable excipients, for use in treating or delaying the development of a disease, disorder, or condition selected from: a kidney disease; or a disease, disorder, or condition selected from the group consisting of chronic kidney disease, focal segmental glomerulosclerosis (FSGS), hypertension-attributed kidney disease, human immunodeficiency virus-associated nephropathy (HIVAN), sickle-cell nephropathy, lupus nephritis, diabetic kidney disease, APOL1-associated nephropathy, viral nephropathy, COVID-19 associated nephropathy, preeclampsia, and sepsis.

In one aspect, provided herein is a kit, comprising (i) a compound of formula (A), or any embodiment or variation thereof, such as a compound of formula (I), (II), (III), (B-2), (B-5), or (C-1), or a stereoisomer or tautomer thereof, or a pharmaceutically acceptable salt of any of the foregoing, and (ii) instructions for use in treating an APOL1-mediated disease, disorder, or condition in an individual in need thereof. In another variation, provided herein is a kit, comprising (i) a compound of formula (A'), or any embodiment or variation thereof, such as a compound of formula (I), (II), (III), (B-2), (B-5), or (C-1), or a stereoisomer or tautomer thereof, or a pharmaceutically acceptable salt of any of the foregoing, and (ii) instructions for use in treating an APOL1-mediated disease, disorder, or condition in an individual in need thereof.

Also described herein are methods of preparing a compound of formula (A) or (A'), or any embodiment or variation thereof, such as a compound of formula (I), (II), (III), (B-2), (B-5), or (C-1), or a stereoisomer or tautomer thereof, or a pharmaceutically acceptable salt of any of the foregoing.

### DETAILED DESCRIPTION OF THE INVENTION

Unless clearly indicated otherwise, the terms "a," "an," and the like, refer to one or more.

As used herein, "about" a parameter or value includes and describes that parameter or value per se. For example, "about X" includes and describes X per se.

"Individual" refers to mammals and includes humans and non-human mammals. Examples of individuals include, but are not limited to, some primates and humans. In some embodiments, individual refers to a human.

As used herein, an "at risk" individual is an individual who is at risk of developing a disease or condition. An individual "at risk" may or may not have a detectable disease or condition, and may or may not have displayed detectable disease prior to the treatment methods described herein. "At risk" denotes that an individual has one or more so-called risk factors, which are measurable parameters that correlate with development of a disease or condition and are known in the art. An individual having one or more of these risk factors has a higher probability of developing the disease or condition than an individual without these risk factor(s).

"Treatment" or "treating" is an approach for obtaining beneficial or desired results including clinical results. Beneficial or desired results may include one or more of the following: decreasing one or more symptom resulting from the disease or condition; diminishing the extent of the disease or condition; slowing or arresting the development of one or more symptom associated with the disease or condition (*e.g.,* stabilizing the disease or condition, preventing or delaying the worsening or progression of the disease or condition); and relieving the disease, such as by causing the regression of clinical symptoms (*e.g.,* ameliorating the disease state, enhancing the effect of another medication, delaying the progression of the disease, increasing the quality of life, and/or prolonging survival).

As used herein, "delaying" development of a disease or condition means to defer, hinder, slow, retard, stabilize and/or postpone development of the disease or condition. This delay can be of varying lengths of time, depending on the history of the disease and/or individual being treated. As is evident to one skilled in the art, a sufficient or significant delay can, in effect, encompass prevention, in that the individual does not develop the disease or condition.

As used herein, the term "therapeutically effective amount" or "effective amount" intends such amount of a compound of the disclosure or a pharmaceutically salt thereof sufficient to effect treatment when administered to an individual. As is understood in the art, an effective amount may be in one or more doses, *e.g.,* a single dose or multiple doses may be required to achieve the desired treatment endpoint. An effective amount may be considered in the context of administering one or more therapeutic agents, and a single agent may be considered to be given in an effective amount if, in conjunction with one or more other agents, a desirable or beneficial result may be or is achieved.

As used herein, "unit dosage form" refers to physically discrete units, suitable as unit dosages, each unit containing a predetermined quantity of active ingredient, or compound, which may be in a pharmaceutically acceptable carrier.

As used herein, by "pharmaceutically acceptable" is meant a material that is not biologically or otherwise undesirable, *e.g.,* the material may be incorporated into a pharmaceutical composition administered to an individual without causing significant undesirable biological effects.

The term "alkyl", as used herein, refers to an unbranched or branched saturated hydrocarbon chain. As used herein, alkyl has 1-20 carbons (*i.e.,* C₁₋₂₀alkyl), 1-16 carbons (*i.e.,* C₁₋₁₆alkyl), 1-12 carbons (*i.e.,* C₁₋₁₂alkyl), 1-10 carbons (*i.e.,* C₁₋₁₀alkyl), 1-8 carbons (*i.e.,* C₁₋₈alkyl), 1-6 carbons (*i.e.,* C₁₋₆alkyl), 1-4 carbons (*i.e.,* C₁₋₄alkyl), or 1-3 carbons (*i.e.,* C₁₋₃alkyl). Examples of alkyl groups include, but are not limited to, methyl, ethyl, propyl, iso-propyl, n-butyl, sec-butyl, iso-butyl, tert-butyl, pentyl, 2-pentyl, iso-pentyl, neo-pentyl, hexyl, 2-hexyl, 3-hexyl, and 3-methylpentyl. When an alkyl residue having a specific number of carbons is named by chemical name or molecular formula, all positional isomers having that number of carbon atoms may be encompassed-for example, "butyl" includes n-butyl, sec-butyl, iso-butyl, and tert-butyl; and "propyl" includes n-propyl and iso-propyl. Certain commonly used alternative names may be used and will be understood by those of ordinary skill in the art. For instance, a divalent group, such as a divalent "alkyl" group, may be referred to as an "alkylene".

The term "alkoxy", as used herein, refers to an -O-alkyl moiety. Examples of alkoxy groups include, but are not limited to, methoxy, ethoxy, *n*-propoxy, *iso*-propoxy, *n-*butoxy, *tert*-butoxy, *sec*-butoxy, *n*-pentoxy, *n*-hexoxy, and 1,2-dimethylbutoxy.

The term "aryl", as used herein, refers to a fully unsaturated carbocyclic ring moiety. The term "aryl" encompasses monocyclic and polycyclic fused-ring moieties. As used herein, aryl encompasses ring moieties comprising, for example, 6 to 20 annular carbon atoms (*i.e.,* C₆₋₂₀aryl), 6 to 16 annular carbon atoms (*i.e.,* C₆₋₁₆aryl), 6 to 12 annular carbon atoms (*i.e.,* C₆₋₁₂aryl), or 6 to 10 annular carbon atoms (*i.e.,* C₆₋₁₀aryl). Examples of aryl moieties include, but are not limited to, phenyl, naphthyl, fluorenyl, and anthryl.

The term "cycloalkyl", as used herein, refers to a saturated or partially unsaturated carbocyclic ring moiety. The term "cycloalkyl" encompasses monocyclic and polycyclic ring moieties, wherein the polycyclic moieties may be fused, branched, or spiro. Cycloalkyl includes cycloalkenyl groups, wherein the ring moiety comprises at least one annular double bond. Cycloalkyl includes any polycyclic carbocyclic ring moiety comprising at least one non-aromatic ring, regardless of the point of attachment to the remainder of the molecule. As used herein, cycloalkyl includes rings comprising, for example, 3 to 20 annular carbon atoms (*i.e.,* a C₃₋₂₀cycloalkyl), 3 to 16 annular carbon atoms (*i.e.,* a C₃₋₁₆cycloalkyl), 3 to 12 annular carbon atoms (*i.e.,* a C₃₋₁₂cycloalkyl), 3 to 10 annular carbon atoms (*i.e.,* a C₃₋₁₀cycloalkyl), 3 to 8 annular carbon atoms (*i.e.,* a C₃₋₈cycloalkyl), 3 to 6 annular carbon atoms (*i.e.,* a C₃₋₆cycloalkyl), or 3 to 5 annular carbon atoms (*i.e.,* a C₃₋₅cycloalkyl). Monocyclic cycloalkyl ring moieties include, for example, cyclopropyl, cyclobutyl, cyclopentyl, cyclohexyl, cycloheptyl, and cyclooctyl. Polycyclic groups include, for example, bicyclo[2.2.1]heptanyl, bicyclo[2.2.2]octanyl, adamantyl, norbomyl, decalinyl, 7,7-dimethyl -bicyclo [2.2.1]heptanyl, and the like. Still further, cycloalkyl also includes spiro cycloalkyl ring moieties, for example, spiro[2.5]octanyl, spiro[4.5]decanyl, or spiro [5.5]undecanyl.

The term "halo", as used herein, refers to atoms occupying groups VIIA of The Periodic Table and includes fluorine (fluoro), chlorine (chloro), bromine (bromo), and iodine (iodo).

The term "heteroaryl", as used herein, refers to an aromatic (fully unsaturated) ring moiety that comprises one or more annular heteroatoms independently selected from the group consisting of nitrogen, oxygen, and sulfur. The term "heteroaryl" includes both monocyclic and polycyclic fused-ring moieties. As used herein, a heteroaryl comprises, for example, 5 to 20 annular atoms (*i.e.,* a 5-20 membered heteroaryl), 5 to 16 annular atoms (*i.e.,* a 5-16 membered heteroaryl), 5 to 12 annular atoms (*i.e.,* a 5-12 membered heteroaryl), 5 to 10 annular atoms (*i.e.,* a 5-10 membered heteroaryl), 5 to 8 annular atoms (*i.e.,* a 5-8 membered heteroaryl), or 5 to 6 annular atoms (*i.e.,* a 5-6 membered heteroaryl). Any monocyclic or polycyclic aromatic ring moiety comprising one or more annular heteroatoms is considered a heteroaryl, regardless of the point of attachment to the remainder of the molecule (*i.e.,* the heteroaryl moiety may be attached to the remainder of the molecule through any annular carbon or any annular heteroatom of the heteroaryl moiety). Examples of heteroaryl groups include, but are not limited to, acridinyl, benzimidazolyl, benzothiazolyl, benzindolyl, benzofuranyl, benzothiazolyl, benzothiadiazolyl, benzonaphthofuranyl, benzoxazolyl, benzothienyl (benzothiophenyl), benzotriazolyl, benzo[4,6]imidazo[1,2-a]pyridyl, carbazolyl, cinnolinyl, dibenzofuranyl, dibenzothiophenyl, furanyl, isothiazolyl, imidazolyl, indazolyl, indolyl, indazolyl, isoindolyl, isoquinolyl, isoxazolyl, naphthyridinyl, oxadiazolyl, oxazolyl, 1-oxidopyridinyl, 1-oxidopyrimidinyl, 1-oxidopyrazinyl, 1-oxidopyridazinyl, phenazinyl, phthalazinyl, pteridinyl, purinyl, pyrrolyl, pyrazolyl, pyridinyl, pyrazinyl, pyrimidinyl, pyridazinyl, quinazolinyl, quinoxalinyl, quinolinyl, quinuclidinyl, isoquinolinyl, thiazolyl, thiadiazolyl, triazolyl, tetrazolyl, and triazinyl. Examples of the fused-heteroaryl rings include, but are not limited to, benzo[d]thiazolyl, quinolinyl, isoquinolinyl, benzo[b]thiophenyl, indazolyl, benzo[d]imidazolyl, pyrazolo[1,5-a]pyridinyl, and imidazo[1,5-a]pyridinyl, wherein the heteroaryl can be bound *via* either ring of the fused system.

The term "heterocyclyl", as used herein, refers to a saturated or partially unsaturated cyclic moiety that encompasses one or more annular heteroatoms independently selected from the group consisting of nitrogen, oxygen, and sulfur. The term "heterocyclyl" includes both monocyclic and polycyclic ring moieties, wherein the polycyclic ring moieties may be fused, bridged, or spiro. Any non-aromatic monocyclic or polycyclic ring moiety comprising at least one annular heteroatom is considered a heterocyclyl, regardless of the point of attachment to the remainder of the molecule (*i.e.,* the heterocyclyl moiety may be attached to the remainder of the molecule through any annular carbon or any annular heteroatom of the heterocyclyl moiety). Further, the term heterocyclyl is intended to encompass any polycyclic ring moiety comprising at least one annular heteroatom wherein the polycyclic ring moiety comprises at least one non-aromatic ring, regardless of the point of attachment to the remainder of the molecule. As used herein, a heterocyclyl comprises, for example, 3 to 20 annular atoms (*i.e.,* a 3-20 membered heterocyclyl), 3 to 16 annular atoms (*i.e.,* a 3-16 membered heterocyclyl), 3 to 12 annular atoms (*i.e.,* a 3-12 membered heterocyclyl), 3 to 10 annular atoms (*i.e.,* a 3-10 membered heterocyclyl), 3 to 8 annular atoms (*i.e.,* a 3-8 membered heterocyclyl), 3 to 6 annular atoms (*i.e.,* a 3-6 membered heterocyclyl), 3 to 5 annular atoms (*i.e.,* a 3-5 membered heterocyclyl), 5 to 8 annular atoms (*i.e.,* a 5-8 membered heterocyclyl), or 5 to 6 annular atoms (*i.e.,* a 5-6 membered heterocyclyl). Examples of heterocyclyl groups include, *e.g.,* azetidinyl, azepinyl, benzodioxolyl, benzo[b][1,4]dioxepinyl, 1,4-benzodioxanyl, benzopyranyl, benzodioxinyl, benzopyranonyl, benzofuranonyl, dioxolanyl, dihydropyranyl, hydropyranyl, thienyl[1,3]dithianyl, decahydroisoquinolyl, furanonyl, imidazolinyl, imidazolidinyl, indolinyl, indolizinyl, isoindolinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, oxiranyl, oxetanyl, phenothiazinyl, phenoxazinyl, piperidinyl, piperazinyl, 4-piperidonyl, pyrrolidinyl, pyrazolidinyl, quinuclidinyl, thiazolidinyl, tetrahydrofuryl, tetrahydropyranyl, trithianyl, tetrahydroquinolinyl, thiophenyl (*i.e.,* thienyl), thiomorpholinyl, thiamorpholinyl, 1-oxo-thiomorpholinyl, and 1,1-dioxo-thiomorpholinyl. Examples of spiro heterocyclyl rings include, but are not limited to, bicyclic and tricyclic ring systems, such as oxabicyclo[2.2.2]octanyl, 2-oxa-7-azaspiro[3.5]nonanyl, 2-oxa-6-azaspiro[3.4]octanyl, and 6-oxa-1-azaspiro[3.3]heptanyl. Examples of fused heterocyclyl rings include, but are not limited to, 1,2,3,4-tetrahydroisoquinolinyl, 4,5,6,7-tetrahydrothieno[2,3-c]pyridinyl, indolinyl, and isoindolinyl, where the heterocyclyl can be bound via either ring of the fused system.

The terms "optional" and "optionally", as used herein, mean that the subsequently described event or circumstance may or may not occur and that the description includes instances where the event or circumstance occurs and instances where it does not. Accordingly, the term "optionally substituted" infers that any one or more (*e.g.,* 1, 2, 1 to 5, 1 to 3, 1 to 2, etc.) hydrogen atoms on the designated atom or moiety or group may be replaced or not replaced by an atom or moiety or group other than hydrogen. By way of illustration and not limitation, the phrase "methyl optionally substituted with one or more chloro" encompasses - CH₃, -CH₂Cl, -CHCl₂, and -CCl₃ moieties.

It is understood that aspects and embodiments described herein as "comprising" include "consisting of" and "consisting essentially of " embodiments.

The term "pharmaceutically acceptable salt", as used herein, of a given compound refers to salts that retain the biological effectiveness and properties of the given compound and which are not biologically or otherwise undesirable. "Pharmaceutically acceptable salts" include, for example, salts with inorganic acids, and salts with an organic acid. In addition, if the compounds described herein are obtained as an acid addition salt, the free base can be obtained by basifying a solution of the acid salt. Conversely, if the product is a free base, an addition salt, particularly a pharmaceutically acceptable addition salt, may be produced by dissolving the free base in a suitable organic solvent and treating the solution with an acid, in accordance with conventional procedures for preparing acid addition salts from base compounds. *See, e.g.,* Handbook of Pharmaceutical Salts Properties, Selection, and Use, International Union of Pure and Applied Chemistry, John Wiley & Sons (2008), which is incorporated herein by reference. Those skilled in the art will recognize various synthetic methodologies that may be used to prepare nontoxic pharmaceutically acceptable addition salts. Pharmaceutically acceptable acid addition salts may be prepared from inorganic or organic acids. Salts derived from inorganic acids include, *e.g.,* hydrochloric acid, hydrobromic acid, sulfuric acid, nitric acid, phosphoric acid, and the like. Salts derived from organic acids include, *e.g.,* acetic acid, propionic acid, gluconic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluene-sulfonic acid, salicylic acid, trifluoroacetic acid, and the like. Likewise, pharmaceutically acceptable base addition salts can be prepared from inorganic or organic bases. Salts derived from inorganic bases include, by way of example only, sodium, potassium, lithium, aluminum, ammonium, calcium, and magnesium salts. Salts derived from organic bases include, but are not limited to, salts of primary, secondary, and tertiary amines. Specific examples of suitable amines include, by way of example only, isopropylamine, trimethyl amine, diethyl amine, tri(iso-propyl) amine, tri(n-propyl) amine, ethanolamine, 2-dimethylaminoethanol, piperazine, piperidine, morpholine, N-ethylpiperidine, and the like.

Isotopically labeled forms of the compounds depicted herein may be prepared. Isotopically labeled compounds have structures depicted herein, except that one or more atoms are replaced by an atom having a selected atomic mass or mass number. Examples of isotopes that can be incorporated into the disclosed compounds include isotopes of hydrogen, carbon, nitrogen, oxygen, phosphorous, fluorine, chlorine, and iodine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ³⁶Cl, ¹²³I, and ¹²⁵I, respectively. In some embodiments, a compound of formula (A), or formula (A') is provided wherein one or more hydrogen is replaced by deuterium or tritium.

Some of the compounds provided herein may exist as tautomers. Tautomers are in equilibrium with one another. By way of illustration, amide containing compounds may exist in equilibrium with imidic acid tautomers. Regardless of which tautomer is shown and regardless of the nature of the equilibrium among tautomers, the compounds of this disclosure are understood by one of ordinary skill in the art to comprise both amide and imidic acid tautomers. Thus, for example, amide-containing compounds are understood to include their imidic acid tautomers. Likewise, imidic-acid containing compounds are understood to include their amide tautomers.

Also provided herein are prodrugs of the compounds depicted herein, or a pharmaceutically acceptable salt thereof. Prodrugs are compounds that may be administered to an individual and release, *in vivo,* a compound depicted herein as the parent drug compound. It is understood that prodrugs may be prepared by modifying a functional group on a parent drug compound in such a way that the modification is cleaved *in vivo* to release the parent drug compound. *See, e.g.,* Rautio, J., Kumpulainen, H., Heimbach, T. et al. Prodrugs: design and clinical applications. Nat Rev Drug Discov 7, 255-270 2008.

The compounds of the present disclosure, or their pharmaceutically acceptable salts, may include an asymmetric center and may thus give rise to enantiomers, diastereomers, and other stereoisomeric forms that may be defined, in terms of absolute stereochemistry, as (*R*)- or (*S*)- (or as (D)- or (L)- for amino acids). The present disclosure is meant to include all such possible isomers, as well as their racemic and optically pure forms and mixtures thereof in any ratio. Optically active (+) and (-), (*R*)- and (*S*)-, or (D)- and (L)- isomers may be prepared using chiral synthons or chiral reagents, or may be resolved using conventional techniques, for example, chromatography and/or fractional crystallization. Conventional techniques for the preparation/isolation of individual enantiomers include chiral synthesis from a suitable optically pure precursor or the resolution of the racemate (or the racemate of a salt or derivative) using, for example, chiral high pressure liquid chromatography (HPLC), and chiral SFC (supercritical fluid chromatography). When the compounds described herein contain olefinic double bonds or other centers of geometric asymmetry, unless specified otherwise, it is intended that the present disclosure includes both E and Z geometric isomers. Likewise, cis- and trans- are used in their conventional sense to describe relative spatial relationships.

A "stereoisomer" refers to a compound made up of the same atoms bonded by the same bonds, but having different three-dimensional structures, which are not interchangeable. The present disclosure contemplates various stereoisomers, or mixtures thereof, and includes "enantiomers," which refers to two stereoisomers whose structures are non-superimposable mirror images of one another. "Diastereomers" are stereoisomers that have at least two asymmetric atoms, but which are not mirror images of each other.

Where enantiomeric and/or diastereomeric forms exist of a given structure, flat bonds indicate that all stereoisomeric forms of the depicted structure may be present, *e.g.,*

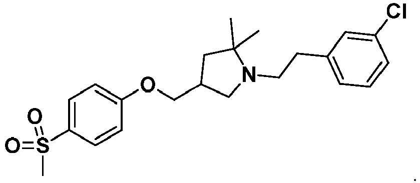

Where enantiomeric forms exist of a given structure, flat bonds and the presence of a " * " symbol indicate that the composition is made up of at least 90%, by weight, of a single isomer with unknown stereochemistry, *e.g.,*

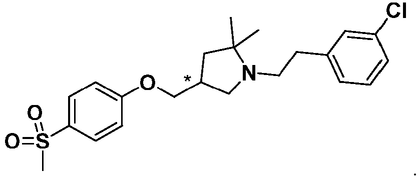

Where enantiomeric and/or diastereomeric forms exist of a given structure, wedged or hashed bonds indicate the composition is made up of at least 90%, by weight, of a single enantiomer or diastereomer with known stereochemistry, *e.g.,*

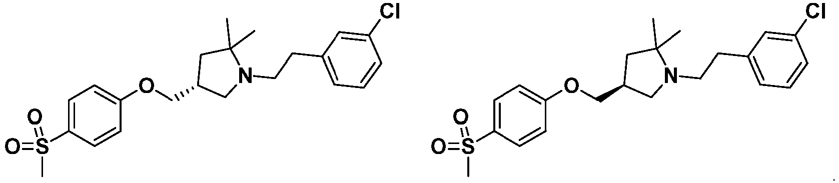

Where enantiomeric and/or diastereomeric forms exist of a given structure with two stereocenters, flat bonds and the presence of two "&1" symbols indicate the composition is made up of a pair of enantiomers with unknown relative stereochemistry, *e.g.,*

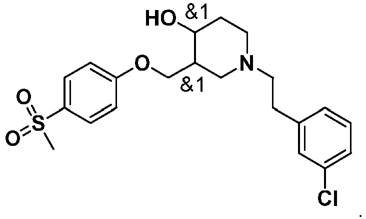

Where enantiomeric and/or diastereomeric forms exist of a given structure with two stereocenters, wedged and/or dashed bonds and the presence of two "&1" symbols indicate the composition is made up of a pair of enantiomers with known relative stereochemistry, *e.g.,*

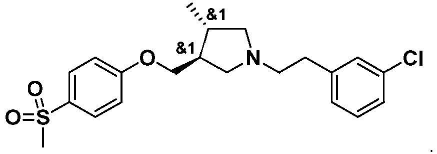

Where enantiomeric and/or diastereomeric forms exist of a given structure with two stereocenters, wedged and/or dashed bonds and the presence of two "or1" symbols indicate the composition is made up of at least 90%, by weight, a single stereoisomer with known relative stereochemistry but unknown absolute stereochemistry, *e.g.,*

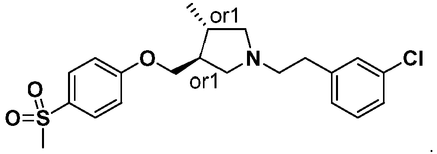

Where enantiomeric and/or diastereomeric forms exist of a given structure with two stereocenters, flat bonds and the presence of two " * " symbols indicate the composition is made up of at least 90%, by weight, of a single enantiomer or diastereomer with unknown stereochemistry, *e.g.,*

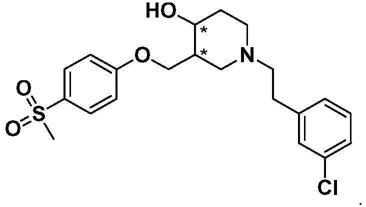

Abbreviations used are those conventional in the art and are in accordance with the Periodic Table of the Elements, CAS version, *Handbook of Chemistry and Physics,* 75^{th} Ed. The following examples are intended to be illustrative only and not limiting in any way.

| | | | |
|---|---|---|---|
| °C | degrees Celsius | LiHMDS | lithium bis(trimethylsilyl)amide |
| µL | microliter | mCPBA or m-CPBA | meta-chloroperoxybenzoic acid |
| [M+XX]⁺ | observed mass | MeOH | methanol |
| AC₅₀ | half-maximal activity concentration | MeCN | acetonitrile |
| AcOH | acetic acid | m | multiplet (NMR) |
| AIBN | azobisisobutyronitrile | mg | milligrams |
| app | apparent (NMR) | min | minutes |
| b | broad (as in "br s" to indicate a broad singlet) | mL | milliliter |
| BH₃ · THF | borane-tetrahydrofuran complex | mmol | millimole |
| BBr₃ | boron tribromide | mM | millimolar |
| Calc'd | calculated | M | molarity or molar |
| Cbz-Cl or CbzCl | benzyl chloroformate | MS | mass spectrometry |
| CO₂ | carbon dioxide | MsCl | methanesulfonyl chloride |
| Cs₂CO₃ | cesium carbonate | MTBE | methyl *tert*-butyl ether |
| *d* | deuterated (NMR solvents) | n/a | not applicable |
| d | doublet (NMR) | NH₄ | ammonium |
| dd | doublet of doublets (NMR) | NH₄OH | ammonium hydroxide |
| DCE | 1,2-dichloroethane | NH₄HCO₃ | ammonium bicarbonate |
| DCM | dichloromethane | Na₂SO₄ | sodium sulfate |
| DIAD | diisopropyl azodicarboxylate | NaBH₃CN | sodium cyanoborohydride |
| DMF | *N,N*-dimethylformamide | NMR | nuclear magnetic resonance |
| DMP | Dess-Martin periodinane | NaOH | sodium hydroxide |
| EC₅₀ | half-maximal effective concentration | Pd(dppf)Cl₂ | [1,1'-bis(diphenylphosphino)ferrocene ]dichloropalladium(II) |
| EtOAc | ethyl acetate | Pd/C | palladium on carbon |
| EtOH | ethanol | pH | potential of hydrogen |
| g | grams | PPh₃ | triphenyl phosphine |
| h | hours | s | singlet (NMR) |
| H | hydrogen | SFC | supercritical fluid chromatography |
| HCl | hydrochloric acid | t | triplet (NMR) |
| HPLC | high-performance liquid chromatography | TBAB | tetrabutylammonium bromide |
| In vacuo | in a vacuum | TEA | triethylamine |
| IUPAC | International Union of Pure and Applied Chemistry | TFA | trifluoroacetic acid |
| MHz | megahertz | THF | tetrahydrofuran |
| *J* | J-coupling value (NMR) | TMAD | tetramethylazodicarboxamide |
| K₂CO₃ | potassium carbonate | TMSCl | trimethylsilyl chloride |

### COMPOUNDS

Provided herein is a compound of formula (A'): 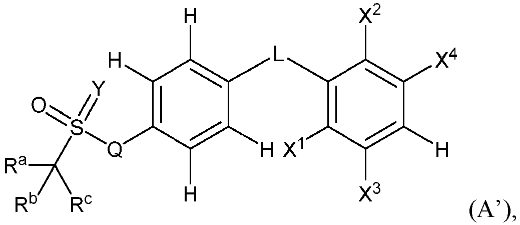 or a stereoisomer or tautomer thereof, or a pharmaceutically acceptable salt of any of the foregoing, wherein:
Q is absent or is -N-(C₁₋₆alkyl);
Y is O or -N-(C₁₋₆alkyl),
   provided that, when Q is -N(C₁₋₆alkyl), then Y is O;
R^{a}, R^{b}, and R^{c} are each independently H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl,
or any two of R^{a}, R^{b}, and R^{c} are taken, together with the atoms to which they are attached, to form a C₃₋₆cycloalkyl or a 3-6 membered heterocyclyl, and the other of R^{a}, R^{b}, and R^{c} is H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl;
L is selected from the group consisting of:
   (i) 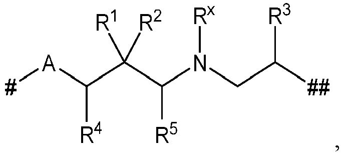 wherein
      A is O, NH, N(C₁₋₆alkyl), CH₂, or CH(C₁₋₆alkyl);
      R^{x} is H,
      or R^{x} is taken together with one of R¹ and R², and the atoms to which they are attached, to form a 3-8 membered heterocyclyl, wherein the 3-8 membered heterocyclyl is substituted with *n* independently selected R^{g} substituents, wherein *n* is an integer from 0-6, and R^{g} is -OH, halo, C₁₋₆alkyl, or C₁₋₆alkoxy;
      R¹ and R² are independently H, halo, or -OH,
      or one of R¹ and R² is taken together with R^{x}, and the atoms to which they are attached, to form a 3-8 membered heterocyclyl, wherein the 3-8 membered heterocyclyl is substituted with *n* independently selected R^{g} substituents, wherein *n* is an integer from 0-6, and R^{g} is -OH, halo, C₁₋₆alkyl, or C₁₋₆alkoxy, and the other of R¹ and R² is H, halo, or - OH;
      R³ is H, -OH, halo, or C₁₋₆alkoxy; and
      R⁴ and R⁵ are independently H,
      or R⁴ and R⁵ are taken, together with the atoms to which they are attached, to form a C₃₋₈cycloalkyl,
      provided that either:
         (1) R^{x} is taken together with one of R¹ and R², and the atoms to which they are attached, to form a 3-8 membered heterocyclyl, wherein the 3-8 membered heterocyclyl is optionally substituted with one or more -OH, C₁₋₆alkyl, or C₁₋₆alkoxy, or
         (2) R⁴ and R⁵ are taken, together with the atoms to which they are attached, to form a C₃₋₈cycloalkyl,
   (ii) 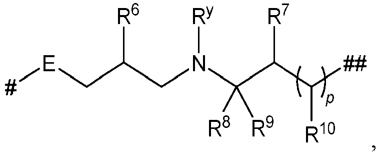 wherein
      E is O, NH, N(C₁₋₆alkyl), CH₂, or CH(C₁₋₆alkyl);
      *p* is 0 or 1,
         provided that, when *p* is 1, then E is O;
      R⁶ is H or -OH;
      R^{y} is H,
      or R^{y} is taken together with R⁷, and the atoms to which they are attached, to form a 3-8 membered heterocyclyl,
      or R^{y} is taken together with one of R⁸ and R⁹, and the atoms to which they are attached, to form a 3-8 membered heterocyclyl;
      R⁷ is H,
      or R⁷ is taken together with R^{y}, and the atoms to which they are attached, to form a 3-8 membered heterocyclyl;
      R⁸ and R⁹ are independently H or C₁₋₆alkyl,
      or one of R⁸ and R⁹ is taken together with R^{y}, and the atoms to which they are attached, to form a 3-8 membered heterocyclyl, and the other of R⁸ and R⁹ is H or C₁₋₆alkyl,
      or one of R⁸ and R⁹ is taken together with R¹⁰, and the atoms to which they are attached, to form a C₃₋₈cycloalkyl, and the other of R⁸ and R⁹ is H or C₁₋₆alkyl; and
      R¹⁰ is H,
      or R¹⁰ is taken together with one of R⁸ and R⁹, and the atoms to which they are attached, to form a C₃₋₈cycloalkyl,
      provided that:
         (1) R^{y} is taken together with R⁷, and the atoms to which they are attached, to form a 3-8 membered heterocyclyl, or
         (2) R^{y} is taken together with one of R⁸ and R⁹, and the atoms to which they are attached, to form a 3-8 membered heterocyclyl, or
         (3) one of R⁸ and R⁹ is taken together with R¹⁰ and the atoms to which they are attached, to form a C₃₋₈cycloalkyl, and
   (iii) 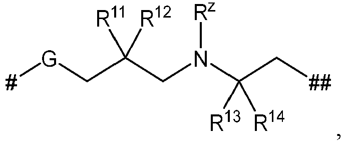 wherein
      G is O, NH, N(C₁₋₆alkyl), CH₂, or CH(C₁₋₆alkyl);
      R^{z} is H or C₁₋₆alkyl, wherein the C₁₋₆alkyl is optionally substituted with one or more C₃₋₈cycloalkyl;
      R¹¹ and R¹² are independently H, -OH, halo, or C₁₋₆alkyl; and
      R¹³ and R¹⁴ are independently H, C₁₋₆alkyl, or C₃₋₈cycloalkyl,
      or R¹³ and R¹⁴ are taken, together with the atoms to which they are attached, to form a 3-8 membered heterocyclyl,
wherein, for each of (i)-(iii), # denotes the point of attachment to the phenyl ring bearing moiety Q, and ## denotes the point of attachment to the phenyl ring bearing moieties X¹-X⁴; and
X¹, X², X³, and X⁴ are, independently of each other, H, halo, -CN, C₁₋₆alkyl, C₁₋₆alkoxy, or SF₅ wherein the C₁₋₆alkyl or C₁₋₆alkoxy is optionally substituted with one or more halo,
   provided that at least one of X¹, X², X³, and X⁴ is halo, -CN, C₁₋₆alkyl, C₁₋₆alkoxy, or SF₅, wherein the C₁₋₆alkyl or C₁₋₆alkoxy is optionally substituted with one or more halo.

Provided herein is a compound of formula (A): 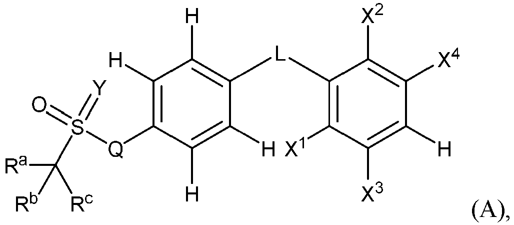 or a stereoisomer or tautomer thereof, or a pharmaceutically acceptable salt of any of the foregoing, wherein:
Q is absent or is -N-(C₁₋₆alkyl);
Y is O or -N-(C₁₋₆alkyl),
   provided that, when Q is -N(C₁₋₆alkyl), then Y is O;
R^{a}, R^{b}, and R^{c} are each independently H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl,
or any two of R^{a}, R^{b}, and R^{c} are taken, together with the atoms to which they are attached, to form a C₃₋₆cycloalkyl or a 3-6 membered heterocyclyl, and the other of R^{a}, R^{b}, and R^{c} is H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl;
L is selected from the group consisting of:
   (i) 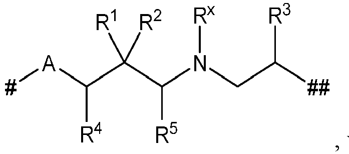 wherein
      A is O, NH, N(C₁₋₆alkyl), CH₂, or CH(C₁₋₆alkyl);
      R^{x} is H,
      or R^{x} is taken together with one of R¹ and R², and the atoms to which they are attached, to form a 3-8 membered heterocyclyl, wherein the 3-8 membered heterocyclyl is substituted with *n* independently selected R^{g} substituents, wherein *n* is an integer from 0-6, and R^{g} is -OH, halo, C₁₋₆alkyl, or C₁₋₆alkoxy;
      R¹ and R² are independently H, halo, or -OH,
      or one of R¹ and R² is taken together with R^{x}, and the atoms to which they are attached, to form a 3-8 membered heterocyclyl, wherein the 3-8 membered heterocyclyl is substituted with *n* independently selected R^{g} substituents, wherein *n* is an integer from 0-6, and R^{g} is -OH, halo, C₁₋₆alkyl, or C₁₋₆alkoxy, and the other of R¹ and R² is H, halo, or - OH;
      R³ is H, -OH, halo, or C₁₋₆alkoxy; and
      R⁴ and R⁵ are independently H,
      or R⁴ and R⁵ are taken, together with the atoms to which they are attached, to form a C₃₋₈cycloalkyl,
      provided that either:
         (1) R^{x} is taken together with one of R¹ and R², and the atoms to which they are attached, to form a 3-8 membered heterocyclyl, wherein the 3-8 membered heterocyclyl is optionally substituted with one or more -OH, C₁₋₆alkyl, or C₁₋₆alkoxy, or
         (2) R⁴ and R⁵ are taken, together with the atoms to which they are attached, to form a C₃₋₈cycloalkyl,
   (ii) 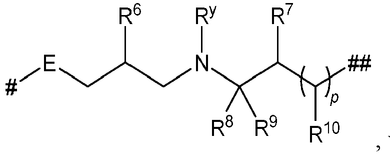 wherein
      E is O, NH, N(C₁₋₆alkyl), CH₂, or CH(C₁₋₆alkyl);
      *p* is 0 or 1,
         provided that, when *p* is 1, then E is O;
      R⁶ is H or -OH;
      R^{y} is H,
      or R^{y} is taken together with R⁷, and the atoms to which they are attached, to form a 3-8 membered heterocyclyl,
      or R^{y} is taken together with one of R⁸ and R⁹, and the atoms to which they are attached, to form a 3-8 membered heterocyclyl;
      R⁷ is H,
      or R⁷ is taken together with R^{y}, and the atoms to which they are attached, to form a 3-8 membered heterocyclyl;
      R⁸ and R⁹ are independently H or C₁₋₆alkyl,
      or one of R⁸ and R⁹ is taken together with R^{y}, and the atoms to which they are attached, to form a 3-8 membered heterocyclyl, and the other of R⁸ and R⁹ is H or C₁₋₆alkyl,
      or one of R⁸ and R⁹ is taken together with R¹⁰, and the atoms to which they are attached, to form a C₃₋₈cycloalkyl, and the other of R⁸ and R⁹ is H or C₁₋₆alkyl; and
      R¹⁰ is H,
      or R¹⁰ is taken together with one of R⁸ and R⁹, and the atoms to which they are attached, to form a C₃₋₈cycloalkyl,
      provided that:
         (1) R^{y} is taken together with R⁷, and the atoms to which they are attached, to form a 3-8 membered heterocyclyl, or
         (2) R^{y} is taken together with one of R⁸ and R⁹, and the atoms to which they are attached, to form a 3-8 membered heterocyclyl, or
         (3) one of R⁸ and R⁹ is taken together with R¹⁰ and the atoms to which they are attached, to form a C₃₋₈cycloalkyl, and
   (iii) 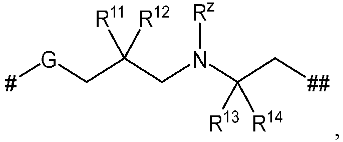 wherein
      G is O, NH, N(C₁₋₆alkyl), CH₂, or CH(C₁₋₆alkyl);
      R^{z} is H or C₁₋₆alkyl, wherein the C₁₋₆alkyl is optionally substituted with one or more C₃₋₈cycloalkyl;
      R¹¹ and R¹² are independently H, -OH, halo, or C₁₋₆alkyl; and
      R¹³ and R¹⁴ are independently H, C₁₋₆alkyl, or C₃₋₈cycloalkyl,
      or R¹³ and R¹⁴ are taken, together with the atoms to which they are attached, to form a 3-8 membered heterocyclyl,
wherein, for each of (i)-(iii), # denotes the point of attachment to the phenyl ring bearing moiety Q, and ## denotes the point of attachment to the phenyl ring bearing moieties X¹-X⁴; and
X¹, X², X³, and X⁴ are, independently of each other, H, halo, -CN, C₁₋₆alkyl, or C₁₋₆alkoxy, wherein the C₁₋₆alkyl or C₁₋₆alkoxy is optionally substituted with one or more halo,
   provided that at least one of X¹, X², X³, and X⁴ is halo, -CN, C₁₋₆alkyl, or C₁₋₆alkoxy, wherein the C₁₋₆alkyl or C₁₋₆alkoxy is optionally substituted with one or more halo.

Any embodiments provided herein of a compound of formula (A), or a stereoisomer or tautomer thereof, or a pharmaceutically acceptable salt of any of the foregoing, or any variation or embodiment thereof, are also embodiments of a compound of formula (A'), or a stereoisomer or tautomer thereof, or a pharmaceutically acceptable salt of any of the foregoing, or any variation or embodiment thereof.

In some embodiments, provided is a compound of formula (A), or a stereoisomer or tautomer thereof, or a pharmaceutically acceptable salt of any of the foregoing, wherein L is 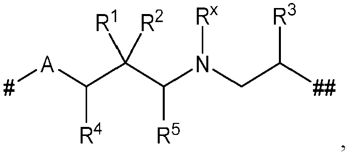 such that the compound of formula (A) is a compound of formula (I): 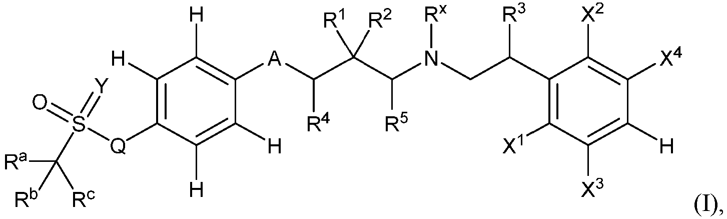 or a stereoisomer or tautomer thereof, or a pharmaceutically acceptable salt of any of the foregoing. In some variations, the X¹, X², X³, X⁴, R¹, R², R³, R⁴, R⁵, R^{a}, R^{b}, R^{c}, R^{x}, A Q, and Y of formula (I) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (I), or a stereoisomer or tautomer thereof, or a pharmaceutically acceptable salt of any of the foregoing, wherein A is O, such that the compound is of formula (I-A1): 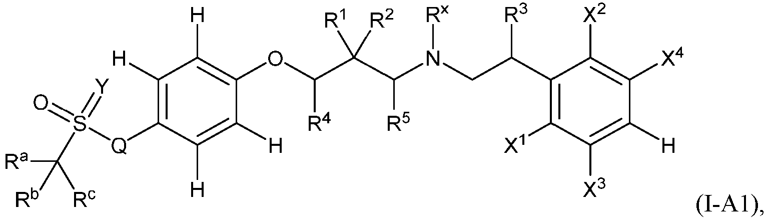 or a stereoisomer or tautomer thereof, or a pharmaceutically acceptable salt of any of the foregoing. In some variations, the X¹, X², X³, X⁴, R¹, R², R³, R⁴, R⁵, R^{a}, R^{b}, R^{c}, R^{x}, Q, and Y of formula (I-A1) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (I), or a stereoisomer or tautomer thereof, or a pharmaceutically acceptable salt of any of the foregoing, wherein A is NH, such that the compound is of formula (I-A2): 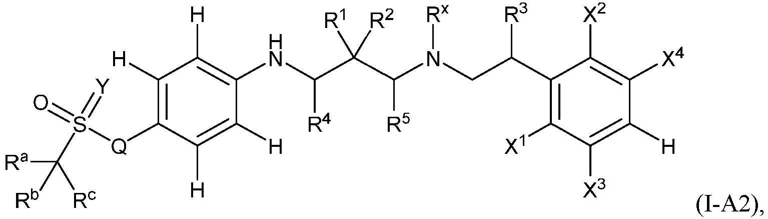 or a stereoisomer or tautomer thereof, or a pharmaceutically acceptable salt of any of the foregoing. In some embodiments, A is N(C₁₋₆alkyl). In some embodiments, A is N(CH₃). In some variations, X¹, X², X³, X⁴, R¹, R², R³, R⁴, R⁵, R^{a}, R^{b}, R^{c}, R^{x}, Q, and Y of formula (I-A2) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (I), or a stereoisomer or tautomer thereof, or a pharmaceutically acceptable salt of any of the foregoing, wherein A is CH₂, such that the compound is of formula (I-A3): 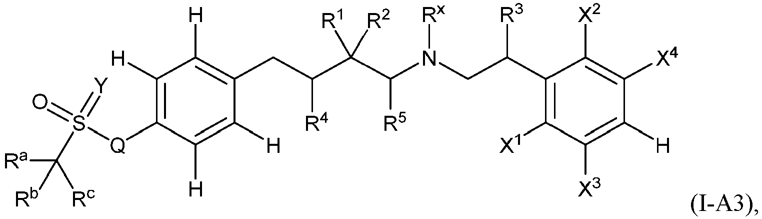 or a stereoisomer or tautomer thereof, or a pharmaceutically acceptable salt of any of the foregoing. In some embodiments, A is CH(C₁₋₆alkyl). In some embodiments, A is CH(CH₃). In some variations, X¹, X², X³, X⁴, R¹, R², R³, R⁴, R⁵, R^{a}, R^{b}, R^{c}, R^{x}, Q, and Y of formula (I-A3) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (I), such as a compound of formula (I-A1), (I-A2), or (I-A3), or a pharmaceutically acceptable salt of any of the foregoing, wherein R^{x} is taken together with one of R¹ and R², and the atoms to which they are attached, to form a 3-8 membered heterocyclyl, wherein the 3-8 membered heterocyclyl is substituted with *n* independently selected R^{g} substituents, wherein *n* is an integer from 0-6, and R^{g} is -OH, halo, C₁₋₆alkyl, or C₁₋₆alkoxy. In some embodiments, R^{x} is taken together with one of R¹ and R², and the atoms to which they are attached, to form a 3-6 membered heterocyclyl, wherein the 3-6 membered heterocyclyl is substituted with *n* independently selected R^{g} substituents, wherein *n* is an integer from 0-6, and R^{g} is -OH, halo, C₁₋₆alkyl, or C₁₋₆alkoxy. In some embodiments, R^{x} is taken together with one of R¹ and R², and the atoms to which they are attached, to form a 5-8 membered heterocyclyl, wherein the 5-8 membered heterocyclyl is substituted with *n* independently selected R^{g} substituents, wherein *n* is an integer from 0-6, and R^{g} is -OH, halo, C₁₋₆alkyl, or C₁₋₆alkoxy. In some embodiments, R^{x} is taken together with one of R¹ and R², and the atoms to which they are attached, to form a 5-6 membered heterocyclyl, wherein the 5-6 membered heterocyclyl is substituted with *n* independently selected R^{g} substituents, wherein *n* is an integer from 0-6, and R^{g} is -OH, halo, C₁₋₆alkyl, or C₁₋₆alkoxy. R^{x} is taken together with one of R¹ and R², and the atoms to which they are attached, to form a 5-membered heterocyclyl, wherein the 5-membered heterocyclyl is substituted with *n* independently selected R^{g} substituents, wherein *n* is an integer from 0-6, and R^{g} is -OH, halo, C₁₋₆alkyl, or C₁₋₆alkoxy. R^{x} is taken together with one of R¹ and R², and the atoms to which they are attached, to form a 6-membered heterocyclyl, wherein the 6-membered heterocyclyl is substituted with *n* independently selected R^{g} substituents, wherein *n* is an integer from 0-6, and R^{g} is -OH, halo, C₁₋₆alkyl, or C₁₋₆alkoxy. In some variations, the the embodiments provided herein also apply to a compound of formula (A') or a stereoisomer or tautomer thereof, or a pharmaceutically acceptable salt of any of the foregoing, or any variation or embodiment thereof.

In some embodiments, provided herein is a compound of formula (A) or formula (I), such as a compound of formula (I-A1), or a pharmaceutically acceptable salt of any of the foregoing, wherein R^{x} is taken together with one of R¹ and R², and the atoms to which they are attached, to form a 5-membered heterocyclyl, wherein the 5-membered heterocyclyl is substituted with *n* independently selected R^{g} substituents, wherein *n* is an integer from 0-6, and R^{g} is -OH, halo, C₁₋₆alkyl, or C₁₋₆alkoxy, wherein the compound is of formula (I-B1): 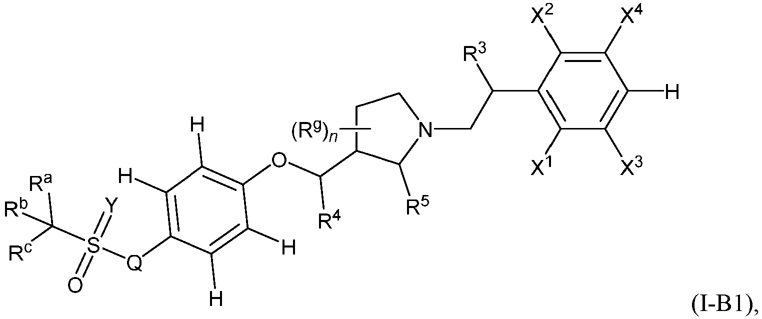 or a stereoisomer or tautomer thereof, or a pharmaceutically acceptable salt of any of the foregoing. In some variations, X¹, X², X³, X⁴, R³, R⁴, R⁵, R^{a}, R^{b}, R^{c}, R^{g}, R^{x}, Q, Y and *n* of formula (I-B1) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (I), such as a compound of formula (I-A2), or a pharmaceutically acceptable salt of any of the foregoing, wherein R^{x} is taken together with one of R¹ and R², and the atoms to which they are attached, to form a 5-membered heterocyclyl, wherein the 5-membered heterocyclyl is substituted with *n* independently selected R^{g} substituents, wherein *n* is an integer from 0-6, and R^{g} is -OH, halo, C₁₋₆alkyl, or C₁₋₆alkoxy, wherein the compound is of formula (I-B2): 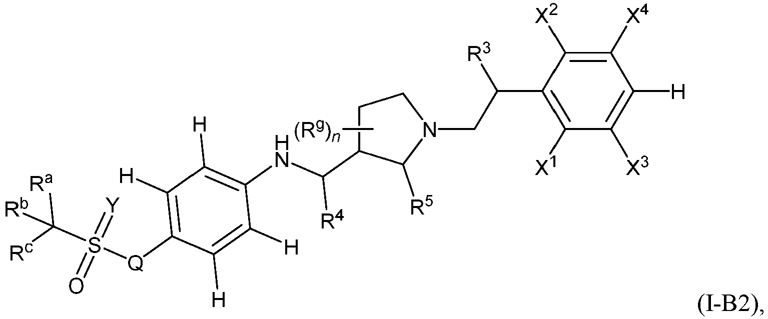 or a stereoisomer or tautomer thereof, or a pharmaceutically acceptable salt of any of the foregoing. In some variations, X¹, X², X³, X⁴, R³, R⁴, R⁵, R^{a}, R^{b}, R^{c}, R^{g}, R^{x}, Q, Y and *n* of formula (I-B2) are as defined of formula (A'), or a stereoisomer or tautomer thereof, or a pharmaceutically acceptable salt of any of the foregoing, or any variation or embodiment thereof.

In some embodiments, provided herein is a compound of formula (A) or formula (I), such as a compound of formula (I-A3), or a pharmaceutically acceptable salt of any of the foregoing, wherein R^{x} is taken together with one of R¹ and R², and the atoms to which they are attached, to form a 5-membered heterocyclyl, wherein the 5-membered heterocyclyl is substituted with *n* independently selected R^{g} substituents, wherein *n* is an integer from 0-6, and R^{g} is -OH, halo, C₁₋₆alkyl, or C₁₋₆alkoxy, wherein the compound is of formula (I-B3): 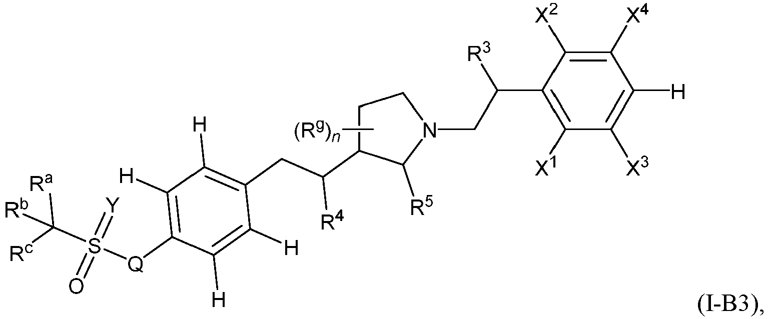 or a stereoisomer or tautomer thereof, or a pharmaceutically acceptable salt of any of the foregoing. In some variations, X¹, X², X³, X⁴, R³, R⁴, R⁵, R^{a}, R^{b}, R^{c}, R^{g}, R^{x}, Q, Y and *n* of formula (I-B3) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (I), such as a compound of formula (I-A1), or a pharmaceutically acceptable salt of any of the foregoing, wherein R^{x} is taken together with one of R¹ and R², and the atoms to which they are attached, to form a 6-membered heterocyclyl, wherein the 6-membered heterocyclyl is substituted with *n* independently selected R^{g} substituents, wherein *n* is an integer from 0-6, and R^{g} is -OH, halo, C₁₋₆alkyl, or C₁₋₆alkoxy, wherein the compound is of formula (I-C1): 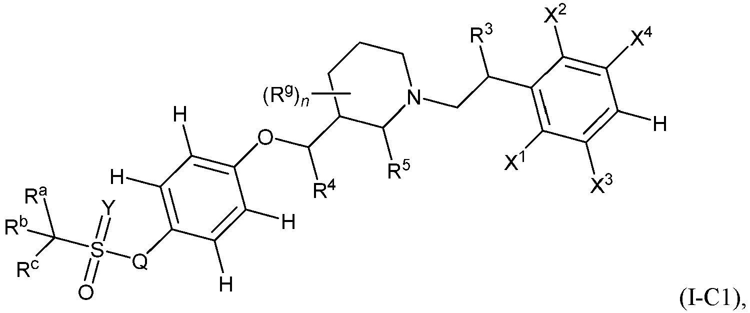 or a stereoisomer or tautomer thereof, or a pharmaceutically acceptable salt of any of the foregoing. In some variations, X¹, X², X³, X⁴, R³, R⁴, R⁵, R^{a}, R^{b}, R^{c}, R^{g}, R^{x}, Q, Y and *n* of formula (I-C1) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (I), such as a compound of formula (I-A2), or a pharmaceutically acceptable salt of any of the foregoing, wherein R^{x} is taken together with one of R¹ and R², and the atoms to which they are attached, to form a 6-membered heterocyclyl, wherein the 6-membered heterocyclyl is substituted with *n* independently selected R^{g} substituents, wherein *n* is an integer from 0-6, and R^{g} is -OH, halo, C₁₋₆alkyl, or C₁₋₆alkoxy, wherein the compound is of formula (I-C2): 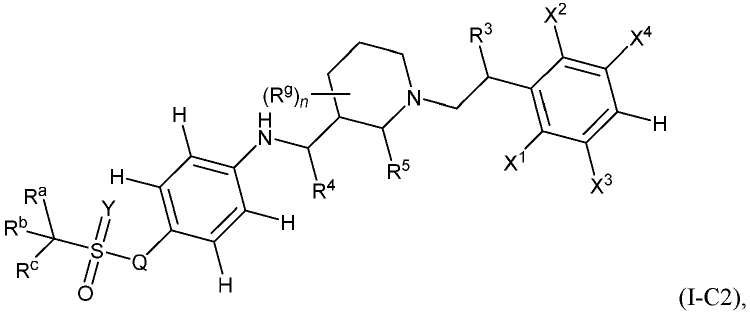 or a stereoisomer or tautomer thereof, or a pharmaceutically acceptable salt of any of the foregoing. In some variations, X¹, X², X³, X⁴, R³, R⁴, R⁵, R^{a}, R^{b}, R^{c}, R^{g}, R^{x}, Q, Y and *n* of formula (I-C2) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (I), such as a compound of formula (I-A3), or a pharmaceutically acceptable salt of any of the foregoing, wherein R^{x} is taken together with one of R¹ and R², and the atoms to which they are attached, to form a 6-membered heterocyclyl, wherein the 6-membered heterocyclyl is substituted with *n* independently selected R^{g} substituents, wherein *n* is an integer from 0-6, and R^{g} is -OH, halo, C₁₋₆alkyl, or C₁₋₆alkoxy, wherein the compound is of formula (I-C3): 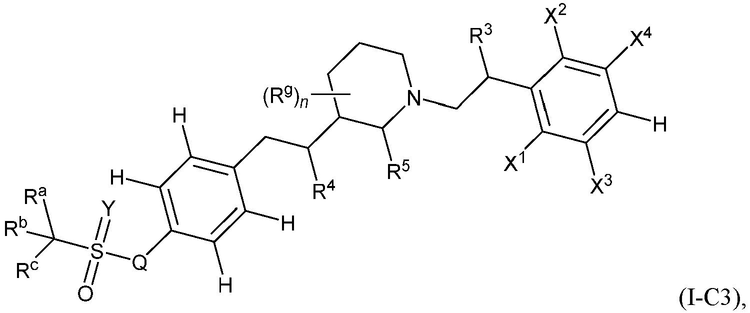 or a stereoisomer or tautomer thereof, or a pharmaceutically acceptable salt of any of the foregoing. In some variations, X¹, X², X³, X⁴, R³, R⁴, R⁵, R^{a}, R^{b}, R^{c}, R^{g}, R^{x}, Q, Y and *n* of formula (I-C3) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (I), such as a compound of formula (I-A1), or a pharmaceutically acceptable salt of any of the foregoing, wherein R^{x} is taken together with one of R¹ and R², and the atoms to which they are attached, to form a 6-membered heterocyclyl, wherein the 6-membered heterocyclyl is substituted with *n* independently selected R^{g} substituents, wherein *n* is an integer from 0-6, and R^{g} is -OH, halo, C₁₋₆alkyl, or C₁₋₆alkoxy, wherein the compound is of formula (I-D1): 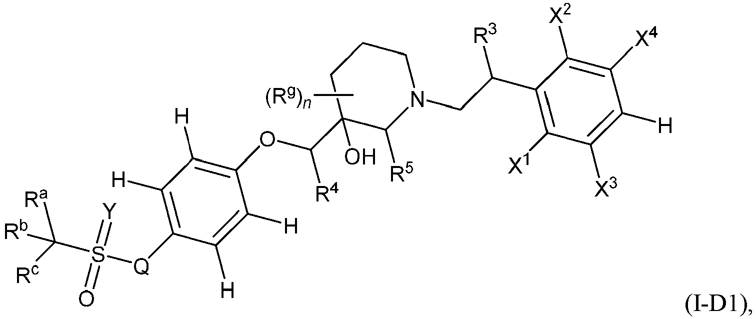 or a stereoisomer or tautomer thereof, or a pharmaceutically acceptable salt of any of the foregoing. In some variations, X¹, X², X³, X⁴, R³, R⁴, R⁵, R^{a}, R^{b}, R^{c}, R^{g}, R^{x}, Q, Y and *n* of formula (I-D1) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (I), such as a compound of formula (I-A2), or a pharmaceutically acceptable salt of any of the foregoing, wherein R^{x} is taken together with one of R¹ and R², and the atoms to which they are attached, to form a 6-membered heterocyclyl, wherein the 6-membered heterocyclyl is substituted with *n* independently selected R^{g} substituents, wherein *n* is an integer from 0-6, and R^{g} is -OH, halo, C₁₋₆alkyl, or C₁₋₆alkoxy, wherein the compound is of formula (I-D2): 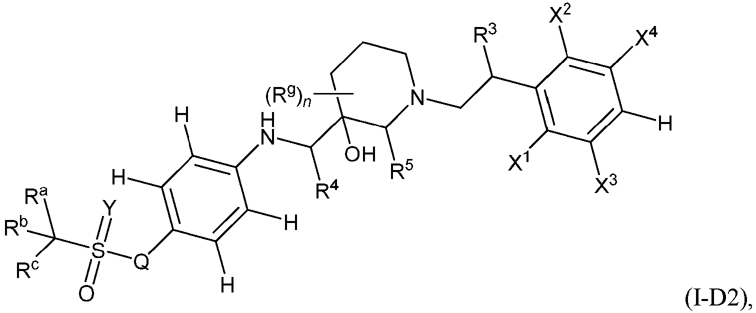 or a stereoisomer or tautomer thereof, or a pharmaceutically acceptable salt of any of the foregoing. In some variations, X¹, X², X³, X⁴, R³, R⁴, R⁵, R^{a}, R^{b}, R^{c}, R^{g}, R^{x}, Q, Y and *n* of formula (I-D2) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (I), such as a compound of formula (I-A3), or a pharmaceutically acceptable salt of any of the foregoing, wherein R^{x} is taken together with one of R¹ and R², and the atoms to which they are attached, to form a 6-membered heterocyclyl, wherein the 6-membered heterocyclyl is substituted with *n* independently selected R^{g} substituents, wherein *n* is an integer from 0-6, and R^{g} is -OH, halo, C₁₋₆alkyl, or C₁₋₆alkoxy, wherein the compound is of formula (I-D3): 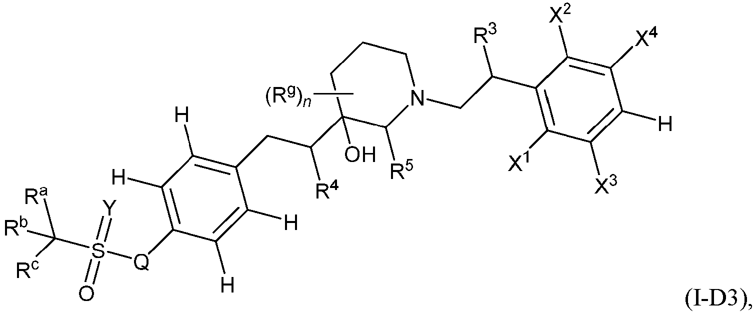 or a stereoisomer or tautomer thereof, or a pharmaceutically acceptable salt of any of the foregoing. In some variations, X¹, X², X³, X⁴, R³, R⁴, R⁵, R^{a}, R^{b}, R^{c}, R^{g}, R^{x}, Q, Y and *n* of formula (I-D3) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (I), such as a compound of formula (I-A1), or a pharmaceutically acceptable salt of any of the foregoing, wherein R^{x} is taken together with one of R¹ and R², and the atoms to which they are attached, to form a 6-membered heterocyclyl, wherein the 6-membered heterocyclyl is substituted with *n* independently selected R^{g} substituents, wherein *n* is an integer from 0-6, and R^{g} is -OH, halo, C₁₋₆alkyl, or C₁₋₆alkoxy, wherein the compound is of formula (I-E1): 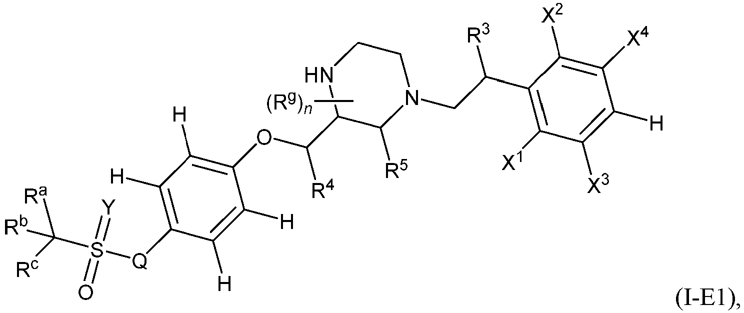 or a stereoisomer or tautomer thereof, or a pharmaceutically acceptable salt of any of the foregoing. In some variations, X¹, X², X³, X⁴, R³, R⁴, R⁵, R^{a}, R^{b}, R^{c}, R^{g}, R^{x}, Q, Y and *n* of formula (I-E1) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (I), such as a compound of formula (I-A2), or a pharmaceutically acceptable salt of any of the foregoing, wherein R^{x} is taken together with one of R¹ and R², and the atoms to which they are attached, to form a 6-membered heterocyclyl, wherein the 6-membered heterocyclyl is substituted with *n* independently selected R^{g} substituents, wherein *n* is an integer from 0-6, and R^{g} is -OH, halo, C₁₋₆alkyl, or C₁₋₆alkoxy, wherein the compound is of formula (I-E2): 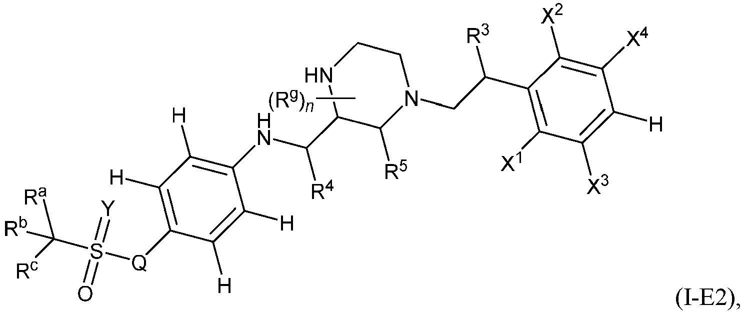 or a stereoisomer or tautomer thereof, or a pharmaceutically acceptable salt of any of the foregoing. In some variations, X¹, X², X³, X⁴, R³, R⁴, R⁵, R^{a}, R^{b}, R^{c}, R^{g}, R^{x}, Q, Y and *n* of formula (I-E2) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (I), such as a compound of formula (I-A3), or a pharmaceutically acceptable salt of any of the foregoing, wherein R^{x} is taken together with one of R¹ and R², and the atoms to which they are attached, to form a 6-membered heterocyclyl, wherein the 6-membered heterocyclyl is substituted with *n* independently selected R^{g} substituents, wherein *n* is an integer from 0-6, and R^{g} is -OH, halo, C₁₋₆alkyl, or C₁₋₆alkoxy, wherein the compound is of formula (I-E3): 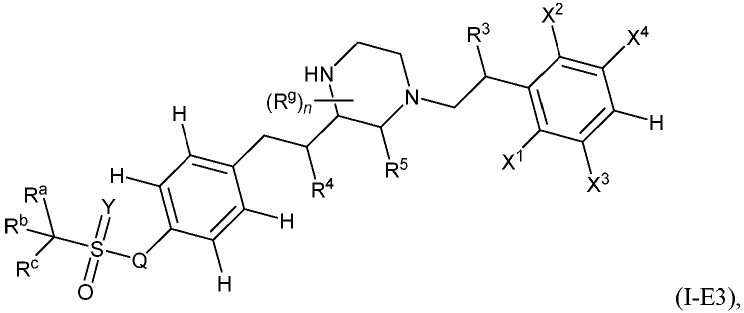 or a stereoisomer or tautomer thereof, or a pharmaceutically acceptable salt of any of the foregoing. In some variations, X¹, X², X³, X⁴, R³, R⁴, R⁵, R^{a}, R^{b}, R^{c}, R^{g}, R^{x}, Q, Y and *n* of formula (I-E3) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (I), such as a compound of formula (I-E1), (I-E2), or (I-E3), or a stereoisomer or tautomer thereof, or a pharmaceutically acceptable salt of any of the foregoing, wherein n is 0.

In some embodiments, provided herein is a compound of formula (A') or formula (I), such as a compound of formula (I-E1), (I-E2), or (I-E3), or a stereoisomer or tautomer thereof, or a pharmaceutically acceptable salt of any of the foregoing, wherein n is 0 or 1. In some embodiments, *n* is 0. In some embodiments, *n is* 1.

In some embodiments, provided herein is a compound of formula (A) or formula (I), such as a compound of formula (I-A1), or a pharmaceutically acceptable salt of any of the foregoing, wherein R^{x} is taken together with one of R¹ and R², and the atoms to which they are attached, to form a 7-membered heterocyclyl, wherein the 7-membered heterocyclyl is substituted with *n* independently selected R^{g} substituents, wherein *n* is an integer from 0-6, and R^{g} is -OH, halo, C₁₋₆alkyl, or C₁₋₆alkoxy, wherein the compound is of formula (I-F1): 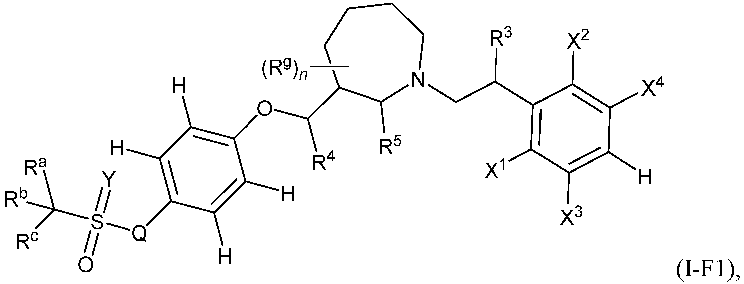 or a stereoisomer or tautomer thereof, or a pharmaceutically acceptable salt of any of the foregoing. In some variations, X¹, X², X³, X⁴, R³, R⁴, R⁵, R^{a}, R^{b}, R^{c}, R^{g}, R^{x}, Q, Y and *n* of formula (I-F1) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (I), such as a compound of formula (I-A2), or a pharmaceutically acceptable salt of any of the foregoing, wherein R^{x} is taken together with one of R¹ and R², and the atoms to which they are attached, to form a 7-membered heterocyclyl, wherein the 7-membered heterocyclyl is substituted with *n* independently selected R^{g} substituents, wherein *n* is an integer from 0-6, and R^{g} is -OH, halo, C₁₋₆alkyl, or C₁₋₆alkoxy, wherein the compound is of formula (I-F2): 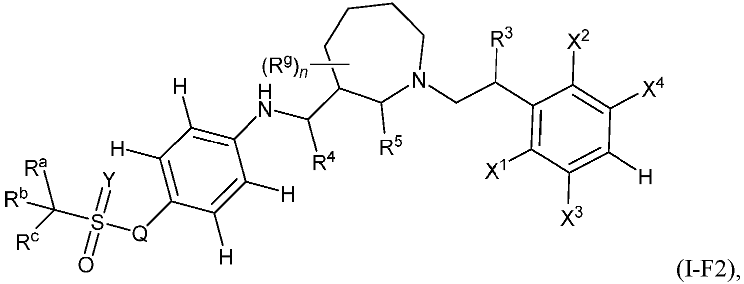 or a stereoisomer or tautomer thereof, or a pharmaceutically acceptable salt of any of the foregoing. In some variations, X¹, X², X³, X⁴, R³, R⁴, R⁵, R^{a}, R^{b}, R^{c}, R^{g}, R^{x}, Q, Y and *n* of formula (I-F2) are as defined for compound of formula (A'), or a stereoisomer or tautomer thereof, or a pharmaceutically acceptable salt of any of the foregoing, or any variation or embodiment thereof.

In some embodiments, provided herein is a compound of formula (A) or formula (I), such as a compound of formula (I-A3), or a pharmaceutically acceptable salt of any of the foregoing, wherein R^{x} is taken together with one of R¹ and R², and the atoms to which they are attached, to form a 7-membered heterocyclyl, wherein the 7-membered heterocyclyl is substituted with *n* independently selected R^{g} substituents, wherein *n* is an integer from 0-6, and R^{g} is -OH, halo, C₁₋₆alkyl, or C₁₋₆alkoxy, wherein the compound is of formula (I-F3): 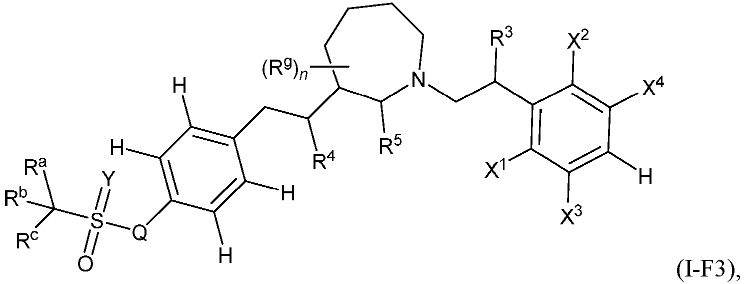 or a stereoisomer or tautomer thereof, or a pharmaceutically acceptable salt of any of the foregoing. In some variations, X¹, X², X³, X⁴, R³, R⁴, R⁵, R^{a}, R^{b}, R^{c}, R^{g}, R^{x}, Q, Y and *n* of formula (I-F3) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (I), such as a compound of formula (I-A1), or a pharmaceutically acceptable salt of any of the foregoing, wherein R^{x} is taken together with one of R¹ and R², and the atoms to which they are attached, to form a 7-membered heterocyclyl, wherein the 7-membered heterocyclyl is substituted with *n* independently selected R^{g} substituents, wherein *n* is an integer from 0-6, and R^{g} is -OH, halo, C₁₋₆alkyl, or C₁₋₆alkoxy, wherein the compound is of formula (I-G1): 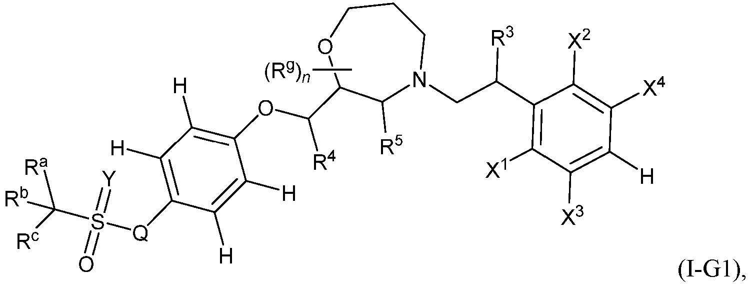 or a stereoisomer or tautomer thereof, or a pharmaceutically acceptable salt of any of the foregoing. In some variations, X¹, X², X³, X⁴, R³, R⁴, R⁵, R^{a}, R^{b}, R^{c}, R^{g}, R^{x}, Q, Y and *n* of formula (I-G1) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (I), such as a compound of formula (I-A2), or a pharmaceutically acceptable salt of any of the foregoing, wherein R^{x} is taken together with one of R¹ and R², and the atoms to which they are attached, to form a 7-membered heterocyclyl, wherein the 7-membered heterocyclyl is substituted with *n* independently selected R^{g} substituents, wherein *n* is an integer from 0-6, and R^{g} is -OH, halo, C₁₋₆alkyl, or C₁₋₆alkoxy, wherein the compound is of formula (I-G2): 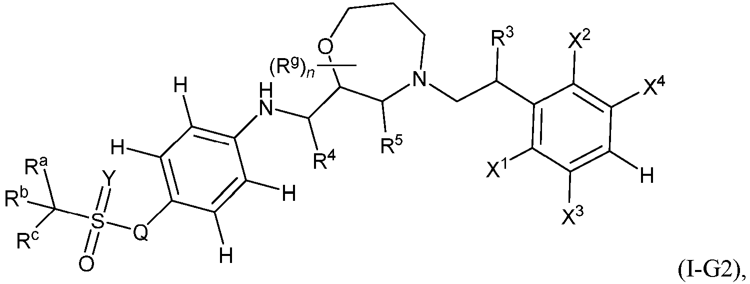 or a stereoisomer or tautomer thereof, or a pharmaceutically acceptable salt of any of the foregoing. In some variations, X¹, X², X³, X⁴, R³, R⁴, R⁵, R^{a}, R^{b}, R^{c}, R^{g}, R^{x}, Q, Y and *n* of formula (I-G2) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (I), such as a compound of formula (I-A3), or a pharmaceutically acceptable salt of any of the foregoing, wherein R^{x} is taken together with one of R¹ and R², and the atoms to which they are attached, to form a 7-membered heterocyclyl, wherein the 7-membered heterocyclyl is substituted with *n* independently selected R^{g} substituents, wherein *n* is an integer from 0-6, and R^{g} is -OH, halo, C₁₋₆alkyl, or C₁₋₆alkoxy, wherein the compound is of formula (I-G3): 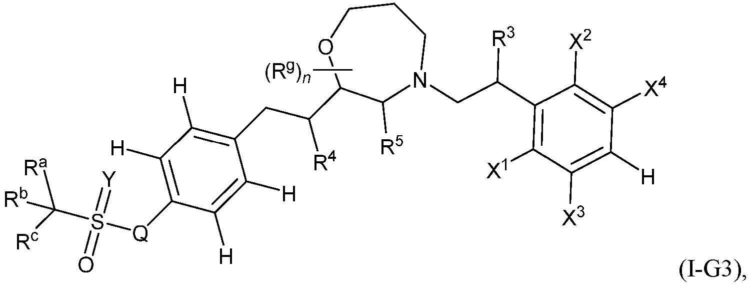 or a stereoisomer or tautomer thereof, or a pharmaceutically acceptable salt of any of the foregoing. In some variations, X¹, X², X³, X⁴, R³, R⁴, R⁵, R^{a}, R^{b}, R^{c}, R^{g}, R^{x}, Q, Y and *n* of formula (I-G3) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (I), such as a compound of formula (I-A1), (I-A2), (I-A3), (I-B1), (I-B2), (I-B3), (I-C1), (I-C2), (I-C3), (I-D1), (I-D2), (I-D3), (I-E1), (I-E2), (I-E3), (I-F1), (I-F2), (I-F3), (I-G1), (I-G2), or (I-G3), or a stereoisomer or tautomer thereof, or a pharmaceutically acceptable salt of any of the foregoing, wherein *n* is an integer from 0-6. In some embodiments, *n* is an integer from 0-5. In some embodiments, *n* is an integer from 0-4. In some embodiments, *n* is an integer from 0-3. In some embodiments, *n* is an integer from 0-2. In some embodiments, *n is* 0 or 1. In some embodiments, *n* is an integer from 1-6. In some embodiments, *n* is an integer from 1-5. In some embodiments, *n* is an integer from 1-4. In some embodiments, *n* is an integer from 1-3. In some embodiments, *n is* 1 or 2. In some embodiments, *n* is 0. In some embodiments, *n* is 1. In some embodiments, *n* is 2. In some variations, the embodiments provided herein also apply to a compound of formula (A') or a stereoisomer or tautomer thereof, or a pharmaceutically acceptable salt of any of the foregoing, or any variation or embodiment thereof.

In some embodiments, provided herein is a compound of formula (A) or formula (I), such as a compound of formula (I-A1), (I-A2), (I-A3), (I-B1), (I-B2), (I-B3), (I-C1), (I-C2), (I-C3), (I-D1), (I-D2), (I-D3), (I-E1), (I-E2), (I-E3), (I-F1), (I-F2), (I-F3), (I-G1), (I-G2), or (I-G3), or a stereoisomer or tautomer thereof, or a pharmaceutically acceptable salt of any of the foregoing, wherein R^{g} is -OH, halo, C₁₋₆alkyl, or C₁₋₆alkoxy. In some embodiments, R^{g} is - OH, C₁₋₆alkyl, or C₁₋₆alkoxy. In some embodiments, R^{g} is -OH, halo, C₁₋₃alkyl, or C₁₋₃alkoxy. In some embodiments, R^{g} is -OH, methyl, or methoxy. In some embodiments, R^{g} is -OH. In some embodiments, R^{g} is C₁₋₆alkyl. In some embodiments, R^{B} is methyl. In some embodiments, R^{g} is C₁₋₆alkoxy. In some embodiments, R^{g} is methoxy. In some embodiments, R^{g} is halo. In some embodiments, R^{g} is fluoro. In some variations, the embodiments provided herein also apply to a compound of formula (A') or a stereoisomer or tautomer thereof, or a pharmaceutically acceptable salt of any of the foregoing, or any variation or embodiment thereof.

In some embodiments, provided herein is a compound of formula (A) or formula (I), such as a compound of formula (I-A1), (I-A2), (I-A3), (I-B1), (I-B2), (I-B3), (I-C1), (I-C2), (I-C3), (I-D1), (I-D2), (I-D3), (I-E1), (I-E2), (I-E3), (I-F1), (I-F2), (I-F3), (I-G1), (I-G2), or (I-G3), or a stereoisomer or tautomer thereof, or a pharmaceutically acceptable salt of any of the foregoing, wherein *n* is 1 and R^{g} is C₁₋₆alkyl. In some embodiments, *n* is 1 and R^{g} is methyl. In some embodiments, *n* is 2 and each R^{g} is C₁₋₆alkyl. In some embodiments, *n* is 2 and each R^{g} is methyl. In some embodiments, *n* is 1 and R^{g} is -OH. In some embodiments, *n* is 1 and R^{g} is C₁₋₆alkoxy. In some embodiments, *n* is 1 and R^{g} is methoxy.

In some embodiments, provided herein is a compound of formula (A') or formula (I), such as a compound of formula (I-A1), (I-A2), (I-A3), (I-B1), (I-B2), (I-B3), (I-C1), (I-C2), (I-C3), (I-D1), (I-D2), (I-D3), (I-E1), (I-E2), (I-E3), (I-F1), (I-F2), (I-F3), (I-G1), (I-G2), or (I-G3), or a stereoisomer or tautomer thereof, or a pharmaceutically acceptable salt of any of the foregoing, wherein *n* is 1 and R^{g} is C₁₋₆alkyl. In some embodiments, *n* is 1 and R^{g} is methyl. In some embodiments, *n* is 2 and each R^{g} is C₁₋₆alkyl. In some embodiments, *n* is 2 and each R^{g} is methyl. In some embodiments, *n* is 1 and R^{g} is -OH. In some embodiments, *n* is 1 and R^{g} is C₁₋₆alkoxy. In some embodiments, *n* is 1 and R^{g} is methoxy. In some embodiments, *n* is 2, one or R^{g} is methyl, and the other of R^{g} is -OH.

In some embodiments, provided herein is a compound of formula (A) or formula (I), such as a compound of formula (I-A1), (I-A2), (I-A3), (I-B1), (I-B2), (I-B3), (I-C1), (I-C2), (I-C3), (I-D1), (I-D2), (I-D3), (I-E1), (I-E2), (I-E3), (I-F1), (I-F2), (I-F3), (I-G1), (I-G2), or (I-G3), or a stereoisomer or tautomer thereof, or a pharmaceutically acceptable salt of any of the foregoing, wherein R⁴ and R⁵ are independently H. In some variations, the embodiments provided herein also apply to a compound of formula (A') or a stereoisomer or tautomer thereof, or a pharmaceutically acceptable salt of any of the foregoing, or any variation or embodiment thereof.

In some embodiments, provided herein is a compound of formula (A) or formula (I), or a pharmaceutically acceptable salt of any of the foregoing, wherein R⁴ and R⁵ are taken, together with the atoms to which they are attached, to form a C₃₋₈cycloalkyl. In some embodiments, R⁴ and R⁵ are taken, together with the atoms to which they are attached, to form a C₃₋₆cycloalkyl. In some embodiments, R⁴ and R⁵ are taken, together with the atoms to which they are attached, to form cyclobutyl. In some variations, the embodiments provided herein also apply to a compound of formula (A') or a stereoisomer or tautomer thereof, or a pharmaceutically acceptable salt of any of the foregoing, or any variation or embodiment thereof.

In some embodiments, provided herein is a compound of formula (A) or formula (I), such as a compound of formula (I-A1), or a pharmaceutically acceptable salt of any of the foregoing, wherein R⁴ and R⁵ are taken, together with the atoms to which they are attached, to form cyclobutyl, wherein the compound is of formula (I-H1): 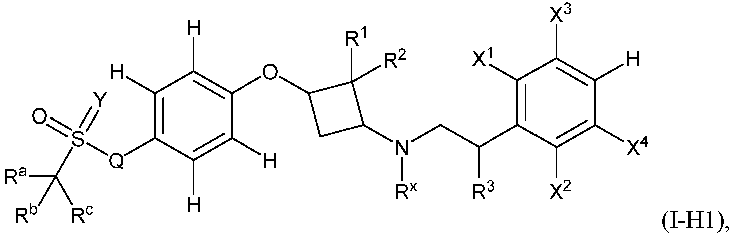 or a stereoisomer or tautomer thereof, or a pharmaceutically acceptable salt of any of the foregoing. In some variations, X¹, X², X³, X⁴, R³, R⁴, R⁵, R^{a}, R^{b}, R^{c}, R^{x}, Q, and Y of formula (I-H1) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (I), such as a compound of formula (I-A2), or a pharmaceutically acceptable salt of any of the foregoing, wherein R⁴ and R⁵ are taken, together with the atoms to which they are attached, to form cyclobutyl, wherein the compound is of formula (I-H2): 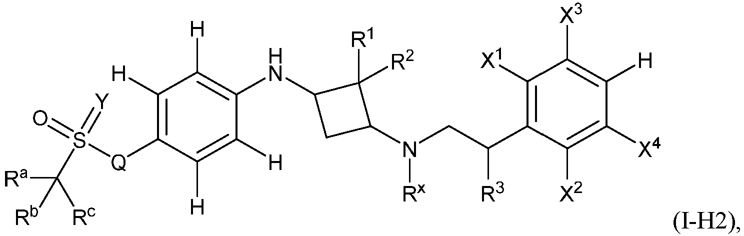 or a stereoisomer or tautomer thereof, or a pharmaceutically acceptable salt of any of the foregoing. In some variations, X¹, X², X³, X⁴, R³, R⁴, R⁵, R^{a}, R^{b}, R^{c}, R^{x}, Q, and Y of formula (I-H2) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (I), such as a compound of formula (I-A3), or a pharmaceutically acceptable salt of any of the foregoing, wherein R⁴ and R⁵ are taken, together with the atoms to which they are attached, to form cyclobutyl, wherein the compound is of formula (I-H3): 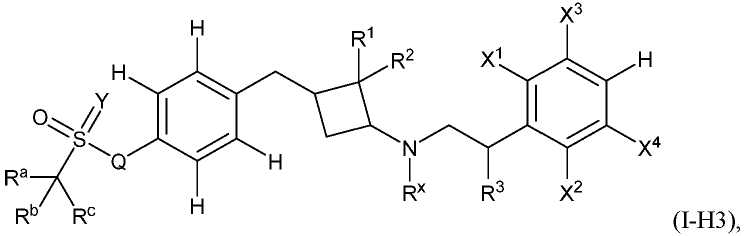 or a stereoisomer or tautomer thereof, or a pharmaceutically acceptable salt of any of the foregoing. In some variations, X¹, X², X³, X⁴, R³, R⁴, R⁵, R^{a}, R^{b}, R^{c}, R^{x}, Q, and Y of formula (I-H3) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (I), such as a compound of formula (I-H1), (I-H2), or (I-H3), or a stereoisomer or tautomer thereof, or a pharmaceutically acceptable salt of any of the foregoing, wherein R¹ and R² are each H. In some embodiments, provided herein is a compound of formula (A) or formula (I), such as a compound of formula (I-H1), (I-H2), or (I-H3), or a stereoisomer or tautomer thereof, or a pharmaceutically acceptable salt of any of the foregoing, wherein R^{x} is H. In some embodiments, R¹, R², and R^{x} are each H. In some embodiments, the embodiments provided herein also apply to a compound of formula (A') or a stereoisomer or tautomer thereof, or a pharmaceutically acceptable salt of any of the foregoing, or any variation or embodiment thereof.

In some embodiments, provided herein is a compound of formula (A) or formula (I), such as a compound of formula (I-A1), (I-A2), (I-A3), (I-B1), (I-B2), (I-B3), (I-C1), (I-C2), (I-C3), (I-D1), (I-D2), (I-D3), (I-E1), (I-E2), (I-E3), (I-F1), (I-F2), (I-F3), (I-G1), (I-G2), (I-G3), (I-H1), (I-H2), or (I-H3), or a stereoisomer or tautomer thereof, or a pharmaceutically acceptable salt of any of the foregoing, wherein R³ is H, -OH, halo, or C₁₋₆alkoxy. In some embodiments, R³ is H. In some embodiments, R³ is -OH, halo, or C₁₋₆alkoxy. In some embodiments, R³ is H or -OH. In some embodiments, R³ is -OH. In some embodiments, R³ is halo. In some embodiments, R³ is C₁₋₆alkoxy. In some embodiments, R³ is methoxy.

In some embodiments, provided herein is a compound of formula (A) or formula (I), such as a compound of formula (I-A1), (I-A2), (I-A3), (I-B1), (I-B2), (I-B3), (I-C1), (I-C2), (I-C3), (I-D1), (I-D2), (I-D3), (I-E1), (I-E2), (I-E3), (I-F1), (I-F2), (I-F3), (I-G1), (I-G2), or (I-G3), or a stereoisomer or tautomer thereof, or a pharmaceutically acceptable salt of any of the foregoing, wherein R³, R⁴, and R⁵ are each H. In some embodiments, provided herein is a compound of formula (A) or formula (I), such as a compound of formula (I-H1), (I-H2), or (I-H3), or a stereoisomer or tautomer thereof, or a pharmaceutically acceptable salt of any of the foregoing, wherein R¹, R², R³, and R^{x} are each H. In some embodiments, the embodiments provided herein also apply to a compound of formula (A') or a stereoisomer or tautomer thereof, or a pharmaceutically acceptable salt of any of the foregoing, or any variation or embodiment thereof.In some embodiments, provided herein is a compound of formula (A), or a stereoisomer or tautomer thereof, or a pharmaceutically acceptable salt of any of the foregoing, wherein L is 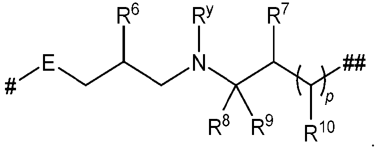 In some embodiments, L is 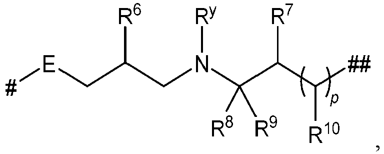 and the compound of formula (A) is a compound of formula (II): 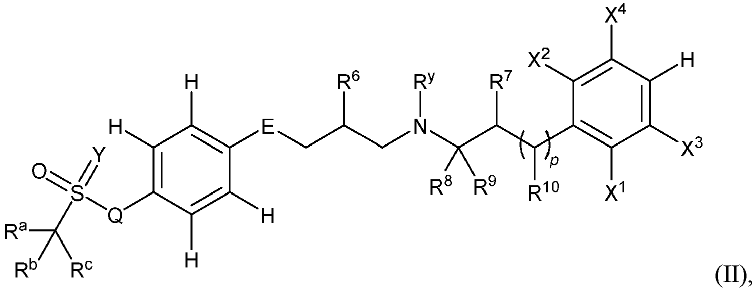 or a stereoisomer or tautomer thereof, or a pharmaceutically acceptable salt of any of the foregoing. In some variations, X¹, X², X³, X⁴, R⁶, R⁷, R⁸, R⁹, R¹⁰, R^{a}, R^{b}, R^{c}, R^{y}, *p,* E, Q, and Y of formula (II) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (II), or a stereoisomer or tautomer thereof, or a pharmaceutically acceptable salt of any of the foregoing, wherein E is O, wherein the compound is of formula (II-A1): 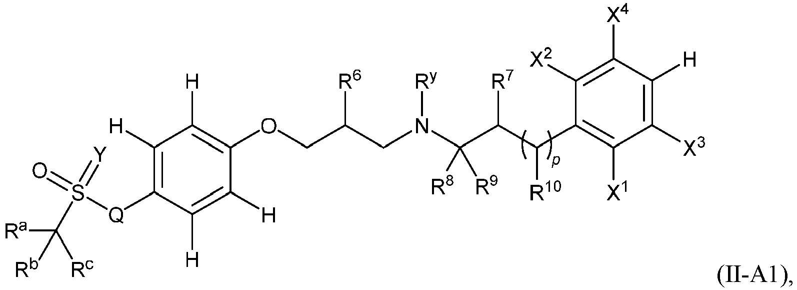 or a stereoisomer or tautomer thereof, or a pharmaceutically acceptable salt of any of the foregoing. In some variations, X¹, X², X³, X⁴, R⁶, R⁷, R⁸, R⁹, R¹⁰, R^{a}, R^{b}, R^{c}, R^{y}, *p,* Q, and Y of formula (II-A1) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (II), or a stereoisomer or tautomer thereof, or a pharmaceutically acceptable salt of any of the foregoing, wherein E is NH, wherein the compound is of formula (II-A2): 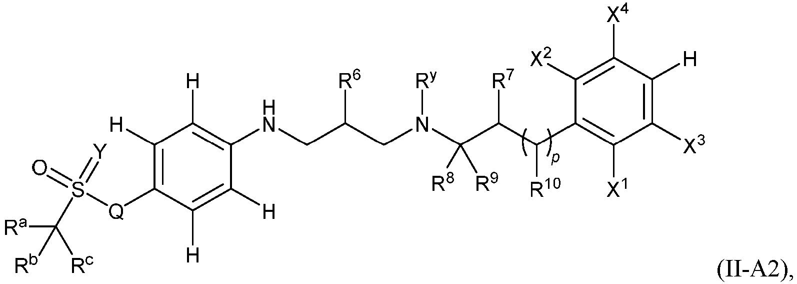 or a stereoisomer or tautomer thereof, or a pharmaceutically acceptable salt of any of the foregoing. In some embodiments, E is N(C₁₋₆alkyl). In some embodiments, E is N(CH₃). In some variations, X¹, X², X³, X⁴, R⁶, R⁷, R⁸, R⁹, R¹⁰, R^{a}, R^{b}, R^{c}, R^{y}, *p,* Q, and Y of formula (II-A2) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (II), or a stereoisomer or tautomer thereof, or a pharmaceutically acceptable salt of any of the foregoing, wherein E is CH₂, wherein the compound is of formula (II-A3): 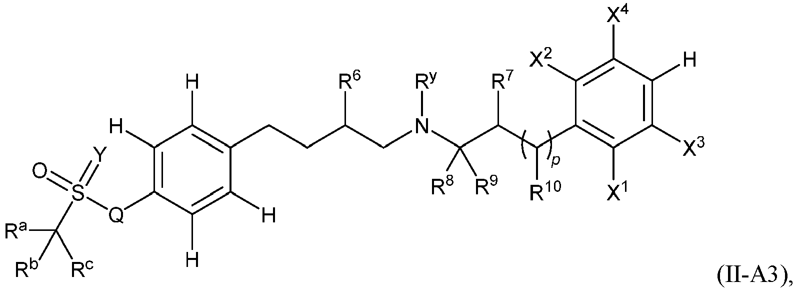 or a stereoisomer or tautomer thereof, or a pharmaceutically acceptable salt of any of the foregoing. In some embodiments, E is CH(C₁₋₆alkyl). In some embodiments, E is CH(CH₃). In some variations, X¹, X², X³, X⁴, R⁶, R⁷, R⁸, R⁹, R¹⁰, R^{a}, R^{b}, R^{c}, R^{y}, *p,* Q, and Y of formula (II-A3) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (II), or a stereoisomer or tautomer thereof, or a pharmaceutically acceptable salt of any of the foregoing, wherein R^{y} is taken together with R⁷, and the atoms to which they are attached, to form a 3-8 membered heterocyclyl. In some embodiments, R^{y} is taken together with R⁷, and the atoms to which they are attached, to form a 3-6 membered heterocyclyl. In some embodiments, R^{y} is taken together with R⁷, and the atoms to which they are attached, to form a 5-8 membered heterocyclyl. In some embodiments, R^{y} is taken together with R⁷, and the atoms to which they are attached, to form a 5-6 membered heterocyclyl. In some embodiments, R^{y} is taken together with R⁷, and the atoms to which they are attached, to form a 5-membered heterocyclyl. In some embodiments, R^{y} is taken together with R⁷, and the atoms to which they are attached, to form a 6-membered heterocyclyl. In some variations, the embodiments provided herein also apply to a compound of formula (A') or a stereoisomer or tautomer thereof, or a pharmaceutically acceptable salt of any of the foregoing, or any variation or embodiment thereof.

In some embodiments of the foregoing, provided herein is a compound of formula (A) or formula (II), such as a compound of formula (II-A1), (II-A2), or (II-A3), or a stereoisomer or tautomer thereof, or a pharmaceutically acceptable salt of any of the foregoing, wherein *p* is 1. In other embodiments, provided herein is a compound of formula (A) or formula (II), such as a compound of formula (II-A1), (II-A2), or (II-A3), or a stereoisomer or tautomer thereof, or a pharmaceutically acceptable salt of any of the foregoing, wherein *p* is 0. In some variations, the embodiments provided herein also apply to a compound of formula (A') or a stereoisomer or tautomer thereof, or a pharmaceutically acceptable salt of any of the foregoing, or any variation or embodiment thereof.

In some embodiments, provided herein is a compound of formula (A) or formula (II), such as a compound of formula (II-A1), or a stereoisomer or tautomer thereof, or a pharmaceutically acceptable salt of any of the foregoing, wherein R^{y} is taken together with R⁷, and the atoms to which they are attached, to form a 6-membered heterocyclyl, and *p* is 1, wherein the compound is of formula (II-B1): 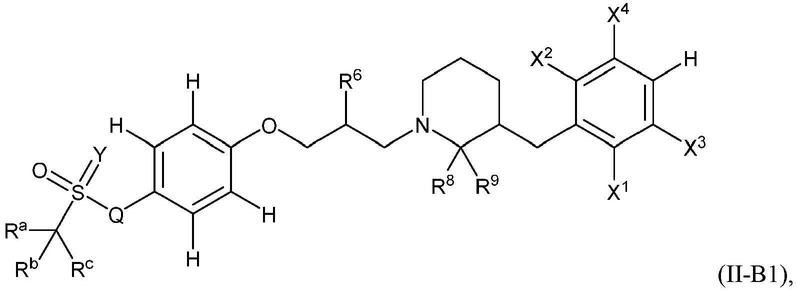 or a stereoisomer or tautomer thereof, or a pharmaceutically acceptable salt of any of the foregoing. In some variations, X¹, X², X³, X⁴, R⁶, R⁸, R⁹, R¹⁰, R^{a}, R^{b}, R^{c}, Q, and Y of formula (II-B1) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (II), such as a compound of formula (II-A1), or a stereoisomer or tautomer thereof, or a pharmaceutically acceptable salt of any of the foregoing, wherein R^{y} is taken together with R⁷, and the atoms to which they are attached, to form a 5-membered heterocyclyl, and *p* is 0, wherein the compound is of formula (II-C1): 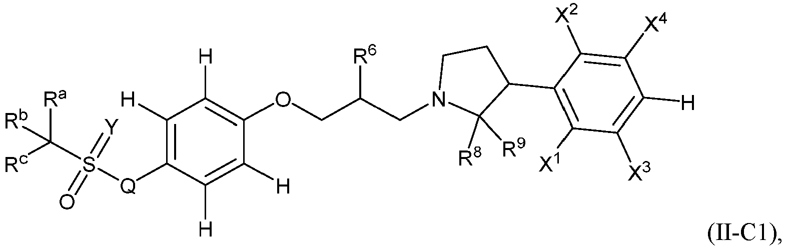 or a stereoisomer or tautomer thereof, or a pharmaceutically acceptable salt of any of the foregoing. In some variations, X¹, X², X³, X⁴, R⁶, R⁸, R⁹, R¹⁰, R^{a}, R^{b}, R^{c}, Q, and Y of formula (II-C1) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (II), such as a compound of formula (II-A2), or a stereoisomer or tautomer thereof, or a pharmaceutically acceptable salt of any of the foregoing, wherein R^{y} is taken together with R⁷, and the atoms to which they are attached, to form a 5-membered heterocyclyl, and *p* is 0, wherein the compound is of formula (II-C2): 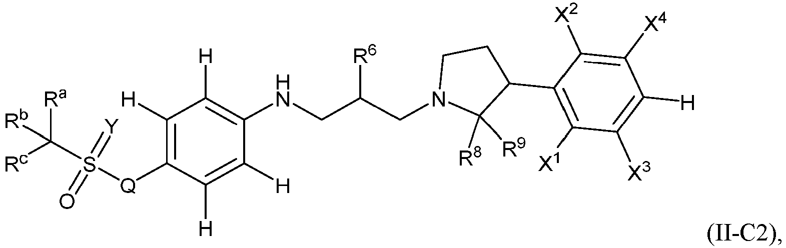 or a stereoisomer or tautomer thereof, or a pharmaceutically acceptable salt of any of the foregoing. In some variations, X¹, X², X³, X⁴, R⁶, R⁸, R⁹, R¹⁰, R^{a}, R^{b}, R^{c}, Q, and Y of formula (II-C2) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (II), such as a compound of formula (II-A3), or a stereoisomer or tautomer thereof, or a pharmaceutically acceptable salt of any of the foregoing, wherein R^{y} is taken together with R⁷, and the atoms to which they are attached, to form a 5-membered heterocyclyl, and *p* is 0, wherein the compound is of formula (II-C3): 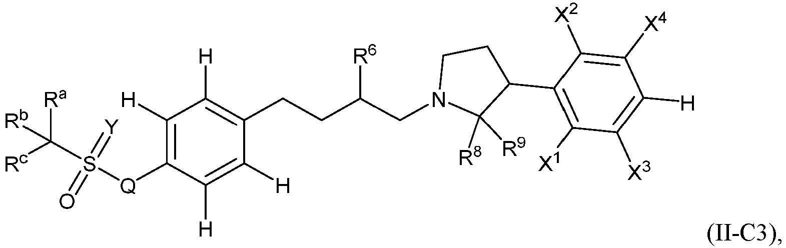 or a stereoisomer or tautomer thereof, or a pharmaceutically acceptable salt of any of the foregoing. In some variations, X¹, X², X³, X⁴, R⁶, R⁸, R⁹, R¹⁰, R^{a}, R^{b}, R^{c}, Q, and Y of formula (II-C3) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (II), such as a compound of formula (II-A1), or a stereoisomer or tautomer thereof, or a pharmaceutically acceptable salt of any of the foregoing, wherein R^{y} is taken together with R⁷, and the atoms to which they are attached, to form a 6-membered heterocyclyl, and *p* is 0, wherein the compound is of formula (II-D1): 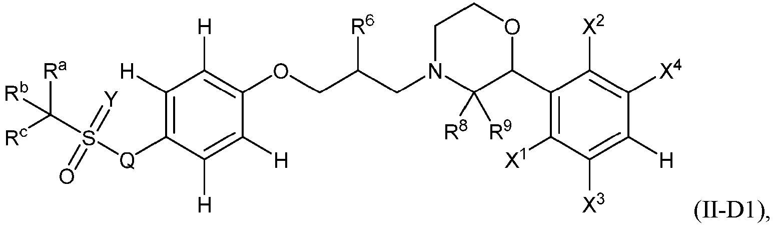 or a stereoisomer or tautomer thereof, or a pharmaceutically acceptable salt of any of the foregoing. In some variations, X¹, X², X³, X⁴, R⁶, R⁸, R⁹, R¹⁰, R^{a}, R^{b}, R^{c}, Q, and Y of formula (II-D1) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (II), such as a compound of formula (II-A2), or a stereoisomer or tautomer thereof, or a pharmaceutically acceptable salt of any of the foregoing, wherein R^{y} is taken together with R⁷, and the atoms to which they are attached, to form a 6-membered heterocyclyl, and *p* is 0, wherein the compound is of formula (II-D2): 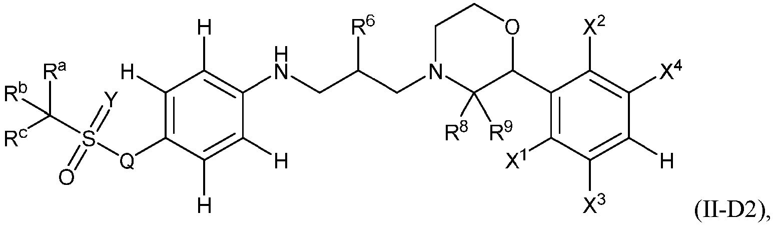 or a stereoisomer or tautomer thereof, or a pharmaceutically acceptable salt of any of the foregoing. In some variations, X¹, X², X³, X⁴, R⁶, R⁸, R⁹, R¹⁰, R^{a}, R^{b}, R^{c}, Q, and Y of formula (II-D2) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (II), such as a compound of formula (II-A3), or a stereoisomer or tautomer thereof, or a pharmaceutically acceptable salt of any of the foregoing, wherein R^{y} is taken together with R⁷, and the atoms to which they are attached, to form a 6-membered heterocyclyl, and *p* is 0, wherein the compound is of formula (II-D3): 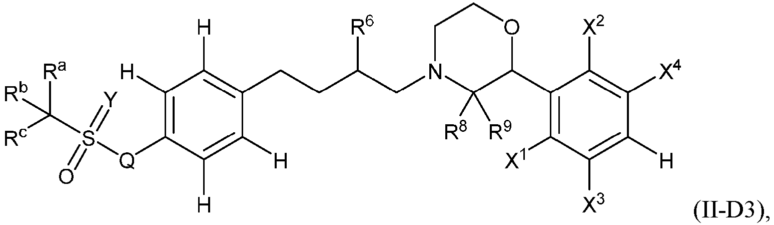 or a stereoisomer or tautomer thereof, or a pharmaceutically acceptable salt of any of the foregoing. In some variations, X¹, X², X³, X⁴, R⁶, R⁸, R⁹, R¹⁰, R^{a}, R^{b}, R^{c}, Q, and Y of formula (II-D3) are as defined for a compound of formula (A'), or a stereoisomer or tautomer thereof, or a pharmaceutically acceptable salt of any of the foregoing, or any variation or embodiment thereof.

In some embodiments of the foregoing, provided herein is a compound of formula (A) or formula (II), such as a compound of formula (II-A1), (II-A2), (II-A3), (II-B1), (II-C1), (II-C2), (II-C3), (II-D1), (II-D2), or (II-D3), or a stereoisomer or tautomer thereof, or a pharmaceutically acceptable salt of any of the foregoing, wherein R⁶ is -OH. In some embodiments, R⁶ is -OH, R⁸ is C₁₋₆alkyl, and R⁹ is C₁₋₆alkyl. In some embodiments, R⁶ is -OH, R⁸ is methyl, and R⁹ is methyl. In some embodiments, R⁶ is -OH, R⁸ is H, and R⁹ is H. In some variations, the embodiments provided herein also apply to a compound of formula (A') or a stereoisomer or tautomer thereof, or a pharmaceutically acceptable salt of any of the foregoing, or any variation or embodiment thereof.

In some embodiments, provided herein is a compound of formula (A) or formula (II), or a stereoisomer or tautomer thereof, or a pharmaceutically acceptable salt of any of the foregoing, wherein one of R⁸ and R⁹ is taken together with R¹⁰, and the atoms to which they are attached, to form a C₃₋₈cycloalkyl. In some embodiments, one of R⁸ and R⁹ is taken together with R¹⁰, and the atoms to which they are attached, to form a C₃₋₆cycloalkyl. In some embodiments, one of R⁸ and R⁹ is taken together with R¹⁰, and the atoms to which they are attached, to form a C₃₋₄cycloalkyl. In some embodiments, one of R⁸ and R⁹ is taken together with R¹⁰, and the atoms to which they are attached, to form cyclobutyl. In some variations, the embodiments provided herein also apply to a compound of formula (A') or a stereoisomer or tautomer thereof, or a pharmaceutically acceptable salt of any of the foregoing, or any variation or embodiment thereof.

In some embodiments, provided herein is a compound of formula (A) or formula (II), or a stereoisomer or tautomer thereof, or a pharmaceutically acceptable salt of any of the foregoing, wherein one of R⁸ and R⁹ is taken together with R¹⁰, and the atoms to which they are attached, to form cyclobutyl, wherein the compound is of formula (II-E1): 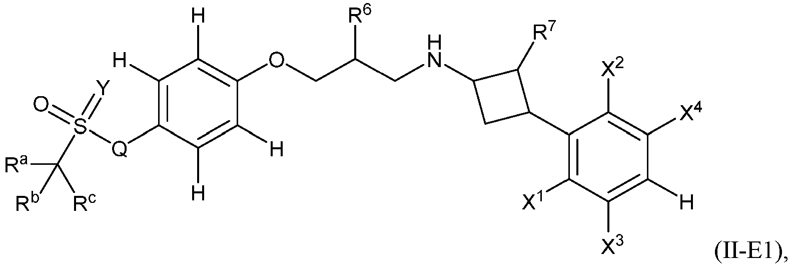 or a stereoisomer or tautomer thereof, or a pharmaceutically acceptable salt of any of the foregoing. In some variations, X¹, X², X³, X⁴, R⁶, R⁸, R⁹, R¹⁰, R^{a}, R^{b}, R^{c}, Q, and Y of formula (II-E1) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (II), such as a compound of formula (II-E1), or a stereoisomer or tautomer thereof, or a pharmaceutically acceptable salt of any of the foregoing, wherein R⁶ is -OH and R⁷ is H. In some variations, the embodiments provided herein also apply to a compound of formula (A') or a stereoisomer or tautomer thereof, or a pharmaceutically acceptable salt of any of the foregoing, or any variation or embodiment thereof.

In some embodiments, provided herein is a compound of formula (A), or a stereoisomer or tautomer thereof, or a pharmaceutically acceptable salt of any of the foregoing, wherein L is 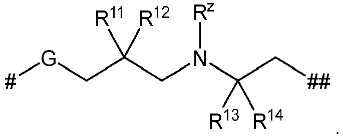 In some embodiments, provided herein is a compound of formula (A), or a stereoisomer or tautomer thereof, or a pharmaceutically acceptable salt of any of the foregoing, wherein L is 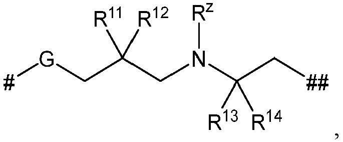 wherein the compound is of formula (III): 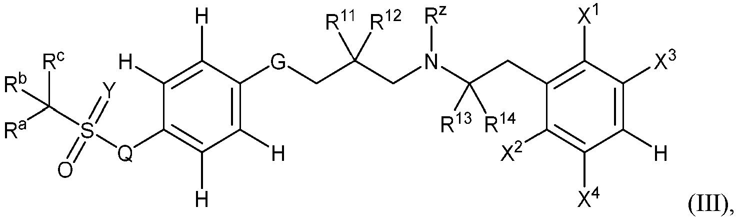 or a stereoisomer or tautomer thereof, or a pharmaceutically acceptable salt of any of the foregoing. In some variations, X¹, X², X³, X⁴, R¹¹, R¹², R¹³, R¹⁴, R^{a}, R^{b}, R^{c}, R^{z}, G, Q, and Y of formula (III) are as defined for a compound of formula (A') or a stereoisomer or tautomer thereof, or a pharmaceutically acceptable salt of any of the foregoing, or any variation or embodiment thereof.

In some embodiments, provided herein is a compound of formula (A) or formula (III), or a stereoisomer or tautomer thereof, or a pharmaceutically acceptable salt of any of the foregoing, wherein G is O, wherein the compound is of formula (III-A1): 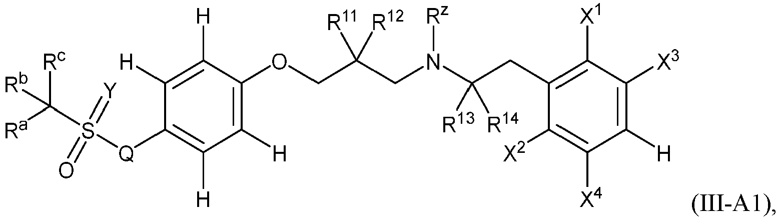 or a stereoisomer or tautomer thereof, or a pharmaceutically acceptable salt of any of the foregoing. In some variations, X¹, X², X³, X⁴, R¹¹, R¹², R¹³, R¹⁴, R^{a}, R^{b}, R^{c}, R^{z}, Q, and Y of formula (III-A1) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (III), or a stereoisomer or tautomer thereof, or a pharmaceutically acceptable salt of any of the foregoing, wherein G is NH, wherein the compound is of formula (III-A2): 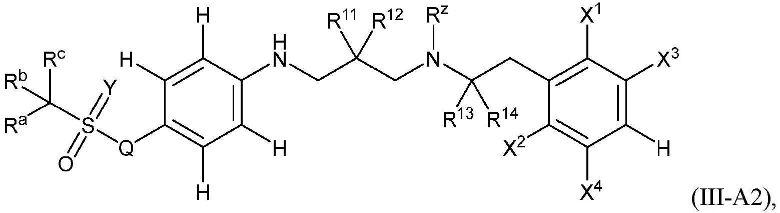 or a stereoisomer or tautomer thereof, or a pharmaceutically acceptable salt of any of the foregoing. In some embodiments, G is N(C₁₋₆alkyl). In some embodiments, G is N(CH₃). In some variations, X¹, X², X³, X⁴, R¹¹, R¹², R¹³, R¹⁴, R^{a}, R^{b}, R^{c}, R^{z}, Q, and Y of formula (III-A2) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (III), or a stereoisomer or tautomer thereof, or a pharmaceutically acceptable salt of any of the foregoing, wherein G is CH₂, wherein the compound is of formula (III-A3): 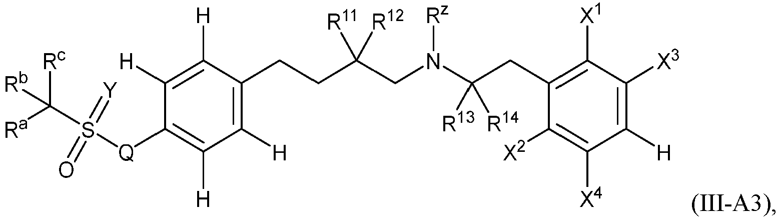 or a stereoisomer or tautomer thereof, or a pharmaceutically acceptable salt of any of the foregoing. In some embodiments, G is CH(C₁₋₆alkyl). In some embodiments, G is CH(CH₃). In some variations, X¹, X², X³, X⁴, R¹¹, R¹², R¹³, R¹⁴, R^{a}, R^{b}, R^{c}, R^{z}, Q, and Y of formula (III-A3) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (III), such as a compound of formula (III-A1), (III-A2), or (III-A3), or a stereoisomer or tautomer thereof, or a pharmaceutically acceptable salt of any of the foregoing, wherein R^{z} is H. In some embodiments, R^{z} is C₁₋₆alkyl, wherein the C₁₋₆alkyl is optionally substituted with one or more C₃₋₈cycloalkyl. In some embodiments, R^{z} is unsubstituted C₁₋₆alkyl. In some embodiments, R^{z} is unsubstituted C₁₋₃alkyl. In some embodiments, R^{z} is unsubstituted methyl or unsubstituted ethyl. In some embodiments, R^{z} is unsubstituted ethyl. In some embodiments, R^{z} is C₁₋₆alkyl, wherein the C₁₋₆alkyl is substituted with one or more C₃₋₈cycloalkyl. In some embodiments, R^{z} is C₁₋₆alkyl, wherein the C₁₋₆alkyl is substituted with one or more C₃₋₆cycloalkyl. In some embodiments, R^{z} is methyl, wherein the methyl is substituted with cyclopropyl. In some variations, the embodiments provided herein also apply to a compound of formula (A') or a stereoisomer or tautomer thereof, or a pharmaceutically acceptable salt of any of the foregoing, or any variation or embodiment thereof.

In some embodiments, provided herein is a compound of formula (A) or formula (III), such as a compound of formula (III-A1), (III-A2), or (III-A3), or a stereoisomer or tautomer thereof, or a pharmaceutically acceptable salt of any of the foregoing, wherein one of R¹¹ and R¹² is -OH and the other of R¹¹ and R¹² is H, halo, or C₁₋₆alkyl. In some embodiments, one of R¹¹ and R¹² is -OH and the other of R¹¹ and R¹² is H. In some embodiments, one of R¹¹ and R¹² is -OH and the other of R¹¹ and R¹² is C₁₋₆alkyl. In some embodiments, one of R¹¹ and R¹² is -OH and the other of R¹¹ and R¹² is methyl. In some variations, the embodiments provided herein also apply to a compound of formula (A') or a stereoisomer or tautomer thereof, or a pharmaceutically acceptable salt of any of the foregoing, or any variation or embodiment thereof.

In some embodiments, provided herein is a compound of formula (A) or formula (III), such as a compound of formula (III-A1), (III-A2), or (III-A3), or a stereoisomer or tautomer thereof, or a pharmaceutically acceptable salt of any of the foregoing, wherein R¹³ and R¹⁴ are both H. In some embodiments, R¹³ and R¹⁴ are both C₁₋₆alkyl. In some embodiments, R¹³ and R¹⁴ are both methyl. In some embodiments, one of R¹³ and R¹⁴ is H and the other of R¹³ and R¹⁴ is C₁₋₆alkyl or C₃₋₈cycloalkyl. In some embodiments, one of R¹³ and R¹⁴ is H and the other of R¹³ and R¹⁴ is C₁₋₆alkyl. In some embodiments, one of R¹³ and R¹⁴ is H and the other of R¹³ and R¹⁴ is methyl. In some embodiments, one of R¹³ and R¹⁴ is H and the other of R¹³ and R¹⁴ is C₃₋₈cycloalkyl. In some embodiments, one of R¹³ and R¹⁴ is H and the other of R¹³ and R¹⁴ is cyclopropyl. In some embodiments, R¹³ and R¹⁴ are taken, together with the atoms to which they are attached, to form a 3-8 membered heterocyclyl. In some embodiments, R¹³ and R¹⁴ are taken, together with the atoms to which they are attached, to form a 3-6 membered heterocyclyl. In some embodiments, R¹³ and R¹⁴ are taken, together with the atoms to which they are attached, to form a 5-8 membered heterocyclyl. In some embodiments, R¹³ and R¹⁴ are taken, together with the atoms to which they are attached, to form a 5-6 membered heterocyclyl. In some embodiments, R¹³ and R¹⁴ are taken, together with the atoms to which they are attached, to form tetrahydrofuranyl. In some variations, the embodiments provided herein also apply to a compound of formula (A') or a stereoisomer or tautomer thereof, or a pharmaceutically acceptable salt of any of the foregoing, or any variation or embodiment thereof.

In some embodiments, provided herein is a compound of formula (A), such as a compound of formula (I), (I-A1), (I-A2), (I-A3), (I-B1), (I-B2), (I-B3), (I-C1), (I-C2), (I-C3), (I-D1), (I-D2), (I-D3), (I-E1), (I-E2), (I-E3), (I-F1), (I-F2), (I-F3), (I-G1), (I-G2), (I-G3), (I-H1), (I-H2), (I-H3), (II), (II-A1), (II-A2), (II-A3), (II-B1), (II-C1), (II-C2), (II-C3), (II-D1), (II-D2), (II-D3), (II-E1), (III), (III-A1), (III-A2), or (III-A3), or a stereoisomer or tautomer thereof, or a pharmaceutically acceptable salt of any of the foregoing, wherein X¹, X², X³, and X⁴ are, independently of each other, H, halo, -CN, C₁₋₆alkyl, or C₁₋₆alkoxy, wherein the C₁₋₆alkyl or C₁₋₆alkoxy is optionally substituted with one or more halo, provided that at least one of X¹, X², X³, and X⁴ is halo, -CN, C₁₋₆alkyl, or C₁₋₆alkoxy, wherein the C₁₋₆alkyl or C₁₋₆alkoxy is optionally substituted with one or more halo. In some embodiments, three of X¹, X², X³, and X⁴ are H and one of X¹, X², X³, and X⁴ is halo, -CN, C₁₋₆alkyl, or C₁₋₆alkoxy, wherein the C₁₋₆alkyl or C₁₋₆alkoxy is optionally substituted with one or more halo. In some embodiments, two of X¹, X², X³, and X⁴ are H and two of X¹, X², X³, and X⁴ are independently halo, -CN, C₁₋₆alkyl, or C₁₋₆alkoxy, wherein the C₁₋₆alkyl or C₁₋₆alkoxy is optionally substituted with one or more halo.

In some embodiments, provided herein is a compound of formula (A'), such as a compound of formula (I), (I-A1), (I-A2), (I-A3), (I-B1), (I-B2), (I-B3), (I-C1), (I-C2), (I-C3), (I-D1), (I-D2), (I-D3), (I-E1), (I-E2), (I-E3), (I-F1), (I-F2), (I-F3), (I-G1), (I-G2), (I-G3), (I-H1), (I-H2), (I-H3), (II), (II-A1), (II-A2), (II-A3), (II-B1), (II-C1), (II-C2), (II-C3), (II-D1), (II-D2), (II-D3), (II-E1), (III), (III-A1), (III-A2), or (III-A3), or a stereoisomer or tautomer thereof, or a pharmaceutically acceptable salt of any of the foregoing, wherein X¹, X², X³, and X⁴ are, independently of each other, H, halo, -CN, C₁₋₆alkyl, C₁₋₆alkoxy, or SF⁵, wherein the C₁₋₆alkyl or C₁₋₆alkoxy is optionally substituted with one or more halo, provided that at least one of X¹, X², X³, and X⁴ is halo, -CN, C₁₋₆alkyl, C₁₋₆alkoxy, or SF⁵, wherein the C₁₋₆alkyl or C₁₋₆alkoxy is optionally substituted with one or more halo. In some embodiments, three of X¹, X², X³, and X⁴ are H and one of X¹, X², X³, and X⁴ is halo, -CN, C₁₋₆alkyl, C₁₋₆alkoxy, or SF⁵, wherein the C₁₋₆alkyl or C₁₋₆alkoxy is optionally substituted with one or more halo. In some embodiments, two of X¹, X², X³, and X⁴ are H and two of X¹, X², X³, and X⁴ are independently halo, -CN, C₁₋₆alkyl, C₁₋₆alkoxy, or SF⁵, wherein the C₁₋₆alkyl or C₁₋₆alkoxy is optionally substituted with one or more halo.

In some embodiments, provided herein is a compound of formula (A), such as a compound of formula (I), (I-A1), (I-A2), (I-A3), (I-B1), (I-B2), (I-B3), (I-C1), (I-C2), (I-C3), (I-D1), (I-D2), (I-D3), (I-E1), (I-E2), (I-E3), (I-F1), (I-F2), (I-F3), (I-G1), (I-G2), (I-G3), (I-H1), (I-H2), (I-H3), (II), (II-A1), (II-A2), (II-A3), (II-B1), (II-C1), (II-C2), (II-C3), (II-D1), (II-D2), (II-D3), (II-E1), (III), (III-A1), (III-A2), or (III-A3), or a stereoisomer or tautomer thereof, or a pharmaceutically acceptable salt of any of the foregoing, wherein X¹ and X² are each H, one of X³ and X⁴ is H, and the other of X³ and X⁴ is halo. In some embodiments, X¹ and X² are each H, one of X³ and X⁴ is H, and the other of X³ and X⁴ is chloro. In some variations, the embodiments provided herein also apply to a compound of formula (A') or a stereoisomer or tautomer thereof, or a pharmaceutically acceptable salt of any of the foregoing, or any variation or embodiment thereof.In some embodiments, provided herein is a compound of formula (A), such as a compound of formula (I), (I-A1), (I-A2), (I-A3), (I-B1), (I-B2), (I-B3), (I-C1), (I-C2), (I-C3), (I-D1), (I-D2), (I-D3), (I-E1), (I-E2), (I-E3), (I-F1), (I-F2), (I-F3), (I-G1), (I-G2), (I-G3), (I-H1), (I-H2), (I-H3), (II), (II-A1), (II-A2), (II-A3), (II-B1), (II-C1), (II-C2), (II-C3), (II-D1), (II-D2), (II-D3), (II-E1), (III), (III-A1), (III-A2), or (III-A3), or a stereoisomer or tautomer thereof, or a pharmaceutically acceptable salt of any of the foregoing, wherein one of X¹ and X² is H and the other of X¹ and X² is C₁₋₆alkyl, and X³ and X⁴ are each H. In some embodiments, one of X¹ and X² is H and the other of X¹ and X² is ethyl, and X³ and X⁴ are each H. In some embodiments, one of X¹ and X² is H and the other of X¹ and X² is C₁₋₆alkoxy, wherein the C₁₋₆alkoxy is optionally substituted with one or more halo, and X³ and X⁴ are each H. In some embodiments, one of X¹ and X² is H and the other of X¹ and X² is methoxy, and X³ and X⁴ are each H. In some embodiments, one of X¹ and X² is H and the other of X¹ and X² is -O-CHF₂, and X³ and X⁴ are each H. In some embodiments, one of X¹ and X² is H and the other of X¹ and X² is halo, and one of X³ and X⁴ is H and the other of X³ and X⁴ is halo. In some embodiments, one of X¹ and X² is H and the other of X¹ and X² is chloro, and one of X³ and X⁴ is H and the other of X³ and X⁴ is chloro. In some embodiments, X¹ and X² are each H, and X³ and X⁴ are each halo. In some embodiments, X¹ and X² are each H, and X³ and X⁴ are each chloro. In some embodiments, X¹ and X² are each H, and one of X³ and X⁴ is H and the other of X³ and X⁴ is - CN. In some embodiments, provided herein is a compound of formula (A), such as a compound of formula (I), (I-A1), (I-A2), (I-A3), (I-B1), (I-B2), (I-B3), (I-C1), (I-C2), (I-C3), (I-D1), (I-D2), (I-D3), (I-E1), (I-E2), (I-E3), (I-F1), (I-F2), (I-F3), (I-G1), (I-G2), (I-G3), (I-H1), (I-H2), (I-H3), (II), (II-A1), (II-A2), (II-A3), (II-B1), (II-C1), (II-C2), (II-C3), (II-D1), (II-D2), (II-D3), (II-E1), (III), (III-A1), (III-A2), or (III-A3), or a stereoisomer or tautomer thereof, or a pharmaceutically acceptable salt of any of the foregoing, wherein one of X¹ and X² is H and the other of X¹ and X² is C₁₋₆alkyl, and X³ and X⁴ are each H. In some embodiments, one of X¹ and X² is H and the other of X¹ and X² is ethyl, and X³ and X⁴ are each H. In some embodiments, one of X¹ and X² is H and the other of X¹ and X² is C₁₋₆alkoxy, wherein the C₁₋₆alkoxy is optionally substituted with one or more halo, and X³ and X⁴ are each H. In some embodiments, one of X¹ and X² is H and the other of X¹ and X² is methoxy, and X³ and X⁴ are each H. In some embodiments, one of X¹ and X² is H and the other of X¹ and X² is -O-CHF₂, and X³ and X⁴ are each H. In some embodiments, one of X¹ and X² is H and the other of X¹ and X² is -O-CHF₂, and one of X³ and X⁴ is H and the other of X³ and X⁴ is chloro. In some embodiments, one of X¹ and X² is H and the other of X¹ and X² is halo, and one of X³ and X⁴ is H and the other of X³ and X⁴ is halo. In some embodiments, one of X¹ and X² is H and the other of X¹ and X² is C₁₋₆alkyl, and one of X³ and X⁴ is H and the other of X³ and X⁴ is halo. In some embodiments, one of X¹ and X² is H and the other of X¹ and X² is methyl, and one of X³ and X⁴ is H and the other of X³ and X⁴ is halo. In some embodiments, one of X¹ and X² is H and the other of X¹ and X² is C₁₋₆alkoxy, one of X³ and X⁴ is H and the other of X³ and X⁴ is halo. In some embodiments, one of X¹ and X² is H and the other of X¹ and X² is methoxy, one of X³ and X⁴ is H and the other of X³ and X⁴ is halo. In some embodiments, one of X¹ and X² is H and the other of X¹ and X² is chloro, and one of X³ and X⁴ is H and the other of X³ and X⁴ is chloro. In some embodiments, X¹ and X² are each H, and X³ and X⁴ are each halo. In some embodiments, X¹ and X² are each H, and X³ and X⁴ are each chloro. In some embodiments, X¹ and X² are each H, one of X³ and X⁴ is chloro and the other of X³ and X⁴ is fluoro. In some embodiments, X¹ and X² are each H, and one of X³ and X⁴ is H and the other of X³ and X⁴ is -CN. In some embodiments, X¹ and X² are each H, and one of X³ and X⁴ is H and each of X³ and X⁴ is -CN. In some embodiments, X¹ and X² are each H, and one of X³ and X⁴ is C₁₋₆alkyl and the other of X³ and X⁴ is -CN. In some embodiments, X¹ and X² are each H, and one of X³ and X⁴ is halo and the other of X³ and X⁴ is - CN. In some embodiments, X¹ and X² are each H, and one of X³ and X⁴ is -CF₃ and the other of X³ and X⁴ is -CN. In some embodiments, one of X¹ and X² is H and the other of X¹ and X² is C₁₋₆alkyl, one X³ and X⁴ is halo and the other of X³ and X⁴ is -CN. In some embodiments, X¹ and X² are each H, one of X³ and X⁴ is H and the other of X³ and X⁴ is -SF₅.

In some embodiments, the phenyl ring bearing moieties X¹, X², X³, and X⁴ is selected from the group consisting of 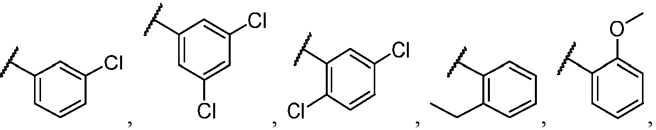 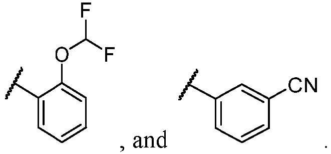 In some embodiments, the phenyl ring bearing moieties X¹, X², X³, and X⁴ is 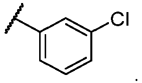 In some embodiments, the phenyl ring bearing moieties X¹, X², X³, and X⁴ is selected from the group consisting of 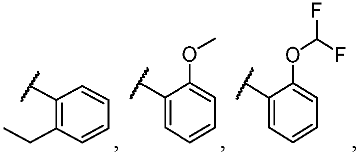 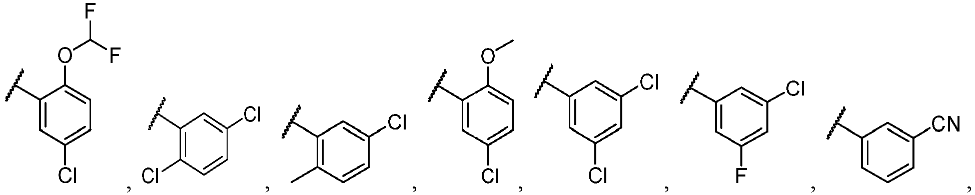 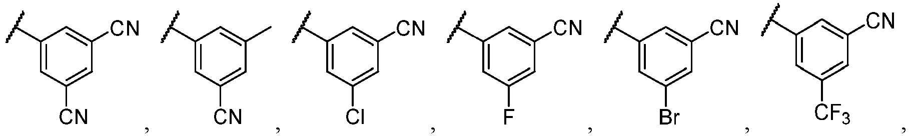 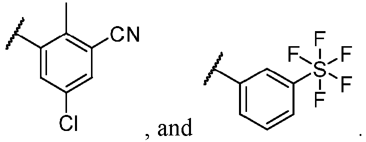 In some embodiments, the phenyl ring bearing moieties X¹, X², X³, and X⁴ is selected from the group consisting of 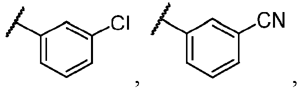 and 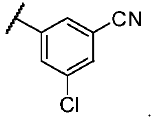 In some embodiments, the phenyl ring bearing moieties X¹, X², X³, and X⁴ is

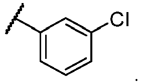

In some embodiments, provided herein is a compound of formula (A), such as a compound of formula (I), (I-A1), (I-A2), (I-A3), (I-B1), (I-B2), (I-B3), (I-C1), (I-C2), (I-C3), (I-D1), (I-D2), (I-D3), (I-E1), (I-E2), (I-E3), (I-F1), (I-F2), (I-F3), (I-G1), (I-G2), (I-G3), (I-H1), (I-H2), (I-H3), (II), (II-A1), (II-A2), (II-A3), (II-B1), (II-C1), (II-C2), (II-C3), (II-D1), (II-D2), (II-D3), (II-E1), (III), (III-A1), (III-A2), or (III-A3), or a stereoisomer or tautomer thereof, or a pharmaceutically acceptable salt of any of the foregoing, wherein Q is absent. In some embodiments, provided herein is a compound of formula (A), or any variation or embodiment thereof, or a stereoisomer or tautomer thereof, or a pharmaceutically acceptable salt of any of the foregoing, wherein the compound is of formula (B): 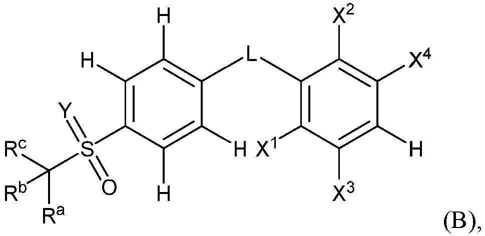 or a stereoisomer or tautomer thereof, or a pharmaceutically acceptable salt of any of the foregoing. In some variations, X¹, X², X³, X⁴, R^{a}, R^{b}, R^{c}, L and Y of formula (B) are as defined for formula (B) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (B), or a stereoisomer or tautomer thereof, or a pharmaceutically acceptable salt of any of the foregoing, wherein Y is O, wherein the compound is of formula (B-1): 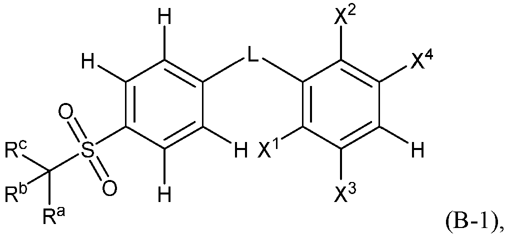 or a stereoisomer or tautomer thereof, or a pharmaceutically acceptable salt of any of the foregoing. In some variations, X¹, X², X³, X⁴, R^{a}, R^{b}, R^{c}, and L of formula (B1) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A), formula (B), or formula (B-1), or a stereoisomer or tautomer thereof, or a pharmaceutically acceptable salt of any of the foregoing, wherein R^{a}, R^{b}, and R^{c} are each independently H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl. In some embodiments, two of R^{a}, R^{b}, and R^{c} are independently H, and one of R^{a}, R^{b}, and R^{c} is C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl. In some embodiments, two of R^{a}, R^{b}, and R^{c} are independently H, and one of R^{a}, R^{b}, and R^{c} is ,

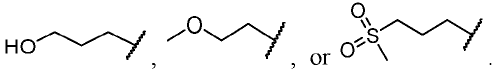

In some embodiments, provided herein is a compound of formula (A'), formula (B), or formula (B-1), or a stereoisomer or tautomer thereof, or a pharmaceutically acceptable salt of any of the foregoing, wherein R^{a}, R^{b}, and R^{c} are each independently H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl. In some embodiments, two of R^{a}, R^{b}, and R^{c} are independently H, and one of R^{a}, R^{b}, and R^{c} is C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl. In some embodiments, two of R^{a}, R^{b}, and R^{c} are independently H, and one of R^{a}, R^{b}, and R^{c} is

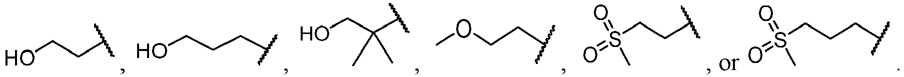

In some embodiments, provided herein is a compound of formula (A), formula (B), or formula (B-1), or a stereoisomer or tautomer thereof, or a pharmaceutically acceptable salt of any of the foregoing, wherein any two of R^{a}, R^{b}, and R^{c} are taken, together with the atoms to which they are attached, to form a C₃₋₆cycloalkyl or a 3-6 membered heterocyclyl, and the other of R^{a}, R^{b}, and R^{c} is H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl. In some embodiments, any two of R^{a}, R^{b}, and R^{c} are taken, together with the atoms to which they are attached, to form a 3-6 membered heterocyclyl, and the other of R^{a}, R^{b}, and R^{c} is H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl. In some embodiments, any two of R^{a}, R^{b}, and R^{c} are taken, together with the atoms to which they are attached, to form oxetanyl, and the other of R^{a}, R^{b}, and R^{c} is H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl. In some variations, the embodiments provided herein also apply to a compound of formula (A') or a stereoisomer or tautomer thereof, or a pharmaceutically acceptable salt of any of the foregoing, or any variation or embodiment thereof.

In some embodiments, provided herein is a compound of formula (A), formula (B), or formula (B-1), or a stereoisomer or tautomer thereof, or a pharmaceutically acceptable salt of any of the foregoing, wherein R^{a}, R^{b}, and R^{c} are each H, wherein the compound is of formula (B-2): 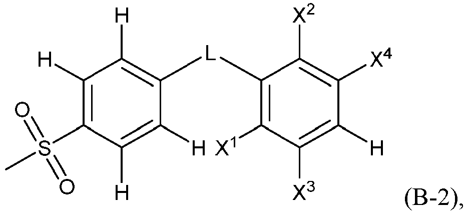 or a stereoisomer or tautomer thereof, or a pharmaceutically acceptable salt of any of the foregoing. In some variations, X¹, X², X³, X⁴, and L of formula (B-2) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A), formula (B), formula (B-1), or formula (B-2), or a stereoisomer or tautomer thereof, or a pharmaceutically acceptable salt of any of the foregoing, wherein one of X¹ and X² is H and the other of X¹ and X² is chloro, X³ is H, and X⁴ is H, wherein the compound is of formula (B-3): 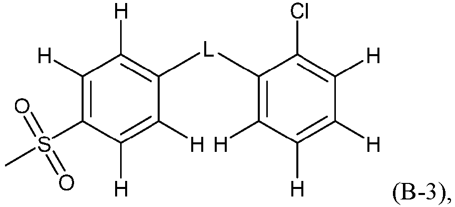 or a stereoisomer or tautomer thereof, or a pharmaceutically acceptable salt of any of the foregoing. In some variations, L of formula (B-3) is as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (B), or a stereoisomer or tautomer thereof, or a pharmaceutically acceptable salt of any of the foregoing, wherein Y is -N(C₁₋₆alkyl). In some embodiments, Y is -N(CH₃), wherein the compound is of formula (B-4): 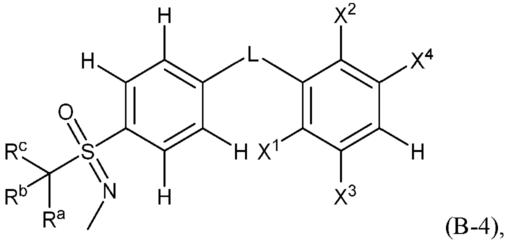 or a stereoisomer or tautomer thereof, or a pharmaceutically acceptable salt of any of the foregoing. In some variations, X¹, X², X³, X⁴, R^{a}, R^{b}, R^{c}, and L of formula (B-4) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A), formula (B), or formula (B-4), or a stereoisomer or tautomer thereof, or a pharmaceutically acceptable salt of any of the foregoing, wherein R^{a}, R^{b}, and R^{c} are each independently H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl. In some embodiments, two of R^{a}, R^{b}, and R^{c} are independently H, and one of R^{a}, R^{b}, and R^{c} is C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl. In some embodiments, two of R^{a}, R^{b}, and R^{c} are independently H, and one of R^{a}, R^{b}, and R^{c} is,

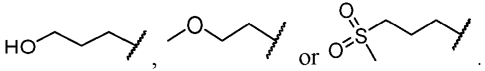

In some embodiments, provided herein is a compound of formula (A'), formula (B), or formula (B-4), or a stereoisomer or tautomer thereof, or a pharmaceutically acceptable salt of any of the foregoing, wherein R^{a}, R^{b}, and R^{c} are each independently H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl. In some embodiments, two of R^{a}, R^{b}, and R^{c} are independently H, and one of R^{a}, R^{b}, and R^{c} is C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl. In some embodiments, two of R^{a}, R^{b}, and R^{c} are independently H, and one of R^{a}, R^{b}, and R^{c} is,

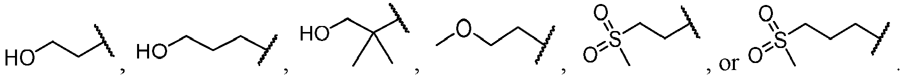

In some embodiments, provided herein is a compound of formula (A), formula (B), or formula (B-4), or a stereoisomer or tautomer thereof, or a pharmaceutically acceptable salt of any of the foregoing, wherein any two of R^{a}, R^{b}, and R^{c} are taken, together with the atoms to which they are attached, to form a C₃₋₆cycloalkyl or a 3-6 membered heterocyclyl, and the other of R^{a}, R^{b}, and R^{c} is H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl. In some embodiments, any two of R^{a}, R^{b}, and R^{c} are taken, together with the atoms to which they are attached, to form a 3-6 membered heterocyclyl, and the other of R^{a}, R^{b}, and R^{c} is H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl. In some embodiments, any two of R^{a}, R^{b}, and R^{c} are taken, together with the atoms to which they are attached, to form oxetanyl, and the other of R^{a}, R^{b}, and R^{c} is H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl.

In some embodiments, provided herein is a compound of formula (A'), formula (B), or formula (B-4), or a stereoisomer or tautomer thereof, or a pharmaceutically acceptable salt of any of the foregoing, wherein any two of R^{a}, R^{b}, and R^{c} are taken, together with the atoms to which they are attached, to form a C₃₋₆cycloalkyl or a 3-6 membered heterocyclyl, and the other of R^{a}, R^{b}, and R^{c} is H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl. In some embodiments, any two of R^{a}, R^{b}, and R^{c} are taken, together with the atoms to which they are attached, to form a 3-6 membered heterocyclyl, and the other of R^{a}, R^{b}, and R^{c} is H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl. In some embodiments, any two of R^{a}, R^{b}, and R^{c} are taken, together with the atoms to which they are attached, to form oxetanyl, and the other of R^{a}, R^{b}, and R^{c} is H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl. In some embodiments, any two of R^{a}, R^{b}, and R^{c} are taken, together with the atoms to which they are attached, to form azetidinyl, and the other of R^{a}, R^{b}, and R^{c} is H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl.

In some embodiments, provided herein is a compound of formula (A), formula (B), or formula (B-4), or a stereoisomer or tautomer thereof, or a pharmaceutically acceptable salt of any of the foregoing, wherein R^{a}, R^{b}, and R^{c} are each H, wherein the compound is of formula (B-5): 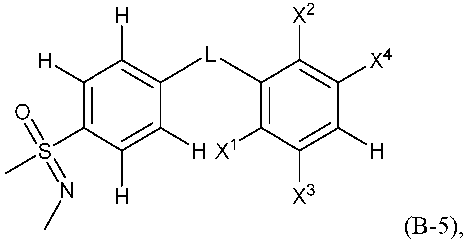 or a stereoisomer or tautomer thereof, or a pharmaceutically acceptable salt of any of the foregoing. In some variations, X¹, X², X³, X⁴, and L of formula (B-5) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A), formula (B), formula (B-4), or formula (B-5), or a stereoisomer or tautomer thereof, or a pharmaceutically acceptable salt of any of the foregoing, wherein one of X¹ and X² is H and the other of X¹ and X² is chloro, X³ is H, and X⁴ is H, wherein the compound is of formula (B-6): 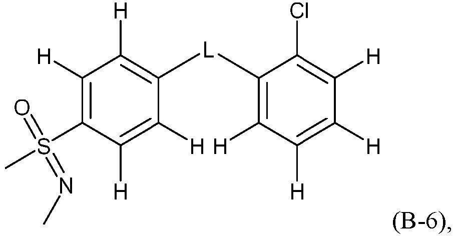 or a stereoisomer or tautomer thereof, or a pharmaceutically acceptable salt of any of the foregoing. In some variations, L of formula (B-6) is as defined for is a compound of formula (A'), or a stereoisomer or tautomer thereof, or a pharmaceutically acceptable salt of any of the foregoing, or any variation or embodiment thereof.

In some embodiments, provided herein is a compound of formula (A), such as a compound of formula (I), (I-A1), (I-A2), (I-A3), (I-B1), (I-B2), (I-B3), (I-C1), (I-C2), (I-C3), (I-D1), (I-D2), (I-D3), (I-E1), (I-E2), (I-E3), (I-F1), (I-F2), (I-F3), (I-G1), (I-G2), (I-G3), (I-H1), (I-H2), (I-H3), (II), (II-A1), (II-A2), (II-A3), (II-B1), (II-C1), (II-C2), (II-C3), (II-D1), (II-D2), (II-D3), (II-E1), (III), (III-A1), (III-A2), or (III-A3), or a stereoisomer or tautomer thereof, or a pharmaceutically acceptable salt of any of the foregoing, wherein Q is -N(C₁₋₆alkyl). In some embodiments, Q is -N(CH₃). In some embodiments, provided herein is a compound of formula (A), or any variation or embodiment thereof, or a stereoisomer or tautomer thereof, or a pharmaceutically acceptable salt of any of the foregoing, wherein the compound is of formula (C): 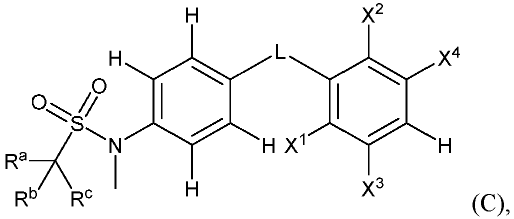 or a stereoisomer or tautomer thereof, or a pharmaceutically acceptable salt of any of the foregoing. In some variations, X¹, X², X³, X⁴, R^{a}, R^{b}, R^{c}, and L of formula (C) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A) or formula (C), or a stereoisomer or tautomer thereof, or a pharmaceutically acceptable salt of any of the foregoing, wherein R^{a}, R^{b}, and R^{c} are each independently H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl. In some embodiments, two of R^{a}, R^{b}, and R^{c} are independently H, and one of R^{a}, R^{b}, and R^{c} is C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl. In some embodiments, two of R^{a}, R^{b}, and R^{c} are independently H, and one of R^{a}, R^{b}, and R^{c} is 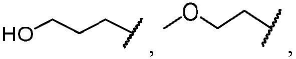 or

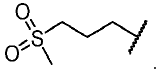

In some embodiments, provided herein is a compound of formula (A') or formula (C), or a stereoisomer or tautomer thereof, or a pharmaceutically acceptable salt of any of the foregoing, wherein R^{a}, R^{b}, and R^{c} are each independently H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl. In some embodiments, two of R^{a}, R^{b}, and R^{c} are independently H, and one of R^{a}, R^{b}, and R^{c} is C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl. In some embodiments, two of R^{a}, R^{b}, and R^{c} are independently H, and one of R^{a}, R^{b}, and R^{c} is

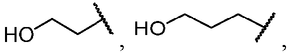

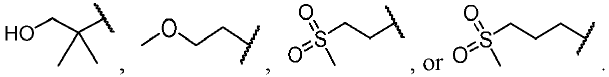

In some embodiments, provided herein is a compound of formula (A) or formula (C), or a stereoisomer or tautomer thereof, or a pharmaceutically acceptable salt of any of the foregoing, wherein any two of R^{a}, R^{b}, and R^{c} are taken, together with the atoms to which they are attached, to form a C₃₋₆cycloalkyl or a 3-6 membered heterocyclyl, and the other of R^{a}, R^{b}, and R^{c} is H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl. In some embodiments, any two of R^{a}, R^{b}, and R^{c} are taken, together with the atoms to which they are attached, to form a 3-6 membered heterocyclyl, and the other of R^{a}, R^{b}, and R^{c} is H or C₁₋₆alkyl, wherein the C₁₋₆alkyl of R^{a}, R^{b}, or R^{c} is independently optionally substituted with one or more -OH, C₁₋₆alkoxy, or - S(O)₂-C₁₋₆alkyl. In some embodiments, any two of R^{a}, R^{b}, and R^{c} are taken, together with the atoms to which they are attached, to form oxetanyl, and the other of R^{a}, R^{b}, and R^{c} is H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl. In some variations, the embodiments provided herein also apply to a compound of formula (A') or a stereoisomer or tautomer thereof, or a pharmaceutically acceptable salt of any of the foregoing, or any variation or embodiment thereof.

In some embodiments, provided herein is a compound of formula (A) or formula (C), or a stereoisomer or tautomer thereof, or a pharmaceutically acceptable salt of any of the foregoing, wherein R^{a}, R^{b}, and R^{c} are each H, wherein the compound is of formula (C-1): 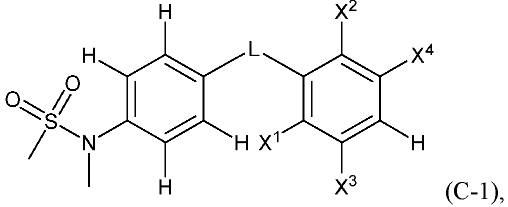 or a stereoisomer or tautomer thereof, or a pharmaceutically acceptable salt of any of the foregoing. In some variations, X¹, X², X³, X⁴, and L of formula (C-1) are as defined for a compound of formula (A'), or a stereoisomer or tautomer thereof, or a pharmaceutically acceptable salt of any of the foregoing, or any variation or embodiment thereof.

In some embodiments, provided herein is a compound of formula (A), formula (C), or formula (C-1), or a stereoisomer or tautomer thereof, or a pharmaceutically acceptable salt of any of the foregoing, wherein one of X¹ and X² is H and the other of X¹ and X² is chloro, X³ is H, and X⁴ is H, wherein the compound is of formula (C-2): 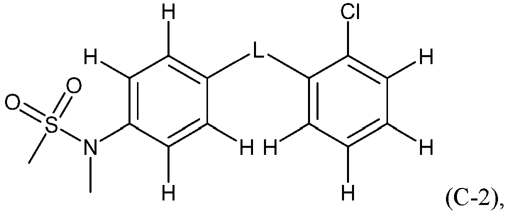 or a stereoisomer or tautomer thereof, or a pharmaceutically acceptable salt of any of the foregoing. In some variations, L of formula (C-2) is as defined for a compound of formula (A'), or a stereoisomer or tautomer thereof, or a pharmaceutically acceptable salt of any of the foregoing, or any variation or embodiment thereof.

In some embodiments, provided herein is a compound of formula (A), such as a compound of formula (B), (B-1), (B-2), (B-3), (B-4), (B-5), (B-6), (C), (C-1), or (C-2), or a stereoisomer or tautomer thereof, or a pharmaceutically acceptable salt of any of the foregoing, wherein L is 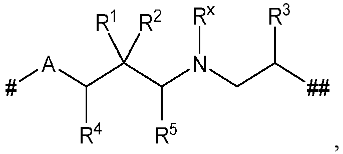 or any embodiment or variation thereof, as described elsewhere herein, including, for example, as in a compound of formula (I), (I-A1), (I-A2), (I-A3), (I-B1), (I-B2), (I-B3), (I-C1), (I-C2), (I-C3), (I-D1), (I-D2), (I-D3), (I-E1), (I-E2), (I-E3), (I-F1), (I-F2), (I-F3), (I-G1), (I-G2), (I-G3), (I-H1), (I-H2), (I-H3), (II), (II-A1), (II-A2), (II-A3), (II-B1), (II-C1), (II-C2), (II-C3), (II-D1), (II-D2), (II-D3), (II-E1), (III), (III-A1), (III-A2), or (III-A3), or a stereoisomer or tautomer thereof, or a pharmaceutically acceptable salt of any of the foregoing.

In some embodiments, provided herein is a compound of formula (A'), such as a compound of formula (B), (B-1), (B-2), (B-3), (B-4), (B-5), (B-6), (C), (C-1), or (C-2), or a stereoisomer or tautomer thereof, or a pharmaceutically acceptable salt of any of the foregoing, wherein L is 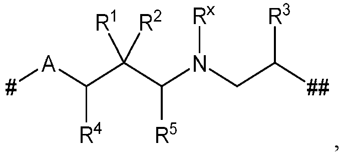 or any embodiment or variation thereof, as described elsewhere herein, including, for example, as in a compound of formula (I), (I-A1), (I-A2), (I-A3), (I-B1), (I-B2), (I-B3), (I-C1), (I-C2), (I-C3), (I-D1), (I-D2), (I-D3), (I-E1), (I-E2), (I-E3), (I-F1), (I-F2), (I-F3), (I-G1), (I-G2), (I-G3), (I-H1), (I-H2), (I-H3), (II), (II-A1), (II-A2), (II-A3), (II-B1), (II-C1), (II-C2), (II-C3), (II-D1), (II-D2), (II-D3), (II-E1), (III), (III-A1), (III-A2), or (III-A3), or a stereoisomer or tautomer thereof, or a pharmaceutically acceptable salt of any of the foregoing.

In some embodiments, provided herein is a compound of formula (A), such as a compound of formula (B), (B-1), (B-2), (B-3), (B-4), (B-5), (B-6), (C), (C-1), or (C-2), or a stereoisomer or tatomer thereof, or a pharmaceutically acceptable salt of any of the foregoing, wherein L is 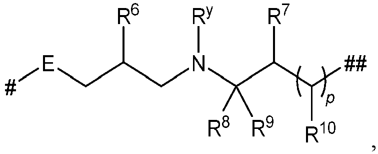 or any embodiment or variation thereof, as described elsewhere herein, including, for example, as in a compound of formula (I), (I-A1), (I-A2), (I-A3), (I-B1), (I-B2), (I-B3), (I-C1), (I-C2), (I-C3), (I-D1), (I-D2), (I-D3), (I-E1), (I-E2), (I-E3), (I-F1), (I-F2), (I-F3), (I-G1), (I-G2), (I-G3), (I-H1), (I-H2), (I-H3), (II), (II-A1), (II-A2), (II-A3), (II-B1), (II-C1), (II-C2), (II-C3), (II-D1), (II-D2), (II-D3), (II-E1), (III), (III-A1), (III-A2), or (III-A3), or a stereoisomer or tautomer thereof, or a pharmaceutically acceptable salt of any of the foregoing.

In some embodiments, provided herein is a compound of formula (A'), such as a compound of formula (B), (B-1), (B-2), (B-3), (B-4), (B-5), (B-6), (C), (C-1), or (C-2), or a stereoisomer or tatomer thereof, or a pharmaceutically acceptable salt of any of the foregoing, wherein L is 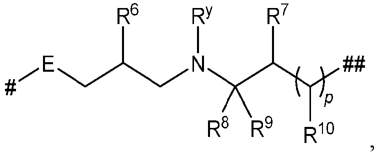 or any embodiment or variation thereof, as described elsewhere herein, including, for example, as in a compound of formula (I), (I-A1), (I-A2), (I-A3), (I-B1), (I-B2), (I-B3), (I-C1), (I-C2), (I-C3), (I-D1), (I-D2), (I-D3), (I-E1), (I-E2), (I-E3), (I-F1), (I-F2), (I-F3), (I-G1), (I-G2), (I-G3), (I-H1), (I-H2), (I-H3), (II), (II-A1), (II-A2), (II-A3), (II-B1), (II-C1), (II-C2), (II-C3), (II-D1), (II-D2), (II-D3), (II-E1), (III), (III-A1), (III-A2), or (III-A3), or a stereoisomer or tautomer thereof, or a pharmaceutically acceptable salt of any of the foregoing.

In some embodiments, provided herein is a compound of formula (A), such as a compound of formula (B), (B-1), (B-2), (B-3), (B-4), (B-5), (B-6), (C), (C-1), or (C-2), or a stereoisomer or tautomer thereof, or a pharmaceutically acceptable salt of any of the foregoing, wherein L is 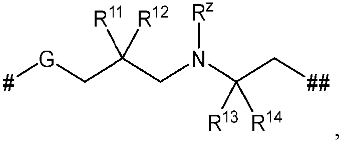 or any embodiment or variation thereof, as described elsewhere herein, including, for example, as in a compound of formula (I), (I-A1), (I-A2), (I-A3), (I-B1), (I-B2), (I-B3), (I-C1), (I-C2), (I-C3), (I-D1), (I-D2), (I-D3), (I-E1), (I-E2), (I-E3), (I-F1), (I-F2), (I-F3), (I-G1), (I-G2), (I-G3), (I-H1), (I-H2), (I-H3), (II), (II-A1), (II-A2), (II-A3), (II-B1), (II-C1), (II-C2), (II-C3), (II-D1), (II-D2), (II-D3), (II-E1), (III), (III-A1), (III-A2), or (III-A3), or a stereoisomer or tautomer thereof, or a pharmaceutically acceptable salt of any of the foregoing.

In some embodiments, provided herein is a compound of formula (A'), such as a compound of formula (B), (B-1), (B-2), (B-3), (B-4), (B-5), (B-6), (C), (C-1), or (C-2), or a stereoisomer or tautomer thereof, or a pharmaceutically acceptable salt of any of the foregoing, wherein L is 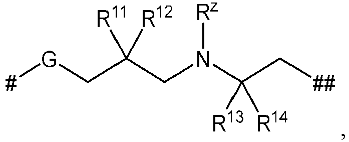 or any embodiment or variation thereof, as described elsewhere herein, including, for example, as in a compound of formula (I), (I-A1), (I-A2), (I-A3), (I-B1), (I-B2), (I-B3), (I-C1), (I-C2), (I-C3), (I-D1), (I-D2), (I-D3), (I-E1), (I-E2), (I-E3), (I-F1), (I-F2), (I-F3), (I-G1), (I-G2), (I-G3), (I-H1), (I-H2), (I-H3), (II), (II-A1), (II-A2), (II-A3), (II-B1), (II-C1), (II-C2), (II-C3), (II-D1), (II-D2), (II-D3), (II-E1), (III), (III-A1), (III-A2), or (III-A3), or a stereoisomer or tautomer thereof, or a pharmaceutically acceptable salt of any of the foregoing.

In some embodiments, provided herein is a compound of formula (A), or formula (A'), or a pharmaceutically acceptable salt of any of the foregoing, wherein L is selected from the group consisting of, 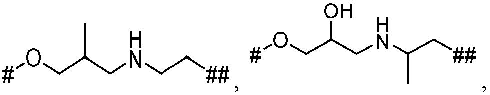 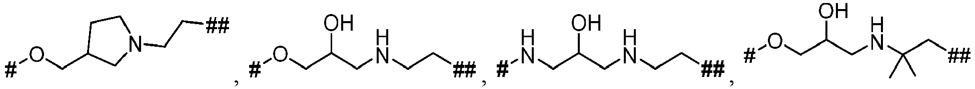 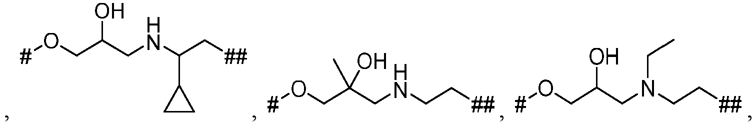 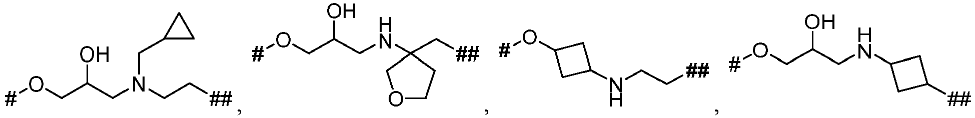 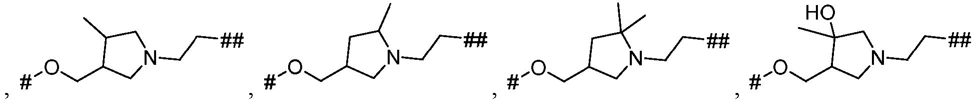 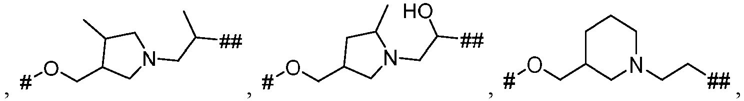 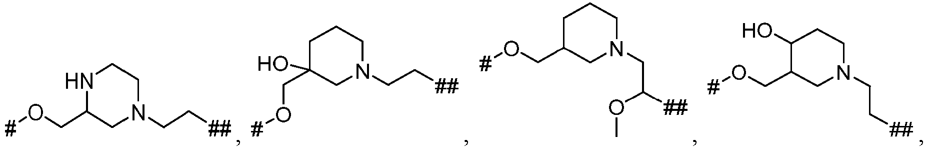 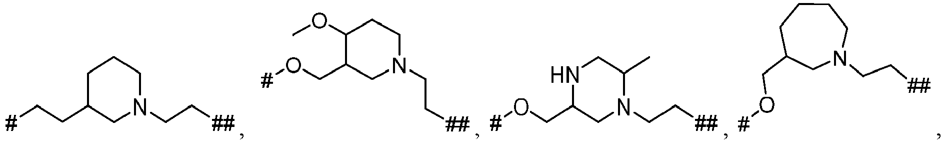 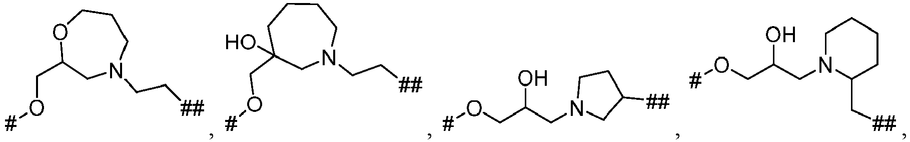 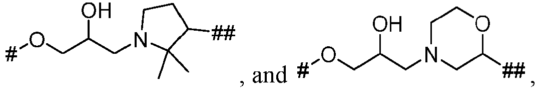 wherein # denotes the point of attachment to the phenyl ring bearing moiety Q, and ## denotes the point of attachment to the phenyl ring bearing moieties X¹-X⁴.

In some embodiments, provided herein is a compound of formula (A), or formula (A'), or a pharmaceutically acceptable salt of any of the foregoing, wherein L and the phenyl ring bearing moieties X¹-X⁴ together form a structure selected from the group consisting of

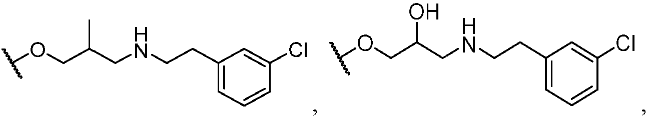

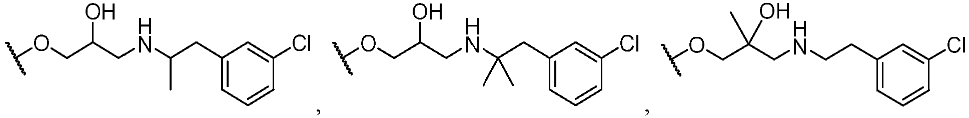

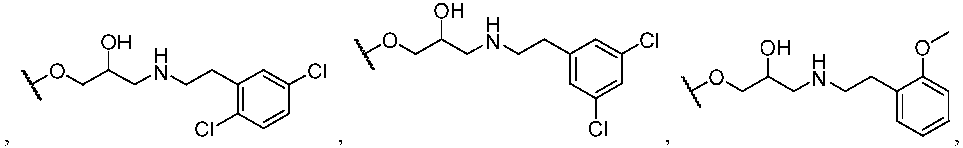

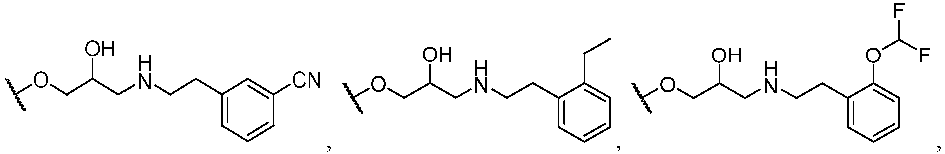

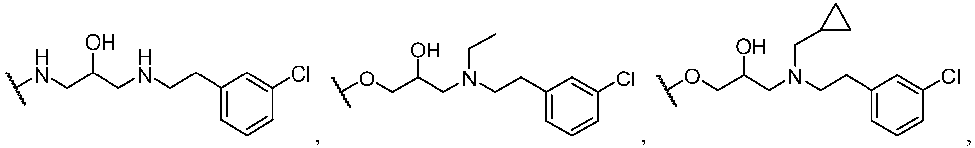

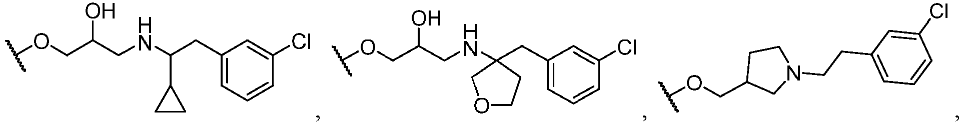

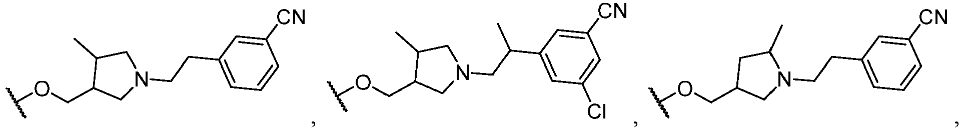

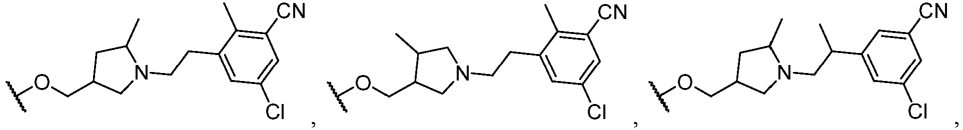

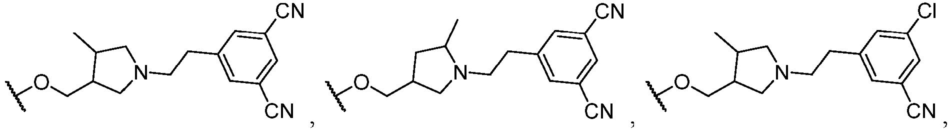

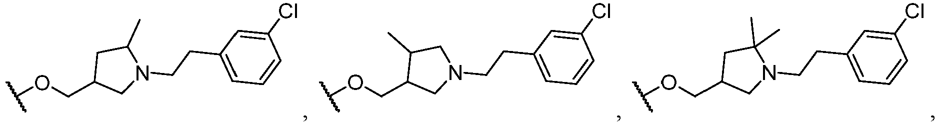

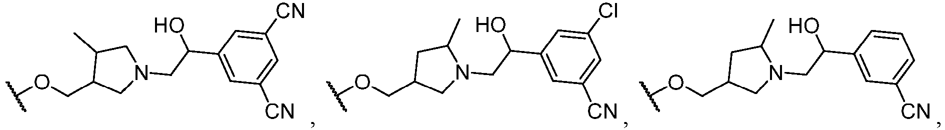

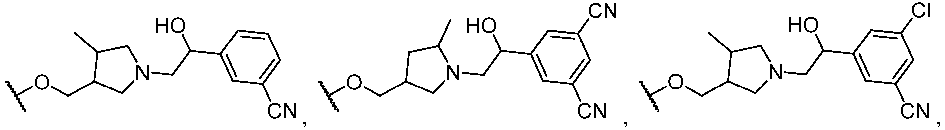

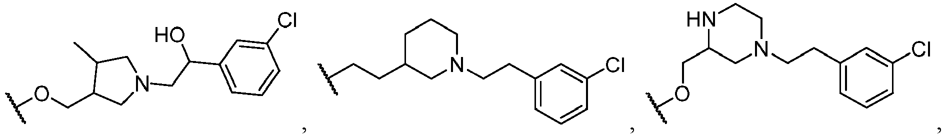

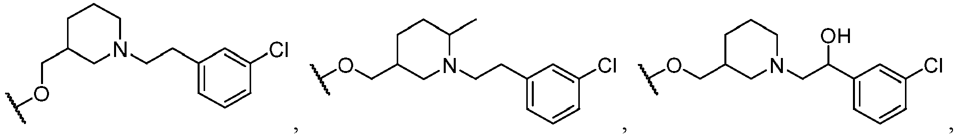

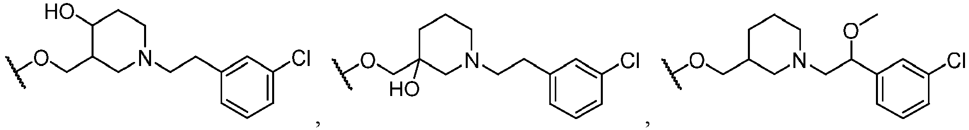

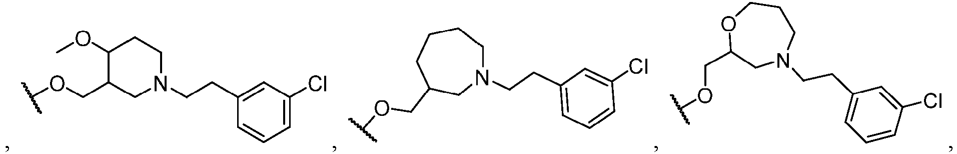

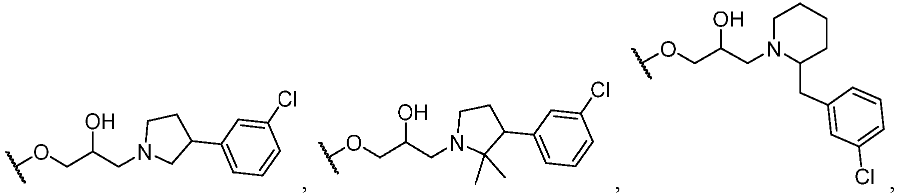

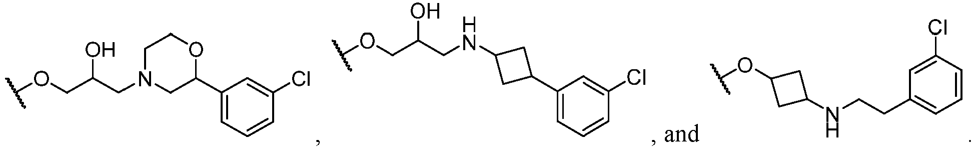

In some embodiments, provided herein is a compound of formula (A), or formula (A'), or a pharmaceutically acceptable salt of any of the foregoing, wherein the phenyl ring bearing Q is selected from the group consisting of 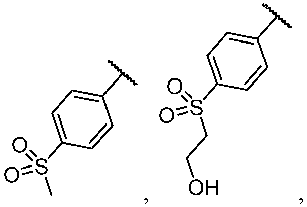 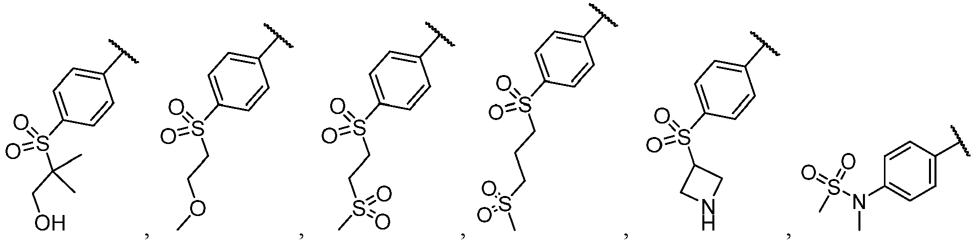 , and

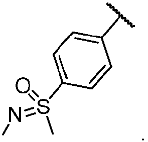

In some embodiments, provided herein is a compound of formula (A'), or a pharmaceutically acceptable salt of any of the foregoing, wherein L and the phenyl ring bearing moieties Q together form a structure selected from the group consisting of

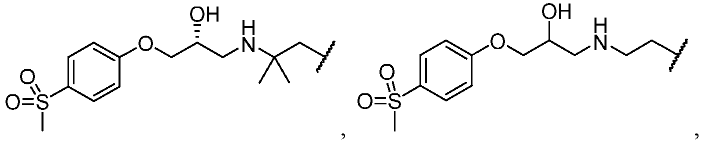

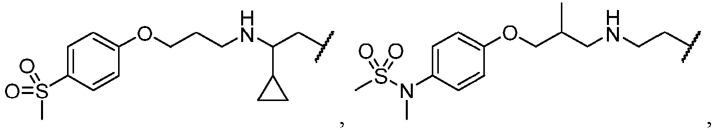

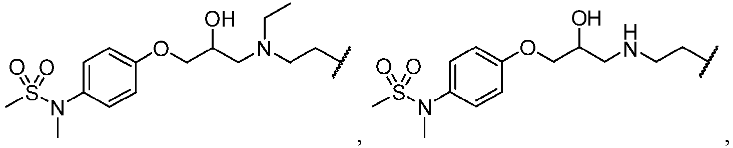

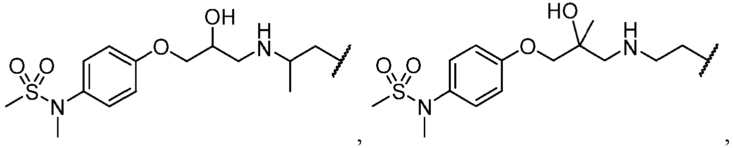

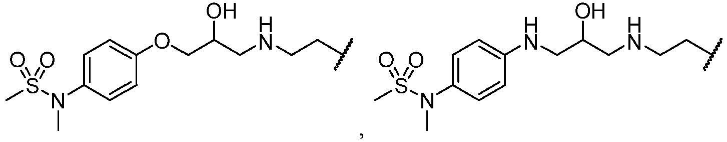

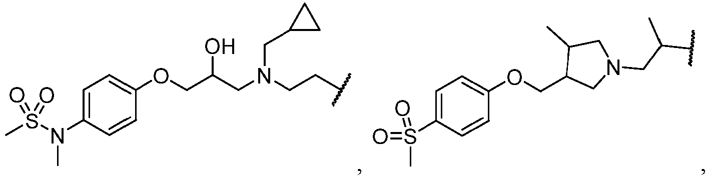

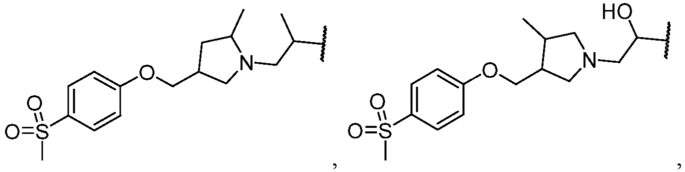

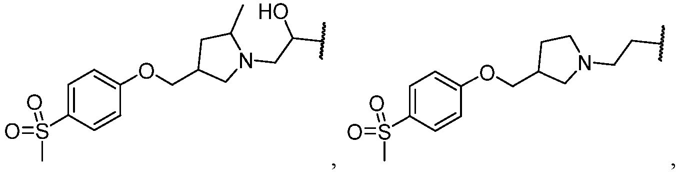

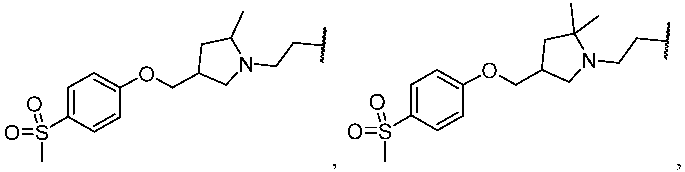

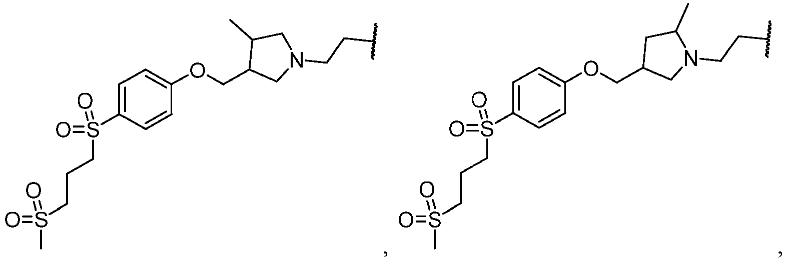

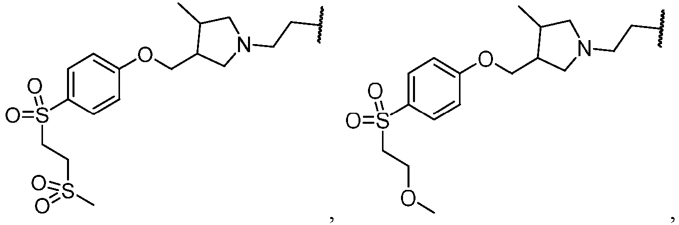

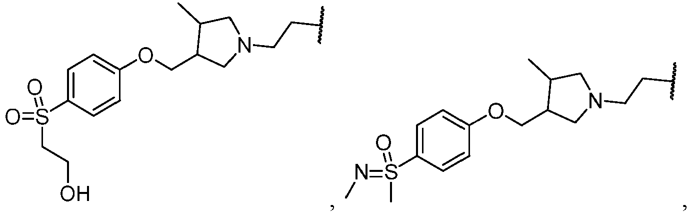

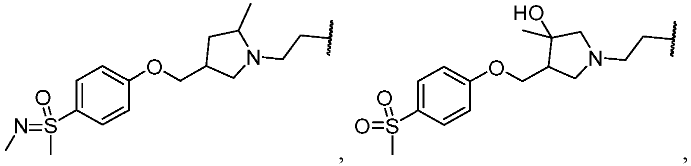

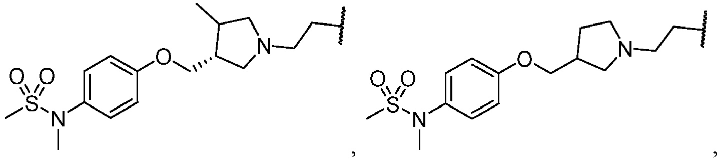

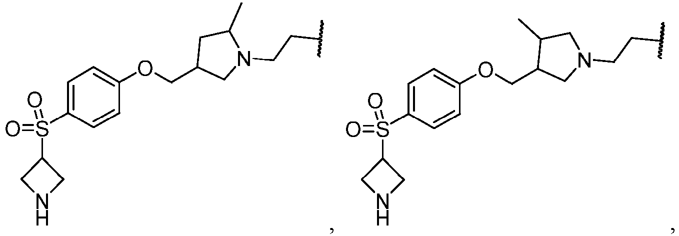

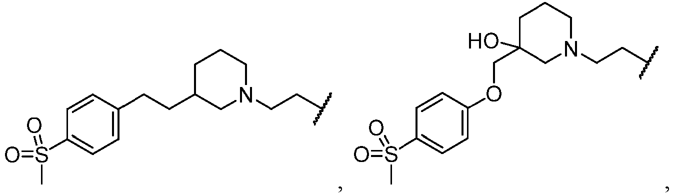

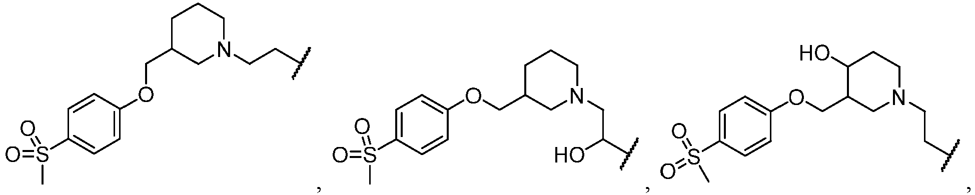

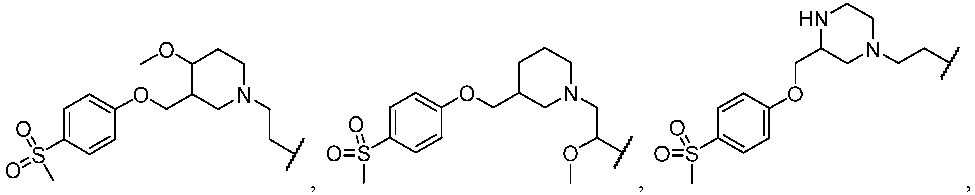

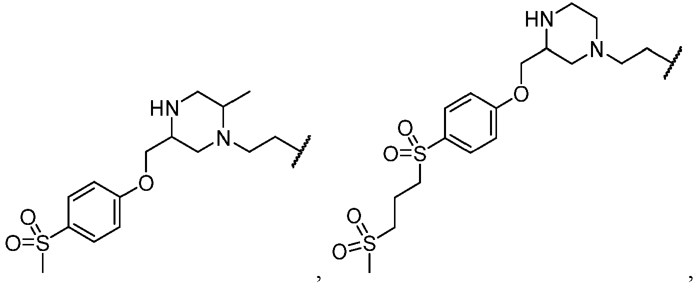

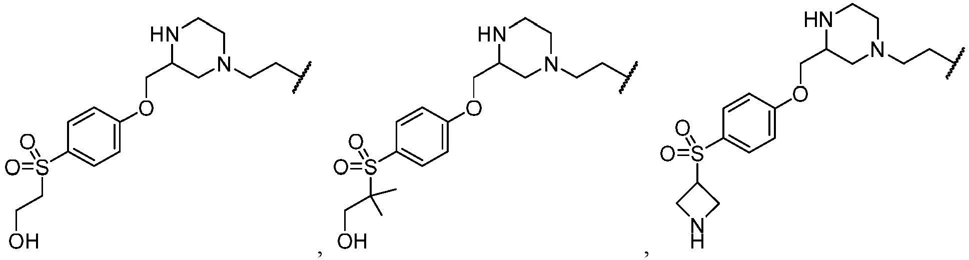

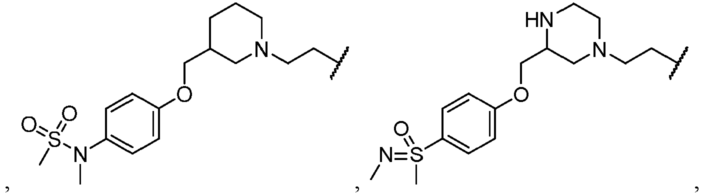

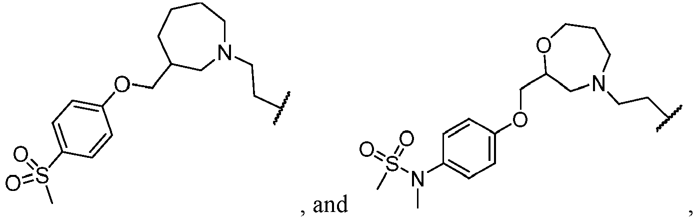

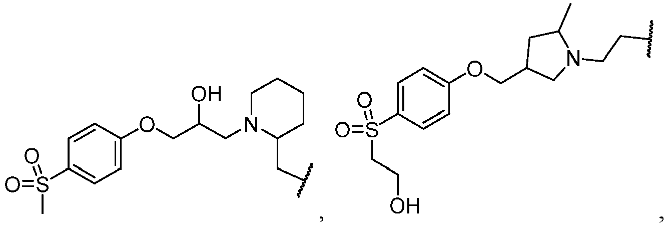

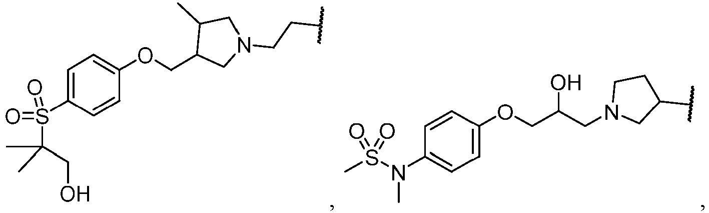

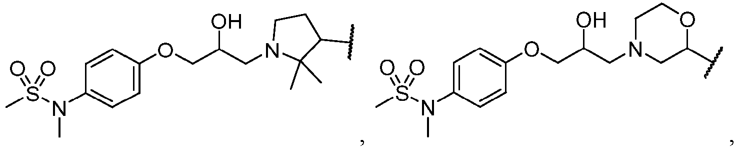

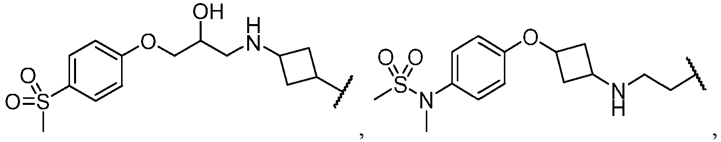

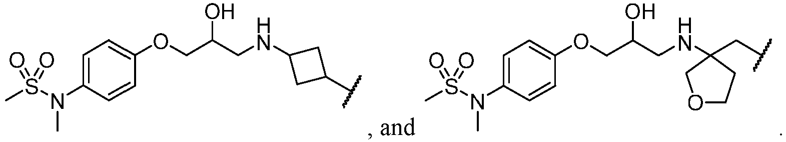

It is to be understood that any variation or embodiment of X¹, X², X³, X⁴, R¹, R², R³, R⁴, R⁵, R⁶, R⁷, R⁸, R⁹, R¹⁰, R¹¹, R¹², R¹³, R¹⁴, R^{a}, R^{b}, R^{c}, R^{g}, R^{x}, R^{y}, R^{z}, *n, p,* A, E, G, L, Q, and Y provided herein can be combined with every other variation or embodiment of X¹, X², X³, X⁴, R¹, R², R³, R⁴, R⁵, R⁶, R⁷, R⁸, R⁹, R¹⁰, R¹¹, R¹², R¹³, R¹⁴, R^{a}, R^{b}, R^{c}, R^{g}, R^{x}, R^{y}, R^{z}, *n, p,* A, E, G, L, Q, and Y, the same as if each and every combination had been individually and specifically described.

In some embodiments, provided herein is a compound of formula (A'), formula (A), or any variation of embodiment thereof, or a stereoisomer or tautomer thereof, or a pharmaceutically acceptable salt of any of the foregoing, wherein the compound is a compound of Table 1, or a stereoisomer or tautomer thereof, or a pharmaceutically acceptable salt of any of the foregoing.

**Table 1**

| Compound No. | Structure | Chemical name |
|---|---|---|
| 1 | 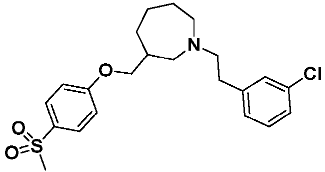 | 1-(3-chlorophenethyl)-3-((4-(methylsulfonyl)phenoxy)methyl)azepane |
| 2 | 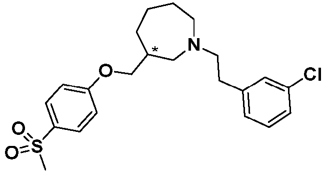 | (*R*) or (*S*)-1-(3-chlorophenethyl)-3 -((4-(methylsulfonyl)phenoxy)methyl)azepane |
| 3 | 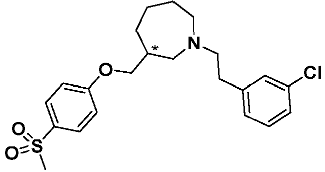 | (*S*) or (*R*)-1-(3-chlorophenethyl)-3 -((4-(methylsulfonyl)phenoxy)methyl)azepane |
| 4 | 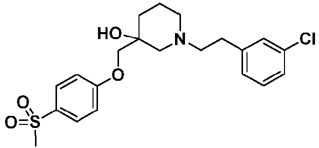 | 1-(3-chlorophenethyl)-3-((4-(methylsulfonyl)phenoxy)methyl)piperidin-3-ol |
| 5 | 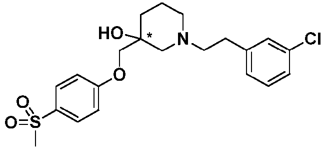 | (*R*) or (*S*)-1-(3-chlorophenethyl)-3 -((4-(methylsulfonyl)phenoxy)methyl)piperidin-3-ol |
| 6 | 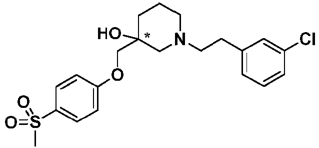 | (*S*) or (*R*) -1-(3-chlorophenethyl)-3 -((4-(methylsulfonyl)phenoxy)methyl)piperidin-3-ol |
| 7 | 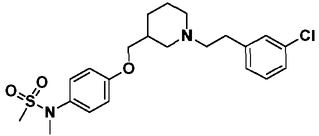 | *N*-(4-((1-(3-chlorophenethyl)piperidin-3-yl)methoxy)phenyl)-*N-*methylmethanesulfonamide |
| 8 | 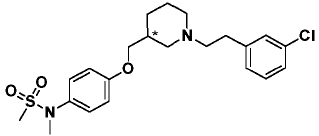 | (*S*) or (*R*)*-N*-(4-((1-(3-chlorophenethyl)piperidin-3-yl)methoxy)phenyl)-*N-*methylmethanesulfonamide |
| 9 | 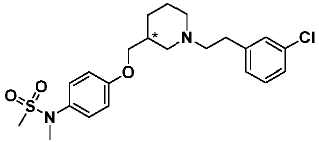 | (*R*) or (*S*)-*N*-(4-((1-(3-chlorophenethyl)piperidin-3-yl)methoxy)phenyl)-*N-*methylmethanesulfonamide |
| 10 | 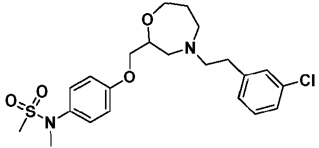 | *N*-(4-((4-(3-chlorophenethyl)-1,4-oxazepan-2-yl)methoxy)phenyl)-*N-*methylmethanesulfonamide |
| 11 | 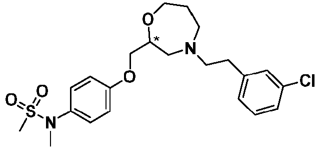 | (*R*) or (*S*)-*N-*(4-((4-(3-chlorophenethyl)-1,4-oxazepan-2-yl)methoxy)phenyl)-*N-*methylmethanesulfonamide |
| 12 | 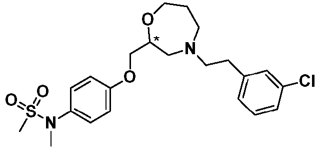 | (*S*) or (*R*)-*N-*(4-((4-(3-chlorophenethyl)-1,4-oxazepan-2-yl)methoxy)phenyl)-*N-*methylmethanesulfonamide |
| 13 | 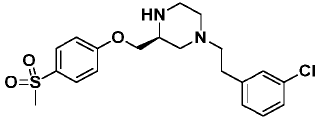 | (*S*)-1-(3-chlorophenethyl)-3-((4-(methylsulfonyl)phenoxy)methyl)piperazine |
| 14 | 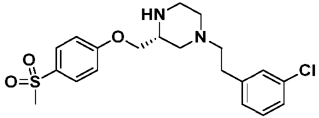 | (*R*)-1-(3-chlorophenethyl)-3-((4-(methylsulfonyl)phenoxy)methyl)piperazine |
| 15 | 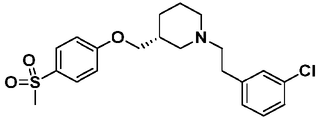 | (*R*)-1-(3-chlorophenethyl)-3-((4-(methylsulfonyl)phenoxy)methyl)piperidine |
| 16 | 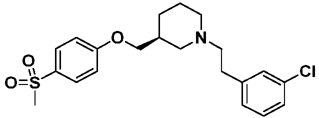 | (*S*)-1-(3-chlorophenethyl)-3-((4-(methylsulfonyl)phenoxy)methyl)piperidine |
| 17 | 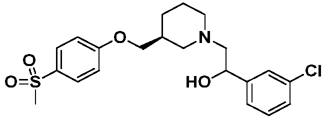 | 1-(3-chlorophenyl)-2-((*S*)-3-((4-(methylsulfonyl)phenoxy)methyl)piperidin-1-yl)ethan-1-ol |
| 18 | 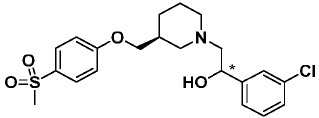 | (*R*) or (*S*)-1-(3-chlorophenyl)-2-((*S*)-3-((4-(methylsulfonyl)phenoxy) methyl)piperidin-1-yl)ethan-1-ol |
| 19 | 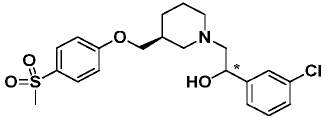 | (*S*) or (*R*)-1-(3-chlorophenyl)-2-((*S*)-3-((4-(methylsulfonyl)phenoxy) methyl)piperidin-1-yl)ethan-1-ol |
| 20 | 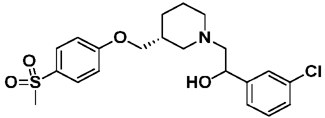 | 1-(3-chlorophenyl)-2-((*R*)-3-((4-(methylsulfonyl)phenoxy) methyl)piperidin-1-yl)ethanol |
| 21 | 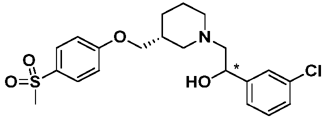 | (*R*) or (*S*)-1-(3-chlorophenyl)-2-((R)-3-((4-(methylsulfonyl)phenoxy) methyl)piperidin-1-yl)ethanol |
| 22 | 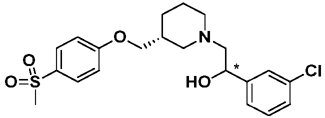 | (*S*) or (*R*)-1-(3-chlorophenyl)-2-((*R*)-3-((4-(methylsulfonyl)phenoxy) methyl)piperidin-1-yl)ethanol |
| 23 | 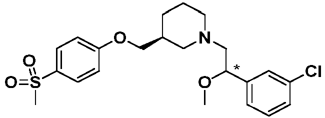 | (*S*)-1-((*R*) or (*S*)-2-(3-chlorophenyl)-2-methoxyethyl)-3-((4-(methylsulfonyl)phenoxy) methyl)piperidine |
| 24 | 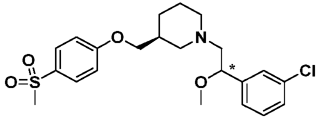 | (*S*)-1-((*S*) or (*R*)-2-(3-chlorophenyl)-2-methoxyethyl)-3-((4-(methylsulfonyl)phenoxy) methyl)piperidine |
| 25 | 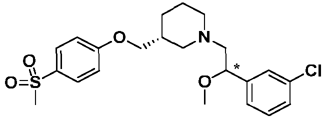 | (*R*)*-*1*-*((*R*) or (*S*)-2-(3-chlorophenyl)-2-methoxyethyl)-3-((4-(methylsulfonyl)phenoxy) methyl)piperidine |
| 26 | 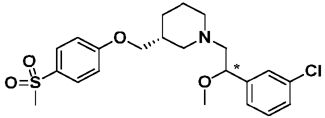 | (*R*)-1-((*S*) or (*R*)-2-(3-chlorophenyl)-2-methoxyethyl)-3-((4-(methylsulfonyl)phenoxy) methyl)piperidine |
| 27 | 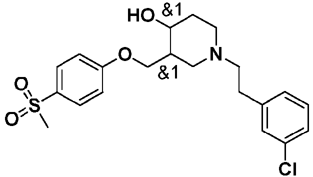 | *rac-trans or cis-*1-(3-chlorophenethyl)-3-((4-(methylsulfonyl)phenoxy) methyl)piperidin-4-ol |
| 28 | 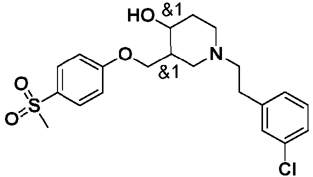 | *rac-cis or trans*-1-(3-chlorophenethyl)-3-((4-(methylsulfonyl)phenoxy) methyl)piperidin-4-ol |
| 29 | 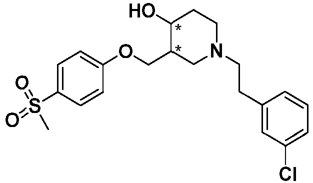 | (3*S*,4*R*) or (3*R*,4*R*) or (3*S*,4*S*) or (3*R*,4*S*)-1-(3-chlorophenethyl)-3-((4-(methylsulfonyl)phenoxy) methyl)piperidin-4-ol |
| 30 | 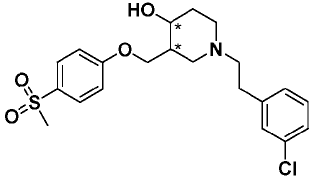 | (3*R*,4*R*) or (3*S*,4*S*) or (3*R*,4*S*) or (3*S*,4*R*)-1-(3-chlorophenethyl)-3-((4-(methylsulfonyl)phenoxy) methyl)piperidin-4-ol |
| | | |
| 31 | 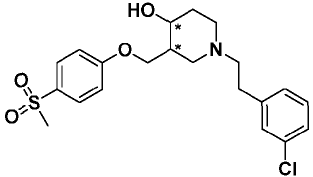 | (3*R*,4*R*) or (3*S*,4*S*) or (3*R*,4*S*) or (3*S*,4*R*)-1-(3-chlorophenethyl)-3-((4-(methylsulfonyl)phenoxy) methyl)piperidin-4-ol |
| 32 | 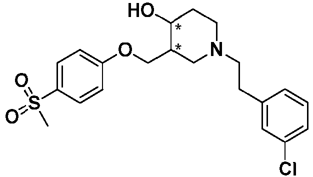 | (3*S*,4*S*) or (3*R*,4*S*) or (3*S*,4*R*) or (3*R*,4*R*)-1-(3-chlorophenethyl)-3-((4-(methylsulfonyl)phenoxy) methyl)piperidin-4-ol |
| 33 | 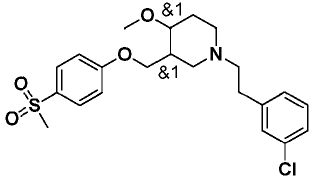 | *rac-trans or cis*-1-(3-chlorophenethyl)-4-methoxy-3-((4-(methylsulfonyl)phenoxy) methyl)piperidine |
| 34 | 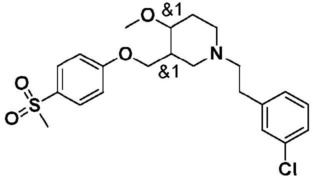 | *rac-cis or trans*-1-(3-chlorophenethyl)-4-methoxy-3-((4-(methylsulfonyl)phenoxy) methyl)piperidine |
| 35 | 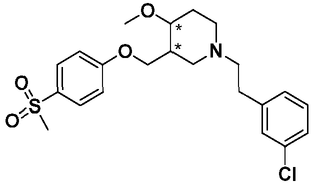 | (3*S*,4*R*) or (3*R*,4*R*) or (3*S*,4*S*) or (3*R*,4*S*)-1-(3-chlorophenethyl)-4-methoxy-3-((4-(methylsulfonyl)phenoxy) methyl)piperidine |
| 36 | 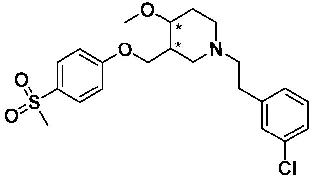 | (3*R*,4*R*) or (3*S*,4*S*) or (3*R*,4*S*) or (3*S*,4*R*)-1-(3-chlorophenethyl)-4-methoxy-3-((4-(methylsulfonyl)phenoxy) methyl)piperidine |
| 37 | 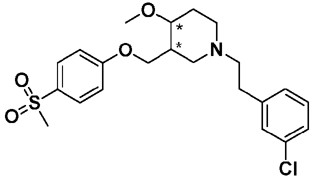 | (3*R*,4*R*) or (3*S*,4*S*) or (3*R*,4*S*) or (3*S*,4*R*)-1-(3-chlorophenethyl)-4-methoxy-3-((4-(methylsulfonyl)phenoxy) methyl)piperidine |
| 38 | 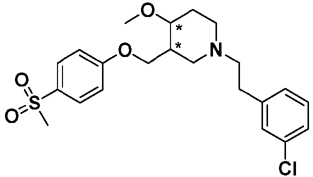 | (3*S*,4*S*) or (3*R*,4*S*) or (3*S*,4*R*) or (3*R*,4*R*)-1-(3-chlorophenethyl)-4-methoxy-3-((4-(methylsulfonyl)phenoxy) methyl)piperidine |
| 39 | 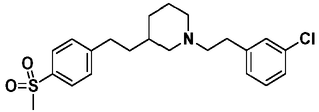 | 1-(3-chlorophenethyl)-3-(4-(methylsulfonyl)phenethyl) piperidine |
| 40 | 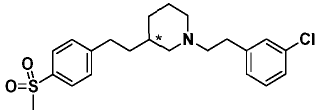 | (*S*) or (*R*)-1-(3-chlorophenethyl)-3-(4-(methylsulfonyl)phenethyl) piperidine |
| 41 | 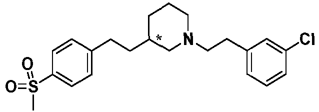 | (*R*) or (*S*)-1-(3-chlorophenethyl)-3-(4-(methylsulfonyl)phenethyl) piperidine |
| 42 | 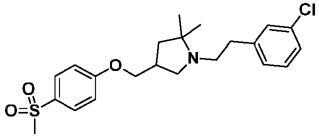 | 1-(3-chlorophenethyl)-2,2-dimethyl-4-((4-(methylsulfonyl)phenoxy) methyl) pyrrolidine |
| 43 | 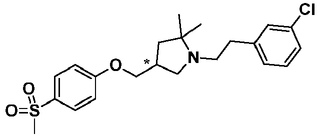 | (*S*) or (*R*)-1-(3-chlorophenethyl)-2,2-dimethyl-4-((4-(methylsulfonyl)phenoxy) methyl) pyrrolidine |
| 44 | 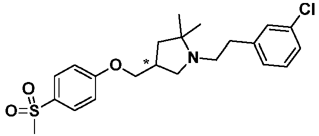 | (*R*) or (*S*)-1-(3-chlorophenethyl)-2,2-dimethyl-4-((4-(methylsulfonyl)phenoxy) methyl)pyrrolidine |
| 45 | 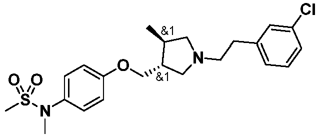 | *rac-trans-N-*(4-((1-(3-chlorophenethyl)-4-methylpyrrolidin-3-yl)methoxy)phenyl)-*N-*methylmethanesulfonamide |
| 46 | 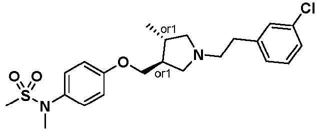 | *N-*(4-(((3*S,4*S) or (3*R*,4*R*) - 1-(3-chlorophenethyl)-4-methylpyrrolidin-3-yl)methoxy)phenyl)-*N-*methylmethanesulfonamide |
| 47 | 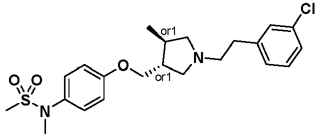 | *N*-(4-(((3*R*,4*R*) or (3*S*,4*S*) - 1-(3-chlorophenethyl)-4-methylpyrrolidin-3-yl)methoxy)phenyl)-*N-*methylmethanesulfonamide |
| 48 | 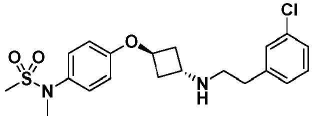 | *trans-N-*(4-(3-((3-chlorophenethyl)amino)cyclobutoxy)phenyl)-*N-*methylmethanesulfonamide |
| 49 | 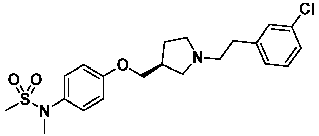 | (*S*)*-N*-(4-((1-(3-chlorophenethyl)pyrrolidin-3-yl)methoxy)phenyl)-*N-*methylmethanesulfonamide |
| 50 | 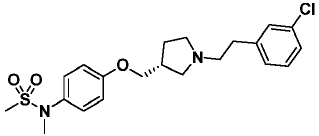 | (*R*)-*N-*(4-((1-(3-chlorophenethyl)pyrrolidin-3-yl)methoxy)phenyl)-*N-*methylmethanesulfonamide |
| 51 | 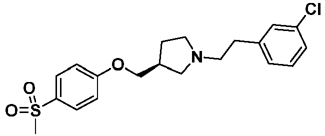 | (*S*)-1-(3-chlorophenethyl)-3-((4-(methylsulfonyl)phenoxy) methyl)pyrrolidine |
| 52 | 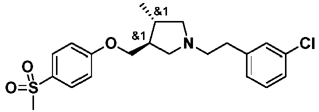 | *rac-trans-*1-(3-chlorophenethyl)-3 -methyl-4-((4-(methylsulfonyl)phenoxy) methyl)pyrrolidine |
| 53 | 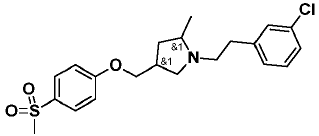 | *rac-trans or cis*-1-(3-chlorophenethyl)-2-methyl-4-((4-(methylsulfonyl)phenoxy) methyl)pyrrolidine |
| 54 | 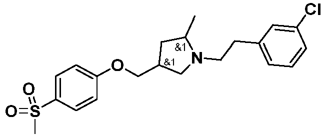 | *rac-cis or trans*-1-(3-chlorophenethyl)-2-methyl-4-((4-(methylsulfonyl)phenoxy) methyl)pyrrolidine |
| 55 | 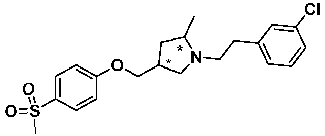 | (2*R*,4*S*) or (2*R*,4*R*) or (2*S*,4*R*) or (2*S*,4*S*)-1-(3-chlorophenethyl)-2-methyl-4-((4-(methylsulfonyl)phenoxy) methyl)pyrrolidine |
| 56 | 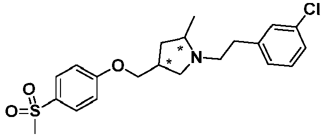 | (2*R*,4*R*) or (2*S*,4*R*) or (2*S*,4*S*) or (2*R*,4*S*)-1-(3-chlorophenethyl)-2-methyl-4-((4-(methylsulfonyl)phenoxy) methyl)pyrrolidine |
| 57 | 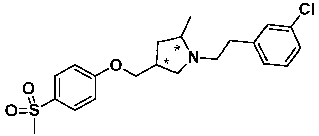 | (2*S*,4*R*) or (2*S*,4*S*) or (2*R*,4*S*) or (2*R*,4*R*)-1-(3-chlorophenethyl)-2-methyl-4-((4-(methylsulfonyl)phenoxy) methyl)pyrrolidine |
| 58 | 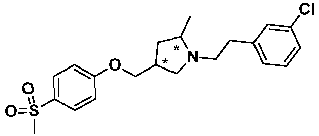 | (2*S*,4*S*) or (2*R*,4*S*) or (2*R*,4*R*) or (2*S*,4*R*)-1-(3-chlorophenethyl)-2-methyl-4-((4-(methylsulfonyl)phenoxy) methyl)pyrrolidine |
| 59 | 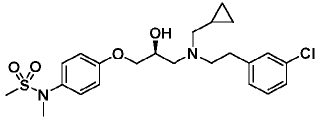 | (*S*)-*N*-(4-(3-((3-chlorophenethyl)(cycloprop ylmethyl)amino)-2-hydroxypropoxy)phenyl)-*N-*methylmethanesulfonamide |
| 60 | 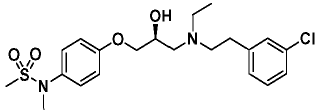 | (*S*)-*N*-(4-(3-((3-chlorophenethyl)(ethyl) amino)-2-hydroxypropoxy)phenyl)-*N-*methylmethanesulfonamide |
| 61 | 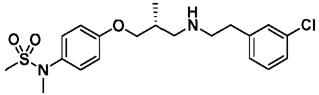 | (*R*)*-N*-(4-(3-((3-chlorophenethyl)amino)-2-methylpropoxy)phenyl)-*N-*methylmethanesulfonamide |
| 62 | 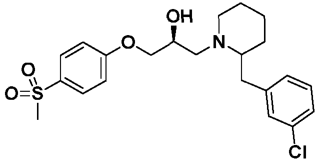 | (2*S*)-1-(2-(3-chlorobenzyl)piperidin-1-yl)-3-(4-(methylsulfonyl)phenoxy) propan-2-ol |
| 63 | 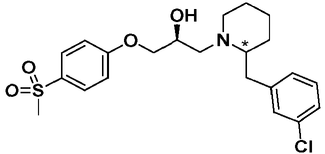 | (*S*)-1-((*S*) or (*R*)-2-(3-chlorobenzyl)piperidin-1-yl)-3-(4-(methylsulfonyl)phenoxy) propan-2-ol |
| 64 | 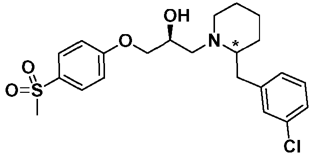 | (*S*)-1-((*R*) or (*S*)-2-(3-chlorobenzyl)piperidin-1-yl)-3-(4-(methylsulfonyl)phenoxy) propan-2- ol |
| 65 | 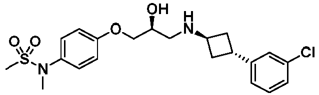 | *N*-(4-((*S*)-3-((*trans*-3-(3-chlorophenyl)cyclobutyl)am ino)-2-hydroxypropoxy)phenyl)-*N-*methylmethanesulfonamide |
| 66 | 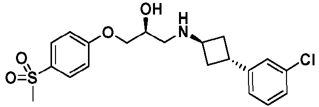 | (*S*)-1-((*trans*-3-(3-chlorophenyl)cyclobutyl)am ino)-3-(4-(methylsulfonyl)phenoxy) propan-2-ol |
| 67 | 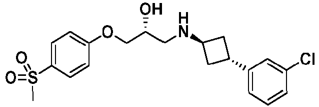 | (*R*)-1-((*trans*-3-(3-chlorophenyl)cyclobutyl)am ino)-3-(4-(methylsulfonyl)phenoxy) propan-2-ol |
| 68 | 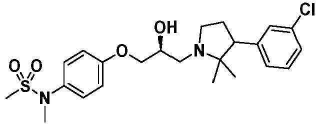 | *N*-(4-((2*S*)-3-(3-(3-chlorophenyl)-2,2-dimethylpyrrolidin-1-yl)-2-hydroxypropoxy)phenyl)-*N-*methylmethanesulfonamide |
| 69 | 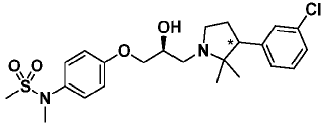 | *N*-(4-((*S*)-3-((*S*) or (*R*)-3-(3-chlorophenyl)-2,2-dimethylpyrrolidin-1-yl)-2-hydroxypropoxy)phenyl)-*N-*methylmethanesulfonamide |
| 70 | 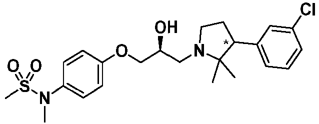 | *N*-(4-((*S*)-3-((*R*) or (*S*)-3-(3-chlorophenyl)-2,2-dimethylpyrrolidin-1-yl)-2-hydroxypropoxy)phenyl)-*N-*methylmethanesulfonamide |
| 71 | 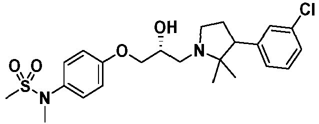 | *N*-(4-((2*R*)-3-(3-(3-chlorophenyl)-2,2-dimethylpyrrolidin-1-yl)-2-hydroxypropoxy)phenyl)-*N-*methylmethanesulfonamide |
| 72 | 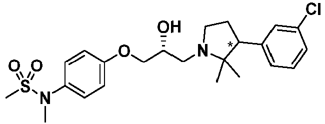 | *N*-(4-((*R*)-3-((*S*) or (R)-3-(3-chlorophenyl)-2,2-dimethylpyrrolidin-1-yl)-2-hydroxypropoxy)phenyl)-*N-*methylmethanesulfonamide |
| 73 | 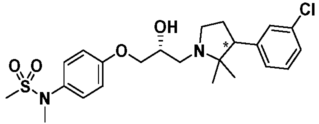 | *N-*(4-((*R*)-3-((*R*) or (*S*)-3-(3-chlorophenyl)-2,2-dimethylpyrrolidin-1-yl)-2-hydroxypropoxy)phenyl)-*N-*methylmethanesulfonamide |
| 74 | 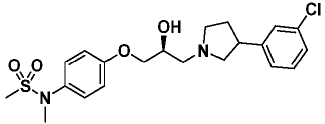 | *N-*(4-((2*S*)-3-(3-(3-chlorophenyl)pyrrolidin-1-yl)-2-hydroxypropoxy)phenyl)-*N-*methylmethanesulfonamide |
| 75 | 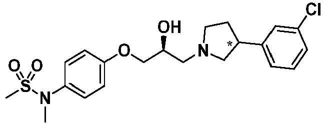 | *N*-(4-((*S*)-3-((*S*) or (*R*)-3-(3-chlorophenyl)pyrrolidin-1-yl)-2-hydroxypropoxy)phenyl)-*N-*methylmethanesulfonamide |
| 76 | 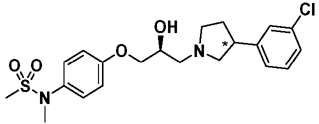 | *N*-(4-((*S*)-3-((*R*) or (*S*)-3-(3-chlorophenyl)pyrrolidin-1-yl)-2-hydroxypropoxy)phenyl)-*N-*methylmethanesulfonamide |
| 77 | 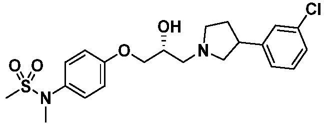 | *N*-(4-((2*R*)-3-(3-(3-chlorophenyl)pyrrolidin-1-yl)-2-hydroxypropoxy)phenyl)-*N-*methylmethanesulfonamide |
| 78 | 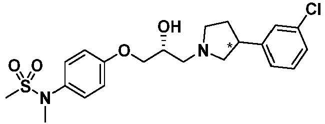 | *N*-(4-((*R*)-3-((*S*) or (*R*)-3-(3-chlorophenyl)pyrrolidin-1-yl)-2-hydroxypropoxy)phenyl)-*N-*methylmethanesulfonamide |
| 79 | 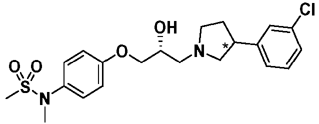 | *N-*(4-((*R*)-3-((*R)* or (*S*)-3-(3-chlorophenyl)pyrrolidin-1-yl)-2-hydroxypropoxy)phenyl)-*N-*methylmethanesulfonamide |
| 80 | 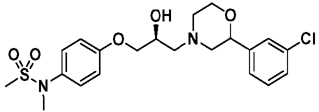 | *N*-(4-((2*S*)-3-(2-(3-chlorophenyl)morpholino)-2-hydroxypropoxy)phenyl)-*N-*methylmethanesulfonamide |
| 81 | 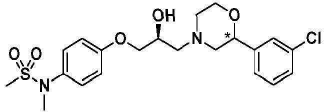 | *N*-(4-((*S*)-3-((*S*) or (*R*)-2-(3-chlorophenyl)morpholino)-2-hydroxypropoxy)phenyl)-*N-*methylmethanesulfonamide |
| 82 | 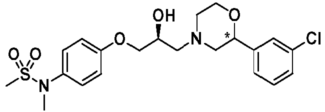 | *N*-(4-((*S*)-3-((*R*) or (*S*)-2-(3-chlorophenyl)morpholino)-2-hydroxypropoxy)phenyl)-*N-*methylmethanesulfonamide |
| 83 | 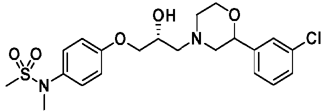 | *N*-(4-((2*R*)-3-(2-(3-chlorophenyl)morpholino)-2-hydroxypropoxy)phenyl)-*N-*methylmethanesulfonamide |
| 84 | 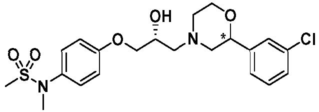 | *N-*(4-((*R*)-3-((*R*) or (*S*)-2-(3-chlorophenyl)morpholino)-2-hydroxypropoxy)phenyl)-*N-*methylmethanesulfonamide |
| 85 | 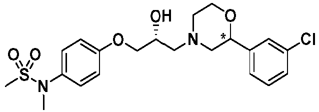 | N-(4-((*R*)-3-((*S*) or (*R*)-2-(3-chlorophenyl)morpholino)-2-hydroxypropoxy)phenyl)-*N-*methylmethanesulfonamide |
| 86 | 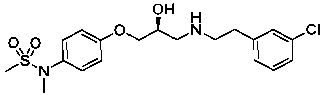 | (*S*)-*N*-(4-(3-((3-chlorophenethyl)amino)-2-hydroxypropoxy)phenyl)-*N-*methylmethanesulfonamide |
| 87 | 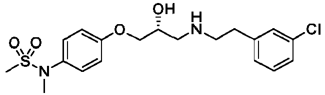 | (*R*)-*N*-(4-(3-((3-chlorophenethyl)amino)-2-hydroxypropoxy)phenyl)-*N-*methylmethanesulfonamide |
| 88 | 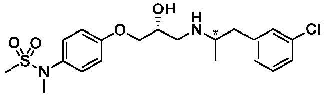 | *N*-(4-((*R*)-3-(((*S*) or (*R*)-1-(3-chlorophenyl)propan-2-yl)amino)-2-hydroxypropoxy)phenyl)-*N-*methylmethanesulfonamide |
| 89 | 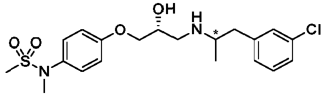 | *N-*(4-((*R*)-3-(((*R*) or (*S*)-1-(3-chlorophenyl)propan-2-yl)amino)-2-hydroxypropoxy)phenyl)-*N-*methylmethanesulfonamide |
| 90 | 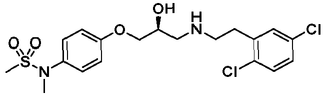 | (*S*)-*N*-(4-(3-((2,5-dichlorophenethyl)amino)-2-hydroxypropoxy)phenyl)-*N-*methylmethanesulfonamide |
| 91 | 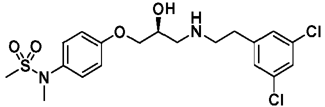 | (*S*)-*N*-(4-(3-((3,5-dichlorophenethyl)amino)-2-hydroxypropoxy)phenyl)-*N-*methylmethanesulfonamide |
| 92 | 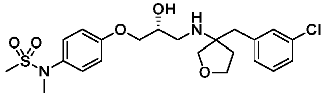 | *N*-(4-((2*R*)-3-((3-(3-chlorobenzyl)tetrahydrofura n-3-yl)amino)-2-hydroxypropoxy)phenyl)-*N-*methylmethanesulfonamide |
| 93 | 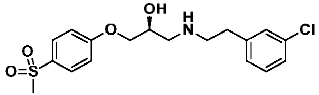 | (*S*)-1-((3-chlorophenethyl)amino)-3-(4-(methylsulfonyl)phenoxy) propan-2-ol |
| 94 | 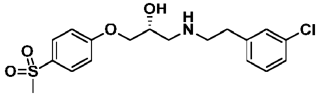 | (*R*)-1-((3-chlorophenethyl)amino)-3-(4-(methylsulfonyl)phenoxy) propan-2-ol |
| 95 | 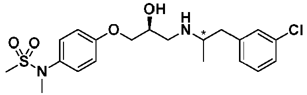 | *N*-(4-((*S*)-3-(((*S*) or (*R*)-1-(3-chlorophenyl)propan-2-yl)amino)-2-hydroxypropoxy)phenyl)-*N-*methylmethanesulfonamide |
| 96 | 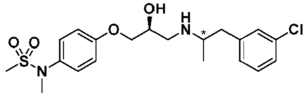 | *N*-(4-((*S*)-3-(((*R*) or (*S*)-1-(3-chlorophenyl)propan-2-yl)amino)-2-hydroxypropoxy)phenyl)-*N-*methylmethanesulfonamide |
| 97 | 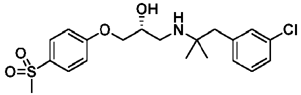 | (*R*)-1-((1-(3-chlorophenyl)-2-methylpropan-2-yl)amino)-3-(4-(methylsulfonyl)phenoxy) propan-2-ol |
| 98 | 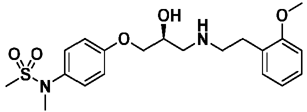 | (*S*)-*N*-(4-(2-hydroxy-3-((2-methoxyphenethyl)amino) propoxy)phenyl)-*N-*methylmethanesulfonamide |
| 99 | 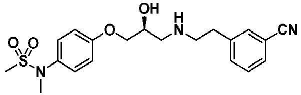 | (*S*)-*N*-(4-(3-((3-cyanophenethyl)amino)-2-hydroxypropoxy)phenyl)-*N-*methylmethanesulfonamide |
| 100 | 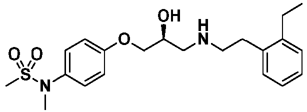 | (*S*)-*N*-(4-(3-((2-ethylphenethyl)amino)-2-hydroxypropoxy)phenyl)-*N-*methylmethanesulfonamide |
| 101 | 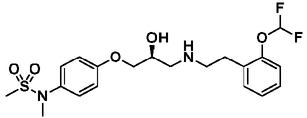 | (*S*)-*N*-(4-(3-((2-(difluoromethoxy)phenethyl) amino)-2-hydroxypropoxy)phenyl)-*N-*methylmethanesulfonamide |
| 102 | 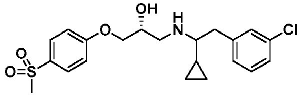 | (2*R*)-1-((2-(3-chlorophenyl)-1-cyclopropylethyl)amino)-3-(4-(methylsulfonyl)phenoxy) propan-2-ol |
| 103 | 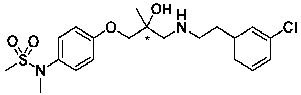 | (*R*) or (*S*)*-N-(*4-(3-((3-chlorophenethyl)amino)-2-hydroxy-2-methylpropoxy)phenyl)-*N-*methylmethanesulfonamide |
| 104 | 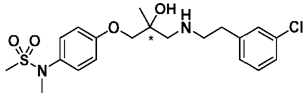 | (*S*) or (*R*)*-N*-(4-(3-((3-chlorophenethyl)amino)-2-hydroxy-2-methylpropoxy)phenyl)-*N-*methylmethanesulfonamide |
| 105 | 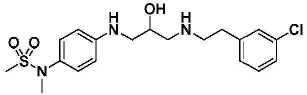 | *N*-(4-((3-((3-chlorophenethyl)amino)-2-hydroxypropyl)amino)phen yl)*-N-*methylmethanesulfonamide |
| 106 | 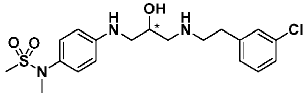 | (*R*) or (*S*)-*N-*(4-((3-((3-chlorophenethyl)amino)-2-hydroxypropyl)amino)phen yl)*-N-*methylmethanesulfonamide |
| 107 | 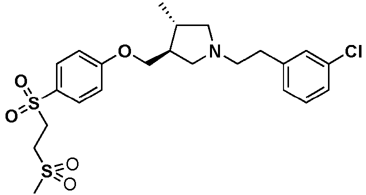 | (3*S*,4*S*)-1-[2-(3-chlorophenyl)ethyl]-3-{[4-(2-methanesulfonylethanesulfo nyl)phenoxy]methyl}-4-methylpyrrolidine |
| 108 | 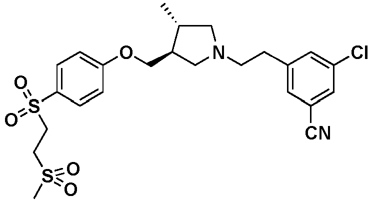 | 3-chloro-5-{2-[(3*S*,4*S*)-3-{[4-(2-methanesulfonylethanesulfo nyl)phenoxy]methyl}-4-methylpyrrolidin-1-yl]ethyl}benzonitrile |
| 109 | 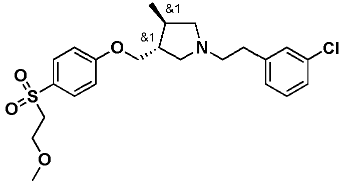 | *rac-trans-*1-[2-(3-chlorophenyl)ethyl]-3-{[4-(2-methoxyethanesulfonyl)phe noxy]methyl}-4-methylpyrrolidine |
| 110 | 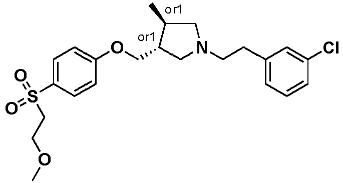 | (3*R,*4*R* or 3*S*,4*S*)-1-[2-(3-chlorophenyl)ethyl]-3-{[4-(2-methoxyethanesulfonyl) phenoxy]methyl}-4-methylpyrrolidine |
| 111 | 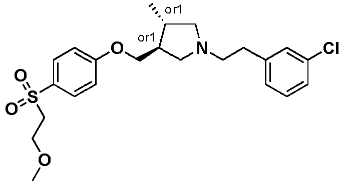 | (3*S*,4*S* or 3*R*,4*R*)-1-[2-(3-chlorophenyl)ethyl]-3-{[4-(2-methoxyethanesulfonyl) phenoxy]methyl}-4-methylpyrrolidine |
| 112 | 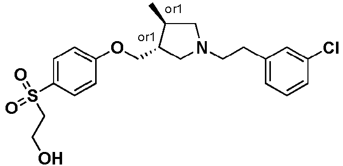 | 2-(4-{[(3*R*,4*R* or 3*S*,4*S*)-1-[2-(3-chlorophenyl)ethyl]-4-methylpyrrolidin-3-yl]methoxy}benzenesulfonyl) ethan-1-ol |
| 113 | 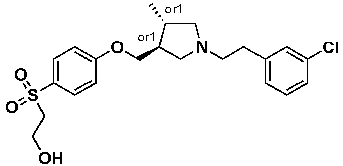 | 2-(4-{[(3*S*,4*S* or 3*R,*4*R*)-1-[2-(3-chlorophenyl)ethyl]-4-methylpyrrolidin-3-yl]methoxy}benzenesulfonyl) ethan-1-ol |
| 114 | 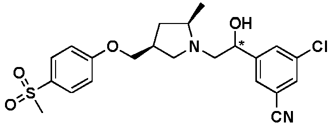 | 3-chloro-5-[(1*S* or 1*R*)-1-hydroxy-2-[(2*R*,4*S*)-4-[(4-methanesulfonylphenoxy) methyl]-2-methylpyrrolidin-1-yl]ethyl]benzonitrile |
| 115 | 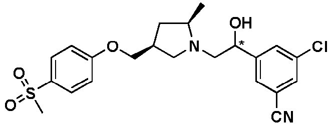 | 3-chloro-5-[(1*R* or 1*S*)-1-hydroxy-2-[(2*R*,4*S*)-4-[(4-methanesulfonylphenoxy)methyl]-2-methylpyrrolidin-1-yl] ethyl]benzonitrile |
| 116 | 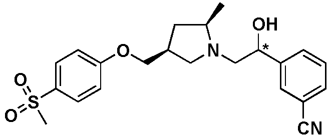 | 3-[(1*R* or 1*S*)-1-hydroxy-2-[(2*R*,4*S*)-4-[(4-methanesulfonylphenoxy) methyl]-2-methylpyrrolidin-1-yl]ethyl]benzonitrile |
| 117 | 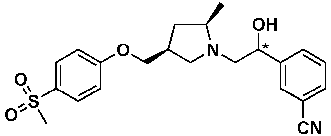 | 3-[(1*S* or 1*R*)-1-hydroxy-2-[(2*R*,4*S*)-4-[(4-methanesulfonylphenoxy) methyl]-2-methylpyrrolidin-1-yl]ethyl]benzonitrile |
| 118 | 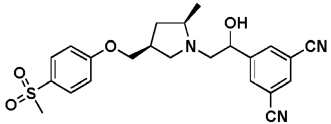 | 5-[(1*R* or 1*S*)-1-hydroxy-2-[(2*R*,4*S*)-4-[(4-methanesulfonylphenoxy) methyl]-2-methylpyrrolidin-1-yl]ethyl]benzene-1,3-dicarbonitrile |
| 119 | 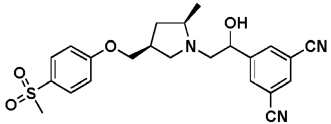 | 5-[(1*S* or 1R)-1-hydroxy-2-[(2*R*,4*S*)-4-[(4-methanesulfonylphenoxy) methyl]-2-methylpyrrolidin-1-yl]ethyl]benzene-1,3-dicarbonitrile |
| 120 | 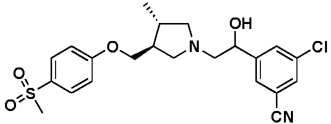 | 3-chloro-5-{1-hydroxy-2-[(3*S*,4*S*)-3-[(4-methanesulfonylphenoxy) methyl]-4-methylpyrrolidin-1-yl]ethyl}benzonitrile |
| 121 | 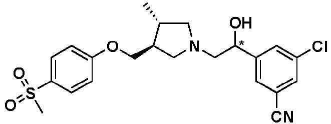 | 3-chloro-5-[(1*S* or 1*R*)-1-hydroxy-2-[(3*S*,4*S*)-3-[(4-methanesulfonylphenoxy)methyl]-4-methylpyrrolidin-1-yl]ethyl]benzonitrile |
| 122 | 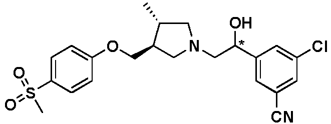 | 3-chloro-5-[(1*R* or 1*S*)-1-hydroxy-2-[(3*S*,4*S*)-3-[(4-methanesulfonylphenoxy) methyl]-4-methylpyrrolidin-1-yl]ethyl]benzonitrile |
| 123 | 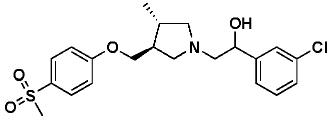 | 1-(3-chlorophenyl)-2-((3*S*,4*S*)-3-methyl-4-((4-(methylsulfonyl)phenoxy) methyl)pyrrolidin-1-yl)ethan-1-ol |
| 124 | 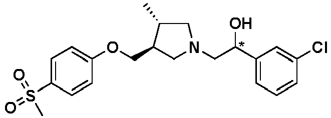 | (1*R* or 1S)-1-(3-chlorophenyl)-2-[(3*S*,4*S*)-3-[(4-methanesulfonylphenoxy) methyl]-4-methylpyrrolidin-1-yl]ethan-1-ol |
| 125 | 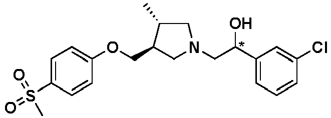 | (1*S* or 1*R*)-1-(3-chlorophenyl)-2-[(3*S*,4*S*)-3-[(4-methanesulfonylphenoxy) methyl]-4-methylpyrrolidin-1-yl]ethan-1-ol |
| 126 | 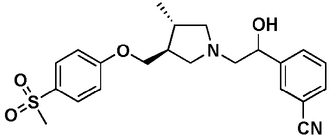 | 3-[1-hydroxy-2-[(3*S*,4*S*)-3-[(4-methanesulfonylphenoxy) methyl]-4-methylpyrrolidin-1-yl]ethyl]benzonitrile |
| 127 | 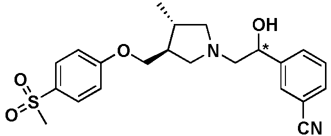 | 3-[(1*S* or 1*R*)-1-hydroxy-2-[(3S,4S)-3-[(4-methanesulfonylphenoxy) methyl]-4-methylpyrrolidin-1-yl]ethyl]benzonitrile |
| 128 | 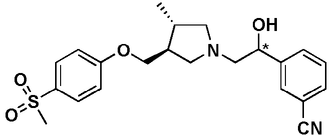 | 3-[(1*R* or 1*S*)-1-hydroxy-2-[(3*S*,4*S*)-3-[(4-methanesulfonylphenoxy) methyl]-4-methylpyrrolidin-1-yl]ethyl]benzonitrile |
| 129 | 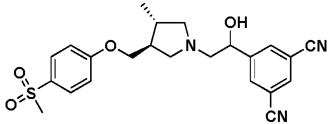 | 5-{1-hydroxy-2-[(3S,4S)-3-[(4-methanesulfonylphenoxy) methyl]-4-methylpyrrolidin-1-yl]ethyl}benzene-1,3-dicarbonitrile |
| 130 | 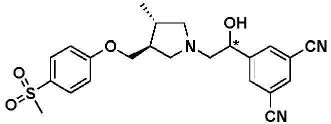 | 5-[(1*R* or 1S)-1-hydroxy-2-[(3*S*,4*S*)-3-[(4-methanesulfonylphenoxy) methyl]-4-methylpyrrolidin-1-yl]ethyl]benzene-1,3-dicarbonitrile |
| 131 | 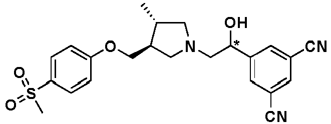 | 5-[(1*S* or 1*R*)-1-hydroxy-2-[(3*S*,4*S*)-3-[(4-methanesulfonylphenoxy) methyl]-4-methylpyrrolidin-1-yl]ethyl]benzene-1,3-dicarbonitrile |
| 132 | 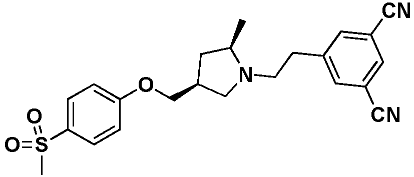 | 5-{2-[(2*R*,4*S*)-4-[(4-methanesulfonylphenoxy) methyl]-2-methylpyrrolidin-1-yl]ethyl}benzene-1,3-dicarbonitrile |
| 133 | 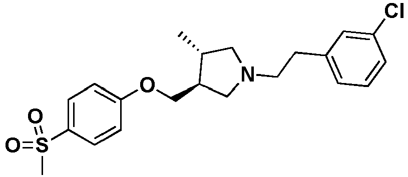 | (3*S*,4*S*)-1-[2-(3-chlorophenyl)ethyl]-3-[(4-methanesulfonylphenoxy) methyl]-4-methylpyrrolidine |
| 134 | 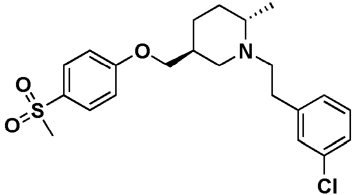 | (2*S*,5*S*)-1-[2-(3-chlorophenyl)ethyl]-5-[(4-methanesulfonylphenoxy) methyl]-2-methylpiperidine |
| 135 | 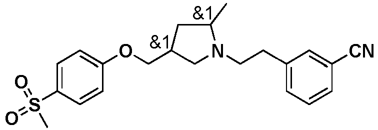 | r*ac-cis* or *trans*-3-{2-[4-[(4-methanesulfonylphenoxy) methyl]-2-methylpyrrolidin-1-yl]ethyl}benzonitrile |
| 136 | 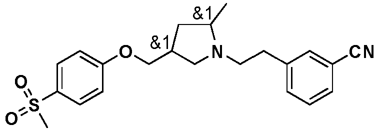 | *rac-trans* or *cis-*3-{2-[4-[(4-methanesulfonylphenoxy) methyl]-2-methylpyrrolidin-1-yl]ethyl}benzonitrile |
| 137 | 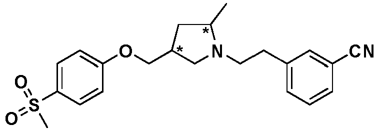 | 3-{2-[(2*S*,4*R* or 2*R*,4*S* or 2*R*,4*R* or 2*S*,4*S*)-4-[(4-methanesulfonylphenoxy) methyl]-2-methylpyrrolidin-1-yl]ethyl}benzonitrile |
| 138 | 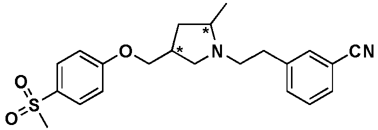 | 3-{2-[(2*R*,4*S* or 2*S*,4*R* or 2*R*,4*R* or 2*S*,4*S*)-4-[(4-methanesulfonylphenoxy)methyl]-2-methylpyrrolidin-1-yl]ethyl}benzonitrile |
| 139 | 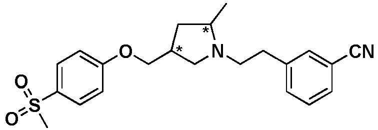 | 3-{2-[(2*S*,4*S* or 2*S*,4*R* or 2*R*,4*S* or 2*R*,4*R*)-4-[(4-methanesulfonylphenoxy) methyl]-2-methylpyrrolidin-1-yl]ethyl}benzonitrile |
| 140 | 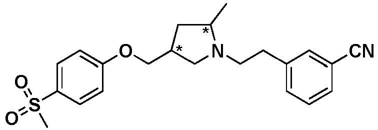 | 3-{2-[(2*R*,4*R* or 2*S*,4*S* or 2*S*,4*R* or 2*R*,4*S*)-4-[(4-methanesulfonylphenoxy) methyl]-2-methylpyrrolidin-1-yl]ethyl}benzonitrile |
| 141 | 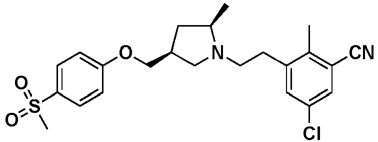 | 5-chloro-3-{2-[(2*R*,4*S*)-4-[(4-methanesulfonylphenoxy) methyl]-2-methylpyrrolidin-1-yl]ethyl}-2-methylbenzonitrile |
| 142 | 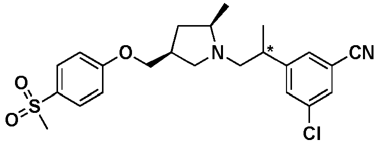 | 3-chloro-5-[(2*R* or 2*S*)-1-[(2R,4S)-4-[(4-methanesulfonylphenoxy) methyl]-2-methylpyrrolidin-1-yl]propan-2-yl]benzonitrile |
| 143 | 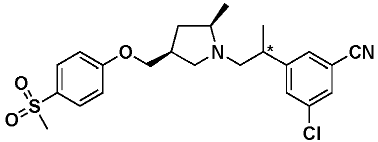 | 3-chloro-5-[(2*S* or 2*R*)-1-[(2R,4S)-4-[(4-methanesulfonylphenoxy) methyl]-2-methylpyrrolidin-1-yl]propan-2-yl]benzonitrile |
| 144 | 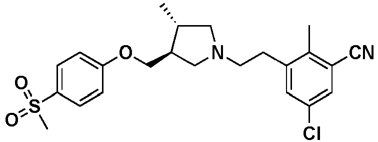 | 5-chloro-3-{2-[(3*S*,4*S*)-3-[(4-methanesulfonylphenoxy) methyl]-4-methylpyrrolidin-1-yl]ethyl}-2-methylbenzonitrile |
| 145 | 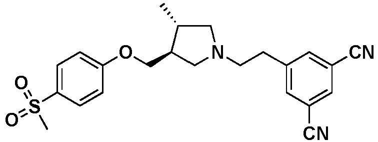 | 5-{2-[(3*S*,4*S*)-3-[(4-methanesulfonylphenoxy) methyl]-4-methylpyrrolidin-1-yl]ethyl}benzene-1,3-dicarbonitrile |
| 146 | 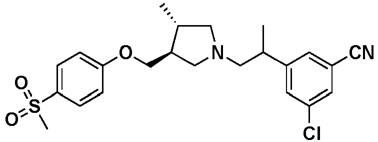 | 3-chloro-5-[1-[(3*S*,4*S*)-3-[(4-methanesulfonylphenoxy) methyl]-4-methylpyrrolidin-1-yl]propan-2-yl]benzonitrile |
| 147 | 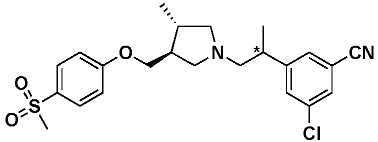 | 3-chloro-5-[(2*R* or 2*S*)-1-[(3S,4S)-3-[(4-methanesulfonylphenoxy) methyl]-4-methylpyrrolidin-1-yl]propan-2-yl]benzonitrile |
| 148 | 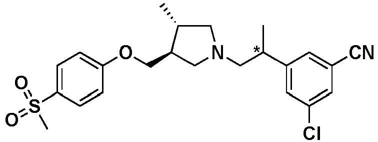 | 3-chloro-5-[(2*S* or 2*R*)-1-[(3S,4S)-3-[(4-methanesulfonylphenoxy) methyl]-4-methylpyrrolidin-1-yl]propan-2-yl]benzonitrile |
| 149 | 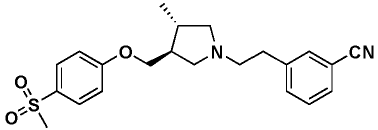 | 3-{2-[(3*S*,4*S*)-3-[(4-methanesulfonylphenoxy) methyl]-4-methylpyrrolidin-1-yl] ethyl } benzonitrile |
| 150 | 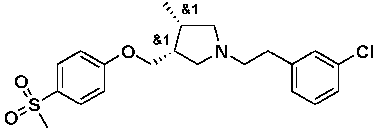 | *rac-cis*-1-[2-(3-chlorophenyl)ethyl]-3-[(4-methanesulfonylphenoxy) methyl]-4-methylpyrrolidine |
| 151 | 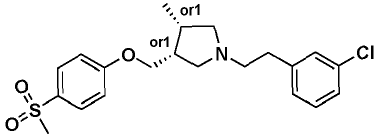 | (3*R*,4*S* or 3*S,*4*R*)-1-[2-(3-chlorophenyl)ethyl]-3-[(4-methanesulfonylphenoxy) methyl]-4-methylpyrrolidine |
| 152 | 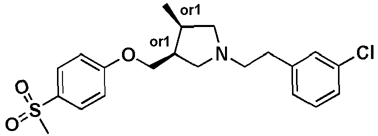 | (3*S*,4*R* or 3*R*,4*S*)-1-[2-(3-chlorophenyl)ethyl]-3-[(4-methanesulfonylphenoxy) methyl]-4-methylpyrrolidine |
| 153 | 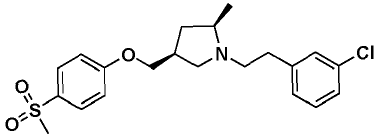 | (2*R*,4*S*)-1-[2-(3-chlorophenyl)ethyl]-4-[(4-methanesulfonylphenoxy) methyl]-2-methylpyrrolidine |
| 154 | 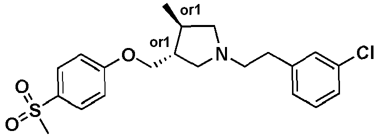 | (3*R*,4*R* or 3*S*,4*S*)-1-[2-(3-chlorophenyl)ethyl]-3-[(4-methanesulfonylphenoxy) methyl]-4-methylpyrrolidine |
| 155 | 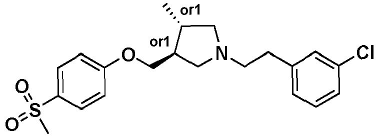 | (3*S*,4*S* or 3*R*,4*R*)-1-[2-(3-chlorophenyl)ethyl]-3-[(4-methanesulfonylphenoxy) methyl]-4-methylpyrrolidine |
| 156 | 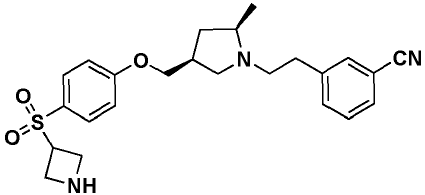 | 3-{2-[(2*R*,4*S*)-4-{ [4-(azetidine-3-sulfonyl)phenoxy]methyl}-2-methylpyrrolidin-1-yl]ethyl}benzonitrile |
| 157 | 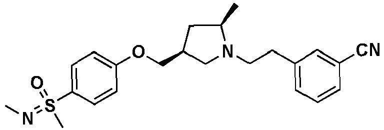 | 3-{2-[(2*R*,4*S*)-2-methyl-4-({4-[methyl(methylimino)oxo-λ⁶-sulfanyl]phenoxy}methyl) pyrrolidin-1-yl]ethyl}benzonitrile |
| 158 | 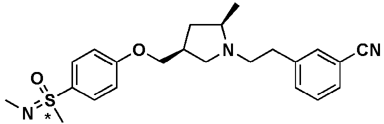 | 3-{2-[(2*R*,4*S*)-2-methyl-4-({4-[(*R* or *S*)methyl(methylimino)oxo-λ⁶-sulfanyl]phenoxy}methyl)p yrrolidin-1-yl]ethyl}benzonitrile |
| 159 | 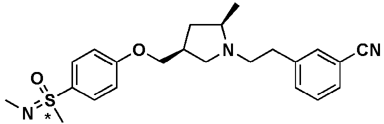 | 3-{2-[(2*R*,4*S*)-2-methyl-4-({4-[(*S* or *R*)methyl(methylimino)oxo-λ⁶-sulfanyl]phenoxy}methyl) pyrrolidin-1-yl]ethyl}benzonitrile |
| 160 | 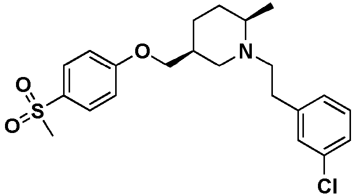 | (2*R*,5*S*)-1-[2-(3-chlorophenyl)ethyl]-5-[(4-methanesulfonylphenoxy) methyl]-2-methylpiperidine |
| 161 | 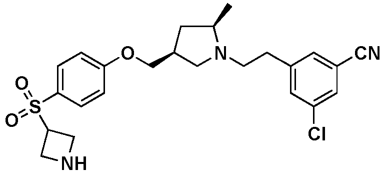 | 3-{2-[(2R,4S)-4-{ [4-(azetidine-3-sulfonyl)phenoxy]methyl}-2-methylpyrrolidin-1-yl]ethyl}-5-chlorobenzonitrile |
| 162 | 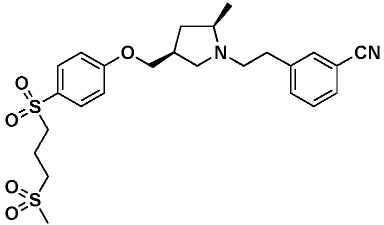 | 3-{2-[(2*R*,4*S*)-4-{[4-(3-methanesulfonylpropanesulf onyl)phenoxy]methyl}-2-methylpyrrolidin-1-yl]ethyl}benzonitrile |
| 163 | 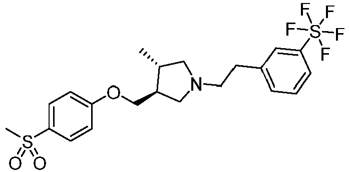 | (3*S*,4*S*)-3-[(4-methanesulfonylphenoxy) methyl]-4-methyl-1-{2-[3-(pentafluoro-λ⁶-sulfanyl)phenyl]ethyl}pyrro lidine |
| 164 | 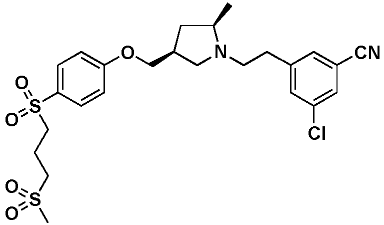 | 3-chloro-5-{2-[(2*R*,4*S*)-4-{[4-(3-methanesulfonylpropanesulf onyl)phenoxy]methyl}-2-methylpyrrolidin-1-yl]ethyl}benzonitrile |
| 165 | 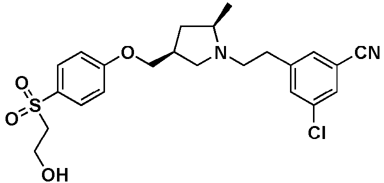 | 3-chloro-5-{2-[(2*R*,4*S*)-4-{[4-(2-hydroxyethanesulfonyl)phe noxy]methyl}-2-methylpyrrolidin-1-yl]ethyl}benzonitrile |
| 166 | 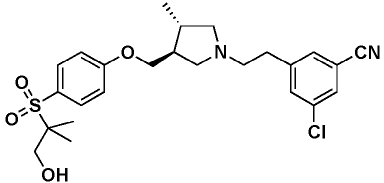 | 3-chloro-5-{2-[(3*S*,4*S*)-3-{[4-(1-hydroxy-2-methylpropane-2-sulfonyl)phenoxy]methyl}-4-methylpyrrolidin-1-yl]ethyl}benzonitrile |
| 167 | 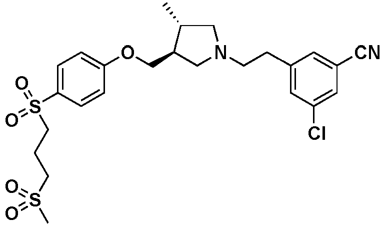 | 3-chloro-5-{2-[(3*S*,4*S*)-3-{[4-(3-methanesulfonylpropanesulf onyl)phenoxy]methyl}-4-methylpyrrolidin-1-yl]ethyl}benzonitrile |
| 168 | 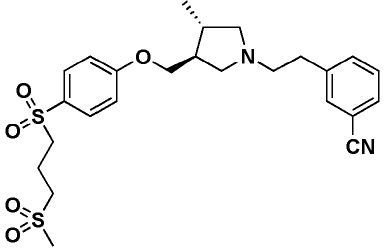 | 3-12-[(3*S*,4*S*)-3-1[4-(3-methanesulfonylpropanesulf onyl)phenoxy]methyl}-4-methylpyrrolidin-1-yl]ethyl}benzonitrile |
| 169 | 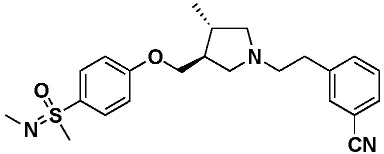 | 3-{2-[(3*S*,4*S*)-3-methyl-4-({4-[methyl(methylimino)oxo-λ⁶-sulfanyl]phenoxy}methyl) pyrrolidin-1-yl]ethyl}benzonitrile |
| 170 | 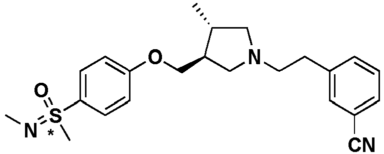 | 3-{2-[(3*S*,4*S*)-3-methyl-4-({4-[(*R* or *S*)-methyl(methylimino)oxo-λ⁶-sulfanyl]phenoxy}methyl) pyrrolidin-1-yl]ethyl}benzonitrile |
| 171 | 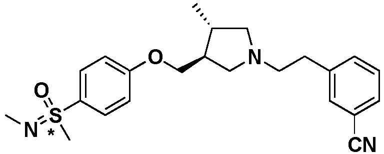 | 3-{2-[(3*S*,4*S*)-3-methyl-4-({4-[(*S* or *R*)-methyl(methylimino)oxo-λ⁶-sulfanyl]phenoxy}methyl) pyrrolidin-1-yl]ethyl}benzonitrile |
| 172 | 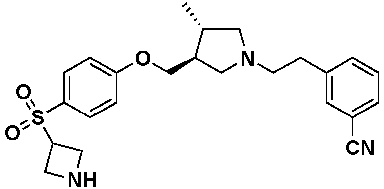 | 3-{2-[(3*S*,4*S*)-3-{[4-(azetidine-3-sulfonyl)phenoxy]methyl}-4-methylpyrrolidin-1-yl]ethyl}benzonitrile |
| 173 | 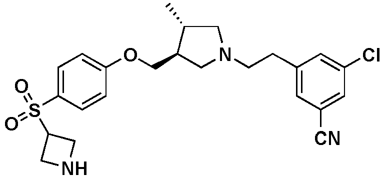 | 3-{2-[(3*S*,4*S*)-3-{ [4-(azetidine-3-sulfonyl)phenoxy]methyl}-4-methylpyrrolidin-1-yl]ethyl}-5-chlorobenzonitrile |
| 174 | 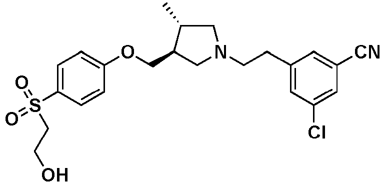 | 3-chloro-5-{2-[(3*S*,4*S*)-3-{[4-(2-hydroxyethanesulfonyl)phe noxy]methyl}-4-methylpyrrolidin-1-yl]ethyl}benzonitrile |
| 175 | 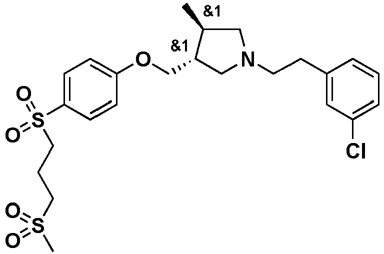 | *rac-trans-*1-[2-(3-chlorophenyl)ethyl]-3-{[4-(3-methanesulfonylpropanesulf onyl)phenoxy]methyl}-4-methylpyrrolidine |
| 176 | 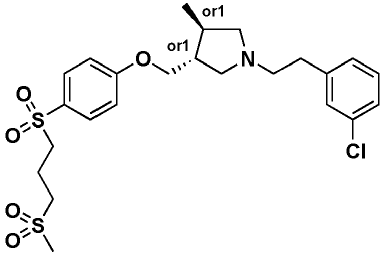 | (3*R*,4*R* or 3*S*,4*S*)-1-[2-(3-chlorophenyl)ethyl]-3-{[4-(3-methanesulfonylpropanesulf onyl)phenoxy]methyl}-4-methylpyrrolidine |
| 177 | 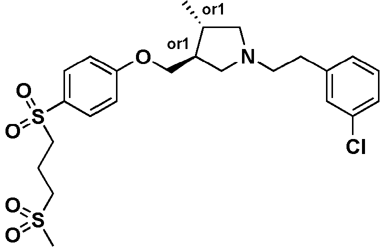 | (3*S*,4*S* or 3*R*,4*R*)-1-[2-(3-chlorophenyl)ethyl]-3-{[4-(3-methanesulfonylpropanesulf onyl)phenoxy]methyl}-4-methylpyrrolidine |
| 181 | 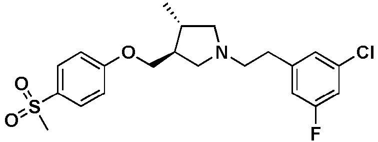 | (3*S*,4*S*)-1-[2-(3-chloro-5-fluorophenyl)ethyl]-3-[(4-methanesulfonylphenoxy) methyl]-4-methylpyrrolidine |
| 182 | 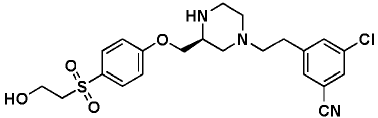 | 3-chloro-5-{2-[(3*S*)-3-{ [4-(2-hydroxyethanesulfonyl) phenoxy]methyl}piperazin-1-yl]ethyl}benzonitrile |
| 183 | 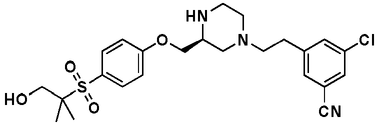 | 3-chloro-5-{2-[(3*S*)-3-{ [4-(1-hydroxy-2-methylpropane-2-sulfonyl)phenoxy]methyl} piperazin-1-yl]ethyl}benzonitrile |
| 184 | 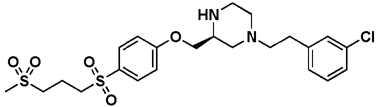 | (3*S*)-1-[2-(3-chlorophenyl)ethyl]-3-{ [4-(3-methanesulfonylpropanesulf onyl)phenoxy]methyl}piper azine |
| 185 | 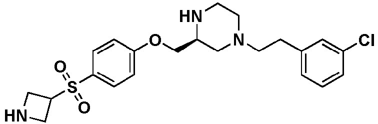 | (3*S*)-3-{ [4-(azetidine-3-sulfonyl)phenoxy]methyl}-1-[2-(3-chlorophenyl)ethyl]piperazi ne |
| 186 | 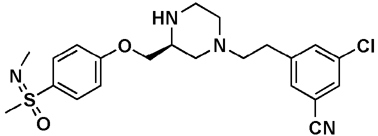 | 3-chloro-5-{2-[(3*S*)-3-(14-[methyl(methylimino)oxo-λ⁶-sulfanyl]phenoxy}methyl) piperazin-1-yl]ethyl}benzonitrile |
| 187 | 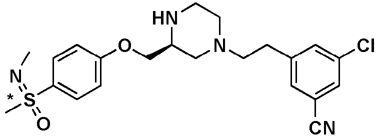 | 3-chloro-5-{2-[(3*S*)-3-({4-[(*S* or *R*)-methyl(methylimino)oxo-λ⁶-sulfanyl]phenoxy}methyl) piperazin-1-yl]ethyl}benzonitrile |
| 188 | 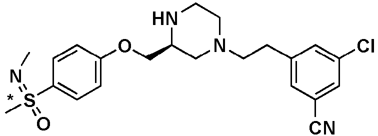 | 3-chloro-5-{2-[(3*S*)-3-({4-[(*R* or *S*)-methyl(methylimino)oxo-λ⁶-sulfanyl]phenoxy}methyl) piperazin-1-yl]ethyl}benzonitrile |
| 189 | 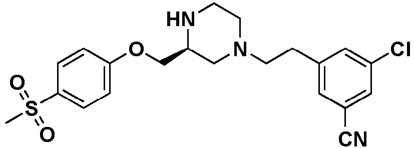 | 3-chloro-5-{2-[(3*S*)-3-[(4-methanesulfonylphenoxy) methyl]piperazin-1-yl]ethyl}benzonitrile |
| 190 | 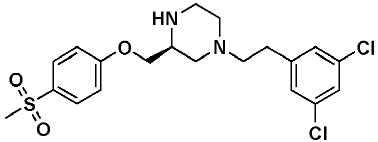 | (3*S*)-1-[2-(3,5-dichlorophenyl)ethyl]-3-[(4-methanesulfonylphenoxy) methyl]piperazine |
| 191 | 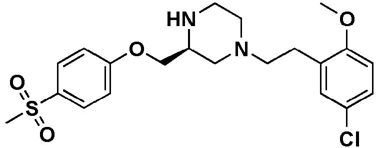 | (3*S*)-1-[2-(5-chloro-2-methoxyphenyl)ethyl]-3-[(4-methanesulfonylphenoxy)m ethyl]piperazine |
| 192 | 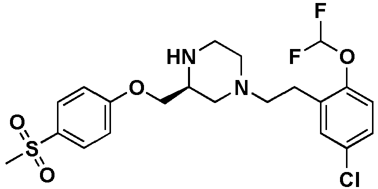 | (3*S*)-1-{2-[5-chloro-2-(difluoromethoxy)phenyl] ethyl}-3-[(4-methanesulfonylphenoxy) methyl]piperazine |
| 193 | 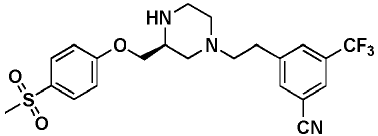 | 3-{2-[(3*S*)-3-[(4-methanesulfonylphenoxy) methyl]piperazin-1-yl]ethyl }-5-(trifluoromethyl)benzonitrile |
| 194 | 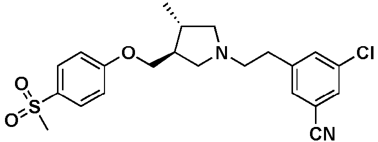 | 3-chloro-5-{2-[(3*S*,4*S*)-3-[(4-methanesulfonylphenoxy) methyl]-4-methylpyrrolidin-1-yl]ethyl}benzonitrile |
| 195 | 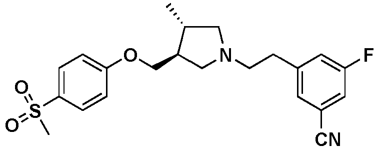 | 3-fluoro-5-{2-[(3*S*,4*S*)-3-[(4-methanesulfonylphenoxy) methyl]-4-methylpyrrolidin-1-yl]ethyl}benzonitrile |
| 196 | 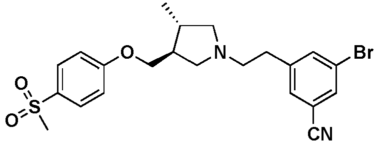 | 3-bromo-5-{2-[(3*S*,4*S*)-3-[(4-methanesulfonylphenoxy) methyl]-4-methylpyrrolidin-1-yl]ethyl}benzonitrile |
| 197 | 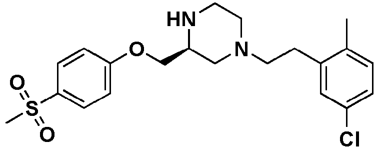 | (3*S*)-1-[2-(5-chloro-2-methylphenyl)ethyl]-3-[(4-methanesulfonylphenoxy) methyl]piperazine |
| 198 | 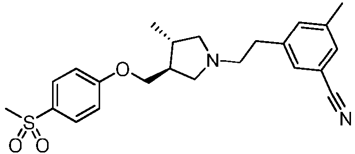 | 3-{2-[(3*S*,4*S*)-3-[(4-methanesulfonylphenoxy) methyl]-4-methylpyrrolidin-1-yl]ethyl}-5-methylbenzonitrile |
| 199 | 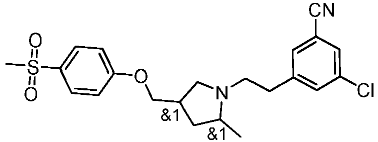 | *rac-cis* or *trans-*3-chloro-5-{2-[4-[(4-methanesulfonylphenoxy) methyl]-2-methylpyrrolidin-1-yl]ethyl}benzonitrile |
| 200 | 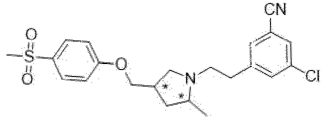 | 3-chloro-5-{2-[(2*R*,4*S* or 2S,4R or 2R,4R or 2*S*,4*S*)-4-[(4-methanesulfonylphenoxy) methyl]-2-methylpyrrolidin-1-yl]ethyl}benzonitrile |
| 201 | 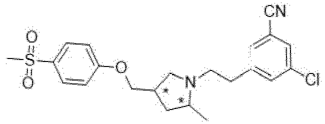 | 3-chloro-5-{2-[(2*S*,4*R* or 2R,4S or 2R,4R or 2S,4S)-4-[(4-methanesulfonylphenoxy) methyl]-2-methylpyrrolidin-1-yl]ethyl}benzonitrile |
| 202 | 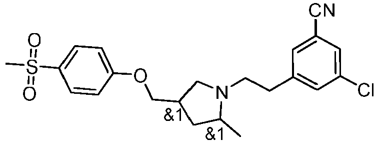 | *rac-trans* or cis-3-chloro-5-{2-[4-[(4-methanesulfonylphenoxy) methyl]-2-methylpyrrolidin-1-yl]ethyl}benzonitrile |
| 203 | 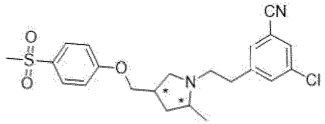 | 3-chloro-5-(2-((2*R*,4*S* or 2*S*,4*R* or 2*R*,4*R* or 2*S*,4*S*)-2-methyl-4-((4-(methylsulfonyl)phenoxy)methyl)pyrrolidin-1-yl)ethyl)benzonitrile |
| 204 | 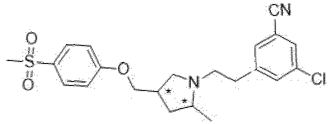 | 3-chloro-5-(2-((2R,4S or 2*S*,4*R* or 2*S*,4*S* or 2*R*.4*R*)-2-methyl-4-((4-(methylsulfonyl)phenoxy) methyl)pyrrolidin-1-yl)ethyl)benzonitrile |
| 205 | 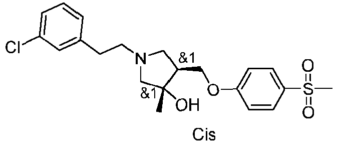 | *rac-cis*-1-[2-(3-chlorophenyl)ethyl]-4-[(4-methanesulfonylphenoxy) methyl]-3-methylpyrrolidin-3-ol |
| 206 | 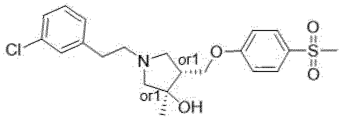 | (3*R*,4*R* or 3*S*,4*S*)-1-[2-(3-chlorophenyl)ethyl]-4-[(4-methanesulfonylphenoxy) methyl]-3-methylpyrrolidin-3-ol |
| 207 | 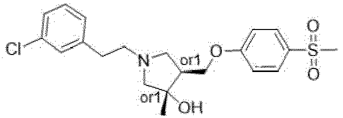 | (3*S*,4*S* or 3*R*,4*R*)-1-(3-chlorophenethyl)-3 -methyl-4-((4-(methylsulfonyl)phenoxy) methyl)pyrrolidin-3-ol |
| 208 | 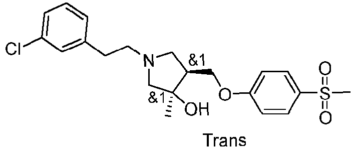 | *rac-trans*-1-[2-(3-chlorophenyl)ethyl]-4-[(4-methanesulfonylphenoxy) methyl]-3-methylpyrrolidin-3-ol |
| 209 | 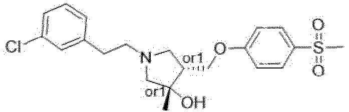 | (3*R*,4*S* or 3*S*,4*R*)-1-[2-(3-chlorophenyl)ethyl]-4-[(4-methanesulfonylphenoxy)methyl]-3-methylpyrrolidin-3-ol |
| 210 | 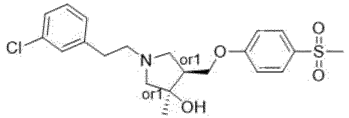 | (3*S*,4*R* or 3*R*,4*S*)-1-[2-(3-chlorophenyl)ethyl]-4-[(4-methanesulfonylphenoxy) methyl]-3-methylpyrrolidin-3-ol |
| 211 | 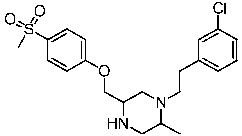 | 1-[2-(3-chlorophenyl)ethyl]-5-[(4-methanesulfonylphenoxy) methyl]-2-methylpiperazine |
| 212 | 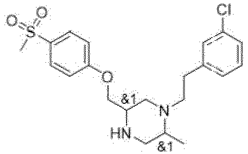 | *rac-cis* or *trans*-1-[2-(3-chlorophenyl)ethyl]-5-[(4-methanesulfonylphenoxy) methyl]-2-methylpiperazine |
| 213 | 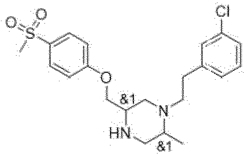 | *rac-trans* or *cis*-1-[2-(3-chlorophenyl)ethyl]-5-[(4-methanesulfonylphenoxy) methyl]-2-methylpiperazine |
| 214 | 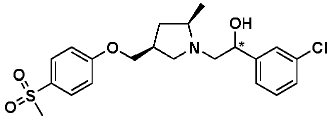 | (1*R*) or (1*S*)-1-(3-chlorophenyl)-2-[(2R,4S)-4-[(4-methanesulfonylphenoxy) methyl]-2-methylpyrrolidin-1-yl]ethan-1-ol |
| 215 | 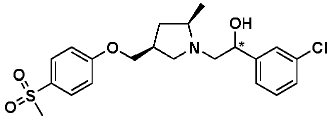 | (1*S*) or (1*R*)-1-(3-chlorophenyl)-2-[(2R,4S)-4-[(4-methanesulfonylphenoxy) methyl]-2-methylpyrrolidin-1-yl]ethan-1-ol |

In some embodiments, a compound of formula (A) or (A') is selected from the group consisting of:
1-(3-chlorophenethyl)-3-((4-(methylsulfonyl)phenoxy)methyl)azepane;
1-(3-chlorophenethyl)-3-((4-(methylsulfonyl)phenoxy)methyl)piperidin-3-ol;
*N*-(4-((1-(3-chlorophenethyl)piperidin-3-yl)methoxy)phenyl)-N-methylmethanesulfonamide;
*N*-(4-((4-(3-chlorophenethyl)-1,4-oxazepan-2-yl)methoxy)phenyl)-*N-*methylmethanesulfonamide;
1-(3-chlorophenethyl)-3-((4-(methylsulfonyl)phenoxy)methyl)piperazine;
1-(3-chlorophenethyl)-3-((4-(methylsulfonyl)phenoxy)methyl)piperidine;
1-(3-chlorophenyl)-2-(3-((4-(methylsulfonyl)phenoxy)methyl)piperidin-1-yl)ethan-1-ol;
1-(3-chlorophenyl)-2-(3-((4-(methylsulfonyl)phenoxy)methyl)piperidin-1-yl)ethanol;
1-(2-(3-chlorophenyl)-2-methoxyethyl)-3-((4-(methylsulfonyl)phenoxy)methyl)piperidine;
1-(3-chlorophenethyl)-3-((4-(methylsulfonyl)phenoxy)methyl)piperidin-4-ol;
1-(3-chlorophenethyl)-4-methoxy-3-((4-(methylsulfonyl)phenoxy) methyl)piperidine;
1-(3-chlorophenethyl)-3-(4-(methylsulfonyl)phenethyl)piperidine;
1-(3-chlorophenethyl)-2,2-dimethyl-4-((4-(methylsulfonyl)phenoxy)methyl)pyrrolidine;
*N*-(4-(1-(3-chlorophenethyl)-4-methylpyrrolidin-3-yl)methoxy)phenyl)-*N-*methylmethanesulfonamide;
*N*-(4-(3-((3-chlorophenethyl)amino)cyclobutoxy)phenyl)-*N*-methylmethanesulfonamide;
*N*-(4-((1-(3-chlorophenethyl)pyrrolidin-3-yl)methoxy)phenyl)-*N*-methylmethanesulfonamide;
1-(3-chlorophenethyl)-3-((4-(methylsulfonyl)phenoxy)methyl)pyrrolidine;
1-(3-chlorophenethyl)-3-methyl-4-((4-(methylsulfonyl)phenoxy)methyl)pyrrolidine;
1-(3-chlorophenethyl)-2-methyl-4-((4-(methylsulfonyl)phenoxy)methyl)pyrrolidine;
*N*-(4-(3-((3-chlorophenethyl)(cyclopropylmethyl)amino)-2-hydroxypropoxy)phenyl)-N-methylmethanesulfonamide;
*N*-(4-(3-((3-chlorophenethyl)(ethyl)amino)-2-hydroxypropoxy)phenyl)-N-methylmethanesulfonamide;
*N*-(4-(3-((3-chlorophenethyl)amino)-2-methylpropoxy)phenyl)-*N*-methylmethanesulfonamide;
1-(2-(3-chlorobenzyl)piperidin-1-yl)-3-(4-(methylsulfonyl)phenoxy)propan-2-ol;
*N*-(4-(3-(3-(3-chlorophenyl)cyclobutyl)amino)-2-hydroxypropoxy)phenyl)-*N-*methylmethanesulfonamide;
1-(3-(3-chlorophenyl)cyclobutyl)amino)-3-(4-(methylsulfonyl)phenoxy)propan-2-ol;
*N*-(4-((2)-3-(3-(3-chlorophenyl)-2,2-dimethylpyrrolidin-1-yl)-2-hydroxypropoxy)phenyl)-*N-*methylmethanesulfonamide;
*N*-(4-((2)-3-(3-(3-chlorophenyl)pyrrolidin-1-yl)-2-hydroxypropoxy)phenyl)-*N-*methylmethanesulfonamide;
*N*-(4-(3-(3-chlorophenyl)pyrrolidin-1-yl)-2-hydroxypropoxy)phenyl)-*N-*methylmethanesulfonamide;
*N*-(4-(3-(2-(3-chlorophenyl)morpholino)-2-hydroxypropoxy)phenyl)-*N-*methylmethanesulfonamide;
*N*-(4-(3-((3-chlorophenethyl)amino)-2-hydroxypropoxy)phenyl)-*N*-methylmethanesulfonamide;
*N*-(4-(3-((1-(3-chlorophenyl)propan-2-yl)amino)-2-hydroxypropoxy)phenyl)-N-methylmethanesulfonamide;
*N*-(4-(3-((2,5-dichlorophenethyl)amino)-2-hydroxypropoxy)phenyl)-N-methylmethanesulfonamide;
*N*-(4-(3-((3,5-dichlorophenethyl)amino)-2-hydroxypropoxy)phenyl)-N-methylmethanesulfonamide;
*N*-(4-(3-((3-(3-chlorobenzyl)tetrahydrofuran-3-yl)amino)-2-hydroxypropoxy)phenyl)-*N-*methylmethanesulfonamide;
1-((3-chlorophenethyl)amino)-3-(4-(methylsulfonyl)phenoxy)propan-2-ol;
1-((1-(3-chlorophenyl)-2-methylpropan-2-yl)amino)-3-(4-(methylsulfonyl)phenoxy)propan-2-ol;
*N*-(4-(2-hydroxy-3-((2-methoxyphenethyl)amino)propoxy)phenyl)-N-methylmethanesulfonamide;
*N*-(4-(3-((3-cyanophenethyl)amino)-2-hydroxypropoxy)phenyl)-*N*-methylmethanesulfonamide;
*N*-(4-(3-((2-ethylphenethyl)amino)-2-hydroxypropoxy)phenyl)-*N*-methylmethanesulfonamide;
*N*-(4-(3-((2-(difluoromethoxy)phenethyl)amino)-2-hydroxypropoxy)phenyl)-*N-*methylmethanesulfonamide;
1-((2-(3-chlorophenyl)-1-cyclopropylethyl)amino)-3-(4-(methylsulfonyl)phenoxy)propan-2-ol;
*N*-(4-(3-((3-chlorophenethyl)amino)-2-hydroxy-2-methylpropoxy)phenyl)-N-methylmethanesulfonamide;
*N*-(4-((3-((3-chlorophenethyl)amino)-2-hydroxypropyl)amino)phenyl)-N-methylmethanesulfonamide;
1-[2-(3-chlorophenyl)ethyl]-3-{[4-(2-methoxyethanesulfonyl)phenoxy]methyl}-4-methylpyrrolidine;
1-[2-(3-chlorophenyl)ethyl]-3-{[4-(2-methoxyethanesulfonyl)phenoxy]methyl}-4-methylpyrrolidine;
1-[2-(3-chlorophenyl)ethyl]-3-{[4-(2-methoxyethanesulfonyl)phenoxy]methyl}-4-methylpyrrolidine;
1-[2-(3-chlorophenyl)ethyl]-4-[(4-methanesulfonylphenoxy)methyl]-3-methylpyrrolidin-3-ol;
1-[2-(3-chlorophenyl)ethyl]-4-[(4-methanesulfonylphenoxy)methyl]-3-methylpyrrolidin-3-ol;
1-[2-(3-chlorophenyl)ethyl]-5-[(4-methanesulfonylphenoxy)methyl]-2-methylpiperidine;
1-[2-(3-chlorophenyl)ethyl]-3-[(4-methanesulfonylphenoxy)methyl]-4-methylpyrrolidine;
1-[2-(3-chlorophenyl)ethyl]-3-[(4-methanesulfonylphenoxy)methyl]-4-methylpyrrolidine;
1-[2-(3-chlorophenyl)ethyl]-3-[(4-methanesulfonylphenoxy)methyl]-4-methylpyrrolidine;
1-[2-(3-chlorophenyl)ethyl]-4-[(4-methanesulfonylphenoxy)methyl]-3-methylpyrrolidin-3-ol;
2-[4-({1-[2-(3-chlorophenyl)ethyl]-4-methylpyrrolidin-3-yl}methoxy)benzenesulfonyl]ethan-1-ol;
1-[2-(3-chlorophenyl)ethyl]-4-[(4-methanesulfonylphenoxy)methyl]-3-methylpyrrolidin-3-ol;
2-[4-({1-[2-(3-chlorophenyl)ethyl]-4-methylpyrrolidin-3-yl}methoxy)benzenesulfonyl]ethan-1-ol;
1-[2-(3-chlorophenyl)ethyl]-4-[(4-methanesulfonylphenoxy)methyl]-3-methylpyrrolidin-3-ol;
1-[2-(3-chlorophenyl)ethyl]-4-[(4-methanesulfonylphenoxy)methyl]-3-methylpyrrolidin-3-ol;
1-[2-(3-chlorophenyl)ethyl]-5-[(4-methanesulfonylphenoxy)methyl]-2-methylpiperazine;
1-[2-(3-chlorophenyl)ethyl]-3-[(4-methanesulfonylphenoxy)methyl]-4-methylpyrrolidine;
1-[2-(3-chlorophenyl)ethyl]-5-[(4-methanesulfonylphenoxy)methyl]-2-methylpiperazine;
1-[2-(3-chlorophenyl)ethyl]-5-[(4-methanesulfonylphenoxy)methyl]-2-methylpiperazine;
1-[2-(3-chlorophenyl)ethyl]-3-[(4-methanesulfonylphenoxy)methyl]-4-methylpyrrolidine;
1-[2-(3-chlorophenyl)ethyl]-3-[(4-methanesulfonylphenoxy)methyl]-4-methylpyrrolidine;
1-[2-(3-chlorophenyl)ethyl]-4-[(4-methanesulfonylphenoxy)methyl]-2-methylpyrrolidine;
1-[2-(3,5-dichlorophenyl)ethyl]-3-[(4-methanesulfonylphenoxy)methyl]piperazine;
1-[2-(5-chloro-2-methylphenyl)ethyl]-3-[(4-methanesulfonylphenoxy)methyl]piperazine;
1-[2-(3-chlorophenyl)ethyl]-3-{[4-(3-methanesulfonylpropanesulfonyl)phenoxy]methyl}piperazine;
3-(2-{3-[(4-methanesulfonylphenoxy)methyl]-4-methylpyrrolidin-1-yl}ethyl)benzonitrile;
3-(2-{4-[(4-methanesulfonylphenoxy)methyl]-2-methylpyrrolidin-1-yl}ethyl)benzonitrile;
3-(2-{4-[(4-methanesulfonylphenoxy)methyl]-2-methylpyrrolidin-1-yl}ethyl)benzonitrile;
3-(2-{4-[(4-methanesulfonylphenoxy)methyl]-2-methylpyrrolidin-1-yl}ethyl)benzonitrile;
1-[2-(3-chlorophenyl)ethyl]-3-{[4-(3-methanesulfonylpropanesulfonyl)phenoxy]methyl}-4-methylpyrrolidine;
3-(2-{4-[(4-methanesulfonylphenoxy)methyl]-2-methylpyrrolidin-1-yl}ethyl)benzonitrile;
3-(2-{4-[(4-methanesulfonylphenoxy)methyl]-2-methylpyrrolidin-1-yl}ethyl)benzonitrile;
3-(2-{4-[(4-methanesulfonylphenoxy)methyl]-2-methylpyrrolidin-1-yl}ethyl)benzonitrile;
1-[2-(3-chlorophenyl)ethyl]-5-[(4-methanesulfonylphenoxy)methyl]-2-methylpiperidine;
3-chloro-5-(1-hydroxy-2-f3-[(4-methanesulfonylphenoxy)methyl]-4-methylpyrrolidin-1-yl}ethyl)benzonitrile;
1-(3-chlorophenyl)-2-{3-[(4-methanesulfonylphenoxy)methyl]-4-methylpyrrolidin-1-yl}ethan-1-ol;
1-[2-(3-chlorophenyl)ethyl]-3-{[4-(3-methanesulfonylpropanesulfonyl)phenoxy]methyl}-4-methylpyrrolidine;
3-chloro-5-(2-{3-[(4-methanesulfonylphenoxy)methyl]piperazin-1-yl}ethyl)benzonitrile;
3-chloro-5-(2-{3-[(4-methanesulfonylphenoxy)methyl]-4-methylpyrrolidin-1-yl}ethyl)benzonitrile;
1-[2-(3-chlorophenyl)ethyl]-3-{[4-(3-methanesulfonylpropanesulfonyl)phenoxy]methyl}-4-methylpyrrolidine;
3-chloro-5-(1-hydroxy-2-{3-[(4-methanesulfonylphenoxy)methyl]-4-methylpyrrolidin-1-yl}ethyl)benzonitrile;
3-chloro-5-(1-hydroxy-2-{3-[(4-methanesulfonylphenoxy)methyl]-4-methylpyrrolidin-1-yl}ethyl)benzonitrile;
1-(3-chlorophenyl)-2-{3-[(4-methanesulfonylphenoxy)methyl]-4-methylpyrrolidin-1-yl}ethan-1-ol;
1-(3-chlorophenyl)-2-{3-[(4-methanesulfonylphenoxy)methyl]-4-methylpyrrolidin-1-yl}ethan-1-ol;
3-chloro-5-(2-{4-[(4-methanesulfonylphenoxy)methyl]-2-methylpyrrolidin-1-yl}ethyl)benzonitrile;
3-chloro-5-(1-hydroxy-2-{4-[(4-methanesulfonylphenoxy)methyl]-2-methylpyrrolidin-1-yl}ethyl)benzonitrile;
3-chloro-5-(1-hydroxy-2-{4-[(4-methanesulfonylphenoxy)methyl]-2-methylpyrrolidin-1-yl}ethyl)benzonitrile;
3-chloro-5-(2-{4-[(4-methanesulfonylphenoxy)methyl]-2-methylpyrrolidin-1-yl}ethyl)benzonitrile;
3-{[4-(azetidine-3-sulfonyl)phenoxy]methyl}-1-[2-(3-chlorophenyl)ethyl]piperazine;
3-chloro-5-[2-(4-{[4-(3-methanesulfonylpropanesulfonyl)phenoxy]methyl}-2-methylpyrrolidin-1-yl)ethyl]benzonitrile;
3-chloro-5-[2-(4-{[4-(2-hydroxyethanesulfonyl)phenoxy]methyl}-2-methylpyrrolidin-1-yl)ethyl]benzonitrile;
3-chloro-5-[2-(3-{[4-(2-hydroxyethanesulfonyl)phenoxy]methyl}-4-methylpyrrolidin-1-yl)ethyl]benzonitrile;
3-fluoro-5-(2-{3-[(4-methanesulfonylphenoxy)methyl]-4-methylpyrrolidin-1-yl}ethyl)benzonitrile;
3-[(4-methanesulfonylphenoxy)methyl]-4-methyl-1-{2-[3-(pentafluoro-lambda6-sulfanyl)phenyl]ethyl } pyrrolidine;
3-chloro-5-[2-(3-{[4-(1-hydroxy-2-methylpropane-2-sulfonyl)phenoxy]methyl}-4-methylpyrrolidin-1-yl)ethyl]benzonitrile;
3-chloro-5-(2-{4-[(4-methanesulfonylphenoxy)methyl]-2-methylpyrrolidin-1-yl}ethyl)benzonitrile;
3-chloro-5-(2-{4-[(4-methanesulfonylphenoxy)methyl]-2-methylpyrrolidin-1-yl}ethyl)benzonitrile;
1-[2-(5-chloro-2-methoxyphenyl)ethyl]-3-[(4-methanesulfonylphenoxy)methyl]piperazine;
3-chloro-5-(2-{4-[(4-methanesulfonylphenoxy)methyl]-2-methylpyrrolidin-1-yl}ethyl)benzonitrile;
3-chloro-5-(2-{4-[(4-methanesulfonylphenoxy)methyl]-2-methylpyrrolidin-1-yl}ethyl)benzonitrile;
1-{2-[5-chloro-2-(difluoromethoxy)phenyl]ethyl}-3-[(4-methanesulfonylphenoxy)methyl]piperazine;
1-(3-chlorophenyl)-2-{4-[(4-methanesulfonylphenoxy)methyl]-2-methylpyrrolidin-1-yl}ethan-1-ol;
1-(3-chlorophenyl)-2-{4-[(4-methanesulfonylphenoxy)methyl]-2-methylpyrrolidin-1-yl}ethan-1-ol;
3-chloro-5-[2-(3-{[4-(2-methanesulfonylethanesulfonyl)phenoxy]methyl}-4-methylpyrrolidin-1-yl)ethyl]benzonitrile;
1-[2-(3-chloro-5-fluorophenyl)ethyl]-3-[(4-methanesulfonylphenoxy)methyl]-4-methylpyrrolidine;
3-[2-(4-{[4-(3-methanesulfonylpropanesulfonyl)phenoxy]methyl}-2-methylpyrrolidin-1-yl)ethyl]benzonitrile;
3-chloro-5-(1-{4-[(4-methanesulfonylphenoxy)methyl]-2-methylpyrrolidin-1-yl}propan-2-yl)benzonitrile;
3-[2-(3-{[4-(3-methanesulfonylpropanesulfonyl)phenoxy]methyl}-4-methylpyrrolidin-1-yl)ethyl]benzonitrile;
3-chloro-5-[2-(3-{[4-(3-methanesulfonylpropanesulfonyl)phenoxy]methyl}-4-methylpyrrolidin-1-yl)ethyl]benzonitrile;
3-chloro-5-(1-{3-[(4-methanesulfonylphenoxy)methyl]-4-methylpyrrolidin-1-yl}propan-2-yl)benzonitrile;
3-chloro-5-(1-{4-[(4-methanesulfonylphenoxy)methyl]-2-methylpyrrolidin-1-yl}propan-2-yl)benzonitrile;
1-[2-(3-chlorophenyl)ethyl]-3-{[4-(2-methanesulfonylethanesulfonyl)phenoxy]methyl}-4-methylpyrrolidine;
3-chloro-5-(1-{3-[(4-methanesulfonylphenoxy)methyl]-4-methylpyrrolidin-1-yl}propan-2-yl)benzonitrile;
5-chloro-3-(2-{3-[(4-methanesulfonylphenoxy)methyl]-4-methylpyrrolidin-1-yl}ethyl)-2-methylbenzonitrile;
5-chloro-3-(2-{4-[(4-methanesulfonylphenoxy)methyl]-2-methylpyrrolidin-1-yl}ethyl)-2-methylbenzonitrile;
3-(1-hydroxy-2-{4-[(4-methanesulfonylphenoxy)methyl]-2-methylpyrrolidin-1-yl}ethyl)benzonitrile;
3-chloro-5-(1-{3-[(4-methanesulfonylphenoxy)methyl]-4-methylpyrrolidin-1-yl}propan-2-yl)benzonitrile;
3-chloro-5-[2-(3-{[4-(1-hydroxy-2-methylpropane-2-sulfonyl)phenoxy]methyl}piperazin-1-yl)ethyl]benzonitrile;
3-(2-{3-[(4-methanesulfonylphenoxy)methyl]piperazin-1-yl}ethyl)-5-(trifluoromethyl)benzonitrile;
3-(2-{3-[(4-methanesulfonylphenoxy)methyl]-4-methylpyrrolidin-1-yl}ethyl)-5-methylbenzonitrile;
3-[2-(3-{[4-(azetidine-3-sulfonyl)phenoxy]methyl}-4-methylpyrrolidin-1-yl)ethyl]benzonitrile;
3-chloro-5-[2-(3-{[4-(2-hydroxyethanesulfonyl)phenoxy]methyl}piperazin-1-yl)ethyl]benzonitrile;
5-(2-{4-[(4-methanesulfonylphenoxy)methyl]-2-methylpyrrolidin-1-yl}ethyl)benzene-1,3-dicarbonitrile;
5-(2-{3-[(4-methanesulfonylphenoxy)methyl]-4-methylpyrrolidin-1-yl}ethyl)benzene-1,3-dicarbonitrile;
3-[2-(4-{[4-(azetidine-3-sulfonyl)phenoxy]methyl}-2-methylpyrrolidin-1-yl)ethyl]-5-chlorobenzonitrile;
3-[2-(3-{[4-(azetidine-3-sulfonyl)phenoxy]methyl}-4-methylpyrrolidin-1-yl)ethyl]-5-chlorobenzonitrile;
3-bromo-5-(2-{3-[(4-methanesulfonylphenoxy)methyl]-4-methylpyrrolidin-1-yl}ethyl)benzonitrile;
3-[2-(4-{[4-(azetidine-3-sulfonyl)phenoxy]methyl}-2-methylpyrrolidin-1-yl)ethyl]benzonitrile;
3-(1-hydroxy-2-{4-[(4-methanesulfonylphenoxy)methyl]-2-methylpyrrolidin-1-yl}ethyl)benzonitrile;
3-{2-[3-methyl-4-({4-[methyl(methylimino)oxo-lambda6-sulfanyl]phenoxy}methyl)pyrrolidin-1-yl]ethyl}benzonitrile;
3-(1-hydroxy-2-{3-[(4-methanesulfonylphenoxy)methyl]-4-methylpyrrolidin-1-yl}ethyl)benzonitrile;
3-(1-hydroxy-2-{3-[(4-methanesulfonylphenoxy)methyl]-4-methylpyrrolidin-1-yl}ethyl)benzonitrile;
3-(1-hydroxy-2-{3-[(4-methanesulfonylphenoxy)methyl]-4-methylpyrrolidin-1-yl}ethyl)benzonitrile;
3-{2-[3-methyl-4-({4-[methyl(methylimino)oxo-lambda6-sulfanyl]phenoxy}methyl)pyrrolidin-1-yl]ethyl}benzonitrile;
3-{2-[3-methyl-4-({4-[methyl(methylimino)oxo-lambda6-sulfanyl]phenoxy}methyl)pyrrolidin-1-yl]ethyl}benzonitrile;
3-{2-[2-methyl-4-({4-[methyl(methylimino)oxo-lambda6-sulfanyl]phenoxy}methyl)pyrrolidin-1-yl]ethyl}benzonitrile;
5-(1-hydroxy-2-{3-[(4-methanesulfonylphenoxy)methyl]-4-methylpyrrolidin-1-yl}ethyl)benzene-1,3-dicarbonitrile;
5-(1-hydroxy-2-{3-[(4-methanesulfonylphenoxy)methyl]-4-methylpyrrolidin-1-yl}ethyl)benzene-1,3-dicarbonitrile;
5-(1-hydroxy-2-{4-[(4-methanesulfonylphenoxy)methyl]-2-methylpyrrolidin-1-yl}ethyl)benzene-1,3-dicarbonitrile;
5-(1-hydroxy-2-{4-[(4-methanesulfonylphenoxy)methyl]-2-methylpyrrolidin-1-yl}ethyl)benzene-1,3-dicarbonitrile;
3-{2-[2-methyl-4-({4-[methyl(methylimino)oxo-lambda6-sulfanyl]phenoxy}methyl)pyrrolidin-1-yl]ethyl}benzonitrile;
3-{2-[2-methyl-4-({4-[methyl(methylimino)oxo-lambda6-sulfanyl]phenoxy}methyl)pyrrolidin-1-yl]ethyl}benzonitrile;
5-(1-hydroxy-2-{3-[(4-methanesulfonylphenoxy)methyl]-4-methylpyrrolidin-1-yl}ethyl)benzene-1,3-dicarbonitrile;
3-chloro-5-{2-[3-({4-[methyl(methylimino)oxo-lambda6-sulfanyl]phenoxy}methyl)piperazin-1-yl]ethyl}benzonitrile;
3-chloro-5-{2-[3-({4-[methyl(methylimino)oxo-lambda6-sulfanyl]phenoxy}methyl)piperazin-1-yl]ethyl}benzonitrile; and
3-chloro-5-{2-[3-({4-[methyl(methylimino)oxo-lambda6-sulfanyl]phenoxy}methyl)piperazin-1-yl]ethyl}benzonitrile,
or a stereoisomer or tautomer thereof, or a pharmaceutically acceptable salt of any of the foregoing. Isotopically labeled forms of any of the foregoing are also embraced, such as deuterated or tritiated forms (wherein at least one hydrogen is replaced by at least one deuterium or tritium) of any of the specific compounds detailed herein. Mixtures of any of the foregoing are also embraced and described. Prodrugs of any of the foregoing are also embraced herein.

In some embodiments, a compound of formula (A) or (A') is selected from the group consisting of:
1-(3-chlorophenethyl)-3-((4-(methylsulfonyl)phenoxy)methyl)azepane;
(R)-1-(3-chlorophenethyl)-3-((4-(methylsulfonyl)phenoxy)methyl)azepane;
(S)-1-(3-chlorophenethyl)-3-((4-(methylsulfonyl)phenoxy)methyl)azepane;
1-(3-chlorophenethyl)-3-((4-(methylsulfonyl)phenoxy)methyl)piperidin-3-ol;
(R)-1-(3-chlorophenethyl)-3-((4-(methylsulfonyl)phenoxy)methyl)piperidin-3-ol;
(S)-1-(3-chlorophenethyl)-3-((4-(methylsulfonyl)phenoxy)methyl)piperidin-3-ol;
N-(4-((1-(3-chlorophenethyl)piperidin-3-yl)methoxy)phenyl)-N-methylmethanesulfonamide;
(S)-N-(4-((1-(3-chlorophenethyl)piperidin-3-yl)methoxy)phenyl)-N-methylmethanesulfonamide;
(R)-N-(4-((1-(3-chlorophenethyl)piperidin-3-yl)methoxy)phenyl)-N-methylmethanesulfonamide;
N-(4-((4-(3-chlorophenethyl)-1,4-oxazepan-2-yl)methoxy)phenyl)-N-methylmethanesulfonamide;
(R)-N-(4-((4-(3-chlorophenethyl)-1,4-oxazepan-2-yl)methoxy)phenyl)-N-methylmethanesulfonamide;
(S)-N-(4-((4-(3-chlorophenethyl)-1,4-oxazepan-2-yl)methoxy)phenyl)-N-methylmethanesulfonamide;
(S)-1-(3-chlorophenethyl)-3-((4-(methylsulfonyl)phenoxy)methyl)piperazine;
(R)-1-(3-chlorophenethyl)-3-((4-(methylsulfonyl)phenoxy)methyl)piperazine;
(R)-1-(3-chlorophenethyl)-3-((4-(methylsulfonyl)phenoxy)methyl)piperidine;
(S)-1-(3-chlorophenethyl)-3-((4-(methylsulfonyl)phenoxy)methyl)piperidine;
1-(3-chlorophenyl)-2-((S)-3-((4-(methylsulfonyl)phenoxy)methyl)piperidin-1-yl)ethan-1-ol;
(R)-1-(3-chlorophenyl)-2-((S)-3-((4-(methylsulfonyl)phenoxy)methyl)piperidin-1-yl)ethan-1-ol;
(S)-1-(3-chlorophenyl)-2-((S)-3-((4-(methylsulfonyl)phenoxy)methyl)piperidin-1-yl)ethan-1-ol;
1-(3-chlorophenyl)-2-((R)-3-((4-(methylsulfonyl)phenoxy)methyl)piperidin-1-yl)ethanol;
(R)-1-(3-chlorophenyl)-2-((R)-3-((4-(methylsuifonyl)phenoxy)methyl)piperidin-1-yl)ethanol;
(S)-1-(3-chlorophenyl)-2-((R)-3-((4-(methylsulfonyl)phenoxy)methyl)piperidin-1-yl)ethanol;
(S)-1-((R)-2-(3-chlorophenyl)-2-methoxyethyl)-3-((4-(methylsulfonyl)phenoxy)methyl)piperidine;
(S)-1-((S)-2-(3-chlorophenyl)-2-methoxyethyl)-3-((4-(methylsulfonyl)phenoxy)methyl)piperidine;
(R)-1-((R)-2-(3-chlorophenyl)-2-methoxyethyl)-3-((4-(methylsulfonyl)phenoxy)methyl)piperidine;
(R)-1-((S)-2-(3-chlorophenyl)-2-methoxyethyl)-3-((4-(methylsulfonyl)phenoxy)methyl)piperidine;
rac-trans or cis-1-(3-chlorophenethyl)-3-((4-(methylsulfonyl)phenoxy)methyl)piperidin-4-ol;
rac-cis or trans-1-(3-chlorophenethyl)-3-((4-(methylsulfonyl)phenoxy)methyl)piperidin-4-ol;
(3S,4R)-1-(3-chlorophenethyl)-3-((4-(methylsulfonyl)phenoxy)methyl)piperidin-4-ol;
(3R,4R)-1-(3-chlorophenethyl)-3-((4-(methylsulfonyl)phenoxy)methyl)piperidin-4-ol;
(3R,4S)-1-(3-chlorophenethyl)-3-((4-(methylsulfonyl)phenoxy)methyl)piperidin-4-ol;
(3S,4S)-1-(3-chlorophenethyl)-3-((4-(methylsulfonyl)phenoxy)methyl)piperidin-4-ol;
rac-trans-1-(3-chlorophenethyl)-4-methoxy-3-((4-(methylsulfonyl)phenoxy) methyl)piperidine;
rac-cis-1-(3-chlorophenethyl)-4-methoxy-3-((4-(methylsulfonyl)phenoxy) methyl)piperidine;
(3S,4R)-1-(3-chlorophenethyl)-4-methoxy-3-((4-(methylsulfonyl)phenoxy)methyl)piperidine;
(3R,4R)-1-(3-chlorophenethyl)-4-methoxy-3-((4-(methylsulfonyl)phenoxy)methyl)piperidine;
(3R,4S)-1-(3-chlorophenethyl)-4-methoxy-3-((4-(methylsulfonyl)phenoxy)methyl)piperidine;
(3S,4S)-1-(3-chlorophenethyl)-4-methoxy-3-((4-(methylsulfonyl)phenoxy)methyl)piperidine;
1-(3-chlorophenethyl)-3-(4-(methylsulfonyl)phenethyl)piperidine;
(S)-1-(3-chlorophenethyl)-3-(4-(methylsulfonyl)phenethyl)piperidine;
(R)-1-(3-chlorophenethyl)-3-(4-(methylsulfonyl)phenethyl)piperidine;
1-(3-chlorophenethyl)-2,2-dimethyl-4-((4-(methylsulfonyl)phenoxy) methyl) pyrrolidine;
(S)-1-(3-chlorophenethyl)-2,2-dimethyl-4-((4-(methylsulfonyl)phenoxy) methyl) pyrrolidine;
(R)-1-(3-chlorophenethyl)-2,2-dimethyl-4-((4-(methylsulfonyl)phenoxy)methyl)pyrrolidine;
rac-trans-N-(4-((1-(3-chlorophenethyl)-4-methylpyrrolidin-3-yl)methoxy)phenyl)-N-methylmethanesulfonamide;
N-(4-(((3S,4S)-1-(3-chlorophenethyl)-4-methylpyrrolidin-3-yl)methoxy)phenyl)-N-methylmethanesulfonamide;
N-(4-(((3R,4R)-1-(3-chlorophenethyl)-4-methylpyrrolidin-3-yl)methoxy)phenyl)-N-methylmethanesulfonamide;
trans-N-(4-(3-((3-chlorophenethyl)amino)cyclobutoxy)phenyl)-N-methylmethanesulfonamide; (S)-N-(4-((1-(3-chlorophenethyl)pyrrolidin-3-yl)methoxy)phenyl)-N-methylmethanesulfonamide;
(R)-N-(4-((1-(3-chlorophenethyl)pyrrolidin-3-yl)methoxy)phenyl)-N-methylmethanesulfonamide;
(S)-1-(3-chlorophenethyl)-3-((4-(methylsulfonyl)phenoxy)methyl)pyrrolidine;
rac-trans-1-(3-chlorophenethyl)-3-methyl-4-((4-(methylsulfonyl)phenoxy)methyl)pyrrolidine;
rac-trans-1-(3-chlorophenethyl)-2-methyl-4-((4-(methylsulfonyl)phenoxy)methyl)pyrrolidine;
rac-cis-1-(3-chlorophenethyl)-2-methyl-4-((4-(methylsulfonyl)phenoxy)methyl)pyrrolidine;
(2R,4S)-1-(3-chlorophenethyl)-2-methyl-4-((4-(methylsulfonyl)phenoxy)methyl)pyrrolidine;
(2R,4R)-1-(3-chlorophenethyl)-2-methyl-4-((4-(methylsulfonyl)phenoxy)methyl)pyrrolidine;
(2S,4R)-1-(3-chlorophenethyl)-2-methyl-4-((4-(methylsulfonyl)phenoxy)methyl)pyrrolidine;
(2S,4S)-1-(3-chlorophenethyl)-2-methyl-4-((4-(methylsulfonyl)phenoxy)methyl)pyrrolidine;
(S)-N-(4-(3-((3-chlorophenethyl)(cyclopropylmethyl)amino)-2-hydroxypropoxy)phenyl)-N-methylmethanesulfonamide;
(S)-N-(4-(3-((3-chlorophenethyl)(ethyl)amino)-2-hydroxypropoxy)phenyl)-N-methylmethanesulfonamide;
(R)-N-(4-(3-((3-chlorophenethyl)amino)-2-methylpropoxy)phenyl)-N-methylmethanesulfonamide;
(2S)-1-(2-(3-chlorobenzyl)piperidin-1-yl)-3-(4-(methylsulfonyl)phenoxy)propan-2-ol;
(S)-1-((S)-2-(3-chlorobenzyl)piperidin-1-yl)-3-(4-(methylsulfonyl)phenoxy)propan-2-ol;
(S)-1-((R)-2-(3-chlorobenzyl)piperidin-1-yl)-3-(4-(methylsulfonyl)phenoxy)propan-2-ol;
N-(4-((S)-3-((trans-3-(3-chlorophenyl)cyclobutyl)amino)-2-hydroxypropoxy)phenyl)-N-methylmethanesulfonamide;
(S)-1-((trans-3-(3-chlorophenyl)cyclobutyl)amino)-3-(4-(methylsulfonyl)phenoxy)propan-2-ol;
(R)-1-((trans-3-(3-chlorophenyl)cyclobutyl)amino)-3-(4-(methylsulfonyl)phenoxy)propan-2-ol;
N-(4-((2S)-3-(3-(3-chlorophenyl)-2,2-dimethylpyrrolidin-1-yl)-2-hydroxypropoxy)phenyl)-N-methylmethanesulfonamide;
N-(4-((S)-3-((S)-3-(3-chlorophenyl)-2,2-dimethylpyrrolidin-1-yl)-2-hydroxypropoxy)phenyl)-N-methylmethanesulfonamide;
N-(4-((S)-3-((R)-3-(3-chlorophenyl)-2,2-dimethylpyrrolidin-1-yl)-2-hydroxypropoxy)phenyl)-N-methylmethanesulfonamide;
N-(4-((2R)-3-(3-(3-chlorophenyl)-2,2-dimethylpyrrolidin-1-yl)-2-hydroxypropoxy)phenyl)-N-methylmethanesulfonamide;
N-(4-((R)-3-((S)-3-(3-chlorophenyl)-2,2-dimethylpyrrolidin-1-yl)-2-hydroxypropoxy)phenyl)-N-methylmethanesulfonamide;
N-(4-((R)-3-((R)-3-(3-chlorophenyl)-2,2-dimethylpyrrolidin-1-yl)-2-hydroxypropoxy)phenyl)-N-methylmethanesulfonamide;
N-(4-((2S)-3-(3-(3-chlorophenyl)pyrrolidin-1-yl)-2-hydroxypropoxy)phenyl)-N-methylmethanesulfonamide;
N-(4-((S)-3-((S)-3-(3-chlorophenyl)pyrrolidin-1-yl)-2-hydroxypropoxy)phenyl)-N-methylmethanesulfonamide;
N-(4-((S)-3-((R)-3-(3-chlorophenyl)pyrrolidin-1-yl)-2-hydroxypropoxy)phenyl)-N-methylmethanesulfonamide;
N-(4-((2R)-3-(3-(3-chlorophenyl)pyrrolidin-1-yl)-2-hydroxypropoxy)phenyl)-N-methylmethanesulfonamide;
N-(4-((R)-3-((S)-3-(3-chlorophenyl)pyrrolidin-1-yl)-2-hydroxypropoxy)phenyl)-N-methylmethanesulfonamide;
N-(4-((R)-3-((R)-3-(3-chlorophenyl)pyrrolidin-1-yl)-2-hydroxypropoxy)phenyl)-N-methylmethanesulfonamide;
N-(4-((2S)-3-(2-(3-chlorophenyl)morpholino)-2-hydroxypropoxy)phenyl)-N-methylmethanesulfonamide;
N-(4-((S)-3-((S)-2-(3-chlorophenyl)morpholino)-2-hydroxypropoxy)phenyl)-N-methylmethanesulfonamide;
N-(4-((S)-3-((R)-2-(3-chlorophenyl)morpholino)-2-hydroxypropoxy)phenyl)-N-methylmethanesulfonamide;
N-(4-((2R)-3-(2-(3-chlorophenyl)morpholino)-2-hydroxypropoxy)phenyl)-N-methylmethanesulfonamide;
N-(4-((R)-3-((R)-2-(3-chlorophenyl)morpholino)-2-hydroxypropoxy)phenyl)-N-methylmethanesulfonamide;
N-(4-((R)-3-((S)-2-(3-chlorophenyl)morpholino)-2-hydroxypropoxy)phenyl)-N-methylmethanesulfonamide;
(S)-N-(4-(3-((3-chlorophenethyl)amino)-2-hydroxypropoxy)phenyl)-N-methylmethanesulfonamide;
(R)-N-(4-(3-((3-chlorophenethyl)amino)-2-hydroxypropoxy)phenyl)-N-methylmethanesulfonamide;
N-(4-((R)-3-(((S)-1-(3-chlorophenyl)propan-2-yl)amino)-2-hydroxypropoxy)phenyl)-N-methylmethanesulfonamide;
N-(4-((R)-3-(((R)-1-(3-chlorophenyl)propan-2-yl)amino)-2-hydroxypropoxy)phenyl)-N-methylmethanesulfonamide;
(S)-N-(4-(3-((2,5-dichlorophenethyl)amino)-2-hydroxypropoxy)phenyl)-N-methylmethanesulfonamide;
(S)-N-(4-(3-((3,5-dichlorophenethyl)amino)-2-hydroxypropoxy)phenyl)-N-methylmethanesulfonamide;
N-(4-((2R)-3-((3-(3-chlorobenzyl)tetrahydrofuran-3-yl)amino)-2-hydroxypropoxy)phenyl)-N-methylmethanesulfonamide;
(S)-1-((3-chlorophenethyl)amino)-3-(4-(methylsulfonyl)phenoxy)propan-2-ol;
(R)-1-((3-chlorophenethyl)amino)-3-(4-(methylsulfonyl)phenoxy)propan-2-ol;
N-(4-((S)-3-(((S)-1-(3-chlorophenyl)propan-2-yl)amino)-2-hydroxypropoxy)phenyl)-N-methylmethanesulfonamide;
N-(4-((S)-3-(((R)-1-(3-chlorophenyl)propan-2-yl)amino)-2-hydroxypropoxy)phenyl)-N-methylmethanesulfonamide;
(R)-1-((1-(3-chlorophenyl)-2-methylpropan-2-yl)amino)-3-(4-(methylsulfonyl)phenoxy)propan-2-ol;
(S)-N-(4-(2-hydroxy-3-((2-methoxyphenethyl)amino)propoxy)phenyl)-N-methylmethanesulfonamide;
(S)-N-(4-(3-((3-cyanophenethyl)amino)-2-hydroxypropoxy)phenyl)-N-methylmethanesulfonamide;
(S)-N-(4-(3-((2-ethylphenethyl)amino)-2-hydroxypropoxy)phenyl)-N-methylmethanesulfonamide;
(S)-N-(4-(3-((2-(difluoromethoxy)phenethyl)amino)-2-hydroxypropoxy)phenyl)-N-methylmethanesulfonamide;
(2R)-1-((2-(3-chlorophenyl)-1-cyclopropylethyl)amino)-3-(4-(methylsulfonyl)phenoxy)propan-2-ol;
(R)-N-(4-(3-((3-chlorophenethyl)amino)-2-hydroxy-2-methylpropoxy)phenyl)-N-methylmethanesulfonamide;
(S)-N-(4-(3-((3-chlorophenethyl)amino)-2-hydroxy-2-methylpropoxy)phenyl)-N-methylmethanesulfonamide;
N-(4-((3-((3-chlorophenethyl)amino)-2-hydroxypropyl)amino)phenyl)-N-methylmethanesulfonamide;
(R)-N-(4-((3-((3-chlorophenethyl)amino)-2-hydroxypropyl)amino)phenyl)-N-methylmethanesulfonamide;
(3S,4S)-1-[2-(3-chlorophenyl)ethyl]-3-{[4-(2-methanesulfonylethanesulfonyl)phenoxy]methyl}-4-methylpyrrolidine;
3-chloro-5-{2-[(3S,4S)-3-{[4-(2-methanesulfonylethanesulfonyl)phenoxy]methyl}-4-methylpyrrolidin-1-yl]ethyl}benzonitrile;
rac-trans-1-[2-(3-chlorophenyl)ethyl]-3-{[4-(2-methoxyethanesulfonyl)phenoxy]methyl}-4-methylpyrrolidine;
(3R,4R)-1-[2-(3-chlorophenyl)ethyl]-3-{[4-(2-methoxyethanesulfonyl)phenoxy]methyl}-4-methylpyrrolidine;
(3S,4S)-1-[2-(3-chlorophenyl)ethyl]-3-{[4-(2-methoxyethanesulfonyl)phenoxy]methyl}-4-methylpyrrolidine;
2-(4-{[(3R,4R)-1-[2-(3-chlorophenyl)ethyl]-4-methylpyrrolidin-3-yl]methoxy}benzenesulfonyl)ethan-1-ol;
2-(4-{[(3S,4S)-1-[2-(3-chlorophenyl)ethyl]-4-methylpyrrolidin-3-yl]methoxy}benzenesulfonyl)ethan-1-ol;
3-chloro-5-[(1S)-1-hydroxy-2-[(2R,4S)-4-[(4-methanesulfonylphenoxy)methyl]-2-methylpyrrolidin-1-yl]ethyl]benzonitrile;
3-chloro-5-[(1R)-1-hydroxy-2-[(2R,4S)-4-[(4-methanesulfonylphenoxy)methyl]-2-methylpyrrolidin-1-yl]ethyl]benzonitrile;
3-[(1R)-1-hydroxy-2-[(2R,4S)-4-[(4-methanesulfonylphenoxy)methyl]-2-methylpyrrolidin-1-yl]ethyl]benzonitrile;
3-[(1S)-1-hydroxy-2-[(2R,4S)-4-[(4-methanesulfonylphenoxy)methyl]-2-methylpyrrolidin-1-yl]ethyl]benzonitrile;
5-[(1R)-1-hydroxy-2-[(2R,4S)-4-[(4-methanesulfonylphenoxy)methyl]-2-methylpyrrolidin-1-yl]ethyl]benzene-1,3-dicarbonitrile;
5-[(1S)-1-hydroxy-2-[(2R,4S)-4-[(4-methanesulfonylphenoxy)methyl]-2-methylpyrrolidin-1-yl]ethyl]benzene-1,3-dicarbonitrile;
3-chloro-5-{1-hydroxy-2-[(3S,4S)-3-[(4-methanesulfonylphenoxy)methyl]-4-methylpyrrolidin-1-yl]ethyl}benzonitrile;
3-chloro-5-[(1S)-1-hydroxy-2-[(3S,4S)-3-[(4-methanesulfonylphenoxy)methyl]-4-methylpyrrolidin-1-yl]ethyl]benzonitrile;
3-chloro-5-[(1R)-1-hydroxy-2-[(3S,4S)-3-[(4-methanesulfonylphenoxy)methyl]-4-methylpyrrolidin-1-yl]ethyl]benzonitrile;
1-(3-chlorophenyl)-2-((3S,4S)-3-methyl-4-((4-(methylsulfonyl)phenoxy)methyl)pyrrolidin-1-yl)ethan-1-ol;
(1R)-1-(3-chlorophenyl)-2-[(3S,4S)-3-[(4-methanesulfonylphenoxy)methyl]-4-methylpyrrolidin-1-yl]ethan-1-ol;
(1S)-1-(3-chlorophenyl)-2-[(3S,4S)-3-[(4-methanesulfonylphenoxy)methyl]-4-methylpyrrolidin-1-yl]ethan-1-ol;
3-[1-hydroxy-2-[(3 S,4S)-3-[(4-methanesulfonylphenoxy)methyl]-4-methylpyrrolidin-1-yl]ethyl]benzonitrile;
3-[(1S)-1-hydroxy-2-[(3S,4S)-3-[(4-methanesulfonylphenoxy)methyl]-4-methylpyrrolidin-1-yl]ethyl]benzonitrile;
3-[(1R)-1-hydroxy-2-[(3S,4S)-3-[(4-methanesulfonylphenoxy)methyl]-4-methylpyrrolidin-1-yl]ethyl]benzonitrile;
5-{1-hydroxy-2-[(3S,4S)-3-[(4-methanesulfonylphenoxy)methyl]-4-methylpyrrolidin-1-yl]ethyl}benzene-1,3-dicarbonitrile;
5-[(1R)-1-hydroxy-2-[(3S,4S)-3-[(4-methanesulfonylphenoxy)methyl]-4-methylpyrrolidin-1-yl]ethyl]benzene-1,3-dicarbonitrile;
5-[(1S)-1-hydroxy-2-[(3S,4S)-3-[(4-methanesulfonylphenoxy)methyl]-4-methylpyrrolidin-1-yl]ethyl]benzene-1,3-dicarbonitrile;
5-{2-[(2R,4S)-4-[(4-methanesulfonylphenoxy)methyl]-2-methylpyrrolidin-1-yl]ethyl}benzene-1,3-dicarbonitrile;
(3S,4S)-1-[2-(3-chlorophenyl)ethyl]-3-[(4-methanesulfonylphenoxy)methyl]-4-methylpyrrolidine;
(2S,5S)-1-[2-(3-chlorophenyl)ethyl]-5-[(4-methanesulfonylphenoxy)methyl]-2-methylpiperidine;
rac-cis-3-{2-[4-[(4-methanesulfonylphenoxy)methyl]-2-methylpyrrolidin-1-yl]ethyl}benzonitrile;
rac-trans-3-{2-[4-[(4-methanesulfonylphenoxy)methyl]-2-methylpyrrolidin-1-yl]ethyl}benzonitrile;
3-{2-[(2S,4R)-4-[(4-methanesulfonylphenoxy)methyl]-2-methylpyrrolidin-1-yl]ethyl}benzonitrile;
3-{2-[(2R,4S)-4-[(4-methanesulfonylphenoxy)methyl]-2-methylpyrrolidin-1-yl]ethyl }benzonitrile;
3-{2-[(2S,4S)-4-[(4-methanesulfonylphenoxy)methyl]-2-methylpyrrolidin-1-yl]ethyl}benzonitrile;
3-{2-[(2R,4R)-4-[(4-methanesulfonylphenoxy)methyl]-2-methylpyrrolidin-1-yl]ethyl}benzonitrile;
5-chloro-3-{2-[(2R,4S)-4-[(4-methanesulfonylphenoxy)methyl]-2-methylpyrrolidin-1-yl]ethyl}-2-methylbenzonitrile;
3-chloro-5-[(2R)-1-[(2R,4S)-4-[(4-methanesulfonylphenoxy)methyl]-2-methylpyrrolidin-1-yl]propan-2-yl]benzonitrile;
3-chloro-5-[(2S)-1-[(2R,4S)-4-[(4-methanesulfonylphenoxy)methyl]-2-methylpyrrolidin-1-yl]propan-2-yl]benzonitrile;
5-chloro-3-{2-[(3S,4S)-3-[(4-methanesulfonylphenoxy)methyl]-4-methylpyrrolidin-1-yl]ethyl}-2-methylbenzonitrile;
5-{2-[(3S,4S)-3-[(4-methanesulfonylphenoxy)methyl]-4-methylpyrrolidin-1-yl]ethyl}benzene-1,3-dicarbonitrile;
3-chloro-5-[1-[(3S,4S)-3-[(4-methanesulfonylphenoxy)methyl]-4-methylpyrrolidin-1-yl]propan-2-yl]benzonitrile;
3-chloro-5-[(2R)-1-[(3S,4S)-3-[(4-methanesulfonylphenoxy)methyl]-4-methylpyrrolidin-1-yl]propan-2-yl]benzonitrile;
3-chloro-5-[(2S)-1-[(3S,4S)-3-[(4-methanesulfonylphenoxy)methyl]-4-methylpyrrolidin-1-yl]propan-2-yl]benzonitrile;
3-{2-[(3S,4S)-3-[(4-methanesulfonylphenoxy)methyl]-4-methylpyrrolidin-1-yl]ethyl}benzonitrile;
rac-cis-1-[2-(3-chlorophenyl)ethyl]-3-[(4-methanesulfonylphenoxy)methyl]-4-methylpyrrolidine;
(3R,4S)-1-[2-(3-chlorophenyl)ethyl]-3-[(4-methanesulfonylphenoxy)methyl]-4-methylpyrrolidine;
(3S,4R)-1-[2-(3-chlorophenyl)ethyl]-3-[(4-methanesulfonylphenoxy)methyl]-4-methylpyrrolidine;
(2R,4S)-1-[2-(3-chlorophenyl)ethyl]-4-[(4-methanesulfonylphenoxy)methyl]-2-methylpyrrolidine;
(3R,4R)-1-[2-(3-chlorophenyl)ethyl]-3-[(4-methanesulfonylphenoxy)methyl]-4-methylpyrrolidine;
(3S,4S)-1-[2-(3-chlorophenyl)ethyl]-3-[(4-methanesulfonylphenoxy)methyl]-4-methylpyrrolidine;
3-{2-[(2R,4S)-4-{[4-(azetidine-3-sulfonyl)phenoxy]methyl}-2-methylpyrrolidin-1-yl]ethyl}benzonitrile;
3-{2-[(2R,4S)-2-methyl-4-({4-[methyl(methylimino)oxo-λ⁶-sulfanyl]phenoxy}methyl)pyrrolidin-1-yl]ethyl}benzonitrile;
3-{2-[(2R,4S)-2-methyl-4-({4-[(R)methyl(methylimino)oxo-λ⁶-sulfanyl]phenoxy}methyl)pyrrolidin-1-yl]ethyl}benzonitrile;
3-{2-[(2R,4S)-2-methyl-4-({4-[(S)methyl(methylimino)oxo-λ⁶-sulfanyl]phenoxy}methyl)pyrrolidin-1-yl]ethyl}benzonitrile;
(2R,5S)-1-[2-(3-chlorophenyl)ethyl]-5-[(4-methanesulfonylphenoxy)methyl]-2-methylpiperidine;
3-{2-[(2R,4S)-4-{[4-(azetidine-3-sulfonyl)phenoxy]methyl}-2-methylpyrrolidin-1-yl]ethyl}-5-chlorobenzonitrile;
3-{2-[(2R,4S)-4-{[4-(3-methanesulfonylpropanesulfonyl)phenoxy]methyl}-2-methylpyrrolidin-1-yl]ethyl}benzonitrile;
(3S,4S)-3-[(4-methanesulfonylphenoxy)methyl]-4-methyl-1-{2-[3-(pentafluoro-λ⁶-sulfanyl)phenyl]ethyl}pyrrolidine;
3-chloro-5-{2-[(2R,4S)-4-{[4-(3-methanesulfonylpropanesulfonyl)phenoxy]methyl}-2-methylpyrrolidin-1-yl]ethyl}benzonitrile;
3-chloro-5-{2-[(2R,4S)-4-{[4-(2-hydroxyethanesulfonyl)phenoxy]methyl}-2-methylpyrrolidin-1-yl]ethyl}benzonitrile;
3-chloro-5-{2-[(3S,4S)-3-{[4-(1-hydroxy-2-methylpropane-2-sulfonyl)phenoxy]methyl}-4-methylpyrrolidin-1-yl]ethyl}benzonitrile;
3-chloro-5-{2-[(3S,4S)-3-{[4-(3-methanesulfonylpropanesulfonyl)phenoxy]methyl}-4-methylpyrrolidin-1-yl]ethyl}benzonitrile;
3-{2-[(3S,4S)-3-{[4-(3-methanesulfonylpropanesulfonyl)phenoxy]methyl}-4-methylpyrrolidin-1-yl]ethyl}benzonitrile;
3-{2-[(3S,4S)-3-methyl-4-({4-[methyl(methylimino)oxo-λ⁶-sulfanyl]phenoxy}methyl)pyrrolidin-1-yl]ethyl}benzonitrile;
3-{2-[(3S,4S)-3-methyl-4-({4-[(R)-methyl(methylimino)oxo-λ⁶-sulfanyl]phenoxy}methyl)pyrrolidin-1-yl]ethyl}benzonitrile;
3-{2-[(3S,4S)-3-methyl-4-({4-[(S)-methyl(methylimino)oxo-λ⁶-sulfanyl]phenoxy}methyl)pyrrolidin-1-yl]ethyl}benzonitrile;
3-{2-[(3S,4S)-3-{[4-(azetidine-3-sulfonyl)phenoxy]methyl}-4-methylpyrrolidin-1-yl]ethyl}benzonitrile;
3-{2-[(3S,4S)-3-{[4-(azetidine-3-sulfonyl)phenoxy]methyl}-4-methylpyrrolidin-1-yl]ethyl}-5-chlorobenzonitrile;
3-chloro-5-{2-[(3S,4S)-3-{[4-(2-hydroxyethanesulfonyl)phenoxy]methyl}-4-methylpyrrolidin-1-yl]ethyl}benzonitrile;
rac-trans-1-[2-(3-chlorophenyl)ethyl]-3-{ [4-(3-methanesulfonylpropanesulfonyl)phenoxy]methyl}-4-methylpyrrolidine;
(3R,4R)-1-[2-(3-chlorophenyl)ethyl]-3-{[4-(3-methanesulfonylpropanesulfonyl)phenoxy]methyl}-4-methylpyrrolidine;
(3S,4S)-1-[2-(3-chlorophenyl)ethyl]-3-{[4-(3-methanesulfonylpropanesulfonyl)phenoxy]methyl}-4-methylpyrrolidine;
(3S,4S)-1-[2-(3-chloro-5-fluorophenyl)ethyl]-3-[(4-methanesulfonylphenoxy)methyl]-4-methylpyrrolidine;
3-chloro-5-{2-[(3S)-3-{[4-(2-hydroxyethanesulfonyl)phenoxy]methyl}piperazin-1-yl]ethyl}benzonitrile;
3-chloro-5-{2-[(3S)-3-{[4-(1-hydroxy-2-methylpropane-2-sulfonyl)phenoxy]methyl}piperazin-1-yl]ethyl}benzonitrile;
(3S)-1-[2-(3-chlorophenyl)ethyl]-3-{[4-(3-methanesulfonylpropanesulfonyl)phenoxy]methyl}piperazine;
(3S)-3-{[4-(azetidine-3-sulfonyl)phenoxy]methyl}-1-[2-(3-chlorophenyl)ethyl]piperazine;
3-chloro-5-{2-[(3S)-3-({4-[methyl(methylimino)oxo-λ⁶-sulfanyl]phenoxy}methyl)piperazin-1-yl]ethyl}benzonitrile;
3-chloro-5-{2-[(3S)-3-({4-[(S)-methyl(methylimino)oxo-λ⁶-sulfanyl]phenoxy}methyl)piperazin-1-yl]ethyl}benzonitrile ;
3-chloro-5-{2-[(3S)-3-({4-[(R)-methyl(methylimino)oxo-λ⁶-sulfanyl]phenoxy}methyl)piperazin-1-yl]ethyl}benzonitrile ;
3-chloro-5-{2-[(3S)-3-[(4-methanesulfonylphenoxy)methyl]piperazin-1-yl]ethyl}benzonitrile;
(3S)-1-[2-(3,5-dichlorophenyl)ethyl]-3-[(4-methanesulfonylphenoxy)methyl]piperazine;
(3S)-1-[2-(5-chloro-2-methoxyphenyl)ethyl]-3-[(4-methanesulfonylphenoxy)methyl]piperazine;
(3S)-1-{2-[5-chloro-2-(difluoromethoxy)phenyl]ethyl}-3-[(4-methanesulfonylphenoxy)methyl]piperazine;
3-{2-[(3S)-3-[(4-methanesulfonylphenoxy)methyl]piperazin-1-yl]ethyl}-5-(trifluoromethyl)benzonitrile;
3-chloro-5-{2-[(3S,4S)-3-[(4-methanesulfonylphenoxy)methyl]-4-methylpyrrolidin-1-yl]ethyl}benzonitrile;
3-fluoro-5-{2-[(3S,4S)-3-[(4-methanesulfonylphenoxy)methyl]-4-methylpyrrolidin-1-yl]ethyl}benzonitrile;
3-bromo-5-{2-[(3S,4S)-3-[(4-methanesulfonylphenoxy)methyl]-4-methylpyrrolidin-1-yl]ethyl}benzonitrile;
(3S)-1-[2-(5-chloro-2-methylphenyl)ethyl]-3-[(4-methanesulfonylphenoxy)methyl]piperazine;
3-{2-[(3S,4S)-3-[(4-methanesulfonylphenoxy)methyl]-4-methylpyrrolidin-1-yl]ethyl}-5-methylbenzonitrile;
rac-cis-3-chloro-5-{2-[4-[(4-methanesulfonylphenoxy)methyl]-2-methylpyrrolidin-1-yl]ethyl}benzonitrile;
3-chloro-5-{2-[(2R,4S)-4-[(4-methanesulfonylphenoxy)methyl]-2-methylpyrrolidin-1-yl]ethyl}benzonitrile;
3-chloro-5-{2-[(2S,4R)-4-[(4-methanesulfonylphenoxy)methyl]-2-methylpyrrolidin-1-yl]ethyl}benzonitrile;
rac-trans-3-chloro-5-{2-[4-[(4-methanesulfonylphenoxy)methyl]-2-methylpyrrolidin-1-yl]ethyl}benzonitrile;
3-chloro-5-(2-((2R,4R)-2-methyl-4-((4-(methylsulfonyl)phenoxy)methyl)pyrrolidin-1-yl)ethyl)benzonitrile;
3-chloro-5-(2-((2S,4S)-2-methyl-4-((4-(methylsulfonyl)phenoxy)methyl)pyrrolidin-1-yl)ethyl)benzonitrile;
rac-cis-1-[2-(3-chlorophenyl)ethyl]-4-[(4-methanesulfonylphenoxy)methyl]-3-methylpyrrolidin-3-ol;
(3R,4R)-1-[2-(3-chlorophenyl)ethyl]-4-[(4-methanesulfonylphenoxy)methyl]-3-methylpyrrolidin-3-ol;
(3S,4S)-1-(3-chlorophenethyl)-3-methyl-4-((4-(methylsulfonyl)phenoxy)methyl)pyrrolidin-3-ol;
rac-trans-1-[2-(3-chlorophenyl)ethyl]-4-[(4-methanesulfonylphenoxy)methyl]-3-methylpyrrolidin-3-ol;
(3R,4S)-1-[2-(3-chlorophenyl)ethyl]-4-[(4-methanesulfonylphenoxy)methyl]-3-methylpyrrolidin-3-ol;
(3S,4R)-1-[2-(3-chlorophenyl)ethyl]-4-[(4-methanesulfonylphenoxy)methyl]-3-methylpyrrolidin-3-ol;
1-[2-(3-chlorophenyl)ethyl]-5-[(4-methanesulfonylphenoxy)methyl]-2-methylpiperazine;
rac-cis-1-[2-(3-chlorophenyl)ethyl]-5-[(4-methanesulfonylphenoxy)methyl]-2-methylpiperazine;
rac-trans-1-[2-(3-chlorophenyl)ethyl]-5-[(4-methanesulfonylphenoxy)methyl]-2-methylpiperazine;
(1R)-1-(3-chlorophenyl)-2-[(2R,4S)-4-[(4-methanesulfonylphenoxy)methyl]-2-methylpyrrolidin-1-yl]ethan-1-ol; and
(1S)-1-(3-chlorophenyl)-2-[(2R,4S)-4-[(4-methanesulfonylphenoxy)methyl]-2-methylpyrrolidin-1-yl]ethan-1-ol,
or a stereoisomer or tautomer thereof, or a pharmaceutically acceptable salt of any of the foregoing. Isotopically labeled forms of any of the foregoing are also embraced, such as deuterated or tritiated forms (wherein at least one hydrogen is replaced by at least one deuterium or tritium) of any of the specific compounds detailed herein. Mixtures of any of the foregoing are also embraced and described. Prodrugs of any of the foregoing are also embraced herein.

Compound Names included in Table 1 and in the lists in the paragraphs above were generated using ChemDraw^{®} software version 18.1.0.458 or Collaborative Drug Discovery Inc. (CDD) CDD Vault update #3.

### COMPOSITIONS

Provided herein are pharmaceutical compositions comprising one or more compounds of formula (A), or formula (A'), or any variation or embodiment thereof, as described elsewhere herein, or a stereoisomer or tautomer thereof, or a pharmaceutically acceptable salt of any of the foregoing. In some embodiments, provided herein is a pharmaceutical composition comprising (i) of formula (A), or formula (A'), or any variation or embodiment thereof, as described elsewhere herein, or a stereoisomer or tautomer thereof, or a pharmaceutically acceptable salt of any of the foregoing, and (ii) one or more pharmaceutically acceptable excipients.

Suitable pharmaceutically acceptable excipients may include, for example, fillers, diluents, sterile aqueous solutions and various organic solvents, permeation enhancers, solubilizers, and adjuvants. Various substances may be embraced by the term excipient, including without limitation any substance used as a binder, disintegrant, coating, compression/encapsulation aid, cream or lotion, lubricant, solutions for parenteral administration, materials for chewable tablets, sweetener or flavoring, suspending/gelling agent, or wet granulation agent. Examples of suitable excipients are well-known to those skilled in the art. Such compositions are prepared in a manner well known in the pharmaceutical art. *See, e.g.,* Remington's Pharmaceutical Sciences, Academic Press, 23rd ed. (2020), which is incorporated herein by reference.

The pharmaceutical compositions may be administered in either single or multiple doses. The pharmaceutical composition may be administered by various methods including, for example, oral, rectal, buccal, intranasal, and transdermal routes. In certain embodiments, the pharmaceutical composition may be administered by intra-arterial injection, intravenously, intraperitoneally, parenterally, intramuscularly, subcutaneously, orally, topically, or as an inhalant.

Compounds as described herein may be administered to individuals in a form of generally accepted oral compositions, such as tablets, coated tablets, gel capsules in a hard or in soft shell, emulsions or suspensions. Examples of carriers, which may be used for the preparation of such compositions, are lactose, corn starch or its derivatives, talc, stearate or its salts, *etc.* Acceptable carriers for gel capsules with soft shell are, for instance, plant oils, wax, fats, semisolid and liquid poly-ols, and so on. In addition, pharmaceutical formulations may contain preservatives, solubilizers, stabilizers, re-wetting agents, emulgators, sweeteners, dyes, adjusters, salts for the adjustment of osmotic pressure, buffers, coating agents or antioxidants.

The specific dose level of a compound as described herein will depend upon a variety of factors such as the age, body weight and sex of the individual as well as the route of administration and other factors. In some embodiments, a dosage is expressed as a number of milligrams of a compound described herein per kilogram of the individual's body weight (mg/kg). Dosages of between about 0.1 mg/kg and 100-150 mg/kg may be appropriate.

The compound may be administered to an individual in accordance with an effective dosing regimen for a desired period of time or duration, such as at least about one month, at least about 2 months, at least about 3 months, at least about 6 months, or at least about 12 months or longer, which in some variations may be for the duration of the individual's life.

### METHODS OF TREATMENT

The compounds provided herein may be used in a method of modulating APOL1 in a cell, comprising exposing the cell to an effective amount of a compound of formula (A), or formula (A'), or any variation or embodiment thereof, as described elsewhere herein, or a stereoisomer or tautomer thereof, or a pharmaceutically acceptable salt of any of the foregoing. The compounds provided herein may also be used in a method of modulating APOL1 in a cell, comprising exposing the cell to a composition comprising an effective amount of a compound of formula (A), or formula (A'), or any variation or embodiment thereof, as described elsewhere herein, or a stereoisomer or tautomer thereof, or a pharmaceutically acceptable salt of any of the foregoing, and one or more pharmaceutically acceptable excipients. Isotopically labeled forms of any of the foregoing are also embraced, including, but not limited to, deuterated or tritiated forms (wherein at least one hydrogen is replaced by at least one deuterium or tritium) of any of the specific compounds detailed herein.

The compounds provided herein may be used in a method of inhibiting APOL1 in a cell, comprising exposing the cell to an effective amount of a compound of formula (A), or formula (A'), or any variation or embodiment thereof, as described elsewhere herein, or a stereoisomer or tautomer thereof, or a pharmaceutically acceptable salt of any of the foregoing. The compounds provided herein may also be used in a method of inhibiting APOL1 in a cell, comprising exposing the cell to a pharmaceutical composition comprising an effective amount of a compound of formula (A), or formula (A'), or any variation or embodiment thereof, as described elsewhere herein, or a stereoisomer or tautomer thereof, or a pharmaceutically acceptable salt of any of the foregoing, and one or more pharmaceutically acceptable excipients.

The compounds provided herein may be used in a method of inhibiting APOL1 in an individual, comprising administering to the individual an effective amount of a compound of formula (A), or formula (A'), or any variation or embodiment thereof, as described elsewhere herein, or a stereoisomer or tautomer thereof, or a pharmaceutically acceptable salt of any of the foregoing. The compounds provided herein may also be used in a method of inhibiting APOL1 in an individual, comprising administering to the individual a pharmaceutical composition comprising an effective amount of a compound of formula (A), or formula (A'), or any variation or embodiment thereof, as described elsewhere herein, or a stereoisomer or tautomer thereof, or a pharmaceutically acceptable salt of any of the foregoing, and one or more pharmaceutically acceptable excipients.

The compounds provided herein may inhibit APOL1 at a concentration of less than 10 µM, less than 1 µM, less than 0.5 µM, or less than 0.1 µM. The compounds provided herein may inhibit APOL1 at a concentration of 1 to 10 µM, 0.01 to 1 µM, or 0.01 to 10 µM.

The compounds provided herein may reduce cell death caused by overexpression of APOL1. The compounds provided herein may reduce cell death caused by overexpression APOL1 at a concentration of less than 10 µM, less than 1 µM, less than 0.5 µM, or less than 0.1 µM. The compounds provided herein may reduce cell death caused by APOL1 overexpression at a concentration of 1 to 10 µM, 0.01 to 1 µM, or 0.01 to 10 µM.

The compounds provided herein may have an EC₅₀ of less than 1 µM, less than 0.5 µM, or less than 0.1 µM. The compounds provided herein may have an EC₅₀ of 1 to 10 µM, 0.01 to 1 µM, or 0.01 to 10 µM.

The compounds provided herein may have an AC₅₀ of less than 1 µM, less than 0.5 µM, or less than 0.1 µM. The compounds provided herein may have an AC₅₀ of 1 to 10 µM, 0.01 to 1 µM, or 0.01 to 10 µM. The AC₅₀ value may reflect the compound's ability to prevent calcium influx by inhibiting APOL1.

The compounds provided herein may inhibit a cation channel. The compounds of the present disclosure may inhibit a calcium channel. The compounds of the present disclosure may reduce calcium transport.

The compounds provided herein may be used in a method of treating an APOL1-mediated disease, disorder, or condition in an individual in need thereof, comprising administering to the individual a therapeutically effective amount of a compound of formula (A), or formula (A'), or any variation or embodiment thereof, as described elsewhere herein, or a stereoisomer or tautomer thereof, or a pharmaceutically acceptable salt of any of the foregoing. The compounds provided herein may also be used in a method of treating an APOL1-mediated disease, disorder, or condition in an individual in need thereof, comprising administering to the individual a pharmaceutical composition comprising a therapeutically effective amount of a compound of formula (A), or formula (A'), or any variation or embodiment thereof, as described elsewhere herein, or a stereoisomer or tautomer thereof, or a pharmaceutically acceptable salt of any of the foregoing, and one or more pharmaceutically acceptable excipients.

Provided herein is a compound of formula (A) or formula (A'), or any variation or embodiment thereof, as described elsewhere herein, or a stereoisomer or tautomer thereof, or a pharmaceutically acceptable salt of any of the foregoing for use in treating or delaying the development of a disease, disorder, or condition in an individual in need thereof selected from a kidney disease; or a disease, disorder, or condition selected from the group consisting of chronic kidney disease, focal segmental glomerulosclerosis (FSGS), hypertension-attributed kidney disease, human immunodeficiency virus-associated nephropathy (HIVAN), sickle-cell nephropathy, lupus nephritis, diabetic kidney disease, APOL1-associated nephropathy, viral nephropathy, COVID-19 associated nephropathy, preeclampsia, and sepsis. Also provided herein is a pharmaceutical composition comprising a therapeutically effective amount of a compound of formula (A), or formula (A'), or any variation or embodiment thereof, as described elsewhere herein, or a stereoisomer or tautomer thereof, or a pharmaceutically acceptable salt of any of the foregoing, and one or more pharmaceutically acceptable excipients for use in treating or delaying the development of a disease, disorder, or condition in an individual in need thereof selected from a kidney disease; or a disease, disorder, or condition selected from the group consisting of chronic kidney disease, focal segmental glomerulosclerosis (FSGS), hypertension-attributed kidney disease, human immunodeficiency virus-associated nephropathy (HIVAN), sickle-cell nephropathy, lupus nephritis, diabetic kidney disease, APOL1-associated nephropathy, viral nephropathy, COVID-19 associated nephropathy, preeclampsia, and sepsis.

In some embodiments, the disease is a chronic kidney disease. In some embodiments, the disease is hypertension-attributed kidney disease. In some embodiments, the kidney disease, disorder, or condition is an APOL1-mediated kidney disease, disorder, or condition. In some embodiments, the kidney disease, disorder, or condition is selected from the group consisting of focal segmental glomerulosclerosis (FSGS), hypertension-attributed kidney disease, viral nephropathy, COVID-19 associated nephropathy, human immunodeficiency virus-associated nephropathy (HIVAN), sickle-cell nephropathy, lupus nephritis, and diabetic kidney disease.

In some embodiments, the disorder is an APOL1-mediated disorder, such as preeclampsia and sepsis. In some embodiments, the individual is genetically predisposed to developing the APOL1-mediated disorder.

The compounds provided herein may also be used in a method of delaying development of progressive renal allograft loss in a kidney transplant recipient comprising administering to the kidney transplant recipient a therapeutically effective amount of a compound of formula (A), or formula (A'), or any variation or embodiment thereof, as described elsewhere herein, or a stereoisomer or tautomer thereof, or a pharmaceutically acceptable salt of any of the foregoing. In some embodiments, the kidney transplant recipient receives a kidney from a high-risk APOL1 genotype donor. In some embodiments, the kidney transplant recipient is administered a therapeutically effective amount of the compound for a period of time before receiving the kidney transplant. In some embodiments, the kidney transplant recipient is administered a therapeutically effective amount of the compound subsequent to receiving the kidney transplant.

The compounds provided herein may also be used in a method of treating a kidney disease, disorder, or condition in an individual in need thereof, comprising administering to the individual a therapeutically effective amount of a compound of formula (A), or formula (A'), or any variation or embodiment thereof, as described elsewhere herein, or a stereoisomer or tautomer thereof, or a pharmaceutically acceptable salt of any of the foregoing, wherein the individual has an APOL1 mutation. Also provided herein is a method of treating a kidney disease, disorder, or condition in an individual in need thereof, comprising administering to the individual a pharmaceutical composition comprising a therapeutically effective amount of a compound of formula (A), or formula (A'), or any variation or embodiment thereof, as described elsewhere herein, or a stereoisomer or tautomer thereof, or a pharmaceutically acceptable salt of any of the foregoing, and one or more pharmaceutically acceptable excipients, wherein the individual has an APOL1 mutation.

The compounds provided herein may also be used in a method of delaying the development of an APOL1-mediated disease, disorder, or condition, comprising administering a compound of formula (A), or formula (A'), or any variation or embodiment thereof, as described elsewhere herein, or a stereoisomer or tautomer thereof, or a pharmaceutically acceptable salt of any of the foregoing, to an individual who is at risk of developing an APOL1-mediated disease, disorder, or condition. In some embodiments, the APOL1-mediated disease, disorder, or condition is preeclampsia or sepsis and the individual has two APOL1 risk alleles. In some embodiments, the APOL1-mediated disease, disorder, or condition is a chronic kidney disease and the individual has any binary combination of G1 and G2 APOL1 risk alleles. In some embodiments, the chronic kidney disease is focal segmental glomerulosclerosis (FSGS), hypertension-attributed kidney disease, human immunodeficiency virus-associated nephropathy (HIVAN), hypertension-attributed kidney disease, sickle cell nephropathy, viral nephropathy, COVID-19 associated nephropathy, lupus nephritis, diabetic kidney disease, or APOL1-associated nephropathy. The compounds as provided herein may also be used in a method of delaying the development of progressive renal allograft loss in an individual who has received a kidney transplantation from a high-risk APOL1 genotype donor.

In some embodiments, the individual has a gain-of-function mutation in APOL1. In some embodiments, the individual has an APOL1 risk allele. In some embodiments, the APOL1 risk allele is a missense variant. In some embodiments, the APOL1 risk allele is a G1 variant. In some embodiments, the G1 variant is G1^{G} (p.S342 G) or G1^{M} (p.I384 M). In some embodiments, the APOL1 risk allele is the G2 variant. In some embodiments, the G2 variant is NYK388-389K. In some embodiments, the APOL1 risk variant is a mutation in the serum resistance-associated (SRA) binding domain of the APOL1 protein.

The compounds provided herein may also be used in a method of inhibiting APOL1 in an individual comprising administering to the individual a therapeutically effective amount of a compound of formula (A), or formula (A'), or any variation or embodiment thereof, as described elsewhere herein, or a stereoisomer or tautomer thereof, or a pharmaceutically acceptable salt of any of the foregoing.

The compounds provided herein may also be used in amethod of preventing kidney failure in an individual comprising administering a therapeutically effective amount of a compound of Formula (A), or formula (A') or any variation or embodiment thereof, as described elsewhere herein, or a stereoisomer or tautomer thereof, or a pharmaceutically acceptable salt of any of the foregoing to the individual. In some embodiments, the compound prevents tissue necrosis. In some embodiments, the compound prevents apoptosis. In some embodiments, the compound reduces inflammation.

In some embodiments, the compounds provided herein reduce or eliminate one or more symptoms of a kidney disease. In some embodiments, the compounds reduce nausea, vomiting, loss of appetite, fatigue and weakness, sleep problems, urinary frequency issues, muscle twinges and cramps, swelling, itching, chest pain, shortness of breath, and/or high blood pressure.

In some embodiments, the compounds provided herein reduce the rate of kidney damage and/or progression of kidney damage. In some embodiments, the compounds provided herein reduce the rate of kidney failure. In some embodiments, the compounds provided herein reverse kidney damage. In some embodiments, the compounds reduce the need for dialysis. In some embodiments, the compounds provided herein delay the need for dialysis at least one month, at least two months, at least three months, or at least one year.

In some embodiments, the compounds reduce the rate of or delay the need for a kidney transplant. For example, in some embodiments, the compounds provided herein delay the need for a kidney transplant at least one month, at least two months, at least three months, at least six months, or at least one year. In some embodiments, the compounds provided herein eliminate the need for a kidney transplant.

In some embodiments, the individual has stage 1, stage 2, stage 3A, stage 3B, stage 4, or stage 5 chronic kidney disease. In some embodiments, kidney function is evaluated using an estimated glomerular filtration rate (eGFR) kidney function test.

In some embodiments, the administration is oral administration.

### KITS

The present disclosure further provides kits for carrying out the methods of the invention. The kits may comprise a compound or pharmaceutically acceptable salt thereof as described herein and suitable packaging. The kits may comprise one or more containers comprising any compound described herein. In one aspect, a kit includes a compound of the disclosure or a pharmaceutically acceptable salt thereof, and a label and/or instructions for use of the compound in the treatment of a disease or disorder described herein. The kits may comprise a unit dosage form of the compound.

Provided herein are kits, comprising (i) a compound of formula (A), or formula (A'), or any variation or embodiment thereof, as described elsewhere herein, or a stereoisomer or tautomer thereof, or a pharmaceutically acceptable salt of any of the foregoing, and (ii) instructions for use in treating an APOL1-mediated disease, disorder, or condition in an individual in need thereof. Also provided herein are kits, comprising (i) a pharmaceutical composition comprising a compound of formula (A), or formula (A'), or any variation or embodiment thereof, as described elsewhere herein, or a stereoisomer or tautomer thereof, or a pharmaceutically acceptable salt of any of the foregoing, and one or more pharmaceutically acceptable excipients; and (ii) instructions for use in treating an APOL1-mediated disease, disorder, or condition in an individual in need thereof.
Articles of manufacture are also provided, wherein the article of manufacture comprises a compound of formula (A), or formula (A'), or any variation or embodiment thereof, as described elsewhere herein, or a stereoisomer or tautomer thereof, or a pharmaceutically acceptable salt of any of the foregoing, in a suitable container. Also provided herein are articles of manufacture, comprising a pharmaceutical composition comprising a compound of formula (A), or formula (A'), or any variation or embodiment thereof, as described elsewhere herein, or a stereoisomer or tautomer thereof, or a pharmaceutically acceptable salt of any of the foregoing, in a suitable container. The container may be a vial, jar, ampoule, preloaded syringe, or intravenous bag.

### METHODS OF ASSAYING APOL1 ACTIVITY

Provided herein is a method of assessing APOL1 inhibition in a cell, comprising inducing APOL1 expression in a cell, contacting the cell with an APOL1 inhibitor, and measuring inhibition of calcium transport. In some embodiments, inducing APOL1 expression comprises contacting the cell with doxycycline. In some embodiments, the cell stably expresses a genetically encoded calcium indicator. In some embodiments, the genetically encoded calcium indicator comprises GCaMP6f. In some embodiments, the cell inducibly expresses APOL1 G2. In some embodiments, the cell stably expresses a genetically encoded calcium indicator and inducibly expresses APOL1 G2. In some embodiments, the APOL1 inhibitor is a compound of formula (A'), or any variation or embodiment thereof, as described elsewhere herein, or a stereoisomer or tautomer thereof, or a pharmaceutically acceptable salt of any of the foregoing

Provided herein is a method of assessing rescue of HEK cell death caused by overexpression of APOL1, inducing APOL1 expression in a cell, contacting the cell with an APOL1 inhibitor, exposing the cell to a luminescence reagent, and measuring luminescence. In some embodiments, inducing APOL1 expression comprises contacting the cell with doxycycline. In some embodiments, the cell overexpresses APOL1G2. In some embodiments, the APOL1 inhibitor is a compound of formula (A'), or any variation or embodiment thereof, as described elsewhere herein, or a stereoisomer or tautomer thereof, or a pharmaceutically acceptable salt of any of the foregoing.

### METHODS OF PREPARING

The present disclosure further provides methods for preparing the compounds of present invention. In some aspect, provided herein are methods of preparing a compound of formula (A), or formula (A'), or any embodiment or variation thereof, such as a compound of formula (I), (II), (III), (B-2), (B-5), or (C-1), or a stereoisomer or tautomer thereof, or a pharmaceutically acceptable salt of any of the foregoing.

In some embodiments, a method for preparing a compound of formula (A) or (A'), or a stereoisomer or tautomer thereof, or a pharmaceutically acceptable salt of any of the foregoing, comprises a step of reacting a compound of formula (A-I1): 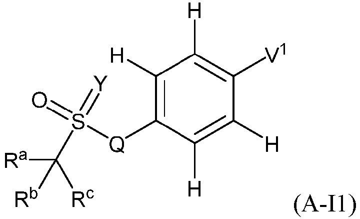 wherein:
Q is absent or is -N-(C₁₋₆alkyl);
Y is O or -N-(C₁₋₆alkyl),
   provided that, when Q is -N(C₁₋₆alkyl), then Y is O;
R^{a}, R^{b}, and R^{c} are each independently H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl,
or any two of R^{a}, R^{b}, and R^{c} are taken, together with the atoms to which they are attached, to form a C₃₋₆cycloalkyl or a 3-6 membered heterocyclyl, and the other of R^{a}, R^{b}, and R^{c} is H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl; and
V¹ is selected from the group consisting of:
   (i) 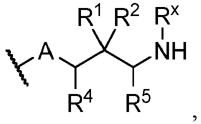 wherein
      A is O, NH, N(C₁₋₆alkyl), CH₂, or CH(C₁₋₆alkyl);
      R^{x} is H,
      or R^{x} is taken together with one of R¹ and R², and the atoms to which they are attached, to form a 3-8 membered heterocyclyl, wherein the 3-8 membered heterocyclyl is substituted with n independently selected R^{g} substituents, wherein n is an integer from 0-6, and R^{g} is -OH, halo, C₁₋₆alkyl, or C₁₋₆alkoxy;
      R¹ and R² are independently H, halo, or -OH,
      or one of R¹ and R² is taken together with R^{x}, and the atoms to which they are attached, to form a 3-8 membered heterocyclyl, wherein the 3-8 membered heterocyclyl is substituted with n independently selected R^{g} substituents, wherein n is an integer from 0-6, and R^{g} is -OH, halo, C₁₋₆alkyl, or C₁₋₆alkoxy, and the other of R¹ and R² is H, halo, or -OH;
      R⁴ and R⁵ are independently H,
      or R⁴ and R⁵ are taken, together with the atoms to which they are attached, to form a C₃₋₈cycloalkyl,
      provided that either:
         (1) R^{x} is taken together with one of R¹ and R², and the atoms to which they are attached, to form a 3-8 membered heterocyclyl, wherein the 3-8 membered heterocyclyl is substituted with *n* independently selected R^{g} substituents, wherein *n* is an integer from 0-6, and R^{g} is -OH, halo, C₁₋₆alkyl, or C₁₋₆alkoxy, or
         (2) R⁴ and R⁵ are taken, together with the atoms to which they are attached, to form a C₃₋₈cycloalkyl,
   (ii) 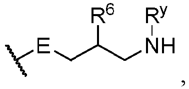 wherein
      E is O, NH, N(C₁₋₆alkyl), CH₂, or CH(C₁₋₆alkyl);
      R⁶ is H or -OH; and
      R^{y} is H; and
   (iii) 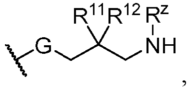 wherein
      G is O, NH, N(C₁₋₆alkyl), CH₂, or CH(C₁₋₆alkyl);
      R^{z} is H or C₁₋₆alkyl, wherein the C₁₋₆alkyl is optionally substituted with one or more C₃₋₈cycloalkyl; and
      R¹¹ and R¹² are independently H, -OH, halo, or C₁₋₆alkyl;
      with:
         a compound of formula (A-I2): 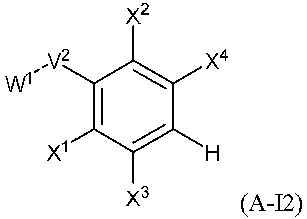
         wherein:
            the dashed line represents a single or double bond;
            W¹ is oxo, halo, or sulfonate ester;
            V² is selected from the group consisting of:
               (i) 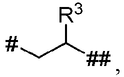 wherein
                  R³ is H, -OH, halo, or C₁₋₆alkoxy;
               (ii) 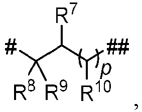
               wherein
               *p* is 0 or 1,
                  provided that, when *p* is 1, then E is O;
               R⁷ is H;
               one of R⁸ and R⁹ is taken together with R¹⁰, and the atoms to which they are attached, to form a C₃₋₈cycloalkyl, and the other of R⁸ and R⁹ is H or C₁₋₆alkyl; and
               R¹⁰ is taken together with one of R⁸ and R⁹, and the atoms to which they are attached, to form a C₃₋₈cycloalkyl; and
   (iii) 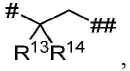 wherein
      R¹³ and R¹⁴ are independently H, C₁₋₆alkyl, or C₃₋₈cycloalkyl,
      or R¹³ and R¹⁴ are taken, together with the atoms to which they are attached, to form a 3-8 membered heterocyclyl,
      wherein, for each of (i)-(iii), # denotes the point of attachment to W¹, and ## denotes the point of attachment to the phenyl ring bearing moieties X¹-X⁴; and
      X¹, X², X³, and X⁴ are, independently of each other, H, halo, -CN, C₁₋₆alkyl, C₁₋₆alkoxy, or SF₅, wherein the C₁₋₆alkyl or C₁₋₆alkoxy is optionally substituted with one or more halo,
         provided that at least one of X¹, X², X³, and X⁴ is halo, -CN, C₁₋₆alkyl, C₁₋₆alkoxy, or SF₅, wherein the C₁₋₆alkyl or C₁₋₆alkoxy is optionally substituted with one or more halo;
      to give a compound of formula (A) or (A') or a stereoisomer or tautomer thereof, or a pharmaceutically acceptable salt of any of the foregoing.

In some embodiments, the compound of formula (A) or (A') is prepared by a step comprising:
a) alkylation of an amine of formula (A-I1) with an alkyl halide, or sulfonate ester compound of formula (A-I2) in the presence of an inorganic or organic base; or
b) reductive amination of an aldehyde or ketone of formula (A-I2) with an amine of formula (A-I1) in the presence of a reducing agent.

In some embodiments, the compound of formula (A) or (A') is prepared by a step comprising alkylation of an amine of formula (A-I1) with an alkyl halide, or sulfonate ester compound of formula (A-I2) in the presence of an inorganic or organic base. In some embodiments, the inorganic base is selected from the group consisting of potassium carbonate, sodium carbonate, and sodium bicarbonate. In some embodiments, the organic base is a tertiary amine. In some embodiments, the organic base is selected from the group consisting of trimethylamine, triethylamine, and diisopropylethyamine.

In some embodiments, the sulfonate ester compound of formula (A-I2) is a mesylate or a tosylate. In some embodiments, the sulfonate ester compound of formula (A-I2) is a mesylate. In some embodiments, the sulfonate ester compound of formula (A-I2) is a tosylate.

In some embodiments, the compound of formula (A) or (A') is prepared by a step comprising reductive amination of an aldehyde or ketone of formula (A-I2) with an amine of formula (I-II). In some embodiments, the reductive amination proceeds under the action of a reducing agent. In some embodiments, the reducing agent is sodium triacetoxyborohydride, or sodium cyanoborohidride.

In some embodiments, a method for preparing a compound of formula (A) or (A'), or a stereoisomer or tautomer thereof, or a pharmaceutically acceptable salt of any of the foregoing, comprises a step of reacting a compound of formula (A-I3): 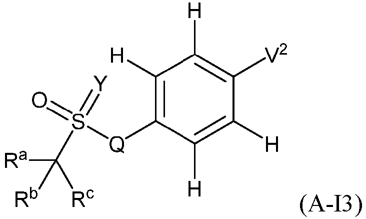 wherein:
Q is absent or is -N-(C₁₋₆alkyl);
Y is O or -N-(C₁₋₆alkyl),
   provided that, when Q is -N(C₁₋₆alkyl), then Y is O;
R^{a}, R^{b}, and R^{c} are each independently H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl,
or any two of R^{a}, R^{b}, and R^{c} are taken, together with the atoms to which they are attached, to form a C₃₋₆cycloalkyl or a 3-6 membered heterocyclyl, and the other of R^{a}, R^{b}, and R^{c} is H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl; and
V² is halo or OH,
with:
   a compound of formula (A-I4): 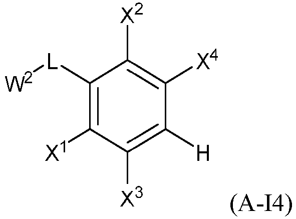
   wherein:
      W² is H, or sulfamate;
      L is selected from the group consisting of:
         (i) 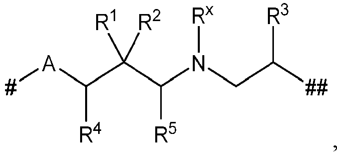 wherein
            A is O, NH, N(C₁₋₆alkyl), CH₂, or CH(C₁₋₆alkyl);
            R^{x} is H,
            or R^{x} is taken together with one of R¹ and R², and the atoms to which they are attached, to form a 3-8 membered heterocyclyl, wherein the 3-8 membered heterocyclyl is substituted with n independently selected R^{g} substituents, wherein n is an integer from 0-6, and R^{g} is -OH, halo, C₁₋₆alkyl, or C₁₋₆alkoxy;
            R¹ and R² are independently H, halo, or -OH,
            or one of R¹ and R² is taken together with R^{x}, and the atoms to which they are attached, to form a 3-8 membered heterocyclyl, wherein the 3-8 membered heterocyclyl is substituted with n independently selected R^{g} substituents, wherein n is an integer from 0-6, and R^{g} is -OH, halo, C₁₋₆alkyl, or C₁₋₆alkoxy, and the other of R¹ and R² is H, halo, or -OH;
            R³ is H, -OH, halo, or C₁₋₆alkoxy; and
            R⁴ and R⁵ are independently H,
            or R⁴ and R⁵ are taken, together with the atoms to which they are attached, to form a C₃₋₈cycloalkyl,
            provided that either:
               (1) R^{x} is taken together with one of R¹ and R², and the atoms to which they are attached, to form a 3-8 membered heterocyclyl, wherein the 3-8 membered heterocyclyl is substituted with *n* independently selected R^{g} substituents, wherein *n* is an integer from 0-6, and R^{g} is -OH, halo, C₁₋₆alkyl, or C₁₋₆alkoxy, or
               (2) R⁴ and R⁵ are taken, together with the atoms to which they are attached, to form a C₃₋₈cycloalkyl,
         (ii) 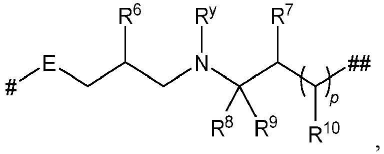 wherein
            E is O, NH, N(C₁₋₆alkyl), CH₂, or CH(C₁₋₆alkyl);
            *p* is 0 or 1,
               provided that, when *p* is 1, then E is O;
            R⁶ is H or -OH;
            R^{y} is H,
            or R^{y} is taken together with R⁷, and the atoms to which they are attached, to form a 3-8 membered heterocyclyl,
            or R^{y} is taken together with one of R⁸ and R⁹, and the atoms to which they are attached, to form a 3-8 membered heterocyclyl;
            R⁷ is H,
            or R⁷ is taken together with R^{y}, and the atoms to which they are attached, to form a 3-8 membered heterocyclyl;
            R⁸ and R⁹ are independently H or C₁₋₆alkyl,
            or one of R⁸ and R⁹ is taken together with R^{y}, and the atoms to which they are attached, to form a 3-8 membered heterocyclyl, and the other of R⁸ and R⁹ is H or C₁₋₆alkyl,
            or one of R⁸ and R⁹ is taken together with R¹⁰, and the atoms to which they are attached, to form a C₃₋₈cycloalkyl, and the other of R⁸ and R⁹ is H or C₁₋₆alkyl; and
            R¹⁰ is H,
            or R¹⁰ is taken together with one of R⁸ and R⁹, and the atoms to which they are attached, to form a C₃₋₈cycloalkyl,
            provided that:
               (1) R^{y} is taken together with R⁷, and the atoms to which they are attached, to form a 3-8 membered heterocyclyl, or
               (2) R^{y} is taken together with one of R⁸ and R⁹, and the atoms to which they are attached, to form a 3-8 membered heterocyclyl, or
               (3) one of R⁸ and R⁹ is taken together with R¹⁰ and the atoms to which they are attached, to form a C₃₋₈cycloalkyl, and
         (iii) 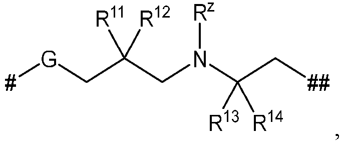 wherein
            G is O, NH, N(C₁₋₆alkyl), CH₂, or CH(C₁₋₆alkyl);
            R^{z} is H or C₁₋₆alkyl, wherein the C₁₋₆alkyl is optionally substituted with one or more C₃₋₈cycloalkyl;
            R¹¹ and R¹² are independently H, -OH, halo, or C₁₋₆alkyl; and
            R¹³ and R¹⁴ are independently H, C₁₋₆alkyl, or C₃₋₈cycloalkyl,
            or R¹³ and R¹⁴ are taken, together with the atoms to which they are attached, to form a 3-8 membered heterocyclyl,
            wherein, for each of (i)-(iii), # denotes the point of attachment to the phenyl ring bearing moiety Q, and ## denotes the point of attachment to the phenyl ring bearing moieties X¹-X⁴; and
            X¹, X², X³, and X⁴ are, independently of each other, H, halo, -CN, C₁₋₆alkyl, C₁₋₆alkoxy, or SF₅, wherein the C₁₋₆alkyl or C₁₋₆alkoxy is optionally substituted with one or more halo,
               provided that at least one of X¹, X², X³, and X⁴ is halo, -CN, C₁₋₆alkyl, or C₁₋₆alkoxy, or SF₅, wherein the C₁₋₆alkyl or C₁₋₆alkoxy is optionally substituted with one or more halo;
            to give a compound of formula (A) or (A') or a stereoisomer or tautomer thereof, or a pharmaceutically acceptable salt of any of the foregoing.

In some embodiments, the compound of formula (A) or (A') is prepared by a step comprising alkylation of an alcohol or amine of formula (A-I4) with an alkyl halide compound of formula (A-I3) in the presence of an inorganic or organic base. In some embodiments, the inorganic base is selected from the group consisting of potassium carbonate, sodium carbonate, and sodium bicarbonate. In some embodiments, the organic base is a tertiary amine. In some embodiments, the organic base is selected from the group consisting of trimethylamine, triethylamine, and diisopropylethyamine.

In some embodiments, the compound of formula (A) or (A') is prepared by a step comprising alkylation of an alcohol of formula (A-I3) with a sulfamate compound of formula (A-I4) in the presence of an inorganic or organic base. In some embodiments, the inorganic base is selected from the group consisting of potassium carbonate, sodium carbonate, and sodium bicarbonate. In some embodiments, the organic base is a tertiary amine. In some embodiments, the organic base is selected from the group consisting of trimethylamine, triethylamine and diisopropylethyamine.

In some embodiments, a method for preparing a compound of formula (A) or (A'), or a stereoisomer or tautomer thereof, or a pharmaceutically acceptable salt of any of the foregoing, comprises a step of reacting a compound of formula (A-I5): 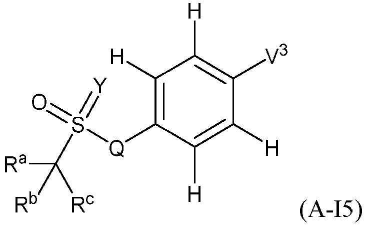 wherein:
Q is absent or is -N-(C₁₋₆alkyl);
Y is O or -N-(C₁₋₆alkyl),
   provided that, when Q is -N(C₁₋₆alkyl), then Y is O;
R^{a}, R^{b}, and R^{c} are each independently H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl,
or any two of R^{a}, R^{b}, and R^{c} are taken, together with the atoms to which they are attached, to form a C₃₋₆cycloalkyl or a 3-6 membered heterocyclyl, and the other of R^{a}, R^{b}, and R^{c} is H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl; and
V³ is selected from the group consisting of:
   (iv) 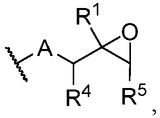 wherein
      A is O, NH, N(C₁₋₆alkyl), CH₂, or CH(C₁₋₆alkyl);
      R¹ is H, halo, or -OH,
      R⁴ and R⁵ are taken, together with the atoms to which they are attached, to form a C₃₋₈cycloalkyl,
   (v) 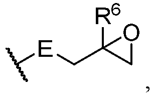 wherein
      E is O, NH, N(C₁₋₆alkyl), CH₂, or CH(C₁₋₆alkyl);
      R⁶ is H or -OH; and
   (vi) 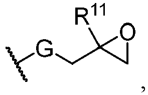 wherein
      G is O, NH, N(C₁₋₆alkyl), CH₂, or CH(C₁₋₆alkyl);
      R¹¹ is H, -OH, halo, or C₁₋₆alkyl;
      with:
         a compound of formula (A-I6): 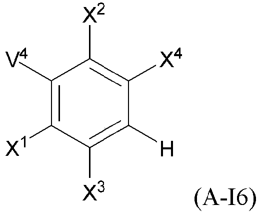
         wherein:
            V⁴ is selected from the group consisting of:
               (iv) 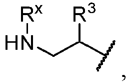 wherein
               R^{x} is H, and
               R³ is H, -OH, halo, or C₁₋₆alkoxy;
   (v) 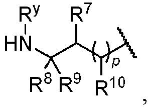 wherein
      *p* is 0 or 1,
         provided that, when *p* is 1, then E is O;
      R^{y} is H,
      or R^{y} is taken together with R⁷, and the atoms to which they are attached, to form a 3-8 membered heterocyclyl,
      or R^{y} is taken together with one of R⁸ and R⁹, and the atoms to which they are attached, to form a 3-8 membered heterocyclyl;
      R⁷ is H,
      or R⁷ is taken together with R^{y}, and the atoms to which they are attached, to form a 3-8 membered heterocyclyl;
      R⁸ and R⁹ are independently H or C₁₋₆alkyl,
      or one of R⁸ and R⁹ is taken together with R^{y}, and the atoms to which they are attached, to form a 3-8 membered heterocyclyl, and the other of R⁸ and R⁹ is H or C₁₋₆alkyl,
      or one of R⁸ and R⁹ is taken together with R¹⁰, and the atoms to which they are attached, to form a C₃₋₈cycloalkyl, and the other of R⁸ and R⁹ is H or C₁₋₆alkyl; and
      R¹⁰ is H,
      or R¹⁰ is taken together with one of R⁸ and R⁹, and the atoms to which they are attached, to form a C₃₋₈cycloalkyl,
      provided that:
         (1) R^{y} is taken together with R⁷, and the atoms to which they are attached, to form a 3-8 membered heterocyclyl, or
         (2) R^{y} is taken together with one of R⁸ and R⁹, and the atoms to which they are attached, to form a 3-8 membered heterocyclyl, or
         (3) one of R⁸ and R⁹ is taken together with R¹⁰ and the atoms to which they are attached, to form a C₃₋₈cycloalkyl, and
   (vi) 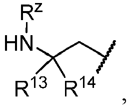 wherein
      R¹³ and R¹⁴ are independently H, C₁₋₆alkyl, or C₃₋₈cycloalkyl,
      or R¹³ and R¹⁴ are taken, together with the atoms to which they are attached, to form a 3-8 membered heterocyclyl; and
      X¹, X², X³, and X⁴ are, independently of each other, H, halo, -CN, C₁₋₆alkyl, C₁₋₆alkoxy, or SF₅, wherein the C₁₋₆alkyl or C₁₋₆alkoxy is optionally substituted with one or more halo,
         provided that at least one of X¹, X², X³, and X⁴ is halo, -CN, C₁₋₆alkyl, C₁₋₆alkoxy, or SF₅, wherein the C₁₋₆alkyl or C₁₋₆alkoxy is optionally substituted with one or more halo;
      to give a compound of formula (A) or (A') or a stereoisomer or tautomer thereof, or a pharmaceutically acceptable salt of any of the foregoing.

In some embodiments, the compound of formula (A) or (A') is prepared by a step comprising alkylation of an epoxide compound of formula (A-I5) with an amine compound of formula (A-I6) in the presence of an organic base. In some embodiments, the organic base is a tertiary amine. In some embodiments, the organic base is selected from the group consisting of trimethylamine, triethylamine and diisopropylethyamine.

### EXAMPLES

The following synthetic reaction schemes, which are detailed in the Schemes, General Procedures, and Examples, are merely illustrative of some of the methods by which the compounds of the present disclosure, or an embodiment or aspect thereof, can be synthesized. Various modifications to these synthetic reaction schemes can be made, as will be apparent to those of ordinary skill in the art.

The starting materials and the intermediates of the synthetic reaction schemes can be isolated and purified if desired using conventional techniques, including but not limited to, filtration, distillation, crystallization, chromatography, and the like. Such materials can be characterized using conventional means, including physical constants and spectral data.

Although certain exemplary embodiments are depicted and described herein, the compounds of the present disclosure, or any variation or embodiment thereof, may be prepared using appropriate starting materials according to the methods described generally herein and/or by methods available to one of ordinary skill in the art.

### Synthetic Examples

As depicted in the Schemes, General Procedures, and Examples below, in certain exemplary embodiments, compounds of formula (A), or formula (A'), or any variation or embodiment thereof, as described elsewhere herein, or a stereoisomer or tautomer thereof, or a pharmaceutically acceptable salt of any of the foregoing, are prepared according to the general procedures. The general methods below, and other methods known to synthetic chemists of ordinary skill in the art, can be applied to all formulae, variations, embodiments, and species described herein.

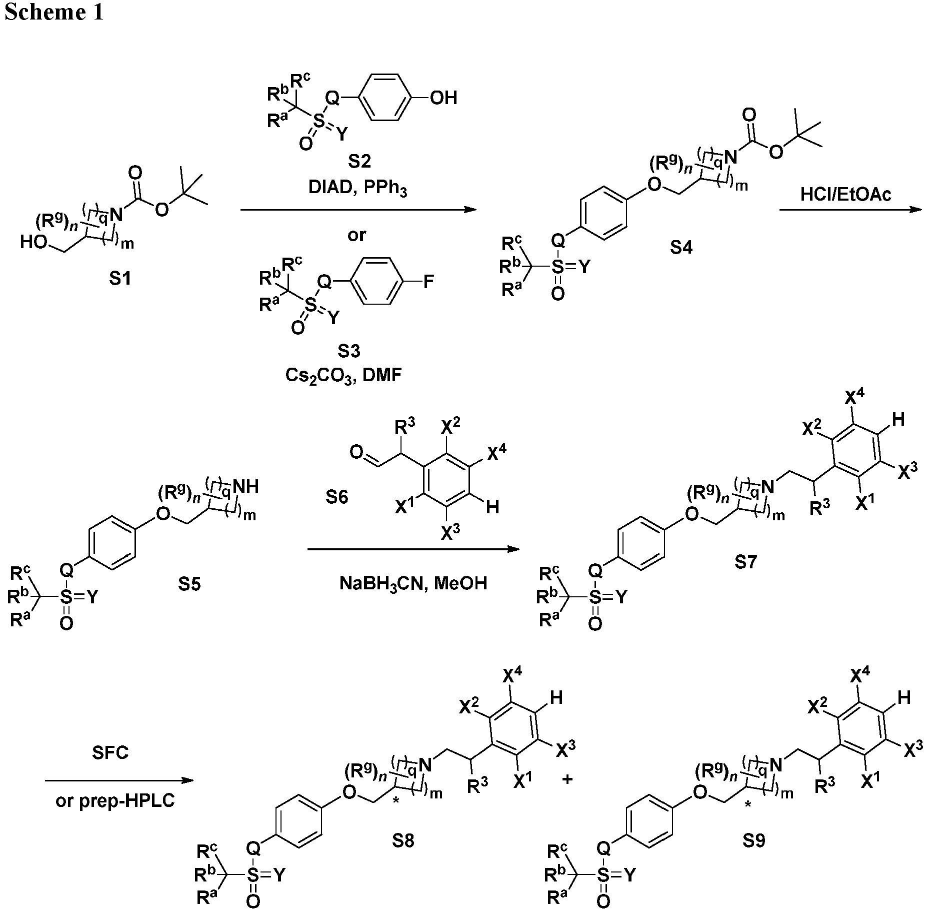

Compounds of formulae **S7-S9** may be prepared by the general synthetic method shown in **Scheme 1.** It is to be understood that, where applicable, the moieties and variables depicted in **Scheme 1** are as defined elsewhere herein for a compound of formula (A), or formula (A'), or any variation or embodiment thereof, or a stereoisomer or tautomer thereof, or a pharmaceutically acceptable salt of any of the foregoing. In addition, with reference to **Scheme 1,** m is an integer from 0-5 and q is an integer from 0-5, provided that 1 ≤ (m + q) ≤ 6.

C-O bond formation may be accomplished through either a Mitsunobu reaction with phenols of formula **S2** or an S_{N}Ar with aryl fluorides of formula **S3** to provide compounds of formula **S4.** Deprotection of the *N-tert*-butyloxycarbonyl (Boc) group may proceed using a protic acid such as hydrochloric acid to give compounds of formula **S5.** Compounds of formula **S7** can be prepared through reductive amination using an aldehyde of formula **S6** and a hydride source such as NaBH₃CN. Chiral preparative SFC or HPLC separation may be utilized to provide two or more single stereoisomers of formulas **S8** and **S9.**

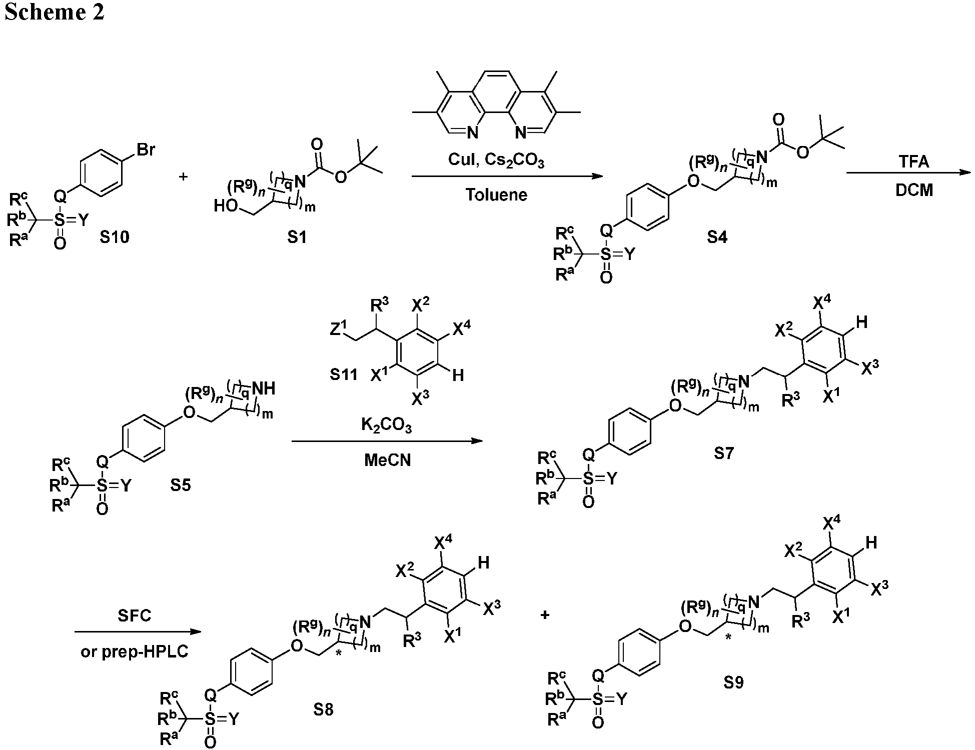

Compounds of formulas **S7-S9** may be prepared by the alternative general synthetic method shown in **Scheme 2.** It is to be understood that, where applicable, the moieties and variables depicted in **Scheme 2** are as defined elsewhere herein for a compound of formula (A), or formula (A'), or any variation or embodiment thereof, or a stereoisomer or tautomer thereof, or a pharmaceutically acceptable salt of any of the foregoing. In addition, with reference to **Scheme 2:** m is an integer from 0-5 and q is an integer from 0-5, provided that 1 ≤ (m + q) ≤ 6; and Z¹ is halo (for example: chloro or bromo) or a sulfonate ester (for example: mesylate).

C-O coupling of an aryl halide of formula **S10** with an alcohol of formula **S1** may be performed using conditions such as copper(I) iodide, Cs₂CO₃, and 3,4,7,8-tetramethyl-1,10-phenanthroline in toluene at an elevated temperature to afford compounds of formula **S4.** Deprotection of the *N*-*tert*-butyloxycarbonyl (Boc) group may proceed using a protic acid such as trifluoroacetic acid to give compounds of formula **S5.** Compounds of formula **S7** can be prepared using a base such as K₂CO₃, an alkyl halide or alkyl sulfonate ester such as alkyl bromide of formula **S11,** and an organic solvent such as MeCN at elevated temperature. Chiral preparative SFC or HPLC separation may be utilized to provide two or more single stereoisomers of formulas **S8** and **S9.**

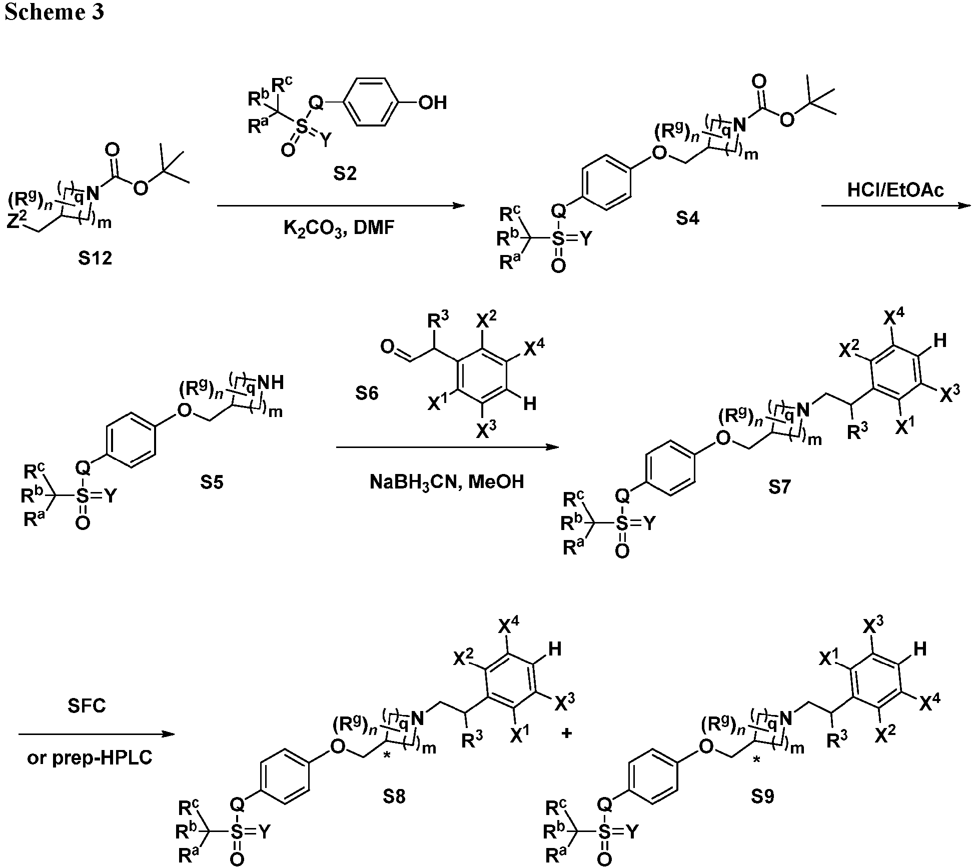

Compounds of formulas **S7-S9** may be prepared by the alternative general synthetic method shown in **Scheme 3.** It is to be understood that, where applicable, the moieties and variables depicted in **Scheme 3** are as defined elsewhere herein for a compound of formula (A), or formula (A'), or any variation or embodiment thereof, or a stereoisomer or tautomer thereof, or a pharmaceutically acceptable salt of any of the foregoing. In addition, with reference to **Scheme 3:** m is an integer from 0-5 and q is an integer from 0-5, provided that 1 ≤ (m + q) ≤ 6; and Z² is halo (for example: chloro or bromo) or a sulfonate ester (for example: mesylate).

C-O bond formation may be accomplished through an S_{N}2 reaction with alkyl halide or alkyl sulfonate ester such as alkyl bromide of formula **S12** with phenols of formula **S2** in the presence of bases such as K₂CO₃ to give compounds of formula **S4.** Deprotection of the *N-tert*-butyloxycarbonyl (Boc) group may proceed using a protic acid such as HCl to give compounds of formula **S5.** Compounds of formula **S7** can be prepared through reductive amination using an aldehyde of formula **S6** and a hydride source such as NaBH₃CN. Chiral preparative SFC or HPLC separation may be utilized to provide two or more single stereoisomers of formula **S8** and **S9.**

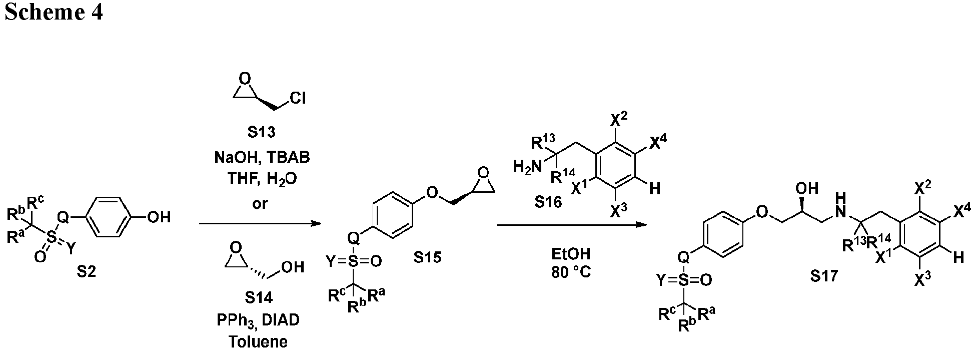

Compounds of formula **S17** may be prepared by the alternative general synthetic method shown in **Scheme 4.** It is to be understood that, where applicable, the moieties and variables depicted in **Scheme 4** are as defined elsewhere herein for a compound of formula (A), or formula (A'), or any variation or embodiment thereof, or a stereoisomer or tautomer thereof, or a pharmaceutically acceptable salt of any of the foregoing.

Compounds of formula S17 with (S)-stereochemistry may be accessed by treating phenols of formula S2 with (R)-2-(chloromethyl)oxirane (S13) in the presence of a phase transfer catalyst such as TBAB and a base such as NaOH. Alternately, treatment of phenol **S2** with (*R*)-oxiran-2-ylmethanol **S14** and Mitsunobu conditions such as PPh₃ and DIAD may also provide **(*S*)-S15.** Epoxide opening to provide compounds of formula **S17** proceeds in the presence of amines of formula **S16,** triethylamine in cases where the salt form of amine **S16** is used, as a solution in ethanol at elevated temperatures. In cases where epimeric -OH stereochemistry of compounds of formula **S17** is desired, the above synthetic scheme may be modified to utilize (*S*)-2-(chloromethyl)oxirane or (*S*)-oxiran-2-ylmethanol.

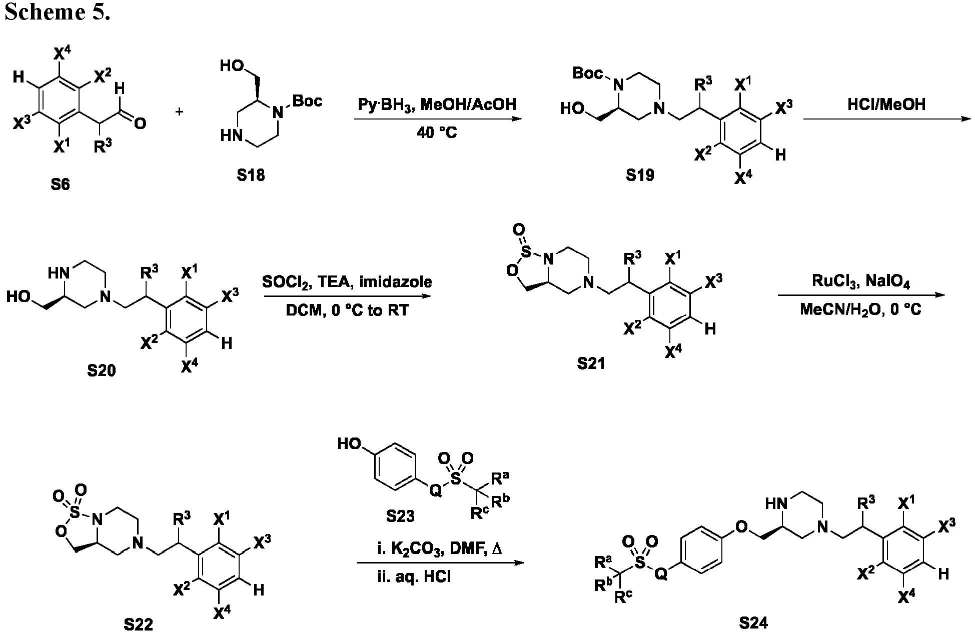

Compounds of formula **S24** may be prepared according to **Scheme 5.** Reductive amination of a mono-protected piperazine such as **S18** with an aldehyde such as **S6** gives compound **S19.** Removal of the the N-*tert*-butyloxycarbonyl (Boc) group upon treatment with a protic acid such as HCl in a solvent such as MeOH gives **S20.** Treatment with thionyl chloride, triethylamine, and imidazole gives rise to **S21.** Oxidation to the oxathiazolidine-2,2-dioxide occurs on treatment with ruthenium (III) chloride and sodium periodate in a mixed solvent system of acetonitrile and water to give **S22.** Heating **S22** with a phenol such as **S23** and potassium carbonate in DMF, followed by treatment with aqueous HCl, gives compounds of formula **S24.** Chiral preparative SFC or HPLC separation may be utilized to provide two or more single stereoisomers of compounds derived from formula **S24.**

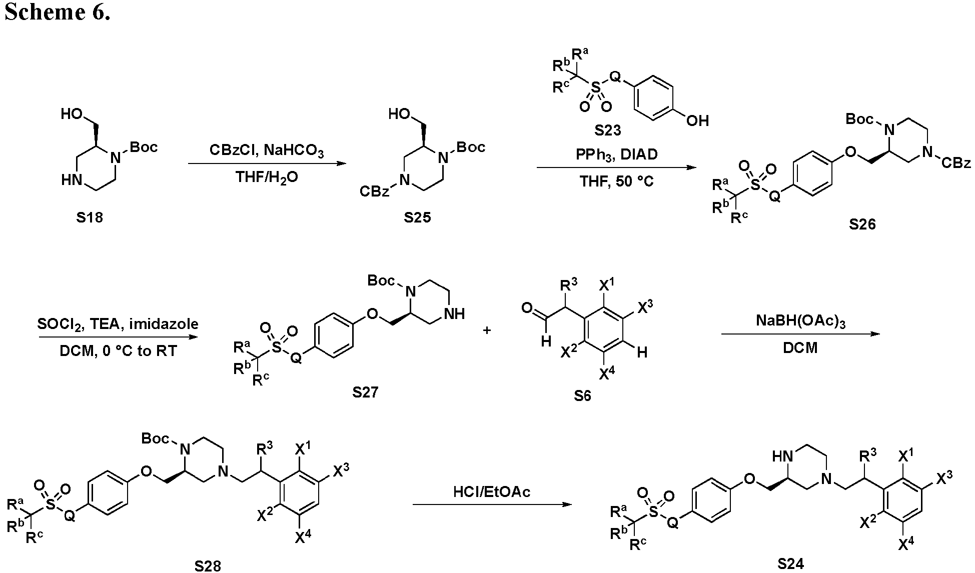

An alternative synthetic approach to compounds of formula **S24** is depicted in **Scheme 6.** Reaction of piperazine **S18** with benzyl chloroformate in the presence of a base such as sodium bicarbonate generates the bis-carbamate **S25.** Mitsunobu coupling of phenol **S23** with triphenylphosphine and DIAD in THF generates **S26.** Selective cleavage of the benzyl carbamate may be achieved by treatment with thionyl chloride, triethylamine, and imidazole in DCM to give **S27,** which may then undergo reductive amination using sodium triacetoxyborohydride provides compounds of formula **S28.** Cleavage of the the N*-tert-*butyloxycarbonyl (Boc) group provides **S24.** Chiral preparative SFC or HPLC separation may be utilized to provide two or more single stereoisomers of compounds derived from formula **S24.**

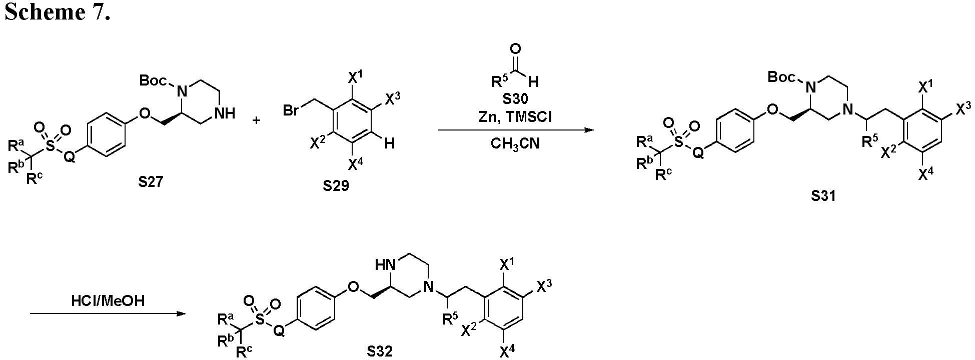

**Scheme 7** outlines an approach to compounds of formula **S32.** Addition of an alkylzinc reagent generated in situ from benzyl bromide **S29** to an iminium ion formed by reaction of piperazine **S27** and aldehyde **S30** gives **S31.** Removal of the *N-tert-*butyloxycarbonyl (Boc) group using a protic acid such as HCl in a solvent such as MeOH gives **S32.** Chiral preparative SFC or HPLC separation may be utilized to provide two or more single stereoisomers of compounds derived from formula **S32.**

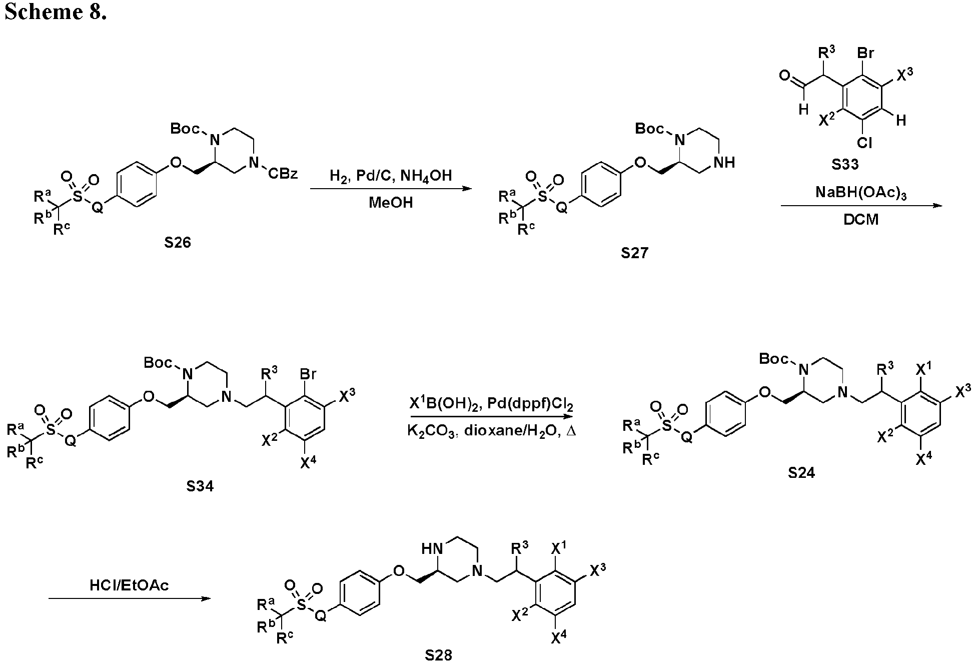

A synthetic approach to compounds of formula **S28** is depicted in Scheme **8**. Selective removal of the benzyl carbamate of **S26** via hydrogenation gives piperazine **S27.** Reductive amination under the action of sodium triacetoxyborohydride generates **S34.** Further manipulation may be accomplished by Suzuki coupling with a boronic acid (X¹B(OH₂)), Pd(dppf)Cl₂ catalyst, and potassium carbonate to give **S24.** Removal of the *N-tert-*butyloxycarbonyl (Boc) group upon reaction with HCl in EtOAc gives compounds of formula **S28.** Chiral preparative SFC or HPLC separation may be utilized to provide two or more single stereoisomers of compounds derived from formula **S28.**

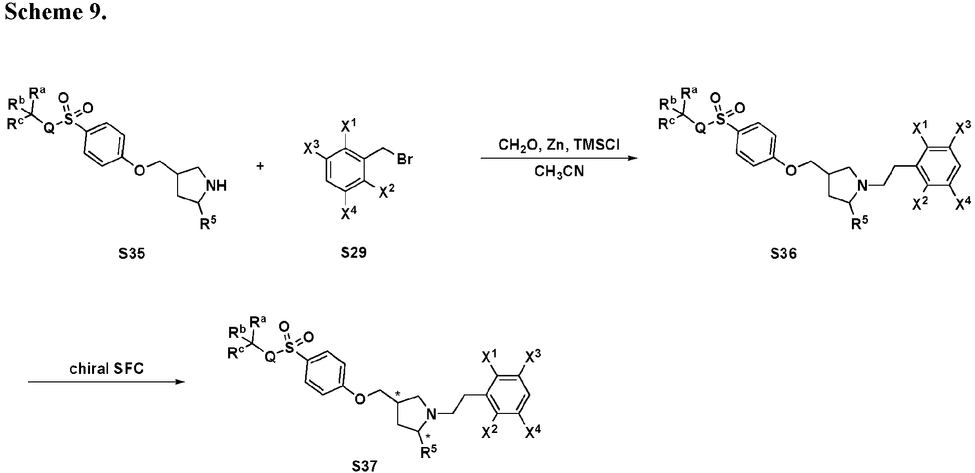

**Scheme 9** depicts an alternate application of the three-component coupling described in **Scheme 7.** Coupling of pyrrolidine **S35,** benzyl bromide **S29,** and formaldehyde provides compounds of formula **S36.** Chiral preparative SFC or HPLC separation may be utilized to provide two or more single stereoisomers of formula **S37.**

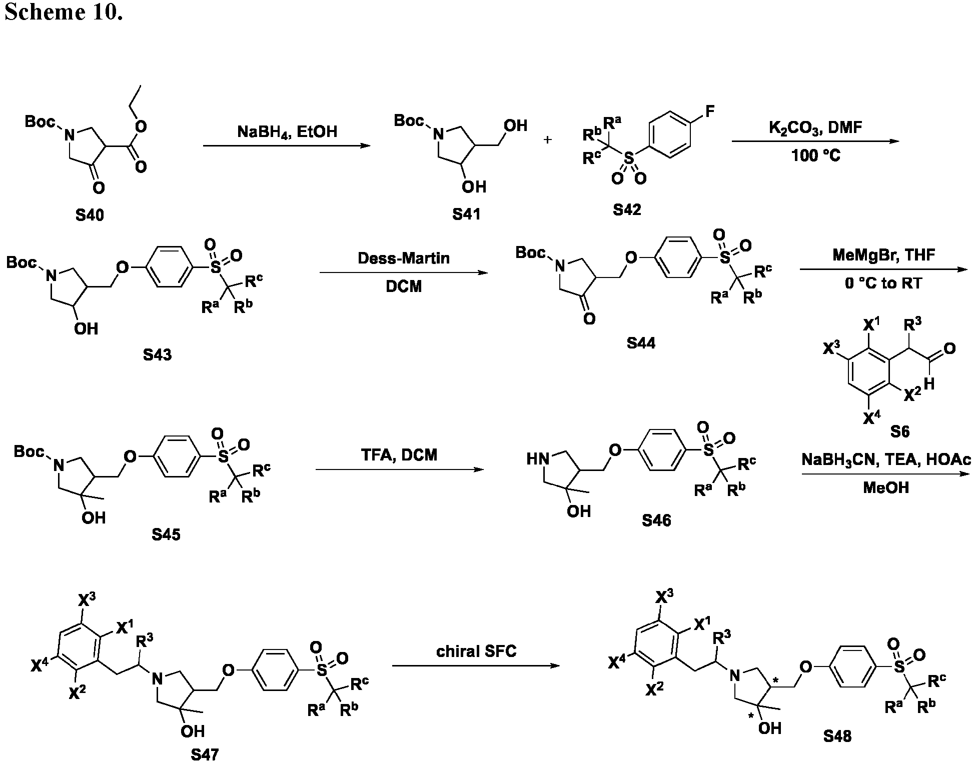

**Scheme 10** depicts an alternative approach to pyrrolidine analogs of formula **S48.** NaBH₄ reduction of β-keto ester **S40** gives diol **S41,** which can undergo S_{N}Ar reaction with fluorobenzene **S42** upon heating in DMF with potassium carbonate as base. Oxidation to ketone **S44** may be achieved with the Dess-Martin periodinane. Reaction with an alkyl metal reagent such as methylmagnesium bromide gives tertiary alcohol **S45,** as a mixture of isomers. Removal of the *N*-*tert*-butyloxycarbonyl (Boc) group with a protic acid such as TFA, followed by reductive amination with aldehyde **S6,** gives pyrrolidine **S47.** Chiral preparative SFC or HPLC separation may be utilized to provide two or more single stereoisomers of formula **S48.**

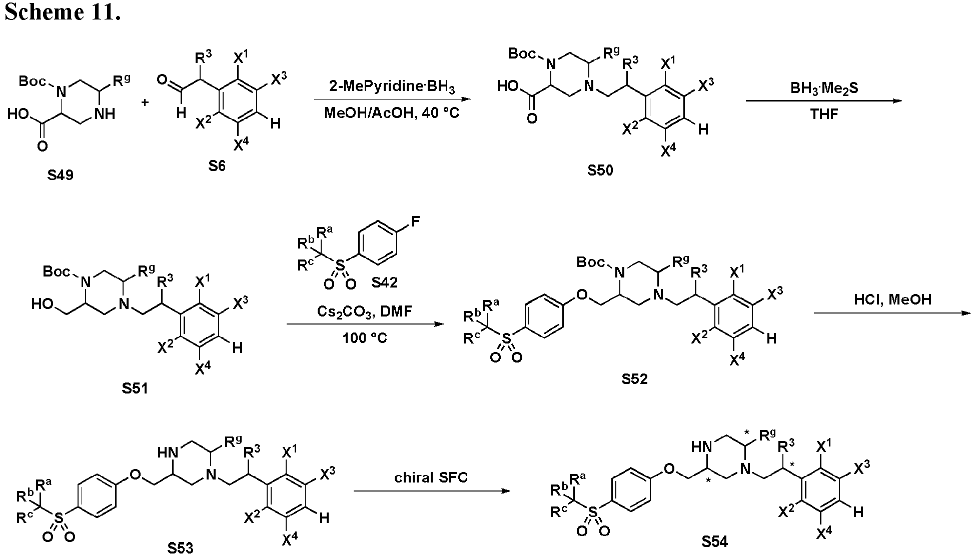

**Scheme 11** depicts a route to analogs bearing substituted piperazine cores. Reductive amination of piperazine **S49** and aldehyde **S6** with 2-methyl pyridine borane complex gives **S50.** Reduction of the carboxylic acid **S50** with borane-dimethylsulfide gives alcohol **S51,** which may undergo S_{N}Ar reaction with fluorobenzene **S42** to give **S52.** Removal of the *N-tert-*butyloxycarbonyl (Boc) group with a protic acid such as HCl in a solvent such as MeOH gives piperazine **S53.** Chiral preparative SFC or HPLC separation may be utilized to provide two or more single stereoisomers of formula **S54.**

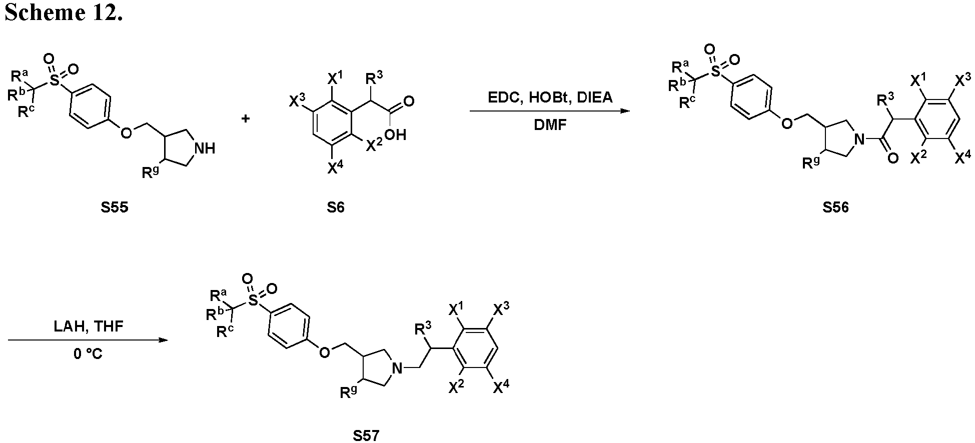

**Scheme 11** depicts an approach to compounds of formula **S57.** Amide bond formation between an amine such as pyrrolidine **S55** and carboxylic acid **S6** using EDC and HOBt with a tertiary amine base such as DIEA to provide **S56.** Amide reduction can be achieved upon treatment with lithium aluminum hydride to give compounds of formula **S57.** Chiral preparative SFC or HPLC separation may be utilized to provide two or more single stereoisomers of compounds derived from formula **S57.**

### General Procedure for Intermediate A

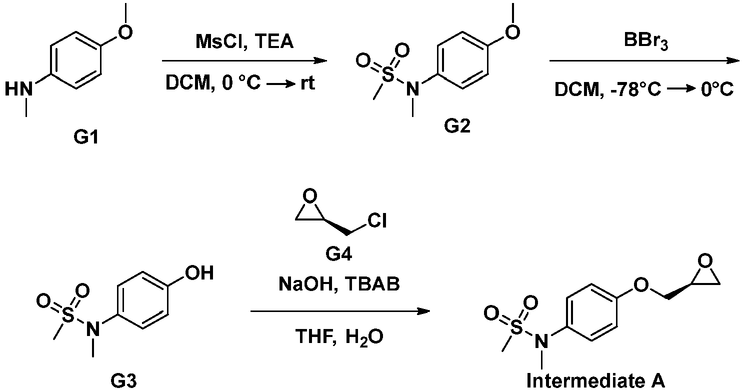

### Step 1: N-(4-methoxyphenyl)-N-methylmethanesulfonamide

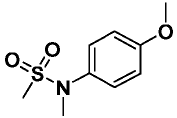

To a solution of 4-methoxy-*N*-methyl-aniline (**G1**, 7.00 g, 51.0 mmol) in DCM (60 mL) was added TEA (14.2 mL, 102 mmol). After cooling the reaction to 0 °C, MsCl (5.13 mL, 66.3 mmol) was added dropwise. The mixture was stirred at room temperature for 1 h. The reaction mixture was cooled to 0 °C and quenched by the addition of water (40 mL). The biphasic mixture was extracted with DCM (60 mL x 2). The combined organic layers were washed with brine (40 mL), dried over Na₂SO₄, filtered and concentrated in vacuo to give *N*-(4-methoxyphenyl)-*N*-methylmethanesulfonamide **(G2),** which was used in the next step without further purification. MS = 216.1 [M+H]⁺.

### Step 2: N-(4-hydroxyphenyl)-N-methylmethanesulfonamide

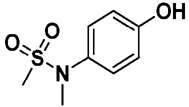

To a -78 °C solution of *N*-(4-methoxyphenyl)-*N*-methylmethanesulfonamide **(G2,** 11.4 g, 53.0 mmol) in DCM (100 mL) was added BBr₃ (10.2 mL, 106 mmol) dropwise. The mixture was warmed to 0 °C and stirred for 2 h. The reaction mixture was quenched by the dropwise addition of water (70 mL), and the mixture was stirred at room temperature for 20 min. Then the mixture was extracted with DCM (100 mL x 3). The combined organic layers were washed with brine (80 mL), dried over Na₂SO₄, filtered and concentrated under in vacuo to give *N*-(4-hydroxyphenyl)-*N*-methyl-methanesulfonamide **(G3).** MS = 202.2 [M+H]⁺.

### Step 3: (S)-N-methyl-N-(4-(oxiran-2-ylmethoxy)phenyl)methanesulfonamide (Intermediate A)

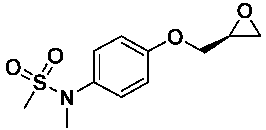

To a solution of *N*-(4-hydroxyphenyl)-*N*-methyl-methanesulfonamide **(G3,** 4.00 g, 19.9 mmol) and (*R*)-2-(chloromethyl)oxirane **(G4,** 3.12 mL, 39.8 mmol) in water (10 mL) and THF (20 mL) was added TBAB (897 mg, 2.78 mmol). Next, a solution of NaOH (1.19 g, 29.8 mmol) in water (10 mL) was added dropwise. The mixture was stirred at room temperature for 12 h. The reaction mixture was diluted by the dropwise addition of water (20 mL), and then extracted with EtOAc (20 mL x 3). The combined organic layers were washed with brine (15 mL), dried over Na₂SO₄, filtered and concentrated in vacuo. The crude residue was purified by normal phase silica gel chromatography (Biotage 40 g cartridge, 0-55% EtOAc in petroleum ether). The resulting residue was further purified by re-crystallization (50 mL of 1:10 EtOAc in petroleum ether) to give (*S*)-*N*-methyl-*N*-(4-(oxiran-2-ylmethoxy)phenyl)methanesulfonamide **(Intermediate A).** MS = 280.0 [M+Na]⁺.

The following intermediates in Table 2 were prepared according to procedures similar to those described for **Intermediate A** using the appropriate starting materials.

**Table 2**

| Intermediate | Structure | IUPAC Name | Exact Mass [M+H]⁺ |
|---|---|---|---|
| **B** | 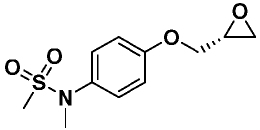 | (*R*)-*N*-methyl-*N*-(4-(oxiran-2-ylmethoxy)phenyl)methane sulfonamide | Calc'd 280.1 |
| | | | Found 280.0 [M+Na]⁺ |
| **C** | 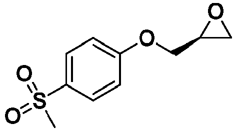 | (*S*)-2-((4-(methylsulfonyl)phenoxy) methyl)oxirane | Calc'd 229.1 |
| | | | Found 229.0 |
| **D** | 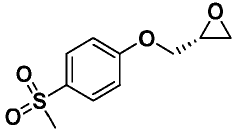 | (*R*)-2-((4-(methylsulfonyl)phenoxy) methyl)oxirane | Calc'd 229.1 |
| | | | Found 229.2 |
| **E** | 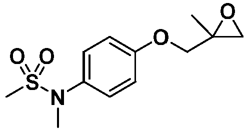 | *N*-methyl-*N*-(4-((2-methyloxiran-2-yl)methoxy)phenyl)methan esulfonamide | Calc'd 193.1 |
| | | | Found 193.1 |
| | | | [M-CH₃O₂S+H]⁺ |
| **F** | 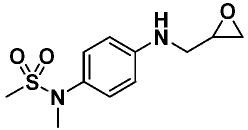 | *N*-methyl-*N*-(4-((oxiran-2-ylmethyl)amino)phenyl)me thanesulfonamide | Calc'd 279.1 Found 279.0 [M+Na]⁺ |
| | | | |

### General Procedure for Intermediate G

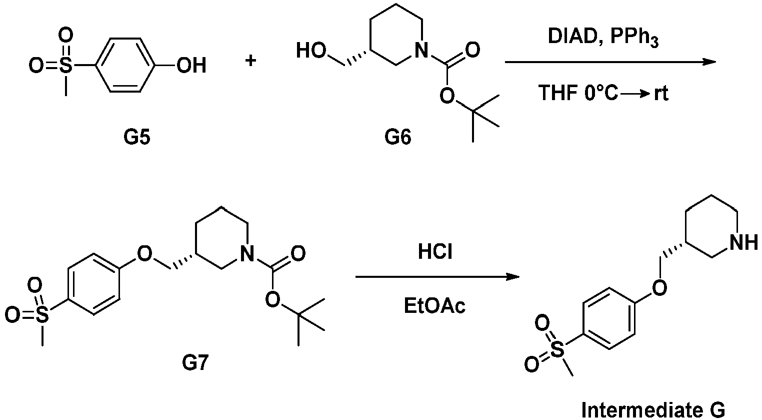

### Step 1: (tert-butyl (R)-3-((4-(methylsulfonyl)phenoxy)methyl)piperidine-1-carboxylate

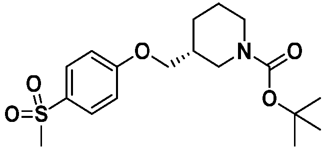

To a solution of *tert-*butyl (*R*)-3-(hydroxymethyl)piperidine-1-carboxylate **(G6,** 1.00 g, 4.64 mmol) and 4-methylsulfonylphenol **(G5,** 800 mg, 4.64 mmol) in THF (20 mL) was added PPh₃ (2.44 g, 9.29 mmol). The mixture was cooled to 0 °C and DIAD (1.88 g, 9.29 mmol, 1.81 mL, 2.00 eq) was added dropwise. After stirring at room temperature for 12 h, the reaction mixture was concentrated in vacuo. The residue was purified by normal phase silica gel chromatography (Biotage 12 g cartridge, 1-25% EtOAc in petroleum ether) to give (*tert*-butyl (*R*)-3-((4-(methylsulfonyl)phenoxy)methyl)piperidine-1-carboxylate **(G7).** MS = 387.2 [M+NH₄]⁺.

### Step 2: (R)-3-((4-(methylsulfonyl)phenoxy)methyl)piperidine

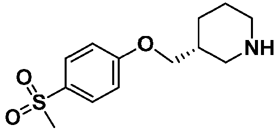

To a solution of (*tert*-butyl (*R*)-3-((4-(methylsulfonyl)phenoxy)methyl)piperidine-1-carboxylate (7, 780 mg, 2.11 mmol, 1.00 eq) in EtOAc (5 mL) was added HCl/EtOAc (4 M, 10 mL). The mixture was stirred at room temperature for 2 h. The suspension was triturated with MTBE (100 mL) and the resulting solid was isolated through filtration to give (*R*)-3-((4-(methylsulfonyl)phenoxy)methyl)piperidine (Intermediate **G**) which was used in the next step without further purification. MS = 270.1 [M+H]⁺.

The following intermediate in Table 3 was prepared according to procedures similar to those described for **Intermediate G** using the appropriate starting materials.

**Table 3**

| Intermediate | Structure | IUPAC Name | Exact Mass [M+H]⁺ |
|---|---|---|---|
| **H** | 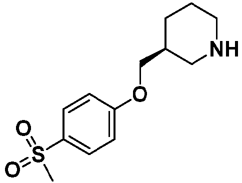 | (*S*)-3-((4-(methylsulfonyl)phenoxy) methyl)piperidine | Calc'd 270.1 |
| | | | Found 270.1 |

### General Procedure for Intermediate I

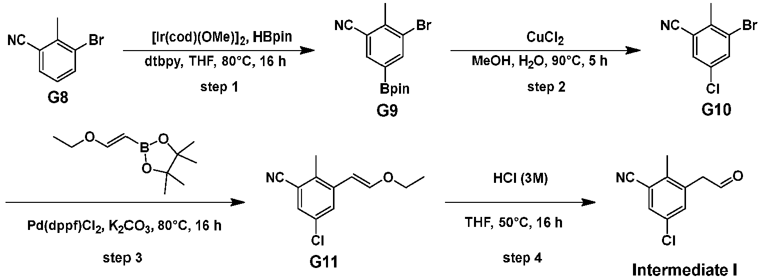

### Step 1: 3-bromo-2-methyl-5-(4,4,5,5-tetramethyl-1,3,2-dioxaborolan-2-yl)benzonitrile

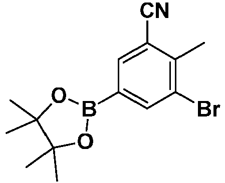

To a solution of 3-bromo-2-methyl-benzonitrile **(G8,** 5.00 g, 25.5 mmol) and 4,4,5,5-tetramethyl-1,3,2-dioxaborolane (32.6 g, 255 mmol) in THF (80 mL) under N₂ was added 4-*tert*-butyl-2-(4-*tert*-butyl-2-pyridyl)pyridine (685 mg, 2.55 mmol) and [Ir(cod)(OMe)]₂ (845 mg, 1.28 mmol). Then the mixture was stirred at 80 °C for 8 h under N₂. The reaction mixture was diluted with H₂O (100 mL) and extracted with EtOAc (100 mL x 3). The combined organic layers were washed with brine (100 mL), dried over Na₂SO₄, filtered and concentrated under reduced pressure. The residue was purified by flash silica gel chromatography (ISCO 80 g cartridge, 0-20% EtOAc:Hexane) to give 3-bromo-2-methyl-5-(4,4,5,5-tetramethyl-1,3,2-dioxaborolan-2-yl)benzonitrile **(G9).** ¹H NMR (400 MHz, CDCl₃): δ 8.15 (s, 1H), 7.98 (s, 1H), 2.65 (s, 3H), 1.35 (s, 12H).

### Step 2: 3-bromo-5-chloro-2-methylbenzonitrile

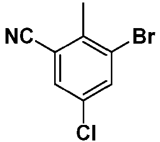

To solution of 3-bromo-2-methyl-5-(4,4,5,5-tetramethyl-1,3,2-dioxaborolan-2-yl)benzonitrile **(G9,** 3.10 g, 9.63 mmol) in MeOH (30 mL) and H₂O (10 mL) was added CuCl₂ (3.88 g, 28.9 mmol) at room temperature, then the mixture was stirred at 90 °C for 16 h. After cooling to room temperature, the reaction mixture was diluted with H₂O (50 mL) and extracted with EtOAc (30 mL x 3). The combined organic layers were washed with brine (20 mL), dried over Na₂SO₄, filtered and concentrated under reduced pressure to give a residue. The residue was purified by flash silica gel chromatography (ISCO 20 g cartridge, 0-5% EtOAc:Hexane) to give 3-bromo-5-chloro-2-methyl-benzonitrile **(G10).** ¹H NMR (400 MHz, CDCl₃): δ 7.78 (s, 1H), 7.57 (s, 1H), 2.61 (s, 3H).

### Step 3: (E)-5-chloro-3-(2-ethoxyvinyl)-2-methylbenzonitrile

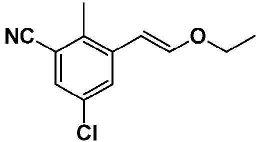

To a solution of 3-bromo-5-chloro-2-methyl-benzonitrile **(G10,** 300 mg, 1.30 mmol) and 2-[(*E*)-2-ethoxyvinyl]-4,4,5,5-tetramethyl-1,3,2-dioxaborolane (296 mg, 1.50 mmol) in 1,4-dioxane (5 mL) under N₂ were added K₂CO₃ (360 mg, 2.60 mmol) and Pd(dppf)Cl₂ (95.2 mg, 130 µmol). Then the mixture was stirred at 80 °C for 16 h. The reaction mixture was allowed to cool to room temperature, diluted with H₂O (10 mL), and extracted with EtOAc (10 mL x 3). The combined organic layers were washed with brine (20 mL), dried over Na₂SO₄, filtered and concentrated under reduced pressure to give a residue. The residue was purified by flash silica gel chromatography (ISCO 4 g cartridge, 0-5% EtOAc:Hexane) to give 5-chloro-3-[(*E*)-2-ethoxyvinyl]-2-methyl-benzonitrile **(G11).** MS = 222.1 [M+H]⁺.

### Step 4: 5-chloro-2-methyl-3-(2-oxoethyl)benzonitrile (Intermediate I)

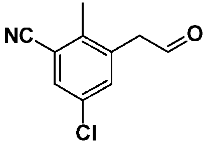

To a solution of 5-chloro-3-[(*E*)-2-ethoxyvinyl]-2-methyl-benzonitrile **(G11,** 1.50 g, 6.77 mmol) in THF (12 mL) was added aqueous HCl (4 M, 12 mL), and then the mixture was stirred at 50 °C for 5 h. The reaction mixture was diluted with H₂O (30 mL) and extracted with EtOAc (10 mL x 3). The combined organic layers were washed with brine (30 mL), dried over Na₂SO₄, filtered and concentrated under reduced pressure to give a residue. The residue was purified by flash silica gel chromatography (ISCO 12 g cartridge, 0-50% EtOAc:Hexane) to give 5-chloro-2-methyl-3-(2-oxoethyl)benzonitrile **(Intermediate I).** MS = 194.0 [M+H]⁺.

### General Procedure for Intermediate J

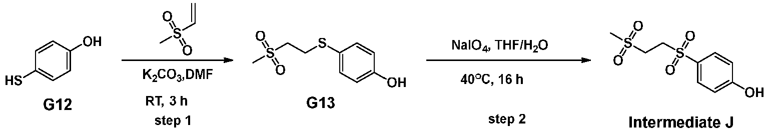

### Step 1: 4-((2-(methylsulfonyl)ethyl)thio)phenol

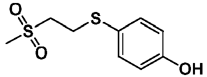

To a solution of 4-sulfanylphenol **(G12,** 4.75 g, 37.7 mmol) and 1-methylsulfonylethylene (4 g, 37.7 mmol) in DMF (40 mL) was added K₂CO₃ (7.81 g, 56.5 mmol). The mixture was stirred at room temperature for 3 h. The reaction mixture was diluted with H₂O (100 mL) and then extracted with EtOAc (100 mL x 3). The combined organic layers were washed with brine (100 mL), dried over Na₂SO₄, filtered and concentrated under reduced pressure to give a residue. The residue was purified by flash silica gel chromatography (Biotage 40 g cartridge, 0-40% EtOAc:Hexane) to give 4-((2-(methylsulfonyl) ethyl) thio) phenol (G13). MS = 231.1 [M-H]⁻.

### Step 2: 4-((2-(methylsulfonyl)ethyl)sulfonyl)phenol (Intermediate J)

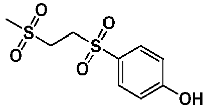

To a solution of 4-(2-methylsulfonylethylsulfanyl) phenol **(G13,** 3.00 g, 12.9 mmol) in THF (40 mL) at room temperature was added a mixture of NaIO₄ (5.50 g, 25.8 mmol) in H₂O (10 mL). The mixture was then stirred at 40 °C for 16 h. The reaction mixture was diluted with saturated aqueous Na₂SO₃ (20 mL), extracted with EtOAc (20 mL x 3), dried over Na₂SO₄, filtered and concentrated under reduced pressure to give a residue. The residue was purified by flash silica gel chromatography (ISCO 40 g cartridge, 0-100% EtOAc:Hexane) to give 4-(2-methylsulfonylethylsulfonyl) phenol **(Intermediate J).** MS = 263.1 [M-H]⁻.

### General Procedure for Intermediate K

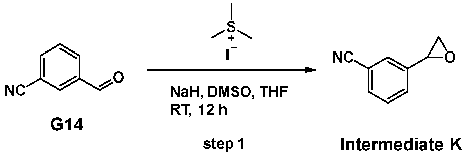

### Step 1: 3-(oxiran-2-yl)benzonitrile (Intermediate K)

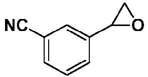

To a suspension of NaH (73 mg, 1.83 mmol, 60% by weight in mineral oil) in THF (5 mL) was added a solution of trimethylsulfonium iodide (374 mg, 1.83 mmol) in DMSO (3 mL) under N₂. After 10 min, a solution of 3-formylbenzonitrile **(G14,** 200 mg, 1.53 mmol) in THF (2 mL) was slowly added. The reaction mixture was degassed and purged with N₂ for 3 times, and then the mixture was stirred at room temperature for 12 h under N₂. The reaction mixture was quenched by addition of H₂O (10 mL) and extracted with EtOAc (10 mL x 3). The combined organic layers were washed with brine (10 mL x 3), dried over Na₂SO₄, filtered and concentrated under reduced pressure to give a residue. The residue was purified by flash silica gel chromatography (Biotage 4 g cartridge, 0-50% EtOAc:Hexane) to give 3-(oxiran-2-yl)benzonitrile **(Intermediate K).** MS = 146.1 [M+H]⁺.

### General Procedure for Intermediate M

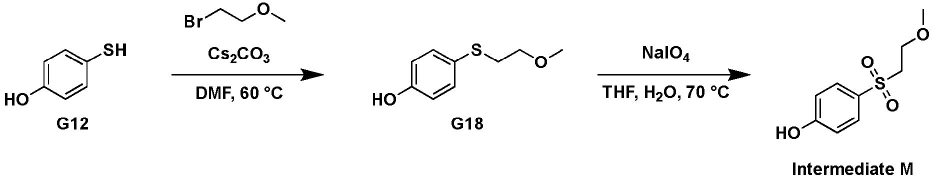

### Step 1: 4-((2-methoxyethyl)thio)phenol

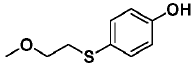

A mixture of 4-sulfanylphenol **(G12,** 1 g, 7.93 mmol,), 1-bromo-2-methoxyethane (1.10 g, 7.93 mmol) and Cs₂CO₃ (2.58 g, 7.93 mmol) in DMF (12 mL) was stirred at 60°C for 3 h. The reaction mixture was poured into ice water (30 mL) and extracted with EtOAc (30 mL x 3). The combined organic layers were washed with brine (50 mL), dried over Na₂SO₄, filtered and concentrated under reduced pressure. The residue was purified by flash silica gel chromatography (Biotage 20 g cartridge, 0-20% EtOAc:Hexane) to give 4-(2-methoxyethylsulfanyl)phenol **(G18).** MS = 183.1 [M-H]⁻.

### Step 2: 4-((2-methoxyethyl)sulfonyl)phenol (Intermediate M)

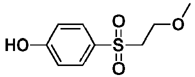

A mixture of 4-(2-methoxyethylsulfanyl)phenol **(G18,** 700 mg, 3.80 mmol) and NaIO₄ (2.44 g, 11.4 mmol) in THF (6 mL) and H₂O (6 mL) was stirred at 70 °C for 16 h. The mixture was filtered, quenched with saturated aqueous Na₂S₂O₃ (15 mL) and extracted with EtOAc (20 mL x 3). The combined organic layers were washed with brine (40 mL), dried over Na₂SO₄, filtered and the filtrate was concentrated under reduced pressure. The residue was purified by flash silica gel chromatography (Biotage 20 g cartridge, 0-70% EtOAc:Hexane) to give 4-(2-methoxyethylsulfonyl)phenol **(Intermediate M).** MS = 217.1 [M+H]⁺.

The following intermediate in Table 4 was prepared according to procedures similar to those described for **Intermediate M** using the appropriate starting materials.

**Table 4**

| Intermediate | Structure | IUPAC Name | Exact Mass [M-H]⁻ |
|---|---|---|---|
| **N** | 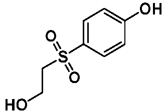 | 4-(2-hydroxy ethylsulfonyl)phenol | Calc'd 201.1 |
| | | | Found 201.1 |

### General Procedure for Intermediate O and Intermediate P

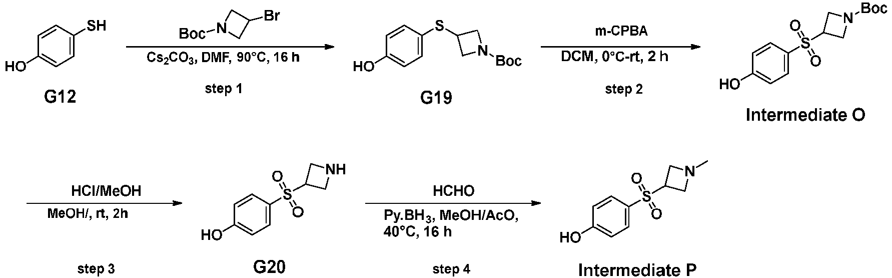

### Step 1: tert-butyl 3-((4-hydroxyphenyl)thio)azetidine-1-carboxylate

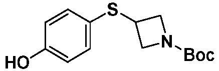

To a solution of *tert-*butyl 3-bromoazetidine-1-carboxylate (1.87 g, 7.93 mmol) in DMF (30 mL) at room temperature were added Cs₂CO₃ (5.16 g, 15.85 mmol) and 4-sulfanylphenol **(G12,** 1.00 g, 7.93 mmol), and the resulting mixture was stirred at 90 °C for 16 h. The reaction mixture was poured into water (30 mL) and extracted with EtOAc (30 mL x 3). The combined organic layers were washed with brine (50 mL), dried over Na₂SO₄, filtered and concentrated under reduced pressure to give a residue. The residue was purified by flash silica gel chromatography (Biotage 12 g cartridge, 0-100% EtOAc:hexane) to give *tert-*butyl 3-(4-hydroxyphenyl)sulfanylazetidine-1-carboxylate **(G19).** MS = 282.1 [M+H]⁺.

### Step 2: tert-butyl 3-((4-hydroxyphenyl)sulfonyl)azetidine-1-carboxylate (Intermediate O)

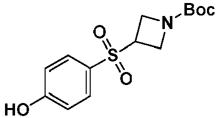

To a solution of *tert-*butyl 3-(4-hydroxyphenyl)sulfanylazetidine-1-carboxylate **(G19,** 50 mg, 178 µmol) in DCM (2 mL) at 0 °C was added m-CPBA (46 mg, 267 µmol, 77% purity) at 0 °C. The mixture was stirred at room temperature for 2 h. The reaction mixture was quenched by addition saturated aqueous Na₂SO₃ (5 mL), and then extracted with EtOAc (5 mL x 3). The combined organic layers were washed with brine (5 mL x 3), dried over Na₂SO₄, filtered and concentrated under reduced pressure to give a residue. The residue was purified by by flash silica gel chromatography (Biotage 4 g cartridge, 0-50% EtOAc:Hexane) to give *tert*-butyl 3-(4-hydroxyphenyl)sulfonylazetidine-1-carboxylate **(Intermediate O).** MS = 258.0 [M-C₄H₈+H]⁺.

### Step 3: 4-(azetidin-3-ylsulfonyl)phenol

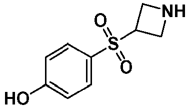

To a solution of *tert-*butyl 3-(4-hydroxyphenyl)sulfonylazetidine-1-carboxylate **(Intermediate O**, 0.5 g, 1.91 mmol) in MeOH (3 mL) was added HCl/MeOH (4 M, 3 mL). The reaction mixture was stirred at room temperature for 2 h. The reaction mixture was then concentrated under reduced pressure to give 4-(azetidin-3-ylsulfonyl)phenol HCl salt **(G20),** which was taken to the next step without further purification. MS = 214.1 [M+H]⁺.

### Step 4: 4-((1-methylazetidin-3-yl)sulfonyl)phenol (Intermediate P)

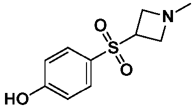

To a solution of 4-(186zetidine-3-ylsulfonyl)phenol **(G20,** 400 mg, 1.60 mmol, HCl salt) in MeOH (5 mL) and AcOH (0.05 mL) were added HCHO (120 mg, 4.00 mmol) and borane-2-methylpyridine complex (205 mg, 1.90 mmol). The mixture was stirred at 40 °C for 16 h. The reaction mixture was quenched by addition of saturated aqueous NaHCO₃ (10 mL), and then extracted with EtOAc (10 mL x 3). The combined organic layers were washed with brine (10 mL), dried over Na₂SO₄, filtered and concentrated under reduced pressure to give a residue. The residue was purified by flash silica gel chromatography (Biotage 12 g cartridge, 0-100% EtOAc:Hexane) to give 4-(1-methylazetidin-3-yl)sulfonylphenol **(Intermediate P).** MS = 228.0 [M+H]⁺.

### General Procedure for Intermediate Q

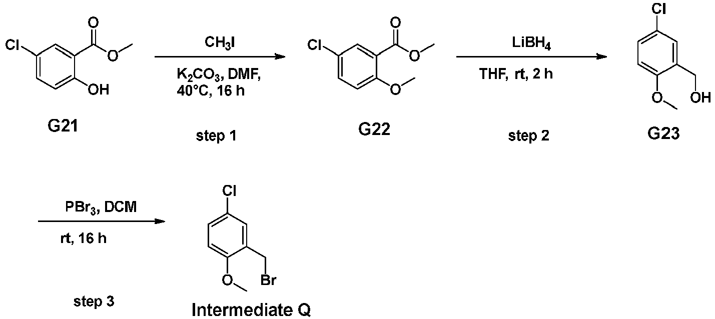

### Step 1: methyl 5-chloro-2-methoxybenzoate

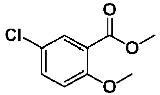

To a solution of methyl 5-chloro-2-hydroxy-benzoate **(G21,** 2.00 g, 10.7 mmol) in DMF (20 mL) were added K₂CO₃ (2.96 g, 21.4 mmol) and CH₃I (7.61 g, 53.6 mmol). The mixture was stirred at 40 °C for 16 h. The reaction mixture was then cooled to room temperature, poured into water (100 mL), and extracted with EtOAc (30 mL x 3). The combined organic layers were washed with brine (80 mL), dried over Na₂SO₄, filtered and concentrated under reduced pressure to give the residue, which was purified by flash silica gel chromatography (ISCO 20 g cartridge, 0-10% EtOAc:Hexane) to give methyl 5-chloro-2-methoxy-benzoate **(G22).** MS = 201.0 [M+H]⁺.

### Step 2: (5-chloro-2-methoxyphenyl)methanol

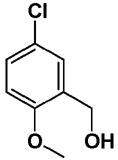

To a solution of methyl 5-chloro-2-methoxy-benzoate **(G22,** 1.50 g, 7.48 mmol) in THF (15 mL) was added LiBH₄ (2 M in THF, 7.5 mL, 15.0 mmol) at 0 °C. The reaction mixture was stirred at room temperature for 2 h. The reaction was quenched with water (20 mL) and extracted with EtOAc (20 mL x 3). The combined organic layers were washed with brine (60 mL), dried over Na₂SO₄, filtered and concentrated to give a residue. The residue was purified by flash silica gel chromatography (ISCO 20 g cartridge, 0-50% EtOAc:Hexane) to give (5-chloro-2-methoxy-phenyl)methanol **(G23).** ¹H NMR (400 MHz, CDCl₃): δ 7.30 (d, *J* = 2.8 Hz, 1H), 7.25 - 7.21 (m, 1H), 6.81 (d, *J* = 8.8 Hz, 1H), 4.66 (s, 2H), 3.86 (s, 3H).

### Step 3: 2-(bromomethyl)-4-chloro-1-methoxybenzene (Intermediate Q)

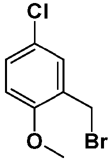

To a solution of (5-chloro-2-methoxy-phenyl)methanol **(G23,** 1.10 g, 6.37 mmol) in DCM (50 mL) at 0 °C was added PBr₃ (1.73 g, 6.37 mmol). The reaction mixture was then stirred at room temperature for 16 h. The mixture was concentrated, diluted with water (50 mL) and neutralized by addition of saturated aqueous NaHCO₃ to pH=7-8, then extracted with DCM (50 mL x 3). The combined organic layers were washed with brine (100 mL), dried over Na₂SO₄, filtered and concentrated to give 2-(bromomethyl)-4-chloro-1-methoxy-benzene **(Intermediate Q).** ¹H NMR (400 MHz, CDCl₃): δ 7.27 (d, *J =* 2.8 Hz, 1H), 7.21 (dd, *J=* 5.6 Hz, 2.8 Hz, 1H), 6.77 (d, *J* = 8.8 Hz, 1H), 4.45 (s, 2H), 3.85 (s, 3H).

### General Procedure for Intermediate R

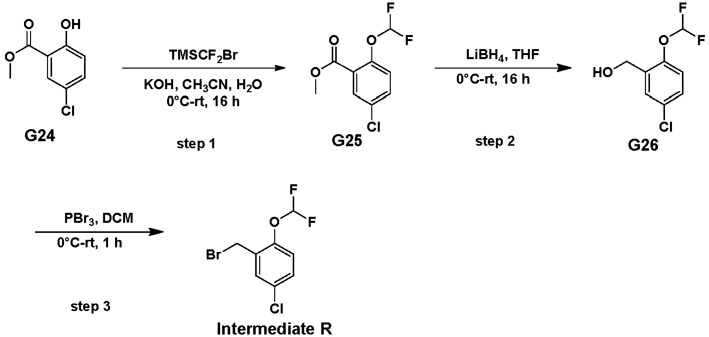

### Step 1: methyl 5-chloro-2-(difluoromethoxy)benzoate

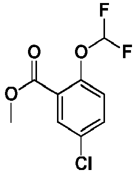

To a mixture of methyl 5-chloro-2-hydroxy-benzoate **(G24,** 1.50 g, 8.04 mmol,) in MeCN (75 mL) and H₂O (37.5 mL) were added KOH (2.71 g, 48.2 mmol) and [bromo(difluoro)methyl]-trimethylsilane (3.27 g, 16.1 mmol) at 0 °C. The mixture was then stirred at room temperature for 16 h. The reaction mixture was poured into water (20 mL) and extracted with EtOAc (20 mL x 3). The combined organic layers were washed with brine (25 mL), dried over Na₂SO₄, filtered and concentrated under reduced presure to give a residue. The residue was purified by flash silica gel chromatography (ISCO 20 g cartridge, 0-20% EtOAc:Hexane) to give methyl 5-chloro-2-(difluoromethoxy)benzoate **(G25).** ¹H NMR (400 MHz, DMSO-*d*₆): δ 7.86 (d, *J =* 2.8 Hz, 1H), 7.75 (dd, *J* = 8.8 Hz, 2.8 Hz, 1H), 7.37 (d, *J* = 9.2 Hz, 1H), 7.20 (t, *J=* 73.6 Hz, 1H), 7.21 - 7.03 (m, 1H), 3.85 (s, 3H).

### Step 2: (5-chloro-2-(difluoromethoxy)phenyl)methanol

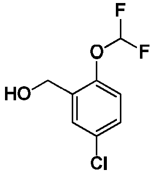

To a mixture of methyl 5-chloro-2-(difluoromethoxy)benzoate **(G25,** 200 mg, 845 µmol) in THF (5 mL) at 0 °C was added LiBH₄ (4 M in THF, 634 µL, 2.54 mmol) dropwise under N₂, the mixture was then stirred at room temperature for 16 h under N₂. The reaction mixture was poured into saturated aqueous NH₄Cl (5 mL), then extracted with EtOAc (15 mL x 3). The combined organic layers were washed with brine (5 mL), dried over anhydrous Na₂SO₄, filtered and concentrated under reduced pressure to give [5-chloro-2-(difluoromethoxy)phenyl]methanol **(G26),** which was used without further purification. MS = 190.9 [M-H₂O+H]⁺.

### Step 3: 2-(bromomethyl)-4-chloro-1-(difluoromethoxy) benzene (Intermediate R)

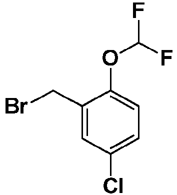

To a solution of [5-chloro-2-(difluoromethoxy)phenyl]methanol **(G26,** 170 mg, 815 µmol) in DCM (3 mL) was added PBr₃ (221 mg, 815 µmol) at 0 °C. The mixture was stirred at room temperature for 1 h. The reaction mixture was then poured into water (15 mL) and extracted with DCM (10 mL x 3). The combined organic layers were washed with brine (5 mL), dried over anhydrous Na₂SO₄, filtered and concentrated under reduced pressure to give a residue. The residue was purified by flash silica gel chromatography (ISCO 4g cartridge, 0-10% EtOAc:Hexane) to give 2-(bromomethyl)-4-chloro-1-(difluoromethoxy)benzene **(Intermediate R).** ¹H NMR (400 MHz, DMSO-*d*₆): δ 7.69 (d, *J =* 2.4 Hz, 1H), 7.50 (dd, *J =* 8.0 Hz, 2.8 Hz, 1H), 7.25 (d, *J* = 8.8 Hz, 1H), 7.31 (t, *J* = 73.2 Hz, 1H), 4.63 (s, 2H).

### General Procedure for Intermediate S

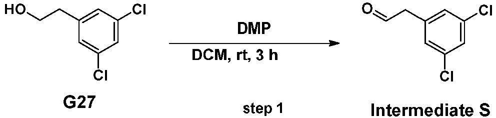

### Step 1: 2-(3, 5-dichlorophenyl) acetaldehyde (Intermediate S)

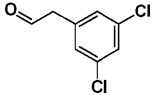

To a mixture of 2-(3,5-dichlorophenyl)ethanol **(G27,** 1.00 g, 5.23 mmol) in DCM (10 mL) was added DMP (2.66 g, 6.28 mmol). The mixture was then stirred at room temperature for 3 h under N₂. The reaction mixture was diluted with H₂O (5 mL) and extracted with EtOAc (8 mL x 3). The combined organic layers were washed with brine (10 mL), dried over Na₂SO₄, filtered and concentrated under reduced pressure to give a residue. The residue was purified by flash silica gel chromatography (Biotage 12 g cartridge, 0-20% EtOAc:Hexane) to give 2-(3, 5-dichlorophenyl) acetaldehyde **(Intermediate S).** ¹H NMR (400 MHz, DMSO-*d*₆): δ 9.68 (s, 1H), 7.53 (s, 1H), 7.47 (s, 1H), 7.46 (s, 1H), 3.86 (s, 2H).

The following intermediate in Table 5 was prepared according to procedures similar to those described for **Intermediate S** using the appropriate starting materials.

**Table 5**

| Intermediate | Structure | IUPAC Name | ¹H NMR (400 MHz, DMSO-*d*₆): |
|---|---|---|---|
| **T** | 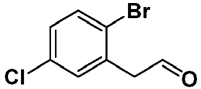 | 2-(2-bromo-5-chlorophenyl)acetaldehyde | δ 9.70 (s, 1H), 7.66 (d, *J* = 8.8 Hz, 1H), 7.51 (d, *J* = 2.4 Hz, 1H), 7.32 (d, *J =* 8.4 Hz, 2.4 Hz, 1H), 3.98 (s, 2H). |
| **U** | 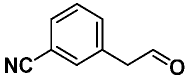 | 3-(2-oxoethyl)benzonitrile | δ 9.80 (t, *J =* 2.0 Hz, 1H), 7.63 - 7.52 (m, 4H), 3.92 - 3.88 (m, 2H). |

### General Procedure for Intermediate V

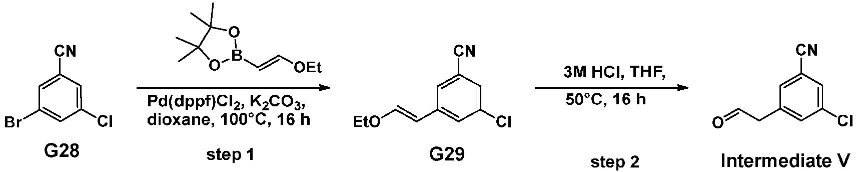

### Step 1: (E)-3-chloro-5-(2-ethoxyvinyl) benzonitrile

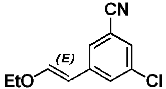

To a solution of 2-[(E)-2-ethoxyvinyl]-4,4,5,5-tetramethyl-1,3,2-dioxaborolane (503 mg, 2.54 mmol) and 3-bromo-5-chloro-benzonitrile **(G28,** 500 mg, 2.31 mmol) in dioxane (10 mL) were added K₂CO₃ (958 mg, 6.93 mmol) and Pd(dppf)Cl₂ (169 mg, 231 µmol) under N₂. The reaction mixture was then stirred at 100 °C for 16 h under N₂. The reaction mixture was diluted with H₂O (10 mL) and extracted with EtOAc (5 mL x 3). The combined organic layers were washed with brine (10 mL), dried over Na₂SO₄, filtered and concentrated under reduced pressure to give (E)-3-chloro-5-(2-ethoxyvinyl)benzonitrile **(G29),** which was taken to the next step without further purification.¹H NMR (400 MHz, CDCl₃): δ 7.40 (s, 1H), 7.36 (s, 1H), 7.35 (s, 1H), 7.05 (d, *J* = 13.2 Hz, 1H), 5.73 (d, *J* = 12.8 Hz, 1H), 3.94 (q, *J* = 7.2 Hz, 2H), 1.37 (t, *J* = 7.2 Hz, 3H).

### Step 2: 3-chloro-5-(2-oxoethyl) benzonitrile (Intermediate V)

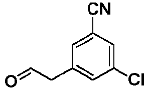

To a solution of (E)-3-chloro-5-(2-ethoxyvinyl)benzonitrile **(G29,** 750 mg, 3.61 mmol) in THF (4 mL) was added HCl (3 M in H₂O, 4 mL). The reaction mixture was then stirred at 50 °C for 16 h. The reaction mixture was diluted with H₂O (10 mL) and extracted with EtOAc (5 mL x 3). The combined organic layers were washed with brine (10 mL), dried over Na₂SO₄, filtered and concentrated under reduced pressure to give a residue. The residue was purified by flash silica gel chromatography (Biotage 4 g cartridge, 0-30% EtOAc:Hexane) to give 3-chloro-5-(2-oxoethyl)benzonitrile **(Intermediate V).** ¹H NMR (400 MHz, CDCl₃): δ 9.81 (s, 1H), 7.60 (s, 1H), 7.46 (s, 1H), 7.42 (s, 1H), 3.80 (s, 2H).

The following intermediate in Table 6 was prepared according to procedures similar to those described for **Intermediate V** using the appropriate starting materials.

**Table 6**

| Intermediate | Structure | IUPAC Name | ¹H NMR (400 MHz, CDCl₃) |
|---|---|---|---|
| **W** | 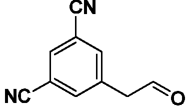 | 5-(2-oxoethyl) isophthalonitrile | δ 9.86 (s, 1H), 7.90 (s, 1H), 7.81 (s, 1H), 7.74 (s, 1H), 3.49 (s, 2H). |

### General Procedure for Intermediate X

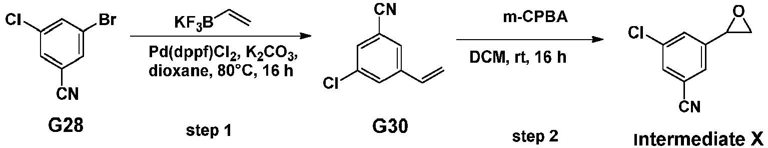

### Step 1: 3-chloro-5-vinylbenzonitrile

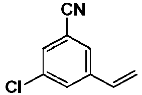

To a solution of 3-bromo-5-chloro-benzonitrile **(G28,** 2.50 g, 11.5 mmol), potassium vinyltrifluoroborate (3.09 g, 23.1 mmol) in dioxane (25 mL) and H₂O (2.5 mL) were added K₂CO₃ (3.19 g, 23.1 mmol) and Pd(dppf)Cl₂ (845 mg, 1.15 mmol). The mixture was purged with N₂ three times and then stirred at 80 °C for 16 h. The reaction mixture was poured into water (50 mL) and extracted with EtOAc (40 mL x 3). The combined organic layers were washed with brine (100 mL), dried over Na₂SO₄, filtered and concentrated under reduced pressure to give a residue. The residue was purified by flash silica gel chromatography (BIOTAGE 20 g cartridge, 0-10% EtOAc:Hexane) to give 3-chloro-5-vinyl-benzonitrile **(G30).** ¹H NMR (400 MHz, CDCl₃): δ 7.60 (s, 1H), 7.56 (s, 1H), 7.52 (s, 1H), 6.69 - 6.62 (m, 1H), 5.85 (d, *J =* 17.6 Hz, 1H), 5.47 (d, *J* = 10.8 Hz, 1H).

### Step 2: 3-chloro-5-(oxiran-2-yl) benzonitrile (Intermediate X)

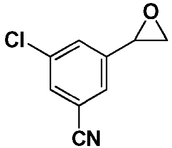

To a solution of 3-chloro-5-vinyl-benzonitrile **(G30,** 500 mg, 3.06 mmol) in DCM (5 mL) was added *m*CPBA (931 mg, 4.58 mmol, 85% purity) at 0 °C, the reaction was stirred at room temperature for 16 h. The reaction mixture was then poured into saturated aqueous Na₂SO₃ (20 mL) and extracted with EtOAc (30 mL x 3). The combined organic layers were washed with brine (120 mL), dried over Na₂SO₄, filtered and concentrated under reduced pressure to give a residue. The residue was purified by flash silica gel chromatography (ISCO 12 g cartridge, 0-100% EtOAc:Hexane) to give 3-chloro-5-(oxiran-2-yl)benzonitrile **(Intermediate X**).The following intermediate in Table 7 was prepared according to procedures similar to those described for **Intermediate X** using the appropriate starting materials.

**Table 7**

| Intermediate | Structure | IUPAC Name | ¹H NMR (400 MHz, CDCl₃): |
|---|---|---|---|
| **Y** | 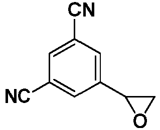 | 5-(oxiran-2-yl) benzene-1, 3-dicarbonitrile | δ 7.69 (s, 1H), 7.62 - 7.61 (m, 2H), 3.77 - 3.75 (m, 1H), 3.07 - 3.05 (m, 1H), 2.58 - 2.56 (m, 1H). |

### General Procedure for Intermediate Z

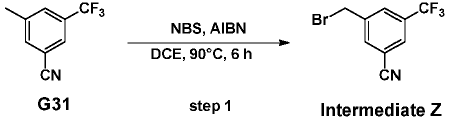

### Step 1: 3-(bromomethyl)-5-(trifluoromethyl) benzonitrile (Intermediate Z)

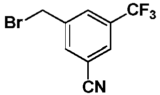

A mixture of 3-methyl-5-(trifluoromethyl) benzonitrile **(G31,** 750 mg, 4.05 mmol), NBS (865 mg, 4.86 mmol) and AIBN (67 mg, 405 µmol) in DCE (7.5 mL) was stirred at 90 °C for 6 h. The mixture was then diluted with DCM (20 mL) and washed with water (20 mL). The organic layer was concentrated under reduced presure. The residue was purified twice by flash silica gel chromatography (Biotage 12 g cartridge, 0-1% EtOAc:Hexane) to give 3-(bromomethyl)-5-(trifluoro methyl)benzonitrile **(Intermediate Z).** MS = 264.0/266.1 [M+H]⁺.

### General Procedure for Intermediate AA

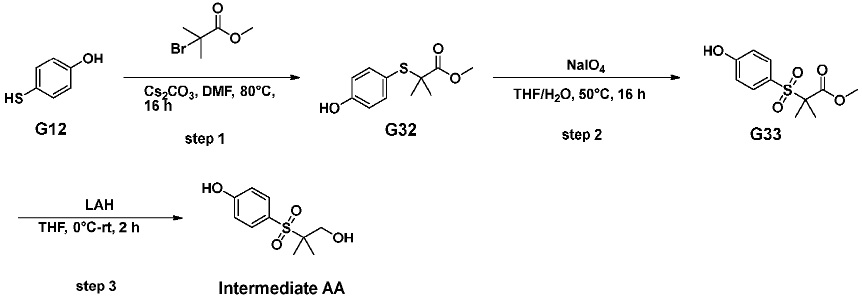

### Step 1: methyl 2-((4-hydroxyphenyl)thio)-2-methylpropanoate

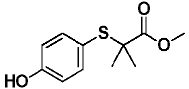

To a mixture of 4-sulfanylphenol **(G12,** 2.00 g, 15.8 mmol) and methyl 2-bromo-2-methyl-propanoate (2.40 g, 13.3 mmol) in DMF (30 mL) was added Cs₂CO₃ (7.20 g, 22.1 mmol). The mixture was then stirred at 80 °C for 16 h. The reaction mixture was diluted with saturated aqueous NH₄Cl (30 mL), extracted with EtOAc (30 mL x 3), dried over Na₂SO₄, filtered and concentrated under reduced pressure to give a residue. The residue was purified by flash silica gel chromatography (ISCO 12 g cartridge, 0-100% EtOAc:Hexane) to give methyl 2-(4-hydroxyphenyl) sulfanyl-2-methyl-propanoate **(G32).** MS = 225.2 [M-H]⁻.

### Step 2: methyl 2-((4-hydroxyphenyl) sulfonyl)-2-methylpropanoate

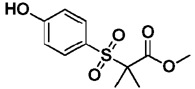

To a solution of methyl 2-(4-hydroxyphenyl) sulfanyl-2-methyl-propanoate **(G32,** 2.30 g, 10.2 mmol) in THF (30 mL) was added a solution of NaIO₄ (6.52 g, 30.5 mmol) in H₂O (6 mL). The mixture was stirred at 50 °C for 16 h. The reaction mixture was quenched with saturated aqueous Na₂SO₃ (15 mL), extracted with EtOAc (10 mL x 3), dried over Na₂SO₄, filtered and concentrated under reduced pressure to give a residue. The residue was purified by flash silica gel chromatography (ISCO 20 g cartridge, 0-30% EtOAc:Hexane) to give methyl 2-(4-hydroxyphenyl) sulfonyl-2-methyl-propanoate **(G33).** MS = 257.1 [M-H]⁻.

### Step 3: 4-((1-hydroxy-2-methylpropan-2-yl) sulfonyl)phenol

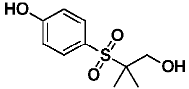

To a solution of methyl 2-(4-hydroxyphenyl) sulfonyl-2-methyl-propanoate **(G33,** 2.30 g, 8.90 mmol) in THF (30 mL) at 0 °C was added LiAlH₄ (507 mg, 13.4 mmol). The mixture was stirred at room temperature for 2 h. The residue was diluted with aqueous NaOH (5 M, 2 mL), dried over Na₂SO₄, filtered and concentrated under reduced pressure to give a residue. The residue was purified by flash silica gel chromatography (ISCO 4 g cartridge, 0-100% EtOAc:Hexane to give 4-(2-hydroxy-1, 1-dimethyl-ethyl) sulfonylphenol **(Intermediate AA).** MS = 229.0 [M-H]⁻.

### General Procedure for Intermediate AB

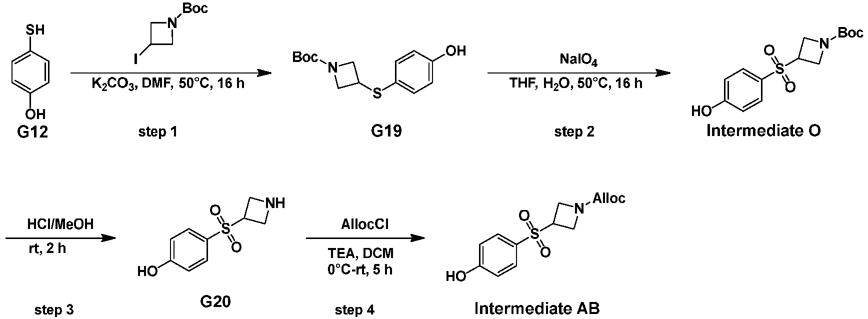

### Step 1: tert-butyl 3-((4-hydroxyphenyl) thio) azetidine-1-carboxylate

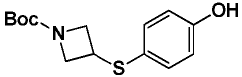

To a solution of 4-sulfanylphenol **(G12,** 10.0 g, 79.2 mmol) in DMF (100 mL) were added K₂CO₃ (10.9 g, 79.2 mmol) and *tert*-butyl 3-iodoazetidine-1-carboxylate (22.4 g, 79.2 mmol). The mixture was then stirred at 40 °C for 16 h. The reaction mixture was diluted with water (200 mL) and extracted with EtOAc (100 mL x 3). The combined organic layers were washed with H₂O (80 mL) and brine (80 mL), dried over (Na₂SO₄), filtered and concentrated under reduced pressure to give a residue. The crude product was triturated with MTBE (100 mL) at room temperature for 5 h and filtered to give tert-butyl 3-(4-hydroxyphenyl)sulfanylazetidine-1-carboxylate **(G19).** MS = 226.1 [M-C₄H₈+H]⁺.

### Step 2: tert-butyl 3-((4-hydroxyphenyl) sulfonyl) azetidine-1-carboxylate

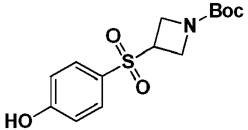

To a solution of *tert*-butyl 3-(4-hydroxyphenyl) sulfanylazetidine-1-carboxylate **(G19,** 6.50 g, 13.9 mmol) in THF (60 mL) and H₂O (20 mL) was added NaIO₄ (8.80 g, 41.6 mmol, ). The mixture was stirred at 50 °C for 16 h. The reaction mixture was cooled to room temperature, quenched with saturated aqueous Na₂SO₃ (60 mL) and extracted with EtOAc (30 mL x 3). The combined organic layers were washed with brine (30 mL), dried over Na₂SO₄, filtered and concentrated under reduced pressure to give a residue. The residue was purified by flash silica gel chromatography (ISCO 20 g cartridge, 0-100% EtOAc:Hexane ) to give *tert-*butyl 3- (4- hydroxyphenyl) sulfonylazetidine-1-carboxylate **(Intermediate O).** MS = 312.2 [M-H]⁻.

### Step 3: 4-(azetidin-3-ylsulfonyl) phenol

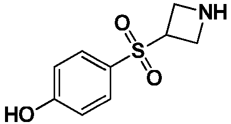

To a solution of *tert*-butyl 3-(4-hydroxyphenyl) sulfonylazetidine-1-carboxylate **(Intermediate O,** 1.5 g, 4.79 mmol) in MeOH (4 mL) was added HCl/MeOH (4 M, 12 mL). The mixture was stirred at room temperature for 2 h. The reaction mixture was concentrated under reduced pressure to give 4-(azetidin-3-ylsulfonyl) phenol HCl salt **(G20),** which was used without further purification. MS = 214.1 [M+H]⁺.

### Step 4: allyl 3-((4-hydroxyphenyl) sulfonyl) azetidine-1-carboxylate (Intermediate AB)

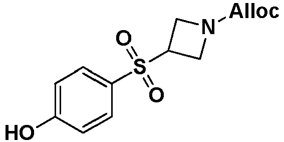

To a solution of 4-(azetidin-3-ylsulfonyl)phenol **(G20,** 1.1 g, 4.41 mmol, HCl salt) in DCM (10 mL) were added TEA (1.11 g, 11.0 mmol, 1.53 mL) and allyl chloroformate (584 mg, 4.85 mmol) at 0 °C. The mixture was then stirred at room temperature for 3 h. The reaction mixture was diluted with H₂O (30 mL) and extracted with DCM (15 mL x 3). The combined organic layers were washed with brine (5 mL), dried over Na₂SO₄, filtered and concentrated under reduced pressure to give a residue. The residue was purified by flash silica gel chromatography (ISCO 12 g cartridge, 0-100% EtOAc:Hexane) to give allyl 3-(4-hydroxyphenyl)sulfonylazetidine-1-carboxylate **(Intermediate AB).** MS = 298.1 [M+H]⁺.

### General Procedure for Intermediate AC

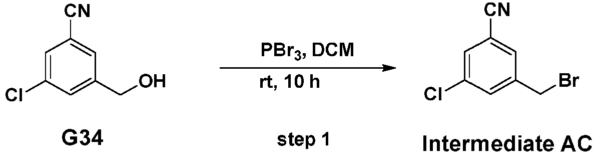

### Step 1: 3-(bromomethyl)-5-chlorobenzonitrile (Intermediate AC)

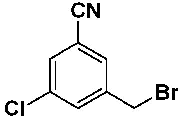

To a solution of 3-chloro-5-(hydroxymethyl)benzonitrile **(G34,** 500 mg, 2.98 mmol) in DCM (10 mL) was added PBr₃ (807 mg, 2.98 mmol) at 0 °C. The reaction mixture was then stirred at room temperature for 10 h, then the reaction mixture was concentrated. The residue was diluted with water (10 mL) and neutralized by addition of saturated aqueous NaHCO₃ to pH = 7, then extracted with EtOAc (10 mL x 3). The combined organic layers were washed with brine (20 mL), dried over Na₂SO₄, filtered and concentrated to give 3-(bromomethyl)-5-chloro-benzonitrile **(Intermediate AC),** which was taken to the next step without further purification. ¹H NMR (400 MHz, CDCl₃): δ 7.63 (s, 1H), 7.58 (s, 2H), 4.43 (s, 2H).

### General Procedure for Intermediate AD

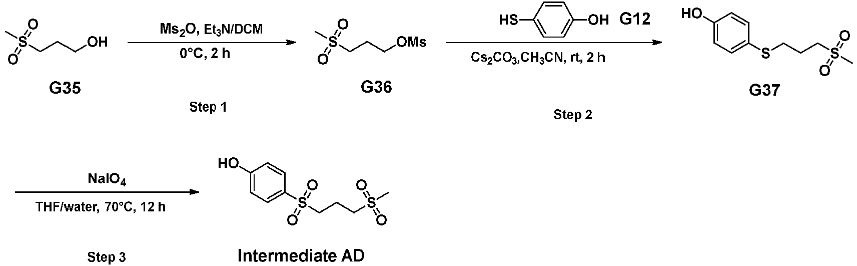

### Step 1: 3-(methylsulfonyl)propyl methanesulfonate

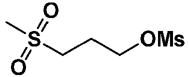

To a solution of 3-methylsulfonylpropan-1-ol **(G35,** 500 mg, 3.62 mmol) in DCM (5 mL) at 0 °C were added Et₃N (732 mg, 7.24 mmol) and methylsulfonyl methanesulfonate (945 mg, 5.43 mmol). The mixture was stirred at 0 °C for 2 h. The reaction mixture was diluted with H₂O (20 mL) and extracted with EtOAc (10 mL x 3). The combined organic layers were washed with brine (30 mL), dried over Na₂SO₄, filtered and concentrated under reduced pressure to give crude 3-(methylsulfonyl)propyl methanesulfonate **(G36),** which was taken to the next step without further purification. ¹H NMR (400 MHz, DMSO-*d*₆): δ 4.30 (t, *J* = 6.4 Hz, 2H), 3.23 - 3.01 (m, 5H), 3.01 (s, 3H), 2.13 - 2.08 (m, 2H).

### Step 2: 4-((3-(methylsulfonyl)propyl)thio)phenol

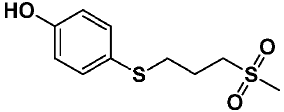

To a mixture of 3-(methylsulfonyl)propyl methanesulfonate **(G36,** 390 mg, 1.80 mmol) and 4-sulfanylphenol **(G12,** 318 mg, 2.52 mmol) in CH₃CN (5 mL) was added Cs₂CO₃ (705 mg, 2.16 mmol). The mixture was then stirred at room temperature for 2 h. The reaction mixture was then diluted with H₂O (20 mL) and extracted with EtOAc (20 mL x 3). The combined organic layers were washed with brine (40 mL), dried over Na₂SO₄, filtered and concentrated under reduced pressure to give a residue. The residue was purified by flash silica gel chromatography (ISCO 12 g cartridge, 0-50% EtOAc:Hexane) to give 4-(3-methylsulfonylpropylsulfanyl)phenol **(G37).** MS = 245.1 [M-H]⁻.

### Step 3: 4-((3-(methylsulfonyl)propyl)sulfonyl)phenol (Intermediate AD)

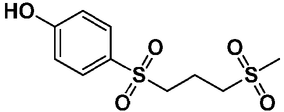

To a solution of 4-(3-methylsulfonylpropylsulfanyl)phenol **(G37,** 370 mg, 1.50 mmol) in THF (2 mL) and H₂O (2 mL) was added NaIO₄ (964 mg, 4.51 mmol) at 0 °C. The mixture was then stirred at 70 °C for 12 h. The reaction mixture was quenched by addition of saturated aqueous Na₂SO₃ (10 mL), and then diluted with H₂O (5 mL) and extracted with EtOAc (15 mL x 3). The combined organic layers were washed with brine (30 mL), dried over Na₂SO₄, filtered and concentrated under reduced pressure to give a residue. The residue was purified by flash silica gel chromatography (ISCO 12 g cartridge, 0-50% EtOAc:Hexane) to give 4-(3-methylsulfonylpropylsulfonyl)phenol **(Intermediate AD).** MS = 277.1 [M-H]⁻.

### General Procedure for Intermediate AE

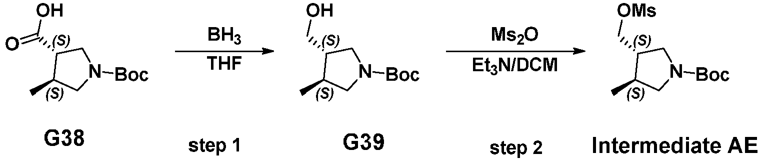

### Step 1: tert-butyl (3S,4S)-3-(hydroxymethyl)-4-methylpyrrolidine-1-carboxylate

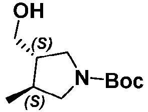

To a solution of (3S,4S)-1-*tert*-butoxycarbonyl-4-methyl-pyrrolidine-3-carboxylic acid **(G38,** 5.00 g, 21.8 mmol) in THF (50 mL) was added BH₃ (10 M in Me₂S, 10.9 mL, 109 mmol) at 0 °C. Then the reaction was stirred at room temperature for 16 h. The reaction mixture was quenched by addition of MeOH (40 mL) and stirred 0.5 h. The mixture was then diluted with water (100 mL) and extracted with EtOAc (60 mL x 3). The combined organic layers were washed with brine (100 mL), dried over Na₂SO₄, filtered and the filtrate was concentrated to give *tert*-butyl (3S,4S)-3-(hydroxymethyl)-4-methyl-pyrrolidine-1-carboxylate **(G39).** MS = 160.2 [M-C₄H₈+H]⁺.

### Step 2: tert-butyl (3S,4S)-3-methyl-4-(((methylsulfonyl)oxy)methyl)pyrrolidine-1-carboxylate (Intermediate AE)

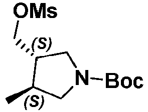

To a solution of *tert*-butyl (3*S*,4*S*)-3-(hydroxymethyl)-4-methyl-pyrrolidine-1-carboxylate **(G39,** 200 mg, 929 µmol) in DCM (3 mL) were added Et₃N (188 mg, 1.86 mmol) and methylsulfonyl methanesulfonate (161 mg, 929 µmol) at 0 °C. The mixture was stirred at room temperature for 2 h. The reaction mixture was then concentrated under reduced pressure. The residue was diluted with saturated aqueous NH₄Cl (5 mL), extracted with EtOAc (5 mL x 3), dried over Na2_{S}O₄, filtered and concentrated under reduced pressure to give *tert*-butyl (3*S*,4*S*)-3-methyl-4-(((methylsulfonyl)oxy)methyl)pyrrolidine-1-carboxylate **(Intermediate AE).** ¹H NMR (400 MHz, CDCl₃): δ 4.32 - 4.28 (m, 1H), 4.16 - 4.14 (m, 1H), 3.65 - 3.61 (m, 3H), 3.20 - 3.18 (m, 1H), 3.03 (s, 3H), 2.97 - 2.95 (m, 1H), 2.33 - 2.20 (m, 4H), 1.46 (s, 9H).

The following intermediate in Table 8 was prepared according to procedures similar to those described for **Intermediate AE** using the appropriate starting materials.

**Table 8**

| Intermediate | Structure | IUPAC Name | ¹H NMR (400 MHz, DMSO-*d*₆): |
|---|---|---|---|
| **AF** | 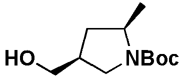 | *tert-*butyl (2*R,*4*S*)-2-methyl-4-(methylsulfonyloxymethyl) pyrrolidine-1-carboxylate | δ 4.24 - 4.14 (m, 2H), 3.73 - 3.71 (m, 1H), 3.63 - 3.58 (m, 1H), 3.18 (s, 3H), 2.96 - 2.92 (m, 1H), 2.25 - 2.23 (m, 1H), 2.21 - 2.18 (m, 1H), 1.39 (s, 9H), 1.32 - 1.27 (m, 1H), 1.86 (d, *J* = 3.2 Hz, 3H). |

In the following Examples, the numbers in the column headed with "No." in each of Tables 9-25 refer to the corresponding compound numbers in Table 1.

### Example 1

### 1-(3-chlorophenethyl)-3-((4-(methylsulfonyl)phenoxy)methyl)azepane (Compound 1)

### (R) or (S)-1-(3-chlorophenethyl)-3-((4-(methylsulfonyl)phenoxy)methyl)azepane (Compound 2)

### (S) or (R)-1-(3-chlorophenethyl)-3-((4-(methylsulfonyl)phenoxy)methyl)azepane (Compound 3)

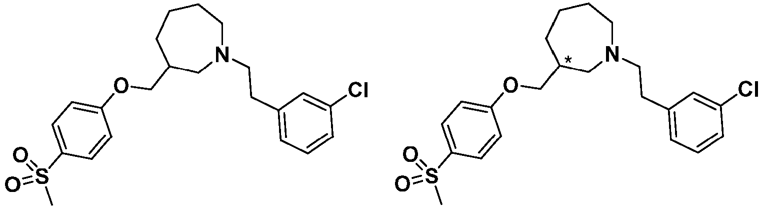

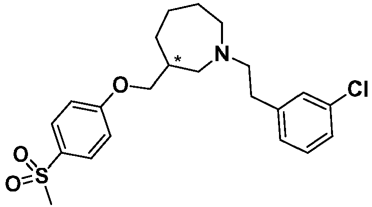

### Step 1: tert-butyl 3-((4-(methylsulfonyl)phenoxy)methyl)azepane-1-carboxylate

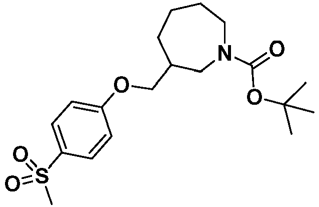

To a mixture of *tert*-butyl-3-(hydroxymethyl)azepane-1-carboxylate (200 mg, 0.872 mmol), 4-(methylsulfonyl)phenol (195 mg, 1.13 mmol), and PPh₃ (297 mg, 1.13 mmol) in THF (5 mL) at 0 °C was added DIAD (509 µL, 2.62 mmol) dropwise. The mixture was stirred at room temperature for 12 h. The reaction mixture was cooled to 0 °C, quenched by the addition of water (20 mL), then extracted with EtOAc (25 mL x 2). The combined organic layers were washed with brine (20 mL), dried over Na₂SO₄, filtered and concentrated in vacuo. The crude residue was purified by normal phase silica gel chromatography (Biotage 10 g cartridge, 0-85% EtOAc in petroleum ether) to give *tert*-butyl 3-((4-(methylsulfonyl)phenoxy)methyl)azepane-1-carboxylate. MS = 384.2 [M+H]⁺.

### Step 2: 3-((4-(methylsulfonyl)phenoxy)methyl)azepane

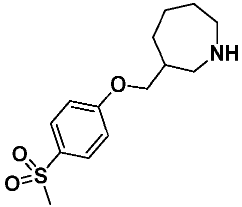

To a solution of tert-butyl 3-((4-(methylsulfonyl)phenoxy)methyl)azepane-1-carboxylate (260 mg, 0.678 mmol) in EtOAc (2 mL) was added HCl/EtOAc (4 M, 2 mL), the mixture was stirred at room temperature for 1 h. The reaction mixture was concentrated in vacuo to give 3-((4-(methylsulfonyl)phenoxy)methyl)azepane (HCl salt), which was used in the next step without further purification. MS = 284.2 [M+H]⁺.

### Step 3: 1-(3-chlorophenethyl)-3-((4-(methylsulfonyl)phenoxy)methyl)azepane (Compound 1)

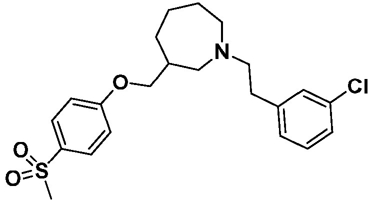

A mixture of 3-((4-(methylsulfonyl)phenoxy)methyl)azepane (200 mg, 0.625 mmol, HCl salt), 2-(3-chlorophenyl)acetaldehyde (142 mg, 0.918 mmol) in MeOH (4 mL) was stirred at room temperature for 1 h, then NaBH₃CN (66.5 mg, 1.06 mmol) was added. The resulting mixture was stirred at room temperature for 1 h. The reaction mixture was quenched by addition of water (1 mL) and concentrated in vacuo. The crude residue was purified by reverse phase preparative HPLC (Waters Xbridge BEH C18 column, 45-80% MeCN/10 mM NH₄HCO₃ in water) to give 1-(3-chlorophenethyl)-3-((4-(methylsulfonyl)phenoxy)methyl)azepane (Compound 1). ¹H NMR (400 MHz, DMSO-*d*₆): δ 7.82 (d, *J* = 8.8 Hz, 2H), 7.28 - 7.22 (m, 3H), 7.14 - 7.11 (m, 3H), 3.89 - 3.82 (m, 2H), 3.14 (s, 3H), 2.77 - 2.70 (m, 1H), 2.70 - 2.60 (m, 7H), 2.06 - 2.05 (m, 1H), 1.72 - 1.51 (m, 5H), 1.35 - 1.33 (m, 1H). MS = 422.3 [M+H]⁺.

### Step 4: (R) or (S)-1-(3-chlorophenethyl)-3-((4-(methylsulfonyl)phenoxy)methyl)azepane (Compound 2); (S) or (R)-1-(3-chlorophenethyl)-3-((4-(methylsulfonyl)phenoxy)methyl)azepane (Compound 3)

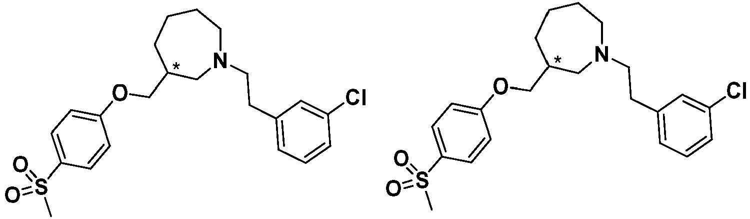

1-(3-chlorophenethyl)-3-((4-(methylsulfonyl)phenoxy)methyl)azepane **(Compound 1, 200** mg, 0.475 mmol) was separated by preparative chiral SFC (Phenomenex-Cellulose-2, 55% (1:1 MeOH:MeCN) with 0.1% NH₄OH in CO₂). The first eluting enantiomer of the title compound, **Compound 2:** ¹H NMR (400 MHz, DMSO-*d*₆): δ 7.82 (d, *J =* 8.8 Hz, 2H), 7.29 - 7.23 (m, 3H), 7.14 - 7.11 (m, 3H), 3.90 - 3.83 (m, 2H), 3.15 (s, 3H), 2.78 - 2.71 (m, 1H), 2.68 - 2.57 (m, 7H), 2.01 - 2.10 (m, 1H), 1.75 - 1.50 (m, 5H), 1.35 - 1.32 (m, 1H). MS = 422.3 [M+H]⁺. The second eluting enantiomer of the title compound, **Compound 3):** ¹H NMR (400 MHz, DMSO-*d*₆): δ 7.82 (d, *J=* 8.8 Hz, 2H), 7.28 - 7.23 (m, 3H), 7.15 - 7.11 (m, 3H), 3.90 - 3.83 (m, 2H), 3.15 (s, 3H), 2.79 - 2.75 (m, 1H), 2.69 - 2.53 (m, 7H), 2.01 - 2.10 (m, 1H), 1.75 - 1.50 (m, 5H), 1.35 - 1.32 (m, 1H). MS = 422.3 [M+H]⁺.

The following compounds in Table 9 were prepared according to procedures similar to those described for Example 1 using the appropriate starting materials.

**Table 9**

| No. | Structure | IUPAC Name | Exact Mass [M+H]⁺ | Chiral Column | Chiral Elution Order |
|---|---|---|---|---|---|
| **4** | 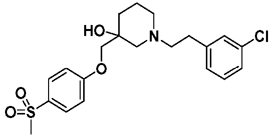 | 1-(3-chlorophenethyl)-3-((4-(methylsulfonyl)phenoxy)methyl)pi peridin-3-ol | Calc'd 424.1 | n/a | n/a |
| | | | Found 424.1 | | |
| **5** | 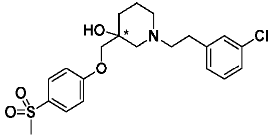 | *(R)* or (*S*)-1-(3-chlorophenethyl)-3-((4-(methylsulfonyl)p henoxy)methyl)pi peridin-3-ol | Calc'd 424.1 | Chiralpak AS-3 | 1^{st} |
| | | | Found 424.2 | | |
| **6** | 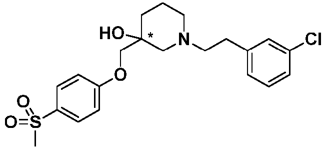 | (*S*) or *(R)* -1-(3-chlorophenethyl)- | Calc'd 424.1 | Chiralpak AS-3 | 2^{nd} |
| | | 3-((4-(methylsulfonyl)p henoxy)methyl)pi peridin-3-ol | Found 424.2 | | |
| **107** | 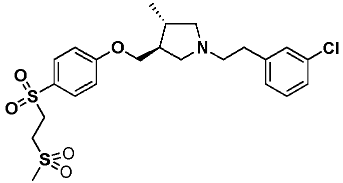 | (3*S*,4*S*)-1-[2-(3-chlorophenyl)ethy l]-3-{[4-(2-methanesulfonyle thanesulfonyl)phe noxy]methyl}-4-methylpyrrolidine | Calc'd 500.1 | n/a | n/a |
| | | | Found 500.1 | | |
| **108** | 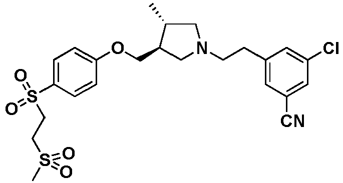 | 3-chloro-5-{2-[(3*S*,4*S*)-3-{[4-(2-methanesulfonyle thanesulfonyl)phe noxy]methyl}-4-methylpyrrolidin-1-yl]ethyl}benzonit rile | Calc'd 525.1 | n/a | n/a |
| | | | Found 525.2 | | |
| **109** | 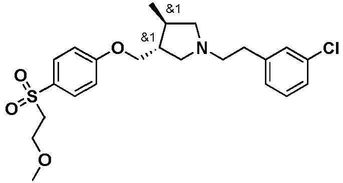 | *rac-trans-1-[2-(3-*chlorophenyl)ethy l]-3-{[4-(2-methoxyethanesul fonyl)phenoxy]m ethyl }-4-methylpyrrolidine | Calc'd 452.2 | n/a | n/a |
| | | | Found 452.1 | | |
| **110** | 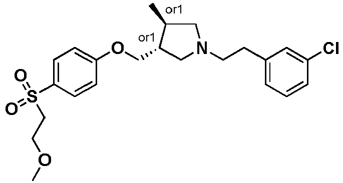 | *(3R,4R* or 3*S*,4*S*)-1-[2-(3-chlorophenyl)ethy l]-3-{[4-(2-methoxyethanesul fonyl)phenoxy]m ethyl }-4-methylpyrrolidine | Calc'd 452.2 | Chiralpak AD | 1st |
| | | | Found 452.1 | | |
| **111** | 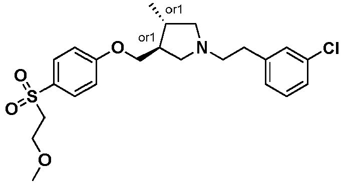 | *(3S,4S* or *3R,4R)-*1-[2-(3-chlorophenyl)ethy l]-3-{[4-(2-methoxyethanesul fonyl)phenoxy]m ethyl }-4-methylpyrrolidine | Calc'd 452.2 | Chiralpak AD | 2nd |
| | | | Found 452.1 | | |
| **112** | 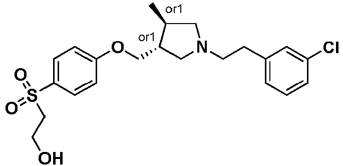 | 2-(4-{[(3*R*,4*R* or 3*S*,4*S*)-1-[2-(3-chlorophenyl)ethy l]-4-methylpyrrolidin-3-yl]methoxy}benz enesulfonyl)ethan -1-ol | Calc'd 438.2 | n/a | n/a |
| | | | Found 438.1 | | |
| | | | | | |
| **113** | 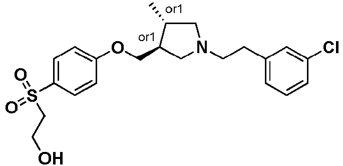 | *2-(4-{[(3S,4S* or 3*R*,4*R*)-1-[2-(3-chlorophenyl)ethy l]-4-methylpyrrolidin-3-yl]methoxy}benz enesulfonyl)ethan -1-ol | Calc'd 438.2 | n/a | n/a |
| | | | Found 438.1 | | |

### Example 2

### N-(4-((1-(3-chlorophenethyl)piperidin-3-yl)methoxy)phenyl)-N-methylmethanesulfonamide (Compound 7)

### (S) or (R) -N-(4-((1-(3-chlorophenethyl)piperidin-3-yl)methoxy)phenyl)-N-methylmethanesulfonamide (Compound 8)

### (R) or (S)-N-(4-((1-(3-chlorophenethyl)piperidin-3-yl)methoxy)phenyl)-N-methylmethanesulfonamide (Compound 9)

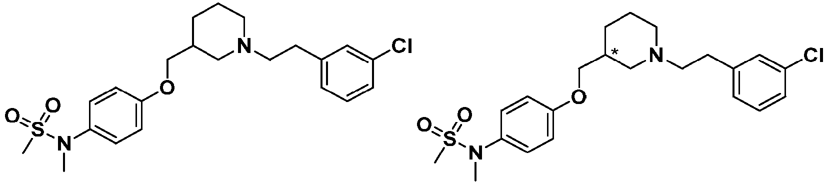

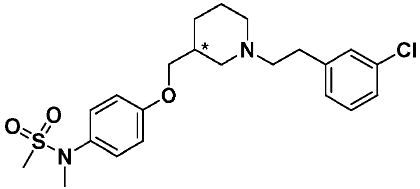

### Step 1: 3-((4-(N-methylmethylsulfonamido)phenoxy)methyl)piperidine-1-carboxylate

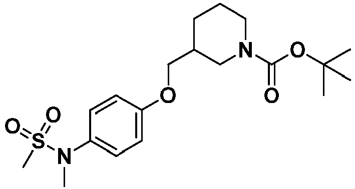

To a mixture of *tert-butyl* 3-(bromomethyl)piperidine-1-carboxylate (300 mg, 1.08 mmol) and *N*-(4-hydroxyphenyl)-*N*-methylmethanesulfonamide (260 mg, 1.29 mmol) in DMF (3 mL) was added K₂CO₃ (447 mg, 3.24 mmol). The mixture was stirred at 60 °C for 12 h. The reaction mixture was cooled to 0 °C and quenched by the addition of water (15 mL), then extracted with EtOAc (25 mL x 2). The combined organic layers were washed with brine (10 mL x 2), dried over Na₂SO₄, filtered and concentrated in vacuo. The residue was purified by normal phase silica gel chromatography (Biotage 10 g cartridge, 0-25% EtOAc in petroleum ether) to give tert-butyl 3-((4-(N-methylmethylsulfonamido)phenoxy)methyl)piperidine-1-carboxylate. MS = 343.2 [M-C₄H₇+H]⁺.

### Step 2: N-methyl-N-(4-(piperidin-3-ylmethoxy)phenyl)methanesulfonamide

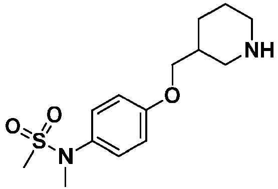

To a solution of *tert-*butyl *3-((4-(N-*methylmethylsulfonamido)phenoxy)methyl) piperidine-1-carboxylate (336 mg, 843.1 mmol) in EtOAc (3 mL) was added HCl/EtOAc (4 M, 3 mL). The mixture was stirred at room temperature for 3 h. The mixture was concentrated in vacuo to give N-methyl-N-(4-(piperidin-3-ylmethoxy)phenyl)methanesulfonamide (HCl salt), which was used in the next step without further purification. MS = 299.3 [M+H]⁺.

### Step 3: N-(4-((1-(3-chlorophenethyl)piperidin-3-yl)methoxy)phenyl)-N-methylmethanesulfonamide

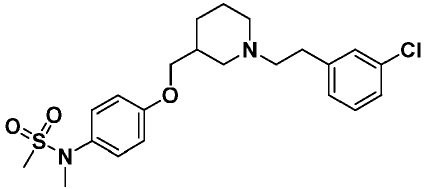

A mixture of *N*-methyl-*N*-(4-(piperidin-3-ylmethoxy)phenyl)methanesulfonamide (181 mg, 0.543 mmol, HCl salt) and 2-(3-chlorophenyl)acetaldehyde (109 mg, 0.706 mmol) in MeOH (2 mL) was stirred at room temperature for 30 min, and then NaBH₃CN (51.2 mg, 0.814 mmol) was added. The resulting mixture was stirred at room temperature for 3 h. The reaction mixture was quenched by the addition of water (0.5 mL) and purified by reverse phase preparative HPLC (Waters Xbridge BEH C18 column, 40-70% MeCN/10 mM NH₄HCO₃ in water) to give N-(4-((1-(3-chlorophenethyl)piperidin-3-yl)methoxy)phenyl)-N-methylmethanesulfonamide **(Compound 7).** ¹H NMR (400 MHz, DMSO-*d*₆): δ 7.32 - 7.19 (m, 6H), 6.94 (d, *J* = 8.8 Hz, 2H), 4.12 - 4.09 (m, 1H), 3.86 - 3.81 (m, 2H), 3.18 (m, 3H), 3.17 - 3.16 (m, 1H), 2.89 (s, 3H), 2.75 - 2.72 (m, 4H), 2.06 - 1.95 (m, 3H), 1.76 - 1.62 (m, 2H), 1.48 - 1.47 (m, 1H), 1.23 - 1.10 (m, 1H). MS = 437.2 [M+H]⁺.

### Step 4: (S) or (R)-N-(4-((1-(3-chlorophenethyl)piperidin-3-yl)methoxy)phenyl)-N-methylmethanesulfonamide (Compound 8); (R) or (S)-N-(4-((1-(3-chlorophenethyl)piperidin-3-yl)methoxy)phenyl)-N-methylmethanesulfonamide (Compound 9)

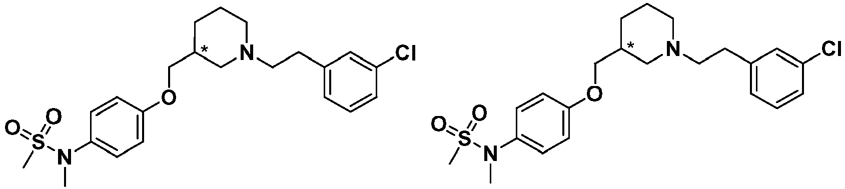

*N*-(4-((1-(3-chlorophenethyl)piperidin-3-yl)methoxy)phenyl)*-N-*methylmethanesulfonamide **(Compound 7,** 96 mg, 0.220 mmol) was separated by preparative chiral SFC (Chiralcel OD-3 column, 35% ethanol with 0.1% NH₄OH in CO₂). The first eluting enantiomer of the title compound, **Compound 8:** ¹H NMR (400 MHz, DMSO-*d*₆): δ 7.33 - 7.19 (m, 6H), 6.94 (d, *J* = 9.2 Hz, 2H), 3.86 - 3.81 (m, 2H), 3.18 (s, 3H), 2.90 (s, 4H), 2.77 - 2.67 (m, 4H), 2.09 - 1.92 (m, 4H), 1.75 - 1.61 (m, 2H), 1.52 - 1.42 (m, 1H), 1.12 -1.09 (m, 1H). MS = 437.2 [M+H]⁺. The second eluting enantiomer of the title compound, **Compound 9:** ¹H NMR (400 MHz, DMSO-*d*₆): δ 7.33 - 7.17 (m, 6H), 6.95 (d, *J=* 9.2 Hz, 2H), 3.89 - 3.84 (m, 2H), 3.18 (s, 3H), 2.90 (s, 4H), 2.78 - 2.72 (m, 4H), 2.08 - 1.96 (m, 4H), 1.74 - 1.65 (m, 2H), 1.50 - 1.49 (m, 1H), 1.14 - 1.04 (m, 1H). MS = 437.2 [M+H]⁺.

The following compounds in Table 10 were prepared according to procedures similar to those described for **Compounds 7-9** using the appropriate starting materials.

**Table 10**

| No. | Structure | IUPAC Name | Exact Mass [M+H]⁺ | Chiral Column | Chiral Elution Order |
|---|---|---|---|---|---|
| **10** | 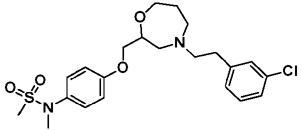 | *N*-(4-((4-(3-chlorophenethyl)-1,4-oxazepan-2-yl)methoxy)phenyl *)-N-*methylmethanesulf onamide | Calc'd 453.2 | n/a | n/a |
| | | | Found 453.2 | | |
| **11** | 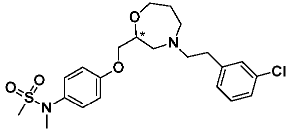 | *(R)* or *(S)-N-*(4-((4-(3-chlorophenethyl)-1,4-oxazepan-2-yl)methoxy)phenyl *)-N-*methylmethanesulf onamide | Calc'd 453.2 | Chiralp ak AD-3 | 1st |
| | | | Found 453.2 | | |
| **12** | 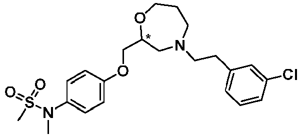 | (*S*) or *(R)- N-(4-*((4-(3-chlorophenethyl)-1,4-oxazepan-2-yl)methoxy)phenyl *)-N-*methylmethanesulf onamide | Calc'd 453.2 | Chiralp ak AD-3 | 2nd |
| | | | Found 453.2 | | |

### Example 3

### (S)-1-(3-chlorophenethyl)-3-((4-(methylsulfonyl)phenoxy)methyl)piperazine (Compound 13)

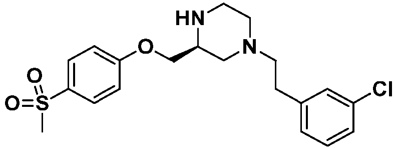

### Step 1: (S)-1-benzyl 4-tert-butyl 2-(hydroxymethyl)piperazine-1,4-dicarboxylate

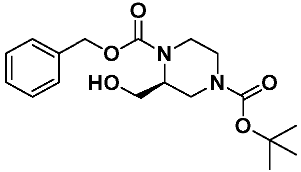

To a mixture of *(S)-tert-*butyl 3-(hydroxymethyl)piperazine-1-carboxylate (1.00 g, 4.62 mmol) and NaHCO₃ (1.17 g, 13.9 mmol) in THF (5 mL) and water (5 mL) at 0 °C was added benzyl chloroformate (986 µL, 6.94 mmol). The mixture was stirred at room temperature for 4 h, then quenched by the addition of water (20 mL) and extracted with EtOAc (20 mL x 3). The combined organic layers were washed with brine (60 mL), dried over Na₂SO₄, filtered and concentrated in vacuo. The crude residue was purified by normal phase silica gel chromatography (Biotage 20 g cartridge, 7-50% EtOAc in petroleum ether) to give (*S*)-1-benzyl 4-*tert*-butyl 2-(hydroxymethyl)piperazine-1,4-dicarboxylate. MS = 373.2 [M+Na]⁺.

### Step 2: (S)-1-benzyl 4-tert-butyl 2-((4-(methylsulfonyl)phenoxy)methyl)piperazine -1,4-dicarboxylate

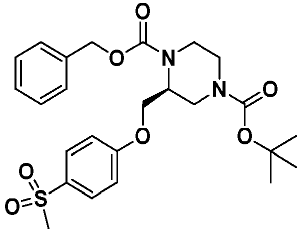

To a mixture of (S)-1-benzyl 4-tert-butyl 2-(hydroxymethyl)piperazine-1,4-dicarboxylate (200 mg, 0.571 mmol), 4-methylsulfonylphenol (98.0 mg, 0.571 mmol) and PPh₃ (225 mg, 0.856 mmol) in THF (4 mL) at 0 °C was added DIAD (222 µL, 1.14 mmol) dropwise. The mixture was stirred at 0 °C for 2 h, and then quenched by the addition of water (20 mL). The mixture was extracted with EtOAc (20 mL x 3). The combined organic layers were washed with brine (60 mL), dried over Na₂SO₄, filtered and concentrated in vacuo. The crude residue was purified by normal phase silica gel chromatography (Biotage 4 g cartridge, 0-40% EtOAc in petroleum ether) to give (S)-1-benzyl 4-tert-butyl 2-((4-(methylsulfonyl)phenoxy)methyl)piperazine-1,4-dicarboxylate. MS = 527.3 [M+Na]⁺.

### Step 3: (S)-benzyl 2-((4-(methylsulfonyl)phenoxy)methyl)piperazine-1-carboxylate

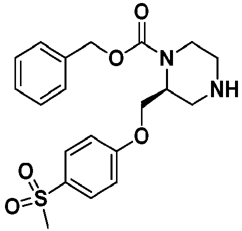

To a solution of (*S*)-1-benzyl *4-tert-*butyl 2-((4-(methylsulfonyl)phenoxy)methyl)piperazine-1,4-dicarboxylate (600 mg, 1.12 mmol) in EtOAc (5 mL) was added HCl/EtOAc (4 M, 12 mL). The mixture was stirred at room temperature for 2 h, then concentrated in vacuo. The residue was diluted with water (20 mL) and washed with EtOAc (20 mL). The aqueous layer pH was adjusted to pH = 8-9 by addition of saturated aqueous NaHCO₃ and extracted with EtOAc (20 mL x 3). The combined organic layers were washed with brine (60 mL), dried over Na₂SO₄, filtered and concentrated in vacuo to give (S)-benzyl 2-((4-(methylsulfonyl)phenoxy)methyl)piperazine-1-carboxylate, which was used in the next step without further purification. MS = 405.2 [M+H]⁺.

### Step 4: (S)-benzyl 4-(3-chlorophenethyl)-2-((4-(methylsulfonyl)phenoxy)methyl) piperazine-1-carboxylate

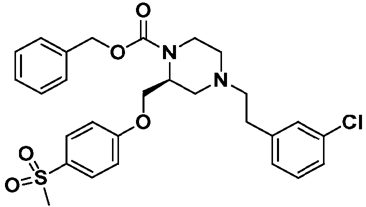

To a solution of (S)-benzyl 2-((4-(methylsulfonyl)phenoxy)methyl)piperazine-1-carboxylate (200 mg, 0.494 mmol) and 2-(3-chlorophenyl)acetaldehyde (76.0 mg, 0.494 mmol) in MeOH (2 mL) at 0 °C was added NaBH₃CN (47.0 mg, 0.742 mmol) slowly. The mixture was stirred at room temperature for 1 h, then was quenched with water (0.05 mL) and concentrated in vacuo. The crude residue was purified by normal phase silica gel chromatography (Biotage 12 g cartridge, 10-50% EtOAc in petroleum ether) to give (S)-benzyl 4-(3-chlorophenethyl)-2-((4-(methylsulfonyl)phenoxy)methyl)piperazine-1-carboxylate. MS = 543.2 [M+H]⁺.

### Step 5: (S)-1-(3-chlorophenethyl)-3-((4-(methylsulfonyl)phenoxy)methyl)piperazine

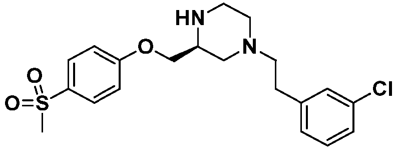

To a solution of (*S*)-benzyl 4-(3-chlorophenethyl)-2-((4-(methylsulfonyl)phenoxy)methyl)piperazine-1-carboxylate (120 mg, 0.221 mmol) and NaI (331 mg, 2.21 mmol) in MeCN (3 mL) at 0 °C was added TMSCl (280 µL, 2.21 mmol) dropwise. The mixture was stirred at room temperature for 16 h, then was quenched with water (0.2 mL) and purified by preparative HPLC (Waters Xbridge BEH C18 column, 30-55% MeCN/10 mM NH₄HCO₃ in water) to give (*S*)-1-(3-chlorophenethyl)-3-((4-(methylsulfonyl)phenoxy)methyl)piperazine **(Compound 13).** ¹H NMR (400 MHz, DMSO-*d*₆, 24/25 H): δ 7.83 (d, *J* = 8.8 Hz, 2H), 7.32 - 7.15 (m, 6H), 4.01 - 3.94 (m, 2H), 3.32 (s, 3H), 3.15 - 3.14 (s, 1H), 2.88 - 2.85 (m, 2H), 2.76 - 2.66 (m, 4H), 2.47 - 2.44 (m, 2H), 2.07 - 2.00 (m, 1H), 1.89 (app t, *J* = 10.0 Hz, 1H). MS = 409.3 [M+H]⁺.

The following compound in Table 11 was prepared according to procedures similar to those described for **Compound 13** using the appropriate starting materials.

**Table 11**

| No. | Structure | IUPAC Name | Exact Mass [M+H]⁺ |
|---|---|---|---|
| **14** | 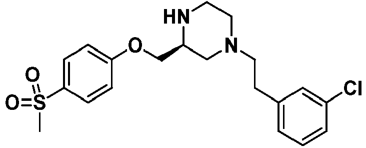 | (R)-1-(3-chlorophenethyl)-3-((4-(methylsulfonyl)phenoxy)met hyl)piperazine | Calc'd 409.1 |
| | | | Found 409.1 |

### Example 4

### (R)-1-(3-chlorophenethyl)-3-((4-(methylsulfonyl)phenoxy)methyl)piperidine (Compound 15)

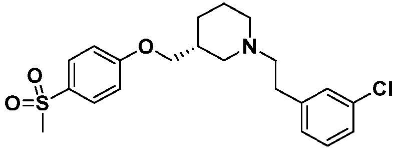

To a solution (R)-3-((4-(methylsulfonyl)phenoxy)methyl)piperidine **(Intermediate G,** 56.9. mg, 0.186 mmol, HCl salt) in DCM (5 mL) was added TEA (155 µL, 1.11 mmol) and 2-(3-chlorophenyl)acetaldehyde (28.70 mg, 0.186 mmol). The mixture was stirred at room temperature for 30 min, then NaBH(OAc)₃ (157 mg, 0.743 mmol) was added. After stirring for 12 h, the reaction mixture was quenched with water (5 mL), and then extracted with EtOAc (5 mL x 3). The combined organic layers were washed with brine (5 mL x 3), dried over Na₂SO₄, filtered and concentrated in vacuo. The crude residue was purified by reverse phase preparative HPLC (Waters Xbridge BEH C18, 50-80% MeCN/10 mM NH₄HCO₃ in water) to give (*R*)-1-(3-chlorophenethyl)-3-((4-(methylsulfonyl)phenoxy)methyl)piperidine **(Compound 15).** ¹H NMR (400 MHz, DMSO-*d₆*): δ 7.82 (d, *J* = 6.8 Hz, 2H), 7.30 - 7.13 (m, 6H), 4.00 - 3.93 (m, 2H), 3.15 (s, 3H), 2.90 (d, *J* = 7.6 Hz, 1H), 2.77 - 2.71 (m, 3H), 2.53 - 2.52 (m, 2H), 2.05 - 1.97 (m, 3H), 1.66 - 1.64 (m, 1H), 1.64 - 1.63 (m, 1H), 1.49 - 1.41 (m, 1H), 1.17 - 1.12 (m, 1H). MS = 408.2 [M+H]⁺.

The following compound in Table 12 was prepared according to procedures similar to those described for **Compound 15** using the appropriate starting materials.

**Table 12**

| No. | Interm ediate | Structure | IUPAC Name | Exact Mass [M+H]⁺ |
|---|---|---|---|---|
| **16** | **H** | 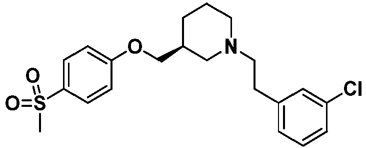 | (S)-1-(3-chlorophenethyl)-3-((4-(methylsulfonyl)phe noxy)methyl)piperid ine | Calc'd 408.1 |
| | | | | Found 408.1 |

### Example 5

### 1-(3-chlorophenyl)-2-((S)-3-((4-(methylsulfonyl)phenoxy)methyl)piperidin-1-yl)ethan-1-ol (Compound 17)

### (R) or (S)-1-(3-chlorophenyl)-2-((S)-3-((4-(methylsulfonyl)phenoxy)methyl)piperidin-1-yl)ethan-1-ol (Compound 18)

### (S) or (R)-1-(3-chlorophenyl)-2-((S)-3-((4-(methylsulfonyl)phenoxy)methyl)piperidin-1-yl)ethan-1-ol (Compound 19)

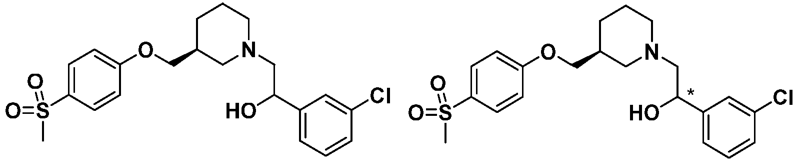

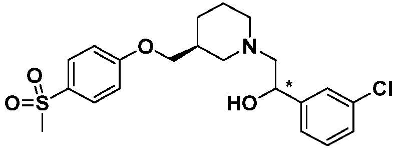

### Step 1: 1-(3-chlorophenyl)-2-((S)-3-((4-(methylsulfonyl)phenoxy)methyl)piperidin-1-yl)ethanol

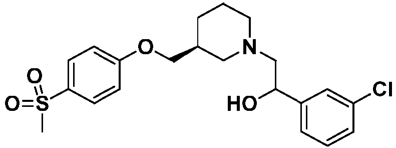

To a solution of (*S*)-3-((4-(methylsulfonyl)phenoxy)methyl)piperidine **(Intermediate H,** 500 mg, 1.63 mmol, HCl salt) and 2-(3-chlorophenyl)oxirane (379 mg, 2.45 mmol) in EtOH (10 mL) was added TEA (455 µL, 3.27 mmol). The mixture was stirred at 80 °C for 8 h, then cooled to room temperature and quenched by the addition of water (1 mL), filtered, and concentrated in vacuo. The crude residue was purified by reverse phase preparative HPLC (Phenomenex Gemini-NX, 25-55% MeCN/10 mM NH₄HCO₃ in water) to give 1-(3-chlorophenyl)-2-((*S*)-3-((4-(methylsulfonyl)phenoxy)methyl)piperidin-1-yl)ethanol **(Compound 17).** ¹H NMR (400 MHz, DMSO-*d*₆): δ 7.82 (d, *J=* 8.8 Hz, 2H), 7.38 (s, 1H), 7.30 - 7.27 (m, 3H), 7.16 - 7.13 (m, 2H), 5.14 (s, 1H), 4.70 - 4.69 (m, 1H), 3.99 - 3.92 (m, 2H), 3.15 (s, 3H), 2.95 - 2.85 (m, 1H), 2.75 - 2.74 (m, 1H), 2.46 - 2.45 (m, 1H), 2.41 - 2.35 (m, 1H), 2.17 - 1.98 (m, 3H), 1.72 - 1.60 (m, 2H), 1.50 - 1.45 (m, 1H), 1.15 - 1.12 (m, 1H). MS = 424.1 [M+H]⁺.

### Step 2: (R) or (S)-1-(3-chlorophenyl)-2-((S)-3-((4-(methylsulfonyl)phenoxy)methyl)piperidin-1-yl)ethan-1-ol (Compound 18); (S) or (R)-1-(3-chlorophenyl)-2-((S)-3-((4-(methylsulfonyl)phenoxy)methyl)piperidin-1-yl)ethan-1-ol (Compound 19)

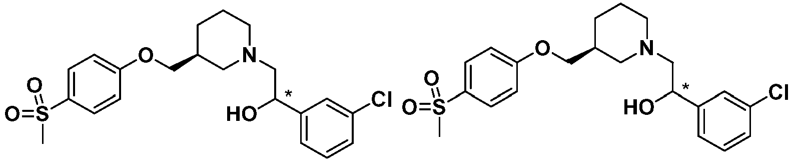

1-(3-chlorophenyl)-2-[(3*S*)-3-[(4-methylsulfonylphenoxy)methyl]-1-piperidyl]ethanol **(Compound 17, 400** mg, 0.944 mmol) was separated by preparative chiral SFC (DAICEL CHIRALPAK AD-3, 55% ethanol with 0.1% NH₄OH in CO₂). The first eluting enantiomer of the title compound, **Compound 18:** ¹H NMR (400 MHz, DMSO-*d*₆): δ 7.83 (d, *J* = 8.8 Hz, 2H), 7.38 (s, 1H), 7.30 - 7.25 (m, 3H), 7.16 - 7.13 (m, 2H), 5.14 (d, *J* = 3.6 Hz, 1H), 4.71 - 4.70 (m, 1H), 3.95 - 3.94 (m, 2H), 3.15 (s, 3H), 2.87 - 2.74 (m, 2H), 2.46 - 2.41 (m, 1H), 2.40 - 2.38 (m, 1H), 2.15 - 1.98 (m, 3H), 1.69 - 1.60 (m, 2H), 1.50 - 1.49 (m, 1H), 1.17 - 1.12 (m, 1H). MS = 424.1 [M+H]⁺. The second eluting enantiomer of the title compound, **Compound 19:** ¹H NMR (400 MHz, DMSO-*d*₆): δ 7.83 (d, *J* = 8.8 Hz, 2H), 7.39 - 7.13 (m, 6H), 5.15 (s, 1H), 4.71 (s, 1H), 3.99 - 3.90 (m, 2H), 3.15 (s, 3H), 2.96 - 2.94 (m, 1H), 2.77 - 2.75 (m, 1H), 2.46 - 2.41 (m, 1H), 2.40 - 2.38 (m, 1H), 2.14 - 2.04 (m, 3H), 1.73 - 1.61 (m, 2H), 1.49 - 1.46 (m, 1H), 1.14 - 1.12 (m, 1H). MS = 424.1 [M+H]⁺.

The following compounds in Table 13 were prepared according to procedures similar to those described for **Compound 17** using the appropriate starting materials.

**Table 13**

| No. | Structure | IUPAC Name | Exact Mass [M+H]⁺ | Chiral Column | Chiral Elutio n Order |
|---|---|---|---|---|---|
| **20** | 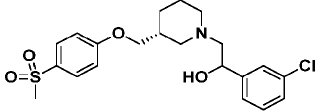 | 1-(3-chlorophenyl)-2-*((R)-3-((4-*(methylsulfonyl) phenoxy)methyl) piperidin-1-yl)ethanol | Calc'd 424.1 | n/a | n/a |
| | | | Found 424.3 | | |
| **21** | 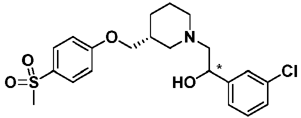 | *(R)* or (S)-1-(3-chlorophenyl)-2-*((R)-3-((4-*(methylsulfonyl) phenoxy)methyl) piperidin-1-yl)ethanol | Calc'd 424.1 | Chiralpak AD-3 | 1st |
| | | | Found 424.2 | | |
| **22** | 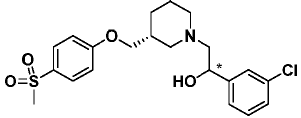 | (*S*) or (*R*)-1-(3-chlorophenyl)-2-*((R)-3-((4-*(methylsulfonyl) phenoxy)methyl) piperidin-1-yl)ethanol | Calc'd 424.1 | Chiralpak AD-3 | 2nd |
| | | | Found 424.2 | | |
| **114** | 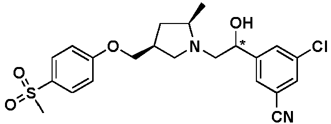 | 3-chloro-5-[(1*S* or 1*R)-1-*hydroxy-2-[(2*R*,4*S*)-4-[(4-methanesulfonyl phenoxy)methyl] -2-methylpyrrolidin -1-yl]ethyl]benzonit rile | Calc'd 449.1 | prep-HPLC Phenome nex Gemini-NX 80 | 1st |
| | | | Found 449.1 | | |
| **115** | 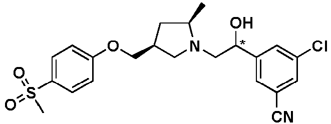 | 3-chloro-5-[(1*R* or 1*S*)-1-hydroxy-2-[(2*R*,4*S*)-4-[(4-methanesulfonyl phenoxy)methyl] -2-methylpyrrolidin -1-yl]ethyl]benzonit rile | Calc'd 449.1 | prep-HPLC Phenome nex Gemini-NX 80 | 2nd |
| | | | Found 449.1 | | |
| **116** | 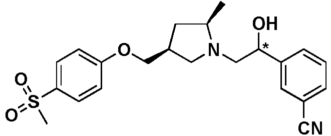 | 3-[(1*R* or 1*S*)-1-hydroxy-2-[(2*R*,4*S*)-4-[(4-methanesulfonyl phenoxy)methyl] -2-methylpyrrolidin -1-yl]ethyl]benzonit rile | Calc'd 415.2 | prep-HPLC Phenome nex Gemini-NX | 2nd |
| | | | Found 415.2 | | |
| **117** | 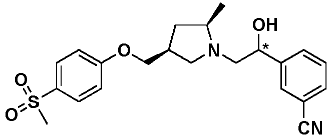 | 3-[(1*S* or *1R)-1-*hydroxy-2-[(2*R*,4*S*)-4-[(4-methanesulfonyl phenoxy)methyl] -2-methylpyrrolidin -1-yl]ethyl]benzonit rile | Calc'd 415.2 | prep-HPLC Phenome nex Gemini-NX | 1st |
| | | | Found 415.2 | | |
| **214** | 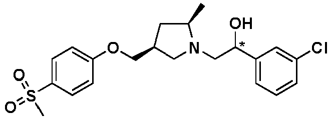 | *(*1*R)* or (1*S*)-1-(3-chlorophenyl)-2-[(2R,4S)-4-[(4-methanesulfonyl phenoxy)methyl] -2-methylpyrrolidin -1-yl]ethan-1-ol | Calc'd 424.1 | prep-HPLC Phenome nex Gemini-NX | 1st |
| | | | Found 424.2 | | |
| **215** | 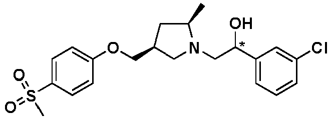 | (1*S*) or (1*R*)-1-(3-chlorophenyl)-2-[(2R,4S)-4-[(4-methanesulfonyl phenoxy)methyl] -2-methylpyrrolidin -1-yl]ethan-1-ol | Calc'd 424.1 | prep-HPLC Phenome nex Gemini-NX | 2nd |
| | | | Found 424.2 | | |
| **118** | 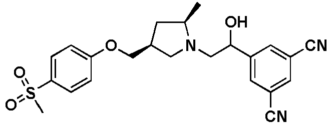 | 5-[(1*R* or 1*S*)-1-hydroxy-2-[(2*R*,4*S*)-4-[(4-methanesulfonyl phenoxy)methyl] -2-methylpyrrolidin -1-yl]ethyl]benzene -1,3-dicarbonitrile | Calc'd 440.2 | prep-HPLC Phenome nex C18 | 1st |
| | | | Found 440.2 | | |
| **119** | 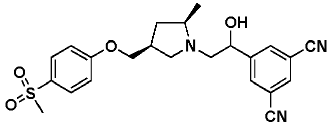 | 5-[(1*S* or *1R)-1-*hydroxy-2-[(2*R*,4*S*)-4-[(4-methanesulfonyl phenoxy)methyl] -2-methylpyrrolidin -1-yl]ethyl]benzene -1,3-dicarbonitrile | Calc'd 440.2 | prep-HPLC Phenome nex C18 | 2nd |
| | | | Found 440.0 | | |
| **120** | 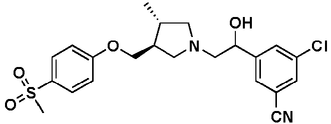 | 3-chloro-5-{1-hydroxy-2-[(3*S*,4*S*)-3-[(4-methanesulfonylphenoxy)methyl] -4-methylpyrrolidin -1-yl]ethyl}benzoni trile | Calc'd 449.1 | n/a | n/a |
| | | | Found 449.0 | | |
| **121** | 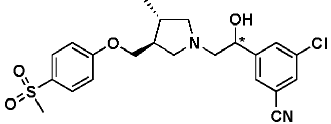 | 3-chloro-5-[(1*S* or *1R)-1-*hydroxy-2-[(3*S*,4*S*)-3-[(4-methanesulfonyl phenoxy)methyl] -4-methylpyrrolidin -1-yl]ethyl]benzonit rile | Calc'd 449.1 | Phenome nex-Cellulose-2 | 1st |
| | | | Found 449.0 | | |
| **122** | 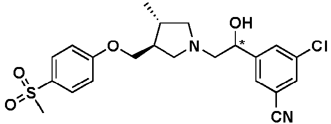 | 3-chloro-5-[(1*R* or 1*S*)-1-hydroxy-2-[(3*S*,4*S*)-3-[(4-methanesulfonyl phenoxy)methyl] -4-methylpyrrolidin -1-yl]ethyl]benzonit rile | Calc'd 449.1 | Phenome nex-Cellulose-2 | 2nd |
| | | | Found 449.0 | | |
| **123** | 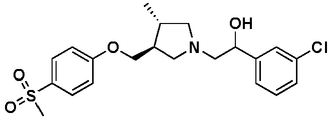 | 1-(3-chlorophenyl)-2-((3*S*,4*S*)-3-methyl-4-((4-(methylsulfonyl) phenoxy)methyl) pyrrolidin-1-yl)ethan-1-ol | Calc'd 424.1 | n/a | n/a |
| | | | Found 424.1 | | |
| **124** | 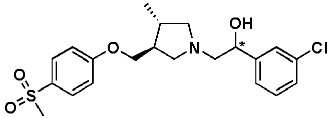 | *(1R* or 1*S*)-1-(3-chlorophenyl)-2-[(3*S*,4*S*)-3-[(4-methanesulfonyl phenoxy)methyl] -4-methylpyrrolidin -1-yl]ethan-1-ol | Calc'd 424.1 | Phenome nex-Cellulose-2 | 1st |
| | | | Found 424.0 | | |
| **125** | 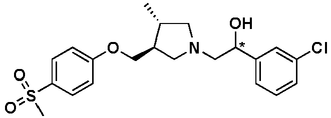 | *(1S* or 1*R*)-1-(3-chlorophenyl)-2-[(3*S*,4*S*)-3-[(4-methanesulfonyl phenoxy)methyl] -4-methylpyrrolidin -1-yl]ethan-1-ol | Calc'd 424.1 | Phenome nex-Cellulose-2 | 2nd |
| | | | Found 424.0 | | |
| **126** | 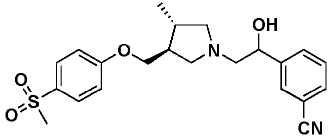 | 3-[1-hydroxy-2-[(3*S*,4*S*)-3-[(4-methanesulfonyl phenoxy)methyl] -4-methylpyrrolidin -1-yl]ethyl]benzonit rile | Calc'd 415.2 | n/a | n/a |
| | | | Found 415.0 | | |
| **127** | 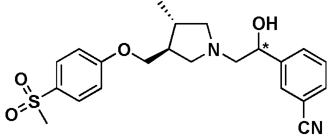 | 3-[(1*S* or 1*R*)-1-hydroxy-2-[(3S,4S)-3-[(4-methanesulfonyl phenoxy)methyl] -4-methylpyrrolidin -1-yl]ethyl]benzonit rile | Calc'd 415.2 | Chiralpk AD | 1st |
| | | | Found 415.0 | | |
| **128** | 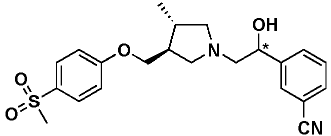 | 3-[(1*R* or 1*S*)-1-hydroxy-2-[(3*S*,4*S*)-3-[(4-methanesulfonyl phenoxy)methyl] -4-methylpyrrolidin -1-yl]ethyl]benzonit rile | Calc'd 415.2 | Chiralpk AD | 2nd |
| | | | Found 415.0 | | |
| **129** | 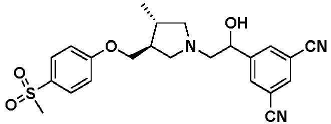 | 5-{ 1-hydroxy-2-[(3 S,4S)-3-[(4-methanesulfonyl phenoxy)methyl] -4-methylpyrrolidin -1-yl]ethyl }benzene -1,3-dicarbonitrile | Calc'd 440.2 | n/a | n/a |
| | | | Found 440.2 | | |
| **130** | 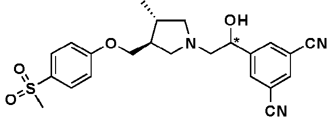 | 5-[(1*R* or 1*S*)-1-hydroxy-2-[(3*S*,4*S*)-3-[(4-methanesulfonyl phenoxy)methyl] -4-methylpyrrolidin -1-yl]ethyl]benzene -1,3-dicarbonitrile | Calc'd 440.2 | Chiralpk AD | 1st |
| | | | Found 440.2 | | |
| **131** | 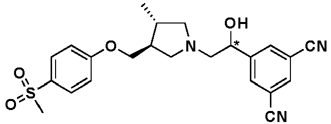 | 5-[(1*S* or *1R)-1-*hydroxy-2-[(3*S*,4*S*)-3-[(4-methanesulfonyl phenoxy)methyl] -4-methylpyrrolidin -1-yl]ethyl]benzene -1,3-dicarbonitrile | Calc'd 440.2 | Chiralpk AD | 2nd |
| | | | Found 440.2 | | |

### Example 6

### (S)-1-((R)-2-(3-chlorophenyl)-2-methoxyethyl)-3-((4-(methylsulfonyl)phenoxy)methyl)piperidine or (S)-1-((S)-2-(3-chlorophenyl)-2-methoxyethyl)-3-((4-(methylsulfonyl)phenoxy)methyl)piperidine (Compound 23)

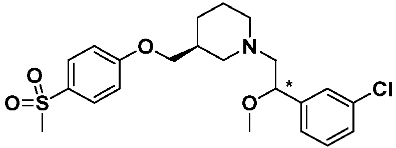

A solution of *(R)* or (*S*)-1-(3-chlorophenyl)-2-((*S*)-3-((4-(methylsulfonyl)phenoxy)methyl)piperidin-1-yl)ethan-1-ol **(Compound 18,** 50.0 mg, 0.118 mmol) in THF (2 mL) at 0° C was added NaH (5.66 mg, 0.142 mmol, 60% dispersion in mineral oil). After stirring at 0 °C for 1 h, MeI (8.08 µL, 0.130 mmol) was added and the mixture was allowed to warm to room temperature. After stirring for 1 h, the reaction mixture was cooled to 0 °C and quenched by the addition of water (5 mL), and extracted with EtOAc (5 mL x 3). The combined organic layers were washed with brine (5 mL), dried over Na₂SO₄, filtered and concentrated in vacuo. The crude residue was purified by reverse phase preparative HPLC (Waters Xbridge BEH C18 column, 35-70% MeCN/10 mM NH₄HCO₃ in water) to give (*S*)-1-*((R)* -2-(3-chlorophenyl)-2-methoxyethyl)-3-((4-(methylsulfonyl)phenoxy)methyl)piperidine or (*S*)-1-((*S*)-2-(3-chlorophenyl)-2-methoxyethyl)-3-((4-(methylsulfonyl)phenoxy)methyl)piperidine **(Compound 23).** ¹H NMR (400 MHz, DMSO-*d*₆): δ 7.83 (d, *J=* 8.8 Hz, 2H), 7.37 - 7.34 (m, 3H), 7.27 - 7.25 (m, 1H), 7.15 - 7.13 (m, 2H), 4.38 (t, *J* = 6.0 Hz, 1H), 3.93 - 3.89 (m, 2H), 3.15 (s, 3H), 3.13 (s, 3H), 2.80 - 2.77 (m, 2H), 2.43 - 2.40 (m, 2H), 2.15 - 2.08 (m, 2H), 1.96 - 1.95 (m, 1H), 1.70 - 1.59 (m, 2H), 1.45 - 1.43 (m, 1H), 1.14 - 1.11 (m, 1H). MS = 438.1 [M+H]⁺.

The following compounds in Table 14 were prepared according to procedures similar to those described for **Compound 23** using the appropriate starting materials.

**Table 14**

| **No.** | **Starting Material** | Structure | IUPAC Name | Exact Mass [M+H]⁺ |
|---|---|---|---|---|
| **24** | **Compound 19** | 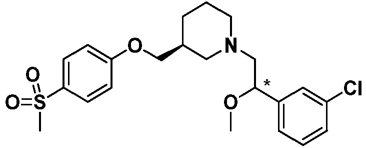 | (*S*)-1-((*S*)-2-(3-chlorophenyl)-2-methoxyethyl)-3-((4-(methylsulfonyl)pheno xy)methyl)piperidine or *(S)-1-((R)-2-(3-*chlorophenyl)-2-methoxyethyl)-3-((4-(methylsulfonyl)pheno xy)methyl)piperidine | Calc'd 438.1 |
| | | | | Found 438.1 |
| **25** | **Compound 21** | 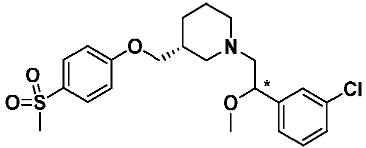 | *(R)-1-((R)-2-(3-*chlorophenyl)-2-methoxyethyl)-3-((4-(methylsulfonyl)pheno xy)methyl)piperidine or *(R)-1-((5)-2-(3-*chlorophenyl)-2-methoxyethyl)-3-((4-(methylsulfonyl)pheno xy)methyl)piperidine | Calc'd 438.1 |
| | | | | Found 438.3 |
| **26** | **Compound 22** | 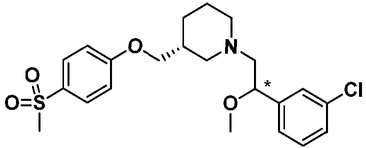 | *(R)-1-((5)-2-(3-*chlorophenyl)-2-methoxyethyl)-3-((4-(methylsulfonyl)pheno xy)methyl)piperidine or (*R*)-1-((*R*)-2-(3-chlorophenyl)-2-methoxyethyl)-3-((4-(methylsulfonyl)pheno xy)methyl)piperidine | Calc'd 438.1 |
| | | | | Found 438.3 |

### Example 7

### rac-trans or cis-1-(3-chlorophenethyl)-3-((4-(methylsulfonyl)phenoxy)methyl)piperidin-4-ol (Compound 27)

### rac-cis or trans-1-(3-chlorophenethyl)-3-((4-(methylsulfonyl)phenoxy)methyl)piperidin-4-ol (Compound 28)

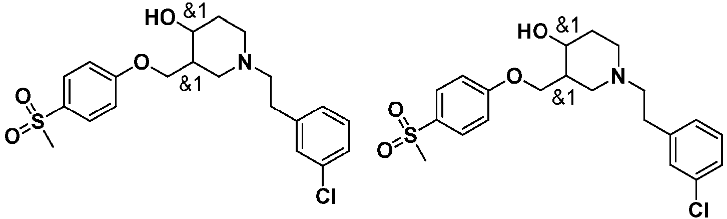

### Step 1: tert-butyl 4-hydroxy-3-(hydroxymethyl)piperidine-1-carboxylate

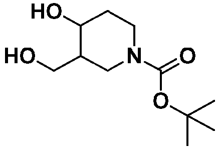

To a 0 °C solution of 1-tert-butyl 3-ethyl 4-oxopiperidine-1,3-dicarboxylate (5.00 g, 18.4 mmol) in EtOH (55 mL) was added NaBH₄ (6.97 g, 184 mmol) slowly. The mixture was stirred at 0 °C for 5 h, and was then quenched with ice water (40 mL). After stirring for 30 min, the solution was extracted with EtOAc (40 mL x 3). The combined organic layers were washed with brine (10 mL x 2), dried over Na₂SO₄, filtered and concentrated in vacuo to give tert-butyl 4-hydroxy-3-(hydroxymethyl)piperidine-1-carboxylate, which was used in the next step without further purification. MS = 176.0 [M-C₄H₇+H]⁺.

### Step 2: tert-butyl 4-hydroxy-3-((4-(methylsulfonyl)phenoxy)methyl)piperidine-1-carboxylate

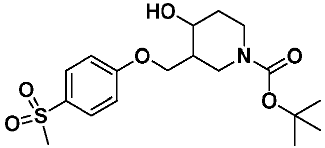

To a solution of tert-butyl 4-hydroxy-3-(hydroxymethyl)piperidine-1-carboxylate (2.80 g, 12.1 mmol) and 1-fluoro-4-methylsulfonyl-benzene (2.11 g, 12.1 mmol) in DMF (28 mL) was added K₂CO₃ (4.18 g, 30.3 mmol). The mixture was stirred at 100 °C for 15 h. After cooling to room temperature, the reaction mixture was quenched with water (50 mL) and extracted with EtOAc (30 mL x 3). The combined organic layers were washed with brine (10 mL x 2), dried over Na₂SO₄, filtered and concentrated in vacuo. The crude residue was purified by normal phase silica gel chromatography (Biotage 20 g cartridge, 0-65% EtOAc in petroleum ether) to give tert-butyl 4-hydroxy-3-((4-(methylsulfonyl)phenoxy)methyl)piperidine-1-carboxylate. MS = 408.1 [M+Na]⁺.

### Step 3: 3-((4-(methylsulfonyl)phenoxy)methyl)piperidin-4-ol

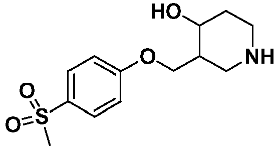

A solution of tert-butyl 4-hydroxy-3-[(4-methylsulfonylphenoxy)methyl]piperidine-1-carboxylate (1.20 g, 3.11 mmol) in HCl/EtOAc (4 M, 15 mL) was stirred at room temperature for 2 h and then concentrated in vacuo to give 3-((4-(methylsulfonyl)phenoxy)methyl)piperidin-4-ol (HCl salt), which was used in the next step without further purification. MS = 286.0 [M+H]⁺.

### Step 4: rac-trans or cis-1-(3-chlorophenethyl)-3-((4-(methylsulfonyl)phenoxy)methyl)piperidin-4-ol (Compound 27); rac-cis or trans-1-(3-chlorophenethyl)-3-((4-(methylsulfonyl)phenoxy)methyl)piperidin-4-ol (Compound 28)

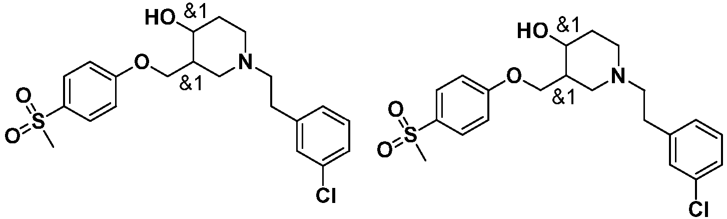

To a solution of 3-((4-(methylsulfonyl)phenoxy)methyl)piperidin-4-ol (970 mg, 3.01 mmol, HCl salt) and 2-(3-chlorophenyl)acetaldehyde (559 mg, 3.62 mmol) in MeOH (10 mL) was added TEA (0.839 mL, 6.03 mmol) and AcOH (34.48 µL, 0.603 mmol). After stirring for 30 min, NaBH₃CN (568 mg, 9.04 mmol) was added. The mixture was stirred at room temperature for 10 h, then was quenched with ice water (10 mL). The mixture was concentrated to remove solvent and extracted with EtOAc (10 mL x 3). The combined organic layers were washed with brine (5 mL), dried over Na₂SO₄, filtered and concentrated in vacuo. The crude residue was purified by reverse phase preparative HPLC (Waters Xbridge BEH C18 column, 30-50% MeCN/10 mM NH₄HCO₃ in water). The first eluting enantiomeric mixture of the title compound, **Compound 27:** ¹H NMR (400 MHz, DMSO-*d*₆): δ 7.86 - 7.81 (m, 2H), 7.33 - 7.14 (m, 6H), 4.81 - 4.78 (m, 1H), 4.26 (app dd, *J=* 10.0 Hz, 3.2 Hz, 1H), 4.01 (app dd, *J* = 9.6 Hz, 8.0 Hz, 1H), 3.48 - 3.36 (m, 1H), 3.15 (s, 3H), 3.02 (d, *J* = 10.0 Hz, 1H), 2.88 - 2.86 (m, 1H), 2.7 - 2.71 (m, 2H), 2.62 - 2.58 (m, 1H), 2.31 - 2.22 (m, 1H), 2.05 - 1.95 (m, 2H), 1.88 - 1.82 (m, 2H), 1.50 - 1.41 (m, 1H). MS = 424.3 [M+H]⁺. The second eluting enantiomeric mixture of the title compound, **Compound 28:** ¹H NMR (400 MHz, DMSO-*d*₆): δ 7.82 (d, *J=* 8.8 Hz, 2H), 7.28 - 7.13 (m, 6H), 4.69 (s, 1H), 4.16 - 4.12 (m, 1H), 4.04 - 3.99 (m, 1H), 3.85 (s, 1H), 3.15 (s, 3H), 2.76 - 2.68 (m, 2H), 2.61 - 2.55 (m, 2H), 2.49 - 2.33 (m, 4H), 2.10 - 2.09 (m, 1H), 1.66 - 1.52 (m, 2H). MS = 424.2 [M+H]⁺.

### Example 8

### (3S,4R) or (3R,4R) or (3S,4S) or (3R,4S)-1-(3-chlorophenethyl)-3-((4-(methylsulfonyl)phenoxy)methyl)piperidin-4-ol (Compound 29)

### (3R,4R) or (3S,4S) or (3R,4S) or (3S,4R) -1-(3-chlorophenethyl)-3-((4-(methylsulfonyl)phenoxy)methyl)piperidin-4-ol (Compound 30)

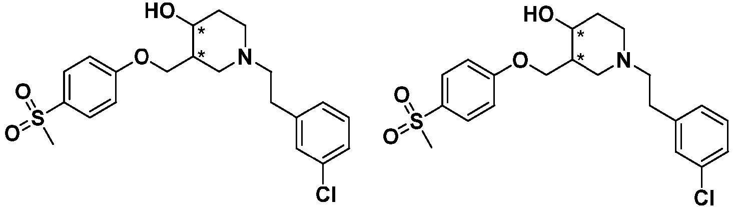

A solution of *rac-trans-* or rac-cis-1-(3-chlorophenethyl)-3-((4-(methylsulfonyl)phenoxy)methyl)piperidin-4-ol **(Compound 27,** 0.035 g, 0.083 mmol) in MeOH (1 mL) was separated by preparative chiral SFC (DAICEL CHIRALPAK IG-3, 60% MeOH with 0.1% NH₄OH in CO₂). The first eluting enantiomer of the title compound, **Compound 29:** ¹H NMR (400 MHz, DMSO-*d*₆): δ 7.84 - 7.80 (m, 2H), 7.32 - 7.14 (m, 6H), 4.79 (d, *J=* 5.2 Hz, 1H), 4.25 (app dd, *J=* 10.0 Hz, 2.8 Hz, 1H), 4.01 (app t, *J* = 8.4 Hz, 1H), 3.46 - 3.38 (m, 1H), 3.15 (s, 3H), 3.06 - 2.97 (m, 1H), 2.92 - 2.82 (m, 1H), 2.77 - 2.68 (m, 2H), 2.64 - 2.56 (m, 2H), 2.07 - 1.95 (m, 2H), 1.89 - 1.77 (m, 2H), 1.51 - 1.40 (m, 1H). MS = 424.2 [M+H]⁺. The second eluting enantiomer of the title compound, **Compound 30:** ¹H NMR (400 MHz, DMSO-*d*₆): δ 7.84 - 7.82 (m, 2H), 7.30 - 7.14 (m, 6H), 4.79 (d, *J* = 5.2 Hz, 1H), 4.25 (app dd, *J* = 9.6 Hz, 2.8 Hz, 1H), 4.03 - 3.98 (m, 1H), 3.29 (s, 1H), 3.15 (s, 3H), 3.02 (d, *J=* 10.4 Hz, 1H), 2.86 (d, *J* = 10.4 Hz, 1H), 2.77 - 2.64 (m, 2H), 2.62 - 2.54 (m, 2H), 2.06 - 1.95 (m, 2H), 1.87 - 1.76 (m, 2H), 1.50 - 1.40 (m, 1H). MS = 424.3 [M+H]⁺.

The following compounds in Table 15 were prepared according to procedures similar to those described for **Compound 29** using the appropriate starting materials.

**Table 15**

| No. | Starting Material | Structure | IUPAC Name | Chiral Column | Chiral Elution Order | Exact Mass [M+H]⁺ |
|---|---|---|---|---|---|---|
| **31** | **Compound 28** | 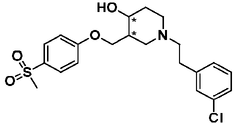 | (3*R*,4*R*) or (3*S*,4*S*) or (3*R*,4*S*) or (3*S*,4*R*) -1-(3-chlorophenet hyl)-3-((4-(methylsulfo nyl)phenoxy) methyl)piperi din-4-ol | DAICEL CHIRAL PAK IG-3 | 1st | Calc'd 424.1 |
| | | | | | | Found 424.2 |
| **32** | **Compound 28** | 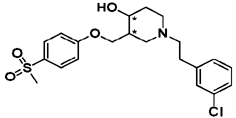 | (3*S*,4*S*) or (3*R*,4*S*) or (3*S*,4*R*) or (3*R*,4*R*) -1-(3-chlorophenet hyl)-3-((4-(methylsulfo nyl)phenoxy)methyl)piperi din-4-ol | DAICEL CHIRAL PAK IG-3 | 2nd | Calc'd 424.1 |
| | | | | | | Found 424.3 |

### Example 9

### rac-trans or cis-1-(3-chlorophenethyl)-4-methoxy-3-((4-(methylsulfonyl)phenoxy) methyl)piperidine (Compound 33)

### rac-cis or trans-1-(3-chlorophenethyl)-4-methoxy-3-((4-(methylsulfonyl)phenoxy) methyl)piperidine (Compound 34)

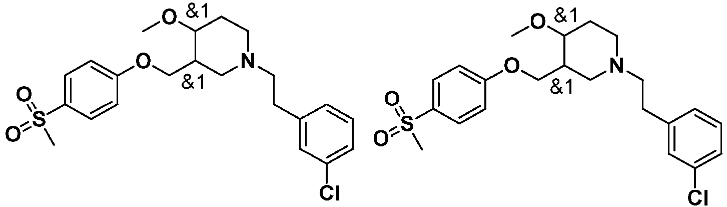

### Step 1: tert-butyl 3-(hydroxymethyl)-4-methoxypiperidine-1-carboxylate

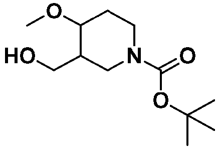

To a 0 °C solution of 1-(*tert*-butoxycarbonyl)-4-methoxypiperidine-3-carboxylic acid (900 mg, 3.47 mmol) in THF (10 mL) was added BH₃•THF (1.0 M, 6.94 mL) dropwise. The mixture was warmed to room temperature and stirred for 2 h. The reaction mixture was cooled to 0 °C and quenched with MeOH (20 mL), and then the adjusted pH=7 by the dropwise addition of aqueous NaHCO₃. The mixture was extracted with EtOAc (50 mL x 3), and the combined organic layers were dried over Na₂SO₄, filtered and concentrated in vacuo to give *tert*-butyl 3-(hydroxymethyl)-4-methoxypiperidine-1-carboxylate, which was used in the next step without further purification. MS = 190.2 [M-C₄H₇+H]⁺.

### Step 2: tert-butyl 4-methoxy-3-((4-(methylsulfonyl)phenoxy)methyl)piperidine-1-carboxylate

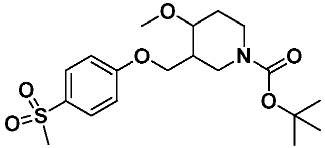

To a mixture of 1-fluoro-4-methylsulfonyl-benzene (508 mg, 2.91 mmol) and *tert-butyl* 3-(hydroxymethyl)-4-methoxy-piperidine-1-carboxylate (715 mg, 2.91 mmol) in DMF (10 mL) was added Cs₂CO₃ (2.85 g, 8.74 mmol). The mixture was stirred at 100 °C for 15 h, then was cooled to room temperature. The reaction mixture was diluted with water (30 mL), and then extracted with EtOAc (50 mL x 3). The combined organic layers were dried over Na₂SO₄, filtered and concentrated in vacuo. The crude residue was purified by normal phase silica gel chromatography (Biotage 20 g cartridge, 0-50% EtOAc in petroleum ether) to give tert-butyl 4-methoxy-3-((4-(methylsulfonyl)phenoxy)methyl)piperidine-1-carboxylate. MS = 344.2 [M-C₄H₇+H]⁺.

### Step 3: 4-methoxy-3-((4-(methylsulfonyl)phenoxy)methyl)piperidine

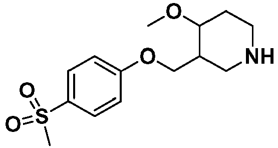

To a 0 °C solution of *tert*-butyl 4-methoxy-3-((4-(methylsulfonyl)phenoxy)methyl)piperidine-1-carboxylate (790 mg, 1.98 mmol) in EtOAc was added HCl/EtOAc (4 M, 10 mL). The mixture was warmed to room temperature and stirred for 1 h. The reaction mixture was filtered, and the filter cake was washed with petroleum ether (10 mL x 3) and concentrated under reduced pressure to give 4-methoxy-3-((4-(methylsulfonyl)phenoxy)methyl)piperidine (HCl salt), which was used in the next step without further purification. MS = 300.1 [M+H]⁺.

### Step 4: rac-trans or cis-1-(3-chlorophenethyl)-4-methoxy-3-((4-(methylsulfonyl)phenoxy) methyl)piperidine (Compound 33); rac-cis or trans-1-(3-chlorophenethyl)-4-methoxy-3-((4-(methylsulfonyl)phenoxy) methyl)piperidine (Compound 34)

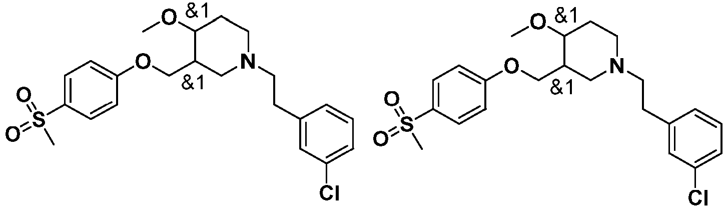

To a mixture of 4-methoxy-3-((4-(methylsulfonyl)phenoxy)methyl)piperidine (527 mg, 1.57 mmol, HCl salt) and 2-(3-chlorophenyl)acetaldehyde (485 mg, 3.14 mmol) in MeOH (8 mL) was added TEA (218 µL, 1.57 mmol) and HOAc (19.1 µL, 0.333 mmol). After stirring at room temperature for 3 h, NaBH₃CN (197 mg, 3.14 mmol) was added. The mixture was stirred at room temperature for 27 h, then was quenched with water (1 mL) and concentrated in vacuo. The residue was purified by reverse phase preparative HPLC (Kromasil C18 column, 45-65% MeCN/10 mM NH₄HCO₃ in water). The first eluting enantiomeric mixture of the title compound, **Compound 33 :** ¹H NMR (400 MHz, DMSO-*d*₆): δ 7.84 (d, *J* = 2.0 Hz, 2H), 7.31 - 7.15 (m, 6H), 4.19 - 403 (m, 2H), 3.25 (s, 3H), 3.24 (s, 3H), 3.16 - 3.13 (m, 1H), 2.98 - 2.84 (m, 2H), 2.75 - 2.50 (m, 4H), 2.12 - 2.05 (m, 4H), 1.37 - 1.35 (m, 1H). MS = 438.2 [M+H]⁺. The second eluting enantiomeric mixture of the title compound, **Compound 34:** ¹H NMR (400 MHz, DMSO-*d*₆): δ 7.83 (d, *J* = 8.8 Hz, 2H), 7.28 - 7.14 (m, 6H), 4.10 - 4.02 (m, 2H), 3.48 (s, 1H), 3.31 (s, 3H), 3.25 (s, 3H), 2.73 - 2.67 (m, 2H), 2.57 - 2.52 (m, 6H), 2.33 - 2.26 (m, 1H), 1.76 - 1.60 (m, 2H). MS = 438.2 [M+H]⁺.

### Example 10

### (3S,4R) or (3R,4R) or (3S,4S) or (3R,4S)-1-(3-chlorophenethyl)-4-methoxy-3-((4-(methylsulfonyl)phenoxy)methyl)piperidine (Compound 35)

### (3R,4R) or (3S,4S) or (3R,4S) or (3S,4R)-1-(3-chlorophenethyl)-4-methoxy-3-((4-(methylsulfonyl)phenoxy)methyl)piperidine (Compound 36)

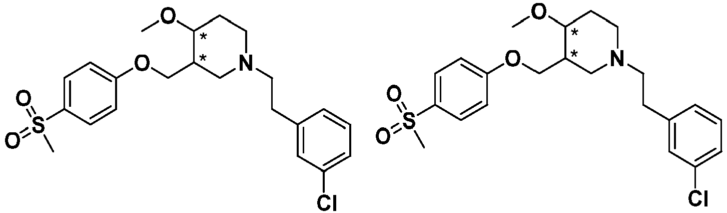

*Rac-trans* or *cis*-1-(3-chlorophenethyl)-4-methoxy-3-((4-(methylsulfonyl)phenoxy) methyl)piperidine **(Compound 33,** 120 mg, 0.275 mmol) was separated by preparative chiral SFC (DAICEL CHIRALPAK AD-3, 50% MeOH with 0.1% NH₄OH in CO₂). The first eluting enantiomer of the title compound, **Compound 35:** ¹H NMR (400 MHz, DMSO-*d*₆): δ 7.84 (d, *J=* 8.8 Hz, 2H), 7.30 - 7.15 (m, 6H), 4.19 - 4.16 (m, 1H), 4.07 - 4.03 (m, 1H), 3.25 (s, 3H), 3.15 (s, 3H), 3.08 (s, 1H), 2.98 - 2.85 (m, 2H), 2.75 - 2.53 (m, 4H), 2.10 - 1.97 (m, 4H), 1.38 - 1.35 (m, 1H). MS = 438.2 [M+H]⁺. The second eluting enantiomer of the title compound, **Compound 36:** ¹H NMR (400 MHz, DMSO-*d*₆): δ 7.83 (d, *J* = 8.4 Hz, 2H), 7.31 - 7.15 (m, 6H), 4.19 - 4.03 (m, 2H), 3.25 (s, 3H), 3.15 (s, 3H), 3.12 (s, 1H), 2.99 - 2.85 (m, 2H), 2.75 - 2.71 (m, 4H), 2.10 - 1.98 (m, 4H), 1.37 - 1.23 (m, 1H). MS = 438.2 [M+H]⁺.

The following compounds in Table 16 were prepared according to procedures similar to those described for **Compounds 35 and 36** using the appropriate starting materials.

**Table 16**

| No. | Starting Material | Structure | IUPAC Name | Chiral Column | Chiral Elution Order | Exact Mass [M+H]⁺ |
|---|---|---|---|---|---|---|
| **37** | **Compound 34** | 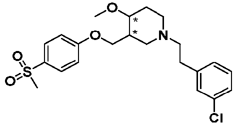 | (3*R*,4*R*) or (3*S*,4*S*) or (3*R*,4*S*) or (3*S*,4*R*) -1-(3-chlorophenethyl)-4-methoxy-3-((4-(methylsulfonyl) phenoxy)methyl) piperidine | DAICEL CHIRAL PAK AD-3 | 1st | Calc'd 438.1 |
| | | | | | | Found 438.2 |
| **38** | **Compound 34** | 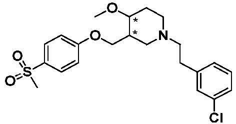 | (3*S*,4*S*) or (3*R*,4*S*) or (3 *S*,4*R*) or (3*R*,4*R*) -1-(3-chlorophenethyl) -4-methoxy-3-((4-(methylsulfonyl) phenoxy)methyl) piperidine | DAICEL CHIRAL PAK AD-3 | 2nd | Calc'd 438.1 |
| | | | | | | Found 438.2 |

### Example 11

### 1-(3-chlorophenethyl)-3-(4-(methylsulfonyl)phenethyl)piperidine (Compound 39)

### (S) or (R)-1-(3-chlorophenethyl)-3-(4-(methylsulfonyl)phenethyl)piperidine (Compound 40)

### (R) or (S)-1-(3-chlorophenethyl)-3-(4-(methylsulfonyl)phenethyl)piperidine (Compound 41)

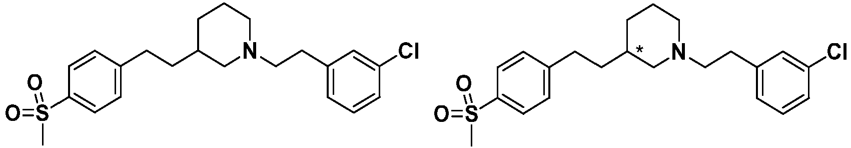

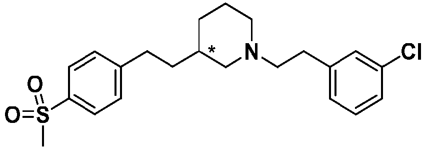

### Step 1: tert-butyl 5-(((trifluoromethyl)sulfonyl)oxy)-3,4-dihydropyridine-1(2H)-carboxylate

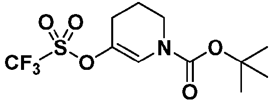

To a solution of *tert-butyl* 3-oxopiperidine-1-carboxylate (2.00 g, 10.0 mmol) in THF (20 mL) at -78 °C was added LiHMDS (6.02 mL, 2 M, 12.1 mmol) dropwise. After 30 min, 1,1,1-trifluoro-N-phenyl-N-(trifluoromethylsulfonyl)methanesulfonamide (4.30 g, 12.1 mmol) was added. The mixture was warmed to room temperature and stirred for 2 h, and then quenched with aqueous NH₄Cl (10 mL) and extracted with EtOAc (30 mL x 3). The combined organic layers were dried over Na₂SO₄, filtered and concentrated in vacuo. The crude residue was purified by normal phase silica gel chromatography (Biotage 10 g cartridge, 0-100% EtOAc in petroleum ether) to give tert-butyl 5-(((trifluoromethyl)sulfonyl)oxy)-3,4-dihydropyridine-1(2H)-carboxylate. MS = 354.0 [M+Na]⁺.

### Step 2: (E)-tert-butyl 5-(4-(methylsulfonyl)styryl)-3,4-dihydropyridine-1(2H)-carboxylate

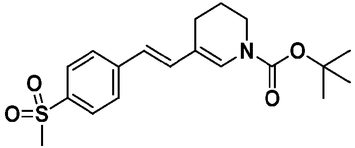

A solution of *tert-butyl* 5-(((trifluoromethyl)sulfonyl)oxy)-3,4-dihydropyridine-1(2H)-carboxylate (682 mg, 2.06 mmol), 1-(methylsulfonyl)-4-vinylbenzene (250 mg, 1.37 mmol), TEA (573 µL, 4.12 mmol), PPh₃ (14.39 mg, 0.055 mmol), and Pd(OAc)₂ (6.16 mg, 0.027 mmol) in DMF (5 mL) was sparged with N₂, then stirred at 60 °C for 5 h. The reaction mixture was cooled to room temperature, filtered, and the filtrate was concentrated in vacuo. The crude residue was purified by normal phase silica gel chromatography (Biotage 10 g cartridge, 0-10% EtOAc in petroleum ether) to give *(E)-tert*-butyl 5-(4-(methylsulfonyl)styryl)-3,4-dihydropyridine-1(2*H*)-carboxylate. MS = 364.1 [M+H]⁺.

### Step 3: tert-butyl 3-(4-(methylsulfonyl)phenethyl)piperidine-1-carboxylate

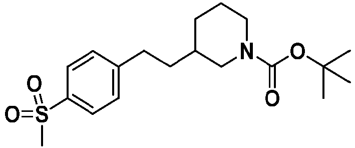

To a solution of *(E)-tert-*butyl 5-(4-(methylsulfonyl)styryl)-3,4-dihydropyridine-1(2H)-carboxylate (230 mg, 0.633 mmol) in MeOH (3 mL) was added Pd/C (50 mg, 10% by weight). The mixture was purged with H₂ twice and stirred under H₂ (15 Psi) at room temperature for 3 h. The reaction solution was filtered through celite, and the filtrate was concentrated in vacuo to give tert-butyl 3-(4-(methylsulfonyl)phenethyl)piperidine-1-carboxylate, which was used in the next step without further purification. MS = 390.1 [M+Na]⁺.

### Step 4: 3-(4-(methylsulfonyl)phenethyl)piperidine

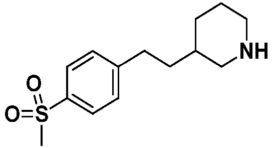

A solution of *tert-butyl* 3-(4-(methylsulfonyl)phenethyl)piperidine-1-carboxylate (250 mg, 0.680 mmol) in HCl/EtOAc (4 M, 5 mL) was stirred at room temperature for 1 h. The reaction was concentrated in vacuo to give 3-(4-(methylsulfonyl)phenethyl)piperidine (HCl salt), which was used in the next step without further purification. MS = 268.3 [M+H]⁺.

### Step 5: 1-(3-chlorophenethyl)-3-(4-(methylsulfonyl)phenethyl)piperidine (Compound 39)

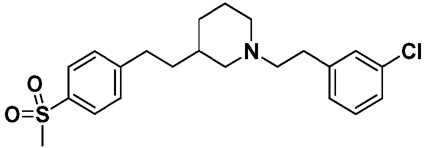

A solution of 3-(4-(methylsulfonyl)phenethyl)piperidine (0.23 g, 0.757 mmol, HCl salt) and 2-(3-chlorophenyl)acetaldehyde (117 mg, 0.757 mmol) in MeOH (5 mL) was stirred at room temperature for 1 h, and then NaBH₃CN (95.1 mg, 1.51 mmol) was added. After stirring for an additional 15 h at room temperature, the mixture was quenched with water (1 mL) and mixture was adjusted to pH 7 via the dropwise addition of aqueous 2 M HCl. The mixture was concentrated under reduced pressure and MeOH (3 mL) was added to the residue. The crude residue was purified by reverse phase preparative HPLC (Waters Xbridge BEH C18 column, 35-65% MeCN/10 mM NH₄HCO₃ in water) to give 1-(3-chlorophenethyl)-3-(4-(methylsulfonyl)phenethyl)piperidine **(Compound 39).** ¹H NMR (400 MHz, DMSO-*d*₆): δ 7.82 (d, *J=* 8.4 Hz, 2H), 7.47 (d, *J* = 8.4 Hz, 2H), 7.32 - 7.15 (m, 4H), 3.31 (s, 3H), 2.80 - 2.68 (m, 6H), 2.48 - 2.44 (m, 2H), 1.99 - 1.90 (m, 1H), 1.74 - 1.37 (m, 7H), 0.94 - 0.86 (m, 1H). MS = 406.2 [M+H]⁺.

### Step 6: (S) or (R)-1-(3-chlorophenethyl)-3-(4-(methylsulfonyl)phenethyl)piperidine (Compound 40); (R) or (S)-1-(3-chlorophenethyl)-3-(4-(methylsulfonyl)phenethyl)piperidine (Compound 41)

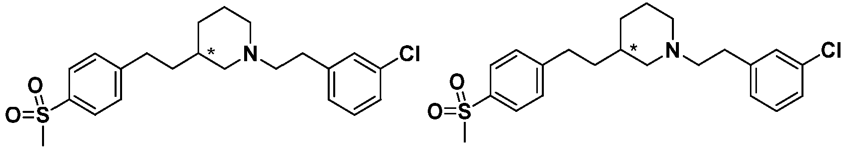

1-(3-chlorophenethyl)-3-(4-(methylsulfonyl)phenethyl)piperidine **(Compound 39, 250** mg, 0.616 mmol) was separated by preparative chiral SFC (DAICEL CHIRALPAK AD-3, 50% MeOH with 0.1% NH₄OH in CO₂). The first eluting enantiomer of the title compound, **Compound** 40: ¹H NMR (400 MHz, DMSO-*d*₆): δ 7.82 (d, *J* = 8.4 Hz, 2H), 7.47 (d, *J=* 8.4 Hz, 2H), 7.32 - 7.17 (m, 4H), 3.18 (s, 3H), 2.84 - 2.68 (m, 6H), 2.48 - 2.44 (m, 2H), 1.93 - 1.90 (m, 1H), 1.74 - 1.41 (m, 7H), 0.92 - 0.89 (m, 1H). MS = 406.1 [M+H]⁺. The second eluting enantiomer of the title compound, **Compound 41:** ¹H NMR (400 MHz, DMSO-*d*₆): δ 7.82 (d, *J* = 8.4 Hz, 2H), 7.47 (d, *J* = 8.4 Hz, 2H), 7.31 - 7.19 (m, 4H), 3.18 (s, 3H), 2.85 - 2.68 (m, 6H), 2.48 - 2.44 (m, 2H), 1.99 - 1.90 (m, 1H), 1.74 - 1.41 (m, 7H), 0.92 - 0.89 (m, 1H). MS = 406.1 [M+H]⁺.

### Example 12

### 1-(3-chlorophenethyl)-2,2-dimethyl-4-((4-(methylsulfonyl)phenoxy) methyl) pyrrolidine (Compound 42)

### (S) or (R)-1-(3-chlorophenethyl)-2,2-dimethyl-4-((4-(methylsulfonyl)phenoxy) methyl) pyrrolidine (Compound 43)

### (R) or (S)-1-(3-chlorophenethyl)-2,2-dimethyl-4-((4-(methylsulfonyl)phenoxy)methyl)pyrrolidine (Compound 44)

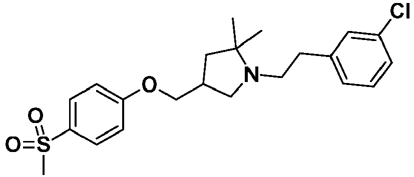

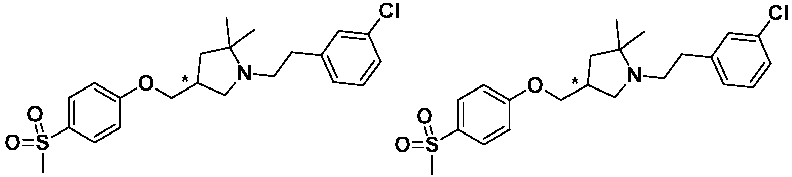

### Step 1: tert-butyl 2,2-dimethyl-4-((4-(methylsulfonyl)phenoxy)methyl)pyrrolidine- 1-carboxylate

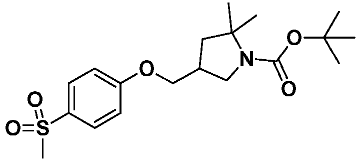

A mixture of tert-butyl 4-(hydroxymethyl)-2,2-dimethylpyrrolidine-1-carboxylate (500 mg, 2.18 mmol), 1-fluoro-4-methylsulfonyl-benzene (380 mg, 2.18 mmol) and Cs₂CO₃ (2.13 g, 6.54 mmol) in DMF (5 mL) was stirred at 100 °C for 16 h. The reaction mixture was cooled to room temperature, quenched by the addition of water (15 mL), and then extracted with EtOAc (15 mL x 3). The combined organic layers were washed with water (15 mL x 3), dried over Na₂SO₄, filtered and concentrated in vacuo. The crude residue was purified by normal phase silica gel chromatography (Biotage 12 g cartridge, 0-45% EtOAc in petroleum ether) to give tert-butyl 2,2-dimethyl-4-((4-(methylsulfonyl)phenoxy)methyl)pyrrolidine-1-carboxylate. MS = 406.2 [M+Na]⁺.

### Step 2: 2,2-dimethyl-4-((4-(methylsulfonyl)phenoxy)methyl)pyrrolidine

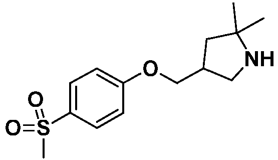

To a solution of tert-butyl 2,2-dimethyl-4-((4-(methylsulfonyl)phenoxy)methyl)pyrrolidine-1-carboxylate (0.85 g, 2.22 mmol) in DCM (10 mL) was added TFA (4 mL). The mixture was stirred at room temperature for 1.5 h, and then was quenched by the addition of water (15 mL). The mixture was concentrated under reduced pressure to remove DCM, and the resulting aqueous layer was extracted with EtOAc (15 mL x 5). The combined organic layers were dried over Na₂SO₄, filtered and concentrated under reduced pressure to give 2,2-dimethyl-4-((4-(methylsulfonyl)phenoxy)methyl)pyrrolidine (TFA salt). MS = 284.1 [M+H]⁺.

### Step 3: 1-(3-chlorophenethyl)-2,2-dimethyl-4-((4-(methylsulfonyl)phenoxy)methyl) pyrrolidine (Compound 42)

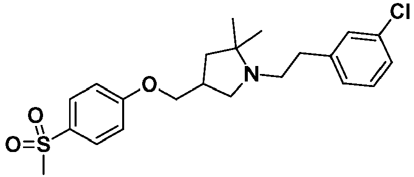

To a solution of 2-(3-chlorophenyl)acetaldehyde (982 mg, 6.35 mmol) and TEA (663 µL, 4.76 mmol) in MeOH (5 mL) was added 2,2-dimethyl-4-((4-(methylsulfonyl)phenoxy)methyl)pyrrolidine (450 mg, 1.18 mmol, TFA salt). After stirring at room temperature for 30 min, the reaction was cooled to 0 °C and NaBH₃CN (299 mg, 4.76 mmol) was added. The resulting mixture was stirred at room temperature for 24 h, then was quenched by the addition of water (0.5 mL) and concentrated in vacuo. The crude residue was purified by reverse phase preparative HPLC (Waters Xbridge BEH C18 column, 50-80% MeCN/10 mM NH₄HCO₃ in water) to give 1-(3-chlorophenethyl)-2,2-dimethyl-4-((4-(methylsulfonyl)phenoxy)methyl)pyrrolidine **(Compound 42).** ¹H NMR (400 MHz, DMSO-*d*₆): δ 7.83 (d, *J=* 8.8 Hz, 2H), 7.30 - 7.14 (m, 6H), 3.95 (d, *J=* 7.2 Hz, 2H), 3.15 (s, 3H), 2.83 - 2.75 (m, 2H), 2.70 - 2.62 (m, 3H), 2.60 - 2.54 (m, 1H), 2.47 - 2.41 (m, 1H), 1.81 (app dd, *J* = 12.4 Hz, 9.2 Hz, 1H), 1.34 (app dd, *J* = 12.4 Hz, 6.8 Hz, 1H), 0.95 (s, 3H), 0.89 (s, 3H). MS = 422.1 [M+H]⁺.

### Step 4: (S) or (R)-1-(3-chlorophenethyl)-2,2-dimethyl-4-((4-(methylsulfonyl)phenoxy) methyl) pyrrolidine (Compound 43); (R) or (S)-1-(3-chlorophenethyl)-2,2-dimethyl-4-((4-(methylsulfonyl)phenoxy)methyl)pyrrolidine (Compound 44)

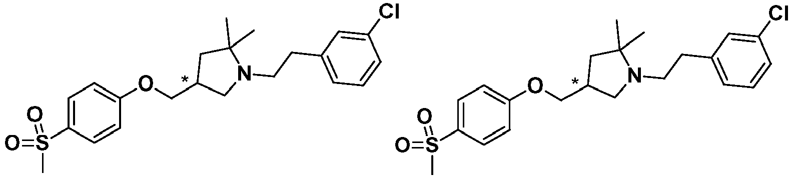

1-(3-chlorophenethyl)-2,2-dimethyl-4-((4-(methylsulfonyl)phenoxy)methyl)pyrrolidine **(Compound 42,** 140 g, 332 mmol) was separated by preparative chiral SFC (DAICEL CHIRALPAK AD-3, 38% MeOH with 0.1% NH₄OH in CO₂). The first eluting enantiomer of the title compound, **Compound 43:** ¹H NMR (400 MHz, DMSO-*d*₆): δ 7.83 (d, *J* = 8.8 Hz, 2H), 7.30 - 7.14 (m, 6H), 3.95 (d, *J* = 7.2 Hz, 2H), 3.15 (s, 3H), 2.83 - 2.76 (m, 2H), 2.70 - 2.62 (m, 3H), 2.55 - 2.54 (m, 1H), 2.46 - 2.45 (m, 1H), 1.81 (app dd, *J* = 12.0 Hz, 9.2 Hz, 1H), 1.34 (app dd, *J* = 12.4 Hz, 7.2 Hz, 1H), 0.94 (s, 3H), 0.89 (s, 3H). MS = 422.1 [M+H]⁺. The second eluting enantiomer of the title compound, **Compound 44:** ¹H NMR (400 MHz, DMSO-*d*₆): δ 7.83 (d, *J* = 8.8 Hz, 2H), 7.30 - 7.14 (m, 6H), 3.95 (d, *J* = 7.2 Hz, 2H), 3.15 (s, 3H), 2.81 - 2.80 (m, 2H), 2.68 - 2.64 (m, 3H), 2.56 - 2.54 (m, 1H), 2.46 - 2.45 (m, 1H), 1.81 (app dd, *J* = 12.4 Hz 9.2 Hz, 1H), 1.34 (d, *J=* 12.4 Hz, 7.2 Hz, 1H), 0.95 (s, 3H), 0.89 (s, 3H). MS = 422.1 [M+H]⁺.

### Example 13

### rac-trans-N-(4-((1-(3-chlorophenethyl)-4-methylpyrrolidin-3-yl)methoxy)phenyl)-N-methylmethanesulfonamide (Compound 45)

### N-(4-(((3S,4S) or (3R,4R) -1-(3-chlorophenethyl)-4-methylpyrrolidin-3-yl)methoxy)phenyl)-N-methylmethanesulfonamide (Compound 46)

### N-(4-(((3R,4R) or (3S,4S) -1-(3-chlorophenethyl)-4-methylpyrrolidin-3-yl)methoxy)phenyl)-N-methylmethanesulfonamide (Compound 47)

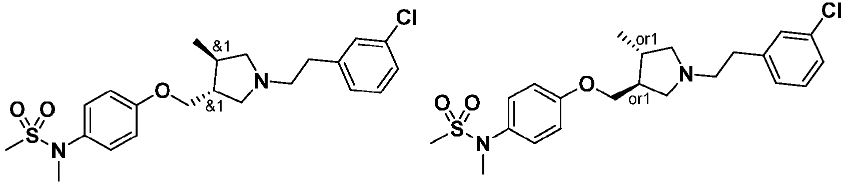

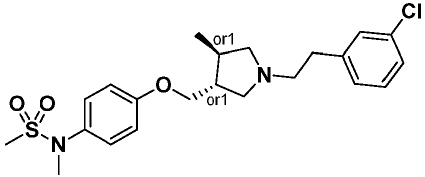

### Step 1: rac-trans-tert-butyl-3-methyl-4-((4-(N-methylmethylsulfonamido)phenoxy)methyl)pyrrolidine-1-carboxylate

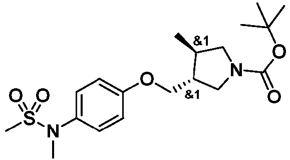

To a solution of N-(4-bromophenyl)-N-methylmethanesulfonamide (300 mg, 1.14 mmol), copper(I) iodide (11 mg, 0.057 mmol), cesium carbonate (0.554 g, 1.704 mmol), and 3,4,7,8-tetramethyl-1,10-phenanthroline (27 mg, 0.11 mmol) in toluene (1.62 mL) was added *rac-trans-tert-butyl-*3-(hydroxymethyl)-4-methylpyrrolidine-1-carboxylate (0.245 g, 1.14 mmol). The mixture was sparged with nitrogen for 1 min and sealed. The reaction was heated to 100 °C for 16 h. After cooling to room temperature, the mixture was diluted with EtOAc (10 mL) and filtered, solids were washed with EtOAc (10 mL x 2), and the filtrate was concentrated in vacuo. The crude residue was dissolved in EtOAc (10 mL) and washed with aqueous 20% citric acid (10 mL). The aqueous layer was extracted with EtOAc (10 mL) and the combined organic layers were dried over Na₂SO₄, filtered and concentrated in vacuo. The crude residue was purified by normal phase silica gel chromatography (Biotage 25 g cartridge, 0-7% MeOH in DCM) to give *rac-trans-tert*-butyl-3-methyl-4-((4-(*N-*methylmethylsulfonamido)phenoxy)methyl)pyrrolidine-1-carboxylate. MS = 421.1 [M+Na]⁺.

### Step 2: rac-trans-N-methyl-N-(4-((-4-methylpyrrolidin-3-yl)methoxy)phenyl)methanesulfonamide

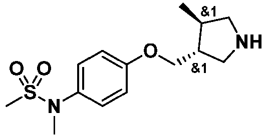

*rac-trans-tert-butyl-3-methyl-4-((4-(N-*methylmethylsulfonamido)phenoxy)methyl)pyrrolidine-1-carboxylate was dissolved in 20% TFA in DCM (3 mL) and allowed to stir at room temperature for 1 h. The reaction was concentrated in vacuo. The crude residue was dissolved in water (10 mL) and washed with DCM (10 mL). The aqueous layer was adjusted to pH = 10 with the dropwise addition of 1 M NaOH. The aqueous layer was extracted with DCM (10 mL x 2), and the combined organic layers were dried over Na₂SO₄, filtered and concentrated in vacuo to give *rac-trans-N-*methyl*-N*-(4-((4-methylpyrrolidin-3-yl)methoxy)phenyl)methanesulfonamide, which was taken to the next step without purification. MS = 298.8 [M+H]⁺.

### Step 3: rac-trans-N-(4-((1-(3-chlorophenethyl)-4-methylpyrrolidin-3-yl)methoxy)phenyl)-N-methylmethanesulfonamide (Compound 45)

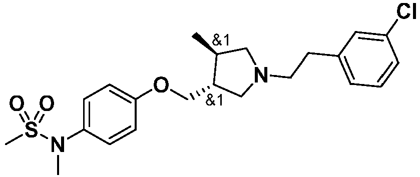

To a solution of *rac-trans-tert-*butyl-3-methyl-4-((4-(*N* methylmethylsulfonamido)phenoxy)methyl)pyrrolidine-1-carboxylate (54 mg, 0.18 mmol) and K₂CO₃ (0.10 g, 0.72 mmol) in MeCN (0.9 mL) was added 1-(2-bromoethyl)-3-chlorobenzene (0.048 g, 0.22 mmol). The mixture was heated to 70 °C and allowed to stir for 12 h. After cooling to the reaction to room temperature, the mixture was concentrated in vacuo. Water (10 mL) was added and the mixture was extracted with EtOAc (10 mL x 3). The combined organic layers were dried over Na₂SO₄, filtered and concentrated in vacuo. The crude residue was purified by reverse phase preparative HPLC (Phenomenex Kinetex C18 column, 0-40% MeCN/water with 0.1% formic acid modifier) to give *rac-trans-N-*(4-((1-(3-chlorophenethyl)-4-methylpyrrolidin-3-yl)methoxy)phenyl)-N-methylmethanesulfonamide **(Compound 45)** ¹H NMR (500 MHz, CDCl₃): δ 7.24 - 7.20 (m, 2H), 7.17 - 7.10 (m, 3H), 7.04 - 7.02 (m, 1H), 6.84 - 6.79 (m, 2H), 3.94 - 3.84 (m, 2H), 3.21 (s, 3H), 3.16 - 3.11 (m, 1H), 2.96 - 2.89 (m, 2H), 2.88 - 2.77 (m, 4H), 2.76 (s, 3H), 2.37 (t, *J* = 9.1 Hz, 1H), 2.27 - 2.18 (m, 1H), 2.18 - 2.07 (m, 1H), 1.10 (d, *J* = 6.7 Hz, 3H). MS = 437.1 [M+H]⁺.

### Step 4: N-(4-(((3S,4S) or (3R,4R) -1-(3-chlorophenethyl)-4-methylpyrrolidin-3-yl)methoxy)phenyl)-N-methylmethanesulfonamide (Compound 46); N-(4-(((3R,4R) or (3S,4S) -1-(3-chlorophenethyl)-4-methylpyrrolidin-3-yl)methoxy)phenyl)-N-methylmethanesulfonamide (Compound 47)

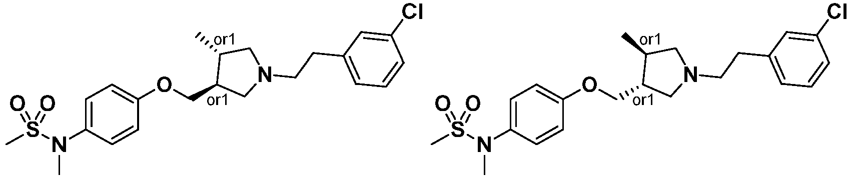

*rac-trans-N-(4-((1-(3-chlorophenethyl)-4-methylpyrrolidin-3-*yl)methoxy)phenyl)-N-methylmethanesulfonamide **(Compound 45,** 26.2 mg, 0.060 mmol) was separated by preparative chiral SFC (Chiralcel OD-H column, 30% isopropanol with 0.25% isopropylamine in CO₂). The first eluting enantiomer of the title compound, Compound 46: ¹H NMR (500 MHz, DMSO-*d*₆): δ 7.34 - 7.16 (m, 6H), 6.98 - 6.92 (m, 2H), 3.94 - 3.91 (m, 1H), 3.87 - 3.84 (m, 1H), 3.18 (s, 3H), 2.90 (s, 3H), 2.85 (app dd, *J* = 8.8, 7.3 Hz, 1H), 2.74 - 2.71 (m, 2H), 2.66 - 2.55 (m, 4H), 2.11 - 2.05 (m, 1H), 2.05 - 2.04 (m, 1H), 1.94 - 1.88 (m, 1H), 1.06 (d, *J=* 6.8 Hz, 3H). MS = 437.1 [M+H]⁺. The second eluting enantiomer of the title compound, **Compound 47:** ¹H NMR (500 MHz, DMSO-*d*₆): δ 7.36 - 7.15 (m, 6H), 6.98 - 6.92 (m, 2H), 3.94 - 3.91 (m, 1H), 3.87 - 3.84 (m, 1H), 3.18 (s, 3H), 2.90 (s, 3H), 2.84 (app dd, *J* = 8.7, 7.3 Hz, 1H), 2.74 - 2.71 (m, 2H), 2.66 - 2.53 (m, 4H), 2.12 - 2.01 (m, 2H), 1.94 - 1.88 (m, 1H), 1.06 (d, *J* = 6.7 Hz, 3H). MS = 437.1 [M+H]⁺.

The following compounds in Table 17 were prepared according to procedures similar to steps 1-3 described for **Compound 45** using the appropriate starting materials.

**Table 17**

| No. | Structure | IUPAC Name | Exact Mass [M+H]⁺ |
|---|---|---|---|
| **48** | 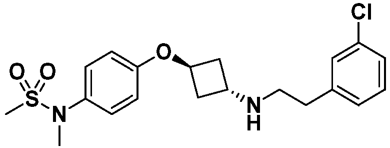 | *trans-N-*(4-(3-((3-chlorophenethyl)amino)cycl obutoxy)phenyl)-*N-*methylmethanesulfonamide | Calc'd 409.1 |
| | | | Found 409.0 |
| **49** | 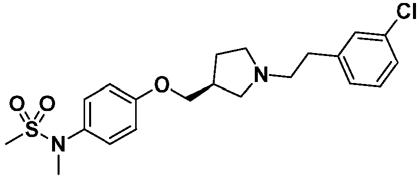 | (*S*)-*N-*(4-((1-(3-chlorophenethyl)pyrrolidin-3-yl)methoxy)phenyl)-*N-*methylmethanesulfonamide | Calc'd 423.1 |
| | | | Found 423.0 |
| **50** | 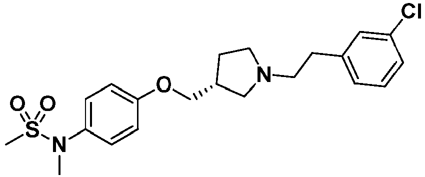 | (*R*)*-N-*(4-((1-(3-chlorophenethyl)pyrrolidin-3-yl)methoxy)phenyl)-*N-*methylmethanesulfonamide | Calc'd 423.1 |
| | | | Found 423.0 |
| **51** | 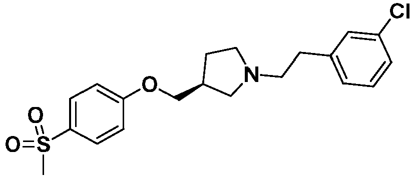 | (*S*)-1-(3-chlorophenethyl)-3-((4-(methylsulfonyl)phenoxy)m ethyl)pyrrolidine | Calc'd 394.1 |
| | | | Found 394.1 |

### Example 14

### rac-trans-1-(3-chlorophenethyl)-3-methyl-4-((4-(methylsulfonyl)phenoxy)methyl)pyrrolidine (Compound 52)

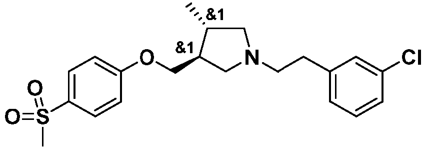

### Step 1: rac-trans-tert-butyl-3-methyl-4-((4-(methylsulfonyl)phenoxy)methyl)pyrrolidine-1-carboxylate

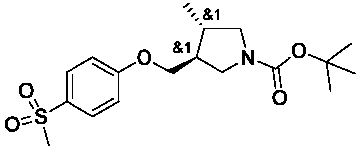

To a solution of 1-fluoro-4-methanesulfonylbenzene (0.405 g, 2.32 mmol) and *rac-trans-tert*-butyl-3-(hydroxymethyl)-4-methylpyrrolidine-1-carboxylate (1000 mg, 4.65 mmol) in DMF (4.6 mL) was added K₂CO₃ (1.28 g, 9.29 mmol). The reaction was heated to 110 °C for 16 h. The reaction mixture was cooled to room temperature and water (50 mL) was added. The mixture was extracted with EtOAc (3 x 50 mL). The combined organic layers were washed with brine (50 mL), dried over Na₂SO₄, filtered and concentrated in vacuo. The crude residue was purified by normal phase flash chromatography (Biotage 25g cartridge, 0-10% MeOH in DCM) to give *rac*-*trans*-*tert*-butyl-3-(4-methanesulfonylphenoxymethyl)-4-methylpyrrolidine-1-carboxylate. MS = 392.0 [M+Na]⁺.

### Step 2: rac-trans-3-methyl-4-((4-(methylsulfonyl)phenoxy)methyl)pyrrolidine

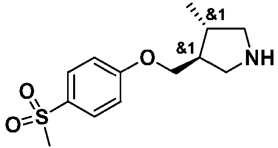

To a solution of *rac*-*trans*-*tert*-butyl-3-(4-methanesulfonylphenoxymethyl)-4-methylpyrrolidine-1-carboxylate (900 mg, 2.44 mmol) in MeOH (5 mL) was added HCl in dioxane (4 M, 5 mL). The reaction was allowed to stir for 2 h at room temperature. The reaction mixture was concentrated in vacuo to give *rac*-*trans*-3-(4-methanesulfonylphenoxymethyl)-4-methylpyrrolidine (HCl salt), which was taken to the next step without purification. MS = 269.9 [M+H]⁺.

### Step 3: rac-trans-1-(3-chlorophenethyl)-3-methyl-4-((4 (methylsulfonyl)phenoxy)methyl)pyrrolidine (Compound 52)

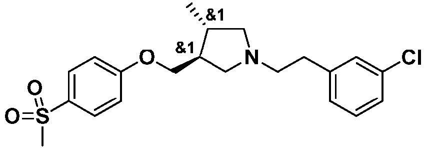

A mixture of *rac-trans*-3-(4-methanesulfonylphenoxymethyl)-4-methylpyrrolidine (100 mg, 0.327 mmol, HCl salt), 1-(2-bromoethyl)-3-chlorobenzene (0.086 g, 0.392 mmol), and K₂CO₃ (0.181 g, 1.308 mmol) was suspended in MeCN (1.6 mL). The resulting mixture was heated to 70 °C and allowed to stir for 18 h. The reaction was cooled to room temperature and concentrated in vacuo. Water (25 mL) was added, and the mixture was extracted with EtOAc (25 mL x 3). The combined organic layers were washed with brine (25 mL), dried over Na₂SO₄, filtered and concentrated in vacuo. The crude residue was purified by reverse phase preparative HPLC (Phenomenex Kinetex C18 column, 5-50% MeCN/water with 0.1% formic acid modifier) to give *rac-trans*-1-(3-chlorophenethyl)-3-methyl-4-((4-(methylsulfonyl)phenoxy)methyl)pyrrolidine **(Compound 52)** ¹H NMR (500 MHz, CD₃CN): δ 7.84 (d, *J=* 8.9 Hz, 2H), 7.31 - 7.15 (m, 4H), 7.09 (d, *J=* 8.9 Hz, 2H), 4.10 - 4.07 (m, 1H), 4.03 - 3.99 (m, 1H), 3.16 - 3.09 (m, 1H), 3.01 (s, 3H), 3.00 - 2.93 (m, 1H), 2.92 - 2.79 (m, 5H), 2.44 - 2.37 (m, 1H), 2.29 - 2.19 (m, 1H), 2.16 - 2.05 (m, 1H), 1.12 (d, *J* = 6.7 Hz, 3H). MS = 408.1 [M+H]⁺.

The following compounds in Table 18 were prepared according to procedures similar to steps 1-3 described for **Compound 52** using the appropriate starting materials.

**Table 18**

| No. | Structure | IUPAC Name | Exact Mass [M+H]⁺ |
|---|---|---|---|
| **132** | 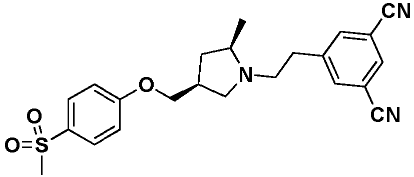 | 5-{2-[(2*R*,4*S*)-4-[(4-methanesulfonylphenoxy)me thyl]-2-methylpyrrolidin-1-yl]ethyl}benzene-1,3-dicarbonitrile | Calc'd 424.2 |
| | | | Found 424.1 |
| **133** | 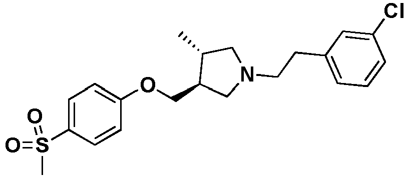 | (3*S*,4*S*)-1-[2-(3-chlorophenyl)ethyl]-3-[(4-methanesulfonylphenoxy)me thyl]-4-methylpyrrolidine | Calc'd 408.1 |
| | | | Found 408.1 |
| **134** | 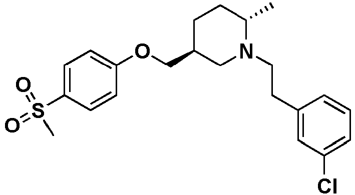 | (2*S*,5*S*)-1-[2-(3-chlorophenyl)ethyl]-5-[(4-methanesulfonylphenoxy)me thyl]-2-methylpiperidine | Calc'd 422.1 |
| | | | Found 422.2 |
| **160** | 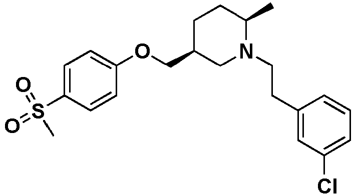 | (2*R*,5*S*)-1-[2-(3-chlorophenyl)ethyl]-5-[(4-methanesulfonylphenoxy)me thyl]-2-methylpiperidine | Calc'd 422.1 |
| | | | Found 422.2 |

### Example 15

### rac-trans or cis-1-(3-chlorophenethyl)-2-methyl-4-((4-(methylsulfonyl)phenoxy)methyl)pyrrolidine (Compound 53)

### rac-cis or trans-1-(3-chlorophenethyl)-2-methyl-4-((4-(methylsulfonyl)phenoxy)methyl)pyrrolidine (Compound 54)

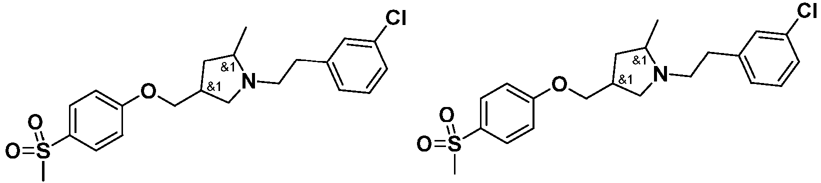

### Step 1: tert-butyl 4-(hydroxymethyl)-2-methylpyrrolidine-1-carboxylate

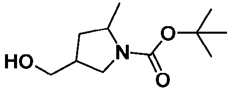

To a 0 °C solution of 1-(tert-butoxycarbonyl)-5-methylpyrrolidine-3-carboxylic acid (450 mg, 1.96 mmol) in THF (10 mL) was added BH₃•THF (1 M, 3.14 mL). The mixture was warmed to room temperature and stirred for 2 h. The reaction mixture was cooled to 0 °C, quenched with aqueous 2 M HCl (20 mL), and stirred at room temperature for 20 min. The mixture was extracted with EtOAc (20 mL x 3). The combined organic layers were washed with saturated aqueous NaHCO₃ (40 mL), brine (40 mL), dried over Na₂SO₄, filtered and concentrated in vacuo to give tert-butyl 4-(hydroxymethyl)-2-methylpyrrolidine-1-carboxylate, which was used in the next step without further purification. MS = 160.1 [M-C₄H₇+H]⁺.

### Step 2: 1-(3-chlorophenethyl)-2,2-dimethyl-4-((4-(methylsulfonyl)phenoxy)methyl) pyrrolidine

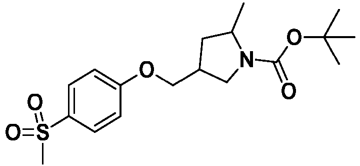

A mixture of tert-butyl 4-(hydroxymethyl)-2-methylpyrrolidine-1-carboxylate (380 mg, 1.77 mmol), 1-fluoro-4-(methylsulfonyl)benzene (399 mg, 2.29 mmol) and Cs₂CO₃ (1.73 g, 5.30 mmol) in DMF (6 mL) was stirred at 100 °C for 16 h. After cooling to room temperature, the mixture was poured into water (30 mL) at 0 °C and extracted with EtOAc (30 mL x 3). The combined organic layers were washed with water (60 mL), brine (60 mL), dried over Na₂SO₄, filtered and concentrated in vacuo. The crude residue was purified by normal phase silica gel chromatography (Biotage 12 g cartridge, 25-50% EtOAc in petroleum ether) to give tert-butyl 2-methyl-4-((4-(methylsulfonyl)phenoxy)methyl)pyrrolidine-1-carboxylate. MS = 314.1 [M+Na]⁺.

### Step 3: 2-methyl-4-[(4-methylsulfonylphenoxy) methyl]pyrrolidine

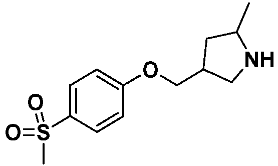

To a 0 °C solution of tert-butyl 2-methyl-4-((4-(methylsulfonyl)phenoxy)methyl)pyrrolidine-1-carboxylate (570 mg, 1.54 mmol) in DCM (5 mL) was added TFA (2.5 mL, 33.7 mmol). The mixture was warmed to room temperature and stirred for 1 h, then was concentrated under reduced pressure. The residue was dissolved in aqueous NaHCO₃ (15 mL) and was extracted with a solution of 1:3 isopropanol : DCM (15 mL x 3). The combined organic layers were dried over Na₂SO₄, filtered and the filtrate was concentrated in vacuo to give 2-methyl-4-[(4-methylsulfonylphenoxy) methyl]pyrrolidine, which was used in the next step without further purification. MS = 270.1 [M+H]⁺.

### Step 4: rac-trans or cis-1-(3-chlorophenethyl)-2-methyl-4-((4-(methylsulfonyl)phenoxy)methyl)pyrrolidine (Compound 53); rac-cis or trans-1-(3-chlorophenethyl)-2-methyl-4-((4-(methylsulfonyl)phenoxy)methyl)pyrrolidine (Compound 54)

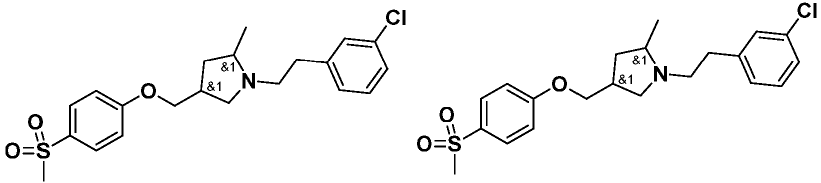

A mixture of 2-methyl-4-((4-(methylsulfonyl)phenoxy)methyl)pyrrolidine (350 mg, 1.30 mmol) and 2-(3-chlorophenyl)acetaldehyde (261 mg, 1.69 mmol) in MeOH (4 mL) was stirred at room temperature for 10 min. After cooling to 0 °C, NaBH₃CN (122 mg, 1.95 mmol) was added, and the mixture was stirred at room temperature for 1 h. The mixture was quenched by the addition of water (0.3 mL) and purified by reverse phase preparative HPLC (Waters Xbridge C18 column, 45-65% MeCN/10 mM NH₄HCO₃ in water). The first eluting enantiomeric mixture of the title compound, **Compound 53:** ¹H NMR (400 MHz, DMSO-*d*₆): δ 7.83 (d, *J=* 8.4 Hz, 2H), 7.33 - 7.20 (m, 4H), 7.16 - 7.14 (m, 2H), 4.0 - 3.94 (m, 2H), 3.35 - 3.33 (m, 1H), 3.15 (s, 3H), 2.97 - 2.95 (m, 1H), 2.78 - 2.71 (m, 2H), 2.57 - 2.56 (m, 1H), 2.46 - 2.45 (m, 1H), 2.29 - 2.28 (m, 1H), 2.06 - 2.01 (m, 1H), 1.73 - 1.71 (m, 1H), 1.54 - 1.53 (m, 1H), 1.01 (d, *J=* 6.0 Hz, 3H). MS = 408.2 [M+H]⁺. The second eluting enantiomeric mixture of the title compound, **Compound 54:** ¹H NMR (400 MHz, DMSO-*d*₆): δ 7.83 (d, *J* = 8.8 Hz, 2H), 7.31 (s, 1H), 7.25 - 7.14 (m, 5H), 3.96 - 3.89 (m, 2H), 3.16 (s, 3H), 3.08 - 3.06 (m, 1H), 2.94 - 2.93 (m, 1H), 2.76 - 2.68 (m, 2H), 2.48 - 2.47 (m, 1H), 2.34 - 2.29 (m, 3H), 2.13 - 2.09 (m, 1H), 1.09 - 1.00 (m, 4H). MS = 408.2 [M+H]⁺.

### Example 16

### (2R,4S) or (2R,4R) or (2S,4R) or (25,45)-1-(3-chlorophenethyl)-2-methyl-4-((4-(methylsulfonyl)phenoxy)methyl)pyrrolidine (Compound 55)

### (2R,4R) or (2S,4R) or (2S,4S) or (2R,4S)-1-(3-chlorophenethyl)-2-methyl-4-((4-(methylsulfonyl)phenoxy)methyl)pyrrolidine (Compound 56)

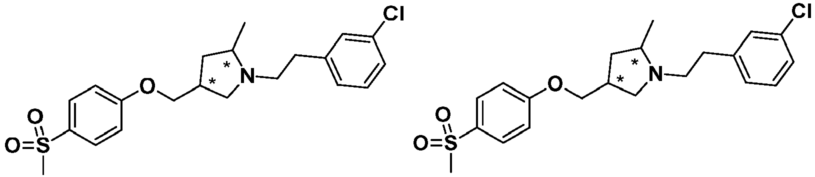

*rac-trans* or cis-1-(3-chlorophenethyl)-2-methyl-4-((4-(methylsulfonyl)phenoxy)methyl)pyrrolidine **(Compound 53,** 230 mg, 0.563 mmol) was separated by preparative chiral SFC (DAICEL CHIRALPAK AD-3, 44% MeOH with 0.1% NH₄OH in CO₂). The first eluting enantiomer of the title compound, **Compound 55:** ¹H NMR (400 MHz, DMSO-*d*₆): δ 7.83 (d, *J* = 8.8 Hz, 2H), 7.33 - 7.21 (m, 4H), 7.17 - 7.14 (m, 2H), 4.04 - 3.94 (m, 2H), 3.32 - 3.31 (m, 1H), 3.15 (s, 3H), 2.98 - 2.96 (m, 1H), 2.78 - 2.70 (m, 2H), 2.56 - 2.55 (m, 1H), 2.44 - 2.43 (m, 1H), 2.27 - 2.26 (m, 1H), 2.03 - 2.02 (m, 1H), 1.76 - 1.72 (m, 1H), 1.57 - 1.49 (m, 1H), 1.01 (d, *J* = 4.8 Hz, 3H). MS = 408.2 [M+H]⁺. The second eluting enantiomer of the title compound, **Compound 56:** ¹H NMR (400 MHz, DMSO-*d*₆): δ 7.83 (d, *J* = 8.8 Hz, 2H), 7.33 - 7.22 (m, 4H), 7.17 - 7.14 (m, 2H), 4.04 - 3.93 (m, 2H), 3.36 - 3.35 (m, 1H), 3.15 (s, 3H), 2.98 - 2.97 (m, 1H), 2.78 - 2.70 (m, 2H), 2.57 - 2.56 (m, 1H), 2.47 - 2.46 (m, 1H), 2.33 - 2.28 (m, 1H), 2.07 - 2.04 (m, 1H), 1.73 - 1.72 (m, 1H), 1.54 - 1.51 (m, 1H), 1.01 (d, *J= 6.0* Hz, 3H). MS = 408.2 [M+H]⁺.

### Example 17

### (2S,4R) or (2S,4S) or (2R,4S) or (2R,4R)-1-(3-chlorophenethyl)-2-methyl-4-((4-(methylsulfonyl)phenoxy)methyl)pyrrolidine (Compound 57)

### (2S,4S) or (2R,4S) or (2R,4R) or (2S,4R)-1-(3-chlorophenethyl)-2-methyl-4-((4-(methylsulfonyl)phenoxy)methyl)pyrrolidine (Compound 58)

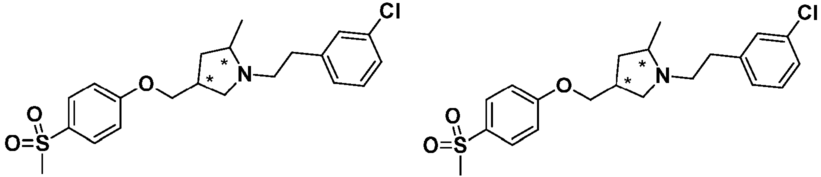

*rac-cis* or *trans-*1-(3-chlorophenethyl)-2-methyl-4-((4-(methylsulfonyl)phenoxy)methyl)pyrrolidine **(Compound 54,** 160 mg, 0.392 µmol) was separated by preparative chiral SFC (DAICEL CHIRALPAK AD-3, 33% MeOH with 0.1% NH₄OH in CO₂). The first eluting enantiomer of the title compound, **Compound 57:** ¹H NMR (400 MHz, DMSO-*d*₆): δ 7.83 (d, *J=* 8.8 Hz, 2H), 7.31 (s, 1H), 7.23 - 7.14 (m, 5H), 3.95 - 3.88 (m, 2H), 3.15 (s, 3H), 3.06 - 3.05 (m, 1H), 2.94 - 2.93 (m, 1H), 2.71 - 2.67 (m, 2H), 2.53 - 2.52 (m, 1H), 2.33 - 2.25 (m, 3H), 2.14 - 2.13 (m, 1H), 1.11 - 0.96 (m, 4H). MS = 408.2 [M+H]⁺. The second eluting enantiomer of the title compound, **Compound 58:** ¹H NMR (400 MHz, DMSO-*d*₆): δ 7.83 (d, *J* = 8.8 Hz, 2H), 7.31 (s, 1H), 7.27 - 7.14 (m, 5H), 3.95 - 3.91 (m, 2H), 3.15 (s, 3H), 3.08 - 3.05 (m, 1H), 2.98 - 2.89 (m, 1H), 2.76 - 2.67 (m, 2H), 2.53 - 2.52 (m, 1H), 2.33 - 2.28 (m, 3H), 2.2 - 2.11 (m, 1H), 1.09 - 0.99 (m, 1H), 0.96 - 0.85 (m, 3H). MS = 408.2 [M+H]⁺.

The following compounds in Table 19 were prepared according to procedures similar to those described for **Example 15** to **17** using the appropriate starting materials.

**Table 19**

| No. | Structure | IUPAC Name | Exact Mass [M+H]⁺ | Chiral purification column | Chiral Elution order |
|---|---|---|---|---|---|
| **135** | 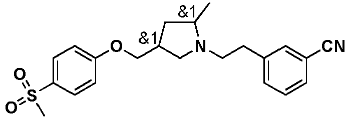 | r*ac-cis* or t*rans-*3-{2-[4-[(4-methanesulfony lphenoxy)methy l]-2-methylpyrrolidi n-1-yl]ethyl}benzon itrile | Calc'd 399.2 | Prep-HPLC Waters Xbridge BEH C18 | 1st |
| | | | Found 399.1 | | |
| **136** | 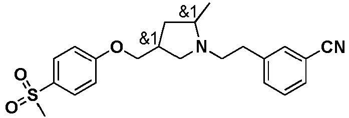 | *rac-trans* or *cis-*3-{2-[4-[(4-methanesulfony lphenoxy)methy l]-2-methylpyrrolidi n-1-yl]ethyl}benzon itrile | Calc'd 399.2 | Prep-HPLC Waters Xbridge BEH C18 | 2nd |
| | | | Found 399.1 | | |
| **137** | 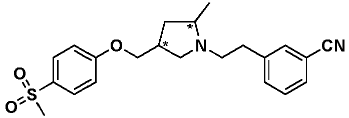 | 3-{2-[(2*S*,4*R* or 2R,4S or 2R,4R or 2*S*,4*S*)-4-[(4-methanesulfony lphenoxy)methy l]-2-methylpyrrolidi n-1-yl]ethyl}benzon itrile | Calc'd 399.2 | Chiralpak AD | 1st |
| | | | Found 399.1 | | |
| **138** | 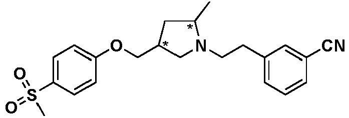 | 3-{2-[(2*R*,4*S* or 2S,4R or 2*R*,4*R* or 2*S*,4*S*)-4-[(4-methanesulfony lphenoxy)methy l]-2-methylpyrrolidi n-1-yl]ethyl}benzon itrile | Calc'd 399.2 | Chiralpak AD | 2nd |
| | | | Found 399.1 | | |
| **139** | 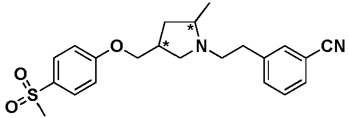 | 3-{2-[(2*S*,4*R* or 2R,4S or *2S,4S* or 2R,4R)-4-[(4-methanesulfony lphenoxy)methy l]-2-methylpyrrolidi n-1-yl]ethyl}benzon itrile | Calc'd 399.2 | Chiralpak AD | 1st |
| | | | Found 399.1 | | |
| **140** | 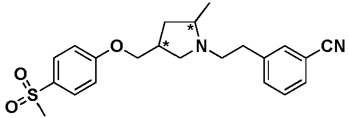 | 3-{2-[(2*S*,4*R* or 2*R*,4*S* or 2*R*,4*R* or 2*S*,4*S*)-4-[(4-methanesulfony lphenoxy)methy l]-2-methylpyrrolidi n-1-yl]ethyl}benzon itrile | Calc'd 399.2 | Chiralpak AD | 2nd |
| | | | Found 399.1 | | |
| **141** | 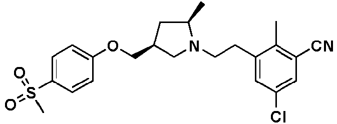 | 5-chloro-3-{2-[(2*R*,4*S*)-4-[(4-methanesulfony lphenoxy)methy l]-2-methylpyrrolidi n-1-yl]ethyl}-2-methylbenzonitr ile | Calc'd 447.1 | n/a | n/a |
| | | | Found 447.2 | | |
| **142** | 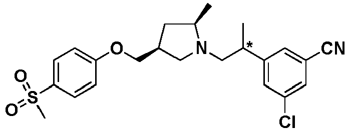 | 3-chloro-5-[(2*R* or 2*S*)-1-[(2*R*,4*S*)-4-[(4-methanesulfony lphenoxy)methy l]-2-methylpyrrolidi n-1-yl]propan-2-yl]benzonitrile | Calc'd 447.1 | prep-HPLC Waters Xbridge BEH C18 | 2nd |
| | | | Found 447.2 | | |
| **143** | 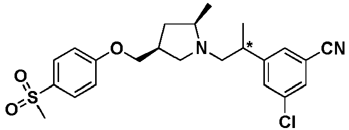 | 3-chloro-5-[(2*S* or 2*R*)-1-[(2*R*,4*S*)-4-[(4-methanesulfony lphenoxy)methy l]-2-methylpyrrolidi n-1-yl]propan-2-yl]benzonitrile | Calc'd 447.1 | prep-HPLC Waters Xbridge BEH C18 | 1st |
| | | | Found 447.2 | | |
| **144** | 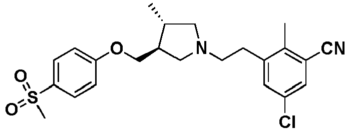 | 5-chloro-3-{2-[(3*S*,4*S*)-3-[(4-methanesulfonylphenoxy)methy l]-4-methylpyrrolidi n-1-yl]ethyl}-2-methylbenzonitr ile | Calc'd 447.1 | n/a | n/a |
| | | | Found 447.2 | | |
| **145** | 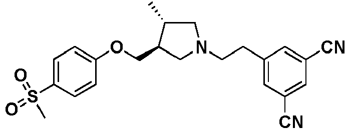 | 5-{2-[(3*S*,4*S*)-3-[(4-methanesulfony lphenoxy)methy l]-4-methylpyrrolidi n-1-yl]ethyl}benzen e-1,3-dicarbonitrile | Calc'd 424.2 | n/a | n/a |
| | | | Found 424.1 | | |
| **146** | 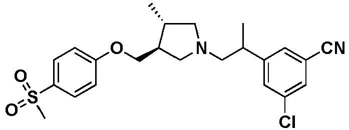 | 3-chloro-5-[1-[(3*S*,4*S*)-3-[( 4-methanesulfony lphenoxy)methy l]-4-methylpyrrolidi n-1-yl]propan-2-yl]benzonitrile | Calc'd 447.1 | n/a | n/a |
| | | | Found 447.1 | | |
| **147** | 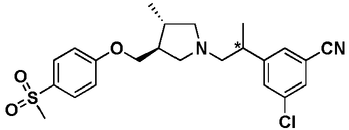 | 3-chloro-5-[(2*R* or 2*S*)-1-[(3 S,4S)-3-[(4-methanesulfony lphenoxy)methy l]-4-methylpyrrolidin-1-yl]propan-2-yl]benzonitrile | Calc'd 447.1 | Chiralpk AD | 1st |
| | | | Found 447.1 | | |
| **148** | 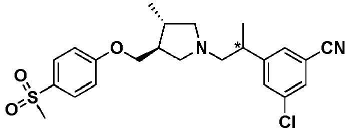 | 3-chloro-5-[(2*S* or 2*R*)-1-[(3 S,4S)-3-[(4-methanesulfony lphenoxy)methy l]-4-methylpyrrolidi n-1-yl]propan-2-yl]benzonitrile | Calc'd 447.1 | Chiralpk AD | 2nd |
| | | | Found 447.1 | | |
| **149** | 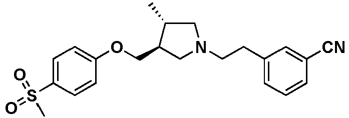 | 3-{ 2-[(3*S*,4*S*)-3-[(4-methanesulfony lphenoxy)methy l]-4-methylpyrrolidi n-1-yl]ethyl}benzon itrile | Calc'd 399.2 | n/a | n/a |
| | | | Found 399.1 | | |
| **150** | 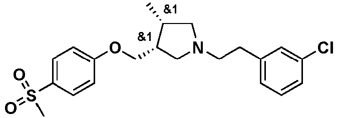 | *rac-cis-1-[2-(3-*chlorophenyl)et hyl]-3-[(4-methanesulfony lphenoxy)methy l]-4-methylpyrrolidi ne | Calc'd 408.1 | n/a | n/a |
| | | | Found 408.3 | | |
| **151** | 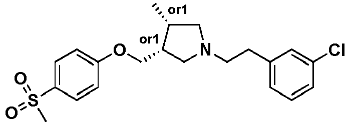 | (3*R*,4*S* or 3*S*,4*R*)-1-[2-(3-chlorophenyl)et hyl]-3-[(4-methanesulfony lphenoxy)methy l]-4-methylpyrrolidi ne | Calc'd 408.1 | Chiralcel OJ | 1st |
| | | | Found 408.3 | | |
| **152** | 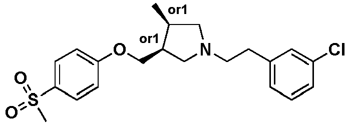 | (3*S*,4*R* or 3*R*,4*S*)-1-[2-(3-chlorophenyl)et hyl]-3-[(4-methanesulfony lphenoxy)methy l]-4-methylpyrrolidi ne | Calc'd 408.1 | Chiralcel OJ | 2nd |
| | | | Found 408.2 | | |
| **153** | 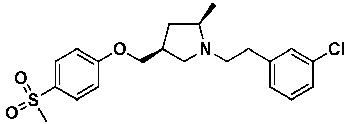 | (2*R*,4*S*)-1-[2-(3-chlorophenyl)et hyl]-4-[(4-methanesulfony lphenoxy)methy l]-2-methylpyrrolidi ne | Calc'd 408.1 | n/a | n/a |
| | | | Found 408.1 | | |
| **154** | 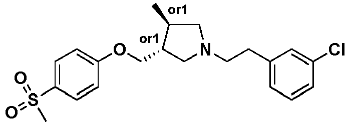 | (3*R*,4*R* or 3*S*,4*S*)-1-[2-(3-chlorophenyl)et hyl]-3-[(4-methanesulfony lphenoxy)methyl]-4-methylpyrrolidi ne | Calc'd 408.1 | Chiralcel OD | 1st |
| | | | Found 408.1 | | |
| **155** | 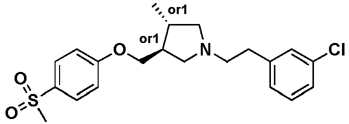 | (3*S*,4*S* or 3*R*,4*R*)-1-[2-(3-chlorophenyl)et hyl]-3-[(4-methanesulfony lphenoxy)methy l]-4-methylpyrrolidi ne | Calc'd 408.1 | Chiralcel OD | 2nd |
| | | | Found 408.1 | | |

### Example 18

### (S)-N-(4-(3-((3-chlorophenethyl)(cyclopropylmethyl)amino)-2-hydroxypropoxy)phenyl)-N-methylmethanesulfonamide (Compound 59)

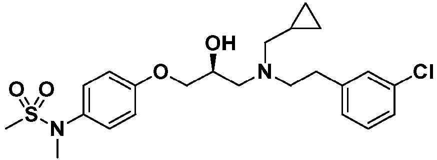

A solution of (*S*)-*N*-(4-(3-((3-chlorophenethyl)amino)-2-hydroxypropoxy)phenyl)-N-methylmethanesulfonamide **(Compound 86,** 30.0 mg, 0.073 mmol), (bromomethyl)cyclopropane (21.1 µL, 0.218 mmol), and K₂CO₃ (40 mg, 0.291 mmol) in MeCN (0.73 mL) was heated at 85 °C for 18 h. After cooling to room temperature, solids were removed via filtration, and the filtrate was concentrated in vacuo. The crude residue was purified by reverse phase preparative HPLC (Phenomenex Kinetex C18 column, 10-70% MeCN/water with 0.1% formic acid modifier) to give (*S*)-*N*-(4-(3-((3-chlorophenethyl)(cyclopropylmethyl)amino)-2-hydroxypropoxy)phenyl)-*N*-methylmethanesulfonamide **(Compound 59).** ¹H NMR (500 MHz, DMSO-*d*₆, 29/31 H): δ 7.25 - 7.13 (m, 4H), 7.10 - 7.05 (m, 2H), 6.84 - 6.74 (m, 2H), 3.86 - 3.73 (m, 2H), 3.70 - 3.67 (m, 1H), 3.08 (s, 3H), 2.79 (s, 3H), 2.77 - 2.56 (m, 4H), 2.51 - 2.40 (m, 1H), 2.35 - 2.33 (m, 2H), 0.79 - 0.68 (m, 1H), 0.39 - 0.27 (m, 2H), 0.06 - -0.07 (m, 2H). MS = 467.1 [M+H]⁺.

The following compound in Table 20 was prepared according to procedures similar to those described for **Compound 59** using the appropriate starting materials.

**Table 20**

| No. | Starting Material | Structure | IUPAC Name | Exact Mass [M+H]⁺ |
|---|---|---|---|---|
| **60** | **Compound 86** | 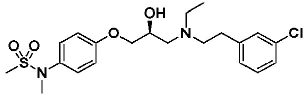 | (*S*)-*N*-(4-(3-((3- | Calc'd 441.2 |
| | | | chlorophenethyl)(ethyl)ami no)-2-hydroxypropoxy)phenyl)-N-methylmethanesulfonamide | Found 441.2 |

### Example 19

### (R)-N-(4-(3-((3-chlorophenethyl)amino)-2-methylpropoxy)phenyl)-N-methylmethanesulfonamide (Compound 61)

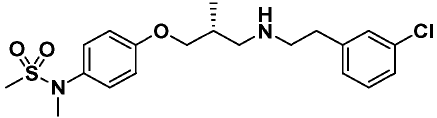

### Step 1: (S)-N-(4-(3-bromo-2-methylpropoxy)phenyl)-N-methylmethanesulfonamide

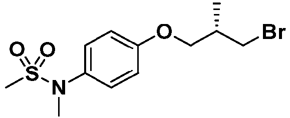

A solution of *N*-(4-hydroxyphenyl)-*N*-methylmethanesulfonamide (124 mg, 0.616 mmol), *(2S*)-3-bromo-2-methylpropan-1-ol (123 mg, 0.801 mmol), and polymer-bound PPh₃ (0.411 g, 3 mmol/g, 1.23 mmol) in toluene (2 mL) was stirred at room temperature for 20 min, and then cooled to 0 °C. Diisopropylazodioxcarboxylate (153 µL, 0.77 mmol) was added dropwise, the mixture was warmed to room temperature and then stirred for 12 h. Solids were removed by filtration and washed with DCM, and the filtrate was concentrated in vacuo. The crude residue was purified by normal phase silica gel chromatography (Biotage 10 g cartridge, 0-60% EtOAc in hexanes) to give (*S*)-*N*-(4-(3-bromo-2-methylpropoxy)phenyl)-*N-*methylmethanesulfonamide. MS = 338.9 [M+H]⁺.

### Step 2: (R)-N-(4-(3-((3-chlorophenethyl)amino)-2-methylpropoxy)phenyl)-N-methylmethanesulfonamide (Compound 61)

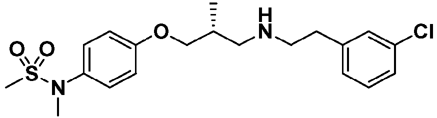

A solution of *N*-(4-((2S)-3-bromo-2-methylpropoxy)phenyl)-*N-*methylmethanesulfonamide (155 mg, 0.461 mmol), 2-(3-chlorophenyl)ethanamine (192 µL, 1.38 mmol), and K₂CO₃ (191 mg, 1.38 mmol) in MeCN was stirred at 80 °C for 18 h. After cooling to room temperature, solids were removed by filtration and washed with DCM, and the filtrate was concentrated in vacuo. The residue was taken up in DCM (3 mL) and washed with water (3 mL). The organic layer was dried over Na₂SO₄, filtered and the filtrate was concentrated in vacuo. The crude residue was purified by reverse phase preparative HPLC (Phenomenex Kinetex C18 column, 0-70% MeCN/water with 0.1% formic acid modifier). A second purification was performed by normal phase silica gel chromatography (Biotage 10 g cartridge, 0-20% MeOH in DCM) to give (R)-N-(4-(3-((3-chlorophenethyl)amino)-2-methylpropoxy)phenyl)-*N-*methylmethanesulfonamide **(Compound 61).** ¹H NMR (500 MHz, CD₃CN): δ 8.45 - 8.10 (m, 1H), 7.51 - 7.17 (m, 6H), 6.98 - 6.80 (m, 2H), 3.92 (d, *J* = 6.1 Hz, 2H), 3.24 (s, 3H), 2.98 (d, *J* = 8.8 Hz, 2H), 2.85 (s, 6H), 2.79 - 2.65 (m, 1H), 2.25 - 2.18 (m, 1H), 1.03 (dd, *J* = 6.8, 2.4 Hz, 3H). MS = 411.2 [M+H]⁺.

### Example 20

### (2S)-1-(2-(3-chlorobenzyl)piperidin-1-yl)-3-(4-(methylsulfonyl)phenoxy)propan-2-ol (Compound 62)

### (2S)-1-((S) or (R) -2-(3-chlorobenzyl)piperidin-1-yl)-3-(4-(methylsulfonyl)phenoxy)propan-2-ol (Compound 63)

### (2S)-1-((R) or (S)-2-(3-chlorobenzyl)piperidin-1-yl)-3-(4-(methylsulfonyl)phenoxy)propan-2- ol (Compound 64)

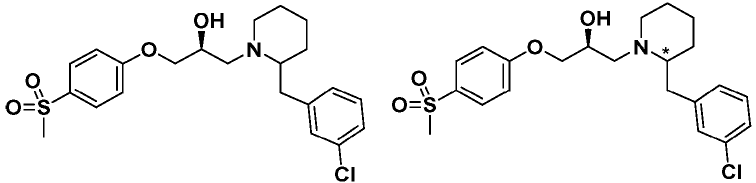

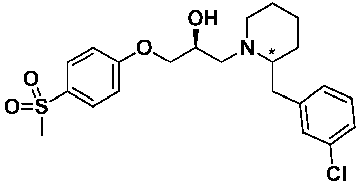

### Step 1: (2S)-1-(2-(3-chlorobenzyl)piperidin-1-yl)-3-(4-(methylsulfonyl)phenoxy)propan-2-ol (Compound 62)

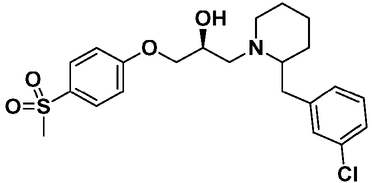

To a solution of (*S*)-2-((4-(methylsulfonyl)phenoxy)methyl)oxirane **(Intermediate C,** 50 mg, 0.22 mmol) in EtOH (1.5 mL) was added 2-[(3-chlorophenyl)methyl]piperidine (55 mg, 0.25 mmol). The mixture was heated to 80 °C and allowed to stir for 18 h. After cooling the reaction to room temperature, the mixture was concentrated in vacuo. The crude residue was purified by reverse phase preparative HPLC (Phenomenex Kinetex C18 column, 0-40% MeCN/water with 0.1% formic acid modifier) to give (2*S*)-1-(2-(3-chlorobenzyl)piperidin-1-yl)-3-(4-(methylsulfonyl)phenoxy)propan-2-ol **(Compound 62).** ¹H NMR (500 MHz, DMSO-*d*₆): δ 8.15 (s, 1H), 7.86 - 7.77 (m, 2H), 7.32 - 7.24 (m, 2H), 7.23 - 7.19 (m, 1H), 7.17 - 7.13 (m, 3H), 4.16 - 4.00 (m, 1H), 4.01 - 3.83 (m, 2H), 3.15 (s, 3H), 3.04 - 2.91 (m, 1H), 2.89 - 2.76 (m, 2H), 2.73 - 2.70 (m, 1H), 2.62 - 2.54 (m, 2H), 2.46 - 2.35 (m, 1H), 1.62 - 1.57 (m, 1H), 1.54 - 1.35 (m, 3H), 1.35 - 1.24 (m, 1H), 1.24 - 1.05 (m, 1H). MS = 437.9 [M+H]⁺.

### Step 2: (S)-1-((S) or (R) -2-(3-chlorobenzyl)piperidin-1-yl)-3-(4-(methylsulfonyl)phenoxy)propan-2-ol (Compound 63); (S)-1-((R) or (S)-2-(3-chlorobenzyl)piperidin-1-yl)-3-(4-(methylsulfonyl)phenoxy)propan-2-ol (Compound 64)

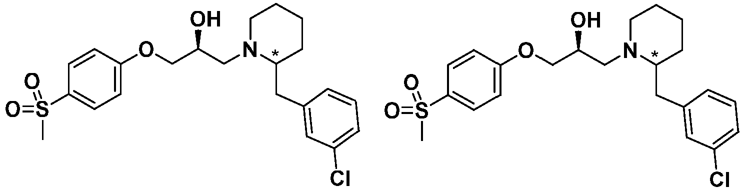

(2*S*)-1-(2-(3-chlorobenzyl)piperidin-1-yl)-3-(4-(methylsulfonyl)phenoxy)propan-2-ol was separated by preparative chiral SFC (ChromegaChiral CC5 column, 45% MeOH with 0.25% isopropylamine in CO₂). The first eluting diastereomer of the title compound, **Compound 63:** ¹H NMR (500 MHz, CD₃CN): δ 7.91 - 7.79 (m, 2H), 7.31 - 7.27 (m, 2H), 7.23 (d, *J =* 8.0 Hz, 1H), 7.18 (d, *J* = 7.6 Hz, 1H), 7.13 (d, *J =* 8.8 Hz, 2H), 4.69 (br. s, 1H), 4.11 - 4.08 (m, 1H), 4.03 - 3.98 (m, 2H), 3.04 - 3.00 (m, 4H), 2.98 - 2.87 (m, 3H), 2.72 (app dd, *J =* 13.2, 9.3 Hz, 1H), 2.67 - 2.60 (m, 2H), 1.75 - 1.65 (m, 1H), 1.62 - 1.56 (m, 3H), 1.47 - 1.41 (m, 1H), 1.41 - 1.33 (m, 1H). MS = 437.9 [M+H]⁺. The second eluting diastereomer of the title compound, **Compound 64:** ¹H NMR (500 MHz, CD₃CN): δ 7.86 (d, *J =* 8.6 Hz, 2H), 7.33 - 7.26 (m, 2H), 7.22 (d, *J* = 8.1 Hz, 1H), 7.18 (d, *J* = 7.6 Hz, 1H), 7.12 (d, *J =* 8.5 Hz, 2H), 4.69 (br s, 1H), 4.09 (app d, *J* = 7.2 Hz, 1H), 4.01 - 3.98 (m, 2H), 3.12 - 3.00 (m, 5H), 2.86 - 2.82 (m, 2H), 2.72 (app dd, *J =* 12.6, 4.7 Hz, 1H), 2.65 (app dd, *J =* 13.4, 8.8 Hz, 1H), 2.54 - 2.49 (m, 1H), 1.71 - 1.67 (m, 1H), 1.63 - 1.53 (m, 3H), 1.43 - 1.38 (m, 1H), 1.36 - 1.32 (m, 1H). MS = 438.0 [M+H].⁺

The following compounds in Table 21 were prepared according to procedures similar to those described for Compounds **63** - **64** using the appropriate starting materials.

**Table 21**

| No. | Intermediate | Structure | IUPAC Name | Exact Mass [M+H]⁺ | Chiral Column | Chiral Elution Order |
|---|---|---|---|---|---|---|
| **65** | **A** | 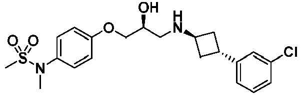 | *N*-(4-((*S*)-3-((*trans*-3-(3-chlorophenyl)cycl obutyl)amino)-2-hydroxypropoxy)p henyl)-*N-*methylmethanesulf onamide | Calc'd 439.1 | n/a | n/a |
| | | | | Found 439.1 | | |
| **66** | **C** | 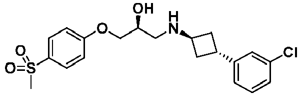 | (*S*)-1-((*trans*-3-(3-chlorophenyl)cycl obutyl)amino)-3-(4-(methylsulfonyl)ph enoxy)propan-2-ol | Calc'd 410.1 | n/a | n/a |
| | | | | Found 410.1 | | |
| **67** | **D** | 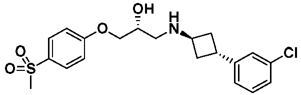 | (*R*)-1-((*trans*-3-(3-chlorophenyl)cycl obutyl)amino)-3-(4-(methylsulfonyl)ph enoxy)propan-2-ol | Calc'd 410.1 | n/a | n/a |
| | | | | Found 410.1 | | |
| **68** | **A** | 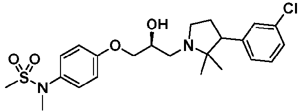 | *N*-(4-((2*S*)-3-(3-(3-chlorophenyl)-2,2-dimethylpyrrolidin -1-yl)-2-hydroxypropoxy) phenyl)-*N-*methylmethanesulfnamide | Calc'd 467.2 | n/a | n/a |
| | | | | Found 467.1 | | |
| **69** | **A** | 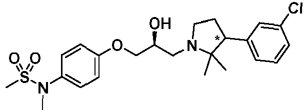 | *N*-(4-((*S*)-3-((*S*) or (*R*)-3-(3-chlorophenyl)-2,2-dimethylpyrrolidin -1-yl)-2-hydroxypropoxy) phenyl)-*N-*methylmethanesulfonamide | Calc'd 467.2 | Chiralcel OX-H | 1st |
| | | | | Found 467.1 | | |
| **70** | **A** | 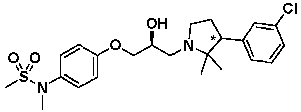 | *N*-(4-((*R*)- (*S*)-3-((*R*) or (*S*)-3-(3-chlorophenyl)-2,2-dimethylpyrrolidin -1-yl)-2-hydroxypropoxy) phenyl)-*N-*methylmethanesulfonamide | Calc'd 467.2 | Chiralcel OX-H | 2nd |
| | | | | Found 467.1 | | |
| **71** | **B** | 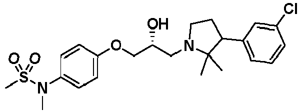 | *N*-(4-((2*R*)-3-(3-(3-chlorophenyl)-2,2-dimethylpyrrolidin -1-yl)-2-hydroxypropoxy) phenyl)-*N-*methylmethanesulfonamide | Calc'd 467.2 | n/a | n/a |
| | | | | Found 467.0 | | |
| **72** | **B** | 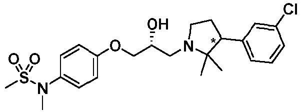 | *N*-(4-((*R*)-3-((*S*) or (*R*)-3-(3-chlorophenyl)-2,2-dimethylpyrrolidin -1-yl)-2-hydroxypropoxy) phenyl)-*N-*methylmethanesulfonamide | Calc'd 467.2 | Chiralcel OX-H | 1st |
| | | | | Found 467.1 | | |
| **73** | **B** | 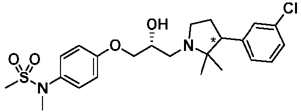 | *N*-(4-((*R*)-3-((*R*) or (*S*)-3-(3-chlorophenyl)-2,2-dimethylpyrrolidin -1-yl)-2-hydroxypropoxy) phenyl)-*N-*methylmethanesulfonamide | Calc'd 467.2 | Chiralcel OX-H | 2nd |
| | | | | Found 467.1 | | |
| **74** | **A** | 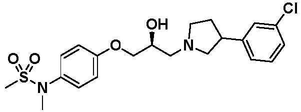 | *N*-(4-((2*S*)-3-(3-(3-chlorophenyl)pyrr olidin-1-yl)-2-hydroxypropoxy) phenyl)-*N-*methylmethanesulfonamide | Calc'd 439.1 | n/a | n/a |
| | | | | Found 439.1 | | |
| **75** | **A** | 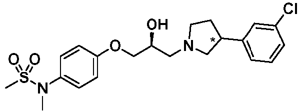 | *N*-(4-((*S*)-3-((S) or (*R*)-3-(3-chlorophenyl)pyrr olidin-1-yl)-2-hydroxypropoxy) phenyl)-*N-*methylmethanesulfonamide | Calc'd 439.1 | Chiralcel OD-H | 1st |
| | | | | Found 439.2 | | |
| **76** | **A** | 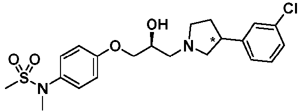 | *N*-(4-((*S*)-3-((*R*) or (*S*)-3-(3-chlorophenyl)pyrr olidin-1-yl)-2-hydroxypropoxy) phenyl)-*N-*methylmethanesulfonamide | Calc'd 439.1 | Chiralc el OD-H | 2nd |
| | | | | Found 439.1 | | |
| **77** | **B** | 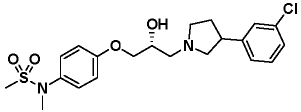 | *N*-(4-((2*R*)-3-(3-(3-chlorophenyl)pyrr olidin-1-yl)-2-hydroxypropoxy) phenyl)-*N-*methylmethanesulfonamide | Calc'd 439.1 | n/a | n/a |
| | | | | Found 438.9 | | |
| **78** | **B** | 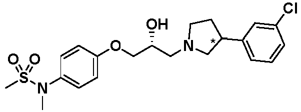 | *N*-(4-((*R*)-3-((*S*) or (*R*)-3-(3-chlorophenyl)pyrr olidin-1-yl)-2-hydroxypropoxy) phenyl)-*N-*methylmethanesulfonamide | Calc'd 439.1 | Regis-pack | 1st |
| | | | | Found 439.0 | | |
| **79** | **B** | 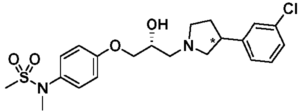 | *N*-(4-((*R*)-3-((*R*) or (*S*)-3-(3-chlorophenyl)pyrr olidin-1-yl)-2-hydroxypropoxy)p henyl)-*N-*methylmethanesulf onamide | Calc'd 439.1 | Regis-pack | 2nd |
| | | | | Found 439.0 | | |
| **80** | **A** | 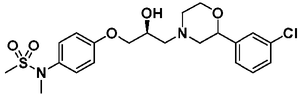 | *N*-(4-((2*S*)-3-(2-(3-chlorophenyl)morp holino)-2-hydroxypropoxy)phenyl)-*N-*methylmethanesulf onamide | Calc'd 455.1 | n/a | n/a |
| | | | | Found 455.1 | | |
| **81** | **A** | 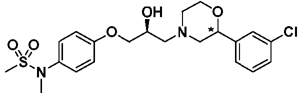 | *N*-(4-((*S*)-3-((*S*) or (R)-2-(3-chlorophenyl)morp holino)-2-hydroxypropoxy) phenyl)-*N-*methylmethanesulf onamide | Calc'd 455.1 | Chrome gaChiral CCA | 1st |
| | | | | Found 454.9 | | |
| **82** | **A** | 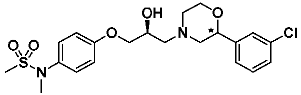 | *N*-(4-((*S*)-3-((*R*) or (*S*)-2-(3-chlorophenyl)morp holino)-2-hydroxypropoxy) phenyl)-N-methylmethanesulf onamide | Calc'd 455.1 | Chrome gaChiral CCA | 2nd |
| | | | | Found 454.9 | | |
| **83** | **B** | 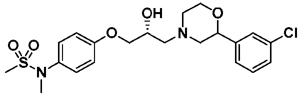 | *N*-(4-((2*R*)-3-(2-(3-chlorophenyl)morp holino)-2-hydroxypropoxy)p henyl)-*N-*methylmethanesulf onamide | Calc'd 455.1 | n/a | n/a |
| | | | | Found 455.0 | | |
| **84** | **B** | 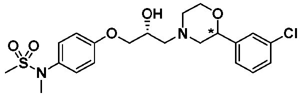 | *N*-(4-((*R*)-3-((*R*) or (*S*)-2-(3-chlorophenyl) morpholino)-2-hydroxypropoxy)phenyl)-*N-*methylmethanesulf onamide | Calc'd 455.1 | Chrome gaChiral CCA | 1st |
| | | | | Found 455.1 | | |
| **85** | **B** | 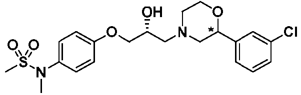 | *N*-(4-((*R*)-3-((*S*) or (*R*)-2-(3-chlorophenyl)morp holino)-2-hydroxypropoxy)p henyl)-*N-*methylmethanesulf onamide | Calc'd 455.1 | Chrome gaChiral CCA | 2nd |
| | | | | Found 455.1 | | |
| **86** | **A** | 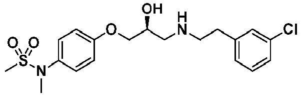 | (*S*)-*N*-(4-(3-((3-chlorophenethyl)a mino)-2-hydroxypropoxy)p henyl)-*N-*methylmethanesulf onamide | Calc'd 413.1 | n/a | n/a |
| | | | | Found 413.0 | | |
| **87** | **B** | 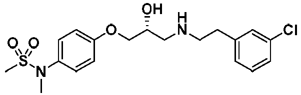 | (*R*)-*N*-(4-(3-((3-chlorophenethyl)a mino)-2-hydroxypropoxy)p henyl)-*N-*methylmethanesulf onamide | Calc'd 413.1 | n/a | n/a |
| | | | | Found 413.0 | | |
| **88** | **B** | 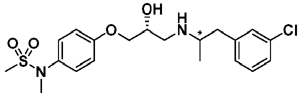 | *N*-(4-((*R*)-3-(((*S*) or (*R*)-1-(3-chlorophenyl)prop an-2-yl)amino)-2-hydroxypropoxy)p henyl)-*N*-methylmethanesulf onamide | Calc'd 427.1 | Chiralcel OX-H | 2nd |
| | | | | Found 427.1 | | |
| **89** | **B** | 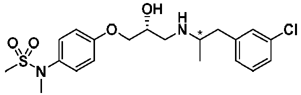 | *N*-(4-((*R*)-3-(((*R*) or (*S*)-1-(3-chlorophenyl)prop an-2-yl)amino)-2-hydroxypropoxy)p henyl)-*N-*methylmethanesulf onamide | Calc'd 427.1 | Chiralcel OX-H | 1st |
| | | | | Found 427.1 | | |
| **90** | **A** | 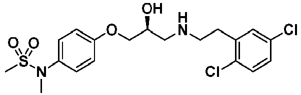 | (*S*)-*N*-(4-(3-((2,5-dichlorophenethyl) amino)-2-hydroxypropoxy)p henyl)-*N-*methylmethanesulf onamide | Calc'd 447.1 | n/a | n/a |
| | | | | Found 447.0 | | |
| **91** | **A** | 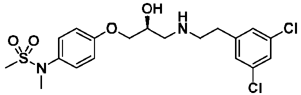 | (*S*)-*N*-(4-(3-((3,5-dichlorophenethyl) amino)-2-hydroxypropoxy)p henyl)-*N-*methylmethanesulf onamide | Calc'd 447.1 | n/a | n/a |
| | | | | Found 447.0 | | |
| **92** | **B** | 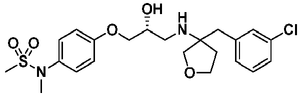 | *N*-(4-((2*R*)-3-((3-(3-chlorobenzyl)tetra hydrofuran-3-yl)amino)-2-hydroxypropoxy)p henyl)-*N*-methylmethanesulf onamide | Calc'd 469.2 | n/a | n/a |
| | | | | Found 469.1 | | |
| **93** | **C** | 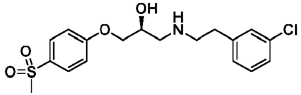 | (*S*)-1-((3-chlorophenethyl)a mino)-3-(4-(methylsulfonyl)ph enoxy)propan-2-ol | Calc'd 384.1 | n/a | n/a |
| | | | | Found 384.1 | | |
| **94** | **D** | 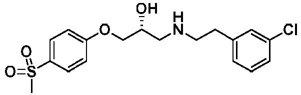 | (*R*)-1-((3-chlorophenethyl)a mino)-3-(4-(methylsulfonyl)ph enoxy)propan-2-ol | Calc'd 384.1 | n/a | n/a |
| | | | | Found 384.1 | | |
| **95** | **A** | 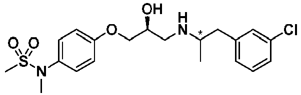 | *N*-(4-((*S*)-3-(((*S*) or (*R*)-1-(3-chlorophenyl)prop an-2-yl)amino)-2-hydroxypropoxy)p henyl)-*N-*methylmethanesulf onamide | Calc'd 427.1 | Chrome gaChira 1 CCA | 2nd |
| | | | | Found 427.1 | | |
| **96** | **A** | 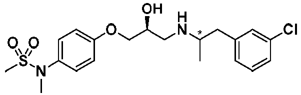 | *N*-(4-((*S*)-3-(((*R*) or (*S*)-1-(3-chlorophenyl)prop an-2-yl)amino)-2-hydroxypropoxy)p henyl)-*N-*methylmethanesulf onamide | Calc'd 427.1 | Chrome gaChira 1 CCA | 1st |
| | | | | Found 427.1 | | |
| **97** | **D** | 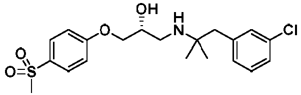 | (*R*)-1-((1-(3-chlorophenyl)-2-methylpropan-2-yl)amino)-3-(4-(methylsulfonyl)ph enoxy)propan-2-ol | Calc'd 412.1 | n/a | n/a |
| | | | | Found 412.0 | | |
| **98** | **A** | 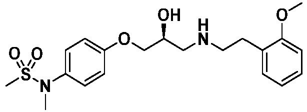 | (*S*)-*N*-(4-(2-hydroxy-3-((2-methoxyphenethyl )amino)propoxy)p henyl)-*N-*methylmethanesulf onamide | Calc'd 409.2 | n/a | n/a |
| | | | | Found 408.9 | | |
| **99** | **A** | 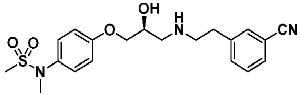 | (*S*)-*N*-(4-(3-((3-cyanophenethyl)a mino)-2-hydroxypropoxy)p henyl)-*N-*methylmethanesulf onamide | Calc'd 404.2 | n/a | n/a |
| | | | | Found 404.3 | | |
| **100** | **A** | 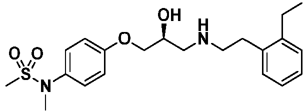 | (*S*)-*N*-(4-(3-((2-ethylphenethyl)am ino)-2-hydroxypropoxy)p henyl)-*N-*methylmethanesulf onamide | Calc'd 407.2 | n/a | n/a |
| | | | | Found 407.2 | | |
| **101** | **A** | 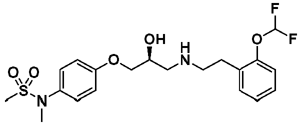 | (*S*)-*N*-(4-(3-((2-(difluoromethoxy) phenethyl)amino)-2-hydroxypropoxy) phenyl)-*N-*methylmethanesulf onamide | Calc'd 445.2 | n/a | n/a |
| | | | | Found 445.1 | | |
| **102** | **D** | 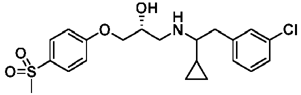 | (2*R*)-1-((2-(3-chlorophenyl)-1-cyclopropylethyl) amino)-3-(4-(methylsulfonyl) phenoxy)propan-2-ol | Calc'd 424.1 | n/a | n/a |
| | | | | Found 424.1 | | |
| **103** | **E** | 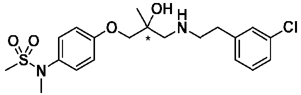 | (*R*) or (*S*)-*N*-(4-(3-((3-chlorophenethyl)a mino)-2-hydroxy-2-methylpropoxy)ph enyl)-*N-*methylmethanesulf onamide | Calc'd 427.1 | Pheno menex Cellulose-2 | 1st |
| | | | | Found 427.1 | | |
| **104** | **E** | 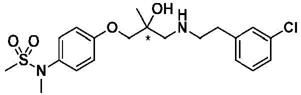 | (*S*) or (*R*)-*N*-(4-(3-((3-chlorophenethyl)a mino)-2-hydroxy-2-methylpropoxy) phenyl)-*N-*methylmethanesulf onamide | Calc'd 427.1 | Pheno menex Cellulo se-2 | 2nd |
| | | | | Found 427.1 | | |
| **105** | **F** | 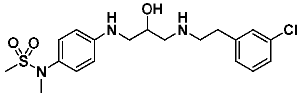 | *N-(4-((3-((3-*chlorophenethyl) amino)-2-hydroxypropyl)am ino)phenyl)-*N-*methylmethanesulf onamide | Calc'd 412.1 | n/a | n/a |
| | | | | Found 412.1 | | |
| **106** | **F** | 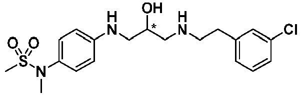 | (*R*) or (*S*)-*N*-(4-((3-((3-chlorophenethyl) amino)-2-hydroxypropyl) amino)phenyl)-*N-*methylmethanesulfonamide | Calc'd 412.1 | Chiralp ak IG-3 | 2nd |
| | | | | Found 412.1 | | |

### Example 21

### 3-{2-[(2R,4S)-4-{[4-(azetidine-3-sulfonyl)phenoxy]methyl}-2-methylpyrrolidin-1-yl]ethyl}benzonitrile (Compound 156)

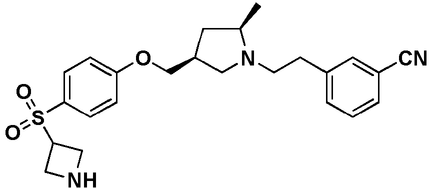

### Step 1: (2R,4S)-tert-butyl 4-((4-((1-((allyloxy)carbonyl)azetidin-3-yl)sulfonyl)phenoxy)methyl)-2-methylpyrrolidine-1-carboxylate

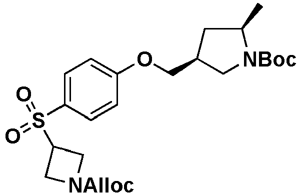

To a solution of allyl 3-((4-hydroxyphenyl)sulfonyl)azetidine-1-carboxylate **(Intermediate AB,** 400 mg, 1.35 mmol) in DMF (4 mL) were added K₂CO₃ (371.86 mg, 2.69 mmol) and *tert*-butyl (2*R*,4*S*)-2-methyl-4-(methylsulfonyloxymethyl)pyrrolidine-1-carboxylate **(Intermediate AF,** 434 mg, 1.48 mmol). The mixture was stirred at 80 °C for 16 h. The reaction mixture was partitioned between water (15 mL) and EtOAc (10 mL). The organic phase was separated, washed with brine (10 mL), dried over Na₂SO₄, filtered and concentrated under reduced pressure to give (2*R*,4*S*)-*tert*-butyl 4-((4-((1-((allyloxy)carbonyl)azetidin-3-yl)sulfonyl)phenoxy)methyl)-2-methylpyrrolidine-1-carboxylate. MS = 395.1 [M- C₅H₉O₂+H]⁺

### Step 2: allyl 3-((4-(((3S,SR)-5-methylpyrrolidin-3-yl)methoxy)phenyl)sulfonyl)azetidine-1-carboxylate

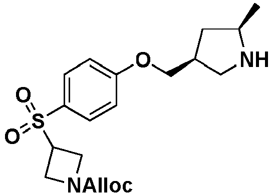

To a solution of (2*R*,4*S*)-*tert*-butyl 4-((4-((1-((allyloxy)carbonyl)azetidin-3-yl)sulfonyl)phenoxy)methyl)-2-methylpyrrolidine-1-carboxylate (730 mg, 1.48 mmol) in MeOH (2 mL) was added HCl/MeOH (4 M, 10 mL). The mixture was stirred at room temperature for 2 h. MeOH was removed under reduced pressure to give allyl 3-((4-(((3S,5R)-5-methylpyrrolidin-3-yl)methoxy)phenyl)sulfonyl)azetidine-1-carboxylate HCl salt. MS =395.2 [M+H]⁺.

### Step 3: allyl 3-[4-[[(3S,5R)-1-[2-(3-cyanophenyl)ethyl]-5-methyl-pyrrolidin-3-yl]methoxy]phenyl]sulfonylazetidine-1-carboxylate

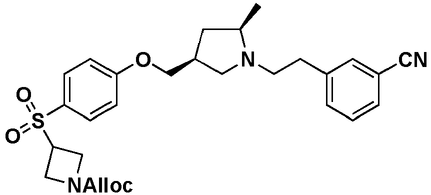

To a mixture of allyl 3-[4-[[(3*S*,5*R*)-5-methylpyrrolidin-3-yl]methoxy]phenyl]sulfonylazetidine-1-carboxylate (120 mg, 278 µmol, HCl salt), TEA (28.2 mg, 278 µmol) and 3-(2-oxoethyl)benzonitrile (52.6 mg, 362 µmol) in MeOH (5 mL) was added AcOH (16.7 mg, 278 µmol) followed by NaBH₃CN (35.0 mg, 557 µmol). The mixture was stirred at room temperature for 16 h. The reaction mixture was quenched by addition of water (5 mL) and adjusted to pH = 7 with saturated aq NaHCO₃. The aqueous phase was extracted with EtOAc (10 mL x 3). The combined organic layers were washed with brine (10 mL), dried over Na₂SO₄, filtered and concentrated under reduced pressure to give a residue. The residue was purified by flash silica gel chromatography (ISCO 4 g cartridge, 0-100% EtOAc:Hexane) to give allyl 3-[4-[[(3S,5R)-1-[2-(3-cyanophenyl)ethyl]-5-methyl-pyrrolidin-3-yl]methoxy]phenyl]sulfonylazetidine-1-carboxylate. MS = 524.3 [M+H]⁺.

### Step 4: 3-{2-[(2R,4S)-4-{[4-(azetidine-3-sulfonyl)phenoxy]methyl}-2-methylpyrrolidin-1-yl]ethyl}benzonitrile (Compound 156)

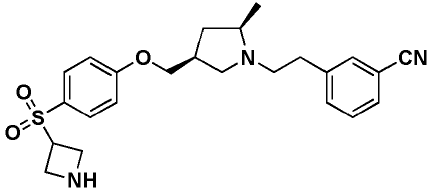

To a solution of allyl 3-[4-[[(3*S*,5*R*)-1-[2-(3-cyanophenyl)ethyl]-5-methylpyrrolidin-3-yl]methoxy]phenyl]sulfonylazetidine-1-carboxylate (60.0 mg, 115 µmol) in THF (5 mL) were added morpholine (39.9 mg, 458 µmol) and Pd(PPh₃)₄ (26.5 mg, 22.9 µmol). The mixture was then stirred at room temperature for 3 h. The reaction mixture was filtered and concentrated under reduced pressure to give a residue. The residue was purified by flash silica gel chromatography (ISCO 4 g cartridge, 0-100% EtOAc:Hexane, then 0-20% MeOH:EtOAc) to give a crude product. The product was further purified by preparative reverse phase HPLC (Waters Xbridge BEH C18 column, 25-55% MeCN:10 mM NH₄HCO₃ in H₂O) to give 3-{2-[(2*R*,4*S*)-4-{[4-(azetidine-3-sulfonyl)phenoxy]methyl}-2-methylpyrrolidin-1-yl]ethyl}benzonitrile **(Compound 156).** ¹H NMR (400 MHz, DMSO-*d*₆): δ 7.78 (d, *J* = 8.8 Hz, 2H), 7.72 (s, 1H), 7.64 - 7.57 (m, 2H), 7.42 (t, *J =* 7.6 Hz, 1H), 7.16 (d, *J =* 8.8 Hz, 2H), 4.47 - 7.39 (m, 1H), 3.90 (d, *J* = 7.2 Hz, 2H), 3.72 (t, *J =* 8.0 Hz, 2H), 3.49 (t, *J =* 8.4 Hz, 2H), 3.07 (d, *J* = 8.0 Hz, 1H), 2.99 - 2.96 (m, 1H), 2.81 - 2.75 (m, 2H), 2.35 - 2.30 (m, 5H), 2.28 - 2.12 (m, 1H), 1.07 - 1.02 (m, 1H), 0.98 (d, *J =* 6.0 Hz, 1H). MS = 440.1 [M+H]⁺.

The following compounds in Table 22 were prepared according to procedures similar to those described for **Compound 156** using the appropriate starting materials.

**Table 22**

| No. | Structure | IUPAC Name | Exact Mass [M+H]⁺ | Chiral column | Chiral Elution order |
|---|---|---|---|---|---|
| **157** | 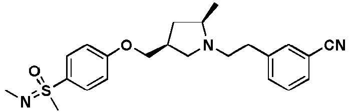 | 3-{2-[(2*R*,4*S*)-2-methyl-4-({4-[methyl(methyli mino)oxo-λ⁶-sulfanyl]phenoxy }methyl)pyrrolidi n-1-yl]ethyl }benzonit rile | Calc'd 412.2 | n/a | n/a |
| | | | Found 412.3 | | |
| **158** | 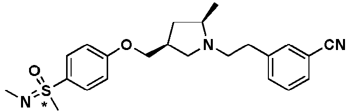 | 3-{2-[(2*R*,4*S*)-2-methyl-4-({4-[(*R* or *S*)methyl(methyli mino)oxo-λ⁶-sulfanyl]phenoxy }methyl)pyrrolidi n-1-yl]ethyl }benzonit rile | Calc'd 412.2 | Chiralpak AD | 1st |
| | | | Found 412.3 | | |
| **159** | 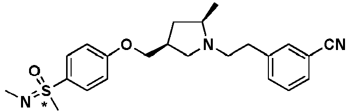 | 3-{2-[(2*R*,4*S*)-2-methyl-4-({4-[(*S* or *R*)methyl(methyli mino)oxo-λ⁶-sulfanyl]phenoxy }methyl)pyrrolidi n-1-yl]ethyl}benzonit rile | Calc'd 412.2 | Chiralpak AD | 2nd |
| | | | Found 412.3 | | |
| **161** | 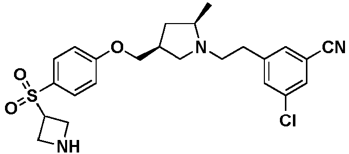 | 3-{2-[(2*R*,4*S*)-4-{[4-(azetidine-3-sulfonyl)phenoxy ]methyl}-2-methylpyrrolidin-1-yl]ethyl}-5-chlorobenzonitril e | Calc'd 474.2 | n/a | n/a |
| | | | Found 474.2 | | |
| **162** | 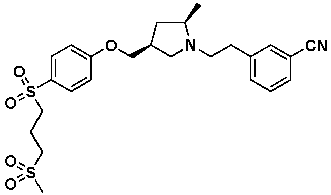 | 3-{2-[(2*R*,4*S*)-4-{[4-(3-methanesulfonylp ropanesulfonyl)p henoxy]methyl}-2-methylpyrrolidin-1-yl]ethyl }benzonit rile | Calc'd 505.2 | n/a | n/a |
| | | | Found 505.3 | | |
| **164** | 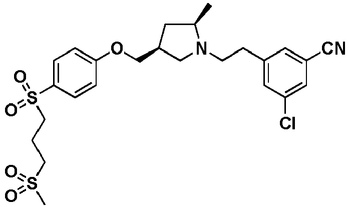 | 3-chloro-5-{2-[(2*R*,4*S*)-4-{[4-(3-methanesulfonylp ropanesulfonyl)p henoxy]methyl}-2-methylpyrrolidin-1-yl]ethyl }benzonit rile | Calc'd 539.1 | n/a | n/a |
| | | | Found 539.2 | | |
| **165** | 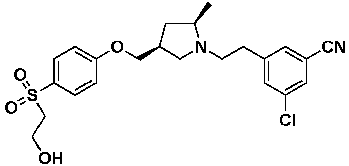 | 3-chloro-5-{2-[(2*R*,4*S*)-4-{[4-(2-hydroxyethanesul fonyl)phenoxy]m ethyl }-2-methylpyrrolidin-1-yl]ethyl }benzonit rile | Calc'd 463.1 | n/a | n/a |
| | | | Found 463.2 | | |
| **166** | 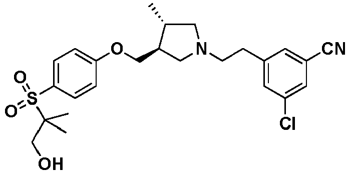 | 3-chloro-5-{2-[(3*S*,4*S*)-3-{[4-(1-hydroxy-2-methylpropane-2-sulfonyl)phenoxy ]methyl}-4-methylpyrrolidin-1-yl]ethyl }benzonit rile | Calc'd 491.2 | n/a | n/a |
| | | | Found 491.1 | | |
| **167** | 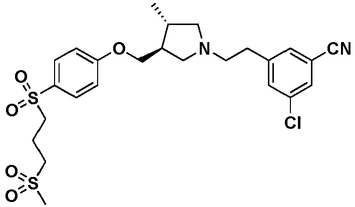 | 3-chloro-5-{2-[(3*S*,4*S*)-3-{[4-(3-methanesulfonylp ropanesulfonyl)p henoxy]methyl}-4-methylpyrrolidin-1-yl]ethyl }benzonit rile | Calc'd 539.1 | n/a | n/a |
| | | | Found 539.2 | | |
| **168** | 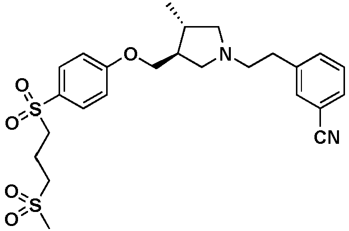 | 3-{2-[(3*S*,4*S*)-3-{[4-(3-methanesulfonylp ropanesulfonyl)p henoxy]methyl}-4-methylpyrrolidin-1-yl]ethyl }benzonit rile | Calc'd 505.2 | n/a | n/a |
| | | | Found 505.5 | | |
| **169** | 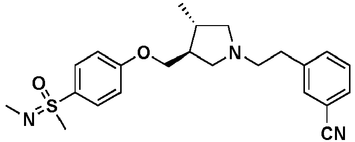 | 3-{2-[(3*S*,4*S*)-3-methyl-4-({4-[methyl(methyli mino)oxo-λ⁶-sulfanyl]phenoxy }methyl)pyrrolidi n-1-yl]ethyl }benzonit rile | Calc'd 412.2 | n/a | n/a |
| | | | Found 412.3 | | |
| **170** | 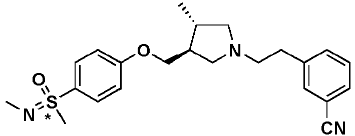 | 3-{2-[(3*S*,4*S*)-3-methyl-4-({4-[(*R* or *S*)-methyl(methylim ino)oxo-λ⁶-sulfanyl]phenoxy }methyl)pyrrolidi n-1-yl]ethyl }benzonit rile | Calc'd 412.2 | Chiralpak IC | 1st |
| | | | Found 412.3 | | |
| **171** | 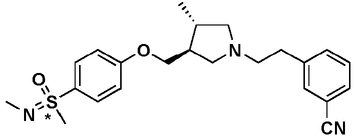 | 3-{2-[(3*S*,4*S*)-3-methyl-4-({4-[(*S* or *R*)-methyl(methylim ino)oxo-λ⁶-sulfanyl]phenoxy }methyl)pyrrolidi n-1-yl]ethyl }benzonit rile | Calc'd 412.2 | Chiralpak IC | 2nd |
| | | | Found 412.2 | | |
| **172** | 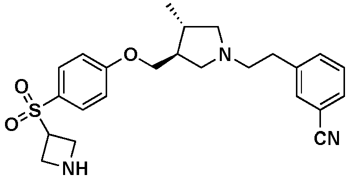 | 3-{2-[(3*S*,4*S*)-3-{[4-(azetidine-3-sulfonyl)phenoxy ]methyl}-4-methylpyrrolidin-1-yl]ethyl }benzonit rile | Calc'd 440.2 | n/a | n/a |
| | | | Found 440.1 | | |
| **173** | 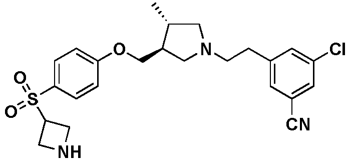 | 3-{2-[(3*S*,4*S*)-3-{[4-(azetidine-3-sulfonyl)phenoxy ]methyl}-4-methylpyrrolidin-1-yl]ethyl}-5-chlorobenzonitril e | Calc'd 474.2 | n/a | n/a |
| | | | Found 474.2 | | |
| **174** | 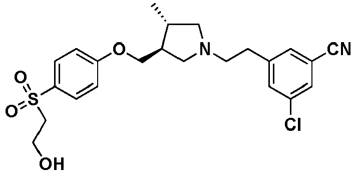 | 3-chloro-5-{2-[(3*S*,4*S*)-3-{[4-(2-hydroxyethanesul fonyl)phenoxy]methyl }-4-methylpyrrolidin-1-yl]ethyl }benzonit rile | Calc'd 463.1 | n/a | n/a |
| | | | Found 463.1 | | |
| **175** | 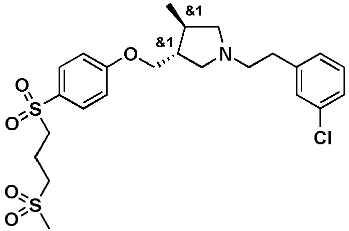 | *rac*-trans-1-[2-(3-chlorophenyl)eth yl]-3-{[4-(3-methanesulfonylp ropanesulfonyl)p henoxy]methyl}-4-methylpyrrolidine | Calc'd 514.1 | n/a | n/a |
| | | | Found 514.1 | | |
| **176** | 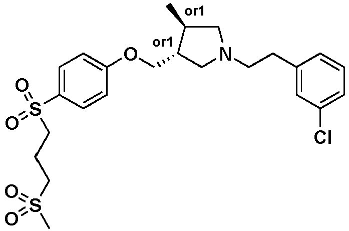 | (3*R*,4*R* or 3*S*,4*S*)-1-[2-(3-chlorophenyl)eth yl]-3-{[4-(3-methanesulfonylp ropanesulfonyl)p henoxy]methyl}-4-methylpyrrolidine | Calc'd 514.1 | Chiralpak AD | 1st |
| | | | Found 514.2 | | |
| **177** | 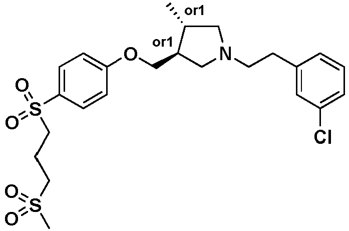 | (3*S*,4*S* or 3*R*,4*R*)-1-[2-(3-chlorophenyl)eth yl]-3-{[4-(3-methanesulfonylp ropanesulfonyl)p henoxy]methyl}-4-methylpyrrolidine | Calc'd 514.1 | Chiralpak AD | 2nd |
| | | | Found 514.2 | | |

### Example 22

### (3S)-1-[2-(3-chlorophenyl)ethyl]-3-{[4-(3-methanesulfonylpropanesulfonyl)phenoxy]methyl}piperazine (Compound 184)

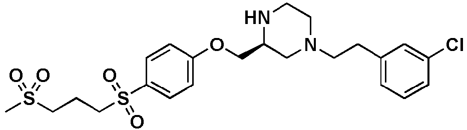

### Step 1: (S)-tert-butyl 4-(3-chlorophenethyl)-2-(hydroxymethyl)piperazine-1-carboxylate

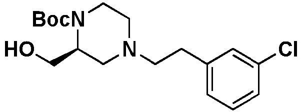

To a mixture of *tert*-butyl (2*S*)-2-(hydroxymethyl)piperazine-1-carboxylate (3.0 g, 13.9 mmol) and 2-(3-chlorophenyl)acetaldehyde (2.79 g, 18.0 mmol) in MeOH (30 mL) and AcOH (1.5 mL) was added 2-methylpyridine borane complex (1.78 g, 16.6 mmol). The mixture was stirred at 40 °C for 2 h. The reaction mixture was concentrated under reduced pressure. The residue was diluted with water (50 mL), adjusted to pH=7 with 2 M aqueous NaHCO₃ and extracted with EtOAc (50 mL x 3). The combined organic layers were washed with brine (50 mL), dried over Na₂SO₄, filtered and concentrated under reduced pressure to give a residue. The residue was purified by flash silica gel chromatography (Biotage 40 g cartridge, 0-100% EtOAc:Hexane) to give (*S*)-*tert*-butyl 4-(3-chlorophenethyl)-2-(hydroxymethyl)piperazine-1-carboxylate. MS = 355.3 [M+H]⁺.

### Step 2: (S)-(4-(3-chlorophenethyl)piperazin-2-yl)methanol

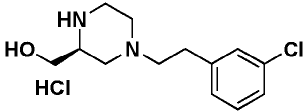

A solution of (*S*)-*tert*-butyl 4-(3-chlorophenethyl)-2-(hydroxymethyl)piperazine-1-carboxylate (3.50 g, 9.86 mmol) in HCl/MeOH (4 M, 20 mL) was stirred at room temperature for 2 h. The reaction mixture was concentrated under reduced pressure to give (*S*)-(4-(3-chlorophenethyl)piperazin-2-yl)methanol HCl salt, which was used without further purification. MS = 255.1 [M+H]⁺.

### Step 3: (3aS)-5-(3-chlorophenethyl)hexahydro-[1,2,3]oxathiazolo[3,4-a]pyrazine 1-oxide

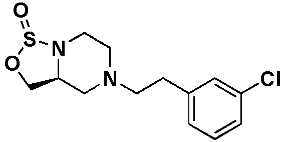

To a solution of (*S*)-(4-(3-chlorophenethyl)piperazin-2-yl)methanol (2.50 g, 8.58 mmol, HCl salt) in DCM (30 mL) were added imidazole (1.75 g, 25.7 mmol) and TEA (2.17 g, 21.5 mmol). The mixture was cooled to 0 °C, and SOCl₂ (1.53 g, 12.9 mmol) was added. The mixture was stirred at room temperature for 16 h. The reaction mixture was quenched by addition of water (30 mL) and extracted with DCM (30 mL x 3). The combined organic layers were washed with brine (30 mL), dried over Na₂SO₄, filtered and concentrated under reduced pressure to give a residue. The residue was purified by flash silica gel chromatography (Biotage 40 g cartridge, 0-100% EtOAc:Hexane) to give (3*aS*)-5-(3-chlorophenethyl)hexahydro-[1,2,3]oxathiazolo[3,4-a]pyrazine 1-oxide. MS= 301.0 [M+H]⁺.

### Step 4: (S)-5-(3-chlorophenethyl)hexahydro-[1,2,3]oxathiazolo[3,4-a]pyrazine 1,1-dioxide

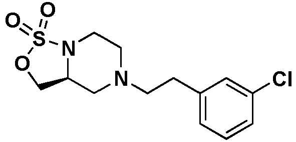

To a solution of (3*aS*)-5-(3-chlorophenethyl)hexahydro-[1,2,3]oxathiazolo[3,4-a]pyrazine 1-oxide (1.80 g, 5.98 mmol) in MeCN (40 mL) were added RuCl₃ (13 mg, 59.8 µmol) and a solution of NaIO₄ (1.92 g, 8.98 mmol) in water (10 mL) under N₂. The mixture was stirred at room temperature for 2 h. The reaction mixture was quenched by saturated aqueous Na₂SO₃ (100 mL), then was diluted with water (40 mL) and extracted with EtOAc (30 mL x 3). The combined organic layers were washed with brine (50 mL), dried over Na₂SO₄, filtered and concentrated under reduced pressure to give a residue. The residue was purified by flash silica gel chromatography (Biotage 20 g cartridge, 0-100% EtOAc:Hexane) to give (*S*)-5-(3-chlorophenethyl)hexahydro-[1,2,3]oxathiazolo[3,4-a]pyrazine 1,1-dioxide. MS = 317.0 [M+H]⁺.

### Step 5: (3S)-1-[2-(3-chlorophenyl)ethyl]-3-{[4-(3-methanesulfonylpropanesulfonyl)phenoxy]methyl}piperazine (Compound 184)

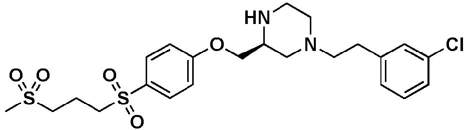

To a mixture of 4-(3-methanesulfonylpropanesulfonyl)phenol (Intermediate AD, 48 mg, 174 µmol) and (*S*)-5-(3-chlorophenethyl)hexahydro-[1,2,3]oxathiazolo[3,4-a]pyrazine 1,1-dioxide (50 mg, 158 µmol) in DMF (3 mL) was added K₂CO₃ (33 mg, 237 µmol). The reaction was stirred at 60 °C for 16 h. The reaction mixture was filtered and the filtrate was quenched with 3.0 M aqueous HCl (106 µL). The mixture was stirred for 2 h, solids were removed by filtration, and the filtrate was concentrated in vacuo. The residue was purified by preparative reverse phase HPLC (Phenomenex Gemini-NX, 18-48% MeCN:10 mM NH₄HCO₃ in H₂O) to give (3*S*)-1-[2-(3-chlorophenyl)ethyl]-3-{[4-(3-methanesulfonylpropanesulfonyl)phenoxy]methyl}piperazine **(Compound 184).** ¹H NMR (400 MHz, DMSO-*d*₆): δ = 7.81 (d, *J =* 8.8 Hz, 2H), 7.32-7.31 (m, 1H), 7.29-7.27 (m, 1H), 7.24-7.18 (m, 4H), 4.02-3.97 (m, 2H), 3.42-3.38 (m, 3H), 3.20 (t, *J* = 8.0 Hz, 2H), 3.02-3.00 (m, 1H), 2.96 (s, 3H), 2.87 (t, *J =* 9.2 Hz, 2H), 2.76-2.66 (m, 5H), 2.06-1.87 (m, 5H). MS = 515.1 [M+H]⁺.

The following compounds in Table 23 were prepared according to procedures similar to those described for **Compound 184** using the appropriate starting materials.

**Table 23**

| No. | Structure | IUPAC Name | Exact Mass [M+H]⁺ | Chiral column | Chiral eluting order |
|---|---|---|---|---|---|
| **182** | 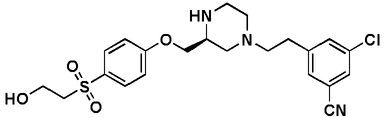 | 3-chloro-5-{2-[(3*S*)-3-{[4-(2-hydroxyethane sulfonyl)phen oxy]methyl}pi perazin-1-yl]ethyl }benzo nitrile | Calc'd 464.1 | n/a | n/a |
| | | | Found 464.1 | | |
| **183** | 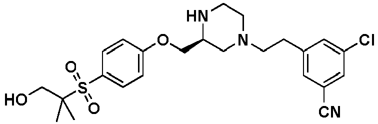 | 3-chloro-5-{2-[(3*S*)-3-{[4-(1-hydroxy-2-methylpropane -2-sulfonyl)phen oxy]methyl}pi perazin-1-yl]ethyl }benzo nitrile | Calc'd 492.2 | n/a | n/a |
| | | | Found 492.1 | | |
| **185** | 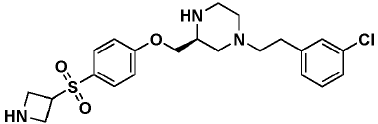 | (3*S*)-3-{[4-(azetidine-3-sulfonyl)phen oxy]methyl}-1-[2-(3-chlorophenyl) ethyl]piperazi ne | Calc'd 450.2 | n/a | n/a |
| | | | Found 450.1 | | |
| **186** | 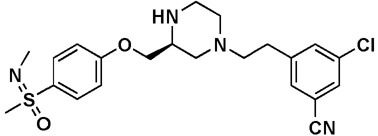 | 3-chloro-5-{2-[(3*S*)-3-({4-[methyl(methy limino)oxo-λ⁶-sulfanyl]pheno xy}methyl)pip erazin-1-yl]ethyl }benzo nitrile | Calc'd 447.2 | n/a | n/a |
| | | | Found 447.2 | | |
| **187** | 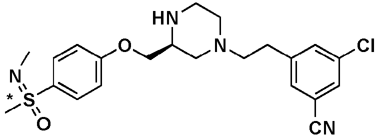 | 3-chloro-5-{2-[(3*S*)-3-({4-[(*S* or *R*)-methyl(methyl imino)oxo-λ⁶-sulfanyl]pheno xy}methyl)pip erazin-1-yl]ethyl }benzo nitrile | Calc'd 447.2 | Chiralpak IC | 1st |
| | | | Found 447.2 | | |
| **188** | 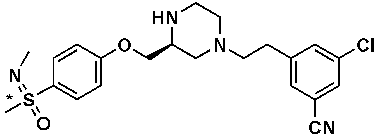 | 3-chloro-5-{2-[(3S)-3-({4-[(*R* or *S*)-methyl(methyl imino)oxo-λ⁶-sulfanyl]pheno xy}methyl)pip erazin-1-yl]ethyl }benzo nitrile | Calc'd 447.2 | Chiralpak IC | 2nd |
| | | | Found 447.1 | | |

### Example 23

### 3-chloro-5-{2-[(3S)-3-[(4-methanesulfonylphenoxy)methyl]piperazin-1-yl]ethyl}benzonitrile (Compound 189)

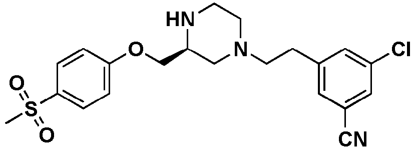

### Step 1: (S)-4-benzyl 1-tert-butyl 2-(hydroxymethyl)piperazine-1,4-dicarboxylate

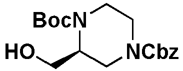

To a mixture of (*S*)-*tert*-butyl 2-(hydroxymethyl)piperazine-1-carboxylate (4.00 g, 18.5 mmol) and NaHCO₃ (4.66 g, 55.5 mmol) in THF (25 mL) and H₂O (25 mL) was added CbzCl (4.73 g, 27.7 mmol) at 0 °C. Then the mixture was stirred at room temperature for 3 h. The mixture was extracted with EtOAc (30 mL x 3). The combined organic layers were washed with brine (50 mL), dried over Na₂SO₄, filtered and concentrated under reduced pressure. The residue was purified by flash silica gel chromatography (Biotage 40 g cartridge, 0-40% EtOAc:Hexane) to give (S)-4-benzyl 1-*tert*-butyl 2-(hydroxymethyl)piperazine-1,4-dicarboxylate. MS = 251.1 [M-C₅H₉O₂+H]⁺

### Step 2: (S)-4-benzyl 1-tert-butyl 2-((4-(methylsulfonyl)phenoxy)methyl)piperazine-1,4-dicarboxylate

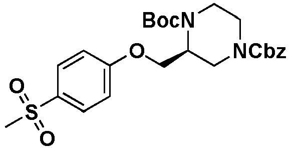

To a solution of (*S*)-4-benzyl 1-*tert*-butyl 2-(hydroxymethyl)piperazine-1,4-dicarboxylate (450 mg, 1.28 mmol) and 4-(methylsulfonyl)phenol (**G5,** 221 mg, 1.28 mmol) in THF (8 mL) at room temperature were added tributylphosphine (520 mg, 2.57 mmol) and TMAD (442 mg, 2.57 mmol). The mixture was then stirred at 40 °C for 16 h under N₂. Brine was then added (10 mL) and the mixture was extracted with EtOAc (8 mL x 3). The combined organic layers were dried over Na₂SO₄, filtered and concentrated under reduced pressure. The residue was purified by flash silica gel chromatography (Biotage 12 g cartridge, 0-100% EtOAc:Hexane) to give (S)-4-benzyl 1-*tert*-butyl 2-((4-(methylsulfonyl)phenoxy)methyl)piperazine-1,4-dicarboxylate which was taken to the next step without further purification. MS = 449.1 [M-C₄H₈+H]⁺.

### Step 3: (S)-tert-butyl 2-((4-(methylsulfonyl)phenoxy)methyl)piperazine-1-carboxylate

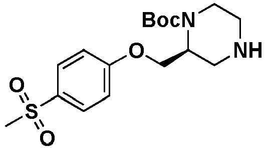

To a suspension of Pd/C (100 mg, 10% by weight in mineral oil) in MeOH (15 mL) was added (*S*)-4-benzyl 1-*tert*-butyl 2-((4-(methylsulfonyl)phenoxy)methyl)piperazine-1,4-dicarboxylate (500 mg, 991 µmol). The suspension was degassed under vacuum and purged with H₂ three times. The mixture was then stirred under H₂ (15 psi) at room temperature for 1 h. The mixture was filtered through a pad of Celite, and the filter cake was rinsed with MeOH (8 mL x 3). The combined filtrates were concentrated under reduced pressure. The residue was dissolved in saturated citric acid solution (30 mL) and extracted with EtOAc (10 mL x 3). The aqueous layer was adjusted to pH = 8 to 9 with saturated aqueous NaHCO₃, and then extracted with EtOAc (20 mL x 2). The combined organic layers were dried over Na₂SO₄, filtered and concentrated under reduced pressure to give (*S*)*-tert*-butyl 2-((4-(methylsulfonyl)phenoxy)methyl)piperazine-1-carboxylate, which was taken to the next step without further purification. MS = 271.1 [M-C₅H₉O₂+H]⁺.

### Step 4: (S)-tert-butyl 4-(3-chloro-5-cyanophenethyl)-2-((4-(methylsulfonyl)phenoxy) methyl)piperazine-1-carboxylate

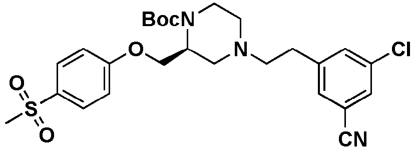

To a mixture of (*S*)-*tert*-butyl 2-((4-(methylsulfonyl)phenoxy)methyl)piperazine-1-carboxylate (500 mg, 1.35 mmol) and 3-chloro-5-(2-oxoethyl)benzonitrile (606 mg, 3.37 mmol) in MeOH (10 mL) and HOAc (0.5 mL) was added NaBH₃CN (170 mg, 2.70 mmol) at 0 °C. The mixture was then stirred at room temperature for 2 h under N₂. The reaction was quenched by addition of water (10 mL) at 0 °C, and then adjusted to pH = 7 with saturated aqueous NaHCO₃ and extracted with EtOAc (15 mL x 3). The combined organic layers were washed with brine (30 mL x 2), dried over Na₂SO₄, filtered and concentrated under reduced pressure to give a residue. The residue was purified by flash silica gel chromatography (Biotage 12 g cartridge, 0-40% EtOAc:Hexane) to give (*S*)-*tert*-butyl 4-(3-chloro-5-cyanophenethyl)-2-((4- (methylsulfonyl)phenoxy) methyl)piperazine-1-carboxylate. MS = 534.2 [M+H]⁺.

### Step 5: 3-chloro-5-{2-[(3S)-3-[(4-methanesulfonylphenoxy)methyl]piperazin-1-yl]ethyl}benzonitrile (Compound 189)

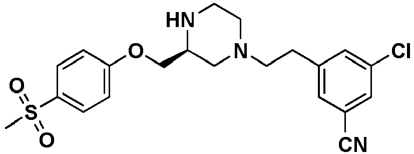

To a solution of (*S*)-*tert*-butyl4-(3-chloro-5-cyanophenethyl)-2-((4-(methylsulfonyl)phenoxy) methyl)piperazine-1-carboxylate (600 mg, 1.12 mmol) in EtOAc (10 mL) was added HCl/EtOAc (4 M, 15 mL). The mixture was stirred at room temperature for 1 h and then concentrated under reduced pressure. The residue was purified by preparative reverse phase HPLC (Phenomenex C18 column, 20-50% MeCN: 10 mM NH₄HCO₃ in H₂O) to give 3-chloro-5-{2-[(3*S*)-3-[(4-methanesulfonylphenoxy)methyl]piperazin-1-yl]ethyl}benzonitrile **(Compound 189).** ¹H NMR (400 MHz, DMSO-*d*₆): δ 7.89 - 7.82 (m, 3H), 7.73 - 7.72 (m, 2H), 7.16 (d, *J* = 8.8 Hz, 2H), 4.01- 3.94 (m, 2H), 3.15 (s, 3H), 3.00 - 2.99 (m, 1H), 2.87 - 2.79 (m, 4H), 2.72 - 2.64 (m, 2H), 2.56 - 2.52 (m, 2H), 2.29 (s, 1H), 2.07 - 2.02 (m, 1H), 1.90 (t, *J* = 9.6 Hz, 1H). MS = 434.0 [M+H]⁺.

The following compound in Table 24 was prepared according to procedures similar to those described for **Compound 189** using the appropriate starting materials.

**Table 24**

| No. | Structure | IUPAC Name | Exact Mass [M+H]⁺ |
|---|---|---|---|
| **190** | 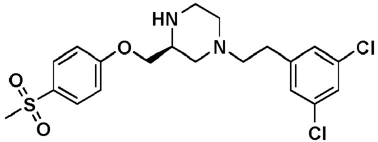 | (3*S*)-1-[2-(3,5-dichlorophenyl)ethyl]-3-[(4-methanesulfonylphenoxy) methyl]piperazine | Calc'd 443.1 |
| | | | Found 443.2 |

### Example 24

### (3S)-1-[2-(5-chloro-2-methoxyphenyl)ethyl]-3-[(4-methanesulfonylphenoxy)methyl]piperazine (Compound 191)

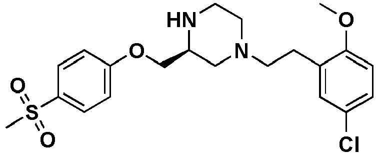

### Step 1: tert-butyl (S)-4-(5-chloro-2-methoxyphenethyl)-2-((4-(methylsulfonyl)phenoxy)methyl)piperazine-1-carboxylate

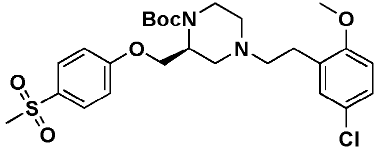

To a solution of Zn (221 mg, 3.37 mmol) and TMSCl (147 mg, 1.35 mmol) in CH₃CN (25 mL) was added two drops of 2-(bromomethyl)-4-chloro-1-methoxybenzene. The mixture was stirred at 40 °C for 1 h. Then HCHO (61 mg, 2.02 mmol), *tert*-butyl (*S*)-2-((4-(methylsulfonyl)phenoxy)methyl)piperazine-1-carboxylate (250 mg, 675 µmol) and 2-(bromomethyl)-4-chloro-1-methoxybenzene (397 mg, 1.69 mmol) were added to the mixture. The reaction was stirred at 40 °C for 16 h. The reaction was concentrated and then diluted with water (25 mL) and extracted with EtOAc (25 mL x 3). The combined organic layers were washed with brine (20 mL), dried over Na₂SO₄, filtered and the filtrate was concentrated to give a residue. The residue was purified by preparative reverse phase HPLC (Phenomenex Gemini-NX, 50-70% MeCN:10 mM NH₄HCO₃ in H₂O) to give *tert*-butyl (*S*)-4-(5-chloro-2-methoxyphenethyl)-2-((4-(methylsulfonyl)phenoxy)methyl)piperazine-1-carboxylate. MS = 539.2 [M+H]⁺.

### Step 2 (3S)-1-[2-(5-chloro-2-methoxyphenyl)ethyl]-3-[(4-methanesulfonylphenoxy)methyl]piperazine (Compound 191)

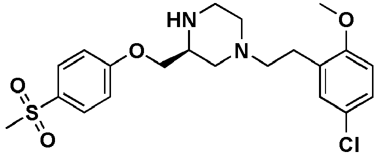

To a solution of *tert*-butyl (*S*)-4-(5-chloro-2-methoxyphenethyl)-2-((4-(methylsulfonyl)phenoxy)methyl)piperazine-1-carboxylate (60 mg, 111 µmol) in MeOH (1 mL) was added HCl/MeOH (4 M, 1 mL). The reaction mixture was stirred at room temperature for 2 h. The reaction mixture was then concentrated under reduced pressure to give (3*S*)-1-[2-(5-chloro-2-methoxyphenyl)ethyl]-3-[(4-methanesulfonylphenoxy)methyl]piperazine HCl salt **(Compound 191).** MS = 439.2 [M+H]⁺, ¹H NMR (400 MHz, DMSO-*d*₆): δ 7.93 - 7.89 (m, 2H), 7.32 - 7.25 (m, 4H), 7.03 (d, *J*=8.8 Hz, 1H), 4.51 - 4.43 (m, 2H), 4.20 - 4.19 (m, 1H), 4.0 - 4.01 (m, 1H), 3.83 - 3.81 (m, 4H), 3.60 - 3.56 (m, 3H), 3.43 - 3.35 (m, 4H), 3.18 (s, 3H), 3.08 - 3.04 (m, 2H).

The following compounds in Table 25 were prepared according to procedures similar to those described for **Compound 191** using the appropriate starting materials.

**Table 25**

| No. | Structure | IUPAC Name | Exact Mass [M+H]⁺ |
|---|---|---|---|
| **192** | 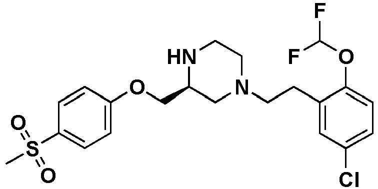 | (3*S*)-1-{2-[5-chloro-2-(difluoromethoxy)phenyl] ethyl}-3-[(4-methanesulfonylphenoxy) methyl]piperazine | Calc'd 475.1 |
| | | | Found 475.2 |
| **193** | 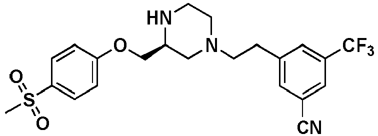 | 3-{2-[(3*S*)-3-[(4-methanesulfonylphenoxy) methyl]piperazin-1-yl]ethyl}-5-(trifluoromethyl)benzonitrile | Calc'd 468.2 |
| | | | Found 468.2 |
| **194** | 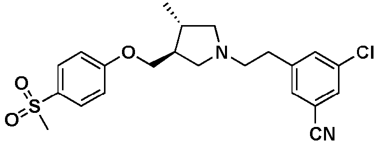 | 3-chloro-5-{2-[(3*S*,4*S*)-3-[(4-methanesulfonylphenoxy) methyl]-4-methylpyrrolidin-1-yl]ethyl}benzonitrile | Calc'd 433.1 |
| | | | Found 433.1 |
| **195** | 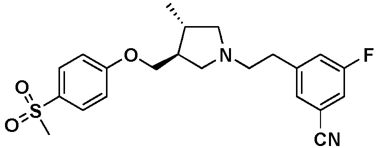 | 3-fluoro-5-{2-[(3*S*,4*S*)-3-[(4-methanesulfonylphenoxy) methyl]-4-methylpyrrolidin-1-yl]ethyl}benzonitrile | Calc'd 417.2 |
| | | | Found 417.2 |
| **196** | 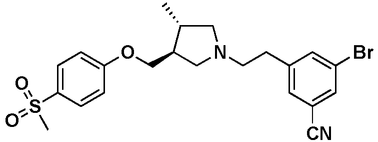 | 3-bromo-5-{2-[(3*S*,4*S*)-3-[(4-methanesulfonylphenoxy) methyl]-4-methylpyrrolidin-1-yl]ethyl}benzonitrile | Calc'd 477.1 |
| | | | Found 477.1 |
| **181** | 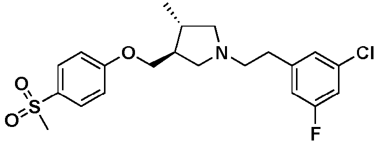 | (3*S*,4*S*)-1-[2-(3-chloro-5-fluorophenyl)ethyl]-3-[(4-methanesulfonylphenoxy) methyl]-4-methylpyrrolidine | Calc'd 426.1 |
| | | | Found 426.1 |

### Example 25

### (3S)-1-[2-(5-chloro-2-methylphenyl)ethyl]-3-[(4-methanesulfonylphenoxy)methyl]piperazine (Compound 197)

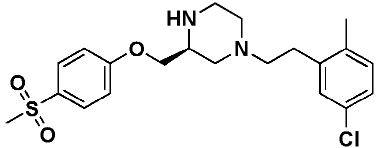

### Step 1: (S)-tert-butyl 2-((4-(methylsulfonyl)phenoxy)methyl)piperazine-1-carboxylate

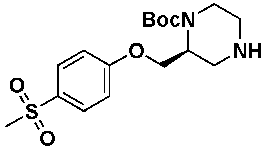

To a suspension of Pd/C (100 mg, 10% by weight in mineral oil) in MeOH (15 mL) was added (*S*)-4-benzyl 1-*tert*-butyl 2-((4-(methylsulfonyl)phenoxy)methyl)piperazine-1,4-dicarboxylate (500 mg, 991 mmol). The suspension was degassed under vacuum and purged with H₂ three times. The mixture was then stirred under H₂ (15 psi) at room temperature for 4 h. The mixture was filtered through a pad of Celite, and the filter cake was rinsed with MeOH (8 mL x 3). The combined filtrate was concentrated under reduced presure. The residue was dissolved in EtOAc (30 mL) and saturated citric acid solution (30 mL). The organic layer was separated, and the aqueous layer was extracted with EtOAc (15 mL). The aqueous layer was adjusted to pH>7 by addition of solid NaHCO₃, and then extracted with EtOAc (20 mL x 2). The combined organic layers were dried over Na₂SO₄, filtered and concentrated under reduced pressure to give (S)-tert-butyl 2-((4-(methylsulfonyl)phenoxy)methyl)piperazine-1-carboxylate, which was taken to the next step without further purification. MS = 271.1 [M-C₅H₉O₂+H]⁺.

### Step 2: (S)-tert-butyl 4-(2-bromo-5-chlorophenethyl)-2-((4-(methylsulfonyl)phenoxy)methyl)piperazine-1-carboxylate

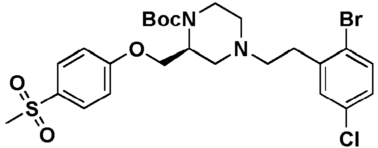

To a solution of (*S*)-*tert*-butyl 2-((4-(methylsulfonyl)phenoxy)methyl)piperazine-1-carboxylate (170 mg, 459 µmol) and 2-(2-bromo-5-chloro-phenyl)acetaldehyde (107 mg, 459 µmol) in DCM (5 mL) at room temperature was added AcOH (459 µmol, 26 uL) followed by NaBH(OAc)₃ (146 mg, 688 µmol). The mixture was then stirred for 1 h. To the mixture was added saturated aqueous NaHCO₃ (15 mL) and DCM (10 mL). The organic layer was separated, and the aqueous layer was extracted with DCM (10 mL). The combined organic layers were dried over Na₂SO₄, filtered and concentrated under reduced pressure. The residue was purified by flash silica gel chromatography (Biotage 12 g cartridge, 0-40% EtOAc:Hexane) to give (*S*)-*tert*-butyl 4-(2-bromo-5-chlorophenethyl)-2-((4-(methylsulfonyl)phenoxy)methyl)piperazine-1-carboxylate. MS = 587.2/589.2 [M+H]⁺

### Step 3: (S)-tert-butyl 4-(5-chloro-2-methylphenethyl)-2-((4-(methylsulfonyl)phenoxy)methyl)piperazine-1-carboxylate

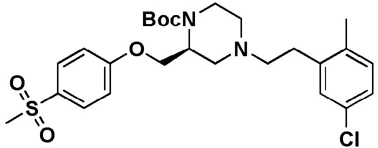

To a mixture of (*S*)-*tert*-butyl 4-(2-bromo-5-chlorophenethyl)-2-((4-(methylsulfonyl)phenoxy)methyl)piperazine-1-carboxylate (130 mg, 221 µmol), methylboronic acid (27 mg, 442 µmol) and K₂CO₃ (61 mg, 442 µmol) in 1,4-dioxane (4 mL) and H₂O (0.4 mL) was added Pd(dppf)Cl₂•CH₂Cl₂ (18 mg, 22.1 µmol) at room temperature under N₂. The resulting mixture was then stirred at 100 °C for 16 h under N₂. The mixture was cooled to room temperature and concentrated under reduced presure. The residue was purified by flash silica gel chromatography (Biotage 12 g cartridge, 0-100% EtOAc:Hexane) to give (*S*)-*tert*-butyl 4-(5-chloro-2-methylphenethyl)-2-((4-(methylsulfonyl)phenoxy)methyl)piperazine-1-carboxylate, which was taken to the next step without further purification. MS = 523.3 [M+H]⁺.

### Step 4: (3S)-1-[2-(5-chloro-2-methylphenyl)ethyl]-3-[(4-methanesulfonylphenoxy)methyl]piperazine (Compound 197)

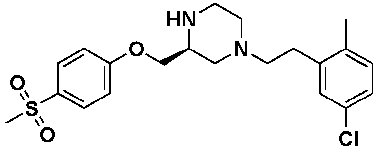

A mixture of (*S*)-*tert*-butyl 4-(5-chloro-2-methylphenethyl)-2-((4-(methylsulfonyl)phenoxy)methyl)piperazine-1-carboxylate (120 mg, 229 µmol) in HCl/EtOAc (4 M, 8 mL) was stirred at room temperature for 0.5 h. The mixture was concentrated under reduced pressure. The solid residue was washed with EtOAc (8 mL), and then purified by preparative reverse phase HPLC (Waters Xbridge BEH C18 column, 30-60% MeCN:10 mM NH₄HCO₃ in H₂O) to give (3*S*)-1-[2-(5-chloro-2-methylphenyl)ethyl]-3-[(4-methanesulfonylphenoxy)methyl]piperazine **(Compound 197).** ¹H NMR (400 MHz, DMSO-*d*₆): δ 7.83 (d, *J* = 9.2 Hz, 2H), 7.23 (s, 1H), 7.18 - 7.13 (m, 4H), 4.02 - 3.96 (m, 2H), 3.15 (s, 3H), 3.04 - 2.95 (m, 1H), 2.92 - 2.86 (m, 2H), 2.79 - 2.64 (m, 4H), 2.47 - 2.43 (m, 3H), 2.32 (s, 3H), 2.10 - 2.00 (m, 1H), 1.92 - 1.87 (m, 1H). MS = 423.3 [M+H]⁺.

### Example 26

### 3-{2-[(3S,4S)-3-[(4-methanesulfonylphenoxy)methyl]-4-methylpyrrolidin-1-yl]ethyl}-5-methylbenzonitrile (Compound 198)

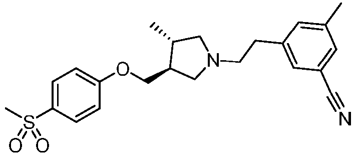

### Step 1: 3-{2-[(3S,4S)-3-[(4-methanesulfonylphenoxy)methyl]-4-methylpyrrolidin-1-yl]ethyl}-5-methylbenzonitrile (Compound 198)

A vial was charged with 3-bromo-5-{2-[(3*S*,4*S*)-3-[(4-methanesulfonylphenoxy)methyl]-4-methylpyrrolidin-1-yl]ethyl}benzonitrile (100 mg, 0.209 mmol), methylboronic acid (190 mg, 0.314 mmol), K₂CO₃ (58 mg, 0.419 mmol), tetrakis(triphenylphosphine)palladium(0) (10 mg, 0.008 mmol), Toluene (1.0 mL) and H₂O (0.21 mL). The mixture was then sparged with nitrogen gas for 1 minute and then heated to 100 °C for 16 h. Reaction was partitioned with H₂O and EtOAc and extracted 3 times. The combined organics layers were dried over Na₂SO₄, filtered and concentrated. The crude oil was purified by prep-HPLC (5-50% MeCN in H₂O with 0.1% formic acid modifier) to give 3-{2-[(3*S*,4*S*)-3-(4-methanesulfonylphenoxymethyl)-4-methylpyrrolidin-1-yl]ethyl}-5-methylbenzonitrile **(Compound 198).** ¹H NMR (500 MHz, CDCl₃): δ 7.82 - 7.77 (m, 2H), 7.27 - 7.17 (m, 3H), 6.97 - 6.92 (m, 2H), 5.00-3.49 (m, 3H), 4.02 - 3.93 (m, 2H), 3.15 (t, *J* = 8.5 Hz, 1H), 2.96 (d, *J* = 1.2 Hz, 3H), 2.92 (t, *J* = 7.4 Hz, 2H), 2.85 (d, *J* = 5.5 Hz, 2H), 2.84 - 2.80 (m, 1H), 2.80 - 2.73 (m, 1H), 2.34 (t, *J* = 9.0 Hz, 1H), 2.28 - 2.21 (m, 1H), 2.18-2.10 (m, 1H), 1.11 (d, *J* = 6.6 Hz, 3H). MS = 413.2 [M+H]⁺.

### Example 27

### rac-cis or trans-3-chloro-5-{2-[4-[(4-methanesulfonylphenoxy)methyl]-2-methylpyrrolidin-1-yl]ethyl}benzonitrile (Compound 199)

### 3-chloro-5-(2-((2S,4R or 2R,4S or 2R,4R or 2S,4S)-2-methyl-4-((4-(methylsulfonyl)phenoxy)methyl)pyrrolidin-1-yl)ethyl)benzonitrile (Compound 200)

### 3-chloro-5-(2-((2R,4S or 2S,4R or 2R,4R or 2S,4S)-2-methyl-4-((4-(methylsulfonyl)phenoxy)methyl)pyrrolidin-1-yl)ethyl)benzonitrile (Compound 201)

### rac-trans or cis-3-chloro-5-{2-[4-[(4-methanesulfonylphenoxy)methyl]-2-methylpyrrolidin-1-yl]ethyl}benzonitrile (Compound 202)

### 3-chloro-5-(2-((2R,4S or 2S,4R or 2R,4R or 2S,4S)-2-methyl-4-((4-(methylsulfonyl)phenoxy)methyl)pyrrolidin-1-yl)ethyl)benzonitrile (Compound 203)

### 3-chloro-5-(2-((2R,4S or 2S,4R or 2S,4S or 2R.4R)-2-methyl-4-((4-(methylsulfonyl)phenoxy)methyl)pyrrolidin-1-yl)ethyl)benzonitrile (Compound 204)

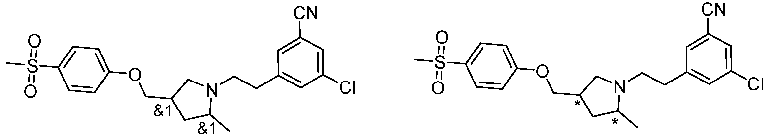

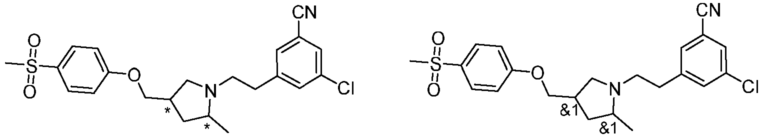

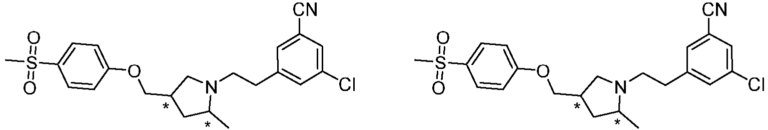

### Step 1: rac-cis or trans-3-chloro-5-{2-[4-[(4-methanesulfonylphenoxy)methyl]-2-methylpyrrolidin-1-yl]ethyl}benzonitrile (Compound 199) and rac-trans or cis-3-chloro-5-{2-[4-[(4-methanesulfonylphenoxy)methyl]-2-methylpyrrolidin-1-yl]ethyl}benzonitrile (Compound 202)

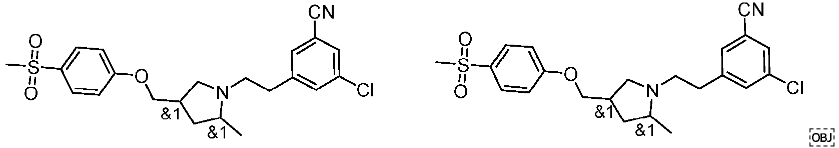

To a mixture of Zn (23 mg, 356 µmol) in MeCN (2 mL) were added three drops of 3-(bromomethyl)-5-chloro-benzonitrile in 0.1 mL of MeCN and TMSCl (200 µmol, 25 uL). The mixture was stirred at 50 °C for 20 min. Then 3-(bromomethyl)-5-chloro-benzonitrile (39 mg, 167 µmol), 2-methyl-4-[(4-methylsulfonylphenoxy)methyl]pyrrolidine (30 mg, 111 µmol) and paraformaldehyde (20 mg, 222 µmol) were added to the mixture successively. The resulting mixture was stirred at room temperature for 1 h. To the mixture was added H₂O (10 mL), then the mixture was extracted with EtOAc (10 mL x 3). The combined organic layers were dried over Na₂SO₄, filtered and the filtrate was concentrated under reduced pressure. The residue was purified by preparative reverse phase HPLC (Waters Xbridge BEH C18 column, 40-65% MeCN:10 mM NH₄HCO₃ in H₂O). The first eluting isomer **(Compound 199) :** ¹H NMR (400 MHz, DMSO-*d*₆): δ 7.84 - 7.81 (m, 3H), 7.76 - 7.74 (m, 2H), 7.17 - 7.14 (m, 2H), 4.04 - 3.93 (m, 2H), 3.35 - 3.34 (m, 1H), 3.15 (s, 3H), 3.01 - 2.98 (m, 1H), 2.80 - 2.70 (m, 2H), 2.46-2.45 (m, 2H), 2.33-2.30 (m, 1H), 2.03 (t, *J =* 7.2 Hz, 1H), 1.72 - 1.71 (m, 1H), 1.53 - 1.52 (m, 1H), 0.98 (d, *J* = 6.0 Hz, 3H). MS = 433.2 [M+H]⁺. The second eluting isomer **(Compound 202):** ¹H NMR (400 MHz, DMSO-*d*₆): δ 7.84 - 7.80 (m, 3H), 7.72 - 7.70 (m, 2H), 7.14 (d, *J =* 8.8 Hz, 2H), 3.89 - 3.87 (m, 2H), 3.15 (s, 3H), 3.07 - 3.04 (m, 1H), 2.96 - 2.95 (m, 1H), 2.78 - 2.76 (m, 2H), 2.52 - 2.51 (m, 1H), 2.33 - 2.27 (m, 3H), 2.11 - 2.07 (m, 1H), 1.06 - 1.01 (m, 1H), 0.96 (d, *J* = 6.0 Hz, 3H). MS = 433.2 [M+H]⁺.

### Step 2: 3-chloro-5-{2-[(2R,4S or 2S,4R or 2R,4R or 2S,4S)-4-[(4-methanesulfonylphenoxy)methyl]-2-methylpyrrolidin-1-yl]ethyl}benzonitrile (Compound 200) and 3-chloro-5-{2-[(2S,4R or 2R,4S or 2R,4R or 2S,4S)-4-[(4-methanesulfonylphenoxy)methyl]-2-methylpyrrolidin-1-yl]ethyl} benzonitrile (Compound 201)

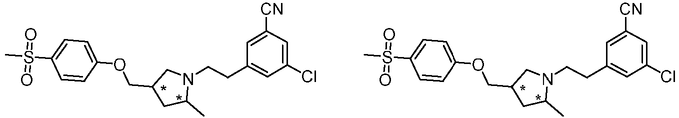

The first eluting isomer from Step 1 was further purified by preparative chiral SFC (CHIRALPAK AD, 60% ethanol with 0.1% NH₄OH in CO₂). The first eluting isomer of the title compound (**Compound 200**): ¹H NMR (400 MHz, DMSO-*d*₆): δ 7.8 - 7.82 (m, 3H), 7.76 - 7.74 (m, 2H), 7.15 (d, *J =* 8.8 Hz, 2H), 4.04 - 3.95 (m, 2H), 3.35 - 3.34 (m, 1H), 3.15 (s, 3H), 3.01 - 2.98 (m, 1H), 2.83 - 2.75 (m, 2H), 2.52 - 2.50 (m, 2H), 2.31 - 2.29 (m, 1H), 2.03 (t, *J =* 7.2 Hz, 1H), 1.72 - 1.71 (m, 1H), 1.53 - 1.50 (m, 1H), 0.98 (d, *J* = 6.0 Hz, 3H). MS = 433.2 [M+H]⁺
The second eluting isomer of the title compound **(Compound 201).** ¹H NMR (400 MHz, DMSO-*d*₆): δ 7.84 - 7.82 (m, 3H), 7.76 - 7.74 (m, 2H), 7.15 (d, *J =* 8.8 Hz, 2H), 4.04 - 3.95 (m, 2H), 3.35 - 3.32 (m, 1H), 3.15 (s, 3H), 3.01 - 2.98 (m, 1H), 2.83 - 2.76 (m, 2H), 2.52 - 2.50 (m, 2H), 2.31 - 2.29 (m, 1H), 2.05 - 2.03 (m, 1H), 1.72 - 1.71 (m, 1H), 1.53 - 1.50 (m, 1H), 0.98 (d, *J* = 5.6 Hz, 3H). MS = 433.2 [M+H]⁺.

### Step 3: 3-chloro-5-(2-((2R,4S or 2S,4R or 2R,4R or 2S,4S)-2-methyl-4-((4-(methylsulfonyl)phenoxy)methyl)pyrrolidin-1-yl)ethyl)benzonitrile (Compound 203) and 3-chloro-5-(2-((2R,4S or 2S,4R or 2S,4S or 2R.4R)-2-methyl-4-((4-(methylsulfonyl)phenoxy)methyl)pyrrolidin-1-yl)ethyl)benzonitrile (Compound 204)

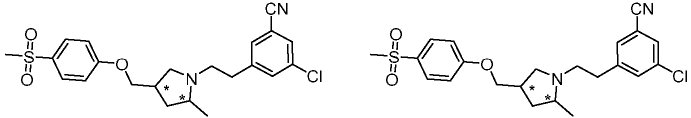

The second eluting isomer from Step 1 (150 mg, 346 µmol) was further purified by preparative chiral SFC (CHIRALPAK AD, 42% ethanol with 0.1% NH₄OH in CO₂). The first eluting isomer of the title compound **(Compound 203):** ¹H NMR (DMSO-*d*₆, 400 MHz): δ 7.84 - 7.81 (m, 3H), 7.73 - 7.70 (m, 2H), 7.14 (d, *J =* 8.8 Hz, 2H), 3.90 - 3.88 (m, 2H), 3.15 (s, 3H), 3.07 - 3.04 (m, 1H), 2.97 - 2.96 (m, 1H), 2.79 - 2.73 (m, 2H), 2.52 - 2.50 (m, 1H), 2.33 - 2.28 (m, 3H), 2.15 - 2.13 (m, 1H), 1.07 - 1.01 (m, 1H), 0.96 (d, *J =* 5.6 Hz, 3H). MS = 433.2 [M+H]⁺.

The second eluting isomer of the title compound **(Compound 204):** ¹H NMR (400 MHz, DMSO-*d*₆): δ 7.86 - 7.81 (m, 3H), 7.73 - 7.70 (m, 2H), 7.17 - 7.13 (m, 2H), 3.90 - 3.88 (m, 2H), 3.15 (s, 3H), 3.05 - 3.04 (m, 1H), 2.99 - 2.98 (m, 1H), 2.81 - 2.76 (m, 2H), 2.59 - 2.57 (m, 1H), 2.33 - 2.27 (m, 3H), 2.15 - 2.14 (m, 1H), 1.07 - 1.01 (m, 1H), 0.96 (d, *J =* 5.6 Hz, 3H). MS = 433.2 [M+H]⁺.

### Example 28

### rac-cis-1-[2-(3-chlorophenyl)ethyl]-4-[(4-methanesulfonylphenoxy)methyl]-3-methylpyrrolidin-3-ol (Compound 205)

### (3R,4R or 3S,4S)-1-[2-(3-chlorophenyl)ethyl]-4-[(4-methanesulfonylphenoxy)methyl]-3-methylpyrrolidin-3-ol (Compound 206)

### (3S,4S or 3R,4R)-1-[2-(3-chlorophenyl)ethyl]-4-[(4-methanesulfonylphenoxy)methyl]-3-methylpyrrolidin-3-ol (Compound 207)

### rac-trans-1-[2-(3-chlorophenyl)ethyl]-4-[(4-methanesulfonylphenoxy)methyl]-3-methylpyrrolidin-3-ol (Compound 208)

### (3R,4S or 3S,4R)-1-[2-(3-chlorophenyl)ethyl]-4-[(4-methanesulfonylphenoxy)methyl]-3-methylpyrrolidin-3-ol pyrrolidine-3-ol (Compound 209)

### (3S,4R or 3R,4S)-1-[2-(3-chlorophenyl)ethyl]-4-[(4-methanesulfonylphenoxy)methyl]-3-methylpyrrolidin-3-ol (Compound 210)

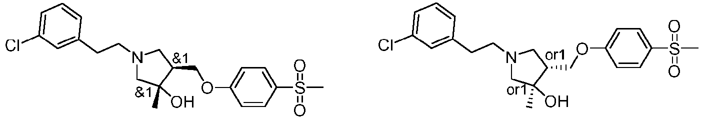

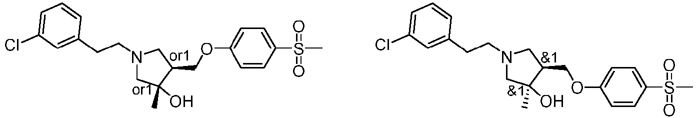

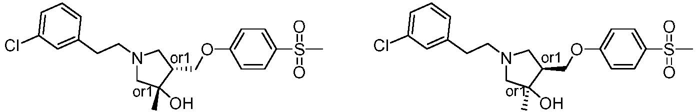

### Step 1: tert-butyl 3-hydroxy-4-(hydroxymethyl)pyrrolidine-1-carboxylate

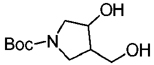

To a solution of 1-*tert*-butyl 3-ethyl 4-oxopyrrolidine-1,3-dicarboxylate (10.0 g, 38.9 mmol) in EtOH (200 mL) at 0 °C was slowly added NaBH₄ (14.7 g, 389 mmol). The mixture was stirred at room temperature for 15 h. The reaction mixture was quenched with ice water (300 mL), and then extracted with EtOAc (100 mL x 3). The combined organic layers were dried over Na₂SO₄, filtered and concentrated under reduced pressure. The residue was purified by flash silica gel chromatography (Biotage 80 g cartridge, 0-100% EtOAc:Hexane) to give *tert*-butyl 3-hydroxy-4-(hydroxymethyl)pyrrolidine-1-carboxylate. MS = 162.1 [M-C₄H₈+H]⁺.

### Step 2: tert-butyl 3-hydroxy-4-((4-(methylsulfonyl)phenoxy)methyl)pyrrolidine-1-carboxylate

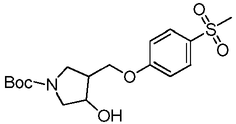

To a mixture of 1-fluoro-4-methylsulfonyl-benzene (5.21 g, 29.92 mmol) and *tert*-butyl 3-hydroxy-4-(hydroxymethyl)pyrrolidine-1-carboxylate (6.50 g, 29.9 mmol) in DMF (50 mL) was added K₂CO₃ (8.27 g, 59.84 mmol). The mixture was stirred at 100 °C for 60 h. The reaction mixture was diluted with water (150 mL), and then extracted with EtOAc (50 mL x 3). The combined organic layers were dried over Na₂SO₄, filtered and concentrated under reduced pressure. The residue was purified by flash silica gel chromatography (Biotage 80 g cartridge, 0-50% EtOAc:Hexane) to give *tert*-butyl 3-hydroxy-4-((4-(methylsulfonyl)phenoxy)methyl)pyrrolidine-1-carboxylate. MS = 316.1 [M-C₄H₈+H]⁺.

### Step 3: tert-butyl 3-((4-(methylsulfonyl)phenoxy)methyl)-4-oxopyrrolidine-1-carboxylate

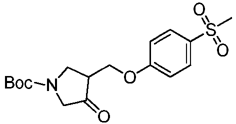

To a solution of *tert*-butyl 3-hydroxy-4-((4-(methylsulfonyl)phenoxy)methyl)pyrrolidine-1-carboxylate (4.90 g, 13.2 mmol) in DCM (50 mL) at 0 °C was added DMP (6.71 g, 15.8 mmol). The mixture was stirred at room temperature for 15 h. The reaction mixture was cooled to 0 °C, quenched with saturated aqueous Na₂SO₃ (100 mL), concentrated, then extracted with EtOAc (50 mL x 5). The combined organic layers were dried over Na₂SO₄, filtered and concentrated under reduced pressure. The residue was purified by flash silica gel chromatography (Biotage 40 g cartridge, 0-100% EtOAc:Hexane) to give *tert*-butyl 3-((4-(methylsulfonyl)phenoxy)methyl)-4-oxopyrrolidine-1-carboxylate. MS = 314.1 [M-C₄H₈+H]⁺.

### Step 4: rac-cis--tert-butyl 3-hydroxy-3-methyl-4-((4-(methylsulfonyl)phenoxy)methyl)pyrrolidine-1-carboxylate and rac-trans--tert-butyl 3-hydroxy-3-methyl-4-((4-(methylsulfonyl)phenoxy)methyl)pyrrolidine-1-carboxylate

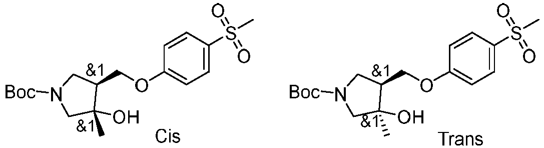

A solution of *tert*-butyl 3-((4-(methylsulfonyl)phenoxy)methyl)-4-oxopyrrolidine-1-carboxylate (370 mg, 1.00 mmol) in THF (2 mL) was degassed and purged with N₂ three times. The mixture was cooled to 0 °C and MeMgBr (3 M in 2-MeTHF, 667 uL, 2.0 mmol) was added dropwise. The mixture was stirred at room temperature for 3 h under N₂ atmosphere. The reaction mixture was cooled to 0 °C, quenched with water (10 mL), and extracted with EtOAc (15 mL x 3). The combined organic layers were dried over Na₂SO₄, filtered and concentrated under reduced pressure. The residue was purified by preparative reverse phase HPLC (Phenomenex Gemini-NX, 20-50% MeCN: 10 mM NH₄HCO₃ in H₂O) to give *rac-cis-tert-*butyl 3-hydroxy-3-methyl-4-((4-(methylsulfonyl)phenoxy)methyl)pyrrolidine-1-carboxylate (the first eluting isomer in HPLC). MS = 330.1 [M-C₄H₈+H]⁺. And *rac-trans-tert-*butyl 3-hydroxy-3-methyl-4-((4-(methylsulfonyl)phenoxy)methyl)pyrrolidine-1-carboxylate (the second eluting isomer in HPLC). MS = 330.1 [M-C₄H₈+H]⁺.

### Step 5: rac-cis-3-methyl-4-((4-(methylsulfonyl)phenoxy)methyl)pyrrolidin-3-ol

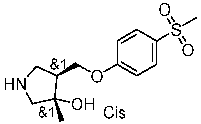

To a solution of the 1st eluting isomer from **Step 4** (40.0 mg, 104 µmol) in DCM (0.3 mL) at 0 °C was added TFA (154 mg, 1.35 mmol). The mixture was stirred at room temperature for 1.5 h. The reaction mixture was concentrated under reduced pressure to give *rac-*cis-3-methyl-4-((4-(methylsulfonyl)phenoxy)methyl)pyrrolidin-3-ol, which was used without further purification. MS = 286.2 [M+H]⁺.

### Step 6: rac-cis-1-[2-(3-chlorophenyl)ethyl]-4-[(4-methanesulfonylphenoxy)methyl]-3-methylpyrrolidin-3-ol (Compound 205)

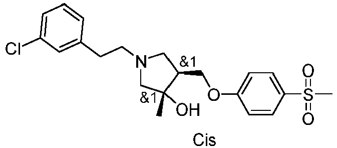

To a solution of the residue from **Step 5** (29.0 mg, 102 µmol) in MeOH (0.3 mL) were added TEA (10.3 mg, 102 µmol), HOAc (5.8 uL, 102 µmol,) and 2-(3-chlorophenyl)acetaldehyde (23.6 mg, 152 µmol). After stirred at room temperature for 1 h, NaBH₃CN (9.58 mg, 152 µmol) was added. The mixture was stirred at room temperature for 2 h, then was cooled to 0 °C and quenched by addition of H₂O (0.2 mL). The residue was purified by preparative reverse phase HPLC (Waters Xbridge Prep OBD C18 column, 30-60% MeCN:10 mM NH₄HCO₃ in H₂O) to give *rac-cis-*1-[2-(3-chlorophenyl)ethyl]-4-[(4-methanesulfonylphenoxy)methyl]-3-methylpyrrolidin-3-ol (**Compound 205**). ¹H NMR (400 MHz DMSO-*d*₆): δ 7.83 (d, *J =* 8.4 Hz, 2H), 7.31 - 7.16 (m, 6H), 4.77 (s, 1H), 4.18 - 4.14 (m, 1H), 3.94 (t, *J =* 8.8 Hz, 1H), 3.15 (s, 3H), 3.05 - 2.95 (m, 1H), 2.73 - 2.61 (m, 6H), 2.44 - 2.42 (m, 2H), 1.21 (s, 3H). MS = 424.2 [M+H]⁺.

### Step 7 (3R,4R or 3S,4S)-1-[2-(3-chlorophenyl)ethyl]-4-[(4-methanesulfonylphenoxy)methyl]-3-methylpyrrolidin-3-ol (Compound 206) and (3S,4S or 3R,4R)-1-(3-chlorophenethyl)-3-methyl-4-((4-(methylsulfonyl)phenoxy)methyl)pyrrolidin-3-ol (Compound 207)

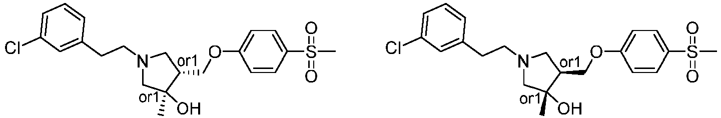

The material obtained from **Step 6** was further purified by chiral SFC (CHIRALPAK IG, 55% isopropanol with 0.1% NH₄OH in CO₂) The first eluting isomer of the title compound (**Compound 206**): ¹H NMR (400 MHz, DMSO-*d*₆): δ 7.83 (d, *J =* 8.8 Hz, 2H), 7.31 -7.24 (m, 4H), 7.21 - 7.16 (m, 2H), 4.79 (s, 1H), 4.18 - 4.14 (m, 1H), 3.95 (t, *J =* 8.8 Hz, 1H), 3.15 (s, 3H), 3.05 - 2.95 (m, 1H), 2.71 - 2.61 (m, 6H), 2.45 - 2.35 (m, 2H), 1.21 (s, 3H). MS = 424.2 [M+H]⁺.The second eluting isomer of the title compound (**Compound 207**): ¹H NMR (400 MHz, DMSO-*d*₆): δ 7.84 (d, *J =* 8.8 Hz, 2H), 7.33 - 7.26 (m, 4H), 7.21 - 7.17 (m, 2H), 4.82 (s, 1H), 4.18 - 4.14 (m, 1H), 4.02 - 3.97 (m, 1H), 3.16 (s, 3H), 3.05 - 2.95 (m, 1H), 2.90 - 2.74 (m, 4H), 2.64 - 2.54 (m, 2H), 2.46 - 2.87 (m, 2H), 1.23 (s, 3H). MS = 424.2 [M+H]⁺.

### Step 8: rac-trans-3-methyl-4-((4-(methylsulfonyl)phenoxy)methyl)pyrrolidin-3-ol

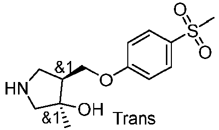

To a solution of the 2^{nd} eluting isomer from **Step 4** (70.0 mg, 182 µmol) in DCM (0.6 mL) at 0 °C was added TFA (308 mg, 2.70 mmol). The mixture was stirred at room temperature for 1.5 h, then was concentrated under reduced pressure to give *rac-trans-*3-methyl-4-((4-(methylsulfonyl)phenoxy)methyl)pyrrolidin-3-ol. MS = 286.1 [M+H]⁺.

### Step 9: rac-trans-1-[2-(3-chlorophenyl)ethyl]-4-[(4-methanesulfonylphenoxy)methyl]-3-methylpyrrolidin-3-ol (Compound 208)

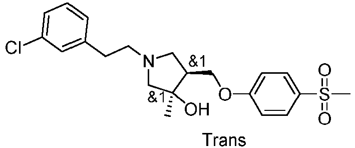

To a solution of the residue from **Step 8** (51.0 mg, 179 µmol) in MeOH (0.5 mL) were added TEA (25 uL, 178.72 µmol), HOAc (10 uL, 178 µmol) and 2-(3-chlorophenyl)acetaldehyde (41.4 mg, 268 µmol). After stirred at room temperature for 1 h, NaBH₃CN (16.9 mg, 268 µmol) was added, and the mixture was stirred at for 2 *h.* The reaction mixture was cooled to 0 °C and quenched by addition H₂O (0.2 mL). The residue was purified by preparative reverse phase HPLC (Waters Xbridge Prep OBD C18 column, 30-60% MeCN:10 mM NH₄HCO₃ in H₂O) to give rac-trans-1-[2-(3-chlorophenyl)ethyl]-4-[(4-methanesulfonylphenoxy)methyl]-3-methylpyrrolidin-3-ol (**Compound 208**). ¹H NMR (400 MHz, DMSO-*d*₆): δ 7.85 - 7.82 (m, 2H), 7.31 - 7.11 (m, 6H), 4.58 (s, 1H), 4.29 - 4.25 (m, 1H), 3.99 (t, *J* = 8.8 Hz, 1H), 3.15 (s, 3H), 2.91 (t, *J =* 8.0 Hz, 1H), 2.74 - 2.54 (m, 7H), 2.27 - 2.20 (m, 1H), 1.32 (s, 3H). MS = 424.3 [M+H]⁺.

### Step 10: (3R,4S or 3S,4R)-1-[2-(3-chlorophenyl)ethyl]-4-[(4-methanesulfonylphenoxy)methyl]-3-methylpyrrolidin-3-ol (Compound 209) and (3S,4R or 3R,4S)-1-[2-(3-chlorophenyl)ethyl]-4-[(4-methanesulfonylphenoxy)methyl]-3-methylpyrrolidin-3-ol (Compound 210)

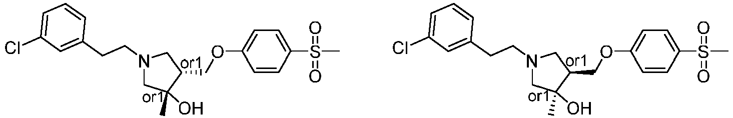

The material obtained from **Step 9** (40 mg, 94 µmol) was further purified by chiral SFC (CHIRALPAK IG, 60% isopropanol with 0.1% NH₄OH in CO₂). The first eluting isomer of the title compound (**Compound 209**): ¹H NMR (400 MHz, DMSO-*d*₆): δ 7.84 (d, *J* = 8.8 Hz, 2H), 7.32 - 7.26 (m, 4H), 7.21 - 7.12 (m, 2H), 4.64 (br s, 1H), 4.30 - 4.26 (m, 1H), 4.00 (t, *J =* 8.8 Hz, 1H), 3.15 (s, 3H), 3.10 - 2.90 (m, 2H), 2.85 - 2.65 (m, 5H), 2.30 - 2.15 (m, 2H), 1.33 (s, 3H). MS = 424.2 [M+H]⁺. The second eluting isomer of the title compound (**Compound 210**): ¹H NMR (400 MHz, DMSO-*d*₆): δ 7.84 (d, *J =* 8.8 Hz, 2H), 7.32 - 7.25 (m, 4H), 7.21 - 7.12 (m, 2H), 4.65 (br s, 1H), 4.30 - 4.26 (m, 1H), 4.00 (t, *J =* 9.2 Hz, 1H), 3.15 (s, 3H), 3.05 - 2.89 (m, 1H), 2.90 - 2.55 (m, 6H), 2.30 - 2.15 (m, 2H), 1.33 (s, 3H). MS = 424.2 [M+H]⁺.

### Example 29

### 1-[2-(3-chlorophenyl)ethyl]-5-[(4-methanesulfonylphenoxy)methyl]-2-methylpiperazine (Compound 211)

### rac-cis or trans-(2R,5S)-1-[2-(3-chlorophenyl)ethyl]-5-[(4-methanesulfonylphenoxy)methyl]-2-methylpiperazine (Compound 212)

### rac-trans or cis-(2R,5S)-1-[2-(3-chlorophenyl)ethyl]-5-[(4-methanesulfonylphenoxy)methyl]-2-methylpiperazine (Compound 213)

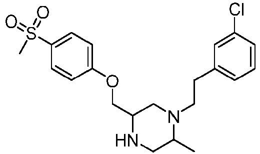

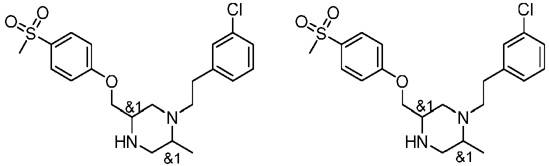

### Step 1: 1-(tert-butoxycarbonyl)-4-(3-chlorophenethyl)-5-methylpiperazine-2-carboxylic acid

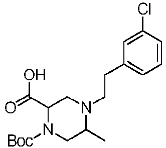

To a solution of 1-(*tert*-butoxycarbonyl)-5-methylpiperazine-2-carboxylic acid (950 mg, 3.89 mmol, ) and 2-(3-chlorophenyl)acetaldehyde (721 mg, 4.67 mmol) in MeOH (10 mL) and AcOH (0.5 mL) was added 2-methylpyridine borane complex (624 mg, 5.83 mmol). The mixture was stirred at 40 °C for 2 h. The reaction mixture was concentrated under reduced pressure to remove MeOH. The residue was diluted with saturated aqueous NaHCO₃ (30 mL) and extracted with 3:1 DCM/iPrOH(10 mL x 3). The combined organic layers were washed with brine (20 mL), dried over Na₂SO₄, filtered and concentrated under reduced pressure. The residue was purified by column chromatography (Biotage 12 g cartridge, 0-9% MeOH/DCM) to give 1-(*tert*-butoxycarbonyl)-4-(3-chlorophenethyl)-5-methylpiperazine-2-carboxylic acid. MS = 383.1 [M+H]⁺.

### Step 2: tert-butyl 4-(3-chlorophenethyl)-2-(hydroxymethyl)-5-methylpiperazine-1-carboxylate

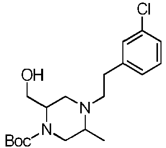

To a solution of 1-(*tert*-butoxycarbonyl)-4-(3-chlorophenethyl)-5-methylpiperazine-2-carboxylic acid (700 mg, 1.83 mmol) in THF (20 mL) was added BH₃-Me₂S (10 M in DMS, 731 uL, 7.3 mmol). The mixture was stirred at room temperature for 2 h. The reaction was poured into MeOH (20 mL) at 0 °C and stirred for 15 min. The mixture was concentrated under reduced pressure. The residue was diluted with saturated aqueous NaHCO₃ (30 mL) and extracted with 3:1 DCM/iPrOH (10 mL x 3). The combined organic layers were washed with brine (20 mL), dried over Na₂SO₄, filtered and concentrated under reduced pressure. The residue was purified by column chromatography (Biotage 12 g cartridge, 2-9% MeOH/DCM) to give *tert-*butyl 4-(3-chlorophenethyl)-2-(hydroxymethyl)-5-methylpiperazine-1-carboxylate. MS = 369.2 [M+H]⁺.

### Step 3: tert-butyl 4-(3-chlorophenethyl)-5-methyl-2-((4-(methylsulfonyl)phenoxy)methyl)piperazine-1-carboxylate

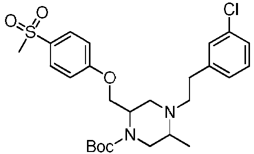

To a solution of *tert*-butyl 4-(3-chlorophenethyl)-2-(hydroxymethyl)-5-methylpiperazine-1-carboxylate (550 mg, 1.49 mmol) and 1-fluoro-4-methylsulfonyl-benzene (312 mg, 1.79 mmol) in DMF (10 mL) was added Cs₂CO₃ (972 mg, 2.98 mmol). The mixture was stirred at 100 °C for 40 h. The reaction mixture was cooled to 0 °C, quenched by addition of saturated aqueous NH₄Cl (50 mL) and extracted with EtOAc (20 mL x 3). The combined organic layers were washed with brine (30 mL), dried over Na₂SO₄, filtered and concentrated under reduced pressure. The residue was purified by column chromatography (Biotage 12 g cartridge, 0-30% EtOAc:Hexane) and further purified by preparative reverse phase HPLC (Phenomenex Luna C18 column, 25-55% MeCN: 0.1% TFA in H₂O) to give *tert-*butyl 4-(3-chlorophenethyl)-5-methyl-2-((4-(methylsulfonyl)phenoxy)methyl)piperazine-1-carboxylate. MS = 523.2 [M+H]⁺.

### Step 4: 1-[2-(3-chlorophenyl)ethyl]-5-[(4-methanesulfonylphenoxy)methyl]-2-methylpiperazine (Compound 211)

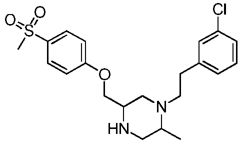

A solution of *tert*-butyl 4-(3-chlorophenethyl)-5-methyl-2-((4-(methylsulfonyl)phenoxy)methyl)piperazine-1-carboxylate (70 mg, 134 µmol) in HCl/MeOH (5 mL) was stirred for 2 h at room temperature. The mixture was concentrated under reduced pressure to give 1-[2-(3-chlorophenyl)ethyl]-5-[(4-methanesulfonylphenoxy)methyl]-2-methylpiperazine **(Compound 211**). ¹H NMR (400 MHz, DMSO-*d*₆): δ 7.92 (d, *J =* 8.8 Hz, 2H), 7.44 - 7.31 (m, 3H), 7.30 - 7.23 (m, 3H), 4.60 - 4.40 (m, 2H), 4.20 - 4.00 (m, 1H), 3.70 - 3.62 (m, 5H), 3.37 - 3.23 (m, 4H), 3.23 - 2.98 (m, 4H), 1.44 (br s, 3H). MS = 423.1 [M+H]⁺.

### Step 5: rac-cis or trans-1-[2-(3-chlorophenyl)ethyl]-5-[(4-methanesulfonylphenoxy)methyl]-2-methylpiperazine (Compound 212) and rac-trans or cis-1-[2-(3-chlorophenyl)ethyl]-5-[(4-methanesulfonylphenoxy)methyl]-2-methylpiperazine (Compound 213)

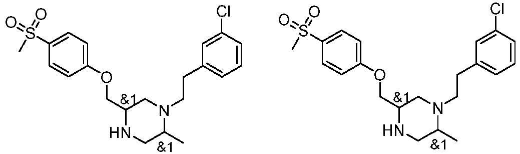

1-(3-chlorophenethyl)-2-methyl-5-((4-(methylsulfonyl)phenoxy)methyl)piperazine (120 mg, 229 µmol) was further purified by chiral SFC (Chiralpak AD, 46% ethanol with 0.1% NH₄OH in CO₂). The first eluting isomer of the title compound **(Compound 212**): ¹H NMR (400 MHz, DMSO-*d*₆): δ 7.87 - 7.82 (m, 2H), 7.31 (s, 1H), 7.29 - 7.14 (m, 5H), 4.10 - 4.01 (m, 2H), 3.16 (s, 3H), 3.10 - 3.02 (m, 1H), 2.78 - 2.53 (m, 9H), 2.35 - 2.17 (m, 1H), 0.95 (d, *J =* 6.4 Hz, 3H). MS = 423.1 [M+H]⁺. The second eluting isomer of the title compound (**Compound 213**): ¹H NMR (400 MHz, DMSO-*d*₆): δ 7.90 (d,*J =* 8.8 Hz, 2H), 7.35 (s, 1H), 7.29 - 7.21 (m, 5H), 4.48 - 4.25 (m, 2H), 3.80 - 3.65 (m, 1H), 3.18 (s, 3H), 3.10 - 3.04 (m, 1H), 2.94 - 2.68 (m, 9H), 1.15 - 1.03 (m, 3H). MS = 423.1 [M+H]⁺.

### Example 30

### (3S,4S)-3-[(4-methanesulfonylphenoxy)methyl]-4-methyl-1-{2-[3-(pentafluoro-λ⁶-sulfanyl)phenyl]ethyl}pyrrolidine (Compound 163)

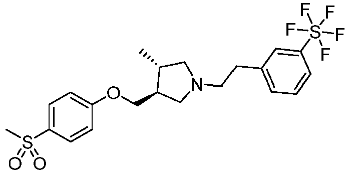

### Step 1: 1-[(3S,4S)-3-(4-methanesulfonylphenoxymethyl)-4-methylpyrrolidin-1-yl]-2-[3-(pentafluoro-λ⁶-sulfanyl)phenyl]ethenone

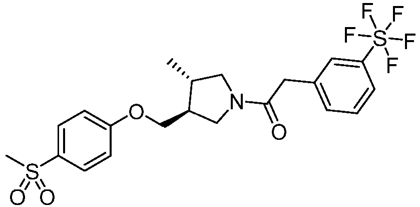

A 20 mL vial was charged with (3*S*,4*S*)-3-(4-methanesulfonylphenoxymethyl)-4-methylpyrrolidine hydrochloride (100 mg, 0.327 mmol), [3-(pentafluoro-λ⁶-sulfanyl)phenyl]acetic acid (0.171 g, 0.654 mmol), EDCI HCl (0.094 g, 0.49 mmol), 1-hydroxybenzotriazole hydrate (0.075 g, 0.49 mmol), and DMF (1.6 mL). Lastly added to this mixture was DIEA (0.127 g, 0.981 mmol). The reaction mixture was stirred at room temperature for 3 h. The reaction mixture was poured into H₂O (20 mL) and extracted with EtOAc (3x 30mL). The combined organic layers were washed with brine, dried over MgSO₄, filtered and concentrated under reduced pressure to give crude 1-[(3*S*,4*S*)-3-(4-methanesulfonylphenoxymethyl)-4-methylpyrrolidin-1-yl]-2-[3-(pentafluoro-λ⁶-sulfanyl)phenyl]ethanone as a yellow oil. MS = 514.0 [M+H]⁺.

### Step 2: (3S,4S)-3-(4-methanesulfonylphenoxymethyl)-4-methyl-1-{2-[3-(pentafluoro-λ⁶-sulfanyl)phenyl]ethyl}pyrrolidine (Compound 163)

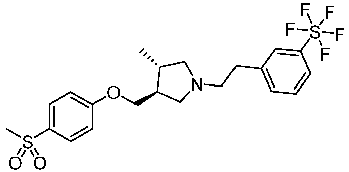

To a solution of 1-[(3*S*,4*S*)-3-(4-methanesulfonylphenoxymethyl)-4-methylpyrrolidin-1-yl]-2-[3-(pentafluoro-λ⁶-sulfanyl)phenyl]ethanone (155 mg, 0.302 mmol) in THF (1.5 mL) at 0 °C was added LiAlH₄ (1 M in THF, 0.905 mL, 0.905 mmol) dropwise. The mixture was stirred at room temperature for 1 h. The reaction was cooled to 0 °C, quenched with H₂O and extracted with DCM (20 mL x2). The combined organics were dried over Na₂SO₄, filtered and concentrated. The resulting crude oil was purified by prep-HPLC (5-90% MeCN in H₂O with 0.1% formic acid modifier) to give (3*S*,4*S*)-3-(4-methanesulfonylphenoxymethyl)-4-methyl-1-{2-[3-(pentafluoro-λ⁶-sulfanyl)phenyl]ethyl}pyrrolidine **(Compound 163**). 1H NMR (500 MHz, CDCl₃) δ 7.82 - 7.78 (m, 2H), 7.57 - 7.54 (m, 1H), 7.54 - 7.52 (m, 1H), 7.36 - 7.30 (m, 2H), 6.97 - 6.92 (m, 2H), 4.03 - 3.94 (m, 2H), 3.26 (dd, *J* = 9.9 Hz, 7.4 Hz, 1H), 3.08 - 2.99 (m, 2H), 2.97 - 2.86 (m, 7H), 2.45 (t, *J* = 9.4 Hz, 1H), 2.34 - 2.25 (m, 1H), 2.23 - 2.14 (m, 1H), 1.12 (d, *J =* 6.7 Hz, 3H).. MS = 500.1 [M+H]⁺.

### Biological Examples

### Example B-1

This example shows that compounds of the present disclosure are able to inhibit calcium transport by APOL1.

A HEK293 clonal cell line was generated to stably express GCaMP6f, a genetically encoded calcium indicator, and inducibly express APOL1 G2 (HEK T-REx/GCaMP6f/APOL1 G2 K6.3). Cells were maintained in the following standard complete medium: DMEM with 4.5 g/L glucose and sodium pyruvate (BioWhittaker, Lonza, BE12-614F), supplemented with 10% FBS Performance Plus (Gibco, 16000044), 1% penicillin-streptomycin (BioWhittaker, DE17-602E), 2 mM ultraglutamine-1 (BioWhittaker cat. BE 17-605/U1), 50 µg/mL Zeocin (InvivoGen, ant-zn), 2.5 µg/mL Blasticidin (InvivoGen, ant-bl-5), and 25 µg/mL Hygromycin (InvivoGen, ant-hg). Standard propagation conditions consisted of plating 9x10⁶, 4x10⁶, 2x10⁶ cells in a T225 flasks to be processed after 2, 3, or 4 days, respectively.

A source plate was generated containing 20 serially diluted compounds in DMSO (duplicate 8-point dose response). Next, 0.8 µL of compounds were transferred from the source plate to a destination plate prefilled with 79.2 µL of Ca²⁺ free Tyrode's buffer (130 mM NaCl, 5 mM KCl, 1 mM MgCl₂, 5 mM NaHCO₃, 20 mM HEPES at pH 7.4). The destination plate was placed on a plate shaker (5 seconds at 2000 rpm) to mix. This process resulted in a destination plate with 2X concentrated compound solutions. All transfer and mixing steps were conducted with an CyBi^{®}-Well dispenser.

Cells were split by gently washing with DPBS (Euroclone, ECB4004L), followed by a 5-minute incubation (humidified, 37°C with 5% CO₂) with trypsin-EDTA solution (Euroclone, ECB3052D). Detached cells were diluted with standard complete medium without selective agents, counted, and plated in a 384 MTP microplate (GR4332CPL, Twin Helix) (10,000 cells/well in 25 µl/well) using a MATRIX WellMate dispenser. Plates were placed into a humidified incubator (37°C with 5% CO₂) overnight. The following day, 20 µL of doxycycline (Sigma, D9891) at 20 ng/mL in standard complete medium was added to cells with a CyBi ^{®}Drop dispenser to induce APOL1 G2 expression. After a 6-hour incubation (humidified, 37°C with 5% CO₂), cells were washed 3 times with Ca²⁺ free Tyrode's Buffer (130 mM NaCl, 5 mM KCl, 1 mM MgCl₂, 5 mM NaHCO₃, 20 mM HEPES at pH 7.4) using a BIOTEK Microplate washer, such that 10 µL of buffer remained in each well after the final wash. Assay plates were then stored at room temperature for 10 minutes. Next, 10µL of diluted compounds were transferred to the assay plate from the 2X compound plate using a CyBi^{®}-Well dispenser. Compound incubation was then carried out at room temperature for 10 minutes. The assay plate was transferred to the FLIProom temperature^{ETRA} and 20 µL of 10 mM Ca²⁺ (final concentration = 5 mM) Tyrode's buffer was injected.

Table B1 below summarizes the data from this experiment. Unless otherwise specified, AC₅₀ and values are reported as the geometric mean of at least 2 assay runs on separate days. Each run represents the average of a technical replicate, where each compound was assayed twice in the same plate. A superscript † symbol indicates a value from the average of a technical replicate from a single assay run, where each compound was assayed twice in the same plate.

The AC₅₀ values in Table B1 below reflect the compound's ability to prevent calcium influx by inhibiting APOL1. As shown in the table, compounds of the present disclosure are able to potently inhibit APOL1-mediated calcium transport at sub micromolar concentrations. Compounds in Table B1 are referred to by the corresponding Compound Number in Table 1, which is also referred to in the synthetic examples. When one or more of the numbered compounds are identified by stereochemistry (for example, (*R*)- or (*S*)-), the specific stereoisomer for which data is provided in Table B1 may be identified by the elution order of such compound as described in the synthetic examples. To illustrate, Compound 2 is the first-eluting enantiomer of step 4 of Example 1 and Compound 3 is the second-eluting enantiomer of step 4 of Example 1. Further, by way of illustration, Compound 27 is the first-eluting enantiomeric mixture in step 4 of Example 7 and Compound 28 is the second-eluting enantiomeric mixture in step 4 of Example 7. Then, Compound 27 is separated into Compound 29 (the first-eluting enantiomer) and Compound 30 (the second-eluting enantiomer) in Example 8, and Compound 28 is separated into Compound 31 (the first-eluting enantiomer) and Compound 32 (the second-eluting enantiomer) in Example 8. Absolute stereochemistry of such compounds may be identified by methods known in the art.

**Table B1**

| Compound No. | APOL1 G2 FLIPR AC₅₀ (µM) |
|---|---|
| 1 | ≤0.253 |
| 2 | 1.01^{†} |
| 3 | 0.205^{†} |
| 4 | 1.07^{†} |
| 5 | 10.1^{†} |
| 6 | 0.942 |
| 7 | <0.253 |
| 8 | 0.266 |
| 9 | 0.151^{†} |
| 10 | 1.72^{†} |
| 11 | 1.19 |
| 12 | 2.39 |
| 13 | 0.143 |
| 14 | 3.34^{†} |
| 15 | 0.287^{†} |
| 16 | 0.231^{†} |
| 17 | 1.37^{†} |
| 18 | 5.73^{†} |
| 19 | 0.48 |
| 20 | 1.76^{†} |
| 21 | 0.623 |
| 22 | 6.41^{†} |
| 23 | 0.64^{†} |
| 24 | 0.501 |
| 25 | 0.469^{†} |
| 26 | 1.54^{†} |
| 27 | 1.50^{†} |
| 28 | 2.00^{†} |
| 29 | 1.98^{†} |
| 30 | 0.864^{†} |
| 31 | 1.02^{†} |
| 32 | 0.731^{†} |
| 33 | 10.8^{†} |
| 34 | 0.991^{†} |
| 35 | 3.78^{†} |
| 36 | 14.2^{†} |
| 37 | 3.84^{†} |
| 38 | <0.253 |
| 39 | 0.249 |
| 40 | <0.118^{†} |
| 41 | 2.11^{†} |
| 42 | 0.477 |
| 43 | 0.237^{†} |
| 44 | 2.22^{†} |
| 45 | 0.0712 |
| 46 | 0.0902 |
| 47 | 0.434 |
| 48 | 1.92^{†} |
| 49 | 0.847 |
| 50 | 1.32^{†} |
| 51 | 0.727 |
| 52 | 0.079^{†} |
| 53 | 0.856^{†} |
| 54 | <0.114 |
| 55 | 1.29^{†} |
| 56 | 0.580^{†} |
| 57 | <5.00 |
| 58 | 0.0727^{†} |
| 59 | 0.245 |
| 60 | <0.267 |
| 61 | 0.376 |
| 62 | 0.396 |
| 63 | 0.637 |
| 64 | 0.570 |
| 65 | 0.258^{†} |
| 66 | 0.468 |
| 67 | 0.34^{†} |
| 68 | 0.916 |
| 69 | 12.87^{†} |
| 70 | 0.295^{†} |
| 71 | 0.943 |
| 72 | 0.314^{†} |
| 73 | 3.06^{†} |
| 74 | 1.29^{†} |
| 75 | 0.558 |
| 76 | 2.83 |
| 77 | 1.67^{†} |
| 78 | 7.01^{†} |
| 79 | 1.29^{†} |
| 80 | 13.9^{†} |
| 81 | >25.0 |
| 82 | 4.35 |
| 83 | >25.0^{†} |
| 84 | >25.0^{†} |
| 85 | 8.48^{†} |
| 86 | 0.557 |
| 87 | 0.352 |
| 88 | 0.338 |
| 89 | 0.449 |
| 90 | 0.515 |
| 91 | 0.472 |
| 92 | 0.682^{†} |
| 93 | 6.54 |
| 94 | 1.62 |
| 95 | 1.54 |
| 96 | 3.77 |
| 97 | 0.314^{†} |
| 98 | 3.92 |
| 99 | 4.14^{†} |
| 100 | 1.26 |
| 101 | 1.30^{†} |
| 102 | 2.19^{†} |
| 103 | 10.9^{†} |
| 104 | <0.382 |
| 105 | 0.946^{†} |
| 106 | 2.79^{†} |
| 107 | 0.0919 |
| 108 | 0.144 |
| 109 | <0.105 |
| 110 | 0.0914 |
| 111 | 0.494 |
| 112 | >0.159 |
| 113 | 0.907 |
| 114 | 0.709 |
| 115 | 0.756 |
| 116 | 0.374 |
| 117 | 1.08 |
| 118 | 0.936 |
| 119 | 0.784 |
| 120 | 0.461 |
| 121 | 0.270 |
| 122 | 0.709 |
| 123 | 0.376 |
| 124 | 0.148 |
| 125 | 2.35 |
| 126 | 0.704 |
| 127 | 2.48 |
| 128 | 0.349 |
| 129 | 1.04 |
| 130 | 2.17 |
| 131 | 0.394 |
| 132 | 0.510 |
| 133 | 0.0383 |
| 134 | 0.277 |
| 135 | >12.5 |
| 136 | 0.207 |
| 137 | >10.0 |
| 138 | 2.50 |
| 139 | >10.0 |
| 140 | 0.107 |
| 141 | 0.737 |
| 142 | 1.23 |
| 143 | 3.16 |
| 144 | 0.423 |
| 145 | 0.356 |
| 146 | 0.937 |
| 147 | 1.11 |
| 148 | 0.615 |
| 149 | 0.0998 |
| 150 | 0.721 |
| 151 | 0.31 |
| 152 | 1.99 |
| 153 | 0.374 |
| 154 | 0.098 |
| 155 | 1.98 |
| 156 | 0.311 |
| 157 | 0.441 |
| 158 | 0.352 |
| 159 | 0.358 |
| 160 | 0.382 |
| 161 | 0.878 |
| 162 | 0.233 |
| 163 | 1.15 |
| 164 | 0.364 |
| 165 | 0.409 |
| 166 | 0.256 |
| 167 | 0.314 |
| 168 | 0.179 |
| 169 | 0.0797 |
| 170 | 0.0375 |
| 171 | 0.212 |
| 172 | 0.104 |
| 173 | 0.491 |
| 174 | 0.157 |
| 175 | <0.0728 |
| 176 | 0.063 |
| 177 | 2.86 |
| 181 | <0.0961 |
| 182 | 0.396 |
| 183 | 0.149 |
| 184 | 0.215 |
| 185 | 0.322 |
| 186 | 6.98 |
| 187 | 0.198 |
| 188 | 0.511 |
| 189 | 0.634 |
| 190 | 0.170 |
| 191 | 0.228 |
| 192 | 0.260 |
| 193 | 1.45 |
| 194 | 0.151 |
| 195 | 0.236 |
| 196 | 0.108 |
| 197 | 0.334 |
| 198 | 0.197 |
| 199 | 0.817 |
| 200 | 0.836 |
| 201 | 0.620 |
| 202 | 0.860 |
| 203 | 9.30 |
| 204 | 0.456 |
| 205 | <0.236 |
| 206 | >25.0 |
| 207 | 0.340 |
| 208 | <0.420 |
| 209 | 3.74 |
| 210 | 0.898 |
| 211 | <0.118 |
| 212 | 2.55 |
| 213 | 0.877 |
| 214 | 0.428 |
| 215 | 0.229 |

### Example B-2

This example shows that the compounds of the present disclosure are able to reduce cell death caused by overexpression of APOL1.

A HEK293 clonal cell line overexpressing APOL1 G2 (HEK293/T-REx APOL1 G2/clone #2) was maintained in 1x DMEM-GlutaMax (Gibco, 10569-010) media with 10% tetracycline-free FBS (Takara Bio USA, 631101), 5 µg/mL Blasticidin (Gibco, A1113903), and 100 µg/mL Zeocin (Invitrogen, R25001) in T75 flasks. In preparation for the assay, this media was aspirated and 2 mL of prewarmed TrypLE Express (Gibco, 12605-010) was added to a flask to detach cells. The flask was then incubated (humidified, 37°C with 5% CO2) for 3-5 minutes. Afterwards, 8 mL of prewarmed cell assay media (1x DMEM-GlutaMax media with 10% tetracycline-free FBS) was added to the trypsinized cells. The suspension was gently mixed, and cells were counted using a Countess Cell Counting Chamber (Invitrogen). The suspension was diluted using cell assay media to generate a working stock solution (166,667 cells/mL). Using a MultiDrop Combi (Thermo Electron Corp), 30 µL (final cell density = 5,000 cells/well) of the working stock solution was dispensed into each well of white 384-well assay ready plates (Nunc^{™}, 164610) containing 6 ng/mL doxycycline, to induce APOL1 expression, and compound. All compounds were plated in a duplicate 8-point dilution series that consisted of 3-fold stepwise dilutions (0.5% DMSO final). Assay plates were incubated (humidified, 37°C with 5% CO2) for 17 hours. After the incubation, the plates were equilibrated at room temperature for 1 hour. Next, 15 µl of CellTiter-Glo^{®} reagent (Promega, G7570) was added to each well using a MultiDrop Combi. Plates were placed on an orbital shaker (500 rpm) for 5 minutes to induce cell lysis and then incubated at room temperature for 10 minutes. Luminescence was measured on an Envision plate reader. Collaborative Drug Discovery software was utilized for graphing data. Plots were generated using a four parameter logistic curve fit.

Table B2 below provides the results from this experiment. Unless otherwise specified, EC₅₀ values are reported as the geometric mean of at least 2 assay runs on separate days. Each run represents the average of a technical replicate, where each compound was assayed twice in the same plate. A superscript † symbol indicates a value from the average of a technical replicate from a single assay run, where each compound was assayed twice in the same plate. Compounds in Table B2 are referred to by the corresponding Compound Number in Table 1, which is also referred to in the synthetic examples. When one or more of the numbered compounds are identified by stereochemistry (for example, (R)- or (S)-), the specific stereoisomer for which data is provided in Table B2 may be identified by the elution order of such compound as described in the synthetic examples. To illustrate, Compound 2 is the first-eluting enantiomer of step 4 of Example 1 and Compound 3 is the second-eluting enantiomer of step 4 of Example 1. Further, by way of illustration, Compound 27 is the first-eluting enantiomeric mixture in step 4 of Example 7 and Compound 28 is the second-eluting enantiomeric mixture in step 4 of Example 7. Then, Compound 27 is separated into Compound 29 (the first-eluting enantiomer) and Compound 30 (the second-eluting enantiomer) in Example 8, and Compound 28 is separated into Compound 31 (the first-eluting enantiomer) and Compound 32 (the second-eluting enantiomer) in Example 8. Absolute stereochemistry of such compounds may be identified by methods known in the art.

Rescue EC₅₀ values reported in Table B2 below represent the half-maximal effective concentration for reversal of cell death caused by overexpression of APOL1. This example demonstrates that compounds of the present disclosure are able to reduce cell death caused by overexpression of APOL1 at sub micromolar concentration.

**Table B2**

| Compound No. | APOL1 G2 HEK293 Rescue EC₅₀ (µM) |
|---|---|
| 1 | 0.528 |
| 2 | 2.96 |
| 3 | 0.295 |
| 4 | 2.35 |
| 5 | 5.37 |
| 6 | 0.495 |
| 7 | 0.322 |
| 8 | 0.132 |
| 9 | 0.263 |
| 10 | 2.45 |
| 11 | 1.00 |
| 12 | 3.00 |
| 13 | 0.445 |
| 14 | >30.0 |
| 15 | 0.567^{†} |
| 16 | 0.329^{†} |
| 17 | 0.671 |
| 18 | 6.32 |
| 19 | 0.374 |
| 20 | 1.36 |
| 21 | 1.02 |
| 22 | 6.59 |
| 23 | 0.677 |
| 24 | 0.334 |
| 25 | 1.01 |
| 26 | 2.24 |
| 27 | 2.13 |
| 28 | 2.34 |
| 29 | >12.2 |
| 30 | 2.41 |
| 31 | >7.89 |
| 32 | 5.77 |
| 33 | >8.67 |
| 34 | 0.779 |
| 35 | 6.13 |
| 36 | >30 |
| 37 | >6.15 |
| 38 | 0.407 |
| 39 | 0.904 |
| 40 | 0.746 |
| 41 | 2.82 |
| 42 | 0.059 |
| 43 | 0.031 |
| 44 | 2.43 |
| 45 | <0.0137 |
| 46 | 0.021 |
| 47 | 0.516 |
| 48 | 2.54 |
| 49 | 0.957 |
| 50 | 2.02 |
| 51 | 1.49 |
| 52 | 0.124 |
| 53 | 1.12 |
| 54 | >30 |
| 55 | 1.68 |
| 56 | 1.68 |
| 57 | 1.68 |
| 58 | <0.041 |
| 59 | 1.06 |
| 60 | 0.306 |
| 61 | 0.933 |
| 62 | 0.642 |
| 63 | 3.96^{†} |
| 64 | 1.12^{†} |
| 65 | 0.955 |
| 66 | 1.38^{†} |
| 67 | 1.35 |
| 68 | 0.591 |
| 69 | >30 |
| 70 | 0.303 |
| 71 | 1.96 |
| 72 | 1.22 |
| 73 | >17.8 |
| 74 | 2.58 |
| 75 | 1.46 |
| 76 | >6.28 |
| 77 | >6.30 |
| 78 | >30.0 |
| 79 | >13.7 |
| 80 | 13.0 |
| 81 | >30.0 |
| 82 | >24.3 |
| 83 | >30.0 |
| 84 | >30.0 |
| 85 | >18.0 |
| 86 | >4.11 |
| 87 | 1.85 |
| 88 | 0.698 |
| 89 | 2.02 |
| 90 | 1.46 |
| 91 | 1.04 |
| 92 | >9.79 |
| 93 | >30 |
| 94 | 8.37 |
| 95 | 6.90 |
| 96 | >30.0 |
| 97 | 0.46 |
| 98 | >20 |
| 99 | >30.0 |
| 100 | 2.73 |
| 101 | 10.1 |
| 102 | >21.8 |
| 103 | >30.0 |
| 104 | 1.86 |
| 105 | >12.4 |
| 106 | 6.24 |
| 107 | 0.0809 |
| 108 | 0.0504 |
| 109 | 0.500 |
| 110 | 0.135 |
| 111 | 1.88 |
| 112 | 0.0298 |
| 113 | 1.07 |
| 114 | 0.439 |
| 115 | 0.0447 |
| 116 | 0.076 |
| 117 | 1.24 |
| 118 | 4.04 |
| 119 | 0.133 |
| 120 | 0.195 |
| 121 | 0.100 |
| 122 | 1.34 |
| 123 | 0.300 |
| 124 | 0.242 |
| 125 | 1.55 |
| 126 | 0.560 |
| 127 | > 10.0 |
| 128 | 0.366 |
| 129 | 0.723 |
| 130 | > 10.0 |
| 131 | 0.460 |
| 132 | 0.0437 |
| 133 | 0.135 |
| 134 | 0.908 |
| 135 | 5.73 |
| 136 | 0.11 |
| 137 | 4.62 |
| 138 | 1.97 |
| 139 | 3.23 |
| 140 | 0.0459 |
| 141 | 0.0789 |
| 142 | 0.131 |
| 143 | 0.349 |
| 144 | 0.256 |
| 145 | 0.243 |
| 146 | 0.273 |
| 147 | 0.618 |
| 148 | 0.0808 |
| 149 | 0.272 |
| 150 | 1.47 |
| 151 | 1.34 |
| 152 | 4.72 |
| 153 | 0.0278 |
| 154 | 0.167 |
| 155 | 2.49 |
| 156 | 0.178 |
| 157 | 0.0389 |
| 158 | 0.039 |
| 159 | 0.0249 |
| 160 | 0.584 |
| 161 | 0.191 |
| 162 | 0.0279 |
| 163 | > 0.385 |
| 164 | 0.0341 |
| 165 | 0.0302 |
| 166 | 0.0179 |
| 167 | 0.0672 |
| 168 | 0.126 |
| 169 | 0.182 |
| 170 | 0.076 |
| 171 | 0.146 |
| 172 | 0.203 |
| 173 | 0.201 |
| 174 | 0.0361 |
| 175 | 0.143 |
| 176 | < 0.0472 |
| 177 | 1.83 |
| 181 | 0.325 |
| 182 | 0.399 |
| 183 | 0.157 |
| 184 | 0.622 |
| 185 | 0.239 |
| 186 | 0.631 |
| 187 | 0.436 |
| 188 | 0.303 |
| 189 | 0.922 |
| 190 | 0.291 |
| 191 | 0.663 |
| 192 | 0.915 |
| 193 | 0.782 |
| 194 | 0.107 |
| 195 | 0.226 |
| 196 | 0.110 |
| 197 | 0.369 |
| 198 | 0.0715 |
| 199 | 2.44 |
| 200 | 1.99 |
| 201 | 1.68 |
| 202 | 0.089 |
| 203 | 3.54 |
| 204 | 0.0423 |
| 205 | 1.59 |
| 206 | > 30.0 |
| 207 | 0.933 |
| 208 | 3.57 |
| 209 | > 19.5 |
| 210 | 3.12 |
| 211 | 1.36 |
| 212 | > 14.3 |
| 213 | 0.964 |
| 214 | 0.679 |
| 215 | 0.0422 |

It is to be understood that, while the disclosure has been described in conjunction with the above embodiments, the foregoing description and examples are intended to illustrate and not limit the scope of the disclosure. Other aspects, advantages and modifications within the scope of the disclosure and as defined by the claims will be apparent to those skilled in the art to which the disclosure pertains.

## Claims

1. A compound of formula (A'): 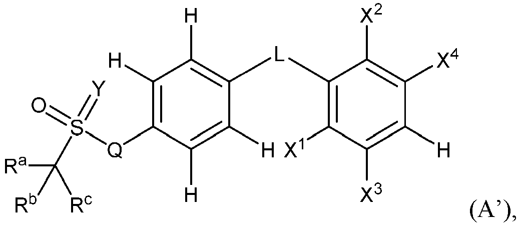 or a stereoisomer or a tautomer thereof, or a pharmaceutically acceptable salt of any of the foregoing, wherein:
Q is absent or is -N-(C₁₋₆alkyl);
Y is O or -N-(C₁₋₆alkyl),
provided that, when Q is -N(C₁₋₆alkyl), then Y is O;
R^{a}, R^{b}, and R^{c} are each independently H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl,
or any two of R^{a}, R^{b}, and R^{c} are taken, together with the atoms to which they are attached, to form a C₃₋₆cycloalkyl or a 3-6 membered heterocyclyl, and the other of R^{a}, R^{b}, and R^{c} is H or C₁₋₆alkyl, wherein the C₁₋₆alkyl of R^{a}, R^{b}, or R^{c} is independently optionally substituted with one or more -OH, C₁₋₆alkoxy, or -S(O)₂-C₁₋₆alkyl;
L is selected from the group consisting of:
(i) 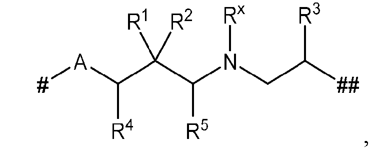 wherein
A is O, NH, N(C₁₋₆alkyl), CH₂, or CH(C₁₋₆alkyl);
R^{x} is H,
or R^{x} is taken together with one of R¹ and R², and the atoms to which they are attached, to form a 3-8 membered heterocyclyl, wherein the 3-8 membered heterocyclyl is substituted with *n* independently selected R^{g} substituents, wherein *n* is an integer from 0-6, and R^{g} is -OH, halo, C₁₋₆alkyl, or C₁₋₆alkoxy;
R¹ and R² are independently H, halo, or -OH,
or one of R¹ and R² is taken together with R^{x}, and the atoms to which they are attached, to form a 3-8 membered heterocyclyl, wherein the 3-8 membered heterocyclyl is substituted with *n* independently selected R^{g} substituents, wherein *n* is an integer from 0-6, and R^{g} is -OH, halo, C₁₋₆alkyl, or C₁₋₆alkoxy, and the other of R¹ and R² is H, halo, or -OH;
R³ is H, -OH, halo, or C₁₋₆alkoxy; and
R⁴ and R⁵ are independently H,
or R⁴ and R⁵ are taken, together with the atoms to which they are attached, to form a C₃₋₈cycloalkyl,
provided that either:
(1) R^{x} is taken together with one of R¹ and R², and the atoms to which they are attached, to form a 3-8 membered heterocyclyl, wherein the 3-8 membered heterocyclyl is substituted with *n* independently selected R^{g} substituents, wherein *n* is an integer from 0-6, and R^{g} is -OH, halo, C₁₋₆alkyl, or C₁₋₆alkoxy, or
(2) R⁴ and R⁵ are taken, together with the atoms to which they are attached, to form a C₃₋₈cycloalkyl,
(ii) 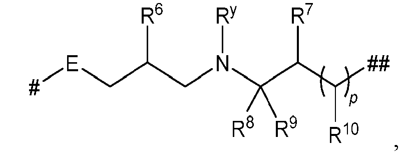 wherein
E is O, NH, N(C₁₋₆alkyl), CH₂, or CH(C₁₋₆alkyl);
*p* is 0 or 1,
provided that, when *p* is 1, then E is O;
R⁶ is H or -OH;
R^{y} is H,
or R^{y} is taken together with R⁷, and the atoms to which they are attached, to form a 3-8 membered heterocyclyl,
or R^{y} is taken together with one of R⁸ and R⁹, and the atoms to which they are attached, to form a 3-8 membered heterocyclyl;
R⁷ is H,
or R⁷ is taken together with R^{y}, and the atoms to which they are attached, to form a 3-8 membered heterocyclyl;
R⁸ and R⁹ are independently H or C₁₋₆alkyl,
or one of R⁸ and R⁹ is taken together with R^{y}, and the atoms to which they are attached, to form a 3-8 membered heterocyclyl, and the other of R⁸ and R⁹ is H or C₁₋₆alkyl,
or one of R⁸ and R⁹ is taken together with R¹⁰, and the atoms to which they are attached, to form a C₃₋₈cycloalkyl, and the other of R⁸ and R⁹ is H or C₁₋₆alkyl; and
R¹⁰ is H,
or R¹⁰ is taken together with one of R⁸ and R⁹, and the atoms to which they are attached, to form a C₃₋₈cycloalkyl,
provided that:
(1) R^{y} is taken together with R⁷, and the atoms to which they are attached, to form a 3-8 membered heterocyclyl, or
(2) R^{y} is taken together with one of R⁸ and R⁹, and the atoms to which they are attached, to form a 3-8 membered heterocyclyl, or
(3) one of R⁸ and R⁹ is taken together with R¹⁰ and the atoms to which they are attached, to form a C₃₋₈cycloalkyl, and
(iii) 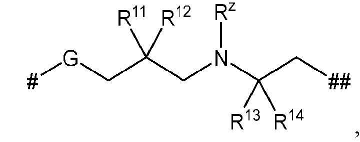 wherein
G is O, NH, N(C₁₋₆alkyl), CH₂, or CH(C₁₋₆alkyl);
R^{z} is H or C₁₋₆alkyl, wherein the C₁₋₆alkyl is optionally substituted with one or more C₃₋₈cycloalkyl;
R¹¹ and R¹² are independently H, -OH, halo, or C₁₋₆alkyl; and
R¹³ and R¹⁴ are independently H, C₁₋₆alkyl, or C₃₋₈cycloalkyl,
or R¹³ and R¹⁴ are taken, together with the atoms to which they are attached, to form a 3-8 membered heterocyclyl,
wherein, for each of (i)-(iii), # denotes the point of attachment to the phenyl ring bearing moiety Q, and ## denotes the point of attachment to the phenyl ring bearing moieties X¹-X⁴; and
X¹, X², X³, and X⁴ are, independently of each other, H, halo, -CN, C₁₋₆alkyl, C₁₋₆alkoxy, or SF₅, wherein the C₁₋₆alkyl or C₁₋₆alkoxy is optionally substituted with one or more halo,
provided that at least one of X¹, X², X³, and X⁴ is halo, -CN, C₁₋₆alkyl, C₁₋₆alkoxy, or SF₅, wherein the C₁₋₆alkyl or C₁₋₆alkoxy is optionally substituted with one or more halo.

2. The compound of claim 1, wherein the compound is a compound of formula (A): 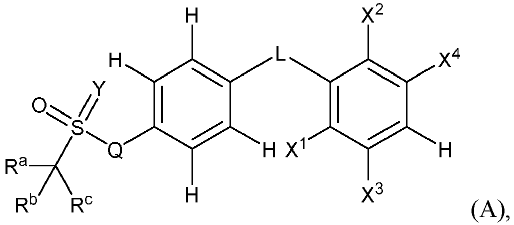 or a stereoisomer or a tautomer thereof, or a pharmaceutically acceptable salt of any of the foregoing, wherein:
X¹, X², X³, and X⁴ are, independently of each other, H, halo, -CN, C₁₋₆alkyl, or C₁₋₆alkoxy, wherein the C₁₋₆alkyl or C₁₋₆alkoxy is optionally substituted with one or more halo,
provided that at least one of X¹, X², X³, and X⁴ is halo, -CN, C₁₋₆alkyl, or C₁₋₆alkoxy, wherein the C₁₋₆alkyl or C₁₋₆alkoxy is optionally substituted with one or more halo.

3. The compound of claim 1 or claim 2, or a stereoisomer or tautomer thereof, or a pharmaceutically acceptable salt of any of the foregoing, wherein L is 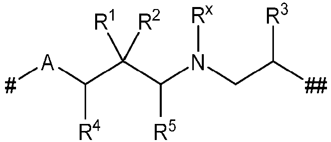 such that the compound is of formula (I): 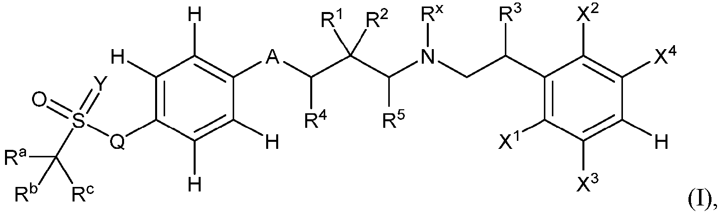
or a stereoisomer or tautomer thereof, or a pharmaceutically acceptable salt of any of the foregoing;
optionally wherein one of (a) or (b) applies:
(a) A is O, such that the compound is of formula (I-A): 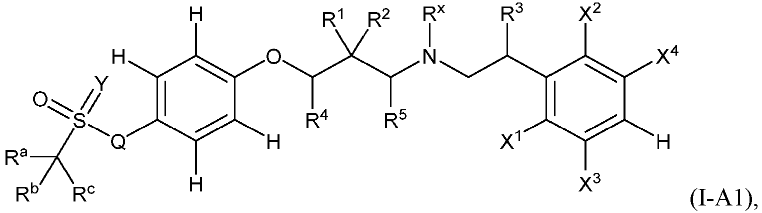 or a stereoisomer or tautomer thereof, or a pharmaceutically acceptable salt of any of the foregoing;
(b) A is CH₂, such that the compound is of formula (I-A3): 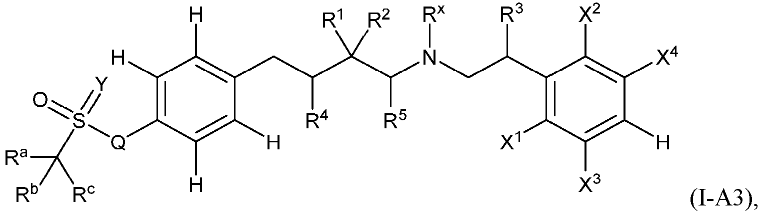
or a stereoisomer or tautomer thereof, or a pharmaceutically acceptable salt of any of the foregoing.

4. The compound of claim 3, or a stereoisomer or tautomer thereof, or a pharmaceutically acceptable salt of any of the foregoing, wherein the compound is of formula (I-A1) and either:
R^{x} is taken together with one of R¹ and R², and the atoms to which they are attached, to form a 3-8 membered heterocyclyl, wherein the 3-8 membered heterocyclyl is substituted with *n* independently selected R^{g} substituents, wherein *n* is an integer from 0-6, and R^{g} is -OH, halo, C₁₋₆alkyl, or C₁₋₆alkoxy; or
R^{x} is taken together with one of R¹ and R², and the atoms to which they are attached, to form a 5-6 membered heterocyclyl, wherein the 5-6 membered heterocyclyl is substituted with *n* independently selected R^{g} substituents, wherein n is an integer from 0-6, and R^{g} is -OH, halo, C₁₋₆alkyl, or C₁₋₆alkoxy.

5. The compound of claim 4, or a stereoisomer or tautomer thereof, or a pharmaceutically acceptable salt of any of the foregoing, wherein the compound is of formula (I-B 1), (I-C1), (I-D1), (I-E1), (I-F1) or (I-G1): 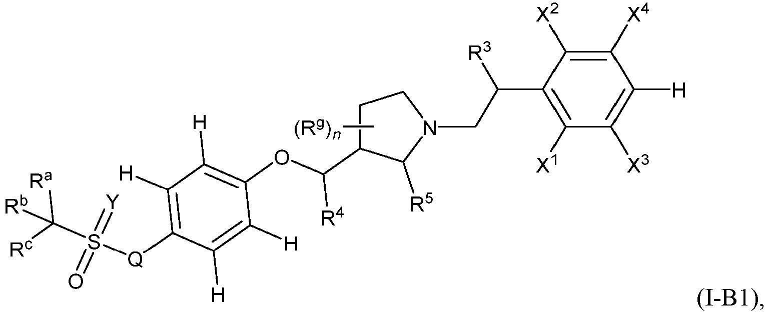
wherein in formula (I-B1), *n* is optionally 0, or *n* is optionally 1 or 2; 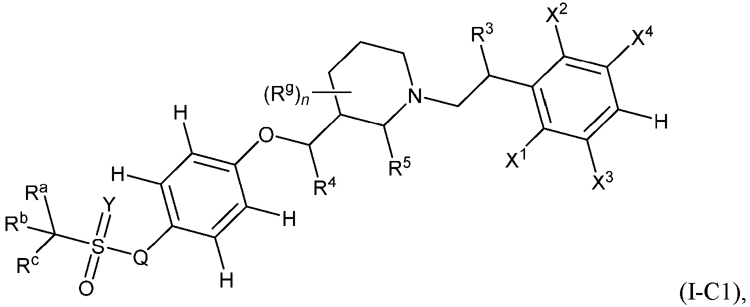
wherein in formula (I-C1) *n* is optionally 0, or *n* is optionally 1 or 2; 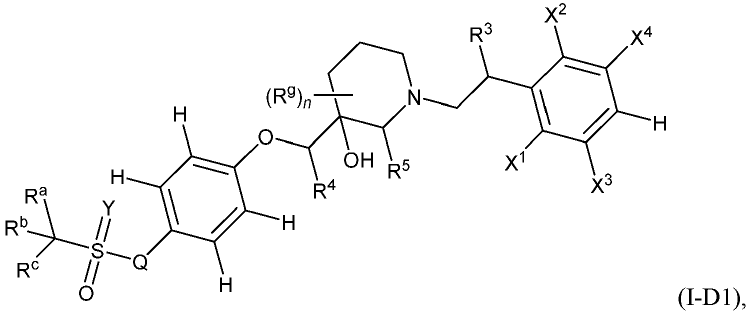
wherein in formula (I-D1) *n* is optionally 0, or *n* is optionally 1 or 2; 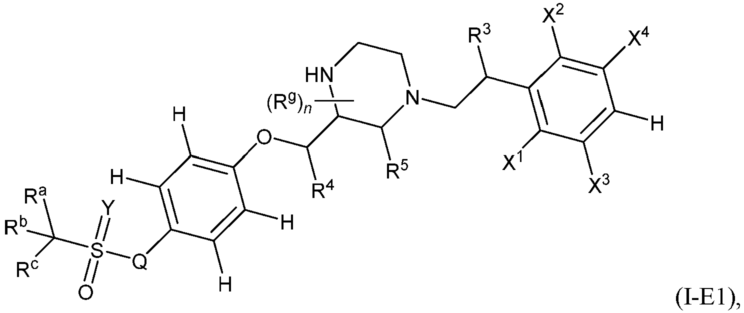
wherein in formula (I-E1) *n* is optionally 0; 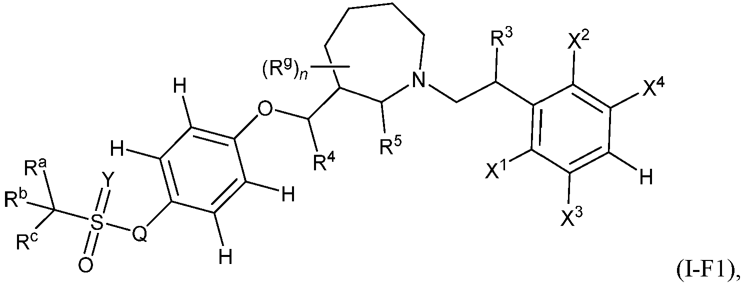
wherein in formula (I-F1) *n* is optionally 0; 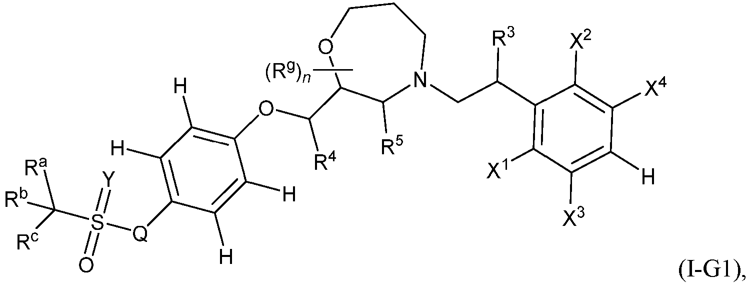
wherein in formula (I-G1) *n* is optionally 0;
or a stereoisomer or tautomer thereof, or a pharmaceutically acceptable salt of any of the foregoing.

6. The compound of claim 3, or a stereoisomer or tautomer thereof, or a pharmaceutically acceptable salt of any of the foregoing, the compound is of formula (I-A) and wherein R⁴ and R⁵ are taken, together with the atoms to which they are attached, to form a C₃₋₈cycloalkyl; optionally wherein the compound is of formula (I-H1): 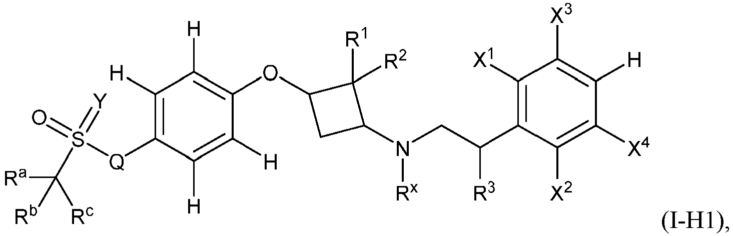
or a stereoisomer or tautomer thereof, or a pharmaceutically acceptable salt of any of the foregoing;
wherein R¹, R², and R³ are optionally each H.

7. The compound of claim 3, or a stereoisomer or tautomer thereof, or a pharmaceutically acceptable salt of any of the foregoing, wherein the compound is of formula (I-A1) and either:
(a) R^{x} is taken together with one of R¹ and R², and the atoms to which they are attached, to form a 3-8 membered heterocyclyl, wherein the 3-8 membered heterocyclyl is substituted with *n* independently selected R^{g} substituents, wherein *n* is an integer from 0-6, and R^{g} is -OH, halo, C₁₋₆alkyl, or C₁₋₆alkoxy, and the other of R¹ and R² is H, halo, or -OH; or
(b) R^{x} is taken together with one of R¹ and R², and the atoms to which they are attached, to form a 5-6 membered heterocyclyl, wherein the 5-6 membered heterocyclyl is substituted with *n* independently selected R^{g} substituents, wherein *n* is an integer from 0-6, and R^{g} is -OH, halo, C₁₋₆alkyl, or C₁₋₆alkoxy, and the other of R¹ and R² is H, halo, or -OH;
optionally wherein the compound is of formula (I-C3): 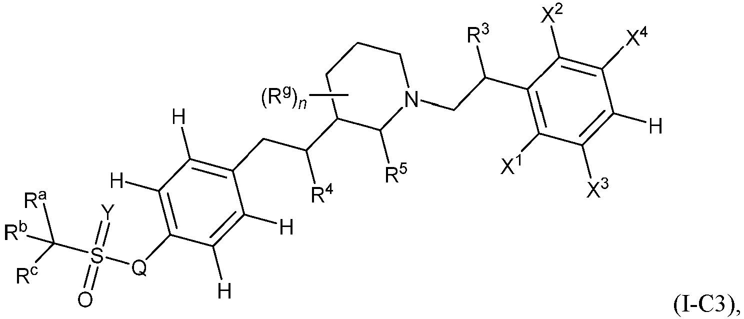
or a stereoisomer or tautomer thereof, or a pharmaceutically acceptable salt of any of the foregoing.

8. The compound of claim 1 or claim 2, or a stereoisomer or tautomer thereof, or a pharmaceutically acceptable salt of any of the foregoing, wherein L is 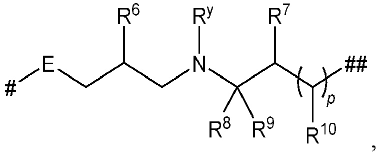 such that the compound is of formula (II): 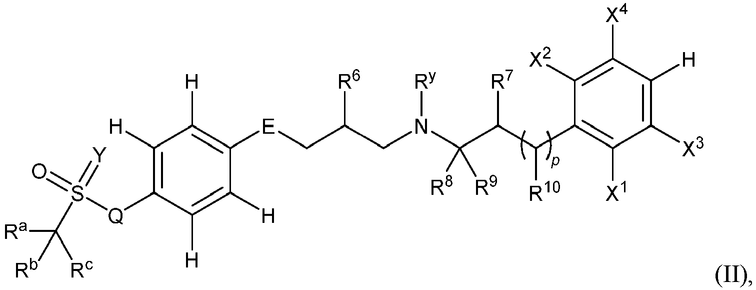
or a stereoisomer or tautomer thereof, or a pharmaceutically acceptable salt of any of the foregoing;
optionally wherein E is O, such that the compound is of formula (II-A1): 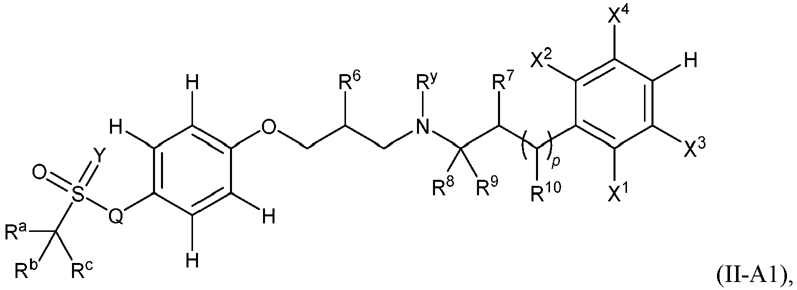
or a stereoisomer or tautomer thereof, or a pharmaceutically acceptable salt of any of the foregoing;
optionally wherein either:
(a) R^{y} in formula (II-A1) is taken together with R⁷, and the atoms to which they are attached, to form a 3-8 membered heterocyclyl; or
(b) R^{y} is taken together with R⁷, and the atoms to which they are attached, to form a 5-6 membered heterocyclyl;
further optionally wherein one of R⁸ and R⁹ is taken together with R¹⁰, and the atoms to which they are attached, to form a C₃₋₈cycloalkyl.

9. The compound of claim 8, or a stereoisomer or tautomer thereof, or a pharmaceutically acceptable salt of any of the foregoing, wherein the compound is of formula (II-B1), (II-C1), (II-D1) or (II-E1): 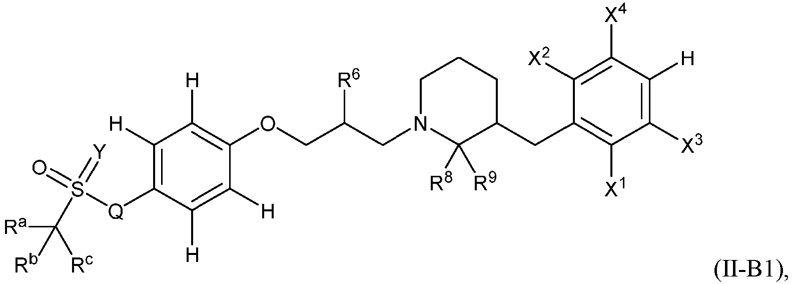 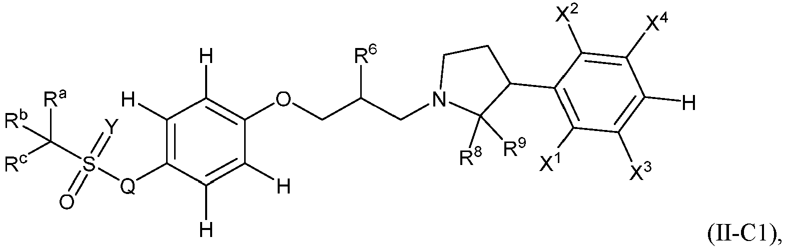 wherein in formula (II-C1) optionally R⁶ is -OH, R⁸ is C₁₋₆alkyl, and R⁹ is C₁₋₆alkyl; 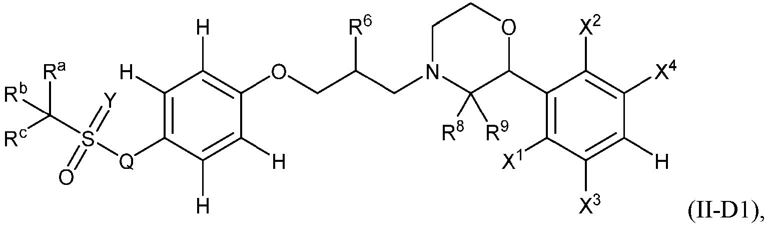 wherein in formula (II-D1) optionally R⁶ is -OH, R⁸ is H, and R⁹ is H; 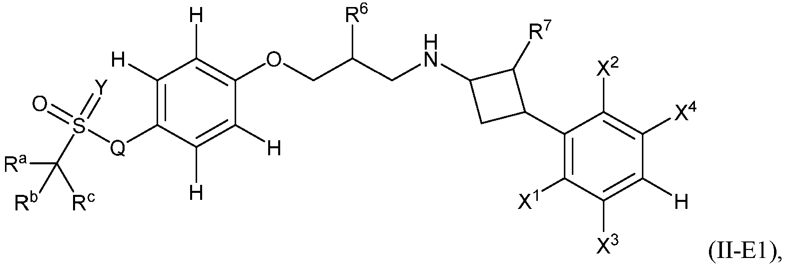 wherein in formula (II-E1) optionally R⁶ is -OH and R⁷ is H;
or a stereoisomer or tautomer thereof, or a pharmaceutically acceptable salt of any of the foregoing.

10. The compound of claim 1 or claim 2, or a stereoisomer or tautomer thereof, or a pharmaceutically acceptable salt of any of the foregoing, wherein L is 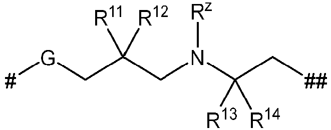 such that the compound is of formula (III): 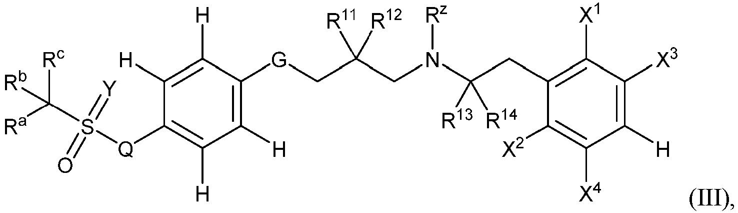 or a stereoisomer or tautomer thereof, or a pharmaceutically acceptable salt of any of the foregoing.

11. The compound of claim 1 or claim 2, or a stereoisomer or tautomer thereof, or a pharmaceutically acceptable salt of any of the foregoing, wherein either:
(a) Q is absent, Y is O, and R^{a}, R^{b}, and R^{c} are each independently H, such that the compound is of formula (B-2): 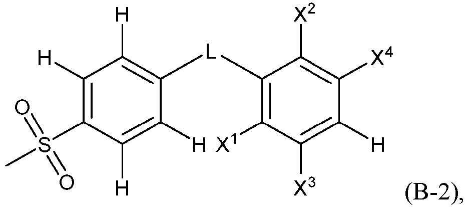 or a stereoisomer or tautomer thereof, or a pharmaceutically acceptable salt of any of the foregoing; or
(b) Q is absent, Y is -N(CH₃), and R^{a}, R^{b}, and R^{c} are each independently H, such that the compound is of formula (B-5): 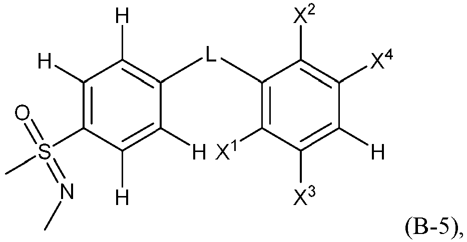 or a stereoisomer or tautomer thereof, or a pharmaceutically acceptable salt of any of the foregoing; or
(c) Q is -N(CH₃), Y is O, and R^{a}, R^{b}, and R^{c} are each independently H, such that the compound is of formula (C-1): 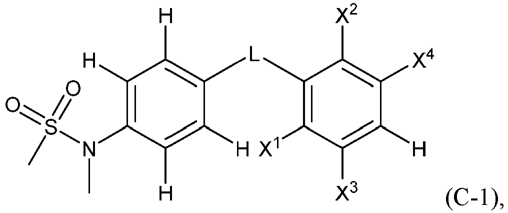 or a stereoisomer or tautomer thereof, or a pharmaceutically acceptable salt of any of the foregoing.

12. The compound of claim 1 or claim 2, or a stereoisomer or tautomer thereof, or a pharmaceutically acceptable salt of any of the foregoing, wherein the compound is selected from the following:
| Compound No. | Structure | Chemical name |
|---|---|---|
| 1 | 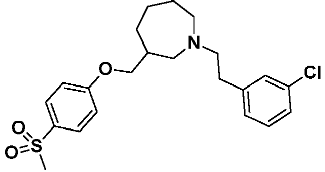 | 1-(3-chlorophenethyl)-3-((4-(methylsulfonyl)phenoxy)methyl) azepane |
| 2 | 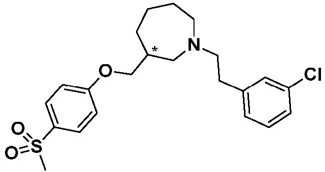 | (*R*) or (*S*)-1-(3-chlorophenethyl)-3-((4-(methylsulfonyl)phenoxy)methyl) azepane |
| 3 | 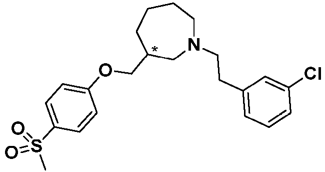 | (*S*) or (*R*)-1-(3-chlorophenethyl)-3-((4-(methylsulfonyl)phenoxy)methyl) azepane |
| 4 | 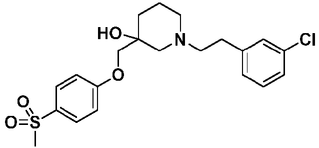 | 1-(3-chlorophenethyl)-3-((4-(methylsulfonyl)phenoxy)methyl) piperidin-3-ol |
| 5 | 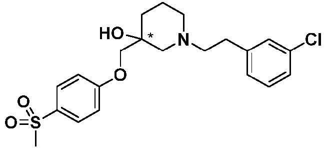 | (*R*) or (*S*)-1-(3-chlorophenethyl)-3-((4-(methylsulfonyl)phenoxy)methyl) piperidin-3-ol |
| 6 | 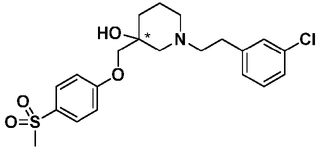 | (*S*) or (*R*) -1-(3-chlorophenethyl)-3-((4-(methylsulfonyl)phenoxy)methyl) piperidin-3-ol |
| 7 | 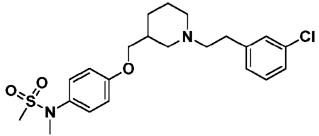 | *N*-(4-((1-(3-chlorophenethyl)piperidin-3-yl)methoxy)phenyl)-*N-*methylmethanesulfonamide |
| 8 | 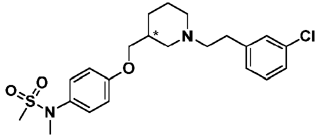 | (*S*) or (*R*)-*N*-(4-((1-(3-chlorophenethyl)piperidin-3-yl)methoxy)phenyl)-*N-*methylmethanesulfonamide |
| 9 | 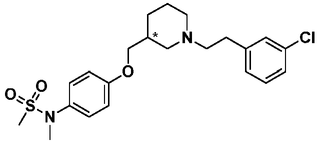 | (*R*) or (*S*)-*N*-(4-((1-(3-chlorophenethyl)piperidin-3-yl)methoxy)phenyl)-*N-*methylmethanesulfonamide |
| 10 | 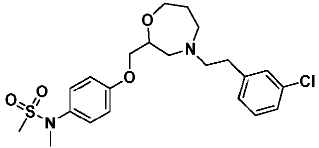 | *N*-(4-((4-(3-chlorophenethyl)-1,4-oxazepan-2-yl)methoxy)phenyl)-*N*-methylmethanesulfonamide |
| 11 | 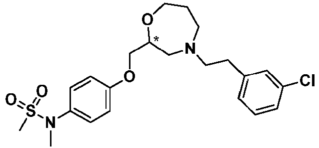 | (*R*) or (*S*)-*N*-(4-((4-(3-chlorophenethyl)-1,4-oxazepan-2-yl)methoxy)phenyl)-*N-*methylmethanesulfonamide |
| 12 | 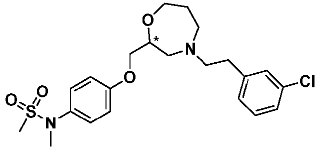 | (*S*) or (*R*)-*N*-(4-((4-(3-chlorophenethyl)-1,4-oxazepan-2-yl)methoxy)phenyl)-*N-*methylmethanesulfonamide |
| 13 | 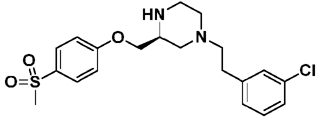 | (*S*)-1-(3-chlorophenethyl)-3-((4-(methylsulfonyl)phenoxy)methyl) piperazine |
| 14 | 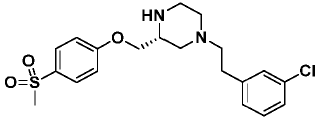 | (*R*)-1-(3-chlorophenethyl)-3-((4-(methylsulfonyl)phenoxy)methyl) piperazine |
| 15 | 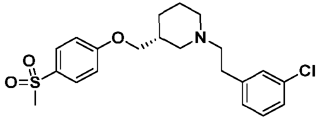 | (*R*)-1-(3-chlorophenethyl)-3-((4-(methylsulfonyl)phenoxy)methyl) piperidine |
| 16 | 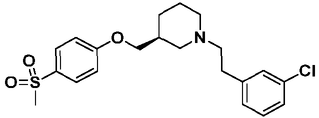 | (*S*)-1-(3-chlorophenethyl)-3-((4-(methylsulfonyl)phenoxy)methyl) piperidine |
| 17 | 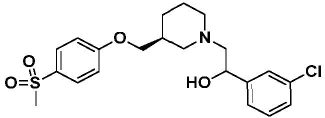 | 1-(3-chlorophenyl)-2-((*S*)-3-((4-(methylsulfonyl)phenoxy)methyl) piperidin-1-yl)ethan-1-ol |
| 18 | 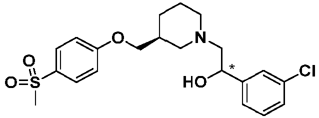 | (*R*) or (*S*)-1-(3-chlorophenyl)-2-((*S*)-3-((4-(methylsulfonyl)phenoxy)methyl) piperidin-1-yl)ethan-1-ol |
| 19 | 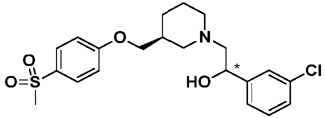 | (*S*) or (*R*)-1-(3-chlorophenyl)-2-((*S*)-3-((4-(methylsulfonyl)phenoxy)methyl) piperidin-1-yl)ethan-1-ol |
| 20 | 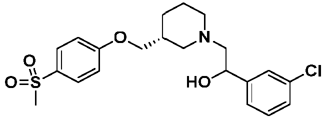 | 1-(3-chlorophenyl)-2-((*R*)-3-((4-(methylsulfonyl)phenoxy)methyl) piperidin-1-yl)ethanol |
| 21 | 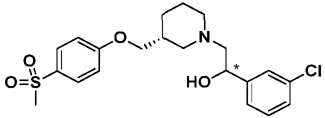 | (*R*) or (*S*)-1-(3-chlorophenyl)-2-((*R*)-3-((4-(methylsulfonyl)phenoxy)methyl) piperidin-1-yl)ethanol |
| 22 | 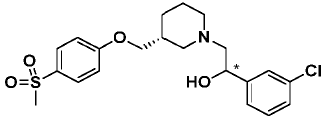 | (*S*) or (*R*)-1-(3-chlorophenyl)-2-((*R*)-3-((4-(methylsulfonyl)phenoxy)methyl) piperidin-1-yl)ethanol |
| 23 | 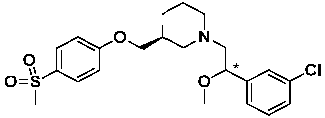 | (*S*)-1-((*R*) or (*S*)-2-(3-chlorophenyl)-2-methoxyethyl)-3-((4-(methylsulfonyl)phenoxy)methyl) piperidine |
| 24 | 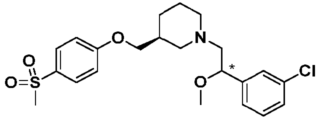 | (*S*)-1-((*S*) or (*R*)-2-(3-chlorophenyl)-2-methoxyethyl)-3-((4-(methylsulfonyl)phenoxy)methyl) piperidine |
| 25 | 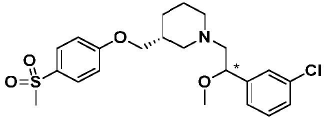 | (*R*)-1-((*R*) or (*S*)-2-(3-chlorophenyl)-2-methoxyethyl)-3-((4-(methylsulfonyl)phenoxy)methyl) piperidine |
| 26 | 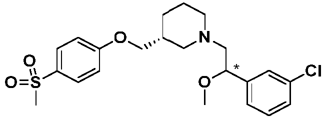 | (*R*)-1-((*S*) or (*R*)-2-(3-chlorophenyl)-2-methoxyethyl)-3-((4-(methylsulfonyl)phenoxy)methyl) piperidine |
| 27 | 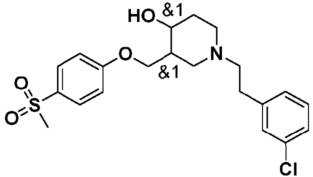 | *rac-trans or cis*-1-(3-chlorophenethyl)-3-((4-(methylsulfonyl)phenoxy)methyl) piperidin-4-ol |
| 28 | 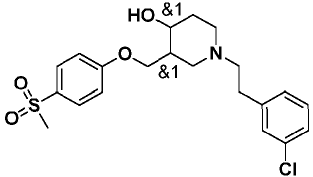 | *rac-cis or trans*-1-(3-chlorophenethyl)-3-((4-(methylsulfonyl)phenoxy)methyl) piperidin-4-ol |
| 29 | 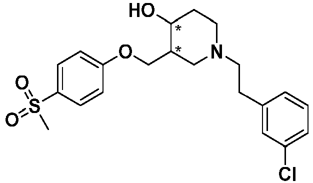 | (3*S*,4*R*) or (3*R,*4*R*) or (3*S*,4*S*) or (3*R*,4*S*)-1-(3-chlorophenethyl)-3-((4-(methylsulfonyl)phenoxy)methyl) piperidin-4-ol |
| 30 | 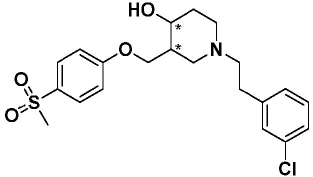 | (3*R*,4*R*) or (3*S*,4*S*) or (3*R*,4*S*) or (3*S*,4*R*) -1-(3-chlorophenethyl)-3-((4-(methylsulfonyl)phenoxy)methyl) piperidin-4-ol |
| 31 | 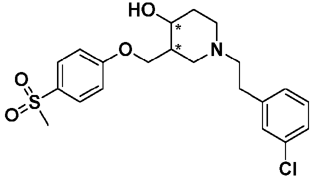 | (3*R*,4*R*) or (3*S*,4*S*) or (3*R*,4*S*) or (3*S*,4*R*) -1-(3-chlorophenethyl)-3-((4-(methylsulfonyl)phenoxy)methyl) piperidin-4-ol |
| 32 | 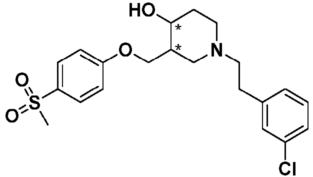 | (3*S*,4*S*) or (3*R*,4*S*) or (3*S*,4*R*) or (3*R*,4*R*) -1-(3-chlorophenethyl)-3-((4-(methylsulfonyl)phenoxy)methyl) piperidin-4-ol |
| 33 | 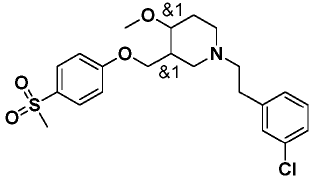 | *rac-trans or cis-1-(3-*chlorophenethyl)-4-methoxy-3-((4-(methylsulfonyl)phenoxy) methyl)piperidine |
| 34 | 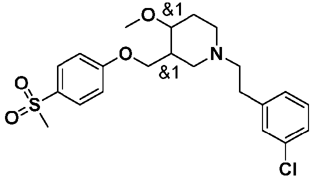 | *rac-cis or trans*-1-(3-chlorophenethyl)-4-methoxy-3-((4-(methylsulfonyl)phenoxy) methyl)piperidine |
| 35 | 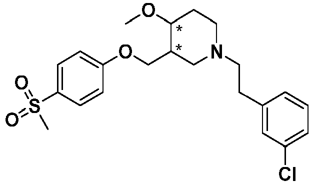 | (3*S*,4*R*) or (3*R,*4*R*) or (3*S*,4*S*) or (3*R*,4*S*)-1-(3-chlorophenethyl)-4-methoxy-3-((4-(methylsulfonyl)phenoxy)methyl) piperidine |
| 36 | 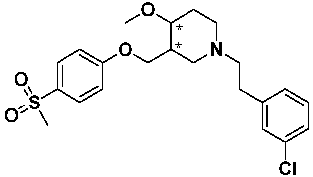 | (3*R*,4*R*) or (3*S*,4*S*) or (3*R*,4*S*) or (3*S*,4*R*)-1-(3-chlorophenethyl)-4-methoxy-3-((4-(methylsulfonyl)phenoxy)methyl) piperidine |
| 37 | 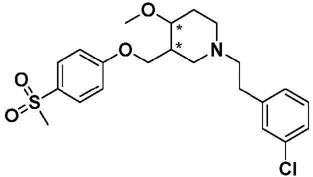 | (3*R*,4*R*) or (3*S*,4*S*) or (3*R*,4*S*) or (3*S*,4*R*) -1-(3-chlorophenethyl)-4-methoxy-3-((4-(methylsulfonyl)phenoxy)methyl) piperidine |
| 38 | 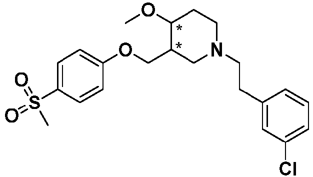 | (3*S*,4*S*) or (3*R*,4*S*) or (3*S*,4*R*) or (3*R*,4*R*) -1-(3-chlorophenethyl)-4-methoxy-3-((4-(methylsulfonyl)phenoxy)methyl) piperidine |
| 39 | 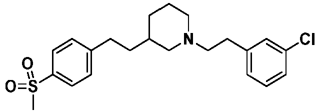 | 1-(3-chlorophenethyl)-3-(4-(methylsulfonyl)phenethyl)piperi dine |
| 40 | 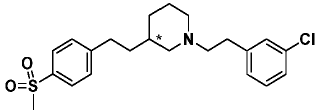 | (*S*) or (*R*)-1-(3-chlorophenethyl)-3-(4-(methylsulfonyl)phenethyl)piperi dine |
| 41 | 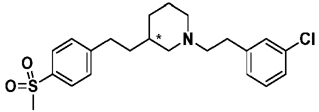 | (*R*) or (*S*)-1-(3-chlorophenethyl)-3-(4-(methylsulfonyl)phenethyl)piperi dine |
| 42 | 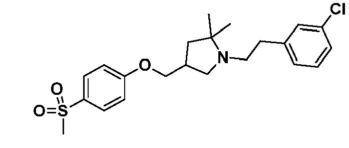 | 1-(3-chlorophenethyl)-2,2-dimethyl-4-((4-(methylsulfonyl)phenoxy) methyl) pyrrolidine |
| 43 | 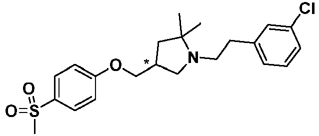 | (*S*) or (*R*)-1-(3-chlorophenethyl)-2,2-dimethyl-4-((4-(methylsulfonyl)phenoxy) methyl) pyrrolidine |
| 44 | 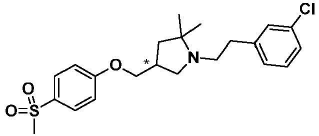 | (*R*) or (*S*)-1-(3-chlorophenethyl)-2,2-dimethyl-4-((4-(methylsulfonyl)phenoxy)methyl) pyrrolidine |
| 45 | 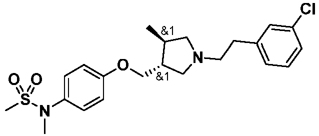 | *rac-trans-N*-(4-((1-(3-chlorophenethyl)-4-methylpyrrolidin-3-yl)methoxy)phenyl)-*N-*methylmethanesulfonamide |
| 46 | 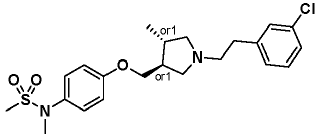 | *N*-(4-(((3*S*,4*S*) or (3*R,*4*R*)-1-(3-chlorophenethyl)-4-methylpyrrolidin-3-yl)methoxy)phenyl)-*N-*methylmethanesulfonamide |
| 47 | 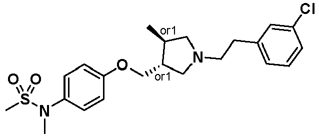 | *N*-(4-(((3*R*,4*R*) or (3*S*,4*S*) -1-(3-chlorophenethyl)-4-methylpyrrolidin-3-yl)methoxy)phenyl)-*N-*methylmethanesulfonamide |
| 48 | 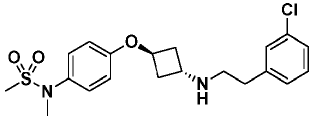 | *trans-N*-(4-(3-((3-chlorophenethyl)amino)cyclobuto xy)phenyl)-*N-*methylmethanesulfonamide |
| 49 | 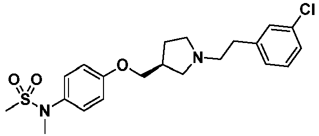 | (*S*)-*N*-(4-((1-(3-chlorophenethyl)pyrrolidin-3-yl)methoxy)phenyl)-*N-*methylmethanesulfonamide |
| 50 | 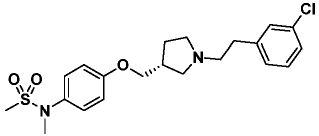 | (*R*)-*N*-(4-((1-(3-chlorophenethyl)pyrrolidin-3-yl)methoxy)phenyl)-*N-*methylmethanesulfonamide |
| 51 | 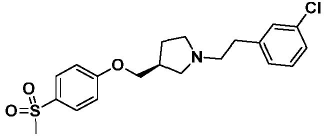 | (*S*)-1-(3-chlorophenethyl)-3-((4-(methylsulfonyl)phenoxy)methyl) pyrrolidine |
| 52 | 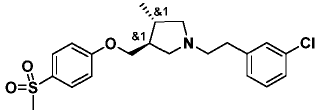 | *rac-trans-*1-(3-chlorophenethyl)-3-methyl-4-((4-(methylsulfonyl)phenoxy)methyl) pyrrolidine |
| 53 | 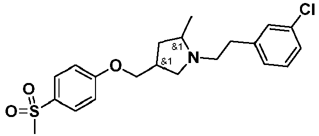 | *rac-trans or cis*-1-(3-chlorophenethyl)-2-methyl-4-((4-(methylsulfonyl)phenoxy)methyl) pyrrolidine |
| 54 | 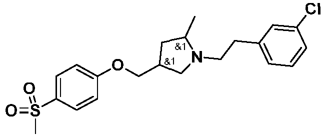 | *rac-cis or trans*-1-(3-chlorophenethyl)-2-methyl-4-((4-(methylsulfonyl)phenoxy)methyl) pyrrolidine |
| 55 | 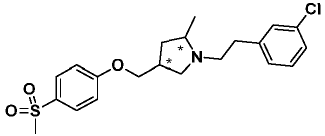 | (2*R*,4*S*) or (2*R*,4*R*) or (2*S,*4*R*) or (2*S*,4*S*)-1-(3-chlorophenethyl)-2-methyl-4-((4-(methylsulfonyl)phenoxy)methyl) pyrrolidine |
| 56 | 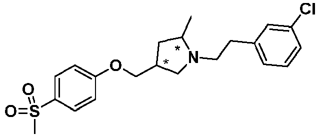 | (2*R*,4*R*) or (*2S,*4*R*) or (2*S*,4*S*) or (2*R*,4*S*)-1-(3-chlorophenethyl)-2-methyl-4-((4-(methylsulfonyl)phenoxy)methyl) pyrrolidine |
| 57 | 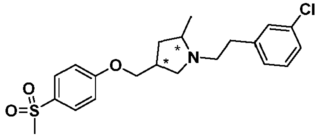 | (2*S*,4*R*) or (2*S*,4*S*) or (2*R*,4*S*) or (2*R*,4*R*)-1-(3-chlorophenethyl)-2-methyl-4-((4-(methylsulfonyl)phenoxy)methyl) pyrrolidine |
| 58 | 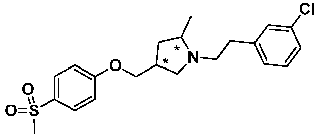 | (2*S*,4*S*) or (2*R*,4*S*) or (2*R*,4*R*) or (2S,4R)-1-(3-chlorophenethyl)-2-methyl-4-((4-(methylsulfonyl)phenoxy)methyl) pyrrolidine |
| 59 | 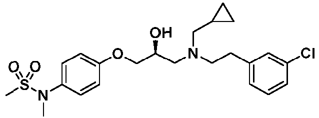 | (*S*)-*N*-(4-(3-((3-chlorophenethyl)(cyclopropylmet hyl)amino)-2-hydroxypropoxy)phenyl)-*N-*methylmethanesulfonamide |
| 60 | 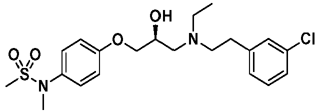 | (*S*)-*N*-(4-(3-((3-chlorophenethyl)(ethyl)amino)-2-hydroxypropoxy)phenyl)-*N-*methylmethanesulfonamide |
| 61 | 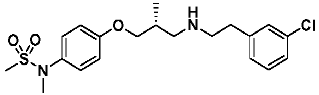 | (*R*)-*N*-(4-(3-((3-chlorophenethyl)amino)-2-methylpropoxy)phenyl)-*N-*methylmethanesulfonamide |
| 62 | 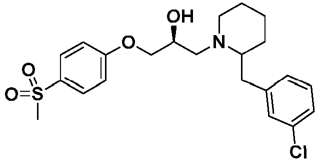 | (2*S*)-1-(2-(3-chlorobenzyl)piperidin-1-yl)-3-(4-(methylsulfonyl)phenoxy)propan-2-ol |
| 63 | 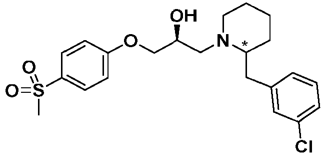 | (*S*)-1-((*S*) or (*R*)-2-(3-chlorobenzyl)piperidin-1-yl)-3-(4-(methylsulfonyl)phenoxy)propan-2-ol |
| 64 | 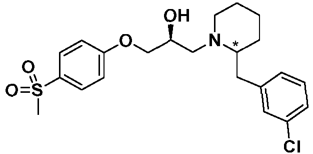 | (*S*)-1-((*R*) or (*S*)-2-(3-chlorobenzyl)piperidin-1-yl)-3-(4-(methylsulfonyl)phenoxy)propan-2-ol |
| 65 | 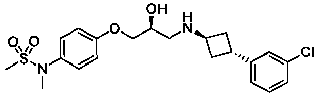 | *N*-(4-((*S*)-3-((*trans*-3-(3-chlorophenyl)cyclobutyl)amino)-2-hydroxypropoxy)phenyl)-*N-*methylmethanesulfonamide |
| 66 | 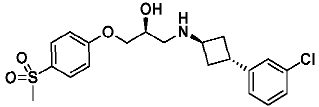 | (*S*)-1-((*trans*-3-(3-chlorophenyl)cyclobutyl)amino)-3-(4-(methylsulfonyl)phenoxy)propan-2-ol |
| 67 | 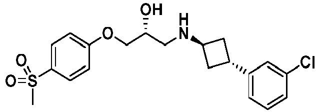 | (*R*)-1-((*trans*-3-(3-chlorophenyl)cyclobutyl)amino)-3-(4-(methylsulfonyl)phenoxy)propan-2-ol |
| 68 | 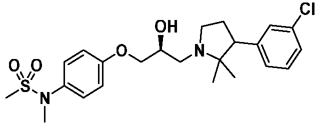 | *N*-(4-((2*S*)-3-(3-(3-chlorophenyl)-2,2-dimethylpyrrolidin-1-yl)-2-hydroxypropoxy)phenyl)-*N-*methylmethanesulfonamide |
| 69 | 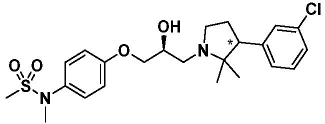 | *N*-(4-((*S*)-3-((*S*) or (*R*)-3-(3-chlorophenyl)-2,2-dimethylpyrrolidin-1-yl)-2-hydroxypropoxy)phenyl)-*N-*methylmethanesulfonamide |
| 70 | 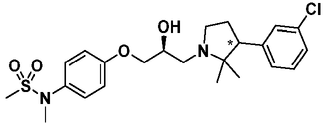 | *N*-(4-((*S*)-3-((*R*) or (*S*)-3-(3-chlorophenyl)-2,2-dimethylpyrrolidin-1-yl)-2-hydroxypropoxy)phenyl)-*N-*methylmethanesulfonamide |
| 71 | 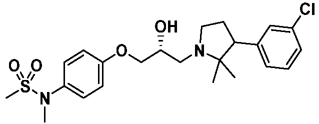 | *N*-(4-((2*R*)-3-(3-(3-chlorophenyl)-2,2-dimethylpyrrolidin-1-yl)-2-hydroxypropoxy)phenyl)-*N-*methylmethanesulfonamide |
| 72 | 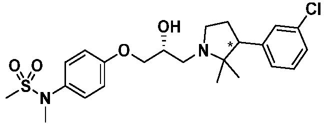 | *N*-(4-((*R*)-3-((*S*) or (*R*)-3-(3-chlorophenyl)-2,2-dimethylpyrrolidin-1-yl)-2-hydroxypropoxy)phenyl)-*N-*methylmethanesulfonamide |
| 73 | 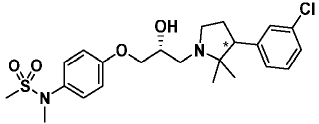 | *N*-(4-((*R*)-3-((*R*) or (*S*)-3-(3-chlorophenyl)-2,2-dimethylpyrrolidin-1-yl)-2-hydroxypropoxy)phenyl)-*N-*methylmethanesulfonamide |
| 74 | 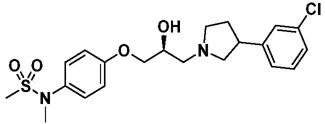 | *N*-(4-((2*S*)-3-(3-(3-chlorophenyl)pyrrolidin-1-yl)-2-hydroxypropoxy)phenyl)-*N-*methylmethanesulfonamide |
| 75 | 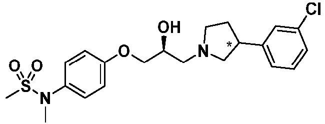 | *N*-(4-((*S*)-3-((*S*) or (*R*)-3-(3-chlorophenyl)pyrrolidin-1-yl)-2-hydroxypropoxy)phenyl)-*N-*methylmethanesulfonamide |
| 76 | 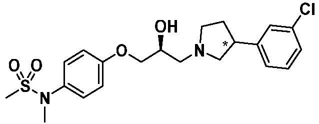 | *N*-(4-((*S*)-3-((*R*) or (*S*)-3-(3-chlorophenyl)pyrrolidin-1-yl)-2-hydroxypropoxy)phenyl)-*N-*methylmethanesulfonamide |
| 77 | 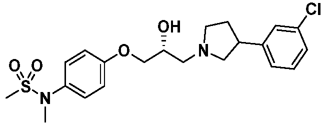 | *N*-(4-((2*R*)-3-(3-(3-chlorophenyl)pyrrolidin-1-yl)-2-hydroxypropoxy)phenyl)-*N-*methylmethanesulfonamide |
| 78 | 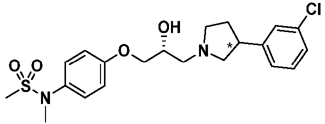 | *N*-(4-((*R*)-3-((*S*) or (*R*)-3-(3-chlorophenyl)pyrrolidin-1-yl)-2-hydroxypropoxy)phenyl)-*N-*methylmethanesulfonamide |
| 79 | 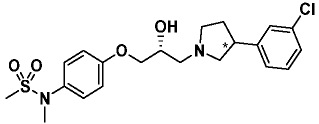 | *N*-(4-((*R*)-3-((*R*) or (*S*)-3-(3-chlorophenyl)pyrrolidin-1-yl)-2-hydroxypropoxy)phenyl)-*N-*methylmethanesulfonamide |
| 80 | 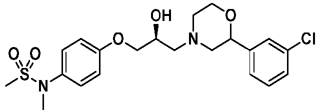 | *N*-(4-((2*S*)-3-(2-(3-chlorophenyl)morpholino)-2-hydroxypropoxy)phenyl)-*N-*methylmethanesulfonamide |
| 81 | 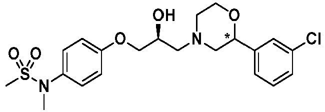 | *N*-(4-((*S*)-3-((*S*) or (*R*)-2-(3-chlorophenyl)morpholino)-2-hydroxypropoxy)phenyl)-*N-*methylmethanesulfonamide |
| 82 | 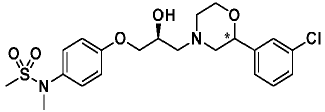 | *N*-(4-((*S*)-3-((*R*) or (*S*)-2-(3-chlorophenyl)morpholino)-2-hydroxypropoxy)phenyl)-*N-*methylmethanesulfonamide |
| 83 | 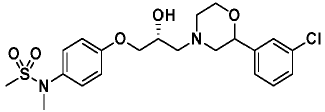 | *N*-(4-((2*R*)-3-(2-(3-chlorophenyl)morpholino)-2-hydroxypropoxy)phenyl)-*N-*methylmethanesulfonamide |
| 84 | 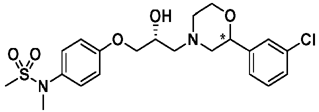 | *N*-(4-((*R*)-3-((*R*) or (*S*)-2-(3-chlorophenyl)morpholino)-2-hydroxypropoxy)phenyl)-*N-*methylmethanesulfonamide |
| 85 | 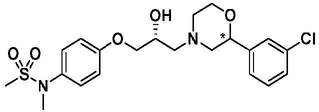 | *N*-(4-((*R*)-3-((*S*) or (*R*)-2-(3-chlorophenyl)morpholino)-2-hydroxypropoxy)phenyl)-*N-*methylmethanesulfonamide |
| 86 | 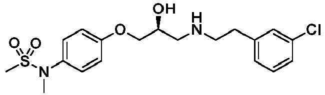 | (*S*)-*N*-(4-(3-((3-chlorophenethyl)amino)-2-hydroxypropoxy)phenyl)-*N-*methylmethanesulfonamide |
| 87 | 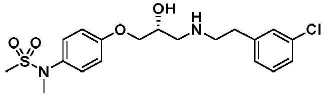 | (*R*)-*N*-(4-(3-((3-chlorophenethyl)amino)-2-hydroxypropoxy)phenyl)-*N-*methylmethanesulfonamide |
| 88 | 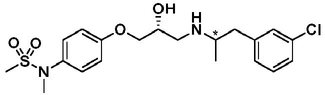 | *N*-(4-((*R*)-3-(((*S*) or (*R*)-1-(3-chlorophenyl)propan-2-yl)amino)-2-hydroxypropoxy)phenyl)-*N-*methylmethanesulfonamide |
| 89 | 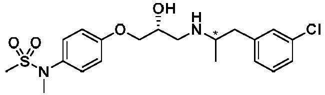 | *N*-(4-((*R*)-3-(((*R*) or (*S*)-1-(3-chlorophenyl)propan-2-yl)amino)-2-hydroxypropoxy)phenyl)-*N-*methylmethanesulfonamide |
| 90 | 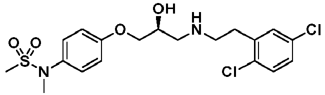 | (*S*)-*N*-(4-(3-((2,5-dichlorophenethyl)amino)-2-hydroxypropoxy)phenyl)-*N-*methylmethanesulfonamide |
| 91 | 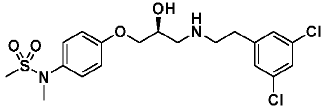 | (*S*)-*N*-(4-(3-((3,5-dichlorophenethyl)amino)-2-hydroxypropoxy)phenyl)-*N-*methylmethanesulfonamide |
| 92 | 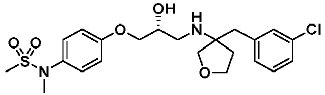 | *N*-(4-((2*R*)-3-((3-(3-chlorobenzyl)tetrahydrofuran-3-yl)amino)-2-hydroxypropoxy)phenyl)-*N-*methylmethanesulfonamide |
| 93 | 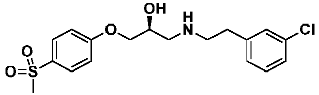 | (*S*)-1-((3-chlorophenethyl)amino)-3-(4-(methylsulfonyl)phenoxy)propan-2-ol |
| 94 | 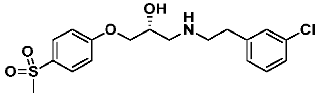 | (*R*)-1-((3-chlorophenethyl)amino)-3-(4-(methylsulfonyl)phenoxy)propan-2-ol |
| 95 | 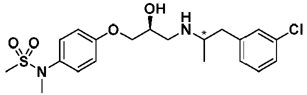 | *N*-(4-((*S*)-3-(((*S*) or (*R*)-1-(3-chlorophenyl)propan-2-yl)amino)-2-hydroxypropoxy)phenyl)-*N-*methylmethanesulfonamide |
| 96 | 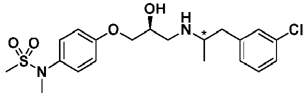 | *N*-(4-((*S*)-3-(((*R*) or (*S*)-1-(3-chlorophenyl)propan-2-yl)amino)-2-hydroxypropoxy)phenyl)-*N-*methylmethanesulfonamide |
| 97 | 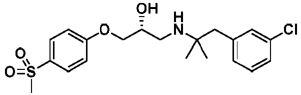 | (*R*)-1-((1-(3-chlorophenyl)-2-methylpropan-2-yl)amino)-3-(4-(methylsulfonyl)phenoxy)propan-2-ol |
| 98 | 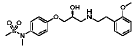 | (*S*)-*N*-(4-(2-hydroxy-3-((2-methoxyphenethyl)amino)propox y)phenyl)-*N-*methylmethanesulfonamide |
| 99 | 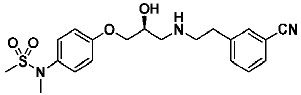 | (*S*)-*N*-(4-(3-((3-cyanophenethyl)amino)-2-hydroxypropoxy)phenyl)-*N-*methylmethanesulfonamide |
| 100 | 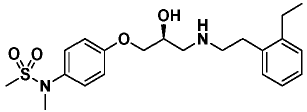 | (*S*)-*N*-(4-(3-((2-ethylphenethyl)amino)-2-hydroxypropoxy)phenyl)-*N-*methylmethanesulfonamide |
| 101 | 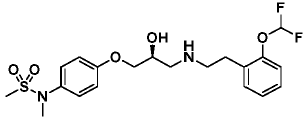 | (*S*)-*N*-(4-(3-((2-(difluoromethoxy)phenethyl)amin o)-2-hydroxypropoxy)phenyl)-*N-*methylmethanesulfonamide |
| 102 | 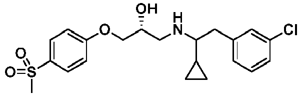 | (2*R*)-1-((2-(3-chlorophenyl)-1-cyclopropylethyl)amino)-3-(4-(methylsulfonyl)phenoxy)propan-2-ol |
| 103 | 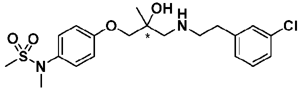 | (*R*) or (*S*)-*N*-(4-(3-((3-chlorophenethyl)amino)-2-hydroxy-2-methylpropoxy)phenyl)-*N-*methylmethanesulfonamide |
| 104 | 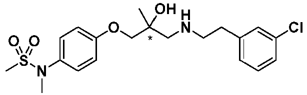 | (*S*) or (*R*)-*N*-(4-(3-((3-chlorophenethyl)amino)-2-hydroxy-2-methylpropoxy)phenyl)-*N-*methylmethanesulfonamide |
| 105 | 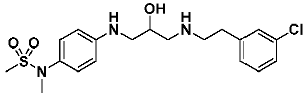 | *N*-(4-((3-((3-chlorophenethyl)amino)-2-hydroxypropyl)amino)phenyl)-*N-*methylmethanesulfonamide |
| 106 | 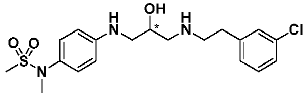 | (*R*) or (*S*)-*N*-(4-((3-((3-chlorophenethyl)amino)-2-hydroxypropyl)amino)phenyl)-*N-*methylmethanesulfonamide |
| 107 | 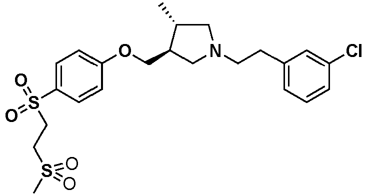 | (3*S*,4*S*)-1-[2-(3-chlorophenyl)ethyl]-3-{[4-(2-methanesulfonylethanesulfonyl)p henoxy]methyl}-4-methylpyrrolidine |
| 108 | 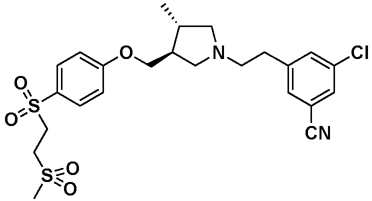 | 3-chloro-5-{2-[(3*S*,4*S*)-3-{[4-(2-methanesulfonylethanesulfonyl)p henoxy]methyl}-4-methylpyrrolidin-1-yl]ethyl}benzonitrile |
| 109 | 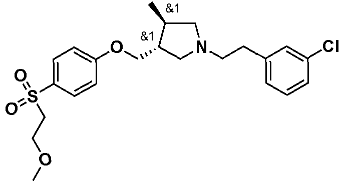 | *rac-trans*-1-[2-(3-chlorophenyl)ethyl]-3-{[4-(2-methoxyethanesulfonyl)phenoxy] methyl}-4-methylpyrrolidine |
| 110 | 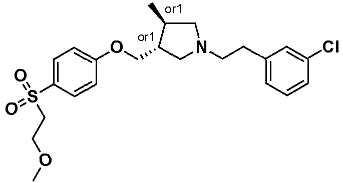 | (3*R*,4*R* or 3*S*,4*S*)-1-[2-(3-chlorophenyl)ethyl]-3-{[4-(2-methoxyethanesulfonyl)phenoxy] methyl}-4-methylpyrrolidine |
| 111 | 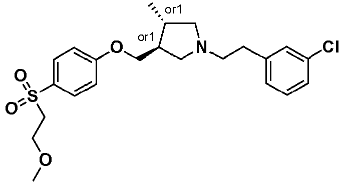 | (3*S*,4*S* or 3*R*,4*R*)-1-[2-(3-chlorophenyl)ethyl]-3-{[4-(2-methoxyethanesulfonyl)phenoxy] methyl}-4-methylpyrrolidine |
| 112 | 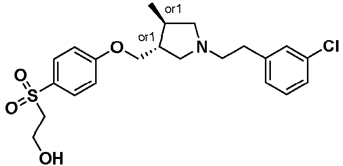 | 2-(4-{[(3*R*,4*R* or 3*S*,4*S*)-1-[2-(3-chlorophenyl)ethyl]-4-methylpyrrolidin-3-yl]methoxy}benzenesulfonyl)etha n-1-ol |
| 113 | 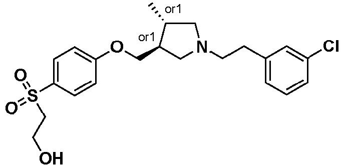 | 2-(4-{[(3*S*,4*S* or 3*R*,4*R*)-1-[2-(3-chlorophenyl)ethyl]-4-methylpyrrolidin-3-yl]methoxy}benzenesulfonyl)etha n-1-ol |
| 114 | 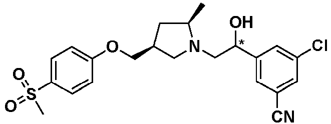 | 3-chloro-5-[(1*S* or 1*R*)-1-hydroxy-2-[(2*R*,4*S*)-4-[(4-methanesulfonylphenoxy)methyl] -2-methylpyrrolidin-1-yl]ethyl]benzonitrile |
| 115 | 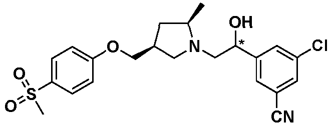 | 3-chloro-5-[(1*R* or 1*S*)-1-hydroxy-2-[(2*R*,4*S*)-4-[(4-methanesulfonylphenoxy)methyl]-2-methylpyrrolidin-1-yl]ethyl]benzonitrile |
| 116 | 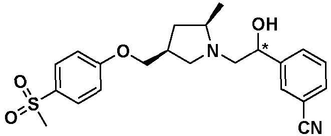 | 3-[(1*R* or 1*S*)-1-hydroxy-2-[(2*R*,4*S*)-4-[(4-methanesulfonylphenoxy)methyl] -2-methylpyrrolidin-1-yl]ethyl]benzonitrile |
| 117 | 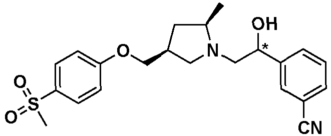 | 3-[(1*S* or 1*R*)-1-hydroxy-2-[(2*R*,4*S*)-4-[(4-methanesulfonylphenoxy)methyl] -2-methylpyrrolidin-1-yl]ethyl]benzonitrile |
| 118 | 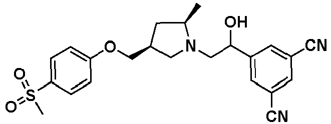 | 5-[(1*R* or 1*S*)-1-hydroxy-2-[(2*R*,4*S*)-4-[(4-methanesulfonylphenoxy)methyl] -2-methylpyrrolidin-1-yl]ethyl]benzene-1,3-dicarbonitrile |
| 119 | 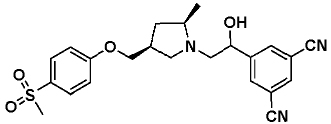 | 5-[(1*S* or 1*R*)-1-hydroxy-2-[(2*R*,4*S*)-4-[(4-methanesulfonylphenoxy)methyl] -2-methylpyrrolidin-1-yl]ethyl]benzene-1,3-dicarbonitrile |
| 120 | 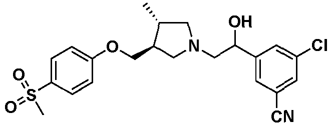 | 3-chloro-5-{1-hydroxy-2-[(3*S*,4*S*)-3-[(4-methanesulfonylphenoxy)methyl] -4-methylpyrrolidin-1-yl]ethyl}benzonitrile |
| 121 | 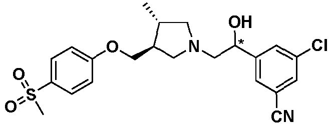 | 3-chloro-5-[(1*S* or 1*R*)-1-hydroxy-2-[(3*S*,4*S*)-3-[(4-methanesulfonylphenoxy)methyl] -4-methylpyrrolidin-1-yl]ethyl]benzonitrile |
| 122 | 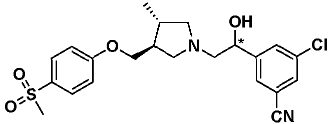 | 3-chloro-5-[(1*R* or 1*S*)-1-hydroxy-2-[(3*S*,4*S*)-3-[(4-methanesulfonylphenoxy)methyl]-4-methylpyrrolidin-1-yl]ethyl]benzonitrile |
| 123 | 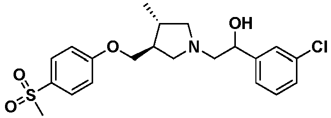 | 1-(3-chlorophenyl)-2-((3*S*,4*S*)-3-methyl-4-((4-(methylsulfonyl)phenoxy)methyl) pyrrolidin-1-yl)ethan-1-ol |
| 124 | 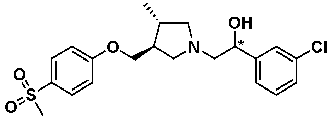 | (1*R* or 1*S*)-1-(3-chlorophenl)-2-[(3*S*,4*S*)-3-[(4-methanesulfonylphenoxy)methyl] -4-methylpyrrolidin-1-yl]ethan-1-ol |
| 125 | 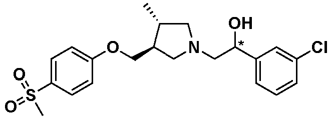 | (1*S* or 1*R*)-1-(3-chlorophenyl)-2-[(3*S*,4*S*)-3-[(4-methanesulfonylphenoxy)methyl] -4-methylpyrrolidin-1-yl]ethan-1-ol |
| 126 | 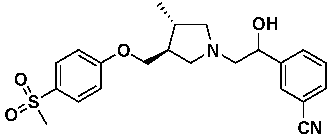 | 3-[1-hydroxy-2-[(3*S*,4*S*)-3-[(4-methanesulfonylphenoxy)methyl] -4-methylpyrrolidin-1-yl]ethyl]benzonitrile |
| 127 | 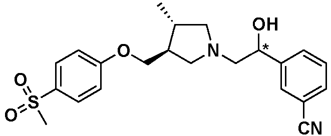 | 3-[(1*S* or 1*R*)-1-hydroxy-2-[(3S,4S)-3-[(4-methanesulfonylphenoxy)methyl] -4-methylpyrrolidin-1-yl]ethyl]benzonitrile |
| 128 | 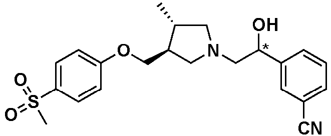 | 3-[(1*R* or 1*S*)-1-hydroxy-2-[(3*S*,4*S*)-3-[(4-methanesulfonylphenoxy)methyl] -4-methylpyrrolidin-1-yl]ethyl]benzonitrile |
| 129 | 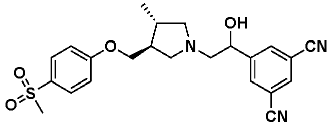 | 5-{1-hydroxy-2-[(3S,4S)-3-[(4-methanesulfonylphenoxy)methyl] -4-methylpyrrolidin-1-yl]ethyl}benzene-1,3-dicarbonitrile |
| 130 | 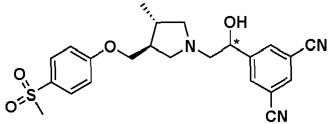 | 5-[(1*R* or 1*S*)-1-hydroxy-2-[(3*S*,4*S*)-3-[(4-methanesulfonylphenoxy)methyl] -4-methylpyrrolidin-1-yl]ethyl]benzene-1,3-dicarbonitrile |
| 131 | 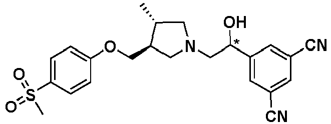 | 5-[(1*S* or 1*R*)-1-hydroxy-2-[(3*S*,4*S*)-3-[(4-methanesulfonylphenoxy)methyl] -4-methylpyrrolidin-1-yl]ethyl]benzene-1,3-dicarbonitrile |
| 132 | 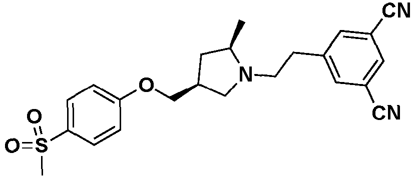 | 5-{2-[(2*R*,4*S*)-4-[(4-methanesulfonylphenoxy)methyl] -2-methylpyrrolidin-1-yl]ethyl}benzene-1,3-dicarbonitrile |
| 133 | 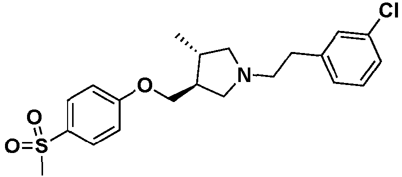 | (3*S*,4*S*)-1-[2-(3-chlorophenyl)ethyl]-3-[(4-methanesulfonylphenoxy)methyl] -4-methylpyrrolidine |
| 134 | 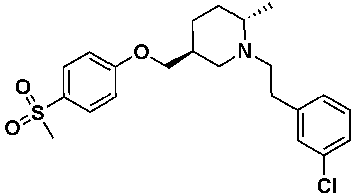 | (2*S*,5*S*)-1-[2-(3-chlorophenyl)ethyl]-5-[(4-methanesulfonylphenoxy)methyl] -2-methylpiperidine |
| 135 | 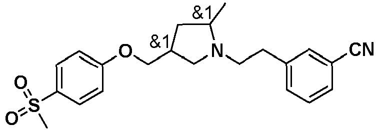 | *rac-cis* or t*rans*-3-{2-[4-[(4-methanesulfonylphenoxy)methyl] -2-methylpyrrolidin-1-yl]ethyl}benzonitrile |
| 136 | 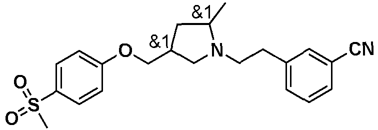 | *rac-trans* or *cis*-3-{2-[4-[(4-methanesulfonylphenoxy)methyl] -2-methylpyrrolidin-1-yl]ethyl}benzonitrile |
| 137 | 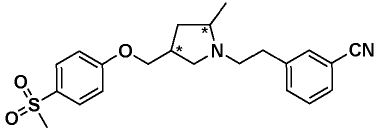 | 3-{2-[(2*S*,4*R* or 2*R*,4*S* or 2*R*,4*R* or 2*S*,4*S*)-4-[(4-methanesulfonylphenoxy)methyl] -2-methylpyrrolidin-1-yl]ethyl}benzonitrile |
| 138 | 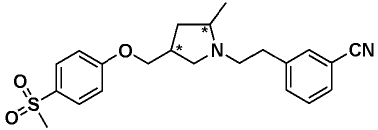 | 3-{2-[(2*R*,4*S* or 2*S*,4*R* or 2*R*,4*R* or 2*S*,4*S*)-4-[(4-methanesulfonylphenoxy)methyl] -2-methylpyrrolidin-1-yl]ethyl}benzonitrile |
| 139 | 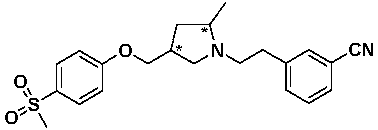 | 3-{2-[(2*S*,4*S* or 2*S*,4*R* or 2*R*,4*S* or 2*R*,4*R*)-4-[(4-methanesulfonylphenoxy)methyl] -2-methylpyrrolidin-1-yl]ethyl}benzonitrile |
| 140 | 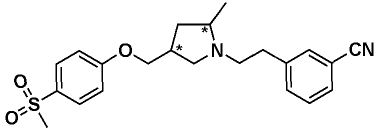 | 3-{2-[(2*R*,4*R* or 2*S*,4*S* or 2*S*,4*R* or 2*R*,4*S*)-4-[(4-methanesulfonylphenoxy)methyl] -2-methylpyrrolidin-1-yl]ethyl}benzonitrile |
| 141 | 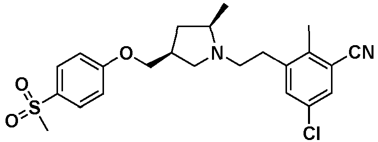 | 5-chloro-3-{2-[(2*R*,4*S*)-4-[(4-methanesulfonylphenoxy)methyl] -2-methylpyrrolidin-1-yl]ethyl}-2-methylbenzonitrile |
| 142 | 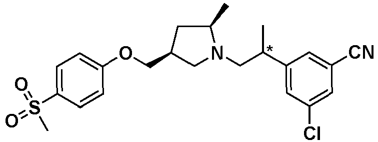 | 3-chloro-5-[(2*R* or 2*S*)-1-[(2R,4S)-4-[(4-methanesulfonylphenoxy)methyl] -2-methylpyrrolidin-1-yl]propan-2-yl]benzonitrile |
| 143 | 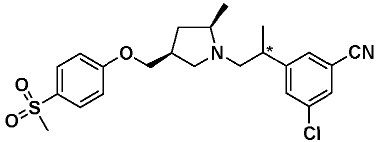 | 3-chloro-5-[(2*S* or 2*R*)-1-[(2R,4S)-4-[(4-methanesulfonylphenoxy)methyl] -2-methylpyrrolidin-1-yl]propan-2-yl]benzonitrile |
| 144 | 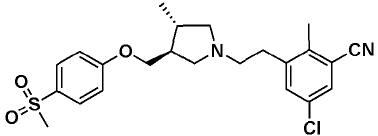 | 5-chloro-3-{2-[(3*S*,4*S*)-3-[(4-methanesulfonylphenoxy)methyl] -4-methylpyrrolidin-1-yl]ethyl}-2-methylbenzonitrile |
| 145 | 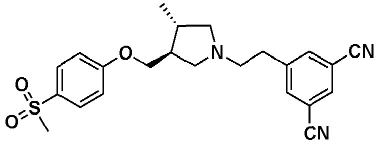 | 5-{2-[(3*S*,4*S*)-3-[(4-methanesulfonylphenoxy)methyl] -4-methylpyrrolidin-1-yl]ethyl}benzene-1,3-dicarbonitrile |
| 146 | 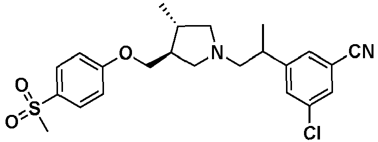 | 3-chloro-5-[1-[(3*S*,4*S*)-3-[(4-methanesulfonylphenoxy)methyl] -4-methylpyrrolidin-1-yl]propan-2-yl]benzonitrile |
| 147 | 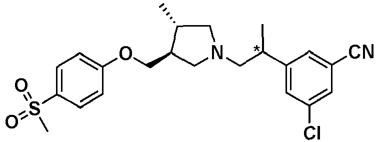 | 3-chloro-5-[(2*R* or 2*S*)-1-[(3S,4S)-3-[(4-methanesulfonylphenoxy)methyl] -4-methylpyrrolidin-1-yl]propan-2-yl]benzonitrile |
| 148 | 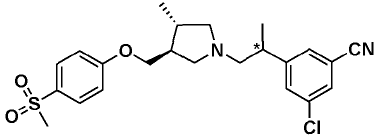 | 3-chloro-5-[(2*S* or 2*R*)-1-[(3S,4S)-3-[(4-methanesulfonylphenoxy)methyl] -4-methylpyrrolidin-1-yl]propan-2-yl]benzonitrile |
| 149 | 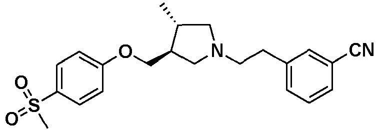 | 3-{2-[(3*S*,4*S*)-3-[(4-methanesulfonylphenoxy)methyl] -4-methylpyrrolidin-1-yl]ethyl}benzonitrile |
| 150 | 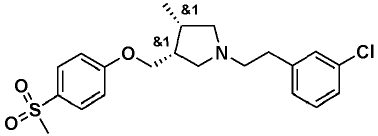 | *rac-cis*-1-[2-(3-chlorophenyl)ethyl]-3-[(4-methanesulfonylphenoxy)methyl] -4-methylpyrrolidine |
| 151 | 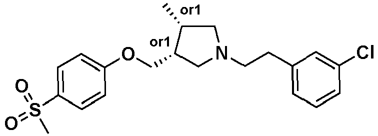 | (3*R*,4*S* or 3*S*,4*R*)-1-[2-(3-chlorophenyl)ethyl]-3-[(4-methanesulfonylphenoxy)methyl] -4-methylpyrrolidine |
| 152 | 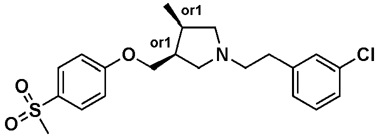 | (3*S*,4*R* or 3*R*,4*S*)-1-[2-(3-chlorophenyl)ethyl]-3-[(4-methanesulfonylphenoxy)methyl] -4-methylpyrrolidine |
| 153 | 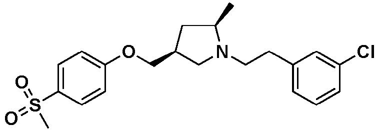 | (2*R*,4*S*)-1-[2-(3-chlorophenyl)ethyl]-4-[(4-methanesulfonylphenoxy)methyl] -2-methylpyrrolidine |
| 154 | 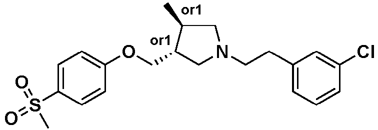 | (3*R*,4*R* or 3*S*,4*S*)-1-[2-(3-chlorophenyl)ethyl]-3-[(4-methanesulfonylphenoxy)methyl] -4-methylpyrrolidine |
| 155 | 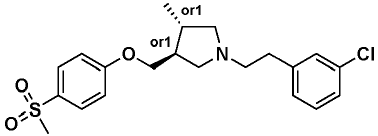 | (3*S*,4*S* or 3*R*,4*R*)-1-[2-(3-chlorophenyl)ethyl]-3-[(4-methanesulfonylphenoxy)methyl] -4-methylpyrrolidine |
| 156 | 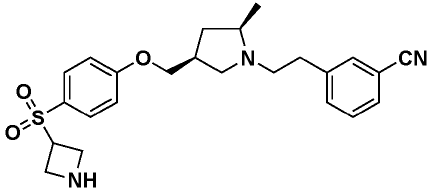 | 3-{2-[(2*R*,4*S*)-4-{[4-(azetidine-3-sulfonyl)phenoxy]methyl}-2-methylpyrrolidin-1-yl]ethyl}benzonitrile |
| 157 | 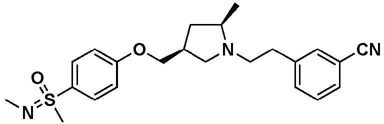 | 3-{2-[(2*R*,4*S*)-2-methyl-4-({4-[methyl(methylimino)oxo-λ⁶-sulfanyl]phenoxy }methyl)pyrroli din-1-yl]ethyl}benzonitrile |
| 158 | 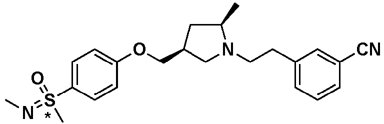 | 3-{2-[(2*R*,4*S*)-2-methyl-4-({4-[(*R* or *S*)methyl(methylimino)oxo-λ⁶-sulfanyl]phenoxy}methyl)pyrroli din-1-yl]ethyl}benzonitrile |
| 159 | 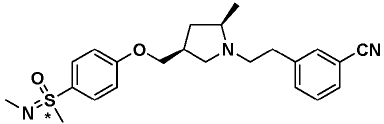 | 3-{2-[(2*R*,4*S*)-2-methyl-4-({4-[(*S* or *R*)methyl(methylimino)oxo-λ⁶-sulfanyl]phenoxy}methyl)pyrroli din-1-yl]ethyl}benzonitrile |
| 160 | 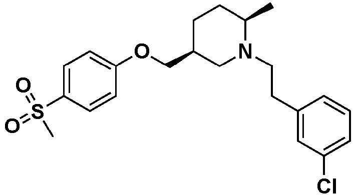 | (2*R*,5*S*)-1-[2-(3-chlorophenyl)ethyl]-5-[(4-methanesulfonylphenoxy)methyl] -2-methylpiperidine |
| 161 | 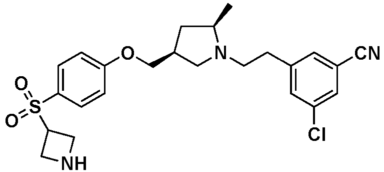 | 3-{2-[(2*R*,4*S*)-4-{[4-(azetidine-3-sulfonyl)phenoxy]methyl}-2-methylpyrrolidin-1-yl]ethyl}-5-chlorobenzonitrile |
| 162 | 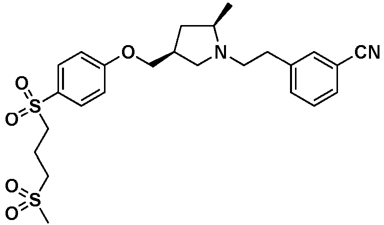 | 3-{2-[(2*R*,4*S*)-4-{ [4-(3-methanesulfonylpropanesulfonyl) phenoxy]methyl}-2-methylpyrrolidin-1-yl]ethyl}benzonitrile |
| 163 | 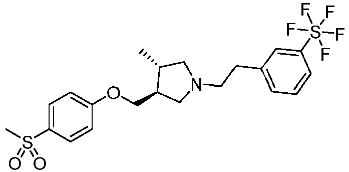 | (3*S*,4*S*)-3-[(4-methanesulfonylphenoxy)methyl] -4-methyl-1-{2-[3-(pentafluoro-λ⁶-sulfanyl)phenyl]ethyl}pyrrolidine |
| 164 | 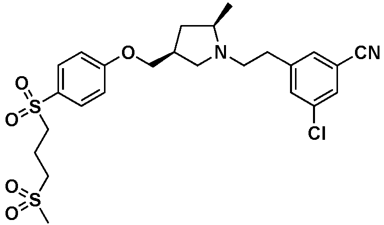 | 3-chloro-5-{2-[(2*R*,4*S*)-4-{[4-(3-methanesulfonylpropanesulfonyl) phenoxy]methyl}-2-methylpyrrolidin-1-yl]ethyl}benzonitrile |
| 165 | 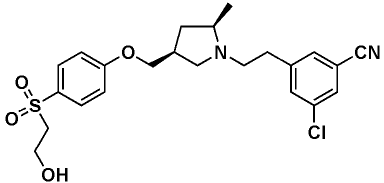 | 3-chloro-5-{2-[(2*R*,4*S*)-4-{[4-(2-hydroxyethanesulfonyl)phenoxy] methyl}-2-methylpyrrolidin-1-yl]ethyl}benzonitrile |
| 166 | 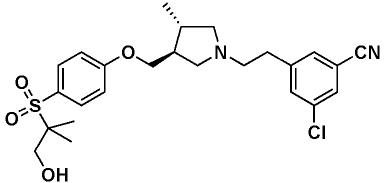 | 3-chloro-5-{2-[(3*S*,4*S*)-3-{[4-(1-hydroxy-2-methylpropane-2-sulfonyl)phenoxy]methyl}-4-methylpyrrolidin-1-yl]ethyl}benzonitrile |
| 167 | 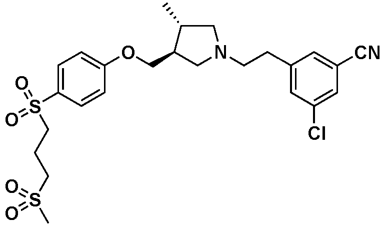 | 3-chloro-5-{2-[(3*S*,4*S*)-3-{[4-(3-methanesulfonylpropanesulfonyl) phenoxy]methyl}-4-methylpyrrolidin-1-yl]ethyl}benzonitrile |
| 168 | 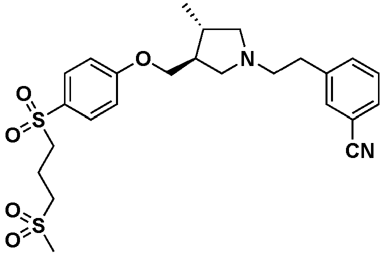 | 3-{ 2-[(3*S*,4*S*)-3-{ [4-(3-methanesulfonylpropanesulfonyl) phenoxy]methyl}-4-methylpyrrolidin-1-yl]ethyl}benzonitrile |
| 169 | 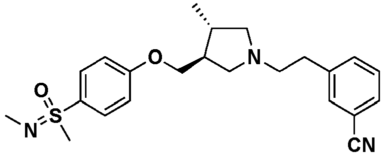 | 3-{2-[(3*S*,4*S*)-3-methyl-4-({4-[methyl(methylimino)oxo-λ⁶-sulfanyl]phenoxy }methyl)pyrroli din-1-yl]ethyl }benzonitrile |
| 170 | 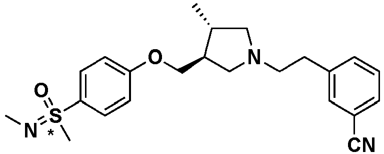 | 3-{2-[(3*S*,4*S*)-3-methyl-4-({4-[(*R* or *S*)-methyl(methylimino)oxo-λ⁶-sulfanyl]phenoxy }methyl)pyrroli din-1-yl]ethyl }benzonitrile |
| 171 | 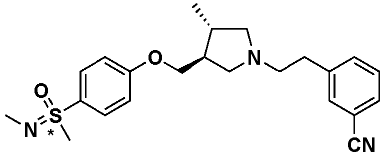 | 3-{2-[(3*S*,4*S*)-3-methyl-4-({4-[(*S* or *R*)-methyl(methylimino)oxo-λ⁶-sulfanyl]phenoxy }methyl)pyrroli din-1-yl]ethyl}benzonitrile |
| 172 | 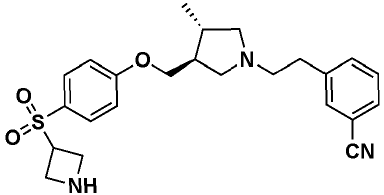 | 3-{2-[(3*S*,4*S*)-3-{[4-(azetidine-3-sulfonyl)phenoxy]methyl}-4-methylpyrrolidin-1-yl]ethyl}benzonitrile |
| 173 | 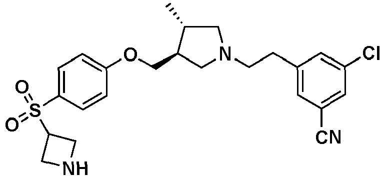 | 3-{2-[(3*S*,4*S*)-3-{[4-(azetidine-3-sulfonyl)phenoxy]methyl}-4-methylpyrrolidin-1-yl]ethyl}-5-chlorobenzonitrile |
| 174 | 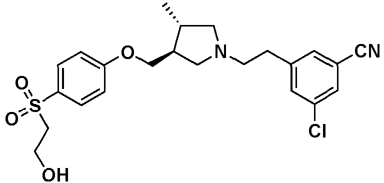 | 3-chloro-5-{2-[(3*S*,4*S*)-3-{[4-(2-hydroxyethanesulfonyl)phenoxyl methyl}-4-methylpyrrolidin-1-yl]ethyl}benzonitrile |
| 175 | 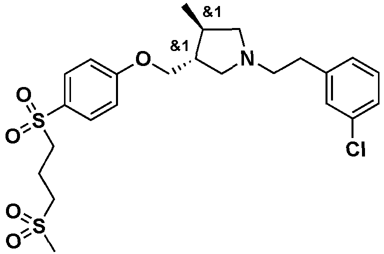 | *rac-trans*-1-[2-(3-chlorophenyl)ethyl]-3-{[4-(3-methanesulfonylpropanesulfonyl) phenoxy]methyl}-4-methylpyrrolidine |
| 176 | 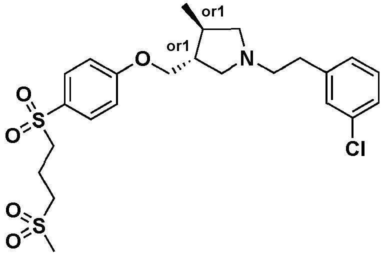 | (3*R*,4*R* or 3*S*,4*S*)-1-[2-(3-chlorophenyl)ethyl]-3-{[4-(3-methanesulfonylpropanesulfonyl) phenoxy]methyl}-4-methylpyrrolidine |
| 177 | 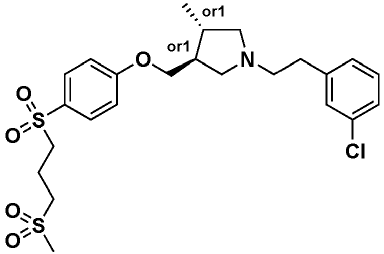 | (3*S*,4*S* or 3*R*,4*R*)-1-[2-(3-chlorophenyl)ethyl]-3-{[4-(3-methanesulfonylpropanesulfonyl) phenoxy]methyl}-4-methylpyrrolidine |
| 181 | 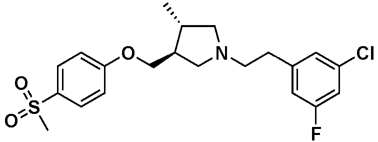 | (3*S*,4*S*)-1-[2-(3-chloro-5-fluorophenyl)ethyl]-3-[(4-methanesulfonylphenoxy)methyl] -4-methylpyrrolidine |
| 182 | 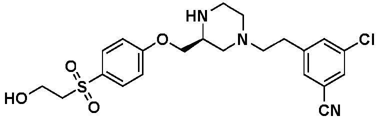 | 3-chloro-5-{2-[(3*S*)-3-{[4-(2-hydroxyethanesulfonyl)phenoxy] methyl}piperazin-1-yl]ethyl}benzonitrile |
| 183 | 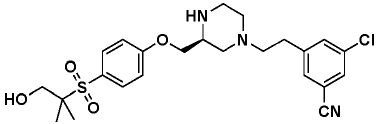 | 3-chloro-5-{2-[(3*S*)-3-1[4-(1-hydroxy-2-methylpropane-2-sulfonyl)phenoxy]methyl}piperaz in-1-yl]ethyl}benzonitrile |
| 184 | 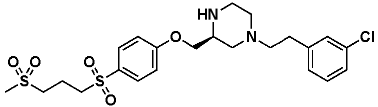 | (3*S*)-1-[2-(3-chlorophenyl)ethyl]-3-{[4-(3-methanesulfonylpropanesulfonyl) phenoxy]methyl}piperazine |
| 185 | 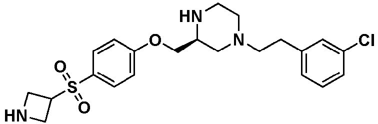 | (3*S*)-3-{[4-(azetidine-3-sulfonyl)phenoxy]methyl}-1-[2-(3-chlorophenyl)ethyl]piperazine |
| 186 | 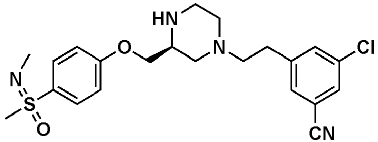 | 3-chloro-5-{2-[(3*S*)-3-({4-[methyl(methylimino)oxo-λ⁶-sulfanyl]phenoxy}methyl)piperaz in-1-yl]ethyl}benzonitrile |
| 187 | 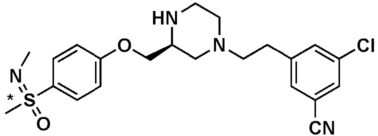 | 3-chloro-5-{2-[(3S)-3-({4-[(*S* or *R*)-methyl(methylimino)oxo-λ⁶-sulfanyl]phenoxy }methyl)piperaz in-1-yl]ethyl }benzonitrile |
| 188 | 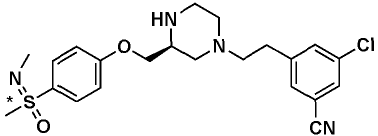 | 3-chloro-5-{2-[(3*S*)-3-({4-[(*R* or *S*)-methyl(methylimino)oxo-λ⁶-sulfanyl]phenoxy }methyl)piperaz in-1-yl]ethyl}benzonitrile |
| 189 | 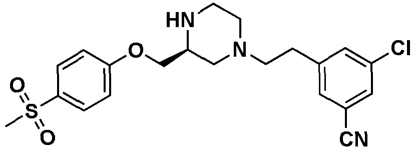 | 3-chloro-5-{2-[(3*S*)-3-[(4-methanesulfonylphenoxy)methyl] piperazin-1-yl]ethyl}benzonitrile |
| 190 | 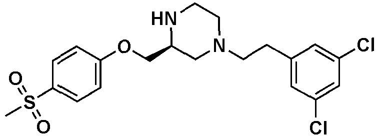 | (3*S*)-1-[2-(3,5-dichlorophenyl)ethyl]-3-[(4-methanesulfonylphenoxy)methyl] piperazine |
| 191 | 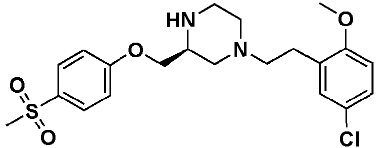 | (3*S*)-1-[2-(5-chloro-2-methoxyphenyl)ethyl]-3-[(4-methanesulfonylphenoxy)methyl] piperazine |
| 192 | 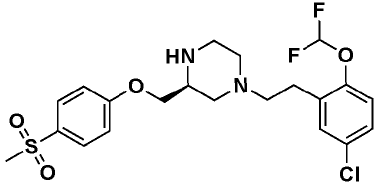 | (3*S*)-1-{2-[5-chloro-2-(difluoromethoxy)phenyl]ethyl}-3-[(4-methanesulfonylphenoxy)methyl] piperazine |
| 193 | 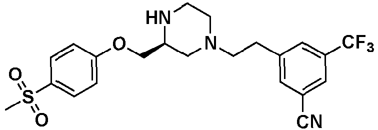 | 3-{2-[(3*S*)-3-[(4-methanesulfonylphenoxy)methyl] piperazin-1-yl]ethyl}-5-(trifluoromethyl)benzonitrile |
| 194 | 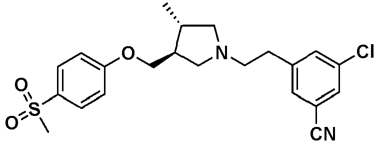 | 3-chloro-5-{2-[(3*S*,4*S*)-3-[(4-methanesulfonylphenoxy)methyl] -4-methylpyrrolidin-1-yl]ethyl}benzonitrile |
| 195 | 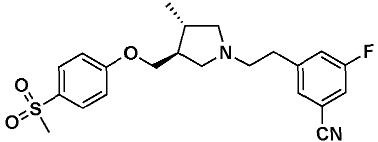 | 3-fluoro-5-{2-[(3*S*,4*S*)-3 -[(4-methanesulfonylphenoxy)methyl] -4-methylpyrrolidin-1-yl]ethyl}benzonitrile |
| 196 | 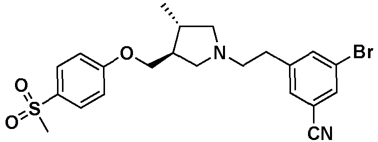 | 3-bromo-5-{2-[(3*S*,4*S*)-3-[(4-methanesulfonylphenoxy)methyl] -4-methylpyrrolidin-1-yl]ethyl}benzonitrile |
| 197 | 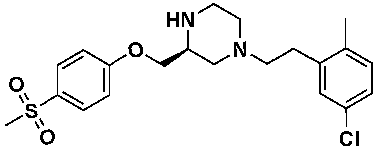 | (3*S*)-1-[2-(5-chloro-2-methylphenyl)ethyl]-3-[(4-methanesulfonylphenoxy)methyl] piperazine |
| 198 | 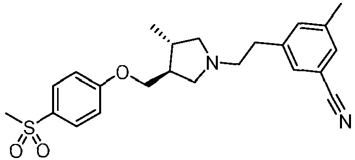 | 3-{2-[(3*S*,4*S*)-3-[(4-methanesulfonylphenoxy)methyl] -4-methylpyrrolidin-1-yl]ethyl}-5-methylbenzonitrile |
| 199 | 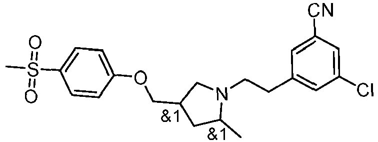 | *rac-cis* or *trans-*3-*chloro*-5-{2-[4-[(4-methanesulfonylphenoxy)methyl] -2-methylpyrrolidin-1-yl]ethyl}benzonitrile |
| 200 | 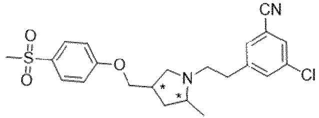 | 3-chloro-5-12-[(2R,4S or *2S,4R* or *2R,4R* or 2*S*,4*S*)-4-[(4-methanesulfonylphenoxy)methyl] -2-methylpyrrolidin-1-yl]ethyl}benzonitrile |
| 201 | 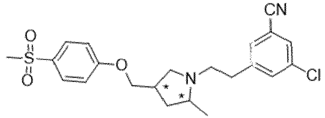 | 3-chloro-5-{2-[(2*S*,4*R* or 2*R*,4*S* or 2*R*,4*R* or 2*S*,4*S*)-4-[(4-methanesulfonylphenoxy)methyl] -2-methylpyrrolidin-1-yl]ethyl}benzonitrile |
| 202 | 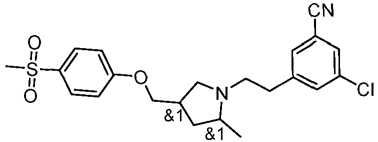 | *rac-trans* or *cis*-3-chloro-5-{2-[4-[(4-methanesulfonylphenoxy)methyl] -2-methylpyrrolidin-1-yl]ethyl}benzonitrile |
| 203 | 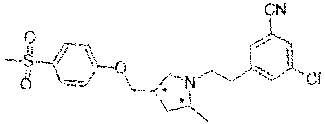 | 3-chloro-5-(2-((2*R*,4*S* or 2*S*,4*R* or 2*R*,4*R* or 2*S*,4*S*)-2-methyl-4-((4-(methylsulfonyl)phenoxy)methyl) pyrrolidin-1-yl)ethyl)benzonitrile |
| 204 | 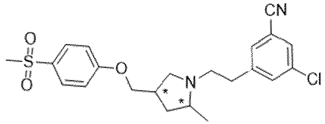 | 3-chloro-5-(2-((2*R*,4*S* or 2*S*,4*R* or 2*S*,4*S* or 2*R.*4*R*)-2-methyl-4-((4-(methylsulfonyl)phenoxy)methyl) pyrrolidin-1-yl)ethyl)benzonitrile |
| 205 | 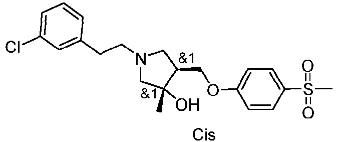 | *rac-cis-*1-[2-(3-chlorophenyl)ethyl]-4-[(4-methanesulfonylphenoxy)methyl] -3-methylpyrrolidin-3-ol |
| 206 | 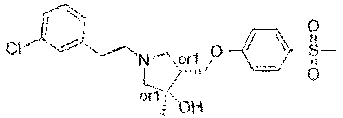 | (3*R*,4*R* or 3*S*,4*S*)-1-[2-(3-chlorophenyl)ethyl]-4-[(4-methanesulfonylphenoxy)methyl] -3-methylpyrrolidin-3-ol |
| 207 | 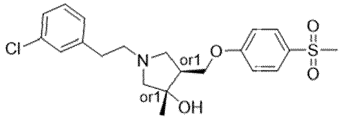 | (3*S*,4*S* or 3*R*,4*R*)-1-(3-chlorophenethyl)-3-methyl-4-((4-(methylsulfonyl)phenoxy)methyl) pyrrolidin-3-ol |
| 208 | 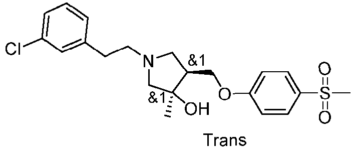 | *rac-trans*-1-[2-(3-chlorophenyl)ethyl]-4-[(4-methanesulfonylphenoxy)methyl] -3-methylpyrrolidin-3-ol |
| 209 | 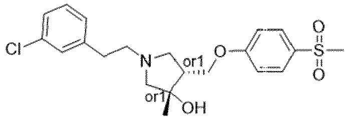 | (3*R*,4*S* or 3*S*,4*R*)-1-[2-(3-chlorophenyl)ethyl]-4-[(4-methanesulfonylphenoxy)methyl] -3-methylpyrrolidin-3-ol |
| 210 | 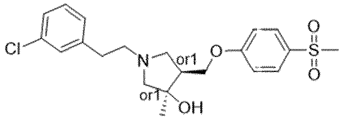 | (3*S*,4*R* or 3*R*,4*S*)-1-[2-(3-chlorophenyl)ethyl]-4-[(4-methanesulfonylphenoxy)methyl] -3-methylpyrrolidin-3-ol |
| 211 | 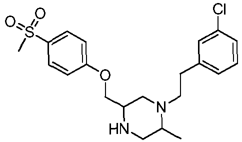 | 1-[2-(3-chlorophenyl)ethyl]-5-[(4-methanesulfonylphenoxy)methyl] -2-methylpiperazine |
| 212 | 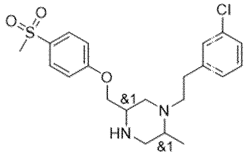 | *rac-cis* or *trans-*1-[2-(3-chlorophenyl)ethyl]-5-[(4-methanesulfonylphenoxy)methyl] -2-methylpiperazine |
| 213 | 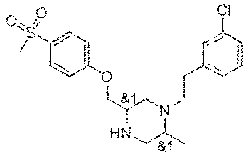 | *rac-trans* or *cis*-1-[2-(3-chlorophenyl)ethyl]-5-[(4-methanesulfonylphenoxy)methyl] -2-methylpiperazine |
| 214 | 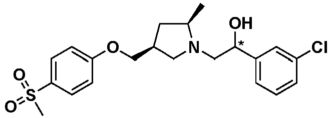 | (1*R*) or (1*S*)-1-(3-chlorophenyl)-2-[(2R,4S)-4-[(4-methanesulfonylphenoxy)methyl] -2-methylpyrrolidin-1-yl]ethan-1-ol |
| 215 | 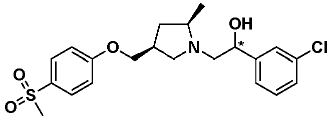 | (1*S*) or (1*R*)-1-(3-chlorophenyl)-2-[(2R,4S)-4-[(4-methanesulfonylphenoxy)methyl] -2-methylpyrrolidin-1-yl]ethan-1-ol |
or a stereoisomer or tautomer thereof, or a pharmaceutically acceptable salt of any of the foregoing.

13. A pharmaceutical composition, comprising (i) a compound of any one of claims 1 to 12, or a stereoisomer or tautomer thereof, or a pharmaceutically acceptable salt of any of the foregoing, and (ii) one or more pharmaceutically acceptable excipients.

14. The compound of any one of claims 1 to 12, or a stereoisomer or tautomer thereof, or a pharmaceutically acceptable salt of any of the foregoing, or the pharmaceutical composition according to claim 13, for use in treating or delaying the development of a disease, disorder, or condition selected from:
a kidney disease;
a disease, disorder, or condition selected from the group consisting of chronic kidney disease, focal segmental glomerulosclerosis (FSGS), hypertension-attributed kidney disease, human immunodeficiency virus-associated nephropathy (HIVAN), sickle-cell nephropathy, lupus nephritis, diabetic kidney disease, APOL1-associated nephropathy, viral nephropathy, COVID-19 associated nephropathy, preeclampsia, and sepsis.

15. A kit, comprising (i) a compound of any one of claims 1 to 12, or a stereoisomer or tautomer thereof, or a pharmaceutically acceptable salt of any of the foregoing, or a pharmaceutical composition of claim 13, and (ii) instructions for use in treating an APOL1-mediated disease, disorder, or condition in an individual in need thereof;
wherein the disease, disorder, or condition is optionally:
a kidney disease; or
a disease, disorder, or condition selected from the group consisting of chronic kidney disease, focal segmental glomerulosclerosis (FSGS), hypertension-attributed kidney disease, human immunodeficiency virus-associated nephropathy (HIVAN), sickle-cell nephropathy, lupus nephritis, diabetic kidney disease, APOL1-associated nephropathy, viral nephropathy, COVID-19 associated nephropathy, preeclampsia, and sepsis;
optionally wherein the individual has an APOL1 mutation, such as a gain-of-function mutation.

## Patentansprüche

1. Verbindung der Formel (A'): 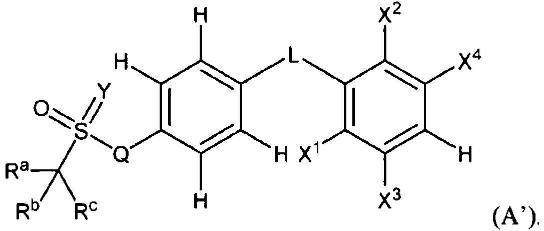
oder ein Stereoisomer oder Tautomer davon oder ein pharmazeutisch annehmbares Salz von beliebigen der Vorgenannten, worin:
Q fehlt oder -N-(C₁₋₆-Alkyl) ist;
Y O oder -N-(C₁₋₆-Alkyl) ist;
mit der Maßgabe, dass, wenn Q -N(C₁₋₆-Alkyl) ist, Y O ist;
R^{a}, R^{b} und R^{c} jeweils unabhängig voneinander H oder C₁₋₆-Alkyl sind, wobei das C₁₋₆-Alkyl von R^{a}, R^{b} oder R^{c} jeweils unabhängig gegebenenfalls mit einem oder mehreren aus -OH, C₁₋₆-Alkoxy und -S(O)₂-C₁₋₆-Alkyl substituiert ist,
oder beliebige zwei von R^{a}, R^{b} und R^{c} zusammen mit den Atomen, an die sie gebunden sind, ein C₃₋₆-Cycloalkyl oder ein 3- bis 6-gliedriges Heterocyclyl bilden und der andere von R^{a}, R^{b} und R^{c} H oder C₁₋₆-Alkyl ist, wobei das C₁₋₆-Alkyl von R^{a}, R^{b} oder R^{c} jeweils unabhängig gegebenenfalls mit einem oder mehreren aus -OH, C₁₋₆-Alkoxy und -S(O)₂-C₁₋₆-Alkyl substituiert ist;
L aus der aus Folgendem bestehenden Gruppe ausgewählt ist:
(i) 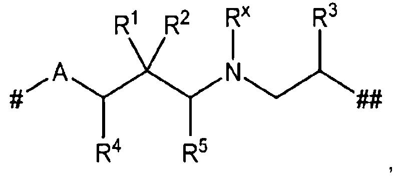 worin
A O, NH, N(C₁₋₆-Alkyl), CH₂ oder CH(C₁₋₆-Alkyl ist,
R^{x} H ist
oder R^{x} zusammen mit einem von R¹ und R² und den Atomen, an die sie gebunden sind, ein 3- bis 8-gliedriges Heterocyclyl bildet, wobei das 3- bis 8-gliedrige Heterocyclyl mit *n* jeweils unabhängig voneinander ausgewählten Substituenten R^{g} substituiert ist, wobei n eine ganze Zahl von 0 bis 6 ist und R^{g} -OH, Halogen, C₁₋₆-Alkyl oder C₁₋₆-Alkoxy ist,
R¹ und R² jeweils unabhängig voneinander H, Halogen oder -OH sind
oder einer von R¹ und R² zusammen mit R^{x} und den Atomen, an die sie gebunden sind, ein 3- bis 8-gliedriges Heterocyclyl bildet, wobei das wobei das 3- bis 8-gliedrige Heterocyclyl mit n unabhängig jeweils voneinander ausgewählten Substituenten R^{g} substituiert ist, wobei n eine ganze Zahl von 0 bis 6 ist und R^{g} -OH, Halogen, C₁₋₆-Alkyl oder C₁₋₆-Alkoxy ist, und der andere von R¹ und R² H, Halogen oder -OH ist,
R³ H, -OH, Halogen oder C₁₋₆-Alkoxy ist und
R⁴ und R⁵ jeweils unabhängig voneinander H sind
oder R⁴ und R⁵ zusammen mit den Atomen, an die sie gebunden sind, ein C₃₋₈-Cycloalkyl bilden,
mit der Maßgabe, dass entweder:
(1) R^{x} zusammen mit einem von R¹ und R² und den Atomen, an die sie gebunden sind, ein 3- bis 8-gliedriges Heterocyclyl bildet, wobei das wobei das 3- bis 8-gliedrige Heterocyclyl mit n unabhängig voneinander ausgewählten Substituenten R^{g} substituiert ist, wobei n eine ganze Zahl von 0 bis 6 ist und R^{g} -OH, Halogen, C₁₋₆-Alkyl oder C₁₋₆-Alkoxy ist, oder
(2) R⁴ und R⁵ zusammen mit den Atomen, an die sie gebunden sind, ein C₃₋₈-Cycloalkyl bilden;
(ii) 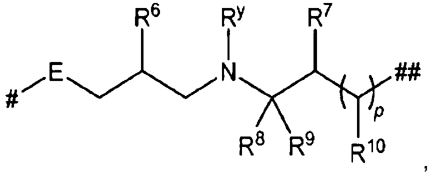 worin
E O, NH, N(C₁₋₆-Alkyl), CH₂ oder CH(C₁₋₆-Alkyl) ist,
*p* = 0 oder 1 ist,
mit der Maßgabe, dass, wenn *p* = 1 ist, E O ist,
R⁶ H oder -OH ist,
R^{y} H ist
oder R^{y} zusammen mit R⁷ und den Atomen, an die sie gebunden sind, ein 3- bis 8-gliedriges Heterocyclyl bildet
oder R^{y} zusammen mit einem von R⁸ und R⁹ und den Atomen, an die sie gebunden sind, ein 3- bis 8-gliedriges Heterocyclyl bildet,
R⁷ H ist
oder R⁷ zusammen mit R^{y} und den Atomen, an die sie gebunden sind, ein 3- bis 8-gliedriges Heterocyclyl bildet,
R⁸ und R⁹ jeweils unabhängig voneinander H oder C₁₋₆-Alkyl sind
oder einer von R⁸ und R⁹ zusammen mit R^{y} und den Atomen, an die sie gebunden sind, ein 3- bis 8-gliedriges Heterocyclyl bildet und der andere von R⁸ und R⁹ H oder C₁₋₆-Alkyl ist
oder einer von R⁸ und R⁹ zusammen mit R¹⁰ und den Atomen, an die sie gebunden sind, ein 3- bis 8-gliedriges Heterocyclyl bildet und der andere von R⁸ und R⁹ H oder C₁₋₆-alkyl ist, und
R¹⁰ H ist
oder R¹⁰ zusammen mit einem von R⁸ und R⁹ und den Atomen, an die sie gebunden sind, ein C₃₋₈-Cycloalkyl bildet,
mit der Maßgabe dass:
(1) R^{y} zusammen mit R⁷ und den Atomen, an die sie gebunden sind, ein 3- bis 8-gliedriges Heterocyclyl bildet oder
(2) R^{y} zusammen mit einem von R⁸ und R⁹ und den Atomen, an die sie gebunden sind, ein 3- bis 8-gliedriges Heterocyclyl bildet oder
(3) einer von R⁸ und R⁹ zusammen mit R¹⁰ und den Atomen, an die sie gebunden sind, ein 3- bis 8-gliedriges Heterocyclyl bildet; und
(iii) 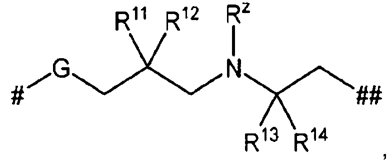 worin
G O, NH, N(C₁₋₆-Alkyl), CH₂ oder CH(C₁₋₆-Alkyl) ist,
R^{z} H oder C₁₋₆-Alkyl ist, wobei das C₁₋₆-Alkyl gegebenenfalls mit einem oder mehreren C₃₋₆-Cycloalkyl substituiert ist,
R¹¹ und R¹² jeweils unabhängig voneinander H, -OH, Halogen oder C₁₋₆-Alkyl sind und
R¹³ und R¹⁴ jeweils unabhängig voneinander H, C₁₋₆-alkyl oder C₃₋₈-Cycloalkyl sind
oder R¹³ und R¹⁴ zusammen mit den Atomen, an die sie gebunden sind, ein 3- bis 8-gliedriges Heterocyclyl bilden,
wobei in jedem von (i) bis (iii) # den Anbindungspunkt an die einen Phenylring tragende Gruppierung Q kennzeichnet und ## den Anbindungspunkt an die einen Phenylring tragenden Gruppierungen X¹-X⁴ kennzeichnet; und
X¹, X², X³ und X⁴ jeweils unabhängig voneinander H, Halogen, -CN, C₁₋₆-alkyl, C₁₋₆-Alkoxy oder SF₅ sind, wobei das C₁₋₆-Alkyl oder C₁₋₆-Alkoxy gegebenenfalls mit einem oder mehreren Halogenen substituiert ist,
mit der Maßgabe, dass zumindest einer von X¹, X², X³ und X⁴ Halogen, -CN, C₁₋₆-Alkyl, C₁₋₆-Alkoxy oder SF₅ ist, wobei das C₁₋₆-Alkyl oder C₁₋₆-Alkoxy gegebenenfalls mit einem oder mehreren Halogenen substituiert ist.

2. Verbindung nach Anspruch 1, wobei die Verbindung eine Verbindung der Formel (A): 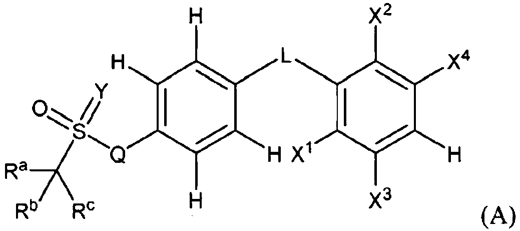 oder ein Stereoisomer oder ein Tautomer davon oder ein pharmazeutisch annehmbares Salz eines beliebigen der Vorgenannten ist, worin:
X¹, X², X³ und X⁴ jeweils unabhängig voneinander H, Halogen, -CN, C₁-C₆-Alkyl oder C₁-C₆-Alkoxy sind, wobei das C₁-C₆-Alkyl oder C₁-C₆-Alkoxy gegebenenfalls mit einem oder mehreren Halogenen substituiert ist,
mit der Maßgabe, dass zumindest einer von X¹, X², X³ und X⁴ Halogen, -CN, C₁₋₆-Alkyl oder C₁₋₆-Alkoxy ist, wobei das C₁₋₆-Alkyl oder C₁₋₆-Alkoxy gegebenenfalls mit einem oder mehreren Halogenen substituiert ist.

3. Verbindung nach Anspruch 1 oder Anspruch 2 oder ein Stereoisomer oder ein Tautomer davon oder ein pharmazeutisch annehmbares Salz eines beliebigen der Vorgenannten,
worin L 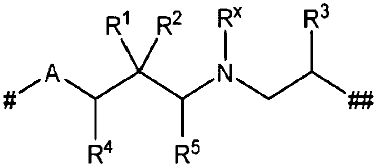 ist, so dass die Verbindung der Formel (I): 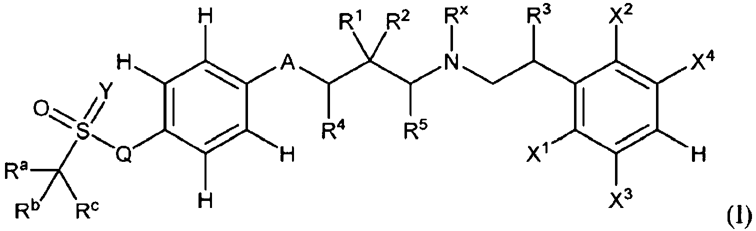
entspricht oder ein Stereoisomer oder ein Tautomer davon oder ein pharmazeutisch annehmbares Salz eines beliebigen der Vorgenannten ist;
wobei gegebenenfalls eines von (a) oder (b) zutrifft:
(a) A ist O, so dass die Verbindung der Formel (I-A1): 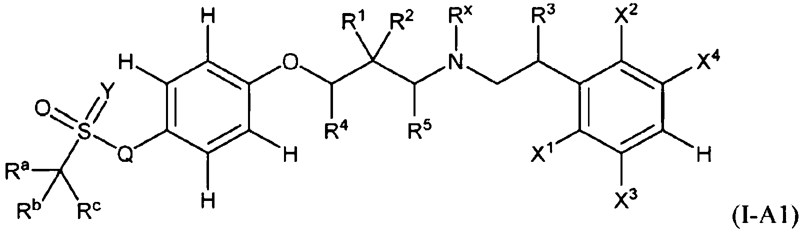 entspricht oder ein Stereoisomer oder ein Tautomer davon oder ein pharmazeutisch annehmbares Salz eines beliebigen der Vorgenannten ist;
(b) A ist CH₂, so dass die Verbindung der Formel (I-A3): 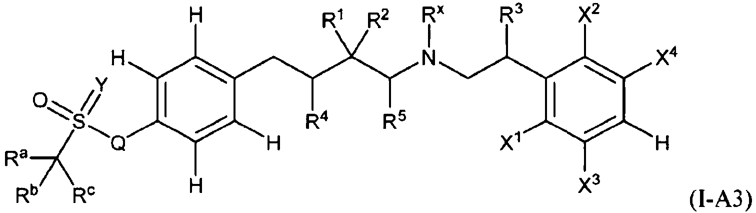
entspricht oder ein Stereoisomer oder ein Tautomer davon oder ein pharmazeutisch annehmbares Salz eines beliebigen der Vorgenannten ist.

4. Verbindung nach Anspruch 3 oder ein Stereoisomer oder ein Tautomer davon oder ein pharmazeutisch annehmbares Salz eines beliebigen der Vorgenannten, wobei die Verbindung der Formel (I-A1) entspricht und entweder:
R^{x} zusammen mit einem von R¹ und R² und den Atomen, an die sie gebunden sind, ein 3- bis 8-gliedriges Heterocyclyl bildet, wobei das 3- bis 8-gliedrige Heterocyclyl mit *n* jeweils unabhängig voneinander ausgewählten Substituenten R^{g} substituiert ist, wobei n eine ganze Zahl von 0 bis 6 ist und R^{g} -OH, Halogen, C₁₋₆-Alkyl oder C₁₋₆-Alkoxy ist; oder
R^{x} zusammen mit einem von R¹ und R² und den Atomen, an die sie gebunden sind, ein 5- bis 6-gliedriges Heterocyclyl bildet, wobei das 3- bis 8-gliedrige Heterocyclyl mit n jeweils unabhängig voneinander ausgewählten Substituenten R^{g} substituiert ist, wobei n eine ganze Zahl von 0 bis 6 ist und R^{g} -OH, Halogen, C₁₋₆-Alkyl oder C₁₋₆-Alkoxy ist.

5. Verbindung nach Anspruch 4 oder ein Stereoisomer oder ein Tautomer davon oder ein pharmazeutisch annehmbares Salz eines beliebigen der Vorgenannten, wobei die Verbindung der Formel (I-B1), (I-C1), (I-D1), (I-E1), (I-F1) oder (I-G1) entspricht: 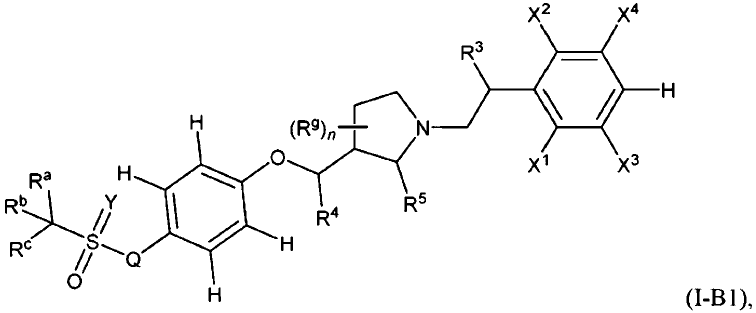 wobei in Formel (I-B1) n gegebenenfalls 0 ist oder n gegebenenfalls 1 oder 2 ist; 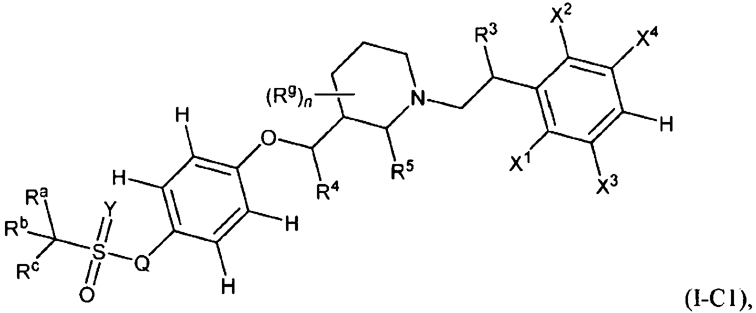 wobei in Formel (I-C1) *n* gegebenenfalls 0 ist oder *n* gegebenenfalls 1 oder 2 ist; 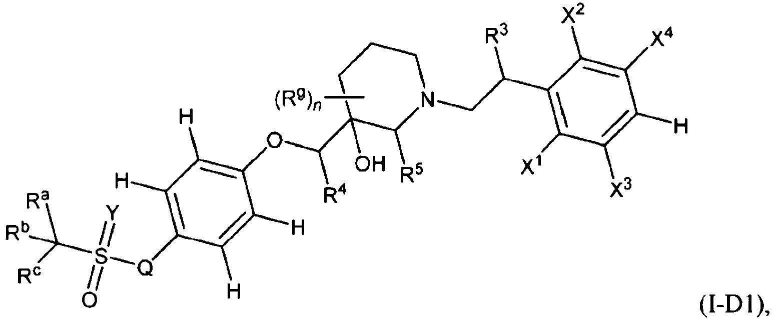 wobei in Formel (I-D1) *n* gegebenenfalls 0 ist oder *n* gegebenenfalls 1 oder 2 ist; 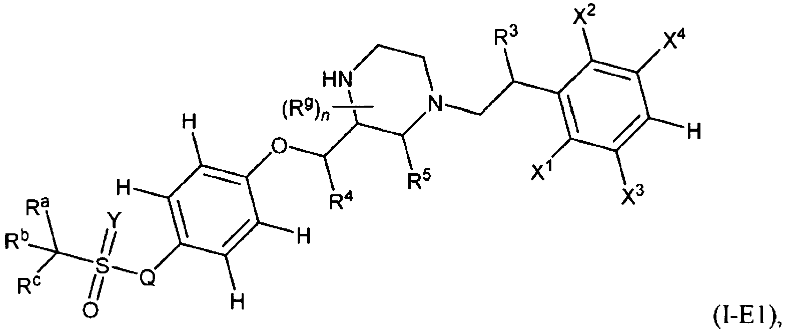 wobei in Formel (I-E1) *n* gegebenenfalls 0 ist; 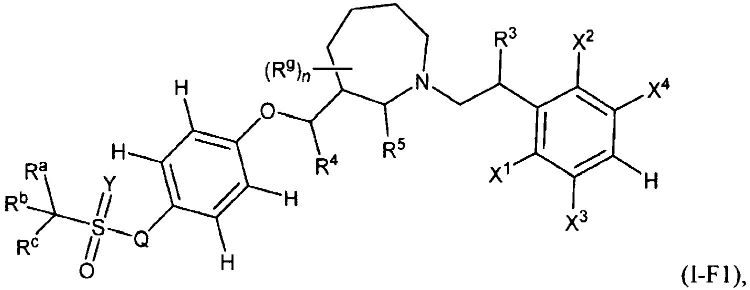 wobei in Formel (I-F1) *n* gegebenenfalls 0 ist; 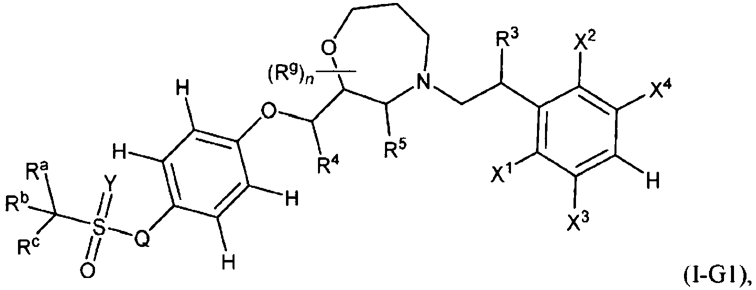 wobei in Formel (I-G1) *n* gegebenenfalls 0 ist;
oder ein Stereoisomer oder ein Tautomer davon oder ein pharmazeutisch annehmbares Salz eines beliebigen der Vorgenannten.

6. Verbindung nach Anspruch 3 oder ein Stereoisomer oder ein Tautomer davon oder ein pharmazeutisch annehmbares Salz eines beliebigen der Vorgenannten, wobei die Verbindung der Formel (I-A) entspricht und worin R⁴ und R⁵ zusammen mit den Atomen, an die sie gebunden sind, ein C₃₋₈-Cycloalkyl bilden, wobei die Verbindung gegebenenfalls der Formel (I-H1): 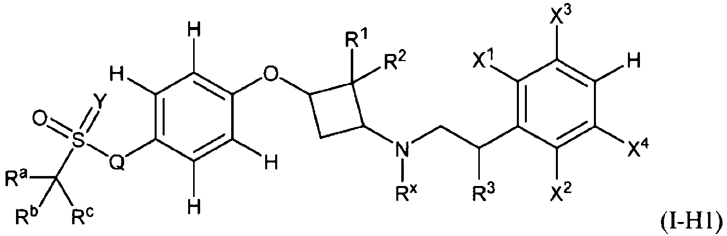
entspricht oder ein Stereoisomer oder ein Tautomer davon oder ein pharmazeutisch annehmbares Salz eines beliebigen der Vorgenannten ist;
worin R¹, R² und R³ gegebenenfalls jeweils H sind.

7. Verbindung nach Anspruch 3 oder ein Stereoisomer oder ein Tautomer davon oder ein pharmazeutisch annehmbares Salz eines beliebigen der Vorgenannten, wobei die Verbindung der Formel (I-A1) entspricht und entweder:
(a) R^{x} zusammen mit einem von R¹ und R² und den Atomen, an die sie gebunden sind, ein 3- bis 8-gliedriges Heterocyclyl bildet, wobei das 3- bis 8-gliedrige Heterocyclyl mit *n* jeweils unabhängig voneinander ausgewählten Substituenten R^{g} substituiert ist, wobei *n* eine ganze Zahl von 0 bis 6 ist und R⁹ -OH, Halogen, C₁₋₆-Alkyl oder C₁₋₆-Alkoxy ist, und der andere von R¹ und R² H, Halogen oder -OH ist; oder
(b) R^{x} zusammen mit einem von R¹ und R² und den Atomen, an die sie gebunden sind, ein 5- bis 6-gliedriges Heterocyclyl bildet, wobei das 5- bis 6-gliedrige Heterocyclyl mit *n* jeweils unabhängig voneinander ausgewählten Substituenten R^{g} substituiert ist, wobei *n* eine ganze Zahl von 0 bis 6 ist und R⁹ -OH, Halogen, C₁₋₆-Alkyl oder C₁₋₆-Alkoxy ist, und der andere von R¹ und R² H, Halogen oder -OH ist;
wobei die Verbindung gegebenenfalls der Formel (I-C3): 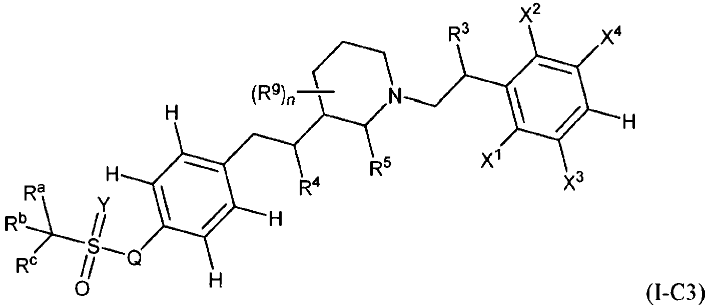
entspricht oder ein Stereoisomer oder ein Tautomer davon oder ein pharmazeutisch annehmbares Salz eines beliebigen der Vorgenannten ist.

8. Verbindung nach Anspruch 1 oder Anspruch 2 oder ein Stereoisomer oder ein Tautomer davon oder ein pharmazeutisch annehmbares Salz eines beliebigen der Vorgenannten,
worin L 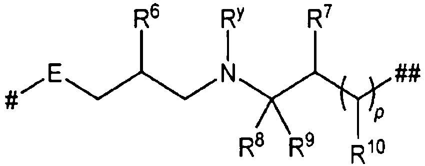 ist, so dass die Verbindung der Formel (II): 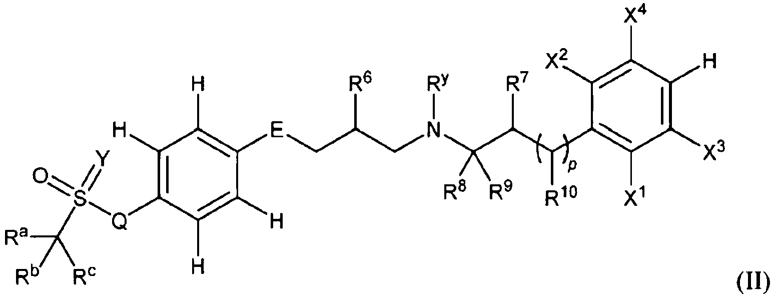
entspricht oder ein Stereoisomer oder ein Tautomer davon oder ein pharmazeutisch annehmbares Salz eines beliebigen der Vorgenannten ist;
worin E gegebenenfalls O ist, so dass die Verbindung der Formel (II-A1): 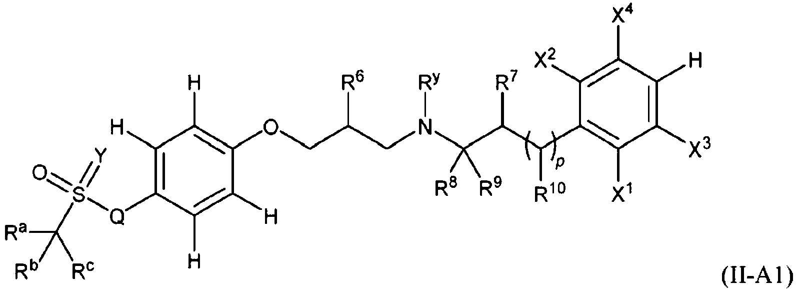
entspricht oder ein Stereoisomer oder ein Tautomer davon oder ein pharmazeutisch annehmbares Salz eines beliebigen der Vorgenannten ist;
worin gegebenenfalls entweder:
(a) R^{y} in Formel (II-A1) zusammen mit R⁷ und den Atomen, an die sie gebunden sind, ein 3- bis 8-gliedriges Heterocyclyl bildet; oder
(b) R^{y} zusammen mit R⁷ und den Atomen, an die sie gebunden sind, ein 5- bis 6-gliedriges Heterocyclyl bildet;
wobei weiters gegebenenfalls einer von R⁸ und R⁹ zusammen mit R¹⁰ und den Atomen, an die sie gebunden sind, ein C₃₋₈-Cycloalkyl bildet.

9. Verbindung nach Anspruch 8 oder ein Stereoisomer oder ein Tautomer davon oder ein pharmazeutisch annehmbares Salz eines beliebigen der Vorgenannten, wobei die Verbindung der Formel (II-B1), (II-C1), (II-D1) oder (II-E1) entspricht: 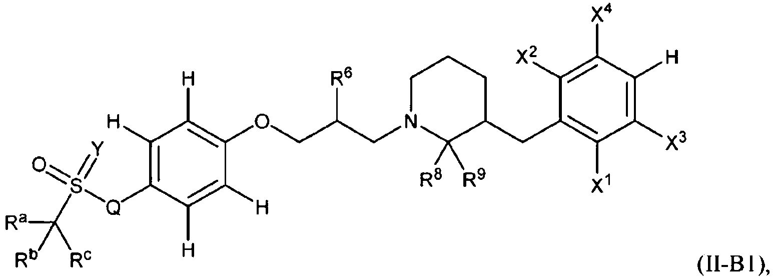 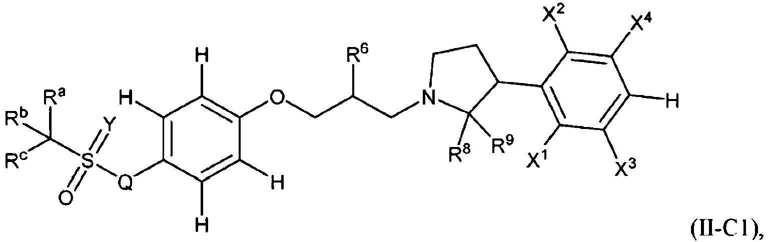 wobei in Formel (II-C1) gegebenenfalls R⁶ -OH ist, R⁸ C₁₋₆-Alkyl ist und R⁹ C₁₋₆-Alkyl ist; 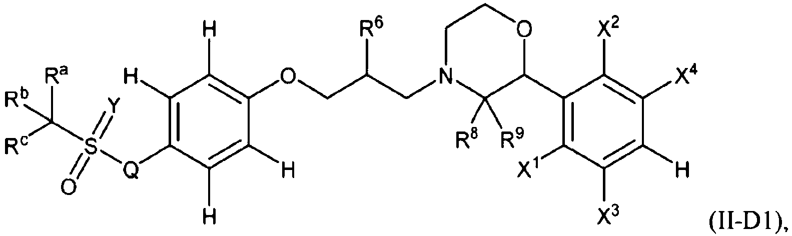 wobei in Formel (II-D1) gegebenenfalls R⁶ -OH ist, R⁸ H ist und R⁹ H ist; 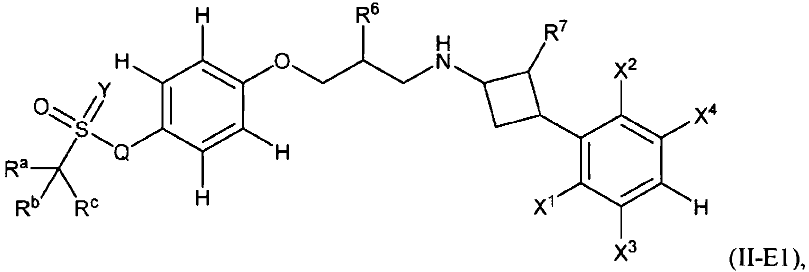 wobei in Formel (II-E1) gegebenenfalls R⁶ -OH ist und R⁷ H ist;
oder ein Stereoisomer oder ein Tautomer davon oder ein pharmazeutisch annehmbares Salz eines beliebigen der Vorgenannten.

10. Verbindung nach Anspruch 1 oder Anspruch 2 oder ein Stereoisomer oder ein Tautomer davon oder ein pharmazeutisch annehmbares Salz eines beliebigen der Vorgenannten, worin L 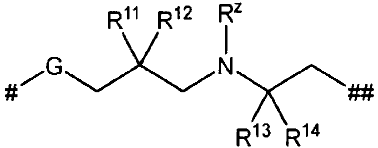 ist, so dass die Verbindung der Formel (III): 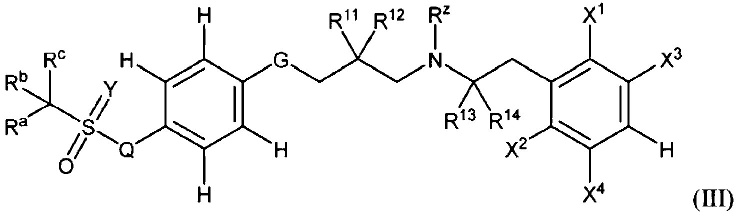 entspricht oder ein Stereoisomer oder Tautomer davon oder ein pharmazeutisch annehmbares Salz eines beliebigen der Vorgenannten ist.

11. Verbindung nach Anspruch 1 oder Anspruch 2 oder ein Stereoisomer oder ein Tautomer davon oder ein pharmazeutisch annehmbares Salz eines beliebigen der Vorgenannten, worin entweder:
(a) Q fehlt, Y O ist und R^{a}, R^{b} und R^{c} jeweils unabhängig voneinander H sind, so dass die Verbindung der Formel (B-2): 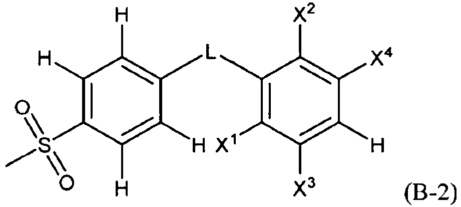 entspricht oder ein Stereoisomer oder Tautomer davon oder ein pharmazeutisch annehmbares Salz eines beliebigen der Vorgenannten ist; oder
(b) Q fehlt, Y -N(CH₃) ist und R^{a}, R^{b} und R^{c} jeweils unabhängig voneinander H sind, so dass die Verbindung der Formel (B-5): 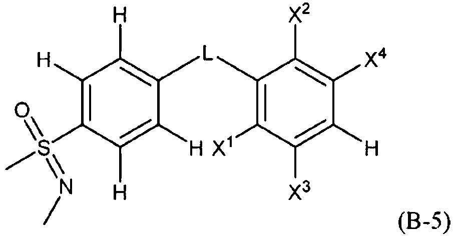 entspricht oder ein Stereoisomer oder Tautomer davon oder ein pharmazeutisch annehmbares Salz eines beliebigen der Vorgenannten ist; oder
(c) Q -N(CH₃) ist, Y O ist und R^{a}, R^{b} und R^{c} jeweils unabhängig voneinander H sind, so dass die Verbindung der Formel (C-1): 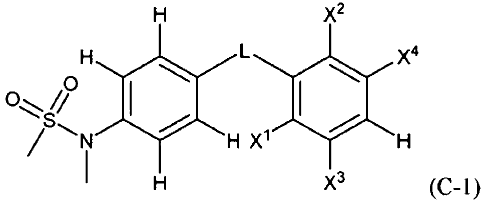 entspricht oder ein Stereoisomer oder Tautomer davon oder ein pharmazeutisch annehmbares Salz eines beliebigen der Vorgenannten ist.

12. Verbindung nach Anspruch 1 oder Anspruch 2 oder ein Stereoisomer oder ein Tautomer davon oder ein pharmazeutisch annehmbares Salz eines beliebigen der Vorgenannten, wobei die Verbindung aus den folgenden ausgewählt ist:
| Verbindung Nr. | Struktur | Chemische Bezeichnung |
|---|---|---|
| 1 | 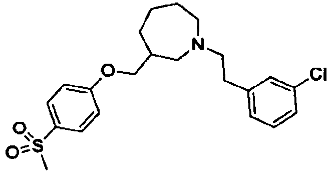 | 1-(3-Chlorphenethyl)-3-((4-(methylsulfonyl)phenoxy)-methyl)azepan |
| 2 | 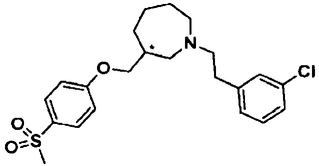 | (R)- oder (S)-1-(3-Chlor-phenethyl)-3-((4-(methylsulfonyl)phenoxy)methyl)-azepan |
| 3 | 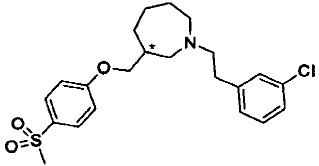 | (S)- oder (R)-1-(3-Chlor-phenethyl)-3-((4-(methylsulfonyl)phenoxy)methyl)-azepan |
| 4 | 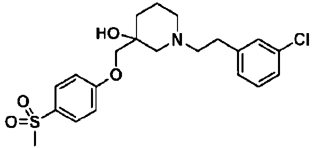 | 1-(3-Chlorphenethyl)-3-((4-(methylsulfonyl)phenoxy)-methyl)piperidin-3-ol |
| 5 | 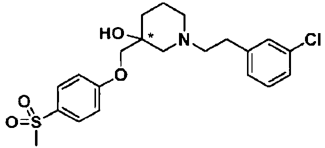 | (R)- oder (S)-1-(3-Chlor-phenethyl)-3-((4-(methylsulfonyl)phenoxy)methyl)-piperidin-3-ol |
| 6 | 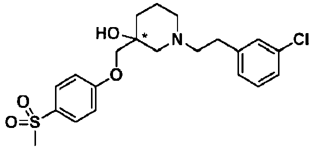 | (S)- oder (R)-1-(3-Chlor-phenethyl)-3-((4-(methylsulfonyl)phenoxy)-methyl)piperidin-3-ol |
| 7 | 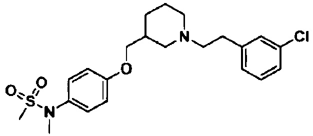 | N-(4-((1-(3-Chlorphenethyl)-piperidin-3-yl)methoxy)phenyl)-N-methylmethansulfonamid |
| 8 | 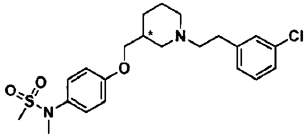 | (S)- oder (R)-N-(4-((1-(3-Chlor-phenethyl)piperidin-3-yl)-methoxy)phenyl)-N-methylmethansulfonamid |
| | | |
| 9 | 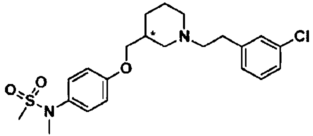 | (R)- oder (S)-N-(4-((1-(3-Chlor-phenethyl)piperidin-3-yl)-methoxy)phenyl)-N-methylmethansulfonamid |
| 10 | 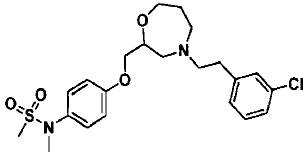 | N-(4-((4-(3-Chlorphenethyl)-1,4-oxazepan-2-yl)-methoxy)phenyl)-N-methylmethansulfonamid |
| 11 | 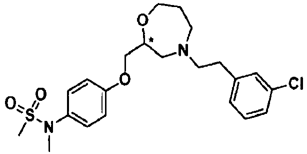 | (R)- oder (S)-N-(4-((4-(3-Chlor-phenethyl)-1,4-oxazepan-2-yl)-methoxy)phenyl)-N-methylmethansulfonamid |
| 12 | 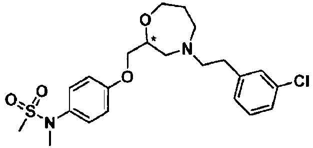 | (S)- oder (R)-N-(4-((4-(3-Chlor-phenethyl)-1,4-oxazepan-2-yl)-methoxy)phenyl)-N-methylmethansulfonamid |
| 13 | 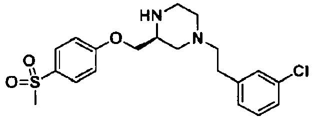 | (S)-1-(3-Chlorphenethyl)-3-((4-(methylsulfonyl)phenoxy)-methyl)piperazin |
| 14 | 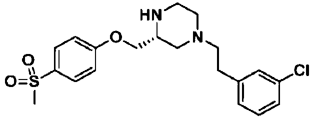 | (R)-1-(3-Chlorphenethyl)-3-((4-(methylsulfonyl)phenoxy)-methyl)piperazin |
| 15 | 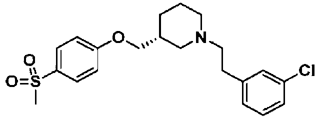 | (R)-1-(3-Chlorphenethyl)-3-((4-(methylsulfonyl)phenoxy)-methyl)piperidin |
| 16 | 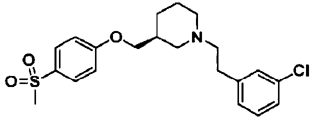 | (S)-1-(3-Chlorphenethyl)-3-((4-(methylsulfonyl)phenoxy)-methyl)piperidin |
| 17 | 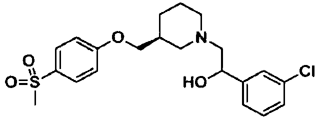 | 1-(3-Chlorphenyl)-2-((S)-3-((4-(methylsulfonyl)phenoxy)-methyl)piperidin-1-yl)ethan-1-ol |
| 18 | 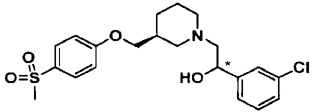 | (R)- oder (S)-1-(3-Chlorphenyl)-2-((S)-3-((4-(methylsulfonyl)phenoxy)methyl)-piperidin-1-yl)ethan-1-ol |
| 19 | 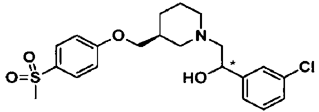 | (S)- oder (R)-1-(3-Chlorphenyl)-2-((S)-3-((4-(methylsulfonyl)phenoxy)methyl)-piperidin-1-yl)ethan-1-ol |
| 20 | 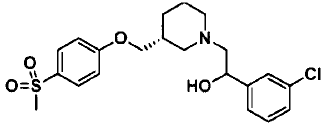 | 1-(3-Chlorphenyl)-2-((R)-3-((4-(methylsulfonyl)phenoxy)-methyl)piperidin-1-yl)ethanol |
| 21 | 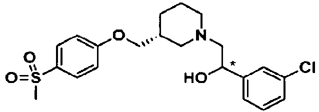 | (R)- oder (S)-1-(3-Chlorphenyl)-2-((R)-3-((4-(methylsulfonyl)phenoxy)methyl)-piperidin-1-yl)ethanol |
| 22 | 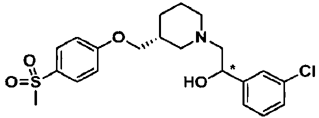 | (S)- oder (R)-1-(3-Chlorphenyl)-2-((R)-3-((4-(methylsulfonyl)phenoxy)methyl)-piperidin-1-yl)ethanol |
| 23 | 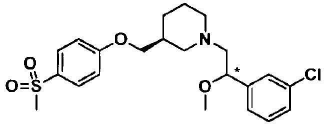 | (S)-1-((R)- oder (S)-2-(3-Chlorphenyl)-2-methoxyethyl)-3-((4-(methylsulfonyl)phenoxy)-methyl)piperidin |
| | | |
| 24 | 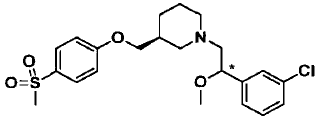 | (S)-1-((S)- oder (R)-2-(3-Chlorphenyl)-2-methoxyethyl)-3-((4-(methylsulfonyl)phenoxy)-methyl)piperidin |
| 25 | 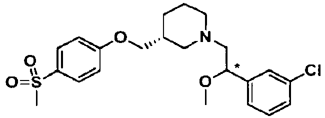 | (R)-1-((R)- oder (S)-2-(3-Chlorphenyl)-2-methoxyethyl)-3-((4-(methylsulfonyl)phenoxy)-methyl)piperidin |
| 26 | 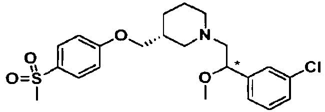 | (R)-1-((S)- oder (R)-2-(3-Chlorphenyl)-2-methoxyethyl)-3-((4-(methylsulfonyl)phenoxy)-methyl)piperidin |
| 27 | 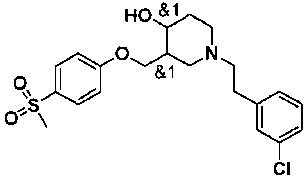 | rac-trans- oder cis-1-(3-Chlor-phenethyl)-3-((4-(methylsulfonyl)phenoxy)methyl)-piperidin-4-ol |
| 28 | 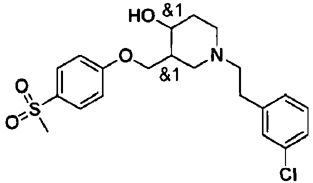 | rac-cis- oder trans-1-(3-Chlor-phenethyl)-3-((4-(methylsulfonyl)phenoxy)methyl)-piperidin-4-ol |
| 29 | 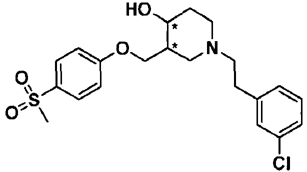 | (3S,4R)- oder (3R,4R)- oder (3S,4S)- oder (3R,4S)-1-(3-Chlorphenethyl)-3-((4-(methylsulfonyl)phenoxy)methyl)-piperidin-4-ol |
| 30 | 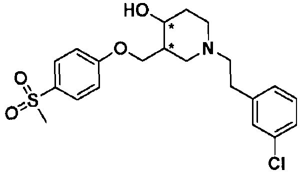 | (3R,4R)- oder (3S,4S)- o der (3R,4S)- oder (3S,4R)-1-(3-Chlorphenethyl)-3-((4-(methylsulfonyl)phenoxy)methyl)-piperidin-4-ol |
| 31 | 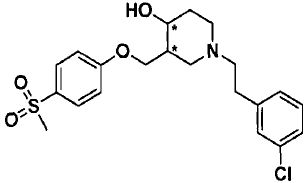 | (3R,4R)- oder (3S,4S)- oder (3R,4S)- oder (3S,4R)-1-(3-Chlorphenethyl)-3-((4-(methylsulfonyl)phenoxy)methyl)-piperidin-4-ol |
| 32 | 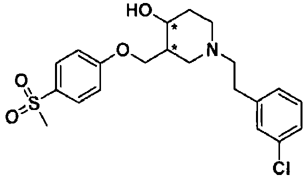 | (3S,4S)- oder (3R,4S)- oder (3S,4R)- oder (3R,4R)-1-(3-Chlorphenethyl)-3-((4-(methylsulfonyl)phenoxy)methyl)-piperidin-4-ol |
| 33 | 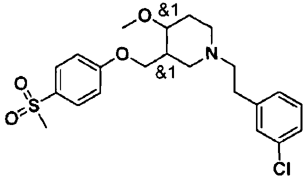 | rac-trans- oder cis-1-(3-Chlorphenethyl)-4-methoxy-3-((4-(methylsulfonyl)phenoxy)-methyl)piperidin |
| 34 | 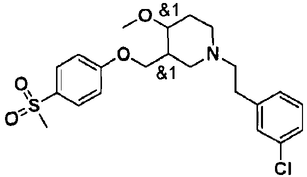 | rac-cis- oder trans-1-(3-Chlorphenethyl)-4-methoxy-3-((4-(methylsulfonyl)phenoxy)-methyl)piperidin |
| 35 | 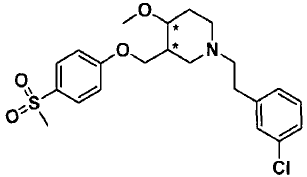 | (3S,4R)- oder (3R,4R)- oder (3S,4S)- oder (3R,4S)-1-(3-Chlorphenethyl)-4-methoxy-3-((4-(methylsulfonyl)phenoxy)-methyl)piperidin |
| | | |
| 36 | 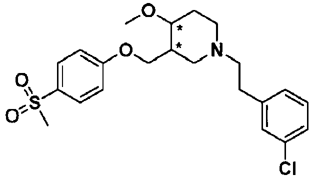 | (3R,4R)- oder (3,4S)- oder (3R,4S)- oder (3S,4R)-1-(3-Chlorphenethyl)-4-methoxy-3-((4-(methylsulfonyl)phenoxy)-methyl)piperidin |
| 37 | 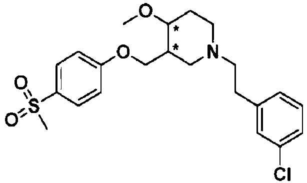 | (3R,4R)- oder (3S,4S)- oder (3R,4S)- oder (3S,4R)-1-(3-Chlorphenethyl)-4-methoxy-3-((4-(methylsulfonyl)phenoxy)-methyl)piperidin |
| 38 | 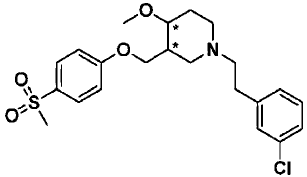 | (3S,4S)- oder (3R,4S)- oder (3S,4R)- oder (3R,4R)-1-(3-Chlorphenethyl)-4-methoxy-3-((4-(methylsulfonyl)phenoxy)-methyl)piperidin |
| 39 | 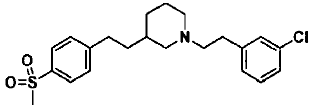 | 1-(3-Chlorphenethyl)-3-(4-(methylsulfonyl)phenethyl)-piperidin |
| 40 | 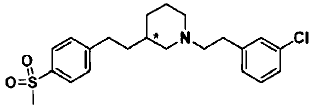 | (S)- oder (R)-1-(3-Chlorphenethyl)-3-(4-(methylsulfonyl)phenethyl)piperidin |
| 41 | 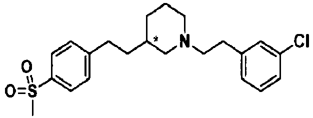 | (R)- oder (S)-1-(3-Chlorphenethyl)-3-(4-(methylsulfonyl)phenethyl)piperidin |
| 42 | 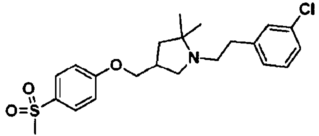 | 1-(3-Chlorphenethyl)-2,2-dimethyl-4-((4-(methylsulfonyl)-phenoxy)methyl)pyrrolidin |
| 43 | 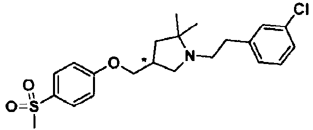 | (S)- oder (R)-1-(3-Chlorphenethyl)-2,2-dimethyl-4-((4-(methylsulfonyl)phenoxy)-methyl)pyrrolidin |
| 44 | 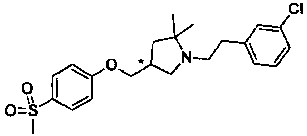 | (R)- oder (S)-1-(3-Chlorphenethyl)-2,2-dimethyl-4-((4-(methylsulfonyl)phenoxy)-methyl)pyrrolidin |
| 45 | 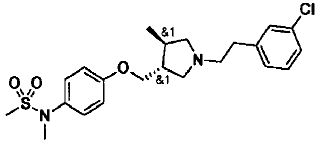 | rac-trans-N-(4-((1-(3-Chlorphenethyl)-4-methylpyrrolidin-3-yl)methoxy)phenyl)-N-methylmethansulfonamid |
| 46 | 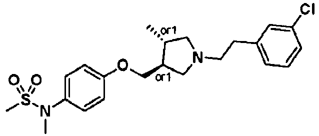 | N-(4-(((3S,4S)- oder (3R,4R)-1-(3-Chlorphenethyl)-4-methylpyrrolidin-3-yl)methoxy)-phenyl)-N-methylmethansulfonamid |
| 47 | 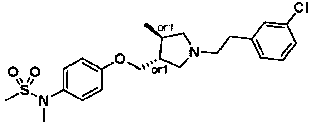 | N-(4-(((3R,4R)- oder (3S,4S)-1-(3-Chlorphenethyl)-4-methylpyrrolidin-3-yl)methoxy)-phenyl)-N-methylmethansulfonamid |
| 48 | 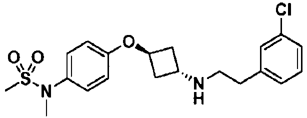 | trans-N-(4-(3-((3-Chlorphenethyl)amino)cyclobutoxy)-phenyl)-N-methylmethansulfonamid |
| 49 | 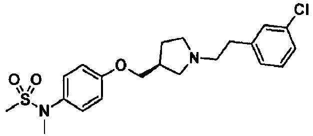 | (S)-N-(4-((1-(3-Chlorphenethyl)pyrrolidin-3-yl)-methoxy)phenyl)-N-methylmethansulfonamid |
| 50 | 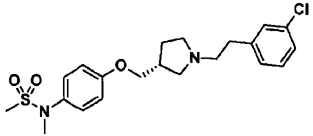 | (R)-N-(4-((1-(3-Chlorphenethyl)pyrrolidin-3-yl)-methoxy)phenyl)-N-methylmethansulfonamid |
| 51 | 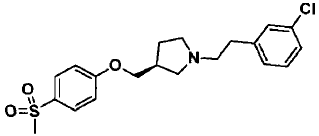 | (S)-1-(3-Chlorphenethyl)-3-((4-(methylsulfonyl)phenoxy)-methyl)pyrrolidin |
| 52 | 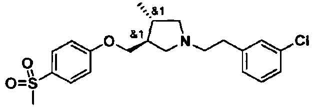 | rac-trans-1-(3-Chlorphenethyl)-3-methyl-4-((4-(methylsulfonyl)-phenoxy)methyl)pyrrolidin |
| 53 | 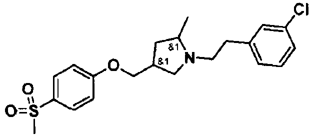 | rac-trans- oder cis-1-(3-Chlorphenethyl)-2-methyl-4-((4-(methylsulfonyl)phenoxy)-methyl)pyrrolidin |
| 54 | 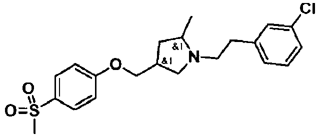 | rac-cis- oder trans-1-(3-Chlorphenethyl)-2-methyl-4-((4-(methylsulfonyl)phenoxy)-methyl)pyrrolidin |
| 55 | 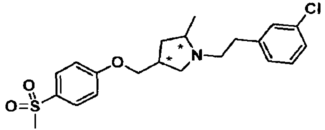 | (2R,4S)- oder (2R,4R)- oder (2S,4R)- oder (2S,4S)-1-(3-Chlorphenethyl)-2-methyl-4-((4-(methylsulfonyl)phenoxy)-methyl)pyrrolidin |
| 56 | 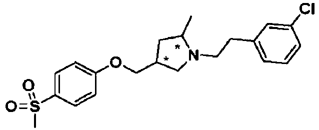 | (2R,4R)- oder (2S,4R)- oder (2S,4S)- oder (2R,4S)-1-(3-Chlorphenethyl)-2-methyl-4-((4-(methylsulfonyl)phenoxy)-methyl)pyrrolidin |
| 57 | 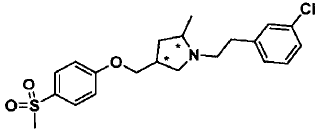 | (2S,4R)- oder (2S,4S)- oder (2R,4S)- oder (2R,4R)-1-(3-Chlorphenethyl)-2-methyl-4-((4-(methylsulfonyl)phenoxy)-methyl)pyrrolidin |
| 58 | 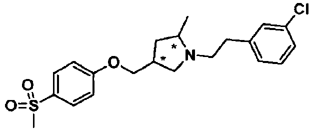 | (2S,4S)- oder (2R,4S)- oder (2R,4R)- oder (2S,4R)-1-(3-Chlorphenethyl)-2-methyl-4-((4-(methylsulfonyl)phenoxy)-methyl)pyrrolidin |
| 59 | 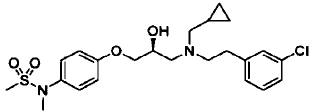 | (S)-N-(4-(3-((3-Chlorphenethyl)(cyclopropylmethyl)-amino)-2-hydroxypropoxy)-phenyl)-N-methylmethansulfonamid |
| 60 | 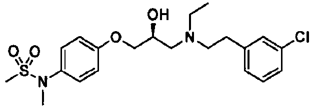 | (S)-N-(4-(3-((3-Chlorphenethyl)(ethyl)amino)-2-hydroxypropoxy)phenyl)-N-methylmethansulfonamid |
| 61 | 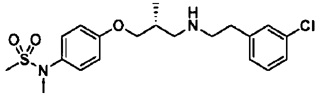 | (R)-N-(4-(3-((3-Chlorphenethyl)amino)-2-methyl-propoxy)phenyl)-N-methylmethansulfonamid |
| 62 | 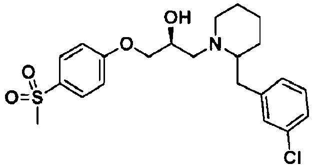 | (2S)-1-(2-(3-Chlorbenzyl)-piperidin-1-yl)-3-(4-(methylsulfonyl)phenoxy)propan-2-ol |
| 63 | 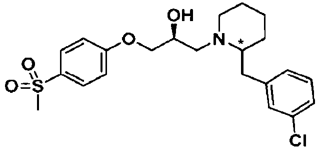 | (S)-1-((S)- oder (R)-2-(3-Chlorbenzyl)piperidin-1-yl)-3-(4-(methylsulfonyl)phenoxy)-propan-2-ol |
| 64 | 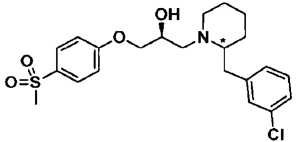 | (S)-1-((R)- oder (S)-2-(3-Chlorbenzyl)piperidin-1-yl)-3-(4-(methylsulfonyl)phenoxy)-propan-2-ol |
| 65 | 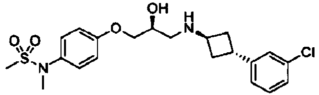 | N-(4-((S)-3-((trans-3-(3-Chlorphenyl)cyclobutyl)amino)-2-hydroxypropoxy)phenyl)-N-methylmethansulfonamid |
| 66 | 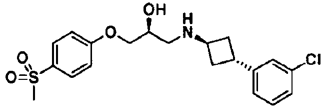 | (S)-1-((trans-3-(3-Chlorphenyl)-cyclobutyl)amino)-3-(4-(methylsulfonyl)phenoxy)propan-2-ol |
| 67 | 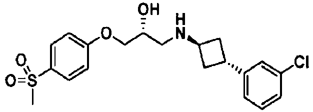 | (R)-1-((trans-3-(3-Chlorphenyl)-cyclobutyl)amino)-3-(4-(methylsulfonyl)phenoxy)propan-2-ol |
| 68 | 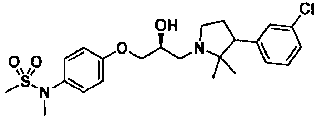 | N-(4-((2S)-3-(3-(3-Chlorphenyl)-2,2-dimethylpyrrolidin-1-yl)-2-hydroxypropoxy)-phenyl)-N-methylmethansulfonamid |
| 69 | 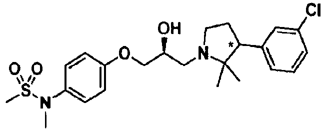 | N-(4-((S)-3-((S)- oder (R)-3-(3-Chlorphenyl)-2,2-dimethylpyrrolidin-1-yl)-2-hydroxypropoxy)phenyl)-N-methylmethansulfonamid |
| 70 | 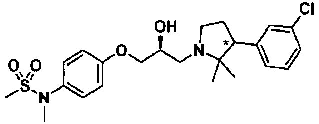 | N-(4-((S)-3-((R)- oder (S)-3-(3-Chlorphenyl)-2,2-dimethylpyrrolidin-1-yl)-2-hydroxypropoxy)phenyl)-N-methylmethansulfonamid |
| 71 | 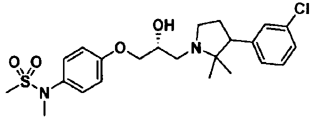 | N-(4-((2R)-3-(3-(3-Chlorphenyl)-2,2-dimethylpyrrolidin-1-yl)-2-hydroxypropoxy)-phenyl)-N-methylmethansulfonamid |
| 72 | 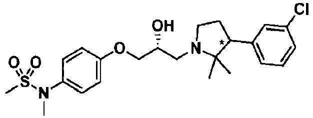 | N-(4-((R)-3-((S)- oder (R)-3-(3-Chlorphenyl)-2,2-dimethylpyrrolidin-1-yl)-2-hydroxypropoxy)phenyl)-N-methylmethansulfonamid |
| 73 | 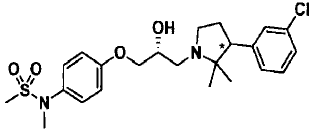 | N-(4-((R)-3-((R)- oder (S)-3-(3-Chlorphenyl)-2,2-dimethylpyrrolidin-1-yl)-2-hydroxypropoxy)phenyl)-N-methylmethansulfonamid |
| 74 | 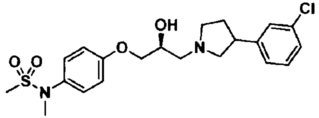 | N-(4-((2S)-3-(3-(3-Chlorphenyl)pyrrolidin-1-yl)-2-hydroxypropoxy)phenyl)-N-methylmethansulfonamid |
| 75 | 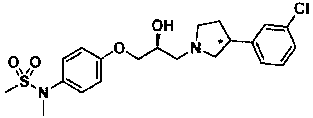 | N-(4-((S)-3-((S)- oder (R)-3-(3-Chlorphenyl)pyrrolidin-1-yl)-2-hydroxypropoxy)phenyl)-N-methylmethansulfonamid |
| 76 | 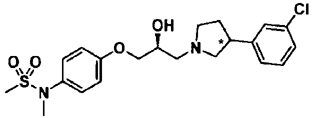 | N-(4-((S)-3-((R)- oder (S)-3-(3-Chlorphenyl)pyrrolidin-1-yl)-2-hydroxypropoxy)phenyl)-N-methylmethansulfonamid |
| 77 | 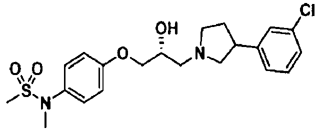 | N-(4-((2R)-3-(3-(3-Chlorphenyl)pyrrolidin-1-yl)-2-hydroxypropoxy)phenyl)-N-methylmethansulfonamid |
| 78 | 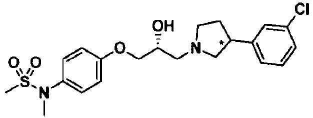 | N-(4-((R)-3-((S)- oder (R)-3-(3-Chlorphenyl)pyrrolidin-1-yl)-2-hydroxypropoxy)phenyl)-N-methylmethansulfonamid |
| 79 | 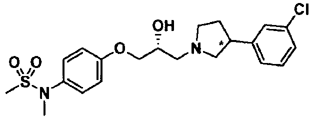 | N-(4-((R)-3-((R)- oder (S)-3-(3-Chlorphenyl)pyrrolidin-1-yl)-2-hydroxypropoxy)phenyl)-N-methylmethansulfonamid |
| 80 | 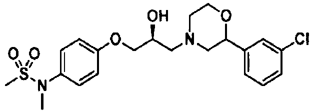 | N-(4-((2S)-3-(2-(3-Chlorphenyl)morpholino)-2-hydroxypropoxy)phenyl)-N-methylmethansulfonamid |
| 81 | 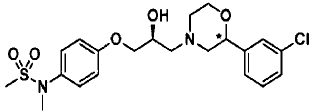 | N-(4-((S)-3-((S)- oder (R)-2-(3-Chlorphenyl)morpholino)-2-hydroxypropoxy)phenyl)-N-methylmethansulfonamid |
| 82 | 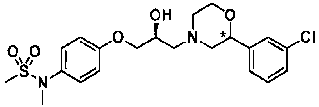 | N-(4-((S)-3-((R)- oder (S)-2-(3-Chlorphenyl)morpholino)-2-hydroxypropoxy)phenyl)-N-methylmethansulfonamid |
| 83 | 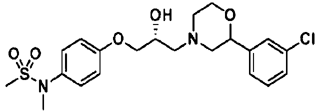 | N-(4-((2R)-3-(2-(3-Chlorphenyl)morpholino)-2-hydroxypropoxy)phenyl)-N-methylmethansulfonamid |
| 84 | 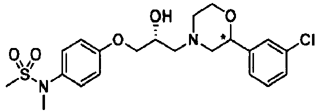 | N-(4-((R)-3-((R)- oder (S)-2-(3-Chlorphenyl)morpholino)-2-hydroxypropoxy)phenyl)-N-methylmethansulfonamid |
| 85 | 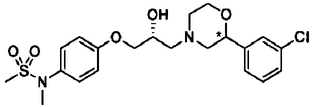 | N-(4-((R)-3-((S)- oder (R)-2-(3-Chlorphenyl)morpholino)-2-hydroxypropoxy)phenyl)-N-methylmethansulfonamid |
| 86 | 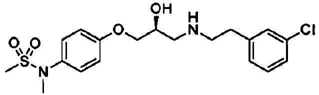 | (S)-N-(4-(3-((3-Chlorphenethyl)amino)-2-hydroxypropoxy)phenyl)-N-methylmethansulfonamid |
| 87 | 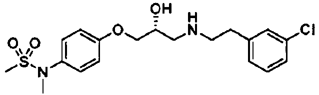 | (R)-N-(4-(3-((3-Chlorphenethyl)amino)-2-hydroxypropoxy)phenyl)-N-methylmethansulfonamid |
| 88 | 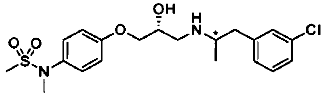 | N-(4-((R)-3-(((S)- oder (R)-1-(3-Chlorphenyl)propan-2-yl)-amino)-2-hydroxypropoxy)-phenyl)-N-methylmethansulfonamid |
| 89 | 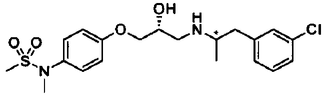 | N-(4-((R)-3-(((R)- oder (S)-1-(3-Chlorphenyl)propan-2-yl)-amino)-2-hydroxypropoxy)-phenyl)-N-methylmethansulfonamid |
| 90 | 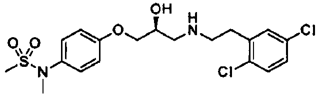 | (S)-N-(4-(3-((2,5-Dichlorphenethyl)amino)-2-hydroxypropoxy)phenyl)-N-methylmethansulfonamid |
| 91 | 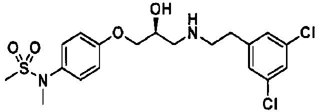 | (S)-N-(4-(3-((3,5-Dichlorphenethyl)amino)-2-hydroxypropoxy)phenyl)-N-methylmethansulfonamid |
| 92 | 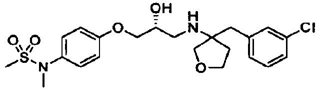 | N-(4-((2R)-3-((3-(3-Chlorbenzyl)tetrahydrofuran-3-yl)-amino)-2-hydroxypropoxy)-phenyl)-N-methylmethansulfonamid |
| 93 | 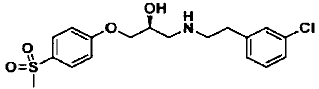 | (S)-1-((3-Chlorphenethyl)-amino)-3-(4-(methylsulfonyl)-phenoxy)propan-2-ol |
| 94 | 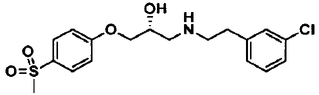 | (R)-1-((3-Chlorphenethyl)-amino)-3-(4-(methylsulfonyl)-phenoxy)propan-2-ol |
| 95 | 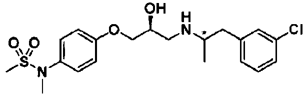 | N-(4-((S)-3-(((S)- oder (R)-1-(3-Chlorphenyl)propan-2-yl)-amino)-2-hydroxypropoxy)-phenyl)-N-methylmethansulfonamid |
| 96 | 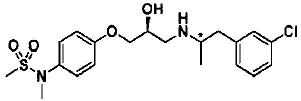 | N-(4-((S)-3-(((R)- oder (S)-1-(3-Chlorphenyl)propan-2-yl)-amino)-2-hydroxypropoxy)-phenyl)-N-methylmethansulfonamid |
| 97 | 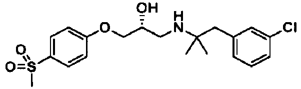 | (R)-1-((1-(3-Chlorphenyl)-2-methylpropan-2-yl)amino)-3-(4-(methylsulfonyl)phenoxy)-propan-2-ol |
| 98 | 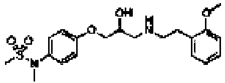 | (S)-N-(4-(2-Hydroxy-3-((2-methoxyphenethyl)amino)-propoxy)phenyl)-N-methylmethansulfonamid |
| 99 | 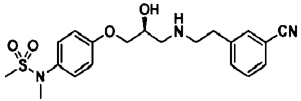 | (S)-N-(4-(3-((3-Cyanophenethyl)amino)-2-hydroxypropoxy)phenyl)-N-methylmethansulfonamid |
| 100 | 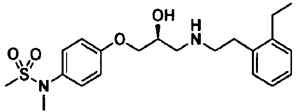 | (S)-N-(4-(3-((2-Ethylphenethyl)-amino)-2-hydroxypropoxy)-phenyl)-N-methylmethansulfonamid |
| 101 | 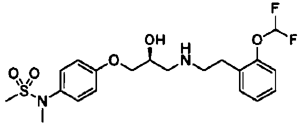 | (S)-N-(4-(3-((2-(Difluormethoxy)phenethyl)amino)-2-hydroxypropoxy)phenyl)-N-methylmethansulfonamid |
| 102 | 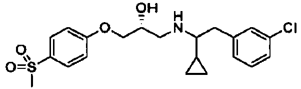 | (2R)-1-((2-(3-Chlorphenyl)-1-cyclopropylethyl)amino)-3-(4-(methylsulfonyl)phenoxy)-propan-2-ol |
| 103 | 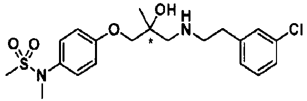 | (R)- oder (S)-N-(4-(3-((3-Chlorphenethyl)amino)-2-hydroxy-2-methylpropoxy)phenyl)-N-methylmethansulfonamid |
| 104 | 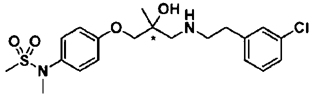 | (S)- oder (R)-N-(4-(3-((3-Chlorphenethyl)amino)-2-hydroxy-2-methylpropoxy)phenyl)-N-methylmethansulfonamid |
| 105 | 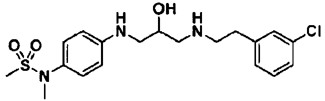 | N-(4-((3-((3-Chlorphenethyl)-amino)-2-hydroxypropyl)-amino)phenyl)-N-methylmethansulfonamid |
| 106 | 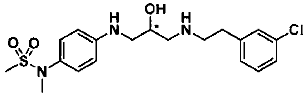 | (R)- oder (S)-N-(4-((3-((3-Chlorphenethyl)amino)-2-hydroxypropyl)amino)phenyl)-N-methylmethansulfonamid |
| 107 | 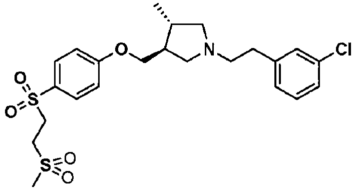 | (3S,4S)-1-[2-(3-Chlorphenyl)-ethyl]-3-{[4-(2-methansulfonylethansulfonyl)phenoxy]methyl}-4-methylpyrrolidin |
| 108 | 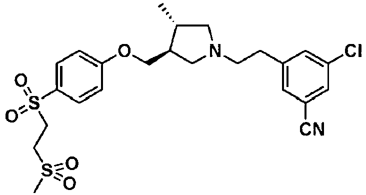 | 3-Chlor-5-{2-[(3S,4S)-3-{[4-(2-methansulfonylethansulfonyl)-phenoxy]methyl}-4-methylpyrrolidin-1-yl]ethyl}benzonitril |
| 109 | 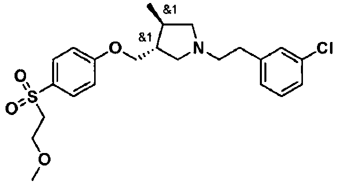 | rac-trans-1-[2-(3-Chlorphenyl)-ethyl]-3-{[4-(2-methoxyethan-sulfonyl)phenoxy]methyl}-4-methylpyrrolidin |
| 110 | 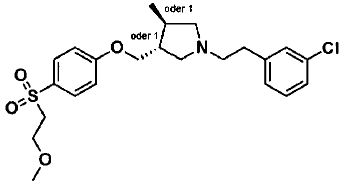 | (3R,4R oder 3S,4S)-1-[2-(3-Chlorphenyl)ethyl]-3-{[4-(2-methoxyethansulfonyl)-phenoxy]methyl}-4-methylpyrrolidin |
| 111 | 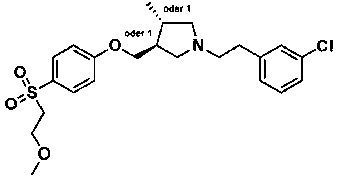 | (3S,4S oder 3R,4R)-1-[2-(3-Chlorphenyl)ethyl]-3-{[4-(2-methoxyethansulfonyl)-phenoxy]methyl}-4-methylpyrrolidin |
| 112 | 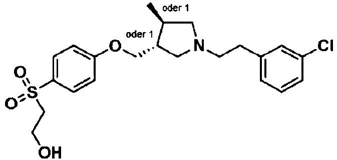 | 2-(4-{[(3R,4R oder 3S,4S)-1-[2-(3-Chlorphenyl)ethyl]-4-methylpyrrolidin-3-yl]methoxy}-benzolsulfonyl)ethan-1-ol |
| 113 | 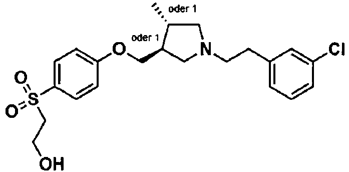 | 2-(4-{[(3S,4S oder 3R,4R)-1-[2-(3-Chlorphenyl)ethyl]-4-methylpyrrolidin-3-yl]methoxy}-benzolsulfonyl)ethan-1-ol |
| 114 | 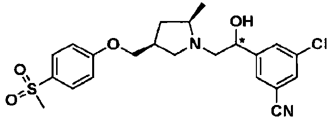 | 3-Chlor-5-[(1S oder 1R)-1-hydroxy-2-[(2R,4S)-4-[(4-methansulfonylphenoxy)-methyl]-2-methylpyrrolidin-1-yl]-ethyl]benzonitril |
| 115 | 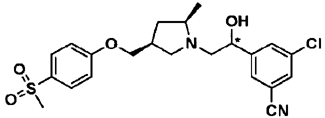 | 3-Chlor-5-[(1R oder 1S)-1-hydroxy-2-[(2R,4S)-4-[(4-methansulfonylphenoxy)-methyl]-2-methylpyrrolidin-1-yl]ethyl]benzonitril |
| 116 | 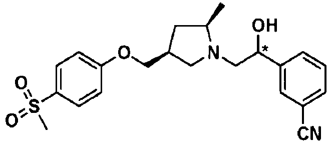 | 3-[(1 R oder 1S)-1-Hydroxy-2-[(2R,4S)-4-[(4-methansulfonyl-phenoxy)methyl]-2-methylpyrrolidin-1-yl]ethyl]benzonitril |
| 117 | 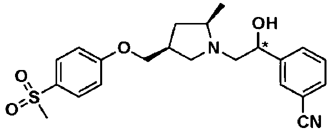 | 3-[(1S oder 1R)-1-Hydroxy-2-[(2R,4S)-4-[(4-methansulfonyl-phenoxy)methyl]-2-methylpyrrolidin-1-yl]ethyl]benzonitril |
| 118 | 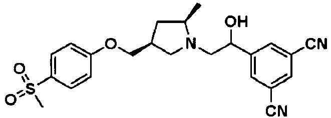 | 5-[(1R oder 1S)-1-Hydroxy-2-[(2R,4S)-4-[(4-methansulfonyl-phenoxy)methyl]-2-methylpyrrolidin-1-yl]ethyl]benzol-1,3-dicarbonitril |
| 119 | 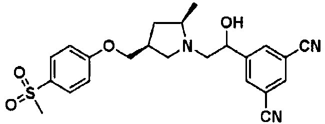 | 5-[(1S oder 1R)-1-Hydroxy-2-[(2R,4S)-4-[(4-methansulfonyl-phenoxy)methyl]-2-methylpyrrolidin-1-yl]ethyl]benzol-1,3-dicarbonitril |
| 120 | 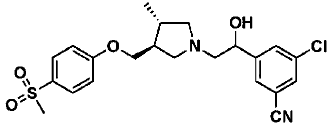 | 3-Chlor-5-{1-hydroxy-2-[(3S,4S)-3-[(4-methansulfonyl-phenoxy)methyl]-4-methylpyrrolidin-1-ylethyl}benzonitril |
| 121 | 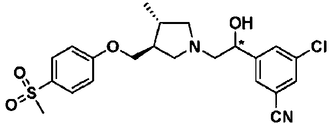 | 3-Chlor-5-[(1Sor1R)-1-hydroxy-2-[(3S,4S)-3-[(4-methan-sulfonylphenoxy)methyl]-4-methylpyrrolidin-1-yl]ethyl]-benzonitril |
| 122 | 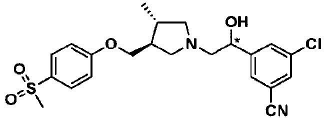 | 3-Chlor-5-[(1R or 1S)-1-hydroxy-2-[(3S,4S)-3-[(4-methan-sulfonylphenoxy)methyl]-4-methylpyrrolidin-1-yl]ethyl]-benzonitril |
| 123 | 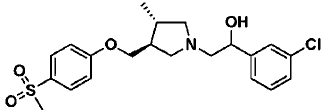 | 1-(3-Chlorphenyl)-2-((3S,4S)-3-methyl-4-((4-(methylsulfonyl-)phenoxy)methyl)pyrrolidin-1-yl)ethan-1-ol |
| 124 | 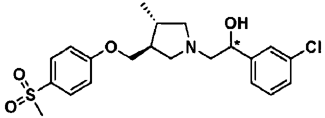 | (1R or 1S)-1-(3-Chlorphenyl)-2-[(3S,4S)-3-[(4methansulfonyl-phenoxy)methyl]-4-methylpyrrolidin-1-yl]ethan-1-ol |
| 125 | 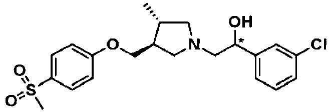 | (1S oder 1R)-1-(3-Chlor-phenyl)-2-[(3S,4S)-3-[(4-methansulfonylphenoxy)-methyl]-4-methylpyrrolidin-1-yl]-ethan-1-ol |
| 126 | 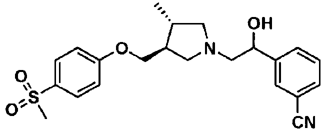 | 3-[1-Hydroxy-2-[(3S,4S)-3-[(4-methansulfonylphenoxy)-methyl]-4-methylpyrrolidin-1-yl]-ethyl]benzonitril |
| 127 | 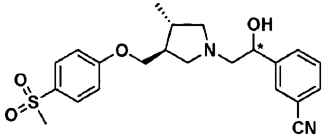 | 3-[(1S oder 1R)-1-Hydroxy-2-[(3S,4S)-3-[(4-methansulfonyl-phenoxy)methyl]-4-methylpyrrolidin-1-yl]ethyl]benzonitril |
| 128 | 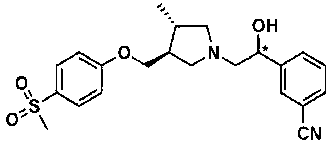 | 3-[(1R oder 1S)-1-Hydroxy-2-[(3S,4S)-3-[(4-methansulfonyl-phenoxy)methyl]-4-methylpyrrolidin-1-yl]ethyl]benzonitril |
| 129 | 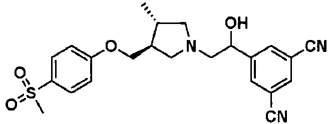 | 5-{1-Hydroxy-2-[(3S,4S)-3-[(4-methansulfonylphenoxy)-methyl]-4-methylpyrrolidin-1-yl]-ethyl}benzol-1,3-dicarbonitril |
| 130 | 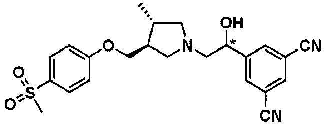 | 5-[(1R oder 1S)-1-Hydroxy-2-[(3S,4S)-3-[(4-methansulfonyl-phenoxy)methyl]-4-methylpyrrolidin-1-yl]ethyl]benzol-1,3-dicarbonitril |
| 131 | 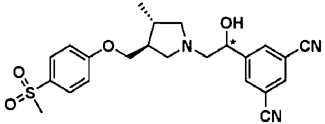 | 5-[(1S oder 1R)-1-Hydroxy-2-[(3S,4S)-3-[(4-methansulfony-lphenoxy)methyl]-4-methylpyrrolidin-1-yl]ethyl]benzol-1,3-dicarbonitril |
| 132 | 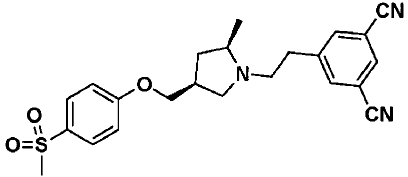 | 5-{2-[(2R,4S)-4-[(4-Methan-sulfonylphenoxy)methyl]-2-methylpyrrolidin-1-ylethyl}-benzol-1,3-dicarbonitril |
| 133 | 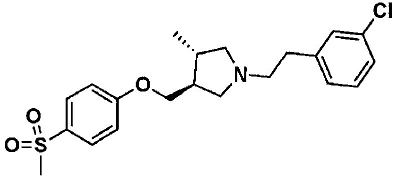 | (3S,4S)-1-[2-(3-Chlorphenyl)-ethyl]-3-[(4-methansulfonyl-phenoxy)methyl]-4-methylpyrrolidin |
| 134 | 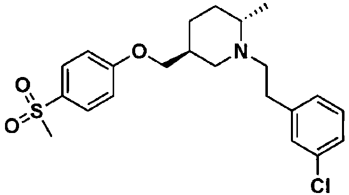 | (2S,5S)-1-[2-(3-Chlorphenyl)-ethyl]-5-[(4-methansulfonyl-phenoxy)methyl]-2-methyl-piperidin |
| 135 | 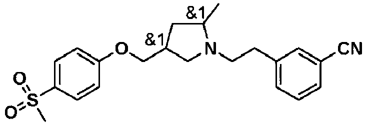 | rac-cis- oder trans-3-{2-[4-[(4-Methansulfonylphenoxy)-methyl]-2-methylpyrrolidin-1-yl]-ethyl}benzonitril |
| 136 | 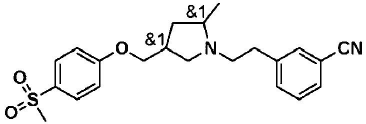 | rac-trans- oder cis-3-{2-[4-[(4-Methansulfonylphenoxy)-methyl]-2-methylpyrrolidin-1-yl]-ethyl}benzonitril |
| 137 | 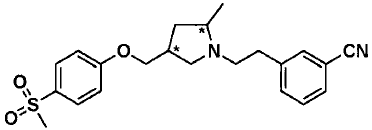 | 3-{2-[(25,4R oder 2R,4S oder 2R,4R oder 25,4S)-4-[(4-Methansulfonylphenoxy)-methyl]-2-methylpyrrolidin-1-yl-ethyl}benzonitril |
| 138 | 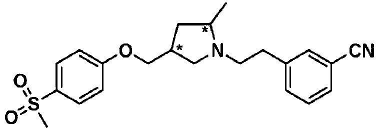 | 3-{2-[(2R,4S oder 2S,4R oder 2R,4R oder 2S,4S)-4-[(4-Methansulfonylphenoxy)-methyl]-2-methylpyrrolidin-1-yl]-ethyl}benzonitril |
| 139 | 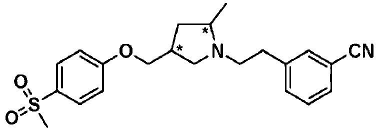 | 3-{2-[(2S,4S oder 2S,4R oder 2R,4S oder 2R,4R)-4-[(4-Methansulfonylphenoxy)-methyl]-2-methylpyrrolidin-1-yl-ethyl}benzonitril |
| 140 | 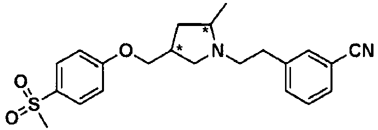 | 3-{2-[(2R,4R oder 2S,4S oder 2S,4R oder 2R,4S)-4-[(4-Methansulfonylphenoxy)-methyl]-2-methylpyrrolidin-1-yl]-ethyl}benzonitril |
| 141 | 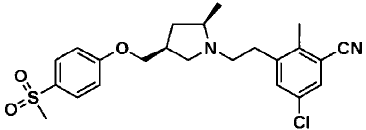 | 5-Chlor-3-{2-[(2R,4S)-4-[(4-methansulfonylphenoxy)-methyl]-2-methylpyrrolidin-1-yl]-ethyl}-2-methylbenzonitril |
| 142 | 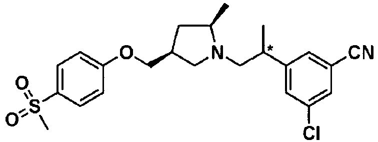 | 3-Chlor-5-[(2R oder 2S)-1-[(2R,4S)-4-[(4-Methansulfonyl-phenoxy)methyl]-2-methylpyrrolidin-1-yl]propan-2-yl]-benzonitril |
| 143 | 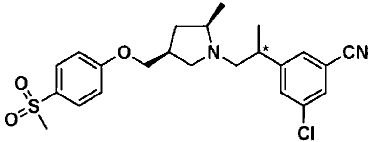 | 3-Chlor-5-[(2S oder 2R)-1-[(2R,4S)-4-[(4-Methansulfonyl-phenoxy)methyl]-2-methylpyrrolidin-1-yl]propan-2-yl]-benzonitril |
| 144 | 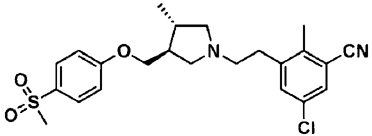 | 5-Chlor-3-{2-[(3S,4S)-3-[(4-methansulfonylphenoxy)-methyl]-4-methylpyrrolidin-1-yl-ethyl}-2-methylbenzonitril |
| 145 | 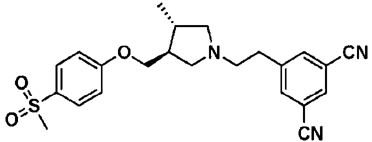 | 5-{2-[(3S,4S)-3-[(4-Methan-sulfonylphenoxy)methyl]-4-methylpyrrolidin-1-ylethyl}-benzol-1,3-dicarbonitril |
| 146 | 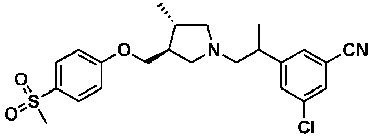 | 3-Chlor-5-[1-[(3S,4S)-3-[(4-methansulfonylphenoxy)-methyl]-4-methylpyrrolidin-1-yl]-propan-2-yl]benzonitril |
| 147 | 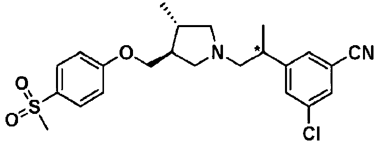 | 3-Chlor-5-[(2R oder 2S)-1-[(3S,4S)-3-[(4-Methansulfonyl-phenoxy)methyl]-4-methylpyrrolidin-1-yl]propan-2-yl]-benzonitril |
| 148 | 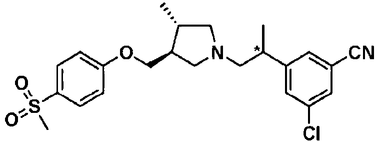 | 3-Chlor-5-[(2S oder 2R)-1-[(3S,4S)-3-[(4-Methansulfonyl-phenoxy)methyl]-4-methylpyrrolidin-1-yl]propan-2-yl]-benzonitril |
| 149 | 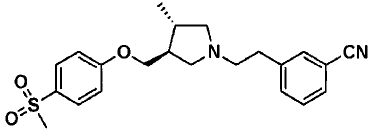 | 3-{2-[(3S,4S)-3-[(4-Methan-sulfonylphenoxy)methyl]-4-methylpyrrolidin-1-yl]ethyl}-benzonitril |
| 150 | 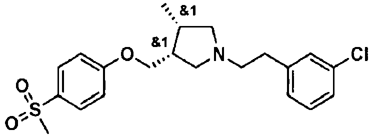 | rac-cis-1-[2-(3-Chlorphenyl)-ethyl]-3-[(4-methansulfonyl-phenoxy)methyl]-4-methylpyrrolidin |
| 151 | 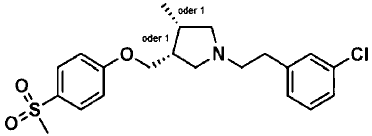 | (3R,4S oder 3S,4R)-1-[2-(3-Chlorphenyl)ethyl]-3-[(4-methansulfonylphenoxy)-methyl]-4-methylpyrrolidin |
| 152 | 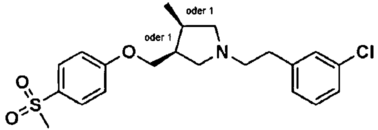 | (3S,4R oder 3R,4S)-1-[2-(3-Chlorphenyl)ethyl]-3-[(4-methansulfonylphenoxy)-methyl]-4-methylpyrrolidin |
| 153 | 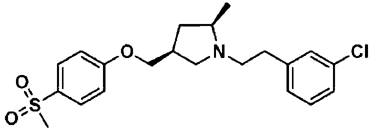 | (2R,4S)-1-[2-(3-Chlorphenyl)-ethyl]-4-[(4-methansulfonyl-phenoxy)methyl]-2-methylpyrrolidin |
| 154 | 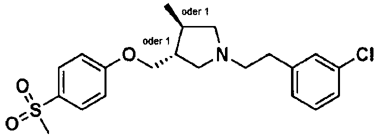 | (3R,4R oder 3S,4S)-1-[2-(3-Chlorphenyl)ethyl]-3-[(4-methansulfonylphenoxy)-methyl]-4-methylpyrrolidin |
| 155 | 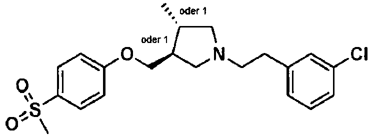 | (3S,4S oder 3R,4R)-1-[2-(3-Chlorphenyl)ethyl]-3-[(4-methansulfonylphenoxy)-methyl]-4-methylpyrrolidin |
| 156 | 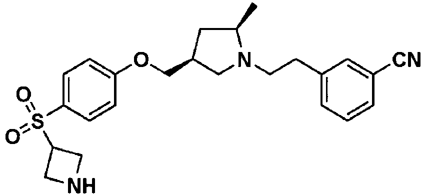 | 3-{2-[(2R,4S)-4-{[4-(Azetidin-3-sulfonyl)phenoxy]methyl}-2-methylpyrrolidin-1-yl]ethyl}-benzonitril |
| 157 | 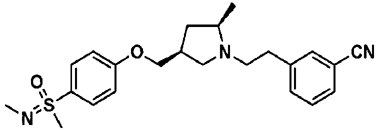 | 3-{2-[(2R,4S)-2-Methyl-4-({4-[methyl(methylimino)oxo-λ⁶-sulfanyl]phenoxy}methyl)-pyrrolidin-1-yl]ethyl}benzonitril |
| 158 | 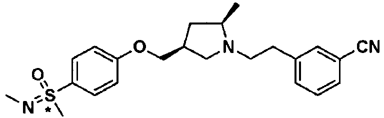 | 3-{2-[(2R,4S)-2-Methyl-4-({4-[(R oder S)methyl(methyl-imino)oxo-λ⁶-sulfanyl]phenoxy}-methyl)pyrrolidin-1-yl]ethyl}-benzonitril |
| 159 | 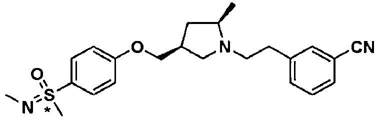 | 3-{2-[(2R,4S)-2-Methyl-4-({4-[(S oder R)-methyl(methyl-imino)oxo-λ⁶-sulfanyl]phenoxy}-methyl)pyrrolidin-1-yl]ethyl}-benzonitril |
| 160 | 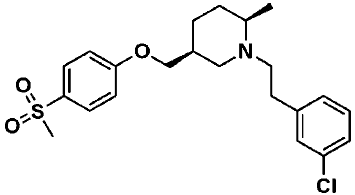 | (2R,5S)-1-[2-(3-Chlorphenyl)-ethyl]-5-[(4-methansulfonyl-phenoxy)methyl]-2-methyl-piperidin |
| 161 | 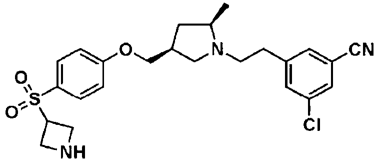 | 3-{2-[(2R,4S)-4-{[4-(Azetidin-3-sulfonyl)phenoxy]methyl}-2-methylpyrrolidin-1-ylethyl}-5-chlorbenzonitril |
| 162 | 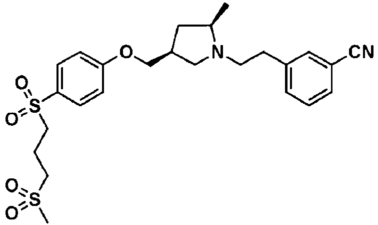 | 3-{2-[(2R,4S)-4-{[4-(3-Methan-sulfonylpropansulfonyl)phenoxy ]methyl}-2-methylpyrrolidin-1-ylethyl}benzonitril |
| 163 | 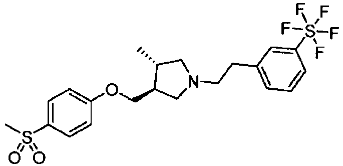 | (3S,4S)-3-[(4-Methansulfonyl-phenoxy)methyl]-4-methyl-1-{2-[3-(pentafluor-λ⁶-sulfanyl)-phenyl]ethyl}pyrrolidin |
| 164 | 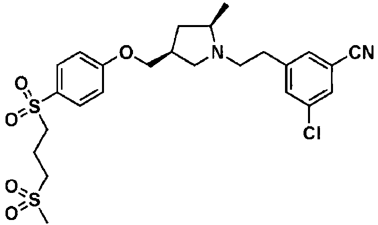 | 3-Chlor-5-{2-[(2R,4S)-4-{[4-(3-methansulfonylpropansulfonyl) phenoxy]methyl}-2-methylpyrrolidin-1-yl]ethyl}benzonitril |
| 165 | 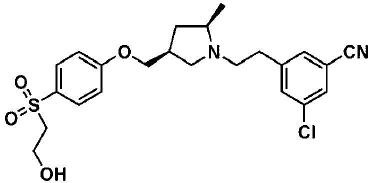 | 3-Chlor-5-{2-[(2R,4S)-4-{[4-(2-hydroxyethansulfonyl)-phenoxy]methyl}-2-methylpyrrolidin-1-yl]ethyl}benzonitril |
| 166 | 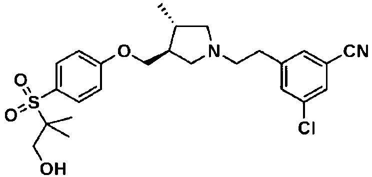 | 3-Chlor-5-{2-[(3S,4S)-3-{[4-(1-hydroxy-2-methylpropan-2-sulfonyl)phenoxy]methyl}-4-methylpyrrolidin-1-yl]ethyl}-benzonitril |
| 167 | 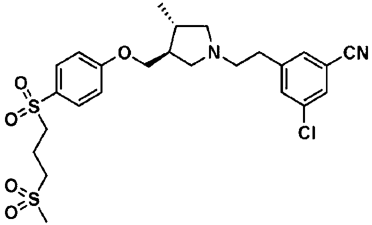 | 3-Chlor-5-{2-[(3S,4S)-3-{[4-(3-methansulfonylpropansulfonyl)-phenoxy]methyl}-4-methylpyrrolidin-1-yl]ethyl}benzonitril |
| 168 | 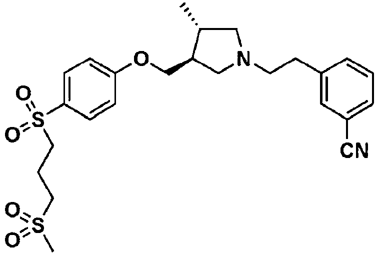 | 3-{2-[(3S,4S)-3-{[4-(3-Methan-sulfonylpropansulfonyl)-phenoxy]methyl}-4-methylpyrrolidin-1-yl]ethyl}benzonitril |
| 169 | 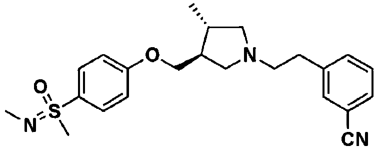 | 3-{2-[(3S,4S)-3-Methyl-4-({4-[methyl(methylimino)oxo-λ⁶-sulfanyl]phenoxy}methyl)-pyrrolidin-1-yl]ethyl}benzonitril |
| 170 | 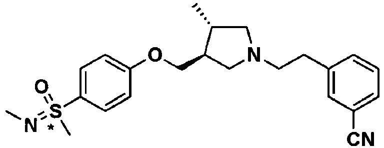 | 3-{2-[(3S,4S)-3-Methyl-4-({4-[(R oder S)-methyl(methyl-imino)oxo-λ⁶-sulfanyl]-phenoxy}methyl)pyrrolidin-1-yl-ethyl}benzonitril |
| 171 | 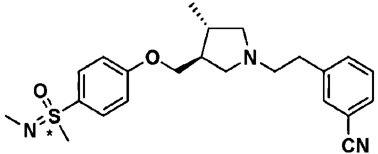 | 3-{2-[(3S,4S)-3-Methyl-4-({4-[(S oder R)-methyl(methylimino)-oxo-λ⁶-sulfanyl]phenoxy}-methyl)pyrrolidin-1-yl]ethyl}-benzonitril |
| 172 | 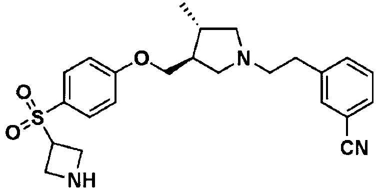 | 3-{2-[(3S,4S)-3-{[4-(Azetidin-3-sulfonyl)phenoxy]methyl}-4-methylpyrrolidin-1-yl]ethyl}-benzonitril |
| 173 | 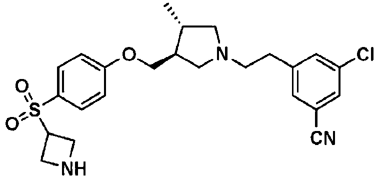 | 3-{2-[(3S,4S)-3-{[4-(Azetidin-3-sulfonyl)phenoxy]methyl}-4-methylpyrrolidin-1-yl]ethyl}-5-chlorbenzonitril |
| 174 | 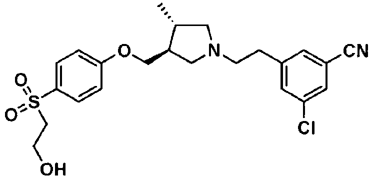 | 3-Chlor-5-{2-[(3S,4S)-3-{[4-(2-hydroxyethansulfonyl)-phenoxy]methyl}-4-methylpyrrolidin-1-yl]ethyl}benzonitril |
| 175 | 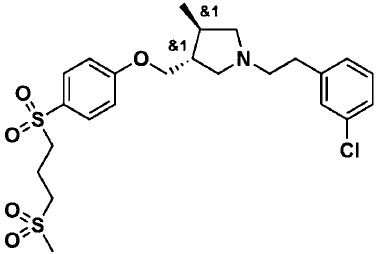 | rac-trans-1-[2-(3-Chlorphenyl)-ethyl]-3-{[4-(3-methansulfonyl-propansulfonyl)phenoxy]-methyl}-4-methylpyrrolidin |
| 176 | 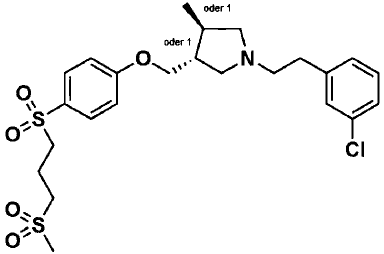 | (3R,4R oder 3S,4S)-1-[2-(3-Chlorphenyl)ethyl]-3-{[4-(3-methansulfonylpropan-sulfonyl)phenoxy]methyl}-4-methylpyrrolidin |
| 177 | 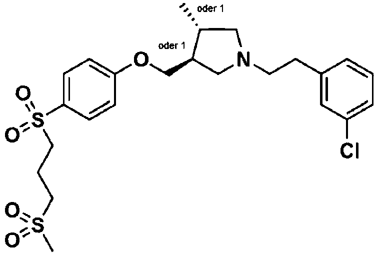 | (3S,4S oder 3R,4R)-1-[2-(3-Chlorphenyl)ethyl]-3-{[4-(3-methansulfonylpropan-sulfonyl)phenoxy]methyl}-4-methylpyrrolidin |
| 181 | 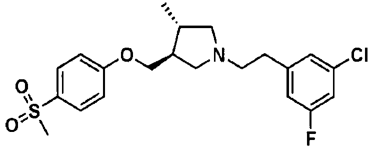 | (3S,4S)-1-[2-(3-Chlor-5-fluor-phenyl)ethyl]-3-[(4-methan-sulfonylphenoxy)methyl]-4-methylpyrrolidin |
| 182 | 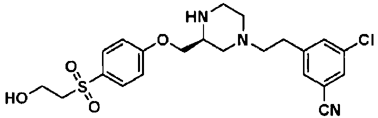 | 3-Chlor-5-{2-[(3S)-3-{[4-(2-hydroxyethansulfonyl)-phenoxy]methyl}piperazin-1-yl]-ethyl}benzonitril |
| 183 | 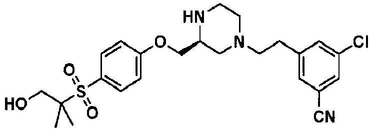 | 3-Chlor-5-{2-[(3S)-3-([4-(1-hydroxy-2-methylpropan-2-sulfonyl)phenoxy]methyl}-piperazin-1-yl]ethyl}benzonitril |
| 184 | 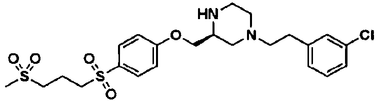 | (3S)-1-[2-(3-Chlorphenyl)ethyl]-3-{[4-(3-methansulfonylpropan-sulfonyl)phenoxy]methyl}-piperazin |
| 185 | 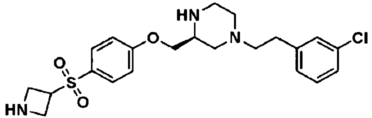 | (3S)-3-{[4-(Azetidin-3-sulfonyl)-phenoxy]methyl}-1-[2-(3-chlorphenyl)ethyl]piperazin |
| 186 | 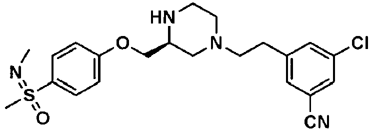 | 3-Chlor-5-{2-[(3S)-3-({4-[methyl(methylimino)oxo-λ⁶-sulfanyl]phenoxy}methyl)-piperazin-1-yl]ethyl}benzonitril |
| 187 | 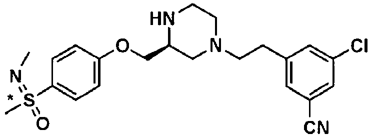 | 3-Chlor-5-{2-[(3S)-3-({4-[(S oder R)-methyl(methylimino)-oxo-λ⁶-sulfanyl]phenoxy}-methyl)piperazin-1-ylethyl}-benzonitril |
| 188 | 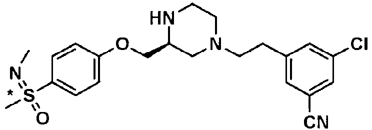 | 3-Chlor-5-{2-[(3S)-3-({4-[(R oder S)-methyl(methylimino)-oxo-λ⁶-sulfanyl]phenoxy}-methyl)piperazin-1-yl]ethyl}-benzonitril |
| 189 | 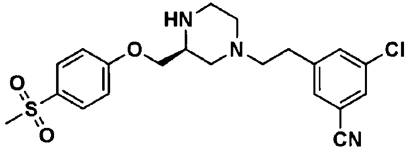 | 3-Chlor-5-{2-[(3S)-3-[(4-methansulfonylphenoxy)-methyl]piperazin-1-yl]ethyl}-benzonitril |
| 190 | 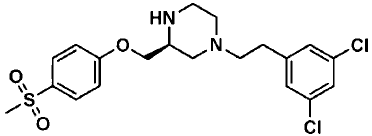 | (3S)-1-[2-(3,5-Dichlorphenyl)-ethyl]-3-[(4-methansulfonyl-phenoxy)methyl]piperazin |
| 191 | 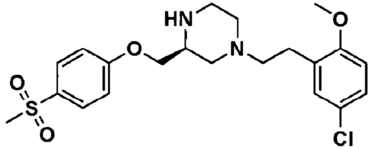 | (3S)-1-[2-(5-Chlor-2-methoxyphenyl)ethyl]-3-[(4-methan-sulfonylphenoxy)methyl]-piperazin |
| 192 | 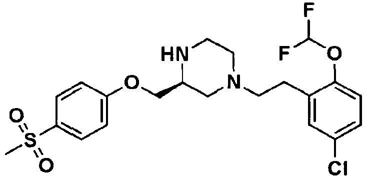 | (3S)-1-{2-[5-Chlor-2-(difluor-methoxy)phenyl]ethyl}-3-[(4-methansulfonylphenoxy)-methyl]piperazin |
| 193 | 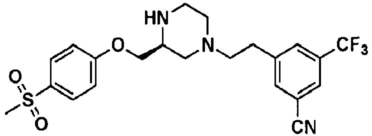 | 3-{2-[(3S)-3-[(4-Methan-sulfonylphenoxy)methyl]-piperazin-1-yl]ethyl}-5-(trifluormethyl)benzonitril |
| 194 | 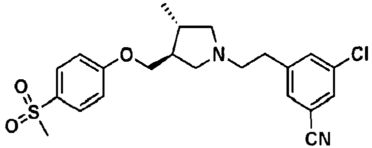 | 3-Chlor-5-{2-[(3S,4S)-3-[(4-methansulfonylphenoxy)-methyl]-4-methylpyrrolidin-1-yl]-ethyl}benzonitril |
| 195 | 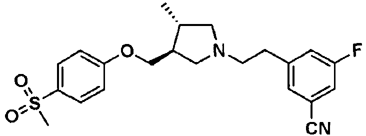 | 3-Fluor-5-{2-[(3S,4S)-3-[(4-methansulfonylphenoxy)-methyl]-4-methylpyrrolidin-1-yl-ethyl}benzonitril |
| 196 | 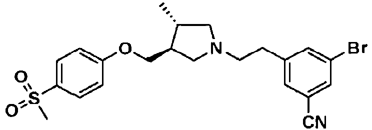 | 3-Brom-5-{2-[(3S,4S)-3-[(4-methansulfonylphenoxy)-methyl]-4-methylpyrrolidin-1-yl]-ethyl}benzonitril |
| 197 | 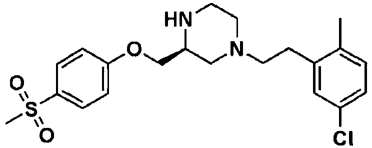 | (3S)-1-[2-(5-Chlor-2-methylphenyl)ethyl]-3-[(4-methan-sulfonylphenoxy)methyl]-piperazin |
| 198 | 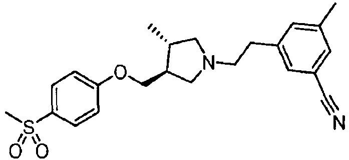 | 3-{2-[(3S,4S)-3-[(4-Methan-sulfonylphenoxy)methyl]-4-methylpyrrolidin-1-yl]ethyl}-5-methylbenzonitril |
| 199 | 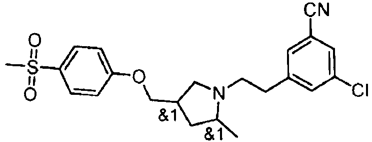 | rac-cis- oder trans-3-Chlor-5-{2-[4-[(4-methansulfonyl-phenoxy)methyl]-2-methylpyrrolidin-1-ylethyl}benzonitril |
| 200 | 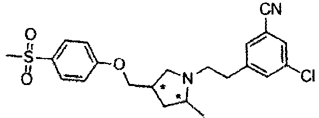 | 3-Chlor-5-{2-[(2R,4S oder 2S,4R oder 2R,4R oder 2S,4S)-4-[(4-Methansulfonyl-phenoxy)methyl]-2-methylpyrrolidin-1-yl]ethyl}benzonitril |
| 201 | 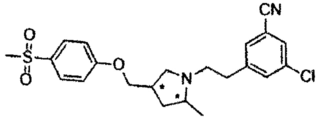 | 3-Chlor-5-{2-[(2S,4R oder 2R,4S oder 2R,4R oder 2S,4S)-4-[(4-Methansulfonyl-phenoxy)methyl]-2-methylpyrrolidin-1-ylethyl}benzonitril |
| 202 | 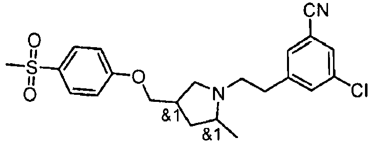 | rac-trans- oder cis-3-Chlor-5-{2-[4-[(4-methansulfonyl-phenoxy)methyl]-2-methylpyrrolidin-1-yl]ethyl}benzonitril |
| 203 | 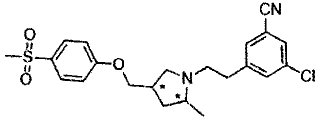 | 3-Chlor-5-(2-((2R,4S oder 2S,4R oder 2R,4R oder 2S,4S)-2-Methyl-4-((4-(methylsulfonyl)phenoxy)methyl)-pyrrolidin-1-yl)ethyl)benzonitril |
| 204 | 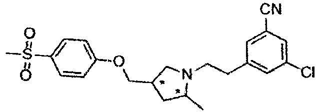 | 3-Chlor-5-(2-((2R,4S oder 2S,4R oder 2S,4S oder 2R,4R)-2-Methyl-4-((4-(methylsulfonyl)phenoxy)methyl)-pyrrolidin-1-yl)ethyl)benzonitril |
| 205 | 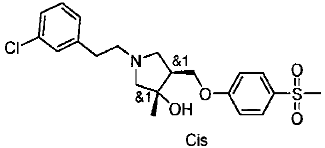 | rac-cis-1-[2-(3-Chlorphenyl)-ethyl]-4-[(4-methansulfonyl-phenoxy)methyl]-3-methylpyrrolidin-3-ol |
| | | |
| 206 | 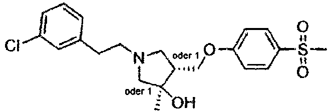 | (3R,4R oder 3S,4S)-1-[2-(3-Chlorphenyl)ethyl]-4-[(4-methansulfonylphenoxy)-methyl]-3-methylpyrrolidin-3-ol |
| 207 | 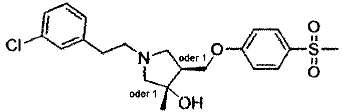 | (3S,4S oder 3R,4R)-1-(3-Chlor-phenethyl)-3-methyl-4-((4-(methylsulfonyl)phenoxy)-methyl)pyrrolidin-3-ol |
| 208 | 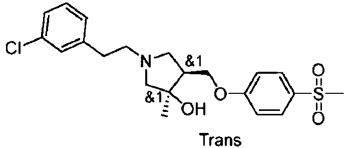 | rac-trans-1-[2-(3-Chlorphenyl)ethyl]-4-[(4-methan-sulfonylphenoxy)methyl]-3-methylpyrrolidin-3-ol |
| 209 | 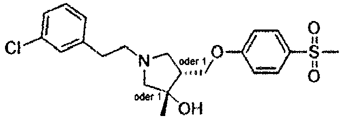 | (3R,4S oder 3S,4R)-1-[2-(3-Chlorphenyl)ethyl]-4-[(4-methansulfonylphenoxy)-methyl]-3-methylpyrrolidin-3-ol |
| 210 | 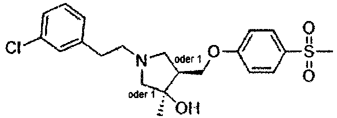 | (3S,4R oder 3R,4S)-1-[2-(3-Chlorphenyl)ethyl]-4-[(4-methansulfonylphenoxy)-methyl]-3-methylpyrrolidin-3-ol |
| 211 | 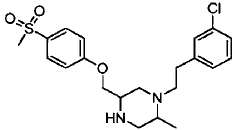 | 1-[2-(3-Chlorphenyl)ethyl]-5-[(4-methansulfonylphenoxy)-methyl]-2-methylpiperazin |
| 212 | 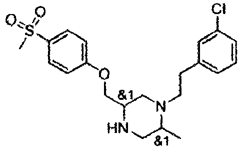 | rac-cis- oder trans-1-[2-(3-Chlorphenyl)ethyl]-5-[(4-methansulfonylphenoxy)-methyl]-2-methylpiperazin |
| 213 | 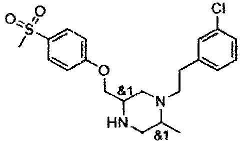 | rac-trans- oder cis-1-[2-(3-Chlorphenyl)ethyl]-5-[(4-methansulfonylphenoxy)-methyl]-2-methylpiperazin |
| 214 | 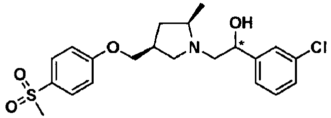 | (1R)- oder (1S)-1-(3-Chlorphenyl)-2-[(2R,4S)-4-[(4-methansulfonylphenoxy)-methyl]-2-methylpyrrolidin-1-yl]-ethan-1-ol |
| 215 | 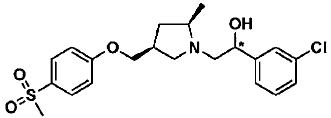 | (1S)- oder (1R)-1-(3-Chlorphenyl)-2-[(2R,4S)-4-[(4-methansulfonylphenoxy)-methyl]-2-methylpyrrolidin-1-yl]ethan-1-ol |
oder ein Stereoisomer oder ein Tautomer davon oder ein pharmazeutisch annehmbares Salz eines beliebigen der Vorgenannten.

13. Pharmazeutische Zusammensetzung, die (i) eine Verbindung nach einem der Ansprüche 1 bis 12 oder ein Stereoisomer oder ein Tautomer davon oder ein pharmazeutisch annehmbares eines beliebigen der Vorgenannten und (ii) einen oder mehrere pharmazeutisch annehmbare Hilfsstoffe umfasst.

14. Verbindung nach einem der Ansprüche 1 bis 12 oder ein Stereoisomer oder ein Tautomer davon oder ein pharmazeutisch annehmbares Salz eines beliebigen der Vorgenannten oder pharmazeutische Zusammensetzung nach Anspruch 13 zur Verwendung bei der Behandlung oder Verzögerung der Entwicklung einer Erkrankung, einer Störung oder eines Leidens, die/das aus den folgenden ausgewählt ist:
einer Nierenerkrankung;
einer Erkrankung, einer Störung oder eines Leidens, die/das aus der aus chronischer Nierenerkrankung, fokaler segmentaler Glumerulosklerose (FSGS), durch Hypertonie bedingter Nierenerkrankung, mit dem humanen Immundefizienzvirus assoziierter Nephropathie (HIVAN), Sichelzellen-Nephropathie, Lupus-Nephritis, diabetischer Nierenerkrankung, APOL1-assoziierter Nephropathie, viraler Nephropathie, COVID-19-assoziierter Nephropathie, Präeklampsie und Sepsis bestehenden Gruppe ausgewählt ist.

15. Set, das (i) eine Verbindung nach einem der Ansprüche 1 bis 12 oder ein Stereoisomer oder ein Tautomer davon oder ein pharmazeutisch annehmbares Salz eines beliebigen der Vorgenannten oder eine pharmazeutische Zusammensetzung nach Anspruch 13 und (ii) Anweisungen zur Verwendung bei der Behandlung einer/s durch APOL1 vermittelten Erkrankung, Störung oder Leidens bei einem bedürftigen Individuum umfasst;
wobei die Erkrankung, die Störung oder das Leiden gegebenenfalls Folgendes ist:
eine Nierenerkrankung;
eine Erkrankung, eine Störung oder ein Leidens, die/das aus der aus chronischer Nierenerkrankung, fokaler segmentaler Glumerulosklerose (FSGS), durch Hypertonie bedingter Nierenerkrankung, mit dem humanen Immundefizienzvirus assoziierter Nephropathie (HIVAN), Sichelzellen-Nephropathie, Lupus-Nephritis, diabetischer Nierenerkrankung, APOL1-assoziierter Nephropathie, viraler Nephropathie, COVID-19-assoziierter Nephropathie, Präeklampsie und Sepsis bestehenden Gruppe ausgewählt ist;
wobei das Individuum gegebenenfalls eine APOL1-Mutation, wie z. B. eine Funktionsgewinn-Mutation aufweist.

## Revendications

1. Composé de formule (A') : 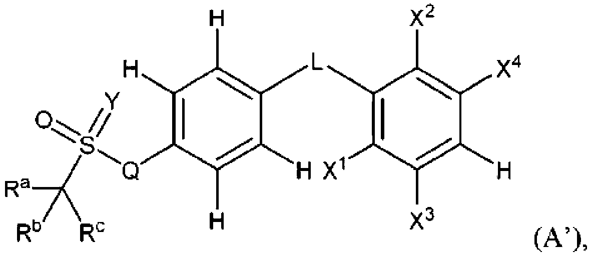 ou un stéréoisomère ou un tautomère de celui-ci, ou un sel pharmaceutiquement acceptable de l'un quelconque des précédents, dans lequel :
Q est absent ou est -N- (alkyle en C₁₋₆) ;
Y est O ou -N- (alkyle en C₁₋₆) ;
à condition que lorsque Q est -N-(alkyle en C₁₋₆), alors Y est O ;
R^{a}, R^{b} et R^{c} sont chacun indépendamment H ou un groupe alkyle en C₁₋₆, le groupe alkyle en C₁₋₆ de R^{a}, R^{b} ou R^{c} étant indépendamment facultativement substitué par un ou plusieurs groupes -OH, alcoxy en C₁₋₆ ou -S(O)₂-alkyle en C₁₋₆, ou deux quelconques parmi R^{a}, R^{b} et R^{c} son pris, conjointement avec les atomes auxquels ils sont liés, pour former un groupe cycloalkyle en C₃₋₆ ou un groupe hétérocyclyle à 3 à 6 chaînons et l'autre parmi R^{a}, R^{b} et R^{c} est H ou un groupe alkyle en C₁₋₆, le groupe alkyle en C₁₋₆ de R^{a}, R^{b} ou R^{c} étant indépendamment facultativement substitué par un ou plusieurs groupes -OH, alcoxy en C₁₋₆ ou -S(O)₂-alkyle en C₁₋₆ ;
L est choisi de le groupe constitué par :
(i) 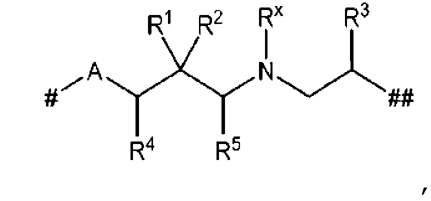 dans lequel
A est O, NH, N(alkyle en C₁₋₆), CH₂ ou CH(alkyle en C₁₋6) ;
R^{x} est H,
ou R^{x} est pris conjointement avec l'un parmi R¹ et R², et les atomes auxquels ils sont liés, pour former un groupe hétérocyclyle à 3 à 8 chaînons, le groupe hétérocyclyle à 3 à 8 chaînons étant substitué par n substituants R^{g} choisis indépendamment, n étant un entier de 0 à 6, et R^{g} étant -OH, un halogène, un groupe alkyle en C₁₋₆ ou un groupe alcoxy en C₁₋₆ ;
R¹ et R² sont indépendamment H, un halogène ou -OH,
ou l'un parmi R¹ et R² est pris conjointement avec R^{x}, et les atomes auxquels ils sont liés, pour former un groupe hétérocyclyle à 3 à 8 chaînons. le groupe hétérocyclyle à 3 à 8 chaînons étant substitué par n substituants R^{g} choisis indépendamment, n étant un entier de 0 à 6, et R^{g} étant -OH, un halogène, un groupe alkyle en C₁₋₆ ou un groupe alcoxy en C₁₋₆, l'autre parmi R¹ et R² étant H, un halogène ou -OH ;
R³ est H, -OH, un halogène ou un groupe alcoxy en C₁₋₆ ; et
R⁴ et R⁵ sont indépendamment H,
ou R⁴ et R⁵ sont pris, conjointement avec les atomes auxquels ils sont liés, pour former un groupe cycloalkyle en C₃₋₈,
à condition que :
(1) R^{x} soit pris conjointement avec l'un parmi R¹ et R², et les atomes auxquels ils sont liés, pour former un groupe hétérocyclyle à 3 à 8 chaînons le groupe hétérocyclyle à 3 à 8 chaînons étant substitué par n substituants R^{g} choisis indépendamment, n étant un entier de 0 à 6, et R^{g} étant -OH, un halogène, un groupe alkyle en C₁₋₆ ou un groupe alcoxy en C₁₋₆ ; ou
(2) R⁴ et R⁵ soient pris conjointement, avec les atomes auxquels ils sont liés, pour former un cycloalkyle en C₃₋₈,
(ii) 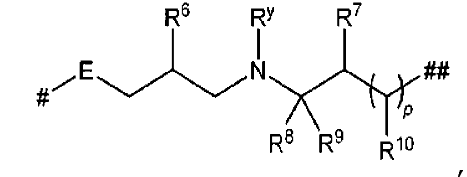 dans lequel
E est O, NH, N(alkyle en C₁₋₆), CH₂ ou CH(alkyle en C₁₋6) ;
p vaut 0 ou 1,
à condition que, lorsque p vaut 1, E est O ;
R⁶ est H ou -OH ;
R^{y} est H,
ou R^{y} est pris conjointement avec R⁷, et les atomes auxquels ils sont liés, pour former un groupe hétérocyclyle à 3 à 8 chaînons,
ou R^{y} est pris conjointement avec l'un parmi R⁸ et R⁹, et les atomes auxquels ils sont liés, pour former un groupe hétérocyclyle à 3 à 8 chaînons ;
R⁷ est H,
ou R⁷ est pris conjointement avec R^{y}, et les atomes auxquels ils sont liés, pour former un groupe hétérocyclyle à 3 à 8 chaînons ;
R⁸ et R⁹ sont indépendamment H ou un groupe alkyle C₁₋₆,
ou l'un parmi R⁸ et R⁹ est pris conjointement avec R^{y}, et les atomes auxquels ils sont liés, pour former un hétérocyclyle à 3 à 8 chaînons, et l'autre parmi R⁸ et R⁹ est H ou un groupe alkyle C₁₋₆,
ou l'un parmi R⁸ et R⁹ est pris conjointement avec R¹⁰, et les atomes auxquels ils sont liés, pour former un groupe cycloalkyle en C₃₋₈, et l' autre parmi R⁸ et R⁹ est H ou un groupe alkyle C₁₋₆ ; et
R¹⁰ est H,
ou R¹⁰ est pris conjointement avec l'un parmi R⁸ et R⁹, et les atomes auxquels ils sont liés, pour former un groupe cycloalkyle en C₃₋₈,
à condition que :
(1) R^{y} soit pris conjointement avec R⁷, et les atomes auxquels ils sont liés, pour former un groupe hétérocyclyle à 3 à 8 chaînons, ou
(2) R^{y} soit pris conjointement avec l'un parmi R⁸ et R⁹, et les atomes auxquels ils sont liés, pour former un groupe hétérocyclyle à 3 à 8 chaînons, ou
(3) l'un parmi R⁸ et R⁹ soit pris conjointement avec R¹⁰ et les atomes auxquels ils sont liés, pour former un groupe cycloalkyle en C₃₋₈, et
(iii) 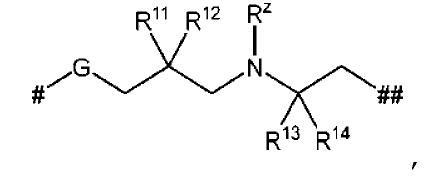 dans lequel
G est O, NH, N(alkyle en C₁₋₆), CH₂ ou CH(alkyle en C₁₋6) ;
R⁷ est H ou un groupe alkyle en C₁₋₆, le groupe alkyle en C₁₋₆ étant éventuellement substitué par un ou plusieurs groupes cycloalkyle en C₃₋₈ ;
R¹¹ et R¹² sont indépendamment H, -OH, un halogène ou un groupe alkyle en C₁₋₆ ; et
R¹³ et R¹⁴ sont indépendamment H, un groupe alkyle en C₁₋₆ ou un groupe cycloalkyle en C₃₋₈,
ou R¹³ et R¹⁴ sont pris, conjointement avec les atomes auxquels ils sont liés, pour former un groupe hétérocyclyle à 3 à 8 chaînons,
dans lequel pour chacun de (i) à (iii), # désigne le point de liaison au fragment Q portant le cycle phényle, et ## désigne le point de liaison aux fragments X¹ à X⁴ portant le cycle phényle ; et
X¹, X²*,* X³ et X⁴ sont, indépendamment les uns des autres, H, un halogène, -CN, un groupe alkyle en C₁₋₆, un groupe alcoxy en C₁₋₆ ou SF₅, le groupe alkyle en C₁₋₆ ou alcoxy en C₁₋₆ étant éventuellement substitué par un ou plusieurs halogènes,
à condition qu'au moins un parmi X¹, X², X³ et X⁴ soit un halogène, -CN, un groupe alkyle en C₁₋₆, un groupe alcoxy en C₁₋₆ ou SF₅, le groupe alkyle en C₁₋₆ ou alcoxy en C₁₋₆ étant éventuellement substitué par un ou plusieurs halogènes.

2. Composé selon la revendication 1, dans lequel le composé est un composé de formule (A) : 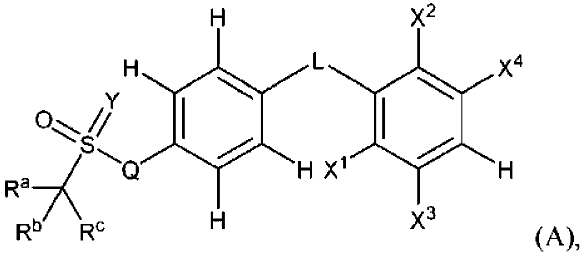 ou un stéréoisomère ou un tautomère de celui-ci, ou un sel pharmaceutiquement acceptable de l'un quelconque des précédents, dans lequel :
X¹, X², X³ et X⁴ sont, indépendamment les uns des autres, H, un halogène, -CN, un groupe alkyle en C₁₋₆ ou un groupe alcoxy en C₁₋₆, le groupe alkyle en C₁₋₆ ou alcoxy en C₁₋₆ étant éventuellement substitué par un ou plusieurs halogènes,
à condition qu'au moins un parmi X¹, X², X³ et X⁴ soit un halogène, -CN, un groupe alkyle en C₁₋₆ ou un groupe alcoxy en C₁₋₆, le groupe alkyle en C₁₋₆ ou alcoxy en C₁₋₆ étant éventuellement substitué par un ou plusieurs halogènes.

3. Composé selon la revendication 1 ou 2, ou un stéréoisomère ou un tautomère de celui-ci, ou un sel pharmaceutiquement acceptable de l'un quelconque des précédents, dans lequel L est 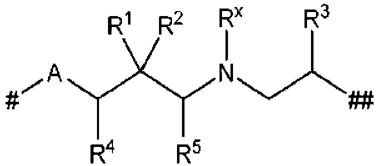 de telle sorte que le composé est de formule (I) : 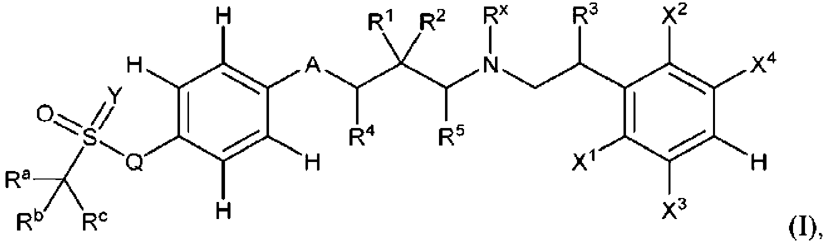
ou un stéréoisomère ou un tautomère de celui-ci, ou un sel pharmaceutiquement acceptable de l'un quelconque des précédents ;
éventuellement dans lequel un de (a) ou (b) s'applique :
(a) A est O, de telle sorte que le composé est de formule (I-A) : 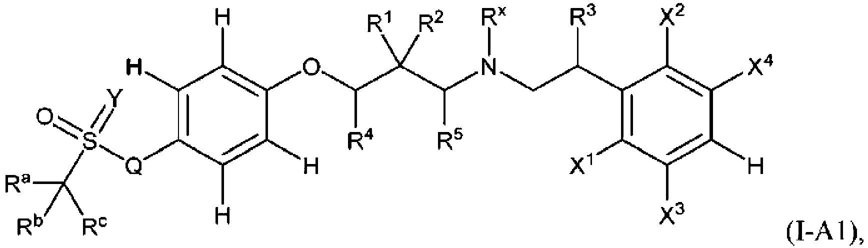 ou un stéréoisomère ou un tautomère de celui-ci, ou un sel pharmaceutiquement acceptable de l'un quelconque des précédents ;
(b) A est CH₂, de telle sorte que le composé est de formule (I-A3) : 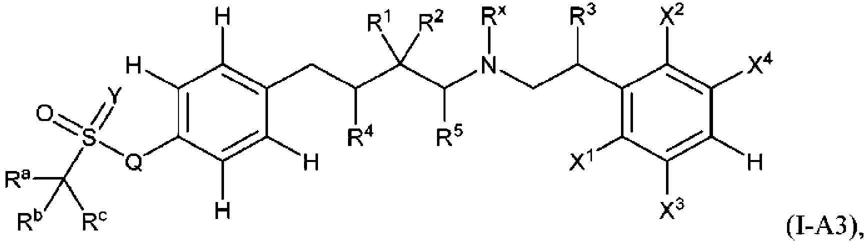
ou un stéréoisomère ou un tautomère de celui-ci, ou un sel pharmaceutiquement acceptable de l'un quelconque des précédents.

4. Composé selon la revendication 3, ou un stéréoisomère ou un tautomère de celui-ci, ou un sel pharmaceutiquement acceptable de l'un quelconque des précédents, dans lequel le composé est de formule (I-A1) et soit :
R^{x} est pris conjointement avec l'un parmi R¹ et R², et les atomes auxquels ils sont liés, pour former un groupe hétérocyclyle à 3 à 8 chaînons, le groupe hétérocyclyle à 3 à 8 chaînons étant substitué par *n* substituants R^{g} choisis indépendamment, *n* étant un entier de 0 à 6, et R^{g} étant -OH, un halogène, un groupe alkyle en C₁₋₆ ou un groupe alcoxy en C₁₋₆ ; soit
R^{x} est pris conjointement avec l'un parmi R¹ et R², et les atomes auxquels ils sont liés, pour former un groupe hétérocyclyle à 5 à 6 chaînons, le groupe hétérocycle à 5 à 6 chaînons étant substitué par *n* substituants R^{g} choisis indépendamment, n étant un entier de 0 à 6, et R^{g} étant -OH, un halogène, un groupe alkyle en C₁₋₆ ou un groupe alcoxy en C₁₋₆.

5. Composé selon la revendication 4, ou un stéréoisomère ou un tautomère de celui-ci, ou un sel pharmaceutiquement acceptable de l'un quelconque des précédents, dans lequel le composé est de formule (I-B1), (I-C1), (I-D1), (I-E1), (I-F1) ou (I-G1) : 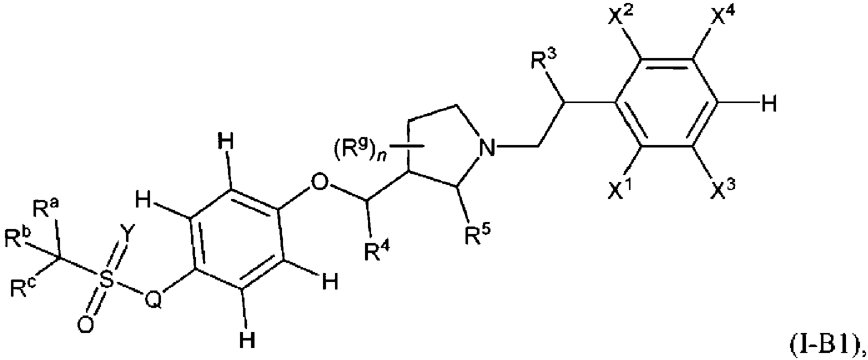
dans lequel dans la formule (I-B1), *n* est éventuellement 0, ou *n* est éventuellement 1 ou 2 ; 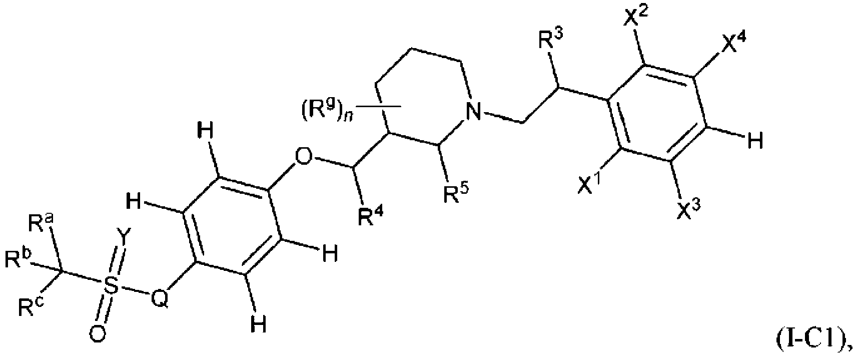
dans lequel dans la formule (I-C1), *n* est éventuellement 0, ou *n* est éventuellement 1 ou 2 ; 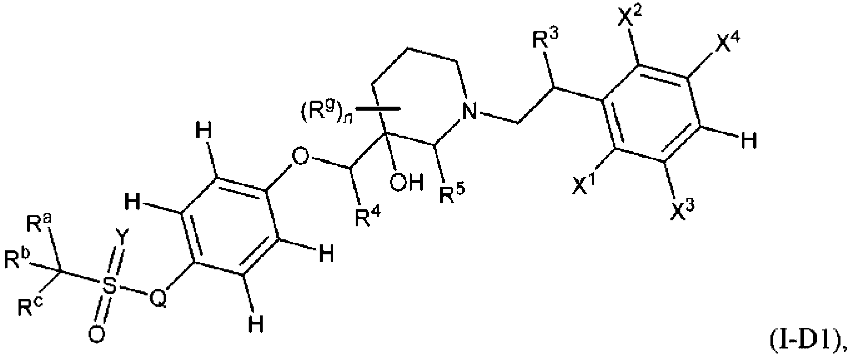
dans lequel dans la formule (I-D1), *n* est éventuellement 0, ou *n* est éventuellement 1 ou 2 ; 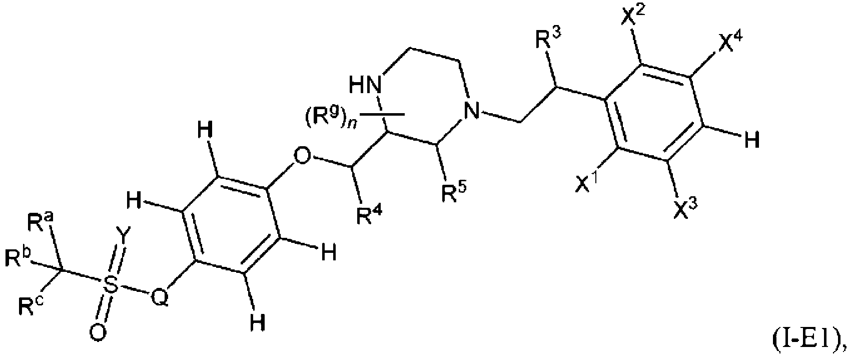
dans lequel dans la formule (I-E1), *n* est éventuellement 0 ; 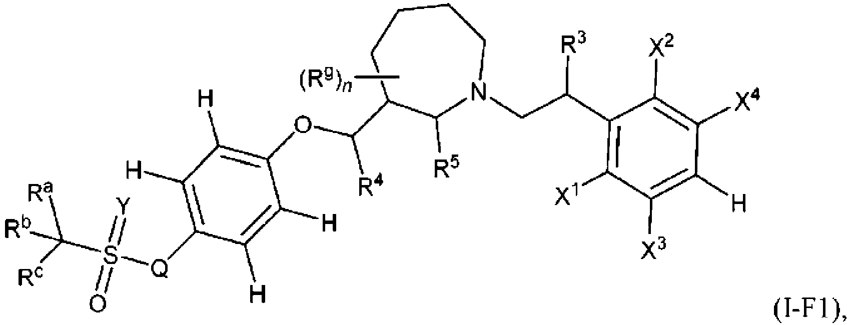
dans lequel dans la formule (I-F1), *n* est éventuellement 0 ; 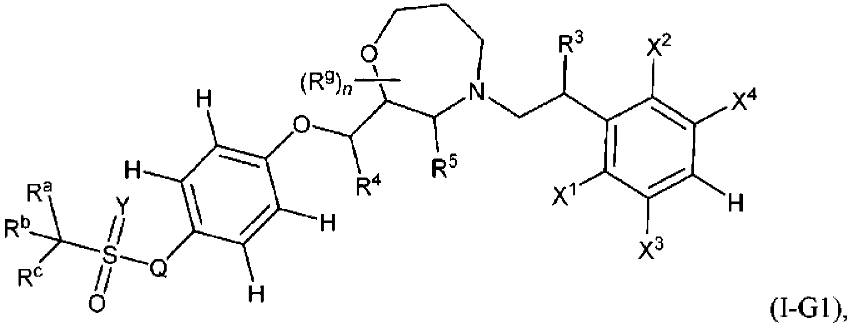
dans lequel dans la formule (I-G1), *n* est éventuellement 0 ;
ou un stéréoisomère ou un tautomère de celui-ci, ou un sel pharmaceutiquement acceptable de l'un quelconque des précédents.

6. Composé selon la revendication 3, ou un stéréoisomère ou un tautomère de celui-ci, ou un sel pharmaceutiquement acceptable de l'un quelconque des précédents, le composé est de formule (I-A) et dans lequel R⁴ et R⁵ sont pris, conjointement avec les atomes auxquels ils sont liés, pour former un groupe cycloalkyle en C₃₋₈ ; éventuellement dans lequel le composé est de formule (I-H1) : 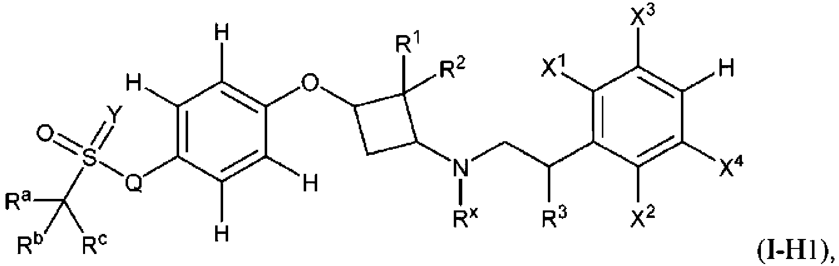
ou un stéréoisomère ou un tautomère de celui-ci, ou un sel pharmaceutiquement acceptable de l'un quelconque des précédents ;
dans lequel R¹, R² et R³ sont éventuellement chacun H.

7. Composé selon la revendication 3, ou un stéréoisomère ou un tautomère de celui-ci, ou un sel pharmaceutiquement acceptable de l'un quelconque des précédents, dans lequel le composé est de formule (I-A1) et soit :
(a) R^{x} est pris conjointement avec l'un parmi R¹ et R², et les atomes auxquels ils sont liés, pour former un groupe hétérocyclyle à 3 à 8 chaînons, le groupe hétérocyclyle à 3 à 8 chaînons étant substitué par *n* substituants R^{g} choisis indépendamment, *n* étant un entier de 0 à 6, et R^{g} étant -OH, un halogène, un groupe alkyle en C₁₋₆ ou un groupe alcoxy en C₁₋₆, et l'autre parmi R¹ et R² étant H, un halogène, ou - OH ; soit
(b) R^{x} est pris conjointement avec l'un parmi R¹ et R², et les atomes auxquels ils sont liés, pour former un groupe hétérocyclyle à 5 à 6 chaînons, le groupe hétérocycle à 5 à 6 chaînons étant substitué par *n* substituants R^{g} choisis indépendamment, *n* étant un entier de 0 à 6, et R^{g} étant -OH, un halogène, un groupe alkyle en C₁₋₆ ou un groupe alcoxy en C₁₋₆, et l'autre parmi R¹ et R² étant H, un halogène, ou - OH ;
éventuellement dans lequel le composé est de formule (I-C3): 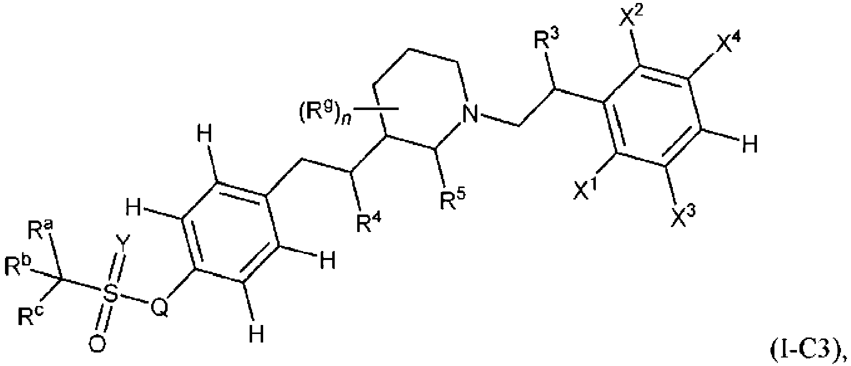
ou un stéréoisomère ou un tautomère de celui-ci, ou un sel pharmaceutiquement acceptable de l'un quelconque des précédents.

8. Composé selon la revendication 1 ou la revendication 2, ou un stéréoisomère ou un tautomère de celui-ci, ou un sel pharmaceutiquement acceptable de l'un quelconque des précédents, dans lequel L est 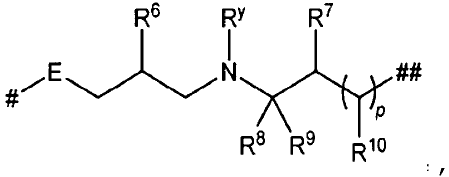 de telle sorte que le composé est de formule (II) : 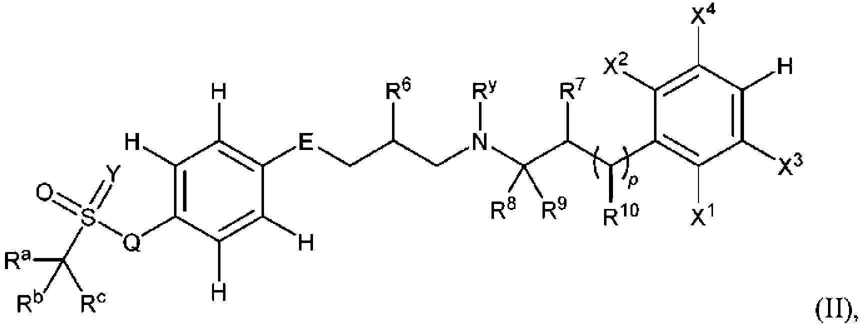
ou un stéréoisomère ou un tautomère de celui-ci, ou un sel pharmaceutiquement acceptable de l'un quelconque des précédents ;
éventuellement dans lequel E est O, de telle sorte que le composé est de formule (II-A1) : 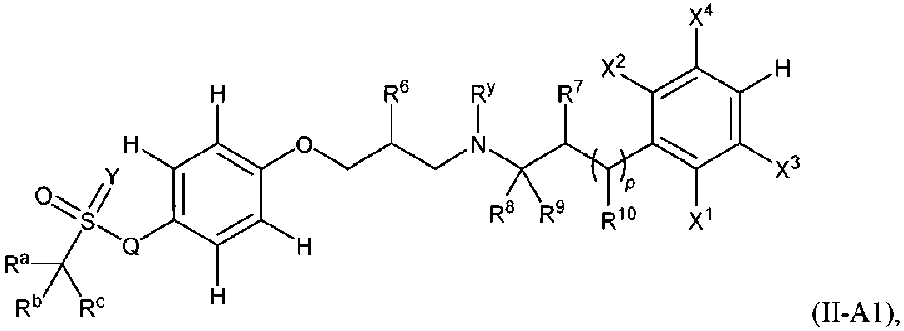
ou un stéréoisomère ou un tautomère de celui-ci, ou un sel pharmaceutiquement acceptable de l'un quelconque des précédents ;
éventuellement dans lequel :
(a) R^{y} dans la formule (II-A1) est pris conjointement avec R⁷, et les atomes auxquels ils sont liés, pour former un groupe hétérocyclyle à 3 à 8 chaînons ; ou
(b) R^{y} est pris conjointement avec R⁷, et les atomes auxquels ils sont liés, pour former un groupe hétérocyclyle à 5 à 6 chaînons ;
éventuellement en outre dans lequel l'un parmi R⁸ et R⁹ est pris conjointement avec R¹⁰, et les atomes auxquels ils sont liés, pour former un groupe cycloalkyle en C₃₋₈.

9. Composé selon la revendication 8, ou un stéréoisomère ou un tautomère de celui-ci, ou un sel pharmaceutiquement acceptable de l'un quelconque des précédents, dans lequel le composé est de formule (II-B1), (II-C1), (II-D1) ou (II-E1): 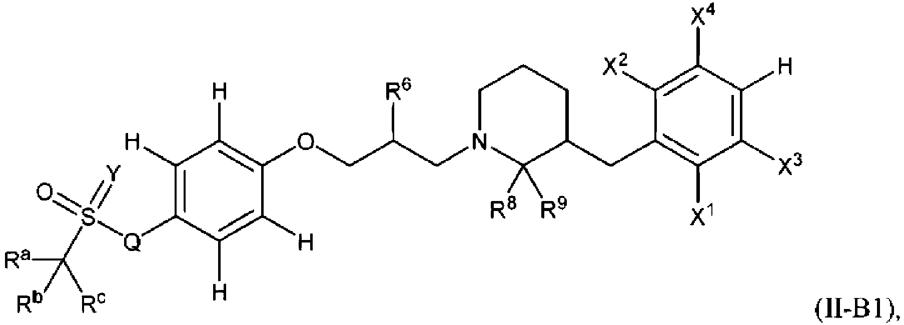 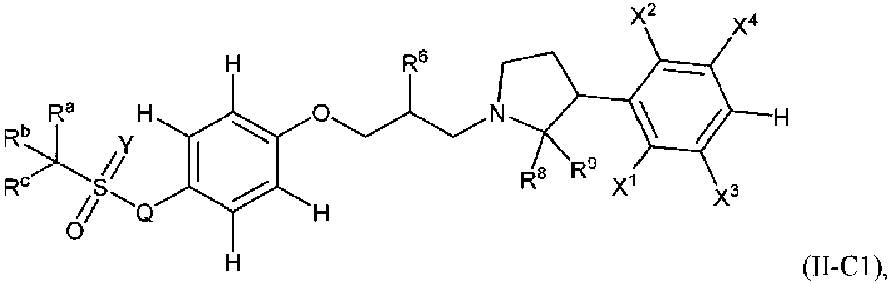
dans lequel dans le formule (II-C1), éventuellement R⁶ est -OH, R⁸ est un groupe alkyle en C₁₋₆ et R⁹ est un groupe alkyle en C₁₋₆ ; 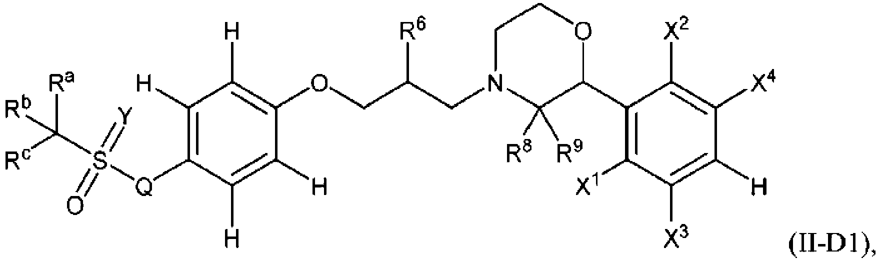
dans lequel dans le formule (II-D1), éventuellement R⁶ est -OH, R⁸ est H et R⁹ est H ; 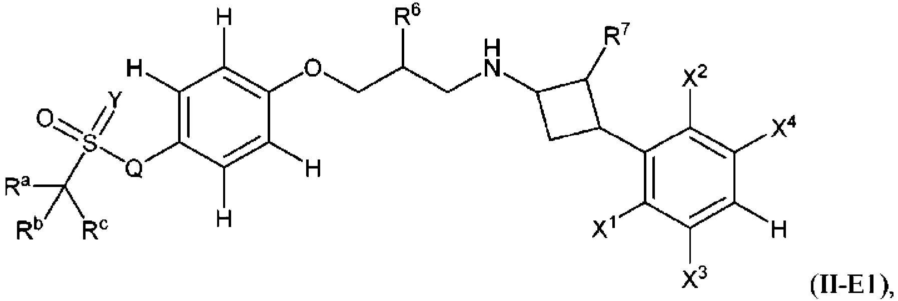
dans lequel dans le formule (II-E1), éventuellement R⁶ est -OH et R⁷ est H ;
ou un stéréoisomère ou un tautomère de celui-ci, ou un sel pharmaceutiquement acceptable de l'un quelconque des précédents.

10. Composé selon la revendication 1 ou la revendication 2, ou un stéréoisomère ou un tautomère de celui-ci, ou un sel pharmaceutiquement acceptable de l'un quelconque des précédents, dans lequel L est 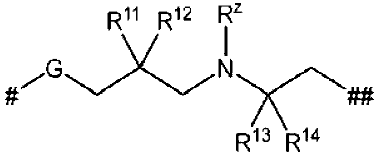 de telle sorte que le composé est de formule (III) : 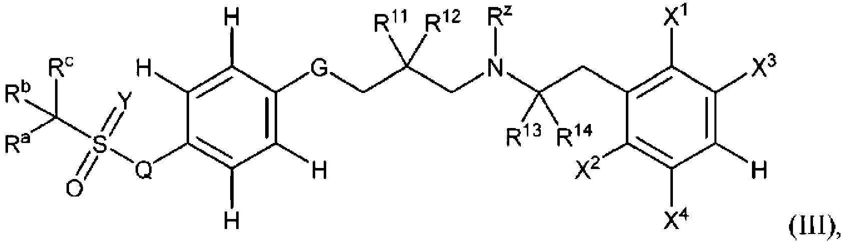 ou un stéréoisomère ou un tautomère de celui-ci, ou un sel pharmaceutiquement acceptable de l'un quelconque des précédents.

11. Composé selon la revendication 1 ou la revendication 2, ou un stéréoisomère ou un tautomère de celui-ci, ou un sel pharmaceutiquement acceptable de l'un quelconque des précédents, dans lequel soit :
(a) Q est absent, Y est O, et R^{a}, R^{b} et R^{c} sont chacun indépendamment H, de telle sorte que le composé est de formule (B-2) : 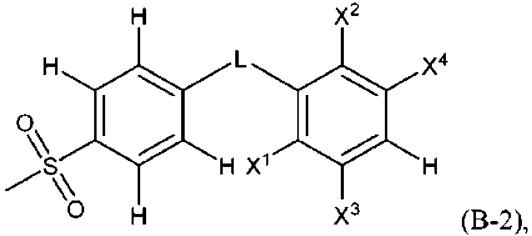 ou un stéréoisomère ou un tautomère de celui-ci, ou un sel pharmaceutiquement acceptable de l'un quelconque des précédents ; soit
(b) Q est absent, Y est -N(CH₃), et R^{a}, R^{b} et R^{c} sont chacun indépendamment H, de telle sorte que le composé est de formule (B-5) : 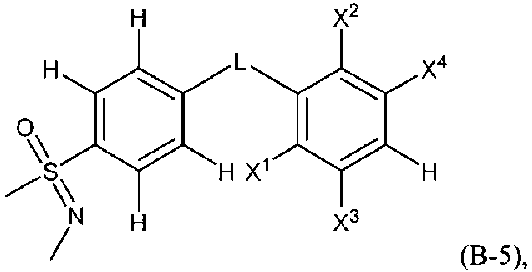 ou un stéréoisomère ou un tautomère de celui-ci, ou un sel pharmaceutiquement acceptable de l'un quelconque des précédents ; soit
(c) Q est -N(CH₃), Y est O, et R^{a}, R^{b} et R^{c} sont chacun indépendamment H, de telle sorte que le composé est de formule (C-1) : 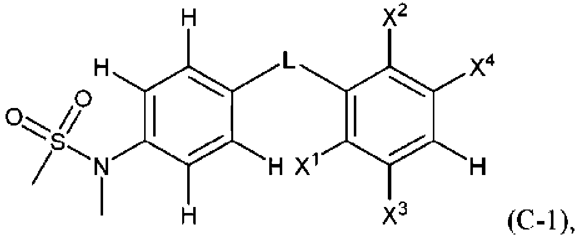 ou un stéréoisomère ou un tautomère de celui-ci, ou un sel pharmaceutiquement acceptable de l'un quelconque des précédents.

12. Composé selon la revendication 1 ou la revendication 2, ou un stéréoisomère ou un tautomère de celui-ci, ou un sel pharmaceutiquement acceptable de l'un quelconque des précédents, dans lequel le composé est choisi parmi les suivants :
| Composé n° | Structure | Nom chimique |
|---|---|---|
| 1 | 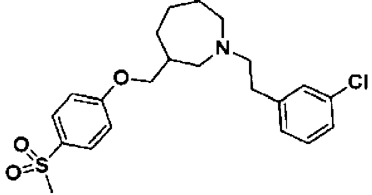 | 1-(3-chlorophénéthyl)-3-((4-(méthylsulfonyl)phénoxy) méthyl)azépane |
| 2 | 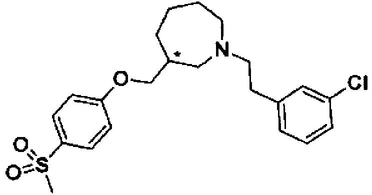 | (*R*) ou (*S*)-1-(3-chlorophénéthyl)-3-((4-(méthylsulfonyl)phénoxy) méthyl)azépane |
| 3 | 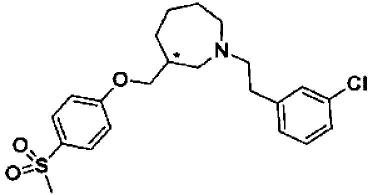 | (*S*) ou (*R*)-1-(3-chlorophénéthyl)-3-((4-(méthylsulfonyl)phénoxy) méthyl)azépane |
| 4 | 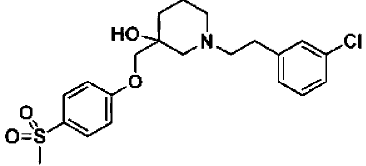 | 1-(3-chlorophénéthyl)-3-((4-(méthylsulfonyl)phénoxy) méthyl)pipéridin-3-ol |
| 5 | 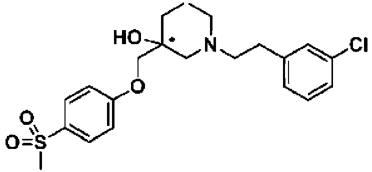 | (*R*) ou (*S*)-1-(3-chlorophénéthyl)-3-((4-(méthylsulfonyl)phénoxy) méthyl)pipéridin-3-ol |
| 6 | 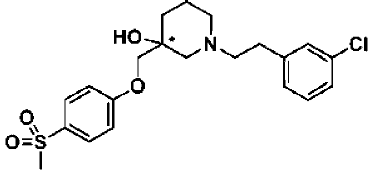 | (*S*) ou (*R*)*-*1*-*(3-chlorophénéthyl)-3-((4-(méthylsulfonyl)phénoxy) méthyl)pipéridin-3-ol |
| 7 | 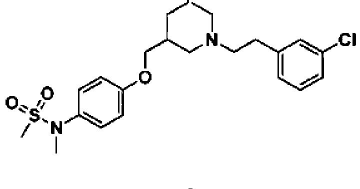 | *N*-(4-((1-(3-chlorophénéthyl)pipéridin -3-yl)méthoxy)phényl)-*N-*méthylméthanesulfonamide |
| 8 | 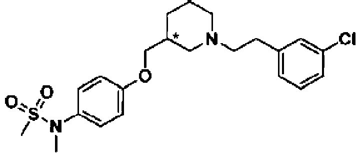 | (*S*) ou (*R*)*-N*-(4-((1-(3-chlorophénéthyl)pipéridin -3-yl)méthoxy)phényl)-*N-*méthylméthanesulfonamide |
| 9 | 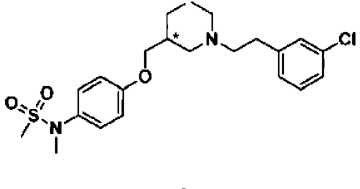 | (*R*) ou (*S*)-*N*-(4-((1-(3-chlorophénéthyl)pipéridin -3-yl)méthoxy)phényl)-*N-*méthylméthanesulfonamide |
| 10 | 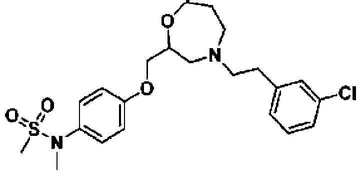 | *N*-(4-((4-(3-chlorophénéthyl)-1,4-oxazépan-2-yl)méthoxy)phényl)-N-méthylméthanesulfonamide |
| 11 | 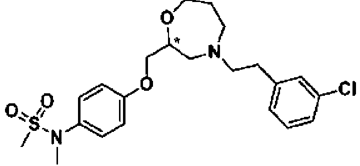 | (*R*) ou (*S*)-*N*-(4-((4-(3-chlorophénéthyl)-1,4-oxazépan-2-yl)méthoxy)phényl)-*N-*méthylméthanesulfonamide |
| 12 | 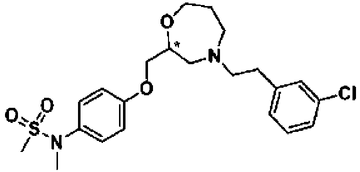 | (*S*) ou (*R*)-*N*-(4-((4-(3-chlorophénéthyl)-1,4-oxazépan-2-yl)méthoxy)phényl)-*N-*méthylméthanesulfonamide |
| 13 | 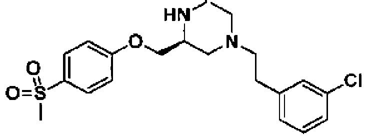 | (*S*)-1-(3-chlorophénéthyl)-3-((4-(méthylsulfonyl)phénoxy) méthyl)pipérazine |
| 14 | 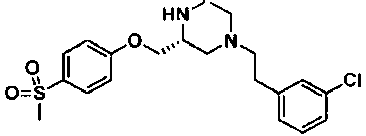 | (*R*)-1-(3-chlorophénéthyl)-3-((4-(méthylsulfonyl)phénoxy) méthyl)pipérazine |
| 15 | 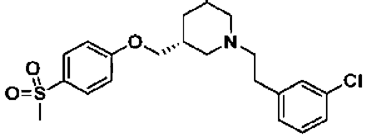 | (*R*)-1-(3-chlorophénéthyl)-3-((4-(méthylsulfonyl)phénoxy) méthyl)pipéridine |
| 16 | 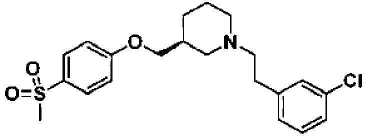 | (*S*)-1-(3-chlorophénéthyl)-3-((4-(méthylsulfonyl)phénoxy) méthyl)pipéridine |
| 17 | 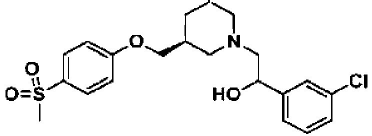 | 1-(3-chlorophényl)-2-((*S*)-3-((4-(méthylsulfonyl)phénoxy) méthyl)pipéridin-1-yl)ethan-1-ol |
| 18 | 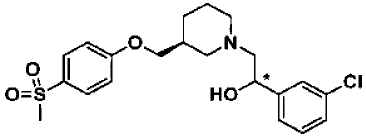 | (*R*) ou (*S*)-1-(3-chlorophényl)-2-((*S*)-3-((4-(méthylsulfonyl)phénoxy) méthyl)pipéridin-1-yl)éthan-1-ol |
| 19 | 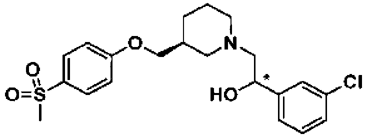 | (*S*) ou (*R*)*-*1-(3-chlorophényl)-2-((*S*)-3-((4-(méthylsulfonyl)phénoxy) méthyl)pipéridin-1-yl)éthan-1-ol |
| 20 | 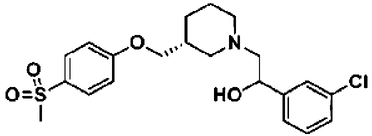 | 1-(3-chlorophényl)-2-((*R*)-3-((4-(méthylsulfonyl)phénoxy) méthyl)pipéridin-1-yl)éthanol |
| 21 | 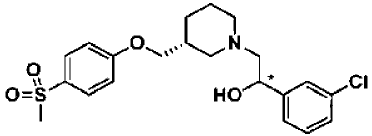 | (*R*) ou (*S*)-1-(3-chlorophényl)-2-((*R*)-3-((4-(méthylsulfonyl)phénoxy) méthyl)pipéridin-1-yl)éthanol |
| 22 | 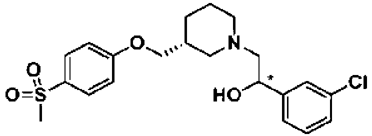 | (S) ou (*R*)*-*1*-*(3-chlorophényl)-2-((*R*)-3-((4-(méthylsulfonyl)phénoxy) méthyl)pipéridin-1-yl)éthanol |
| 23 | 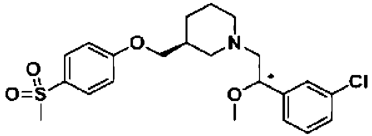 | (*S*)*-*1-((*R*) ou (*S*)-2-(3-chlorophényl)-2-méthoxyéthyl)-3-((4-(méthylsulfonyl)phénoxy) méthyl)pipéridine |
| 24 | 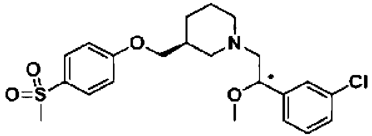 | (*S*)-1-((*S*) ou (*R*)-2-(3-chlorophényl)-2-méthoxyéthyl)-3-((4-(méthylsulfonyl)phénoxy) méthyl)pipéridine |
| 25 | 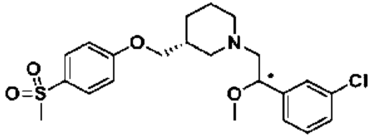 | (*R*)*-*1*-*((*R*) ou (*S*)-2-(3-chlorophényl)-2-méthoxyéthyl)-3-((4-(méthylsulfonyl)phénoxy) méthyl)pipéridine |
| 26 | 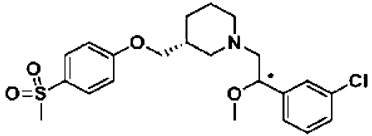 | (*R*)-1-((*S*) ou (*R*)-2-(3-chlorophényl)-2-méthoxyéthyl)-3-((4-(méthylsulfonyl)phénoxy) méthyl)pipéridine |
| 27 | 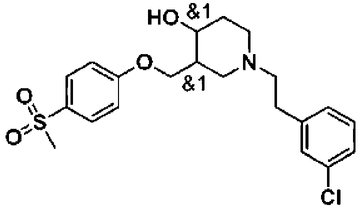 | *rac-trans* ou *cis*-1-(3-chlorophénéthyl)-3-((4-(méthylsulfonyl)phénoxy) méthyl)pipéridin-4-ol |
| 28 | 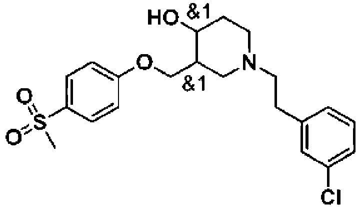 | *rac-cis* ou *trans*-1-(3-chlorophénéthyl)-3-((4-(méthylsulfonyl)phénoxy) méthyl)pipéridin-4-ol |
| 29 | 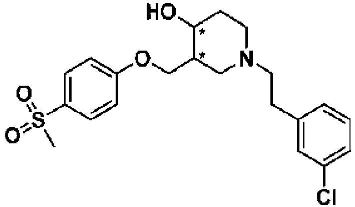 | (3S, 4R) ou (3R, 4R) ou (3S,4S) ou (3R,4S)-1-(3-chlorophénéthyl)-3-((4-(méthylsulfonyl)phénoxy) méthyl)pipéridin-4-ol |
| 30 | 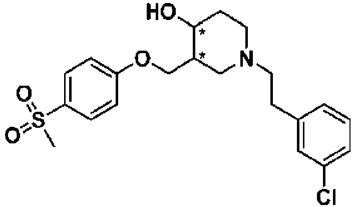 | (3R, 4R) ou (3S,4S) ou (3R, 4S) ou (3S, 4R) -1- (3-chlorophénéthyl)-3-((4-(méthylsulfonyl)phénoxy) méthyl)pipéridin-4-ol |
| 31 | 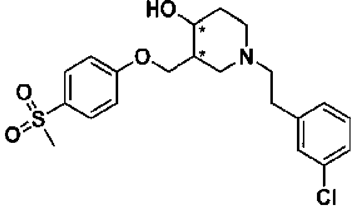 | (3R, 4R) ou (3S,4S) ou (3R, 4S) ou (3S, 4R) -1- (3-chlorophénéthyl)-3-((4-(méthylsulfonyl)phénoxy) méthyl)pipéridin-4-ol |
| 32 | 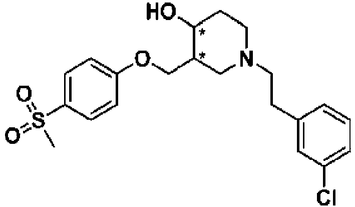 | (3S,4S) ou (3R,4S) ou (3S, 4R) ou (3R, 4R) -1- (3-chlorophénéthyl)-3-((4-(méthylsulfonyl)phénoxy) méthyl)pipéridin-4-ol |
| 33 | 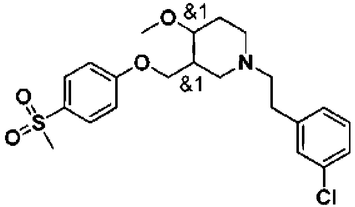 | *rac-trans* ou *cis*-1-(3-chlorophénéthyl)-4-méthoxy-3-((4-(méthylsulfonyl)phénoxy) méthyl)pipéridine |
| 34 | 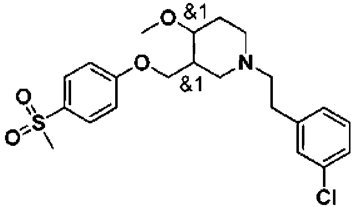 | *rac-cis* ou *trans*-1-(3-chlorophénéthyl)-4-méthoxy-3-((4-(méthylsulfonyl)phénoxy) méthyl)pipéridine |
| 35 | 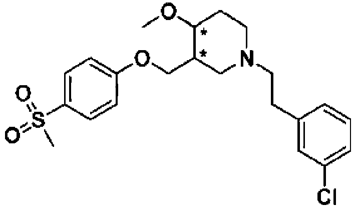 | (3S,4R) ou (3R,4R) ou (3S,4S) ou (3R,4S)-1-(3-chlorophénéthyl)-4-méthoxy-3-((4-(méthylsulfonyl)phénoxy) méthyl)pipéridine |
| 36 | 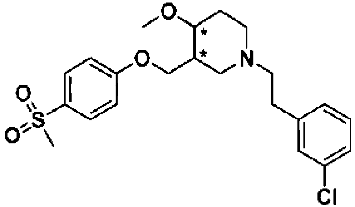 | (3R, 4R) ou (3S,4S) ou (3R, 4S) ou (3S, 4R) -1- (3-chlorophénéthyl)-4-méthoxy-3-((4-(méthylsulfonyl)phénoxy) méthyl)pipéridine |
| 37 | 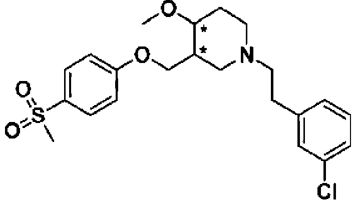 | (3R, 4R) ou (3S,4S) ou (3R, 4S) ou (3S, 4R) -1- (3-chlorophénéthyl)-4-méthoxy-3- ((4-(méthylsulfonyl)phénoxy) méthyl)pipéridine |
| 38 | 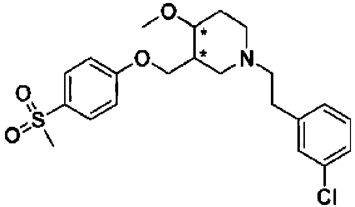 | (3S,4S) ou (3R,4S) ou (3S, 4R) ou (3R, 4R)-1-(3-chlorophénéthyl)-4-méthoxy-3-((4-(méthylsulfonyl)phénoxy) méthyl)pipéridine |
| 39 | 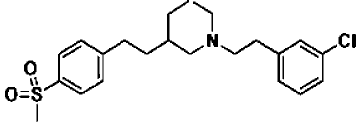 | 1-(3-chlorophénéthyl)-3-(4-(méthylsulfonyl)phénéthyl )pipéridine |
| 40 | 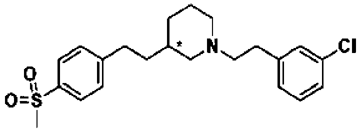 | (*S*) ou (*R*)-1-(3-chlorophénéthyl)-3-(4-(méthylsulfonyl)phénéthyl )pipéridine |
| 41 | 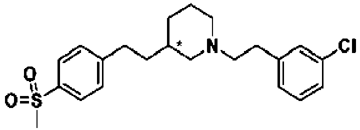 | (*R*) ou (*S*)-1-(3-chlorophénéthyl)-3-(4-(méthylsulfonyl)phénéthyl )piperidine |
| 42 | 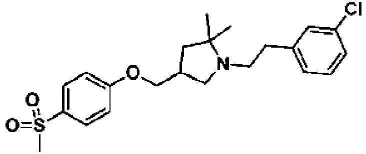 | 1-(3-chlorophenethyl)-2,2-diméthyl-4-((4-(méthylsulfonyl)phénoxy) méthyl)pyrrolidine |
| 43 | 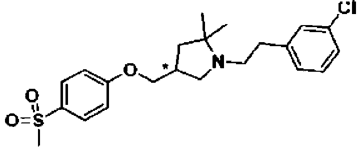 | (*S*) ou (*R*)-1-(3-chlorophénéthyl)-2,2-diméthyl-4-((4-(méthylsulfonyl)phénoxy) méthyl)pyrrolidine |
| 44 | 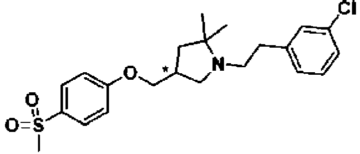 | (*R*) ou (*S*)-1-(3-chlorophénéthyl)-2,2-diméthyl-4-((4-(méthylsulfonyl)phénoxy)méthyl) pyrrolidine |
| 45 | 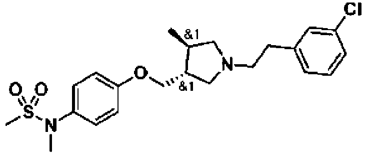 | *rac-trans-N-*(4-(1-(3-chlorophénéthyl)-4-méthylpyrrolidin-3-yl)méthoxy)phényl)-*N-*méthylméthanesulfonamide |
| 46 | 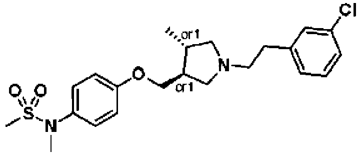 | *N*-(4-((3S,4S) ou (3R, 4R)-(1-(3-chlorophénéthyl)-4-méthylpyrrolidin-3-yl)méthoxy)phényl)-*N-*méthylméthanesulfonamide |
| 47 | 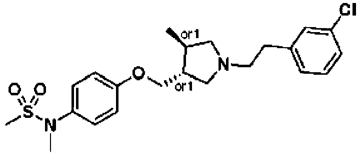 | *N*-(4-((3R, 4R) ou (3S, 4S)-(1-(3-chlorophénéthyl)-4-méthylpyrrolidin-3-yl)méthoxy)phényl)-*N-*méthylméthanesulfonamide |
| 48 | 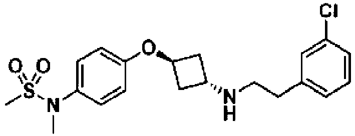 | *trans-N-*(4-(3-((3-chlorophénéthyl)amino)cy clobutoxy)phényl)-*N-*méthylméthanesulfonamide |
| 49 | 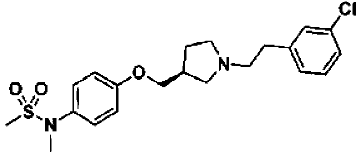 | (*S*)*-N-*(4-((1-(3-chlorophénéthyl) pyrrolidin-3-yl)méthoxy)phényl)-*N-*méthylméthanesulfonamide |
| 50 | 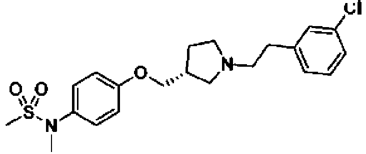 | (*R*)*-N-*(4-((1-(3-chlorophénéthyl) pyrrolidin-3-yl)méthoxy)phényl)-*N-*méthylméthanesulfonamide |
| 51 | 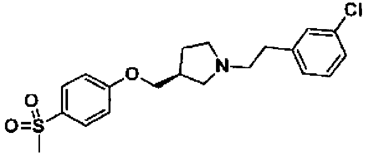 | (*S*)-1-(3-chlorophénéthyl)-3-((4-(méthylsulfonyl)phénoxy) méthyl)pyrrolidine |
| 52 | 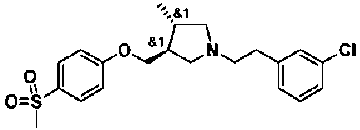 | *rac-trans-*1-(3-chlorophénéthyl)-3-méthyl-4-((4-(méthylsulfonyl)phénoxy) méthyl)pyrrolidine |
| 53 | 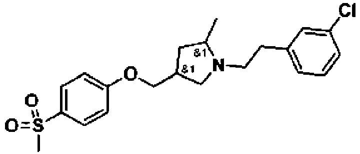 | *rac-trans* ou *cis*-1-(3-chlorophénéthyl)-2-méthyl-4-((4-(méthylsulfonyl)phénoxy) méthyl)pyrrolidine |
| 54 | 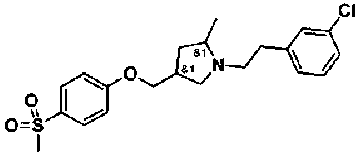 | *rac-cis* ou *trans*-1-(3-chlorophénéthyl)-2-méthyl-4-((4-(méthylsulfonyl)phénoxy) méthyl)pyrrolidine |
| 55 | 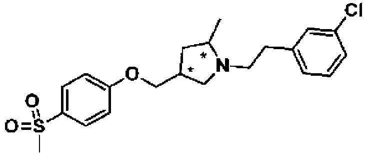 | (2R, 4S) ou (2R, 4R) ou (2S,4R) ou (2S, 4S)-1-(3-chlorophénéthyl)-2-méthyl-4-((4-(méthylsulfonyl)phénoxy) méthyl)pyrrolidine |
| 56 | 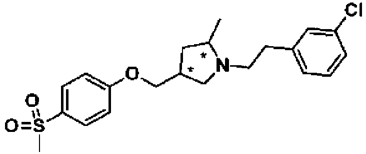 | (2R, 4R) ou (2S, 4R) ou (2S,4S) ou (2R, 4S)-1-(3-chlorophénéthyl)-2-méthyl-4-((4-(méthylsulfonyl)phénoxy) méthyl)pyrrolidine |
| 57 | 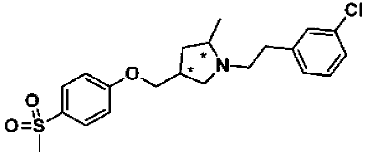 | (2S, 4R) ou (2S, 4S) ou (2R, 4S) ou (2R, 4R)-1-(3-chlorophénéthyl)-2-méthyl-4-((4-(méthylsulfonyl)phénoxy) méthyl)pyrrolidine |
| 58 | 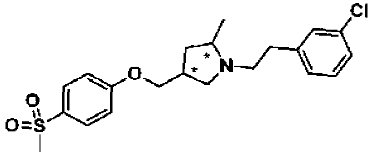 | (2S,4S) ou (2R,4S) ou (2R,4R) ou (2S, 4R)-1-(3-chlorophénéthyl)-2-méthyl-4-((4-(méthylsulfonyl)phénoxy) méthyl)pyrrolidine |
| 59 | 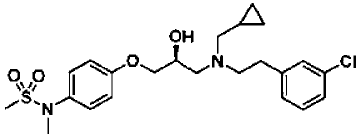 | (*S*)-*N*-(4-(3-((3-chlorophénéthyl) (cyclopr opylméthyl)amino)-2-hydroxypropoxy)phényl)-*N-*méthylméthanesulfonamide |
| 60 | 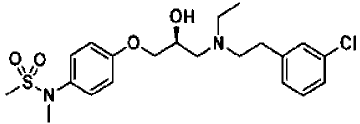 | (*S*)-*N*-(4-(3-((3-chlorophénéthyl)(éthyl) amino)-2-hydroxypropoxy)phényl)-*N-*méthylméthanesulfonamide |
| 61 | 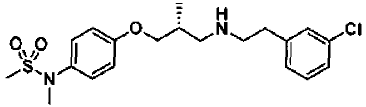 | (*R*)*-N-*(4-(3-((3-chlorophnéthyl)amino)-2-méthylpropoxy)phényl)-N-méthylméthanesulfonamide |
| 62 | 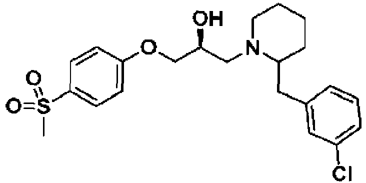 | (2*S*)-1-(2-(3-chlorobenzyl)pipéridin-1-yl)-3-(4-(méthylsulfonyl)phénoxy) propan-2-ol |
| 63 | 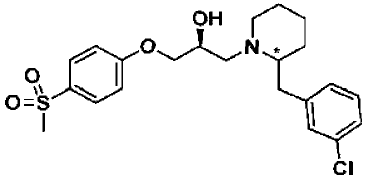 | (*S*)-1-((*S*) ou (*R*)*-*2-(3-chlorobenzyl)pipéridin-1-yl)-3-(4-(méthylsulfonyl)phénoxy) propan-2-ol |
| 64 | 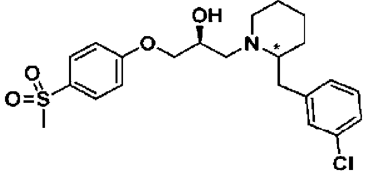 | (*S*)-1-((*R*) ou (*S*)-2-(3-chlorobenzyl)pipéridin-1-yl)-3-(4-(méthylsulfonyl)phénoxy) propan-2-ol |
| 65 | 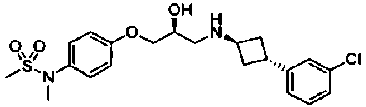 | *N-*(4-((*S*)-3-((trans-3-(3-chlorophényl)cyclobutyl) amino)-2-hydroxypropoxy)phényl)-*N-*méthylméthanesulfonamide |
| 66 | 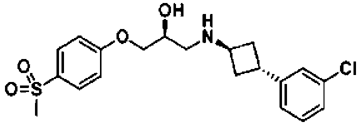 | (*S*)-1-((*trans-*3-(3-chlorophényl)cyclobutyl) amino)-3-(4-(méthylsulfonyl)phénoxy) propan-2-ol |
| 67 | 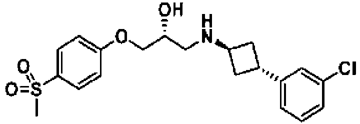 | (*R*)*-*1*-*((t*rans*-3-(3-chlorophényl)cyclobutyl) amino)-3-(4-(méthylsulfonyl)phénoxy) propan-2-ol |
| 68 | 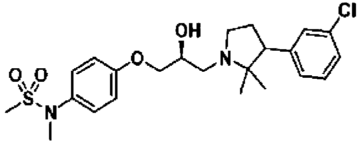 | *N-*(4-((2*S*)*-*3-(3-(3-chlorophényl)-2,2-diméthylpyrrolidin-1-yl)-2-hydroxypropoxy)phényl)-*N-*méthylméthanesulfonamide |
| 69 | 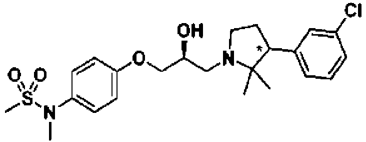 | *N-*(4-((*S*)*-*3*-*((*S*) ou (*R*)-3-(3-chlorophényl)-2,2-diméthylpyrrolidin-1-yl)-2-hydroxypropoxy)phényl)-*N-*méthylméthanesulfonamide |
| 70 | 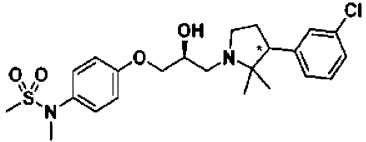 | *N*-(4-((*S*)-3-((*R*) ou (*S*)-3-(3-chlorophényl)-2,2-diméthylpyrrolidin-1-yl)-2-hydroxypropoxy)phényl)-*N-*méthylméthanesulfonamide |
| 71 | 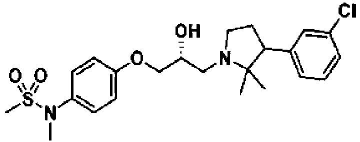 | *N*-(4-((2*R*)-3-(3-(3-chlorophényl)-2,2-diméthylpyrrolidin-1-yl)-2-hydroxypropoxy)phényl)-*N-*méthylméthanesulfonamide |
| 72 | 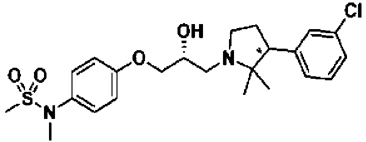 | *N-*(4-((*R*)-3-((*S*) ou (*R*)-3-(3-chlorophényl)-2,2-diméthylpyrrolidin-1-yl)-2-hydroxypropoxy)phényl)-*N-*méthylméthanesulfonamide |
| 73 | 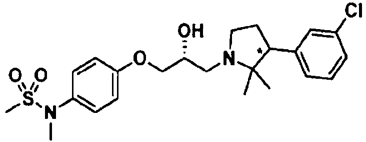 | *N*-(4-((*R*)-3-((*R*) ou (*S*)-3-(3-chlorophényl)-2,2-diméthylpyrrolidin-1-yl)-2-hydroxypropoxy)phényl)-*N-*méthylméthanesulfonamide |
| 74 | 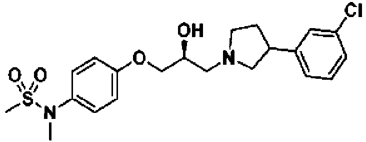 | *N-*(4-((2*S*)-3-(3-(3-chlorophényl)pyrrolidin-1-yl)-2-hydroxypropoxy)phényl)-*N-*méthylméthanesulfonamide |
| 75 | 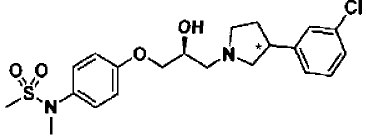 | *N*-(4-((*S*)*-*3-((*S*) ou (*R*)-3-(3-chlorophényl)pyrrolidin-1-yl)-2-hydroxypropoxy)phényl)-*N-*méthylméthanesulfonamide |
| 76 | 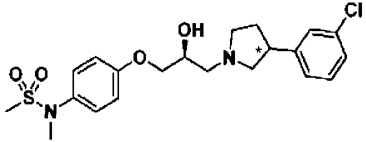 | *N*-(4-((*S*)-3-((*R*) ou (*S*)-3-(3-chlorophényl)pyrrolidin-1-yl)-2-hydroxypropoxy)phényl)-*N-*méthylméthanesulfonamide |
| 77 | 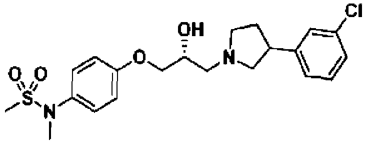 | *N*-(4-((2*R*)-3-(3-(3-chlorophényl)pyrrolidin-1-yl)-2-hydroxypropoxy)phényl)-*N-*méthylméthanesulfonamide |
| 78 | 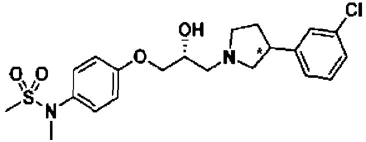 | *N-*(4-((*R*)-3-((*S*) ou (*R*)-3-(3-chlorophényl)pyrrolidin-1-yl)-2-hydroxypropoxy)phényl)-*N-*méthylméthanesulfonamide |
| 79 | 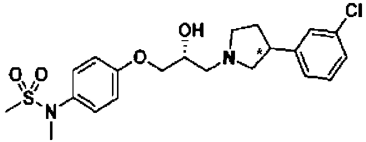 | *N*-(4-((*R*)-3-((*R*) ou (*S*)-3-(3-chlorophényl)pyrrolidin-1-yl)-2-hydroxypropoxy)phényl)-*N-*méthylméthanesulfonamide |
| 80 | 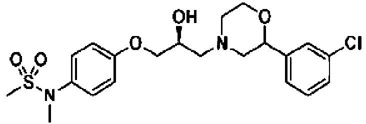 | *N*-(4-((2*S*)-3-(2-(3-chlorophényl)morpholino) -2-hydroxypropoxy)phényl)-*N-*méthylméthanesulfonamide |
| 81 | 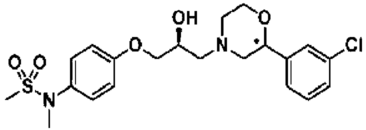 | *N*-(4-((*S*)-3-((*S*) ou (*R*)-2-(3-chlorophényl)morpholino) -2-hydroxypropoxy)phényl)-*N-*méthylméthanesulfonamide |
| 82 | 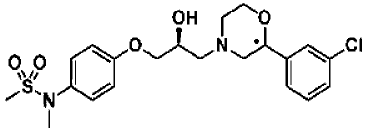 | *N*-(4-((*S*)-3-((*R*) ou (*S*)-2-(3-chlorophényl)morpholino) -2-hydroxypropoxy)phényl)-*N-*méthylméthanesulfonamide |
| 83 | 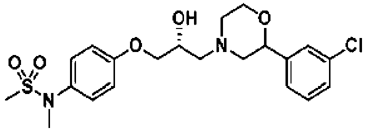 | *N*-(4-((2*R*)-3-(2-(3-chlorophényl)morpholino) -2-hydroxypropoxy)phényl)-*N-*méthylméthanesulfonamide |
| 84 | 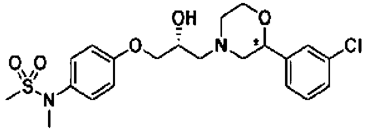 | *N-*(4-((*R*)-3-((*S*) ou (*R*)-2-(3-chlorophényl)morpholino) -2-hydroxypropoxy)phényl)-*N-*méthylméthanesulfonamide |
| 85 | 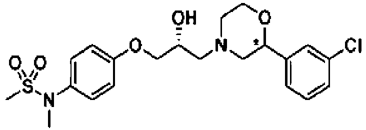 | *N*-(4-((*R*)-3-((*R*) ou (*S*)-2-(3-chlorophényl)morpholino) -2-hydroxypropoxy)phényl)-*N-*méthylméthanesulfonamide |
| 86 | 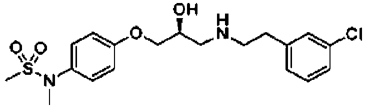 | (*S*)-*N*-(4-(3-((3-chlorophényl)amino)-2-hydroxypropoxy)phényl)-*N-*méthylméthanesulfonamide |
| 87 | 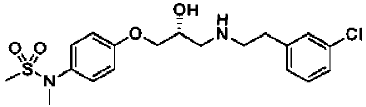 | (*R*)*-N-*(4-(3-((3-chlorophényl)amino)-2-hydroxypropoxy)phényl)-*N-*méthylméthanesulfonamide |
| 88 | 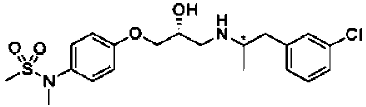 | *N*-(4-((*R*)-3-(((*S*) ou (R)-1-(3-chlorophényl)propan-2-yl)amino)-2-hydroxypropoxy)phényl)-*N-*méthylméthanesulfonamide |
| 89 | 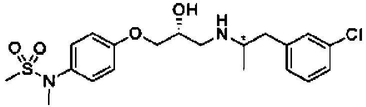 | *N*-(*4-*((*R*)-3-(((*R*) ou (S)-1-(3-chlorophényl)propan-2-yl)amino)-2-hydroxypropoxy)phényl)-N-méthylméthanesulfonamide |
| 90 | 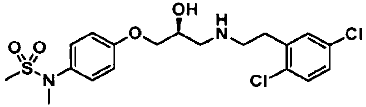 | (*S*)-*N*-(4-(3-((2,5-dichlorophénéthyl) amino) -2-hydroxypropoxy)phényl)-N-méthylméthanesulfonamide |
| 91 | 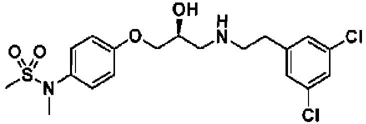 | (*S*)*-N-*(4-(3-((3,5-dichlorophénéthyl)amino) -2-hydroxypropoxy)phényl)-*N-*méthylméthanesulfonamide |
| 92 | 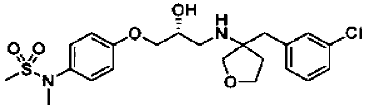 | *N-*(4-((2*R*)-3-((3-(3-chlorobenzyl)tétrahydrof uran-3-yl)amino)-2-hydroxypropoxy)phényl)-*N-*méthylméthanesulfonamide |
| 93 | 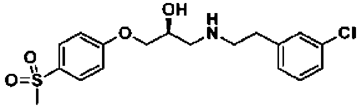 | (*S*)-1-((3-chlorophénéthyl)amino)-3-(4-(méthylsulfonyl)phénoxy) propan-2-ol |
| 94 | 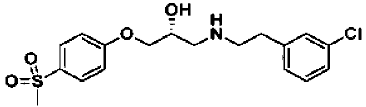 | (*R*)*-*1*-*((3-chlorophénéthyl)amino)-3-(4-(méthylsulfonyl)phénoxy) propan-2-ol |
| 95 | 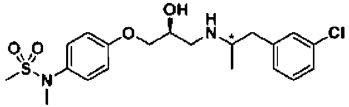 | *N*-(4-((*S*)-3-(((*S*) ou (*R*)-1-(3-chlorophényl)propan-2-yl)amino)-2-hydroxypropoxy)phényl)-*N-*méthylméthanesulfonamide |
| 96 | 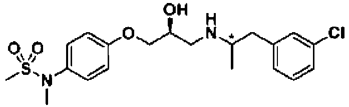 | *N*-(4-((*S*)-3-(((*R*) ou (*S*)-1-(3-chlorophényl)propan-2-yl)amino)-2-hydroxypropoxy)phényl)-*N-*méthylméthanesulfonamide |
| 97 | 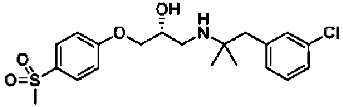 | (*R*)*-*1-((1-(3-chlorophényl)-2-méthylpropan-2-yl)amino)-3-(4-(méthylsulfonyl)phénoxy)propan-2-ol |
| 98 | 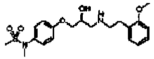 | (*S*)-N-(4*-*(2-hydroxy-3-((2-méthoxyphénéthyl)amino)p ropoxy)phényl)-*N-*méthylméthanesulfonamide |
| 99 | 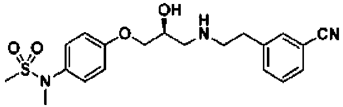 | (*S*)-*N*-(4-(3-((3-cyanophénéthyl)amino)-2-hydroxypropoxy)phényl)-*N-*méthylméthanesulfonamide |
| 100 | 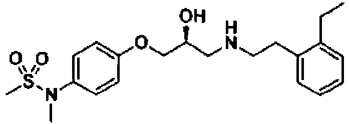 | (*S*)-*N*-(4-(3-((2-éthylphénéthyl)amino)-2-hydroxypropoxy)phényl)-*N-*méthylméthanesulfonamide |
| 101 | 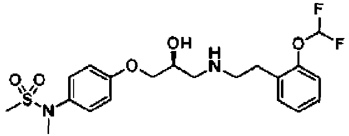 | (*S*)*-N*-(4-(3-((2-(difluorométhoxy)phénéth yl)amino)-2-hydroxypropoxy)phényl)-*N-*méthylméthanesulfonamide |
| 102 | 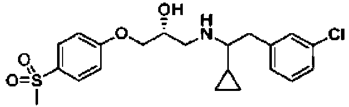 | (2*R*)-1-((2-(3-chlorophényl)-1-cyclopropyléthyl)amino)-3-(4-(méthylsulfonyl)phénoxy) propan-2-ol |
| 103 | 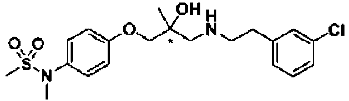 | (*R*) ou (*S*)-*N*-(4-(3-((3-chlorophénéthyl)amino)-2-hydroxy-2-méthylpropoxy)phényl)-*N-*méthylméthanesulfonamide |
| 104 | 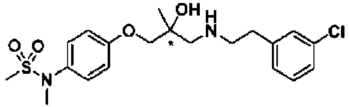 | (*S*) ou (*R*)*-N*-(4-(3-((3-chlorophénéthyl)amino)-2-hydroxy-2-méthylpropoxy)phényl)-N-méthylméthanesulfonamide |
| 105 | 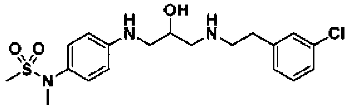 | *N*-(4-((3-((3-chlorophénéthyl)amino)-2-hydroxypropyl)amino)phén yl)-*N-*méthylméthanesulfonamide |
| 106 | 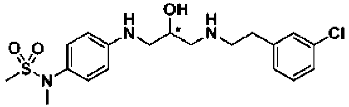 | (*R*) ou (*S*)*-N-*(4-((3-((3-chlorophénéthyl)amino)-2-hydroxypropyl)amino)phén yl)-*N-*méthylméthanesulfonamide |
| 107 | 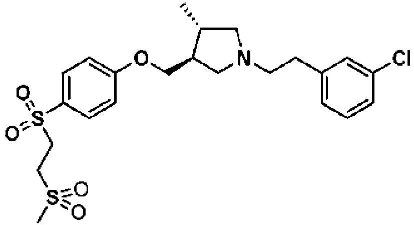 | (3S,4S)-1-[2-(3-chlorophényl)éthyl]-3-{[4-(2-méthoxyéthanesulfonyl)ph énoxy]méthyl}-4-méthylpyrrolidine |
| 108 | 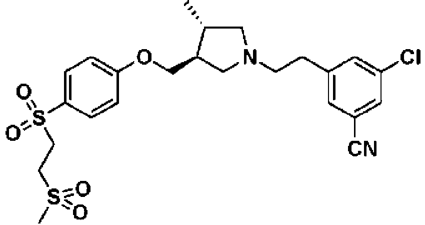 | 3-chloro-5-{2-[(3S,4S)-3-{[4-(2-méthanesulfonyléthanesul fonyl)phénoxy]méthyl}-4-méthylpyrrolidin-1-yl]éthyl}benzonitrile |
| 109 | 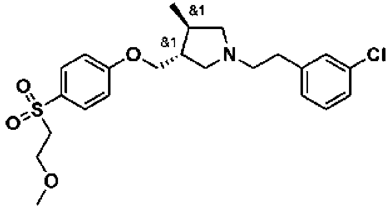 | *rac-trans*-1-[2-(3-chlorophényl)éthyl]-3-{[4-(2-méthoxyéthanesulfonyl)ph énoxy]méthyl}-4-méthylpyrrolidine |
| 110 | 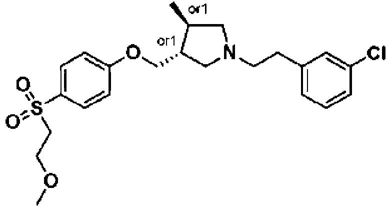 | (3R,4R ou 3S,4S)-1-[2-(3-chlorophényl)éthyl]-3-{[4-(2-méthoxyéthanesulfonyl)ph énoxy]méthyl}-4-méthylpyrrolidine |
| 111 | 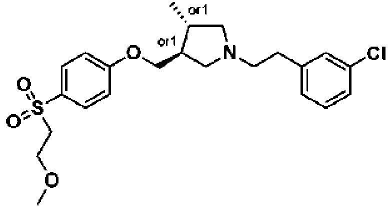 | (3S,4S ou 3R,4R)-1-[2-(3-chlorophényl)éthyl]-3-{[4-(2-méthoxyéthanesulfonyl)ph énoxy]méthyl}-4-méthylpyrrolidine |
| 112 | 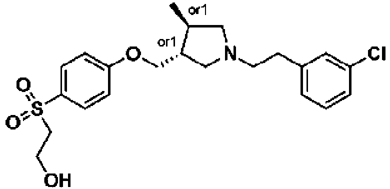 | 2-(4-{[(3R,4R ou 3S,4S)-1-[2-(3-chlorophényl)éthyl]-4-méthylpyrrolidin-3-yl]méthoxy}benzènesulfon yl]éthan-1-ol |
| 113 | 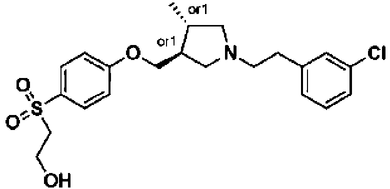 | 2-(4-{[(3S,4S ou 3R,4R)-1-[2-(3-chlorophényl)éthyl]-4-méthylpyrrolidin-3-yl]méthoxy}benzènesulfon yl]éthan-1-ol |
| 114 | 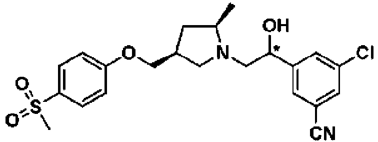 | 3-chloro-5-[(1S ou 1R)-1-hydroxy-2-[(2R,4S)-4-[(4-méthanesulfonylphénoxy)m éthyl]-2-méthylpyrrolidin-1-yl]éthyl]benzonitrile |
| 115 | 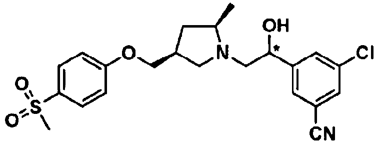 | 3-chloro-5-[(1R ou 1S)-1-hydroxy-2-[(2R,4S)-4-[(4-méthanesulfonylphénoxy)m éthyl]-2-méthylpyrrolidin-1-yl]éthyl]benzonitrile |
| 116 | 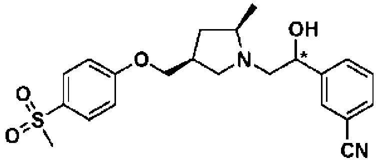 | 3-[(1R ou 1S) -1-hydroxy-2-[(2R,4S)-4-[(4-méthanesulfonylphénoxy)m éthyl]-2-méthylpyrrolidin-1-yl]éthyl]benzonitrile |
| 117 | 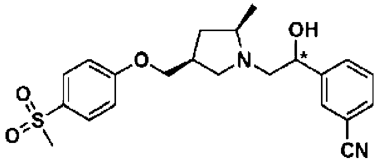 | 3-[(1S ou 1R)-1-hydroxy-2-[(2R,4S)-4-[(4-méthanesulfonylphénoxy)m éthyl]-2-méthylpyrrolidin-1-yl]éthyl]benzonitrile |
| 118 | 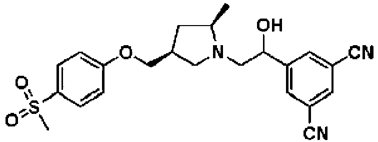 | 5-[(1R ou 1S)-1-hydroxy-2-[(2R,4S)-4-[(4-méthanesulfonylphénoxy)m éthyl]-2-méthylpyrrolidin-1-yl]éthyl]benzène-1,3-diarbonitrile |
| 119 | 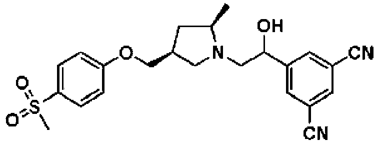 | 5-[(1S ou 1R)-1-hydroxy-2-[(2R,4S)-4-[(4-méthanesulfonylphénoxy)m éthyl]-2-méthylpyrrolidin-1-yl]éthyl]benzène-1,3-diarbonitrile |
| 120 | 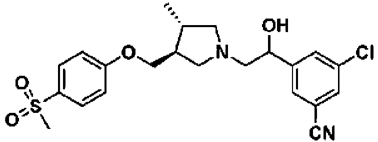 | 3-chloro-5-{1-hydroxy-2-[(3S,4S)-3-[(4-méthanesulfonylphénoxy)m éthyl]-4-méthylpyrrolidin-1-yl]éthyl}benzonitrile |
| 121 | 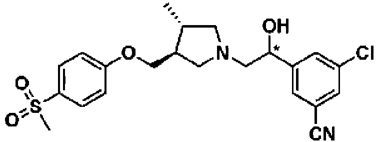 | 3-chloro-5-[(1S ou 1R)-1-hydroxy-2-[(3S,4S)-3-[(4-méthanesulfonylphénoxy)m éthyl]-4-méthylpyrrolidin-1-yl]éthyl]benzonitrile |
| 122 | 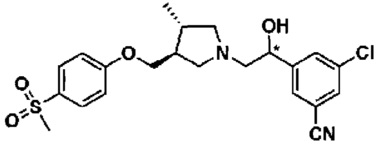 | 3-chloro-5-[(1R ou 1S)-1-hydroxy-2-[(3S,4S)-3-[(4-méthanesulfonylphénoxy)m éthyl]-4-méthylpyrrolidin-1-yl]éthyl]benzonitrile |
| 123 | 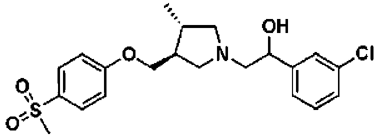 | 1-(3-chlorophényl)-2-((3S,4S)-3-méthyl-4-((4-(méthylsulfonyl)phénoxy) méthyl)pyrrolidin-1-yl)éthan-1-ol |
| 124 | 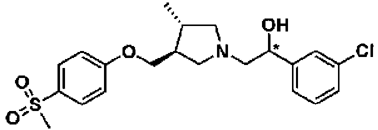 | (1R ou 1S)-1-(3-chlorophényl)-2-[(3S,4S) -3- [(4-méthanesulfonylphénoxy)m éthyl]-4-méthylpyrrolidin-1-yl]éthan-1-ol |
| 125 | 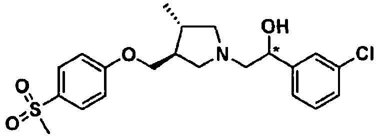 | (1S ou 1R)-1-(3-chlorophényl)-2-[(3S,4S)-3-[(4-méthanesulfonylphénoxy)m éthyl]-4-méthylpyrrolidin-1-yl]éthan-1-ol |
| 126 | 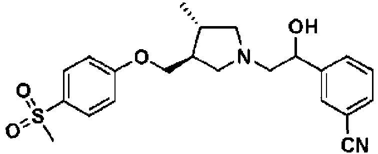 | 3-[1-hydroxy-2-[(3S,4S)-3-[(4-méthanesulfonylphénoxy)m éthyl]-4-méthylpyrrolidin-1-yl]éthyl]benzonitrile |
| 127 | 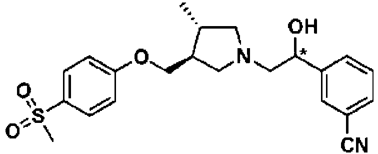 | 3-[(1S ou 1R)-1-hydroxy-2-[(3S,4S)-3-[(4-méthanesulfonylphénoxy)m éthyl]-4-méthylpyrrolidin-1-yl]éthyl]benzonitrile |
| 128 | 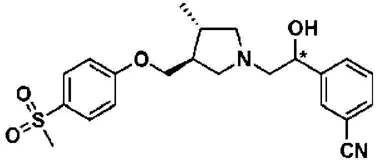 | 3-[(1R ou 1S)-1-hydroxy-2-[(3S,4S)-3-[(4-méthanesulfonylphénoxy)m éthyl]-4-méthylpyrrolidin-1-yl]éthyl]benzonitrile |
| 129 | 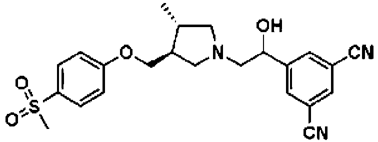 | 5-{1-hydroxy-2-[(3S,4S)-3-[(4-méthanesulfonylphénoxy)m éthyl]-4-méthylpyrrolidin-1-yl]éthyl}benzène-1,3-diarbonitrile |
| 130 | 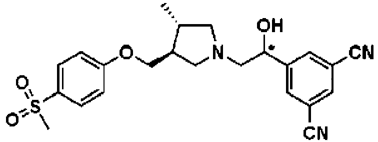 | 5-[(1R ou 1S)-1-hydroxy-2-[(3S,4S)-3-[(4-méthanesulfonylphénoxy)m éthyl]-4-méthylpyrrolidin-1-yl]éthyl]benzène-1,3-diarbonitrile |
| 131 | 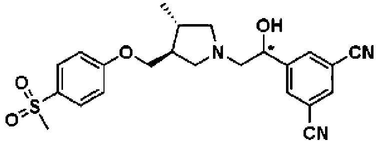 | 5-[(1S ou 1R)-1-hydroxy-2-[(3S,4S)-3-[(4-méthanesulfonylphénoxy)m éthyl]-4-méthylpyrrolidin-1-yl]éthyl]benzène-1,3-diarbonitrile |
| 132 | 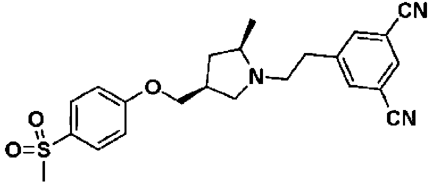 | 5-{2-[(2R,4S)-4-[(4-méthanesulfonylphénoxy)m éthyl]-2-méthylpyrrolidin-1-yl]éthyl}benzène-1,3-dicarbonitrile |
| 133 | 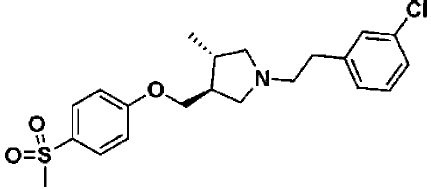 | (3S,4S)-1-[2-(3-chlorophényl)éthyl]-3-[(4-méthanesulfonylphénoxy)m éthyl]-4-méthylpyrrolidine |
| 134 | 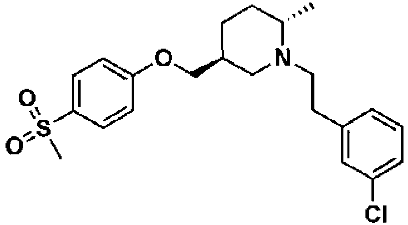 | (2S,5S)-1-[2-(3-chlorophényl)éthyl]-5-[(4-méthanesulfonylphénoxy)m éthyl]-2-méthylpipéridine |
| 135 | 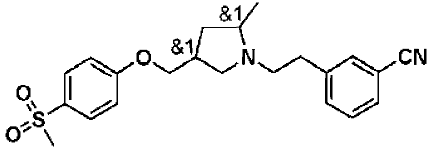 | *rac-cis* ou *trans*-3-{2-[4-[(4-méthanesulfonylphénoxy)m éthyl]-2-méthylpyrrolidin-1-yl]éthyl}benzonitrile |
| 136 | 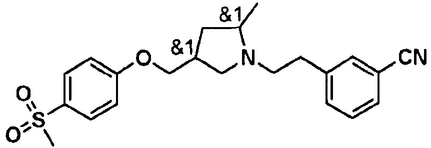 | *rac-trans* ou *cis*-3-{2-(4-[(4-méthanesulfonylphénoxy)m éthyl]-2-méthylpyrrolidin-1-yl]éthyl}benzonitrile |
| 137 | 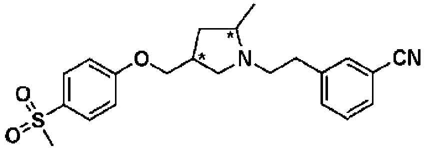 | 3-{2-[(2S,4R ou 2R,4S ou 2R,4R ou 2S,4S)-4-[(4-méthanesulfonylphénoxy)m éthyl]-2-méthylpyrrolidin-1-yl]éthyl}benzonitrile |
| 138 | 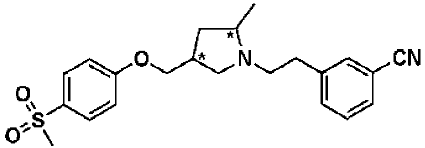 | 3-{2-[(2R,4S ou 2S,4R ou 2R,4R ou 2S,4S)-4-[(4-méthanesulfonylphénoxy)m éthyl]-2-méthylpyrrolidin-1-yl]éthyl}benzonitrile |
| 139 | 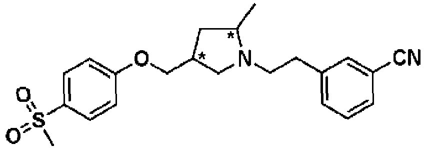 | 3-{2-[(2S,4S ou 2S,4R ou 2R,4S ou 2R,4R)-4-[(4-méthanesulfonylphénoxy)m éthyl]-2-méthylpyrrolidin-1-yl]éthyl}benzonitrile |
| 140 | 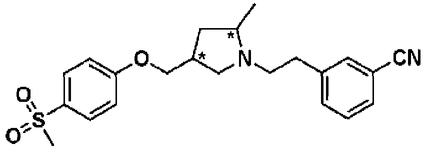 | 3-{2-[(2R,4R ou 2S,4S ou 2S,4R ou 2R,4S)-4-[(4-méthanesulfonylphénoxy)m éthyl]-2-méthylpyrrolidin-1-yl]éthyl}benzonitrile |
| 141 | 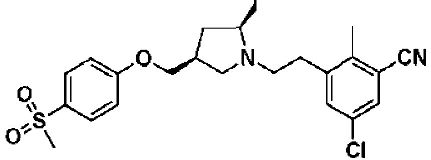 | 5-chloro-3-{2-[(2R,4S)-4-[(4-méthanesulfonylphénoxy)m éthyl]-2-méthylpyrrolidin-1-yl]éthyl}-2-méthylbenzonitrile |
| 142 | 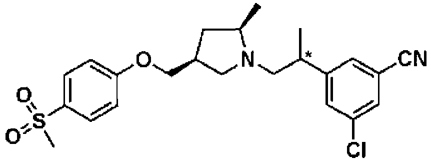 | 3-chloro-5-[(2R ou 2S)-1-[(2R,4S)-4-[(4-méthanesulfonylphénoxy)m éthyl]-2-méthylpyrrolidin-1-yl]propan-2-yl]benzonitrile |
| 143 | 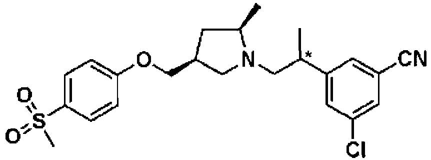 | 3-chloro-5-[(2S ou 2R)-1-[(2R,4S)-4-[(4-méthanesulfonylphénoxy)m éthyl]-2-méthylpyrrolidin-1-yl]propan-2-yl]benzonitrile |
| 144 | 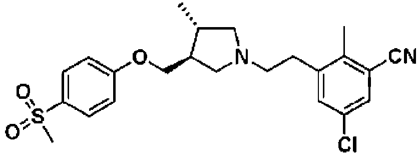 | 5-chloro-3-{2-[(3S,4S)-3-[(4-méthanesulfonylphénoxy)m éthyl]-4-méthylpyrrolidin-1-yl]éthyl}-2-méthylbenzonitrile |
| 145 | 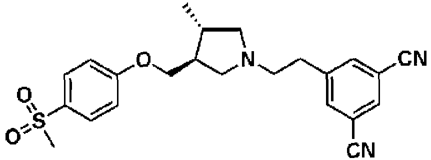 | 5-{2-[(3S,4S)-3-[(4-méthanesulfonylphénoxy)m éthyl]-4-méthylpyrrolidin-1-yl]éthyl}benzène-1,3-dicarbonitrile |
| 146 | 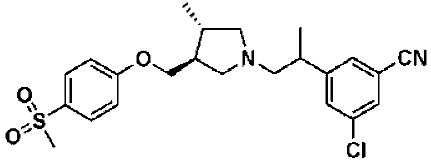 | 3-chloro-5-[1-[(3S,4S)-3-[(4-méthanesulfonylphénoxy)m éthyl]-4-méthylpyrrolidin-1-yl]propan-2-yl]benzonitrile |
| 147 | 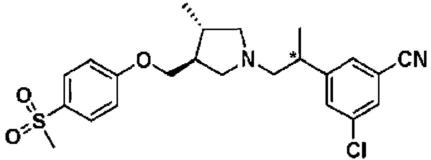 | 3-chloro-5-[(2R ou 2S)-1-[(3S,4S)-3-[(4-méthanesulfonylphénoxy)m éthyl]-4-méthylpyrrolidin-1-yl]propan-2-yl]benzonitrile |
| 148 | 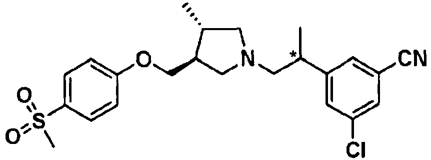 | 3-chloro-5-[(2S ou 2R)-1-[(3S,4S)-3-[(4-méthanesulfonylphénoxy)m éthyl]-4-méthylpyrrolidin-1-yl]propan-2-yl]benzonitrile |
| 149 | 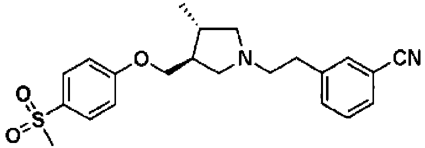 | 3-{2-[(3S,4S)-3-[(4-méthanesulfonylphénoxy)m éthyl]-4-méthylpyrrolidin-1-yl]éthyl}benzonitrile |
| 150 | 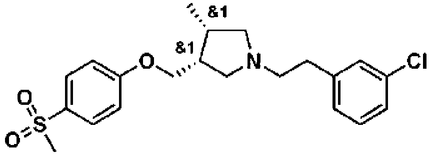 | *rac-cis-*1-[2-(3-chlorophényl)éthyl]-3-[(4-méthanesulfonylphénoxy)m éthyl]-4-méthylpyrrolidine |
| 151 | 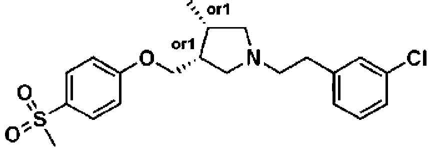 | (3R,4S ou 3S,4R)-1-[2-(3-chlorophényl)éthyl]-3-[(4-méthanesulfonylphénoxy)m éthyl]-4-méthylpyrrolidine |
| 152 | 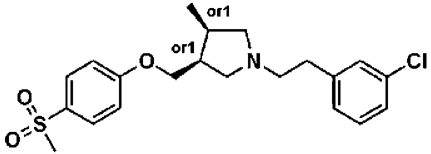 | (3S,4R ou 3R,4S)-1-[2-(3-chlorophényl)éthyl]-3-[(4-méthanesulfonylphénoxy)m éthyl]-4-méthylpyrrolidine |
| 153 | 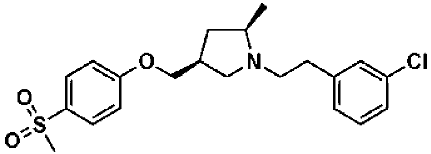 | (2R,4S)-1-[2-(3-chlorophényl)éthyl]-4-[(4-méthanesulfonylphénoxy)m éthyl]-2-méthylpyrrolidine |
| 154 | 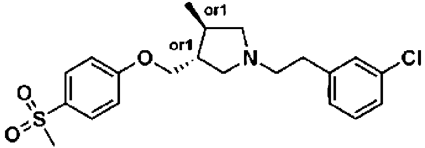 | (3R,4R ou 3S,4S)-1-[2-(3-chlorophényl)éthyl]-3-[(4-méthanesulfonylphénoxy)m éthyl]-4-méthylpyrrolidine |
| 155 | 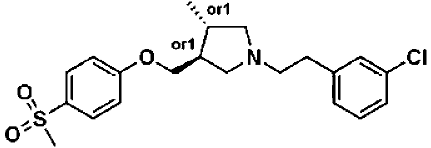 | (3S,4S ou 3R,4R)-1-[2-(3-chlorophényl)éthyl]-3-[(4-méthanesulfonylphénoxy)m éthyl]-4-méthylpyrrolidine |
| 156 | 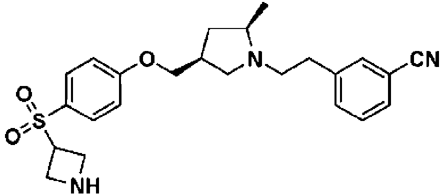 | 3-{2-[(2R,4S)-4-{[4-(azétidine-3-sulfonyl)phénoxy]méthyl} -2-méthylpyrrolidin-1-yl]éthyl}benzonitrile |
| 157 | 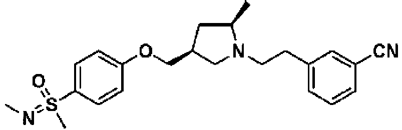 | 3-{2-[(2R,4S)-2-méthyl-4-({4-[méthyl(méthylimino)oxo-λ⁶-sulfanyl]phénoxy]méthyl) pyrrolidin-1-yl]éthyl}benzonitrile |
| 158 | 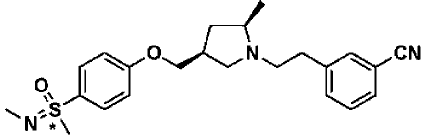 | 3-{2-[(2R,4S)-2-méthyl-4-({4-[(R ou S)méthyl(méthylimino)oxo -λ⁶-sulfanyl]phénoxy]méthyl) pyrrolidin-1-yl]éthyl}benzonitrile |
| 159 | 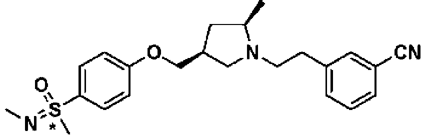 | 3-{2-[(2R,4S)-2-méthyl-4-({4-[(S ou R)méthyl(méthylimino)oxo -λ⁶-sulfanyl]phénoxy]méthyl) pyrrolidin-1-yl]éthyl}benzonitrile |
| 160 | 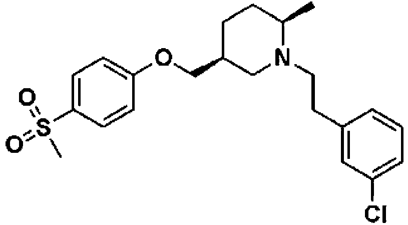 | (2R,5S)-1-[2-(3-chlorophényl)éthyl]-5-[(4-méthanesulfonylphénoxy)m éthyl]-2-méthylpipéridine |
| 161 | 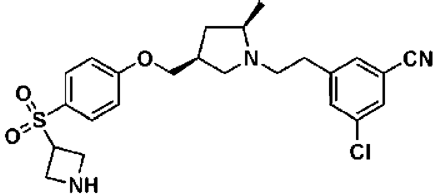 | 3-{2-[(2R,4S)-4-{[4-(azétidine-3-sulfonyl)phénoxy]méthyl} -2-méthylpyrrolidin-1-yl]éthyl}-5-chlorobenzonitrile |
| 162 | 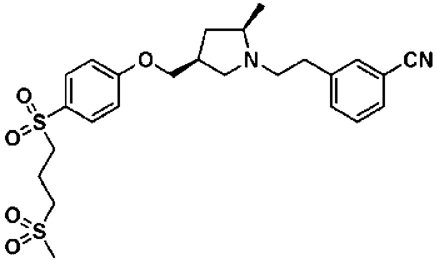 | 3-{2-[(2R,4S)-4-{[4-(3-méthanesulfonylpropanesu lfonyl)phénoxy]méthyl}-2-méthylpyrrolidin-1-yl]éthyl}benzonitrile |
| 163 | 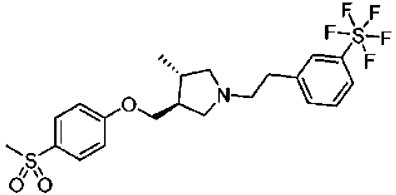 | (3S,4S)-3-[(4-méthanesulfonylphénoxy)m éthyl]-4-méthyl-1-{2-[3-(pentafluoro-λ⁶-sulfanyl)phényl]éthyl}py rrolidine |
| 164 | 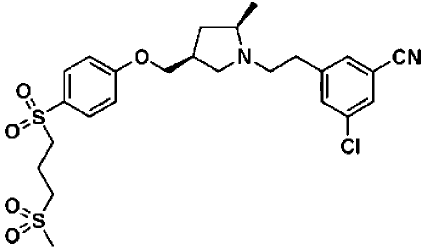 | 3-chloro-5-{2-[(2R,4S)-4-{[4-(3-méthanesulfonylpropanesu lfonyl)phénoxy]méthyl}-2-méthylpyrrolidin-1-yl]éthyl}benzonitrile |
| 165 | 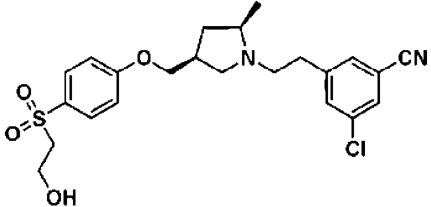 | 3-chloro-5-{2-[(2R,4S)-4-{[4-(2-hydroxyéthanesulfonyl)ph énoxy]méthyl}-2-méthylpyrrolidin-1-yl]éthyl}benzonitrile |
| 166 | 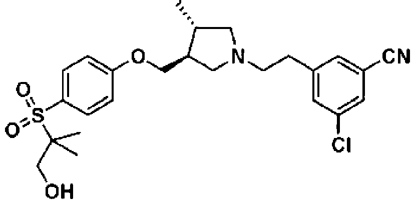 | 3-chloro-5-{2-[(3S,4S)-3-{[4-(1-hydroxy-2-méthylpropane-2-sulfonyl)phénoxy]méthyl} -4-méthylpyrrolidin-1-yl]éthyl}benzonitrile |
| 167 | 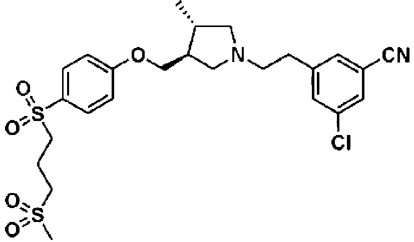 | 3-chloro-5-{2-[(3S,4S)-3-{[4-(3-méthanesulfonylpropanesu lfonyl)phénoxy]méthyl}-4-méthylpyrrolidin-1-yléthyl}benzonitrile |
| 168 | 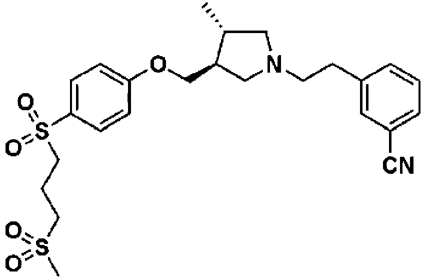 | 3-{2-[(3S,4S)-3-{[4-(3-méthanesulfonylpropanesu lfonyl)phénoxy]méthyl}-4-méthylpyrrolidin-1-yléthyl}benzonitrile |
| 169 | 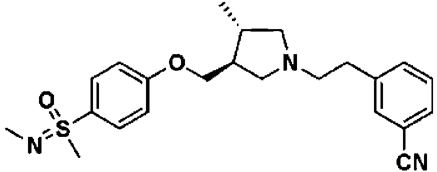 | 3-{2-[(3S,4S)-3-méthyl-4-({4-[méthyl(méthylimino)oxo-λ⁶-sulfanyl]phénoxy}méthyl) pyrrolidin-1-yl]éthyl}benzonitrile |
| 170 | 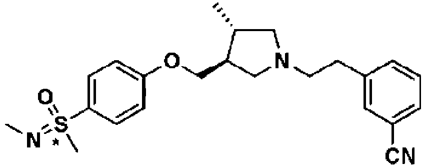 | 3-{2-[(3S,4S)-3-méthyl-4-({4-[(R ou S)-méthyl(méthylimino)oxo-λ⁶-sulfanyl]phénoxy}méthyl) pyrrolidin-1-yl]éthyl}benzonitrile |
| 171 | 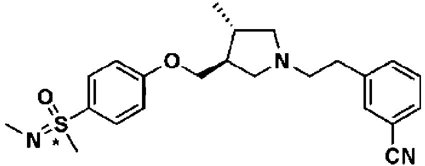 | 3-{2-[(3S,4S)-3-méthyl-4-({4-[(S ou R)-méthyl(méthylimino)oxo-λ⁶-sulfanyl]phénoxy}méthyl) pyrrolidin-1-yl]éthyl}benzonitrile |
| 172 | 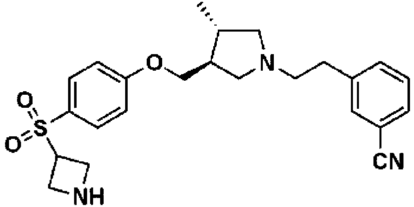 | 3-{2- [(3S, 4S) -3-{ [4-(azétidine-3-sulfonyl)phénoxy]méthyl} -4-méthylpyrrolidin-1-yl]éthyl}benzonitrile |
| 173 | 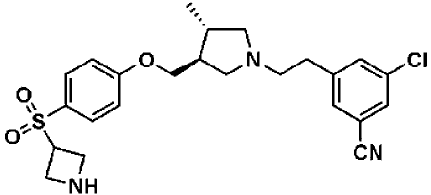 | 3-{2-[(3S,4S)-3-{[4-(azétidine-3-sulfonyl)phénoxy]méthyl} -4-méthylpyrrolidin-1-yl]éthyl}-5-chlorobenzonitrile |
| 174 | 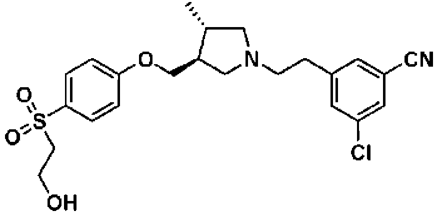 | 3-chloro-5-{2-[(3S,4S)-3-{[4-(2-hydroxyéthanesulfonyl)ph énoxy]méthyl}-4-méthylpyrrolidin-1-yl]éthyl}benzonitrile |
| 175 | 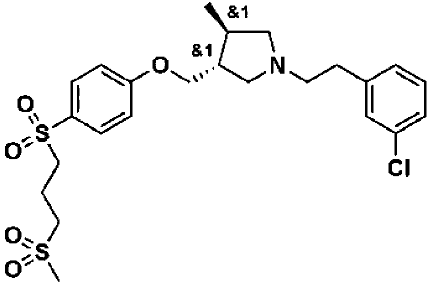 | *rac-trans*-1-[2-(3-chlorophényl)éthyl]-3-{[4-(3-méthanesulfonylpropanesu lfonyl)phénoxy]méthyl}-4-méthylpyrrolidine |
| 176 | 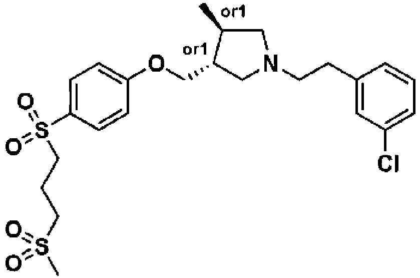 | (3R,4R ou 3S,4S)-1-[2-(3-chlorophényl)éthyl]-3-{[4-(3-méthanesulfonylpropanesu lfonyl)phénoxy]méthyl}-4-méthylpyrrolidine |
| 177 | 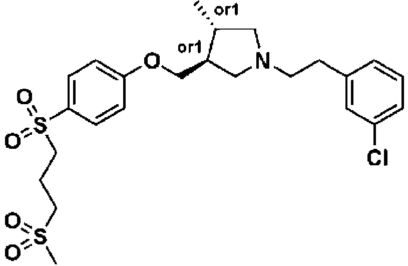 | (3S,4S ou 3R,4R)-1-[2-(3-chlorophényl)éthyl]-3-{[4-(3-méthanesulfonylpropanesu lfonyl)phénoxy]méthyl}-4-méthylpyrrolidine |
| 181 | 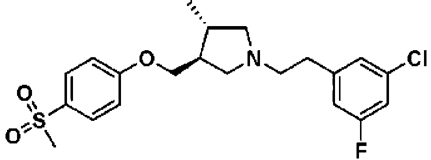 | (3S,4S)-1-[2-(3-chloro-5-fluorophényl)éthyl]-3-[(4-méthanesulfonylphénoxy)m éthyl]-4-méthylpyrrolidine |
| 182 | 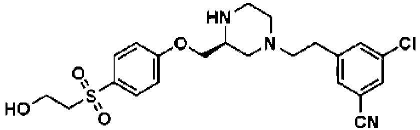 | 3-chloro-5-{2-[(3S)-3-{[4-(2-hydroxyéthanesulfonyl)ph énoxy]méthyl}pipérazin-1-yl]éthyl}benzonitrile |
| 183 | 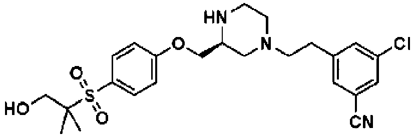 | 3-chloro-5-{2-[(3S)-3-{[4-(1-hydroxy-2-méthylpropane-2-sulfonyl)phénoxy]méthyl} pipérazin-1-yl]éthyl}benzonitrile |
| 184 | 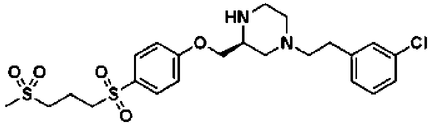 | (3S)-1-[2-(3-chlorophényl)éthyl]-3-{[4-(3-méthanesulfonylpropanesu lfonyl)phénoxy]méthyl}pi pérazine |
| 185 | 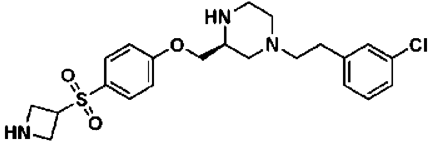 | (3S)-3-{[4-(azétidine-3-sulfonyl)phénoxy]méthyl} -1-[2-(3-chlorophényl)éthyl]pipér azine |
| 186 | 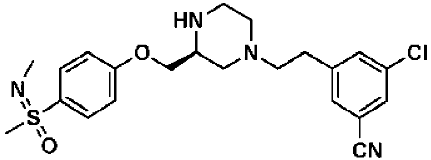 | 3-chloro-5-{2-[(3S)-3-({4-[méthyl(méthylimino)oxo-λ⁶ -sulfanyl]phénoxy}méthyl) pipérazin-1-yl]éthyl}benzonitrile |
| 187 | 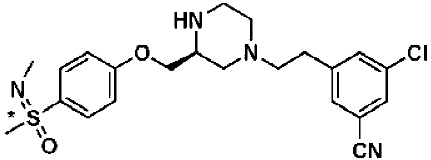 | 3-chloro-5-{2-[(3S)-3-({4-[(S ou R)-méthyl(méthylimino)oxo-λ⁶-sulfanyl]phénoxy}méthyl) pipérazin-1-yl]éthyl]benzonitrile |
| 188 | 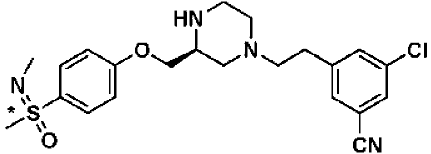 | 3-chloro-5-{2-[(3S)-3-({4-[(R ou S)-méthyl(méthylimino)oxo-λ⁶-sulfanyl]phénoxy}méthyl) pipérazin-1-yl]éthyl]benzonitrile |
| 189 | 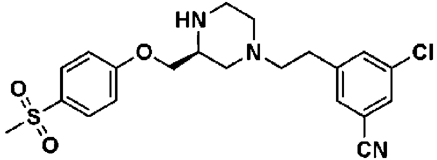 | 3-chloro-5-{2-[(3S)-3-[(4-méthanesulfonylphénoxy)m éthyl]pipérazin-1-yl]éthyl}benzonitrile |
| 190 | 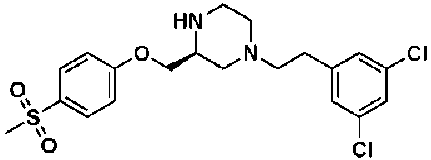 | (3S)-1-[2-(3,5-dichlorophényl)éthyl]-3-[(4-méthanesulfonylphénoxy)m éthyl]pipérazine |
| 191 | 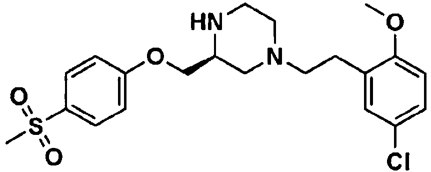 | (3S)-1-[2-(5-chloro-2-méthoxyphényl)éthyl]-3-[(4-méthanesulfonylphénoxy)m éthyl]pipérazine |
| 192 | 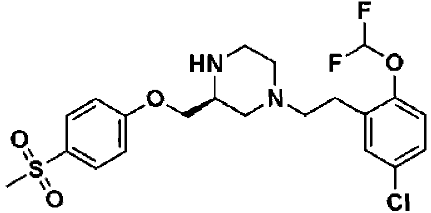 | (3S)-1-{2-[5-chloro-2-(difluorométhoxy)phényl] éthyl}-3-[(4-méthanesulfonylphénoxy)m éthyl]pipérazine |
| 193 | 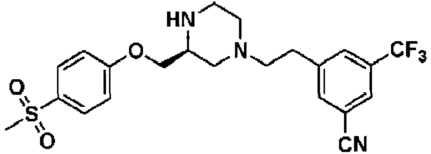 | 3-{2-[(3S)-3-[(4-méthanesulfonylphénoxy)m éthyl]pipérazin-1-yl]éthyl}-5-(trifluorométhyl)benzoni trile |
| 194 | 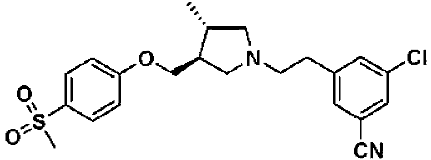 | 3-chloro-5-{2-[(3S,4S)-3-[(4-méthanesulfonylphénoxy)m éthyl]-4-méthylpyrrolidin-1-yl]éthyl}benzonitrile |
| 195 | 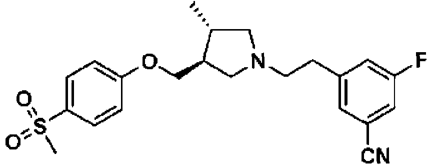 | 3-fluoro-5-{2-[(3S,4S)-3-[(4-méthanesulfonylphénoxy)m éthyl]-4-méthylpyrrolidin-1-yl]éthyl}benzonitrile |
| 196 | 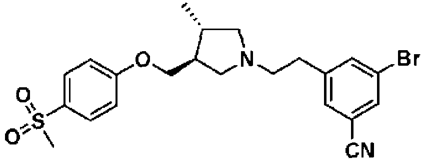 | 3-bromo-5-{2-[(3S,4S)-3-[(4-méthanesulfonylphénoxy)m éthyl]-4-méthylpyrrolidin-1-yl]éthyl}benzonitrile |
| 197 | 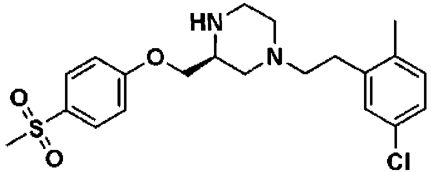 | (3S)-1-[2-(5-chloro-2-méthylphényl)éthyl]-3-[(4-méthanesulfonylphénoxy)m éthyl]pipérazine |
| 198 | 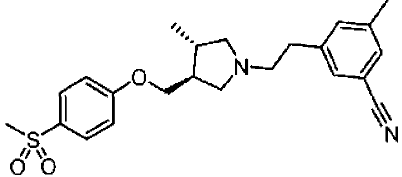 | 3-{2-[(3S,4S)-3-[(4-méthanesulfonylphénoxy) méthyl]-4-méthylpyrrolidin-1-yl]éthyl}-5-méthylbenzonitrile |
| 199 | 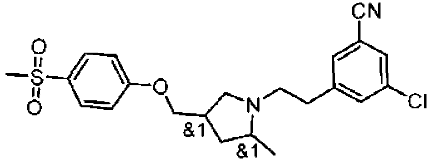 | *rac-cis* ou *trans*-3-chloro-5-{2-[4-[(4-méthanesulfonylphénoxy) méthyl]-2-méthylpyrrolidin-1-yl]éthyl}benzonitrile |
| 200 | 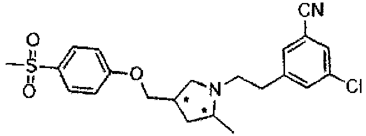 | 3-chloro-5-{2-[(2R,4S ou 2S,4R ou 2R,4R ou 2S,4S)-4-[(4-méthanesulfonylphénoxy) méthyl]-2-méthylpyrrolidin-1-yl]éthyl}benzonitrile |
| 201 | 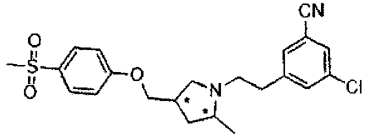 | 3-chloro-5-{2-[(2S,4R ou 2R,4S ou 2R,4R ou 2S,4S)-4-[(4-méthanesulfonylphénoxy) méthyl]-2-méthylpyrrolidin-1-yl]éthyl}benzonitrile |
| 202 | 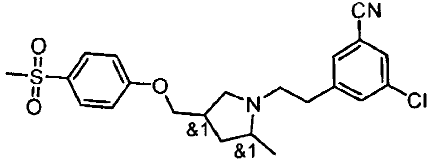 | *rac-trans* ou *cis* -3-chloro-5-{2-[4-[(4-méthanesulfonylphénoxy) méthyl]-2-méthylpyrrolidin-1-yl]éthyl}benzonitrile |
| 203 | 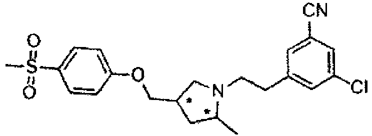 | 3-chloro-5-(2-((2R,4S ou 2S,4R ou 2R,4R ou 2S,4S)-2-méthyl-4-((4-(méthylsulfonyl)phénoxy) méthyl)pyrrolidin-1-yl)éthyl)benzonitrile |
| 204 | 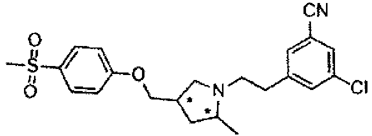 | 3-chloro-5-(2-((2R,4S ou 2S,4R ou 2S,4S ou 2R,4R)-2-methyl-4-((4-(méthylsulfonyl)phénoxy) méthyl)pyrrolidin-1-yl)éthyl)benzonitrile |
| 205 | 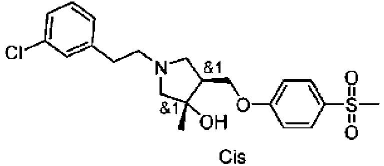 | *rac-cis*-1-[2-(3-chlorophényl)éthyl]-4-[(4-méthanesulfonylphénoxy) méthyl]-3-méthylpyrrolidin-3-ol |
| 206 | 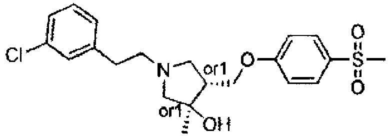 | (3R,4R ou 3S,4S)-1-[2-(3-chlorophényl)éthyl]-4-[(4-méthanesulfonylphénoxy) méthyl]-3-méthylpyrrolidin-3-ol |
| 207 | 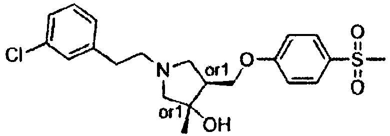 | (3S,4S ou 3R,4R)-1-[2-(3-chlorophényl)éthyl]-4-[(4-méthanesulfonylphénoxy) méthyl]-3-méthylpyrrolidin-3-ol |
| 208 | 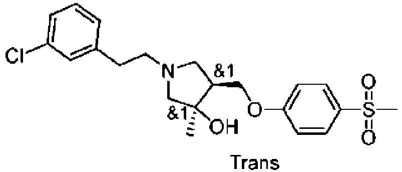 | *rac-trans*-1-[2-(3-chlorophényl)éthyl]-4-[(4-méthanesulfonylphénoxy) méthyl]-3-méthylpyrrolidin-3-ol |
| 209 | 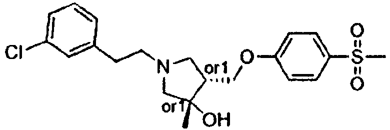 | (3R,4S ou 3S,4R)-1-[2-(3-chlorophényl)éthyl]-4-[(4-méthanesulfonylphénoxy) méthyl]-3-méthylpyrrolidin-3-ol |
| 210 | 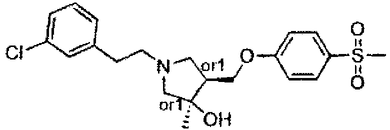 | (3S,4R ou 3R,4S)-1-[2-(3-chlorophényl)éthyl]-4-[(4-méthanesulfonylphénoxy) méthyl]-3-méthylpyrrolidin-3-ol |
| 211 | 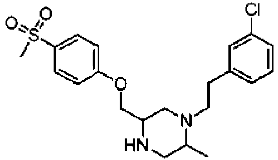 | 1-[2-(3-chlorophényl)éthyl]-5-[(4-méthanesulfonylphénoxy) méthyl]-2-méthylpipérazine |
| 212 | 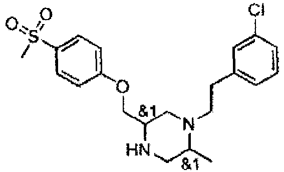 | *rac-cis* ou *trans*-1-[2-(3-chlorophényl)éthyl]-5-[(4-méthanesulfonylphénoxy) méthyl]-2-méthylpipérazine |
| 213 | 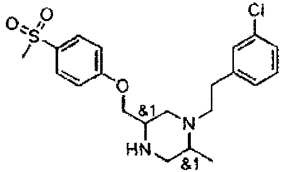 | *rac-trans* ou *cis*-1-[2-(3-chlorophényl)éthyl]-5-[(4-méthanesulfonylphénoxy) méthyl]-2-méthylpipérazine |
| 214 | 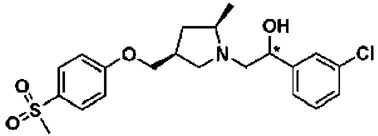 | (1R) ou (1S)-1-(3-chlorophényl)-2-[(2R,4S)-4-[(4-méthanesulfonylphénoxy) méthyl]-2-méthylpyrrolidin-1-yl]éthan-1-ol |
| 215 | 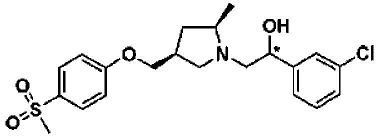 | (1S) ou (1R)-1-(3-chlorophényl)-2-[(2R,4S)-4-[(4-méthanesulfonylphénoxy) méthyl]-2-méthylpyrrolidin-1-yl]éthan-1-ol |
ou un stéréoisomère ou un tautomère de ceux-ci, ou un sel pharmaceutiquement acceptable de l'un quelconque des précédents.

13. Composition pharmaceutique comprenant (i) un composé selon l'une quelconque des revendications 1 à 12, ou un stéréoisomère ou un tautomère de celui-ci, ou un sel pharmaceutiquement acceptable de l'un quelconque des précédents, et (ii) un ou plusieurs excipients pharmaceutiquement acceptables.

14. Composé selon l'une quelconque des revendications 1 à 12, ou un stéréoisomère ou un tautomère de celui-ci, ou un sel pharmaceutiquement acceptable de l'un quelconque des précédents, ou composition pharmaceutique selon la revendication 13, pour une utilisation dans le traitement ou le retardement du développement d'une maladie, d'un trouble ou d'une affection choisi(e) parmi :
une maladie rénale ;
une maladie, un trouble ou une affection choisi(e) dans le groupe constitué par une maladie rénale chronique, une glomérulosclérose segmentaire et focale (GSF), une maladie rénale associée à une hypertension, une néphropathie associée au virus de l'immunodéficience humaine (HIVAN), une néphropathie drépanocytaire, une néphrite lupique, une maladie rénale diabétique, une néphropathie associée à l'APOL1, une néphropathie virale, une néphropathie associée à la COVID-19, une prééclampsie et une septicémie.

15. Kit comprenant (i) un composé selon l'une quelconque des revendications 1 à 12, ou un stéréoisomère ou un tautomère de celui-ci, ou un sel pharmaceutiquement acceptable de l'un quelconque des précédents, ou une composition pharmaceutique selon la revendication 13, et (ii) des instructions pour une utilisation dans le traitement d'une maladie, d'un trouble ou d'une affection médié(e) par APOL1 chez un individu en ayant besoin ;
dans lequel la maladie, le trouble ou l'affection est éventuellement :
une maladie rénale ; ou
une maladie, un trouble ou une affection choisi(e) dans le groupe constitué par une maladie rénale chronique, une glomérulosclérose segmentaire et focale (GSF), une maladie rénale associée à une hypertension, une néphropathie associée au virus de l'immunodéficience humaine (HIVAN), une néphropathie drépanocytaire, une néphrite lupique, une maladie rénale diabétique, une néphropathie associée à l'APOL1, une néphropathie virale, une néphropathie associée à la COVID-19, une prééclampsie et une septicémie ;
éventuellement dans lequel l'individu présente une mutation APOL1, telle qu'une mutation à gain de fonction.
